(19) Europäisches Patentamt
European Patent Office
Office européen des brevets

(11) EP 3 456 814 A2

(12) **EUROPEAN PATENT APPLICATION**

(43) Date of publication:
**20.03.2019 Bulletin 2019/12**

(21) Application number: **18187541.0**

(22) Date of filing: **10.05.2011**

(51) Int Cl.:
***C12N 1/21*** *(2006.01)*
***C12N 9/02*** *(2006.01)*
***C12N 9/10*** *(2006.01)*
***C12N 15/52*** *(2006.01)*
***C12P 7/64*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2011 US 201113006933**
**14.01.2011 US 201113007100**

(62) Document number(s) of the earlier application(s) in accordance with Art. 76 EPC:
**11722657.1 / 2 663 636**

(71) Applicant: **REG Life Sciences, LLC**
**South San Francisco, CA 94080 (US)**

(72) Inventors:
- **Lee, Grace J.**
  **South San Francisco, CA 94080 (US)**
- **Zakharyevich, Kseniya**
  **South San Francisco, CA 94080 (US)**
- **Baron, Tarah S.**
  **South San Francisco, CA 94080 (US)**
- **Schirmer, Andreas**
  **South San Francisco, CA 94080 (US)**
- **Greenfield, Derek L.**
  **South San Francisco, CA 94080 (US)**
- **Hu, Zhihao**
  **South San Francisco, CA 94080 (US)**
- **Holden, Kevin**
  **South San Francisco, CA 94080 (US)**

(74) Representative: **J A Kemp**
**14 South Square**
**Gray's Inn**
**London WC1R 5JJ (GB)**

Remarks:
•This application was filed on 06-08-2018 as a divisional application to the application mentioned under INID code 62.
•Claims filed after the date of receipt of the divisional application (Rule 68(4) EPC).

# (54) PRODUCTION OF BRANCHED CHAIN FATTY ACIDS AND DERIVATIVES THEREOF IN RECOMBINANT MICROBIAL CELLS

(57) Recombinant microbial cells are provided which have been engineered to produce branched chain products such as branched fatty acid derivatives by the fatty acid biosynthetic pathway, and methods of making branched fatty acid derivatives using the recombinant microbial cells.



Printed by Jouve, 75001 PARIS (FR)

## Description

### CROSS-REFERENCE TO RELATED APPLICATIONS

This application is a continuation-in-part of copending U.S. Patent Application No. 13/006,933, filed January 14, 2011, and this application is a continuation-in-part of copending U.S. Patent Application No. 13/007,100, filed January 14, 2011, the entire contents of which are hereby incorporated by reference.

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 631,495 Byte ASCII (Text) file named "708122_ST25.TXT," created on May 10, 2011.

### BACKGROUND

Crude petroleum is a very complex mixture containing a wide range of hydrocarbons. It is converted into a diversity of fuels and chemicals through a variety of chemical processes in refineries. Crude petroleum is a source of transportation fuels as well as a source of raw materials for producing petrochemicals. Petrochemicals are used to make specialty chemicals such as plastics, resins, fibers, elastomers, pharmaceuticals, lubricants, and gels.

Branched hydrocarbons, branched fatty acids and other branched chain fatty acid derivatives (including branched fatty esters, branched fatty aldehydes, and branched fatty alcohols) are known to have additional preferred properties when compared to straight-chain molecules of same molecular weight (i.e., isomers), such as considerably lower melting points which can in turn confer lower pour points when made into industrial chemicals. These additional benefits allow the branched hydrocarbons, branched fatty acids, and other branched fatty acid derivates to confer substantially lower volatility and vapor pressure, and improved stability against oxidation and rancidity, thus making them particularly suited as components or feedstock of cosmetic and pharmaceutical applications, or as components of plasticizers for synthetic resins, solvents for solutions for printing ink and specialty inks, and industrial lubricants.

Such additional preferred properties can also be obtained in unsaturated fatty acid derivatives (including unsaturated hydrocarbons, unsaturated fatty acids, and other unsaturated fatty acid derivates), typically with high degrees of unsaturation, but unsaturation promotes oxidation and can lead to short shelf lives and corrosion problems. Therefore, lower melting points, pour points, volatility, and vapor pressure, as well as improved oxidative stability, are better obtained through branching.

Obtaining branched specialty chemicals from crude petroleum requires a significant financial investment as well as a great deal of thermal energy. It is also an inefficient process because frequently the long chain hydrocarbons in crude petroleum are cracked to produce smaller monomers. These monomers are then used as the raw material to manufacture the more complex specialty chemicals. Furthermore, in the petrochemical industry, it is commonplace to obtain branched chemicals, such as, for example, branched alkanes, branched alkenes, branched fatty acids, branched fatty esters, branched fatty alcohols and branched fatty aldehydes by isomerization of straight-chain hydrocarbons, using various catalytic processes. Expensive catalysts are typically employed in these processes, therefore increasing the costs of manufacturing. The catalysts that are used often become undesirable contaminants that must be removed from the finished products, thus adding further costs to the processes.

The most important transportation fuels -- gasoline, diesel, and jet fuel -- contain distinctively different mixtures of hydrocarbons which are tailored toward optimal engine performance. For example, gasoline comprises predominantly straight chain , branched chain, and aromatic hydrocarbons ranging from about 4 to 12 carbon atoms, while diesel predominantly comprises straight chain hydrocarbons ranging from about 9 to 23 carbon atoms. Diesel fuel quality is evaluated by parameters such as cetane number, kinematic viscosity, oxidative stability, and cloud point (Knothe G., Fuel Process Technol. 86:1059-1070 (2005)). These parameters, among others, are impacted by the hydrocarbon chain length as well as by the degree of branching or saturation of the hydrocarbon.

Microbially-produced fatty acids and other fatty acid derivatives (such as fatty esters, fatty alcohols, and hydrocarbons) can be readily tailored by genetic manipulation. Metabolic engineering enables microbial strains to produce different mixtures of fatty acids and other fatty acid derivatives, which can be optimized in meeting or exceeding fuel standards, and can be tailored to produce other chemicals or precursor molecules that are typically petroleum derived.

There is a need for cost-effective alternatives to petroleum products that do not require exploration, extraction, transportation over long distances, substantial refinement, and avoid the types of environmental damage associated with processing of petroleum. For similar reasons, there is a need for alternative sources of chemicals which are typically derived from petroleum. There is also a need for efficient and cost-effective methods for producing high-quality biofuels, fuel alternatives, and industrial chemicals from renewable energy sources.

Recombinant microbial cells engineered to produce branched chain fatty acids and other branched chain fatty acid derivatives, methods using these recombinant microbial cells to produce branched chain fatty acid derivatives, and compositions produced by these methods, address these needs.

## SUMMARY

The present invention provides novel recombinant microbial cells which produce branched chain fatty acid derivatives. The invention also provides methods of making branched chain fatty acid derivatives comprising culturing recombinant microbial cell of the invention, and other features apparent upon further review.

In a first aspect, the invention provides a recombinant microbial cell comprising: (a) polynucleotides encoding a branched chain alpha-keto acid dehydrogenase (BKD) complex, comprising polypeptides having branched-chain alpha-keto acid dehydrogenase activity, lipoamide acyltransferase activity, and dihydrolipoamide dehydrogenase activity, and (b) polynucleotides encoding a polypeptide having beta-ketoacyl-ACP synthase activity that utilizes a branched acyl-CoA molecule as a substrate, wherein at least one polynucleotide according to (a) or (b) encodes a polypeptide that is exogenous to the recombinant microbial cell or expression of said polynucleotide is modulated in the recombinant microbial cell; and comprising one or more polynucleotides each which encodes a polypeptide having fatty acid derivative enzyme activity, wherein the recombinant microbial cell produces a branched fatty acid derivative when cultured in the presence of a carbon source under conditions effective to express the polynucleotides. In some embodiments, expression of the at least one polynucleotide according to (a) or (b) is modulated by overexpression of the polynucleotide, such as by operatively linking the polynucleotide to an exogenous promoter.

In some embodiments, the recombinant microbial cell according to the first aspect produces a fatty acid derivative composition when the cell is cultured in a culture medium containing a carbon source under conditions effective to express the polynucleotides, the fatty acid derivative composition comprising straight chain fatty acid derivatives and branched chain fatty acid derivatives, and the branched chain fatty acid derivatives comprising anteiso-branched fatty acid derivatives and iso-branched fatty acid derivatives.

In some embodiments, at least 5%, at least 10%, at least 20%, at least 30%, at least 40%, at least 50%, at least 60%, at least 70%, at least 80% or at least 90% of the fatty acid derivatives in the composition produced by the microbial cell of the first aspect are branched chain fatty acid derivatives. In some embodiments, at least 10%, at least 20%, at least 30%, at least 40%, at least 50%, at least 60%, at least 70%, at least 80% or at least 90% of the branched chain fatty acid derivatives in the composition produced by the microbial cell of the first aspect are iso-branched fatty acid derivatives. In some embodiments, at least 10%, at least 20%, at least 30%, at least 40%, at least 50%, at least 60%, at least 70%, at least 80% or at least 90% of the branched fatty acid derivatives in the composition produced by the microbial cell of the first aspect are anteiso-branched fatty acid derivatives. In some embodiments, the recombinant microbial cell of the first aspect produces at least 10 mg/L, at least 25 mg/L, at least 100 mg/L, at least 200 mg/L, at least 500 mg/L, at least 1000 mg/L, or at least 2000 mg/L branched chain fatty acid derivatives when cultured in a culture medium containing a carbon source under conditions effective to express the polynucleotides.

In a second aspect, the invention provides a recombinant microbial cell comprising: (a) polynucleotides encoding a branched chain alpha-keto acid dehydrogenase (BKD) complex, comprising polypeptides having branched-chain alpha-keto acid dehydrogenase activity, lipoamide acyltransferase activity, and dihydrolipoamide dehydrogenase activity, and (b) polynucleotides encoding a polypeptide having beta-ketoacyl-ACP synthase activity that utilizes a branched acyl-CoA molecule as a substrate, and further comprising : (c) polynucleotides encoding polypeptides having aspartokinase activity, homoserine dehydrogenase activity, homoserine kinase activity, threonine synthase activity, and threonine deaminase activity, or (d) polynucleotides encoding polypeptides having (R)-citramalate synthase activity, isopropylmalate isomerase activity, and beta-isopropyl malate dehydrogenase activity, or (c) and (d); and (e) polynucleotides encoding polypeptides having acetohydroxyacid synthase activity, acetohydroxyacid isomeroreductase activity, and dihydroxy acid dehydratase activity; wherein at least one polynucleotide according to (a), (b), (c), (d), or (e) encodes a polypeptide that is exogenous to the recombinant microbial cell or expression of said polynucleotide is modulated in the recombinant microbial cell; and comprising one or more polynucleotides each which encodes a polypeptide having fatty acid derivative enzyme activity, wherein the recombinant microbial cell produces an anteiso-branched fatty acid derivative when cultured in the presence of a carbon source under conditions effective to express the polynucleotides. In some embodiments, expression of the at least one polynucleotide according to (a), (b), (c), (d), or (e) is modulated by overexpression of the polynucleotide, such as by operatively linking the polynucleotide to an exogenous promoter.

In some embodiments, the recombinant microbial cell according to the second aspect produces a fatty acid derivative composition when the cell is cultured in a culture medium containing a carbon source under conditions effective to express the polynucleotides, the fatty acid derivative composition comprising straight chain fatty acid derivatives and branched chain fatty acid derivatives, the branched chain fatty acid derivatives comprising anteiso-branched fatty acid derivatives and iso-branched fatty acid derivatives.

In some embodiments, at least 5%, at least 10%, at least 20%, at least 30%, at least 40%, at least 50%, at least 60%, at least 70%, at least 80% or at least 90% of the fatty acid derivatives in the composition produced by the recombinant microbial cell according to the second aspect are branched chain fatty acid derivatives. In some embodiments, at least 10%, at least 20%, at least 30%, at least 40%, at least 50%, at least 60%, at least 70%, at least 80% or at least 90% of the branched chain fatty acid derivatives in the composition produced by the microbial cell of the second aspect are anteiso-branched fatty acid derivatives. In some embodiments, the recombinant microbial cell of the second aspect produces at least 10 mg/L, at least 25 mg/L, at least 100 mg/L, at least 200 mg/L, at least 500 mg/L, at least 1000 mg/L, or at least 2000 mg/L branched chain fatty acid derivatives when cultured in a culture medium containing a carbon source under conditions effective to express the polynucleotides.

In some embodiments of the first aspect or the second aspect, the fatty acid derivative enzyme activity comprises thioesterase activity, and the branched chain fatty acid derivative produced by the recombinant microbial cell is a branched chain fatty acid. In some embodiments, the recombinant microbial cell produces a fatty acid composition comprising straight chain fatty acids and branched chain fatty acids, the branched chain fatty acids comprising anteiso-branched fatty acids and iso-branched fatty acids . In some embodiments , at least 5%, at least 10%, at least 20%, at least 30%, at least 40%, at least 50%, at least 60%, at least 70%, at least 80% or at least 90% of the fatty acids in the composition are branched fatty acids. In some embodiments, at least 10%, at least 20%, at least 30%, at least 40%, at least 50%, at least 60%, at least 70%, at least 80% or at least 90% of the branched chain fatty acids in the composition produced by the microbial cell are anteiso-branched fatty acids. In some embodiments, the recombinant microbial cell produces at least 10 mg/L, at least 25 mg/L, at least 100 mg/L, at least 200 mg/L, at least 500 mg/L, at least 1000 mg/L, or at least 2000 mg/L branched chain fatty acids or anteiso-branched chain fatty acids when cultured in a culture medium containing a carbon source under conditions effective to express the polynucleotides.

In some embodiments of the first aspect or the second aspect, the fatty acid derivative enzyme activity comprises ester synthase activity, and the branched chain fatty acid derivative produced by the recombinant microbial cell is a branched fatty ester. In some embodiments, the recombinant microbial cell produces a fatty ester composition comprising straight chain fatty esters and branched chain fatty esters, the branched chain esters comprising anteiso-branched fatty esters and iso-branched fatty esters. In some embodiments, at least 5%, at least 10%, at least 20%, at least 30%, at least 40%, at least 50%, at least 60%, at least 70%, at least 80% or at least 90% of the fatty esters in the composition are branched fatty esters . In some embodiments, at least 10%, at least 20%, at least 30%, at least 40%, at least 50%, at least 60%, at least 70%, at least 80% or at least 90% of the branched fatty esters in the composition produced by the microbial cell are anteiso-branched fatty esters or iso-branched fatty esters . In some embodiments, the recombinant microbial cell produces at least 10 mg/L, at least 25 mg/L, at least 100 mg/L, at least 200 mg/L, at least 500 mg/L, at least 1000 mg/L, or at least 2000 mg/L branched chain fatty esters or anteiso-branched fatty esters or iso-branched fatty esters when cultured in a culture medium containing a carbon source under conditions effective to express the polynucleotides.

In some embodiments of the first aspect or the second aspect, the fatty acid derivative enzyme activity comprises fatty aldehyde biosynthesis activity, and the branched chain fatty acid derivative produced by the recombinant microbial cell is a branched fatty aldehyde. In some embodiments, the recombinant microbial cell produces a fatty aldehyde composition comprising straight chain fatty aldehydes and branched chain fatty aldehydes, the branched fatty aldehydes comprising anteiso-branched fatty aldehydes and iso-branched fatty aldehydes. In some embodiments , at least 5%, at least 10%, at least 20%, at least 30%, at least 40%, at least 50%, at least 60%, at least 70%, at least 80% or at least 90% of the fatty aldehydes in the composition are branched fatty aldehydes. In some embodiments, at least 10%, at least 20%, at least 30%, at least 40%, at least 50%, at least 60%, at least 70%, at least 80% or at least 90% of the branched chain fatty aldehydes in the composition produced by the microbial cell are anteiso-branched fatty aldehydes or iso-branched fatty aldehydes. In some embodiments, the recombinant microbial cell produces at least 10 mg/L, at least 25 mg/L, at least 100 mg/L, at least 200 mg/L, at least 500 mg/L, at least 1000 mg/L, or at least 2000 mg/L branched chain fatty aldehydes or anteiso-branched fatty aldehydes or iso-branched fatty aldehydes when cultured in a culture medium containing a carbon source under conditions effective to express the polynucleotides.

In some embodiments of the first aspect or the second aspect, the fatty acid derivative enzyme activity comprises fatty alcohol biosynthesis activity, and the branched chain fatty acid derivative produced by the recombinant microbial cell is a branched fatty alcohol. In some embodiments, the recombinant microbial cell produces a fatty alcohol composition comprising straight chain fatty alcohols and branched chain fatty alcohols, the branched fatty alcohols comprising anteiso-branched fatty alcohols and iso-branched fatty alcohols. In some embodiments, at least 5%, at least 10%, at least 20%, at least 30%, at least 40%, at least 50%, at least 60%, at least 70%, at least 80% or at least 90% of the fatty alcohols in the composition are branched fatty alcohols . In some embodiments, at least 10%, at least 20%, at least 30%, at least 40%, at least 50%, at least 60%, at least 70%, at least 80% or at least 90% of the branched chain fatty alcohols in the composition produced by the microbial cell are anteiso-branched fatty alcohols or iso-branched fatty alcohols . In some embodiments, the recombinant microbial cell produces at least 10 mg/L, at least 25 mg/L, at least 100 mg/L, at least 200 mg/L, at least 500 mg/L, at least 1000 mg/L, or at least 2000 mg/L branched chain fatty alcohols or anteiso-branched fatty alcohols or iso-branched fatty alcohols when cultured in a culture medium containing a carbon source under conditions effective to express the polynucleotides.

In some embodiments of the first aspect or the second aspect, the fatty acid derivative enzyme activity comprises hydrocarbon biosynthesis activity, and the branched chain fatty acid derivative produced by the recombinant microbial cell is a branched hydrocarbon, such as a branched alkane, a branched terminal olefin or a branched internal olefin. In some embodiments, the recombinant microbial cell produces a hydrocarbon composition comprising straight chain hydrocarbons and branched chain hydrocarbons , the branched hydrocarbons comprising anteiso-branched hydrocarbons and iso-branched hydrocarbons . In some embodiments, at least 5%, at least 10%, at least 20%, at least 30%, at least 40%, at least 50%, at least 60%, at least 70%, at least 80% or at least 90% of the hydrocarbons in the composition are branched hydrocarbons . In some embodiments, at least 10%, at least 20%, at least 30%, at least 40%, at least 50%, at least 60%, at least 70%, at least 80% or at least 90% of the branched chain hydrocarbons in the composition produced by the microbial cell are anteiso-branched hydrocarbons or iso-branched hydrocarbons. In some embodiments, the recombinant microbial cell produces at least 10 mg/L, at least 25 mg/L, at least 100 mg/L, at least 200 mg/L, at least 500 mg/L, at least 1000 mg/L, or at least 2000 mg/L branched hydrocarbons or anteiso-branched hydrocarbons or iso-branched hydrocarbons when cultured in a culture medium containing a carbon source under conditions effective to express the polynucleotides.

In various embodiments, the carbon source comprises a monosaccharide, a disaccharide, or an oligosaccharide. In more preferred embodiments, the carbon source comprises a monosaccharide, preferably a hexose or a pentose , preferably a hexose such as glucose. In some embodiments, the carbon source is obtained from biomass, such as a cellulosic hydrolysate. In other embodiments, the carbon source comprises a branched short-chain carboxylic acid such as isobutyrate, isovalerate, or 2-methyl-butyrate.

In various embodiments, the host (e.g., parental) microbial cell is a filamentous fungi, an algae, a yeast, or a prokaryote such as a bacteria. In more preferred embodiments, the host cell is a bacterial cell. In particular embodiments the host cell is an *E. coli* cell or a *Bacillus* cell, preferably an *E. coli* cell.

In one embodiment, the recombinant microbial cell according to the first aspect or the second aspect comprises a polynucleotide encoding a polypeptide having branched-chain alpha-keto acid dehydrogenase activity which is categorized as EC 1.2.4.4. In some embodiments, the polypeptide having branched-chain alpha-keto acid dehydrogenase activity has an alpha subunit and a beta subunit, encoded by a *bkdA* and *bkdB* gene, a *bkdAA* and *bkdAB* gene, or a *Pput_1453* and *Pput_1452* gene. In one embodiment, the polypeptide having branched-chain alpha-keto acid dehydrogenase activity is endogenous to the parental microbial cell, or is exogenous to the parental microbial cell. In another embodiment, expression of the polynucleotide encoding the polypeptide having branched-chain alpha-keto acid dehydrogenase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having branched-chain alpha-keto acid dehydrogenase activity comprises an alpha subunit and a beta subunit. In some embodiments, the alpha subunit comprises a sequence selected from SEQ ID NOs:1, 3, 5, 7, 9, 11, and 13, or a fragment or a variant thereof, or comprises one or more motif selected from SEQ ID NOs:15-21, wherein the alpha subunit combined with a beta subunit has branched-chain alpha-keto acid dehydrogenase activity, and which, when combined with a polypeptide having lipoamide acyltransferase activity and a polypeptide having dihydrolipoamide dehydrogenase activity, catalyzes the conversion of a branched alpha-keto acid to a branched acyl-CoA *in vitro* or *in vivo,* preferably *in vivo.* In some embodiments, the beta subunit comprises a sequence selected from SEQ ID NOs:22, 24, 26, 28, 30, 32, and 34, or a fragment or a variant thereof, or comprises one or more motif selected from SEQ ID NOs:36-42, wherein the beta subunit combined with an alpha subunit has branched-chain alpha-keto acid dehydrogenase activity, and which, when combined with a polypeptide having lipoamide acyltransferase activity and a polypeptide having dihydrolipoamide dehydrogenase activity, catalyzes the conversion of a branched alpha-keto acid to a branched acyl-CoA *in vitro* or *in vivo,* preferably *in vivo.*

In one embodiment, the recombinant microbial cell according to the first aspect or the second aspect comprises a polynucleotide encoding a polypeptide having lipoamide acyltransferase activity which is categorized as EC 2.3.1.168. In some embodiments, the polypeptide having lipoamide acyltransferase activity is encoded by a *bkdB,* a *bkdC,* or a *Pput_1451* gene. In one embodiment, the polypeptide having lipoamide acyltransferase activity is endogenous to the parental microbial cell, or is exogenous to the parental microbial cell. In another embodiment, expression of the polynucleotide encoding the polypeptide having lipoamide acyltransferase activity is modulated in the recombinant microbial cell. In some instances, expression of the polynucleotide is modulated by operatively linking the polynucleotide to an exogenous promoter, such that the polynucleotide is overexpressed in the recombinant microbial cell. In other embodiments, the polypeptide having lipoamide acyltransferase activity comprises a sequence selected from SEQ ID NOs:43, 45, 47, 49, 51, 53, and 55, or a variant or a fragment thereof having lipoamide acyltransferase activity, or comprises one or more sequence motif selected from SEQ ID NOs:57-62, and which, when combined with a polypeptide having dihydrolipoamide dehydrogenase activity and a polypeptide having branched-chain alpha-keto acid dehydrogenase activity, catalyzes the conversion of a branched alpha-keto acid to a branched acyl-CoA *in vitro* or *in vivo,* preferably *in vivo.*

In one embodiment, the recombinant microbial cell according to the first aspect or the second aspect comprises a polynucleotide encoding a polypeptide having lipoamide dehydrogenase activity which is categorized as EC 1.8.1.4. In some embodiments, the polypeptide having dihydrolipoamide dehydrogenase activity is encoded by a *IpdV* gene, a *Pput_1450* gene or a *IpdA* gene. In one embodiment, the polypeptide having dihydrolipoamide dehydrogenase activity is endogenous to the parental microbial cell, or is exogenous to the parental microbial cell. In another embodiment, expression of the polynucleotide encoding the polypeptide having dihydrolipoamide dehydrogenase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having dihydrolipoamide dehydrogenase activity comprises a sequence selected from SEQ ID NOs:63, 65, 67, 69, 71, 73, 75, and 77, or a variant or a fragment thereof having dihydrolipoamide dehydrogenase activity, or comprises one or more sequence motif selected from SEQ ID NOs:79-83 and which, when combined with a polypeptide having lipoamide acyltransferase activity and a polypeptide having branched-chain alpha-keto acid dehydrogenase activity, catalyzes the conversion of a branched alpha-keto acid to a branched acyl-CoA *in vitro* or *in vivo,* preferably *in vivo.*

In one embodiment, the recombinant microbial cell according to the first aspect or the second aspect comprises a polynucleotide encoding a polypeptide having beta-ketoacyl-ACP synthase activity and utilizes a branched acyl-CoA molecule as a substrate, preferably a beta-ketoacyl-ACP synthase III activity categorized as EC 2.3.1.180. In one embodiment, the polypeptide having beta-ketoacyl-ACP synthase activity is encoded by a *fabH* gene. In one embodiment, the polypeptide having beta-ketoacyl-ACP synthase activity is endogenous to the parental microbial cell, or is exogenous to the parental microbial cell. In another embodiment, expression of the polynucleotide encoding the polypeptide having beta-ketoacyl-ACP synthase activity is modulated in the recombinant microbial cell. In some instances, expression of the polynucleotide is modulated by operatively linking the polynucleotide to a strong promoter, such that the polynucleotide is overexpressed in the recombinant microbial cell. In another embodiment, the polypeptide having beta-ketoacyl-ACP synthase activity comprises a sequence selected from SEQ ID NOs:84, 86, 88, 90, 92, 94, 96, 98, 100, 102, 104, 106, and 108, or a variant or a fragment thereof having beta-ketoacyl-ACP synthase activity, or comprises one or more sequence motif selected from SEQ ID NO s:110-115, and which catalyzes the condensation of a branched acyl-CoA with malonyl-ACP to form a branched acyl-ACP *in vitro* or *in vivo,* preferably *in vivo.*

In one embodiment, the recombinant microbial cell according to the first aspect or the second aspect comprises an endogenous polynucleotide sequence (such as, an endogenous *fabH* gene) encoding a polypeptide having beta-ketoacyl-ACP synthase activity that does not utilize a branched acyl-CoA molecule as a substrate, and expression of the endogenous polynucleotide sequence in the recombinant microbial cell is attenuated. In some embodiments, expression of the endogenous polynucleotide is attenuated by deletion of all or part of the sequence of the endogenous polynucleotide in the recombinant microbial cell.

In another embodiment, the recombinant microbial cell according to the first aspect or the second aspect comprises an endogenous polynucleotide sequence (such as, an endogenous *fadE gene*) encoding a polypeptide having acyl-CoA dehydrogenase activity, and expression of the endogenous polynucleotide in the recombinant microbial cell is attenuated. In some embodiments, expression of the endogenous polynucleotide is attenuated by deletion of all or part of the sequence of the endogenous polynucleotide in the recombinant microbial cell.

In one embodiment, the recombinant microbial cell according to the second aspect comprises a polynucleotide encoding a polypeptide having aspartokinase activity which is categorized as EC 2.7.2.4. In some embodiments, the polypeptide having aspartokinase activity is encoded by a *thrA,* a *dapG* or a *hom3* gene. In one embodiment, the polypeptide having aspartokinase activity is endogenous to the parental microbial cell, or is exogenous to the parental microbial cell. In another embodiment, expression of the polynucleotide encoding the polypeptide having aspartokinase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having aspartokinase activity comprises a sequence selected from SEQ ID NOs:116,118,120,122,124, or a variant or a fragment thereof having aspartokinase activity and which catalyzes the conversion of aspartate to aspartyl phosphate *in vitro* or *in vivo,* preferably *in vivo.*

In one embodiment, the recombinant microbial cell according to the second aspect comprises a polynucleotide encoding a polypeptide having homoserine dehydrogenase activity which is categorized as EC 1.1.1.3. In some embodiments, the polypeptide having homoserine dehydrogenase activity is encoded by a *thrA,* a *hom* or a *hom6* gene. In one embodiment, the polypeptide having homoserine dehydrogenase activity is endogenous to the parental microbial cell, or is exogenous to the parental microbial cell. In another embodiment, expression of the polynucleotide encoding the polypeptide having homoserine dehydrogenase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having homoserine dehydrogenase activity comprises a sequence selected from SEQ ID NOs:116, 118, 126, 128, and 130, or a variant or a fragment thereof having homoserine dehydrogenase activity and which catalyzes the conversion of aspartate semialdehyde to homoserine *in vitro* or *in vivo,* preferably *in vivo.*

In a particular embodiment, the recombinant microbial cell according to the second aspect comprises a polynucleotide encoding a polypeptide having aspartokinase and homoserine dehydrogenase activity. In one embodiment, the polypeptide having aspartokinase and homoserine dehydrogenase activity is endogenous to the parental microbial cell, or is exogenous to the parental microbial cell. In another embodiment, expression of the polynucleotide encoding the polypeptide having aspartokinase and homoserine dehydrogenase activity is modulated in the recombinant microbial cell. In some instances, expression of the polynucleotide is modulated by operatively linking the polynucleotide to an exogenous promoter, such that the polynucleotide is overexpressed in the recombinant microbial cell. In one embodiment the polypeptide having aspartokinase and homoserine dehydrogenase activity comprises the sequence SEQ ID NO:116, or a variant or a fragment thereof, such as SEQ ID NO:118, which catalyzes the conversion of aspartate to aspartyl phosphate and the conversion of aspartate semialdehyde to homoserine *in vitro* or *in vivo,* preferably *in vivo.*

In one embodiment, the recombinant microbial cell according to the second aspect comprises a polynucleotide encoding a polypeptide having homoserine kinase activity which is categorized as EC 2.7.1.39. In some embodiments, the polypeptide having homoserine kinase activity is encoded by a *thrB* gene or a *thr1* gene. In one embodiment, the polypeptide having homoserine kinase activity is endogenous to the parental microbial cell, or is exogenous to the parental microbial cell. In another embodiment, expression of the polynucleotide encoding the polypeptide having homoserine kinase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having homoserine kinase activity comprises a sequence selected from SEQ ID NOs:132,134,136,138, or a variant or a fragment thereof having homoserine kinase activity and which catalyzes the conversion of homoserine to O-phospho-L-homoserine *in vitro* or *in vivo,* preferably *in vivo.*

In one embodiment, the recombinant microbial cell according to the second aspect comprises a polynucleotide encoding a polypeptide having threonine synthase activity which is categorized as EC 4.2.3.1. In one embodiment, the polypeptide having threonine synthase activity is encoded by a *thrC* gene. In one embodiment, the polypeptide having threonine synthase activity is endogenous to the parental microbial cell, or is exogenous to the parental microbial cell. In another embodiment, expression of the polynucleotide encoding the polypeptide having threonine synthase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having threonine synthase activity comprises a sequence selected from SEQ ID NOs:140,143,144, or a variant or a fragment thereof having threonine synthase activity and which catalyzes the conversion of O-phospho-L-homoserine to threonine *in vitro* or *in vivo,* preferably *in vivo.*

In one embodiment, the recombinant microbial cell according to the second aspect comprises a polynucleotide encoding a polypeptide having threonine deaminase activity which is categorized as EC 4.3.1.19. In some embodiments, the polypeptide having threonine deaminase activity is encoded by a *tdcB* gene or an *ilvA* gene. In one embodiment, the polypeptide having threonine deaminase activity is endogenous to the parental microbial cell, or is exogenous to the parental microbial cell. In another embodiment, expression of the polynucleotide encoding the polypeptide having threonine deaminase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having threonine deaminase activity comprises a sequence selected from SEQ ID NOs:146,148,150,152, and 154, or a variant or a fragment thereof having threonine deaminase activity and which catalyzes the conversion of threonine to 2-ketobutyrate *in vitro* or *in vivo,* preferably *in vivo.*

In one embodiment, the recombinant microbial cell according to the second aspect comprises a polynucleotide encoding a polypeptide having (R)-citramalate synthase activity which is categorized as EC 2.3.1.182. In one embodiment, the polypeptide having (R)-citramalate synthase activity is encoded by a *cimA* gene. In one embodiment, the polypeptide having (R)-citramalate synthase activity is endogenous to the parental microbial cell, or is exogenous to the parental microbial cell. In another embodiment, expression of the polynucleotide encoding the polypeptide having (R)-citramalate synthase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having (R)-citramalate synthase activity comprises a sequence selected from SEQ ID NOs:156,158,160, and 162, or a variant or a fragment thereof having (R)-citramalate synthase activity and which catalyzes the reaction of acetyl-CoA and pyruvate to (R)-citramalate *in vitro* or *in vivo,* preferably *in vivo.*

In one embodiment, the recombinant microbial cell according to the second aspect comprises a polynucleotide encoding a polypeptide having isopropylmalate isomerase activity which is categorized as EC 4.2.1.33. In one embodiment, the polypeptide having isopropylmalate isomerase activity comprises a large subunit and a small subunit encoded by *leuCD* genes. In one embodiment, the polypeptide having isopropylmalate isomerase activity is endogenous to the parental microbial cell, or is exogenous to the parental microbial cell. In another embodiment, expression of the polynucleotide encoding the polypeptide having isopropylmalate isomerase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having isopropylmalate isomerase activity comprises a large subunit and a small subunit. In other embodiments, the polypeptide having isopropylmalate isomerase activity comprises a large subunit sequence selected from SEQ ID NOs:164 and 168 and a small subunit sequence selected from SEQ ID NOs:166 and 170, or variants or fragments thereof having isopropylmalate isomerase activity and which catalyzes the conversion of (R)-citramalate to citraconate and citraconate to beta-methyl-D-malate *in vitro* or *in vivo,* preferably *in vivo.*

In one embodiment, the recombinant microbial cell according to the second aspect comprises a polynucleotide encoding a polypeptide having beta-isopropylmalate dehydrogenase activity which is categorized as EC 1.1.1.85. In some embodiments, the polypeptide having beta-isopropyl malate dehydrogenase activity is encoded by a *leuB* gene or a *leu2* gene. In one embodiment, the polypeptide having beta-isopropylmalate dehydrogenase activity is endogenous to the parental microbial cell, or is exogenous to the parental microbial cell. In another embodiment, expression of the polynucleotide encoding the polypeptide having beta-isopropylmalate dehydrogenase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having beta-isopropyl malate dehydrogenase activity comprises a sequence selected from SEQ ID NOs:172,174,176, or a variant or a fragment thereof having beta-isopropylmalate dehydrogenase activity and which catalyzes conversion of beta-methyl-D-malate to 2-ketobutyrate *in vitro* or *in vivo,* preferably *in vivo.*

In one embodiment, the recombinant microbial cell according to the second aspect comprises a polynucleotide encoding a polypeptide having acetohydroxyacid synthase activity which is categorized as EC 2.2.1.6. In some embodiments, the polypeptide having acetohydroxyacid synthase activity comprises a large subunit and a small subunit encoded by *ilvBN* genes, *ilvGM* genes or *ilvIH* genes. In one embodiment, the polypeptide having acetohydroxyacid synthase activity is endogenous to the parental microbial cell, or is exogenous to the parental microbial cell. In another embodiment, expression of the polynucleotide encoding the polypeptide having acetohydroxyacid synthase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having acetohydroxyacid synthase activity comprises a sequence selected from SEQ ID NOs:178, 180, 182, 184, 186, 188, 190, and 192, or a variant or a fragment thereof having acetohydroxyacid synthase activity and which catalyzes the conversion of 2-ketobutyrate to 2-keto-3-methylvalerate *in vitro* or *in vivo,* preferably *in vivo.*

In one embodiment, the recombinant microbial cell according to the second aspect comprises a polynucleotide encoding a polypeptide having acetohydroxyacid isomeroreductase activity which is categorized as EC 1.1.1.86. In some embodiments, the polypeptide having acetohydroxyacid isomeroreductase activity is encoded by an *ilvC* gene or an *ilv5* gene. In one embodiment, the polypeptide having acetohydroxyacid isomeroreductase activity is endogenous to the parental microbial cell, or is exogenous to the parental microbial cell. In another embodiment, expression of the polynucleotide encoding the polypeptide having acetohydroxyacid isomeroreductase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having acetohydroxyacid isomeroreductase activity comprises a sequence selected from SEQ ID NOs:194 and 196, or a variant or a fragment thereof having acetohydroxyacid isomeroreductase activity and which catalyzes the conversion of 2-aceto-2-hydroxybutyrate to 2,3-dihydroxy-3-methylvalerate *in vitro* or *in vivo,* preferably *in vivo.*

In one embodiment, the recombinant microbial cell according to the second aspect comprises a polynucleotide encoding a polypeptide having dihydroxy acid dehydratase activity which is categorized as EC 4.2.1.9. In some embodiments, the polypeptide having acetohydroxyacid isomeroreductase activity is encoded by an *ilvD* gene or an *ilv3* gene. In one embodiment, the polypeptide having dihydroxy acid dehydratase activity is endogenous to the parental microbial cell, or is exogenous to the parental microbial cell. In another embodiment, expression of the polynucleotide encoding the polypeptide having dihydroxy acid dehydratase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having dihydroxy acid dehydratase activity comprises a sequence selected from SEQ ID NO:198 and 200, or a variant or a fragment thereof having dihydroxy acid dehydratase activity and which catalyzes the conversion of 2,3-dihydroxy-3-methylvalerate to 2-keto-3-methylvalerate *in vitro* or *in vivo,* preferably *in vivo.*

In other embodiments, a recombinant microbial cell according to the first aspect or the second aspect further comprises one or more polynucleotides encoding one or more polypeptides each having a fatty acid derivative enzyme activity, wherein the recombinant microbial cell produces a branched chain fatty acid derivative when cultured in the presence of a carbon source.

In various embodiments, the fatty acid derivative enzyme activity comprises a thioesterase activity, an ester synthase activity, a fatty aldehyde biosynthesis activity, a fatty alcohol biosynthesis activity, a ketone biosynthesis activity, and/or a hydrocarbon biosynthesis activity. In some embodiments, the recombinant microbial cell comprises polynucleotides encoding two or more polypeptides, each polypeptide having a fatty acid derivative enzyme activity. In more particular embodiments, the recombinant microbial cell expresses or overexpresses one or more polypeptides having fatty acid derivative enzyme activity selected from: (1) a polypeptide having thioesterase activity; (2) a polypeptide having decarboxylase activity; (3)a polypeptide having carboxylic acid reductase activity; (4) a polypeptide having alcohol dehydrogenase activity (EC 1.1.1.1); (5) a polypeptide having aldehyde decarbonylase activity (EC 4.1.99.5); (6) a polypeptide having acyl-CoA reductase activity (EC 1.2.1.50); (7) a polypeptide having acyl-ACP reductase activity; (8) a polypeptide having ester synthase activity (EC 3.1.1.67); (9) a polypeptide having OleA activity; or (10) a polypeptide having OleCD or OleBCD activity; wherein the recombinant microbial cell produces a composition comprising branched fatty acids, branched fatty esters, branched wax esters, branched fatty aldehydes, branched fatty alcohols, branched alkanes, branched alkenes, branched internal olefins, branched terminal olefins, or branched ketones.

In one embodiment, the fatty acid derivative enzyme activity is a thioesterase activity, and the branched chain fatty acid derivative is a branched chain fatty acid, wherein a culture comprising the recombinant microbial cell produces a branched chain fatty acid composition when cultured in the presence of a carbon source. In some embodiments, the polypeptide has a thioesterase activity which is categorized as EC 3.1.1.5, EC 3.1.2.-, or EC 3.1.2.14. In some embodiments, the polypeptide having a thioesterase activity is encoded by a *tesA,* a *tesB,* a *fatA,* or a *fatB* gene. In one embodiment, the polypeptide having thioesterase activity is endogenous to the parental microbial cell, or is exogenous to the parental microbial cell. In another embodiment, expression of the polynucleotide encoding the polypeptide having thioesterase activity is modulated in the recombinant microbial cell. In some instances, expression of the polynucleotide is modulated by operatively linking the polynucleotide to an exogenous promoter, such that the polynucleotide is overexpressed in the recombinant microbial cell. In another embodiment, the polypeptide having thioesterase activity comprises a sequence selected from SEQ ID NO: 202, 204, 206, 208, 210, 212, 214, 216, 218, 220, 222, and 224, or a variant or a fragment thereof having thioesterase activity and which catalyzes the hydrolysis of a branched acyl-ACP to a branched fatty acid, or catalyzes the alcoholysis of a branched acyl-ACP to a branched fatty ester, *in vitro* or *in vivo,* preferably *in vivo.* In some embodiments, the recombinant microbial cell according to the first aspect or the second aspect, further comprising a polynucleotide encoding a polypeptide having thioesterase activity, when cultured in the presence of a carbon source, produces at least 10 mg/L, at least 25 mg/L, at least 100 mg/L, at least 200 mg/L, at least 500 mg/L, at least 1000 mg/L, or at least 2000 mg/L branched chain fatty acids or anteiso-branched chain fatty acids when cultured in a culture medium containing a carbon source under conditions effective to express the polynucleotides. In some embodiments, the recombinant microbial cell according to the first aspect or the second aspect, further comprising a polynucleotide encoding a polypeptide having thioesterase activity, produces a fatty acid composition comprising straight chain fatty acids and branched chain fatty acids, the branched chain fatty acids comprising anteiso-branched fatty acids and iso-branched fatty acids . In some embodiments, at least 5%, at least 10%, at least 20%, at least 30%, at least 40%, at least 50%, at least 60%, at least 70%, at least 80% or at least 90% of the fatty acids in the composition are branched fatty acids. In some embodiments, at least 10%, at least 20%, at least 30%, at least 40%, at least 50%, at least 60%, at least 70%, at least 80% or at least 90% of the branched chain fatty acids in the composition produced by the microbial cell are anteiso-branched fatty acids.

In a third aspect, the invention includes a method of making a composition comprising a branched fatty acid derivative, the method comprising: obtaining a recombinant microbial cell comprising : (a) polynucleotides encoding a branched chain alpha-keto acid dehydrogenase (BKD) complex, comprising polypeptides having branched-chain alpha-keto acid dehydrogenase activity, lipoamide acyltransferase activity, and dihydrolipoamide dehydrogenase activity, and (b) a polynucleotide encoding a polypeptide having beta-ketoacyl-ACP synthase activity that utilizes a branched acyl-CoA molecule as a substrate, wherein at least one polynucleotide according to (a) or (b) encodes a polypeptide that is exogenous to the parental microbial cell or expression of said polynucleotide is modulated in the recombinant microbial cell; the recombinant microbial cell further comprising one or more polynucleotides each which encodes a polypeptide having fatty acid derivative enzyme activity, wherein the recombinant microbial cell produces a branched chain fatty acid derivative when cultured in the presence of a carbon source under conditions effective to express the polynucleotides; culturing the recombinant microbial cell in a culture medium containing a carbon source under conditions effective to express the polynucleotides and produce a fatty acid derivative composition comprising straight-chain fatty acid derivatives and branched fatty acid derivatives, and optionally recovering the composition from the culture medium.

In some embodiments, the fatty acid derivative composition produced by the recombinant cell comprises branched fatty acid derivatives, wherein at least 10%, at least 20%, at least 30%, at least 40%, at least 50%, at least 60%, at least 70%, at least 80% or at least 90% by weight of the fatty acid derivatives in the composition are branched fatty acid derivatives. In some embodiments, the fatty acid derivative composition comprises branched fatty acid derivatives in an amount (e.g., a titer) of at least 10 mg/L, at least 25 mg/L, at least 100 mg/L, at least 200 mg/L, at least 500 mg/L, at least 1000 mg/L, or at least 2000 mg/L.

In various embodiments, the fatty acid derivative enzyme activity comprises a thioesterase activity, an ester synthase activity, a fatty aldehyde biosynthesis activity, a fatty alcohol biosynthesis activity, a ketone biosynthesis activity, and/or a hydrocarbon biosynthesis activity. In some embodiments, the recombinant microbial cell comprises polynucleotides encoding two or more polypeptides, each polypeptide having a fatty acid derivative enzyme activity. In more particular embodiments, the recombinant microbial cell expresses or overexpresses one or more polypeptides having fatty acid derivative enzyme activity selected from: (1) a polypeptide having thioesterase activity; (2) a polypeptide having decarboxylase activity; (3) a polypeptide having carboxylic acid reductase activity; (4) a polypeptide having alcohol dehydrogenase activity (EC 1.1.1.1); (5) a polypeptide having aldehyde decarbonylase activity (EC 4.1.99.5); (6) a polypeptide having acyl-CoA reductase activity (EC 1.2.1.50); (7) a polypeptide having acyl-ACP reductase activity; (8) a polypeptide having ester synthase activity (EC 3.1.1.67); (9) a polypeptide having OleA activity; or (10) a polypeptide having OleCD or OleBCD activity; wherein the recombinant microbial cell produces a composition comprising branched fatty acids, branched fatty esters, branched wax esters, branched fatty aldehydes, branched fatty alcohols, branched alkanes, branched alkenes, branched internal olefins, branched terminal olefins, or branched ketones.

In some embodiments, the fatty acid derivative composition produced by the recombinant cell comprises branched fatty acid derivatives, wherein at least 10%, at least 20%, at least 30%, at least 40%, at least 50%, at least 60%, at least 70%, at least 80% or at least 90% by weight of the fatty acid derivatives in the composition are branched fatty acid derivatives. In some embodiments, the fatty acid derivative composition comprises branched fatty acid derivatives in an amount (e.g., a titer) of at least 10 mg/L, at least 25 mg/L, at least 100 mg/L, at least 200 mg/L, at least 500 mg/L, at least 1000 mg/L, or at least 2000 mg/L. In some embodiments, the fatty acid derivative composition produced by the recombinant microbial cell culture comprises iso-branched fatty acid derivatives, wherein at least 10%, at least 20%, at least 30%, at least 40%, at least 50%, at least 60%, at least 70%, or at least 80% by weight of the branched fatty acid derivatives in the composition are iso-branched fatty acid derivatives.

In a fourth aspect, the invention includes a method of making a composition comprising an anteiso-branched fatty acid derivative, the method comprising: obtaining a recombinant microbial cell (such as, a culture comprising a recombinant microbial cell) comprising : (a) polynucleotides encoding a branched chain alpha-keto acid dehydrogenase (BKD) complex, comprising polypeptides having branched-chain alpha-keto acid dehydrogenase activity, lipoamide acyltransferase activity, and dihydrolipoamide dehydrogenase activity, and (b) a polynucleotide encoding a polypeptide having beta-ketoacyl-ACP synthase activity that utilizes a branched acyl-CoA molecule as a substrate, and comprising (c) polynucleotides encoding polypeptides having aspartokinase activity, homoserine dehydrogenase activity, homoserine kinase activity, threonine synthase activity, and threonine deaminase activity, or (d) polynucleotides encoding polypeptides having (R)-citramalate synthase activity, isopropylmalate isomerase activity, and beta-isopropyl malate dehydrogenase activity, or (c) and (d); and (e) polypeptides having acetohydroxyacid synthase activity, acetohydroxyacid isomeroreductase activity, and dihydroxy acid dehydratase activity; wherein at least one polynucleotide according to (a), (b), (c), (d), or (e) encodes a polypeptide that is exogenous to the recombinant microbial cell or expression of said polynucleotide is modulated in the recombinant microbial cell; the recombinant microbial cell further comprising one or more polynucleotides each which encodes a polypeptide having fatty acid derivative enzyme activity, wherein the recombinant microbial cell produces an anteiso-branched chain fatty acid derivative when cultured in the presence of a carbon source under conditions effective to express the polynucleotides; culturing the recombinant microbial cell in a culture medium containing a carbon source under conditions effective to express the polynucleotides and produce a fatty acid derivative composition comprising straight-chain fatty acid derivatives and branched fatty acid derivatives, the branched fatty acid derivatives comprising anteiso-branched fatty acid derivatives and iso-branched fatty acid derivatives; and optionally recovering the composition from the culture medium.

In various embodiments, the fatty acid derivative enzyme activity comprises a thioesterase activity, an ester synthase activity, a fatty aldehyde biosynthesis activity, a fatty alcohol biosynthesis activity, a ketone biosynthesis activity and/or a hydrocarbon biosynthesis activity, as described hereinabove; wherein the recombinant microbial cell produces a composition comprising anteiso-branched fatty acids, anteiso-branched fatty esters, anteiso-branched wax esters, anteiso-branched fatty aldehydes, anteiso-branched fatty alcohols, anteiso-branched alkanes, anteiso-branched alkenes, anteiso-branched terminal olefins, or anteiso-branched ketones.

In some embodiments, the fatty acid derivative composition produced by the recombinant cell comprises branched fatty acid derivatives, wherein at least 10%, at least 20%, at least 30%, at least 40%, at least 50%, at least 60%, at least 70%, at least 80% or at least 90% by weight of the fatty acid derivatives in the composition are branched fatty acid derivatives. In some embodiments, the fatty acid derivative composition comprises branched fatty acid derivatives in an amount (e.g., a titer) of at least 10 mg/L, at least 25 mg/L, at least 100 mg/L, at least 200 mg/L, at least 500 mg/L, at least 1000 mg/L, or at least 2000 mg/L. In some embodiments, the fatty acid derivative composition produced by the recombinant microbial cell culture comprises anteiso-branched fatty acid derivatives, wherein at least 10%, at least 20%, at least 30%, at least 40%, at least 50%, at least 60%, at least 70%, or at least 80% by weight of the branched fatty acid derivatives in the composition are anteiso-branched fatty acid derivatives. In some embodiments, the fatty acid derivative composition comprises anteiso-branched fatty acid derivatives in an amount (e.g., a titer) of at least 10 mg/L, at least 25 mg/L, at least 100 mg/L, at least 200 mg/L, at least 500 mg/L, at least 1000 mg/L, or at least 2000 mg/L .

These and other objects and features of the invention will become more fully apparent when the following detailed description is read in conjunction with the accompanying figures.

## BRIEF DESCRIPTION OF THE DRAWINGS

**Figure 1** depicts a branched chain fatty acid (BCFA) biosynthetic pathway as described herein.

**Figures 2A and 2B** depict exemplary intermediates and products of the BCFA biosynthetic pathway when supplied with the branched acyl-CoA molecules (A) 2-methyl-butyryl-CoA , which generates the anteiso-branched β-ketoacyl-ACP intermediate 4-methyl-3-oxo-hexanoyl-ACP, leading to production of anteiso-branched fatty acid derivatives, and (B) isobutyryl-CoA, which generates the iso-branched β-ketoacyl-ACP intermediate 4-methyl-3-oxo-pentanoyl-ACP, leading to production of iso-branched fatty acid derivatives. Likewise, the branched acyl-CoA molecule isovaleryl-CoA can produce iso-branched fatty acid derivatives via the iso-branched β-ketoacyl-ACP intermediate 5-methyl-3-oxo-hexanoyl-ACP.

**Figures 3A and 3B** depicts an anteiso-BCFA biosynthetic pathway as described herein.

**Figure 4** shows representative GC-MS traces of (a) fatty acids produced by an *E. coli* strain expressing a leaderless TesA polypeptide and *B. subtilis* FabH1 protein, compared to (b) fatty acids produced by the same *E. coli* strain which does not express the *B. subtilis* FabH1 protein. The peaks corresponding to iso-branched chain C14 fatty acids, straight-chain monounsaturated C14 fatty acids, and straight-chain saturated C14 fatty acids produced by these strains are labeled "iso-C1C14:0", "C1C14:1" and "C1C14:1", respectively, due to the TMAH derivitization procedure used, which converted the fatty acids to fatty acid methyl esters prior to the GC/MS analysis.

**Figure 5** shows a representative GC/MS trace of fatty acids produced by an *E. coli* strain expressing a leaderless TesA polypeptide, *B. subtilis* BKD complex and *B. subtilis* FabH1. The peaks corresponding to iso-branched ("i-"), anteiso-branched ("a-"), monounsaturated (Cn:1) and saturated (Cn:o) fatty acids of chain length (Cn) from C13 to C17 are labeled accordingly.

**Figure 6** shows a representative GC/MS trace of fatty acids produced by an *E. coli* strain expressing a leaderless TesA polypeptide, *P. putida* BKD complex and *B. subtilis* FabH1. The peaks corresponding to iso-branched ("i-"), anteiso-branched ("a-"), monounsaturated (Cn:1) and saturated (Cn:0) fatty acids of chain length (Cn) from C13 to C17 are labeled accordingly.

**Figure 7** shows representative GC/MS traces of fatty alcohols produced by *E. coli* strains expressing (A) *S. elongatus* AAR , plus plasmids expressing the *P. putida* BKD complex and B. *subtilis* FabH1, and (B) the same *E. coli* strain expressing *S. elongatus* AAR but lacking the plasmids expressing BKD and FabH1. Peaks representing branched fatty alcohols are boxed.

## DETAILED DESCRIPTION

The invention is not limited to the specific compositions and methodology described herein, as these may, of course, vary. It is also to be understood that the terminology used herein is for the purpose of describing particular embodiments only, and is not intended to limit the scope of the present invention.

Accession Numbers: Sequence Accession numbers throughout this description were obtained from databases provided by the NCBI (National Center for Biotechnology Information) maintained by the National Institute of Health, U.S.A. (which are identified herein as "NCBI Accession Numbers", or alternatively as "GenBank Accession Numbers"), and from the UniProt Knowledgebase (UniProtKB) and Swiss-Prot databases provided by the Swiss Institute of Bioinformatics (which are identified herein as "UniProtKB Accession Numbers"). Unless otherwise expressly indicated, the sequence identified by an NCBI / GenBank Accession number is version number 1 (that is, the Version Number of the sequence is "AccessionNumber.1"). The NCBI and UniProtKB accession numbers provided herein were those current as of March 31, 2011.

Enzyme Classification (EC) Numbers: EC numbers are established by the Nomenclature Committee of the International Union of Biochemistry and Molecular Biology (IUBMB), description of which is available on the IUBMB Enzyme Nomenclature website on the WOrld Wide Web. EC numbers classify enzymes according to the reaction catalyzed. EC numbers referenced herein are derived from the KEGG Ligand database, maintained by the Kyoto Encyclopedia of Genes and Genomics, sponsored in part by the University of Tokyo. Unless otherwise indicated, EC numbers are as provided in the KEGG database as of March 31, 2011.

Unless defined otherwise, all technical and scientific terms used herein have the same meaning as commonly understood to one of ordinary skill in the art to which this invention belongs. Although any materials and methods similar or equivalent to those described herein can be used in the practice or testing of the invention, the preferred compositions and methods are now described.

Definitions

As used herein, the term "fatty acid" means a carboxylic acid having the formula R(C=O)OH. R represents an aliphatic group, preferably an alkyl group, which can comprise between about 4 and about 22 carbon atoms. Fatty acids can be saturated, monounsaturated, or polyunsaturated. If unsaturated, R can have one or more points of unsaturation. R can be a straight chain or a branched chain. The branched chain may have one or more points of branching. The branched chain can have an iso- or anteiso-conformation.

The term "branched fatty acid" is synonymous with "branched chain fatty acid" and is abbreviated "BCFA" herein. Likewise, the term "branched fatty acid derivative " is synonymous with "branched chain fatty acid derivative" and is abbreviated herein "BCFA derivative". As used herein , the term "branched fatty aldehyde" is synonymous with "branched fatty acid aldehyde", "branched chain fatty aldehyde" and "branched chain fatty acid aldehyde"; the term "branched fatty alcohol" is synonymous with "branched fatty acid alcohol", "branched chain fatty alcohol" and "branched chain fatty acid alcohol"; the term "branched fatty ester" is synonymous with "branched fatty acid ester", "branched chain fatty ester" and "branched chain fatty acid ester"; and so on. As used herein, the term "branched hydrocarbon" is synonymous with "branched chain hydrocarbon", "branched alkane" is synonymous with "branched chain alkane", and so on.

As used herein, an "iso-" branched chain refers to a branched hydrocarbon structure having a methyl on the penultimate carbon atom, whereas an "anteiso-" branched chain refers to a branched hydrocarbon structure having a methyl on the third carbon atom from the end.

The term "branched β-ketoacyl-ACP" as used herein refers to the product of the condensation of a branched acyl-CoA "primer" molecule with malonyl-ACP catalyzed by a beta ketoacyl-ACP synthase III (i.e., FabH) enzyme as represented by part (D) of the BCFA pathway in Figures 1, 2 and 3. This initial branched β-ketoacyl-ACP molecule enters the fatty acid synthase (FAS) cycle, represented by part (E) of Figure 1, where it is subjected to a round of keto reduction, dehydration, and enoyl reduction, forming a branched acyl-ACP molecule which then condenses with another malonyl-ACP molecule followed by another cycle of keto reduction, dehydration, and enoyl reduction, elongating the acyl chain of the branched acyl-ACP by two carbon units per cycle. The "branched acyl-ACP" elongation product is an acyl thioester formed between the carbonyl carbon of a branched alkyl chain and the sulfydryl group of the 4'-phosphopantethionyl moiety of an acyl carrier protein (ACP) and, as used herein, typically has the formula R-C(O)S-ACP, wherein R is a branched alkyl group which may be in the "iso-" or the "anteiso-" configuration. The branched acyl-ACP is an intermediate in the production of branched chain fatty acids and branched chain fatty acid derivatives by the BCFA pathways described herein.

Unless otherwise specified, a "fatty acid derivative" is intended to include any product made at least in part by the fatty acid biosynthetic pathway of the recombinant microbial cell. A fatty acid derivative also includes any product made at least in part by a fatty acid pathway intermediate, such as an acyl-ACP intermediate. The fatty acid biosynthetic pathways described herein can include fatty acid pathway enzymes which can be engineered to produce fatty acid derivatives, and in some instances additional enzymes can be expressed to produce fatty acid derivatives having desired carbon chain characteristics, such as, for example, branched chain fatty acids and branched fatty acid derivatives (including, for example, anteiso-branched fatty acids and derivatives thereof) produced by enzymes of the branched chain fatty acid biosynthetic pathways described herein. Fatty acid derivatives include, but are not limited to, fatty acids, fatty aldehydes, fatty alcohols, fatty esters (such as waxes), hydrocarbons (such as alkanes and alkenes (e.g., terminal olefins and internal olefins)), and ketones.

Likewise, unless otherwise specified, a "branched fatty acid derivative" is intended to include any product made at least in part by a branched-chain fatty acid biosynthetic pathway (i.e., BCFA pathway) of a recombinant microbial cell described herein. A branched fatty acid derivative also includes any product made at least in part from a BCFA pathway intermediate, such as a branched acyl-ACP intermediate. Branched fatty acid derivatives include, but are not limited to, branched fatty acids, branched fatty aldehydes, branched fatty alcohols, branched fatty esters (such as waxes), branched hydrocarbons (such as branched alkanes and branched alkenes (e.g., branched terminal olefins and branched internal olefins)) and branched ketones.

An "endogenous" polypeptide (e.g., a polypeptide "endogenous" to a recombinant microbial cell), refers to a polypeptide encoded by the genome of the parental (i.e., host) cell from which the recombinant cell is engineered.

A "exogenous" polypeptide refers to a polypeptide which is not encoded by the genome of the parental microbial cell. A variant (i.e., mutant) polypeptide is an example of an exogenous polypeptide.

In embodiments of the invention wherein the recombinant polynucleotide sequence encodes an endogenous polypeptide, in some instances the endogenous polypeptide is overexpressed. Overexpression can be achieved by any suitable means. As used herein, "overexpress" means to express or cause to be expressed a polynucleotide or a polypeptide in a cell at a greater concentration than is normally expressed in a corresponding host (for example, wild-type) cell under the same conditions. For example, a polynucleotide can be "overexpressed" in a recombinant microbial cell when the polynucleotide is present in a greater concentration in the recombinant microbial cell as compared to its concentration in a non-recombinant microbial cell of the same species under the same conditions.

The term "increasing the level of expression of an endogenous polypeptide" means to cause the overexpression of a polynucleotide sequence encoding the endogenous polypeptide, or to cause the overexpression of an endogenous polypeptide sequence. The degree of overexpression can be about 1.5-fold or more, about 2-fold or more, about 3-fold or more, about 5-fold or more, about 10-fold or more, about 20-fold or more, about 50-fold or more, about 100-fold or more, or any range therein.

The term "increasing the level of activity of an endogenous polypeptide" means to enhance the biochemical or biological function (e.g., enzymatic activity) of an endogenous polypeptide. The degree of enhanced activity can be about 10% or more, about 20% or more, about 50% or more, about 75% or more, about 100% or more, about 200% or more, about 500% or more, about 1000% or more, or any range therein.

In some embodiments, overexpression of the endogenous polypeptide in the recombinant microbial cell is achieved by the use of an exogenous regulatory element. The term "exogenous regulatory element" generally refers to a regulatory element (such as, an expression control sequence or a chemical compound) originating outside of the host cell. However, in certain embodiments, the term "exogenous regulatory element" (e.g., "exogenous promoter") can refer to a regulatory element derived from the host cell whose function is replicated or usurped for the purpose of controlling the expression of the endogenous polypeptide in the recombinant cell. For example, if the host cell is an *E. coli* cell, and the polypeptide is an endogenous polypeptide, then expression of the endogenous polypeptide the recombinant cell can be controlled by a promoter derived from another *E. coli* gene. In some embodiments, the exogenous regulatory element that causes an increase in the level of expression and/or activity of an endogenous polypeptide is a chemical compound, such as a small molecule.

In some embodiments, the exogenous regulatory element which controls the expression of a polynucleotide (e.g., an endogenous polynucleotide) encoding an endogenous polypeptide is an expression control sequence which is operably linked to the endogenous polynucleotide by recombinant integration into the genome of the host cell. In certain embodiments, the expression control sequence is integrated into a host cell chromosome by homologous recombination using methods known in the art (e.g., Datsenko et al., Proc. Natl. Acad. Sci. U.S.A., 97(12): 6640-6645 (2000)).

Expression control sequences are known in the art and include, for example, promoters, enhancers, polyadenylation signals, transcription terminators, internal ribosome entry sites (IRES), and the like, that provide for the expression of the polynucleotide sequence in a host cell. Expression control sequences interact specifically with cellular proteins involved in transcription (Maniatis et al., Science, 236: 1237-1245 (1987)). Exemplary expression control sequences are described in, for example, Goeddel, Gene Expression Technology: Methods in Enzymology, Vol. 185, Academic Press, San Diego, Calif. (1990).

In the methods of the invention, an expression control sequence is operably linked to a polynucleotide sequence. By "operably linked" is meant that a polynucleotide sequence and expression control sequence(s) are connected in such a way as to permit gene expression when the appropriate molecules (e.g., transcriptional activator proteins) are bound to the expression control sequence(s). Operably linked promoters are located upstream of the selected polynucleotide sequence in terms of the direction of transcription and translation. Operably linked enhancers can be located upstream, within, or downstream of the selected polynucleotide. Additional nucleic acid sequences, such as nucleic acid sequences encoding selection markers, purification moieties, targeting proteins, and the like, can be operatively linked to the polynucleotide sequence, such that the additional nucleic acid sequences are expressed together with the polynucleotide sequence.

In some embodiments, the polynucleotide sequence is provided to the recombinant cell by way of a recombinant vector, which comprises a promoter operably linked to the polynucleotide sequence. In certain embodiments, the promoter is a developmentally-regulated, an organelle-specific, a tissue-specific, an inducible, a constitutive, or a cell-specific promoter.

As used herein, the term "vector" refers to a nucleic acid molecule capable of transporting another nucleic acid, i.e., a polynucleotide sequence, to which it has been linked. One type of useful vector is an episome (i.e., a nucleic acid capable of extra-chromosomal replication). Useful vectors are those capable of autonomous replication and/or expression of nucleic acids to which they are linked. Vectors capable of directing the expression of genes to which they are operatively linked are referred to herein as "expression vectors." In general, expression vectors of utility in recombinant DNA techniques are often in the form of "plasmids," which refer generally to circular double stranded DNA loops that, in their vector form, are not bound to the chromosome. The terms "plasmid" and "vector" are used interchangeably herein, inasmuch as a plasmid is the most commonly used form of vector. However, also included are such other forms of expression vectors that serve equivalent functions and that become known in the art subsequently hereto.

In some embodiments, the recombinant vector comprises at least one sequence selected from the group consisting of (a) an expression control sequence operatively linked to the polynucleotide sequence; (b) a selection marker operatively linked to the polynucleotide sequence; (c) a marker sequence operatively linked to the polynucleotide sequence; (d) a purification moiety operatively linked to the polynucleotide sequence; (e) a secretion sequence operatively linked to the polynucleotide sequence; and (f) a targeting sequence operatively linked to the polynucleotide sequence.

The expression vectors described herein include a polynucleotide sequence described herein in a form suitable for expression of the polynucleotide sequence in a host cell. It will be appreciated by those skilled in the art that the design of the expression vector can depend on such factors as the choice of the host cell to be transformed, the level of expression of polypeptide desired, etc. The expression vectors described herein can be introduced into host cells to produce polypeptides, including fusion polypeptides, encoded by the polynucleotide sequences as described herein.

Expression of genes encoding polypeptides in prokaryotes, for example, *E. coli,* is often carried out with vectors containing constitutive or inducible promoters directing the expression of either fusion or non-fusion polypeptides. Fusion vectors add a number of amino acids to a polypeptide encoded therein, usually to the amino- or carboxy- terminus of the recombinant polypeptide. Such fusion vectors typically serve one or more of the following three purposes: (1) to increase expression of the recombinant polypeptide; (2) to increase the solubility of the recombinant polypeptide; and (3) to aid in the purification of the recombinant polypeptide by acting as a ligand in affinity purification. Often, in fusion expression vectors, a proteolytic cleavage site is introduced at the junction of the fusion moiety and the recombinant polypeptide. This enables separation of the recombinant polypeptide from the fusion moiety after purification of the fusion polypeptide. Examples of such enzymes, and their cognate recognition sequences, include Factor Xa, thrombin, and enterokinase. Exemplary fusion expression vectors include pGEX (Pharmacia Biotech, Inc., Piscataway, NJ; Smith et al., Gene, 67: 31-40 (1988)), pMAL (New England Biolabs, Beverly, MA), and pRITS (Pharmacia Biotech, Inc., Piscataway, N.J.), which fuse glutathione S-transferase (GST), maltose E binding protein, or protein A, respectively, to the target recombinant polypeptide.

Vectors can be introduced into prokaryotic or eukaryotic cells via conventional transformation or transfection techniques. As used herein, the terms "transformation" and "transfection" refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in, for example, Sambrook et al. (*supra*).

For stable transformation of bacterial cells, it is known that, depending upon the expression vector and transformation technique used, only a small fraction of cells will take-up and replicate the expression vector. In order to identify and select these transformants, a gene that encodes a selectable marker (e.g., resistance to an antibiotic) can be introduced into the host cells along with the gene of interest. Selectable markers include those that confer resistance to drugs such as, but not limited to, ampicillin, kanamycin, chloramphenicol, or tetracycline. Nucleic acids encoding a selectable marker can be introduced into a host cell on the same vector as that encoding a polypeptide described herein or can be introduced on a separate vector. Host cells which are stably transformed with the introduced nucleic acid, resulting in recombinant cells, can be identified by growth in the presence of an appropriate selection drug.

Similarly, for stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to an antibiotic) can be introduced into the host cells along with the gene of interest. Preferred selectable markers include those which confer resistance to drugs, such as G418, hygromycin, and methotrexate. Nucleic acids encoding a selectable marker can be introduced into a host cell on the same vector as that encoding a polypeptide described herein or can be introduced on a separate vector. Host cells stably transfected with the introduced nucleic acid, resulting in recombinant cells, can be identified by growth in the presence of an appropriate selection drug.

"Gene knockout", as used herein, refers to a procedure by which a gene encoding a target protein is modified or inactivated so to reduce or eliminate the function of the intact protein. Inactivation of the gene may be performed by general methods such as mutagenesis by UV irradiation or treatment with N-methyl-N'-nitro-N-nitrosoguanidine, site-directed mutagenesis, homologous recombination, insertion-deletion mutagenesis, or "Red-driven integration" (Datsenko et al., Proc. Natl. Acad. Sci. USA, 97:6640-45, 2000). For example, in one embodiment, a construct is introduced into a parental cell, such that it is possible to select for homologous recombination events in the resulting recombinant cell. One of skill in the art can readily design a knock-out construct including both positive and negative selection genes for efficiently selecting transfected (i.e., recombinant) cells that undergo a homologous recombination event with the construct. The alteration in the parental cell may be obtained, for example, by replacing through a single or double crossover recombination a wild type (i.e., endogenous) DNA sequence by a DNA sequence containing the alteration. For convenient selection of transformants (i.e., recombinant cells), the alteration may, for example, be a DNA sequence encoding an antibiotic resistance marker or a gene complementing a possible auxotrophy of the host cell. Mutations include, but are not limited to, deletion-insertion mutations. An example of such an alteration in a recombinant cell includes a gene disruption, i.e., a perturbation of a gene such that the product that is normally produced from this gene is not produced in a functional form. This could be due to a complete deletion, a deletion and insertion of a selective marker, an insertion of a selective marker, a frameshift mutation, an in-frame deletion, or a point mutation that leads to premature termination. In some instances, the entire mRNA for the gene is absent. In other situations, the amount of mRNA produced varies.

The term, "the expression of said polynucleotide sequence is modified relative to the wild type polynucleotide sequence", as used herein means an increase or decrease in the level of expression and/or activity of an endogenous polynucleotide sequence. In some embodiments, an exogenous regulatory element which controls the expression of an endogenous polynucleotide is an expression control sequence which is operably linked to the endogenous polynucleotide by recombinant integration into the genome of the host cell. In some embodiments, the expression control sequence is integrated into a host cell chromosome by homologous recombination using methods known in the art.

As used herein, the term "under conditions effective to express said polynucleotide sequences" means any conditions that allow a recombinant cell to produce a desired fatty acid derivative. Suitable conditions include, for example, fermentation conditions. Fermentation conditions can comprise many parameters, such as temperature ranges, levels of aeration, and media composition. Each of these conditions, individually and in combination, allows the host cell to grow. Exemplary culture media include broths or gels. Generally, the medium includes a carbon source that can be metabolized by a recombinant cell directly. Fermentation denotes the use of a carbon source by a production host, such as a recombinant microbial cell of the invention. Fermentation can be aerobic, anaerobic, or variations thereof (such as micro-aerobic). As will be appreciated by those of skill in the art, the conditions under which a recombinant microbial cell can process a carbon source into a branched acyl-ACP or a desired branched fatty acid derivative (e.g., a branched fatty acid, branched fatty ester, branched fatty aldehyde, branched fatty alcohol, branched alkane, or branched olefin) will vary in part, based upon the specific microorganism. In some embodiments, the process occurs in an aerobic environment. In some embodiments, the process occurs in an anaerobic environment. In some embodiments, the process occurs in a micro-aerobic environment.

As used herein, the phrase "carbon source" refers to a substrate or compound suitable to be used as a source of carbon for prokaryotic or simple eukaryotic cell growth. Carbon sources can be in various forms, including, but not limited to polymers, carbohydrates, acids, alcohols, aldehydes, ketones, amino acids, peptides, and gases (e.g., CO and $CO_2$). Exemplary carbon sources include, but are not limited to, monosaccharides, such as glucose, fructose, mannose, galactose, xylose, and arabinose; oligosaccharides, such as fructo-oligosaccharide and galacto-oligosaccharide; polysaccharides such as starch, cellulose, pectin, and xylan; disaccharides, such as sucrose, maltose, cellobiose, and turanose; cellulosic material and variants such as hemicelluloses, methyl cellulose and sodium carboxymethyl cellulose; saturated or unsaturated fatty acids, succinate, lactate, and acetate; alcohols, such as ethanol, methanol, and glycerol, or mixtures thereof. The carbon source can be a branched short-chain carboxylic acid such as isobutyrate, isovalerate, or 2-methyl-butyrate. The carbon source can be a product of photosynthesis, such as glucose. In certain preferred embodiments, the carbon source is biomass. In another preferred embodiment, the carbon source comprises sucrose. In another preferred embodiment, the carbon source comprises glucose.

As used herein, the term "biomass" refers to any biological material from which a carbon source is derived. In some embodiments, a biomass is processed into a carbon source, which is suitable for bioconversion. In other embodiments, the biomass does not require further processing into a carbon source. The carbon source can be converted into a biofuel. An exemplary source of biomass is plant matter or vegetation, such as corn, sugar cane, or switchgrass. Another exemplary source of biomass is metabolic waste products, such as animal matter (e.g., cow manure). Further exemplary sources of biomass include algae and other marine plants. Biomass also includes waste products from industry, agriculture, forestry, and households, including, but not limited to, fermentation waste, ensilage, straw, lumber, sewage, garbage, cellulosic urban waste, and food leftovers. The term "biomass" also can refer to sources of carbon, such as carbohydrates (e.g., monosaccharides, disaccharides, or polysaccharides).

To determine if conditions are sufficient to allow production of a product or expression of a polypeptide, a recombinant microbial cell can be cultured, for example, for about 4, 8, 12, 24, 36, 48, 72, or more hours. During and/or after culturing, samples can be obtained and analyzed to determine if the conditions allow production or expression. For example, the recombinant microbial cells in the sample or the medium in which the recombinant microbial cells were grown can be tested for the presence of a desired product. When testing for the presence of a branched fatty acid, a branched fatty ester, a branched fatty aldehyde, a branched fatty alcohol, a branched hydrocarbon or other branched fatty acid derivative, assays, such as, but not limited to, gas chromatography (GC), mass spectroscopy (MS), thin layer chromatography (TLC), high-performance liquid chromatography (HPLC), liquid chromatography (LC), GC coupled with a flame ionization detector (FID), GC-MS, and LC-MS can be used. When testing for the expression of a polypeptide, techniques such as, but not limited to, Western blotting and dot blotting may be used.

As used herein, the term "microorganism" means prokaryotic and eukaryotic microbial species from the domains *Archaea, Bacteria* and *Eucarya,* the latter including yeast and filamentous fungi, protozoa, algae, and higher *Protista.* The terms "microbes" and "microbial cells" (i.e., cells from microbes) and are used interchangeably with "microorganisms" and refer to cells or small organisms that can only be seen with the aid of a microscope.

In some embodiments, the host cell is a microbial cell. In some embodiments, the host cell is selected from the genus *Escherichia, Bacillus, Lactobacillus, Pantoea, Zymomonas, Rhodococcus, Pseudomonas, Aspergillus, Trichoderma, Neurospora, Fusarium, Humicola, Rhizomucor, Kluyveromyces, Pichia, Mucor, Myceliophtora, Penicillium, Phanerochaete, Pleurotus, Trametes, Chrysosporium, Saccharomyces, Stenotrophamonas, Schizosaccharomyces, Yarrowia, Streptomyces, Synechococcus, Chlorella, or Prototheca.*

In other embodiments, the host cell is a *Bacillus lentus* cell, a *Bacillus brevis* cell, a *Bacillus stearothermophilus* cell, a *Bacillus lichenoformis* cell, a *Bacillus alkalophilus* cell, a *Bacillus coagulans* cell, a *Bacillus circulans* cell, a *Bacillus pumilis* cell, a *Bacillus thuringiensis* cell, a *Bacillus clausii* cell, a *Bacillus megaterium* cell, a *Bacillus subtilis* cell, or a *Bacillus amyloliquefaciens* cell.

In other embodiments, the host cell is a *Trichoderma koningii* cell, a *Trichoderma viride* cell, a *Trichoderma reesei* cell, a *Trichoderma longibrachiatum* cell, an *Aspergillus awamori* cell, an *Aspergillus fumigates* cell, an *Aspergillus foetidus* cell, an *Aspergillus nidulans* cell, an *Aspergillus niger* cell, an *Aspergillus oryzae* cell, a *Humicola insolens* cell, a *Humicola lanuginose* cell, a *Rhodococcus opacus* cell, a *Rhizomucor miehei* cell, or a *Mucor michei* cell.

In yet other embodiments, the host cell is a *Streptomyces lividans* cell or a *Streptomyces murinus* cell.

In yet other embodiments, the host cell is an *Actinomycetes* cell.

In some embodiments, the host cell is a *Saccharomyces cerevisiae* cell.

In still other embodiments, the host cell is a CHO cell, a COS cell, a VERO cell, a BHK cell, a HeLa cell, a Cvl cell, an MDCK cell, a 293 cell, a 3T3 cell, or a PC12 cell.

In some embodiments, the host cell is a cell from an eukaryotic plant, algae, cyanobacterium, green-sulfur bacterium, green non-sulfur bacterium, purple sulfur bacterium, purple non-sulfur bacterium, extremophile, yeast, fungus, an engineered organism thereof, or a synthetic organism. In some embodiments, the host cell is light-dependent or fixes carbon. In some embodiments, the host cell has autotrophic activity. In some embodiments, the host cell has photoautotrophic activity, such as in the presence of light. In some embodiments, the host cell is heterotrophic or mixotrophic in the absence of light.

In certain embodiments, the host cell is a cell from *Avabidopsis thaliana, Panicum virgatum, Miscanthus giganteus, Zea mays, Botryococcuse braunii, Chlamydomonas reinhardtii, Dunaliela salina, Synechococcus Sp. PCC 7002, Synechococcus Sp. PCC 7942, Synechocystis Sp. PCC 6803, Thermosynechococcus elongates BP-1, Chlorobium tepidum, Chlorojlexus auranticus, Chromatiumm vinosum, Rhodospirillum rubrum, Rhodobacter capsulatus, Rhodopseudomonas palusris, Clostridium ljungdahlii, Clostridiuthermocellum, Penicillium chrysogenum, Pichia pastoris, Saccharomyces cerevisiae, Schizosaccharomyces pombe, Pseudomonas jluorescens, Pantoea citrea* or *Zymomonas mobilis.* In certain embodiments, the host cell is a cell from *Chlorella fusca, Chlorella protothecoides, Chlorella pyrenoidosa, Chlorella kessleri, Chlorella vulgaris, Chlorella saccharophila, Chlorella sorokiniana, Chlorella ellipsoidea, Prototheca stagnora, Prototheca portoricensis, Prototheca moriformis, Prototheca wickerhamii, or Prototheca zopfii.*

In some embodiments, the host cell is a Gram-positive bacterial cell. In other embodiments, the host cell is a Gram-negative bacterial cell.

In certain preferred embodiments, the host cell is an *E. coli* cell. In some embodiments, the *E. coli* cell is a strain B, a strain C, a strain K, or a strain W *E. coli* cell.

In certain embodiments of the invention, the host cell is engineered to express (or overexpress) a transport protein. Transport proteins can export polypeptides and organic compounds (e.g., fatty acids or derivatives thereof) out of a host cell.

As used herein, the term "metabolically engineered" or "metabolic engineering" involves rational pathway design and assembly of polynucleotides corresponding to biosynthetic genes, genes associated with operons, and control elements of such polynucleotides, for the production of a desired metabolite, such as, for example, a branched $\alpha$-keto acid, a branched p-ketoacyl-ACP, a branched acyl-ACP, or a branched fatty acid derivative, in a recombinant cell, such as, a recombinant microbial cell. "Metabolically engineered" can further include optimization of metabolic flux by regulation and optimization of transcription, translation, protein stability and protein functionality using genetic engineering and appropriate culture conditions including the reduction of, disruption, or knocking out of, a competing metabolic pathway that competes with an intermediate leading to a desired pathway. A "biosynthetic gene" can be native to the host cell (i.e., a gene which is not modified from the host cell), or, can be exogenous (heterologous) to the host cell either by virtue of being foreign to the host cell, or by being modified by mutagenesis, recombination, and/or association in the recombinant cell with a exogenous (heterologous) expression control sequence . A biosynthetic gene encodes a "biosynthetic polypeptide" or a "biosynthetic enzyme".

The term "biosynthetic pathway", also referred to as "metabolic pathway", refers to a set of biochemical reactions, catalyzed by biosynthetic enzymes, which convert one chemical species into another. As used herein, the term "fatty acid biosynthetic pathway" (or more simply, "fatty acid pathway") refers to a set of biochemical reactions that produces fatty acid derivatives (e.g., fatty acids, fatty esters, fatty aldehydes, fatty alcohols, alkanes, alkenes, ketones, and so forth). The fatty acid pathway includes fatty acid pathway biosynthetic enzymes (i.e., "fatty acid pathway enzymes") that can be engineered, as described herein, to produce fatty acid derivatives, and in some embodiments can be expressed with additional enzymes to produce fatty acid derivatives having desired carbon chain characteristics. For example, a "branched chain fatty acid biosynthetic pathway" (i.e., a "BCFA pathway") as described herein includes enzymes sufficient to produce branched fatty acid derivatives.

The term "recombinant microbial cell" refers to a microbial cell (i.e., a microorganism) that has been genetically modified (i.e., "engineered") by the introduction of genetic material into a "parental microbial cell" (i.e., a host cell) of choice, thereby modifying or altering the cellular physiology and biochemistry of the parental cell. Through the introduction of genetic material, the recombinant microbial cell acquires a new or improved property compared to that of the parental microbial cell, such as, for example, the ability to produce a new, or greater quantities of, an intracellular metabolite. Recombinant microbial cells provided herein can express a plurality of biosynthetic enzymes (e.g., fatty acid pathway enzymes, such as BCFA pathway enzymes) involved in pathways for the production of, e.g., a branched acyl-CoA, a branched acyl-ACP, or a branched fatty acid derivative (such as a branched fatty acid, branched fatty ester, branched wax ester, branched fatty aldehyde, branched fatty alcohol, branched alkane, branched alkene, branched terminal olefin, branched internal olefin, or branched ketone), from a suitable carbon source. The genetic material introduced into the parental microbial cell contains gene(s), or parts of genes, coding for one or more of the enzymes involved in a biosynthetic pathway (that is, biosynthetic enzymes) for the production of a branched fatty acid derivative, and may also include additional elements for the expression and/or regulation of expression of these genes, such as promoter sequences. Accordingly, recombinant microbial cells described herein have been genetically engineered to express or overexpress biosynthetic enzymes involved in branched chain fatty acid (BCFA) biosynthetic pathways as described herein.

It is understood that the terms "recombinant microbial cell" and "recombinant microorganism" refer not only to the particular recombinant microbial cell/microorganism, but to the progeny or potential progeny of such a microbial cell.

A recombinant microbial cell can, alternatively or in addition to comprising genetic material introduced into the parental microbial cell, include a reduction, disruption, deletion or a "knocking-out" of a gene or polynucleotide to alter the cellular physiology and biochemistry of the parental microbial cell. Through the reduction, disruption, deletion or knocking-out of a gene or polynucleotide (also known as "attenuation" of the gene or polynucleotide), the recombinant microbial cell acquires a new or improved property (such as, for example, the ability to produce a new or greater quantities of an intracellular metabolite, the ability to improve the flux of a metabolite through a desired pathway, and/or the ability to reduce the production of an undesirable by-product) compared to that of the parental microbial cell.

## Engineering Recombinant Microbial Cells to Produce Branched Fatty Acid Derivatives

Branched chain fatty acids are normally produced in bacteria such as *Bacillus, Stenotrophomonas, Streptomyces, Listeria, Staphylococcus, and Streptococcus* (Kaneda, Microbiol. Rev. 55: 288-302 (1991). Branched acyl-CoA molecules are synthesized in such microorganisms by the action of a branched alpha-keto acid dehydrogenase (BKD) complex (Cropp et al., Can J Microbiol 46: 506-14 (2000)). BKD complexes also occur in microorganisms such as *Pseudomonas* that are capable of metabolizing branched-chain amino acids (leucine, isoleucine or valine) or branched-chain $\alpha$-keto acids as carbon sources (Sokatch et al., J. Bacteriol. 148: 647-652 (1981)). Enzymes with beta-ketoacyl ACP synthase III activity (also termed "FabH") that utilize branched-CoA substrates then catalyze the initial condensation of the branched acyl-CoA with malonyl-ACP to form a branched $\beta$-keto acyl-ACP intermediate, which then enters the fatty acid synthase (FAS) cycle to elongate the branched acyl chains.

In nature, some bacteria do not produce branched chain fatty acids; for instance, native *E. coli* lacks components of a BKD complex, and the native *E. coli* beta-ketoacyl ACP synthase (FabH) enzyme only accepts straight-chain acyl-CoA molecules in the condensation with malonyl-ACP, producing straight-chain $\beta$-keto acyl-ACP intermediates and generating straight-chain fatty acids.

The invention is based in part on the discovery that by engineering microorganisms to introduce or to improve biosynthetic pathways involving the generation of branched chain alpha-keto acids and branched chain acyl-CoA intermediates from simple sugars, metabolic flux through branched chain pathway intermediates is created or is enhanced, and compositions of branched fatty acid products are optimized, resulting in efficient microbial production of branched chain fatty acids and branched chain fatty acid derivatives from simple sugars or biomass.

As the ultimate goal is to provide environmentally responsible and cost-effective methods for the production of branched chain fatty derivatives on an industrial scale starting from sugars (such as monosaccharides or disaccharides) or biomass, improvements in yield of microbially produced branched chain molecules, and optimization of the compositions of microbially produced branched chain molecules, such as by increasing the amount of anteiso-branched chain products relative to iso-branched chain products, is desirable. Accordingly, strategies for the overproduction of various pathway intermediates have been examined to increase the flux of metabolites through branched chain fatty acid biosynthetic pathways. Pathways that direct metabolic flux from a starting material, such as a sugar, to a branched acyl-CoA intermediate, to a branched acyl-ACP intermediate, and to a branched fatty acid product or branched fatty acid derivative product, can be engineered in an industrially useful microorganism.

In one aspect, the invention includes a recombinant microbial cell comprising polynucleotides encoding one or more enzymes which participate in the biosynthesis of a branched acyl-ACP intermediate when the microorganism is cultured in the presence of a carbon source under conditions effective to expresses the polynucleotides. In some embodiments, the recombinant microbial cell further comprises one or more polynucleotides each which encodes a polypeptide having a fatty acid derivative enzyme activity, wherein the recombinant microbial cell produces a branch fatty acid derivative when cultured in the presence of a carbon source under conditions sufficient to expresses the polynucleotides. The invention also includes methods of making branched fatty acid derivatives comprising culturing a recombinant microbial cell of the invention.

The recombinant microbial cell can be a filamentous fungi, an algae, a yeast, or a prokaryote such as a bacterium (e.g., an *E. coli* or a *Bacillus sp*).

In general, branched fatty acid derivatives (such as, branched fatty acids, branched fatty esters (including branched fatty acid methyl esters (branched-FAMEs), branched fatty acid ethyl esters (branched-FAEEs), and branched wax esters), branched fatty aldehydes, branched fatty alcohols, branched ketones, and branched hydrocarbons (including branched alkanes, branched alkenes, branched terminal olefins, branched internal olefins)) can be produced in a recombinant microbial cell of the invention via the branched fatty acid biosynthetic pathway ("BCFA pathway") depicted in Figure 1.

To produce a branched fatty acid derivative, the recombinant microbial cell utilizes a branched acyl-CoA molecule as a "primer" for the initiation of the branched fatty acyl chain elongation process. The branched fatty acyl elongation process initially involves condensation of the branched acyl-CoA primer with a malonyl-ACP molecule, catalyzed by a β-ketoacyl ACP synthase III enzyme, to form a branched β-ketoacyl-ACP intermediate (as depicted in step (D) of Figure 1). The branched β-ketoacyl-ACP intermediate undergoes keto-reduction, dehydration and enoyl-reduction at the β-carbon to form an initial branched acyl-ACP intermediate, which undergoes further cycles of condensation with malonyl-ACP, keto-reduction, dehydration, and enoyl-reduction to form branched acyl-ACP intermediates of increasing length. The elongated branched acyl-ACP intermediate is then converted to a branched fatty acid derivative (such as, a branched fatty acid, a branched fatty ester, a branched fatty aldehyde, a branched fatty alcohol, a branched hydrocarbon, or a branched ketone). This is in contrast to the process in, for example, wild-type *E. coli,* which produces straight-chain fatty acids but not branched chain fatty acids. In wild-type *E. coli,* the straight-chain primer molecule acetyl-CoA initially condenses with a malonyl-ACP molecule to form a straight-chain β-keto acyl-ACP intermediate, which likewise undergoes cycles of keto-reduction, dehydration, enoyl-reduction and condensation with additional malonyl-ACP molecules, to ultimately produce, e.g., a straight-chain fatty acid.

The above-noted branched acyl-CoA "primer" molecule can be supplied to the BCFA biosynthetic pathway of the recombinant microbial cell of the invention by a number of methods, as follows.

In one embodiment, a branched acyl-CoA molecule is generated by the native biosynthetic machinery of the microbial cell (e.g., is endogenous to the parental microbial cell). In some such instances, to increase the amount of the branched acyl-CoA molecule produced in the recombinant microbial cell, one or more enzymes endogenous to the parental microbial cell which contribute to the production of branched acyl-CoA (such as, for example, one or more components of a native BKD complex, corresponding to the step labeled (C) of the pathway in Figure 1) can be overexpressed in the recombinant microbial cell.

In another embodiment, a branched acyl-CoA molecule is produced in the recombinant microbial cell by engineering the cell to express exogenous enzymes, such as one or more components of an exogenous BKD complex, which diverts metabolic flux through branched α-keto acid intermediates to produce branched acyl-CoA molecules, as represented by step (C) of Figure 1. This approach is particularly useful in engineering microbial cells such as *E. coli* that do not ordinarily produce branched fatty acids. Polynucleotides encoding components of the BKD complex can be obtained from microorganisms that normally produce branched chain fatty acids or can metabolize branched-chain amino acids or branched α-ketoacids, including, but not limited to, strains of *Bacillus, Pseudomonas, Streptomyces, Listeria, Staphylococcus, and Streptococcus.*

A BKD complex comprises three components: an E1 component having α-keto acid dehydrogenase activity (e.g., EC 1.2.4.4), which, depending on the source, may be a single polypeptide (that is, a monomer), or, two different polypeptides (i.e., a heterodimer) denoted E1alpha and E1beta; an E2 component having lipoamide acyltransferase activity (e.g., EC 2.3.1.168), and a third component, denoted E3, having dihydrolipoamide dehydrogenase activity (e.g., EC 1.8.1.4). Both the E1 (or E1alpha/E1beta) and E2 components of the BKD complex utilize branched substrates. In some instances, an enzyme having dihydrolipoamide dehydrogenase activity (e.g., EC 1.8.1.4) that is endogenous to a microbial cell which does not normally produce branched fatty acids (such as *E. coli*) but which can nevertheless utilize branched chain substrates can be used instead of a BKD E3 component derived from a strain that normally produces branched chain fatty acids or metabolizes branched-chain amino acids or branched α-ketoacids.

In one embodiment, one or more polynucleotide sequences each encoding a polypeptide having a BKD activity (α-keto acid acid dehydrogenase activity, lipoamide acyltransferase activity, or dihydrolipoamide dehydrogenase activity) which is endogenous to the parental microbial cell is overexpressed in the recombinant microbial cell. In another embodiment, one or more polynucleotide sequences each encoding a polypeptide having a BKD activity (a-keto acid acid dehydrogenase activity, lipoamide acyltransferase activity, or dihydrolipoamide dehydrogenase activity) which is exogenous to the parental microbial cell is expressed or overexpressed in the recombinant microbial cell. Polynucleotide sequences encoding polypeptides having BKD activities (a-keto acid dehydrogenase activity, lipoamide acyltransferase activity, dihydrolipoamide dehydrogenase activity) can be obtained from a microorganism that normally produces branched chain fatty acids or can metabolize branched-chain amino acids or branched α-ketoacids (including, but not limited to, strains of *Bacillus, Pseudomonas, Streptomyces, Listeria, Staphylococcus, and Streptococcus*). In some embodiments, the polynucleotide sequence is modified to generate a variant polypeptide having a BDK activity and an improved property, compared to that of the parent polypeptide, which is more suited to the microbial cell and/or to the pathway being engineered; such as, for example, increased catalytic activity or improved stability under conditions in which the recombinant microbial cell is cultured; reduced inhibition (e.g., reduced feedback inhibition) by a cellular metabolite or by a culture media component, and the like. Non-limiting examples of BKD component polypeptides and nucleic acids encoding such polypeptides for use in engineering part (C) of the BCFA biosynthetic pathway are provided in Table 4, below.

In another embodiment, a branched acyl-CoA molecule is produced in the recombinant microbial cell by engineering the cell to express or overexpress certain transport/activation enzymes that participate in the conversion of a branched short-chain carboxylic acid substrate to the branched acyl-CoA molecule. The transport/activation enzymes can include, without limitation, a phosphotransbutyrylase (ligase) (e.g., EC 2.3.1.19) and/or a butyrate kinase (e.g., EC 2.7.2.7). In one embodiment, the recombinant microbial cell expresses a phosphotransbutyrylase (ligase) and a butyrate kinase from *Clostridium acetobutylicum.* The branched short-chain carboxylic acid substrate (such as isobutyrate, isovalerate, or 2-methyl-butyrate) is converted to the branched acyl-CoA molecule (such as, isobutyryl-CoA, isovaleryl-CoA, or 2-methyl-butyryl-CoA). In some instances, such a recombinant microbial cell is cultured in the presence of (i.e., is "fed") the branched short-chain carboxylic acid substrate, e.g., isobutyrate, isovalerate, or 2-methyl-butyrate, resulting in the production of isobutyryl-CoA, isovaleryl-CoA, or 2-methyl-butyryl-CoA, respectively.

In another embodiment, the branched acyl-CoA molecule isobutyryl-CoA is produced in the recombinant microbial cell by engineering the cell to express a crotonyl-CoA reductase (Ccr, EC 1.6.5.5, 1.1.1.1) and an isobutyryl-CoA mutase (large subunit IcmA, EC 5.4.99.2; small subunit IcmB, EC 5.4.99.2) (Han and Reynolds, J. Bacteriol. 179:5157, 1997). Non-limiting examples of *ccr* and *icm* genes include the *ccr, icmA* and *icmB* genes from *Streptomyces coelicolor* (e.g., NCBI Accession Numbers NP_630556, NP_629554, and NP_630904, respectively), and the *ccr, icmA* and *icmB* genes from *Streptomyces cinnamonensis* (e.g., NCBI accession numbers AAD53915, AAC08713, and AJ246005, respectively).

In another embodiment, a branched acyl-CoA molecule is produced in the recombinant microbial cell by engineering the cell to express enzymes that direct metabolic flux from simple starting materials (e.g., sugars, such as glucose) to generate branched α-keto acid intermediates, which are then acted upon by the native biosynthetic machinery of the particular recombinant microbial cell, or, by BKD complex components engineered in the recombinant microbial cell (for example, as described above), to generate branched acyl-CoA molecules. An example of this approach is described in more detail below and is outlined in Figure 3A.

As noted above, the branched acyl-CoA molecule serves as a primer for branched acyl-chain elongation. Initiation of the elongation process involves condensation of the branched acyl-CoA with a malonyl-ACP molecule to form a branched β-ketoacyl-ACP intermediate. This step, as represented by part (D) of Figure 1, is catalyzed in the recombinant microbial cell by an enzyme having β-ketoacyl-ACP synthase III activity (e.g., EC 2.3.1.180) which utilizes a branched acyl-CoA molecule as a substrate (in other words, an enzyme having "branched chain β-ketoacyl-ACP synthase III" activity). The enzyme can be endogenous to the recombinant microbial cell (for example, if the parental microbial cell normally produces branched chain fatty acids), or exogenous the recombinant microbial cell.

In one embodiment, a polynucleotide encoding a polypeptide endogenous to the parental microbial cell having branched chain β-ketoacyl-ACP synthase III activity is overexpressed in the recombinant microbial cell. In another embodiment, a polynucleotide encoding a polypeptide having branched chain β-ketoacyl-ACP synthase III activity which is exogenous to the parental microbial cell is expressed or overexpressed in the recombinant microbial cell. A polynucleotide sequence encoding a polypeptide having branched chain β-ketoacyl-ACP synthase III activity can be obtained from a microbial cell that normally produces branched chain fatty acids (including, but not limited to, strains of *Bacillus, Streptomyces, Listeria, Staphylococcus, and Streptococcus*). In some embodiments, the polynucleotide sequence is modified to generate a variant polypeptide having branched chain β-ketoacyl-ACP synthase III activity and an improved property, compared to that of the parent polypeptide, which is more suited to the microbial cell and/or to the pathway being engineered; such as, for example, increased catalytic activity or improved stability under conditions in which the recombinant microbial cell is cultured; reduced inhibition (e.g., reduced feedback inhibition) by a cellular metabolite or by a culture media component, and the like. Non-limiting examples of β-ketoacyl-ACP synthase III enzymes and genes encoding such enzymes for use in engineering part (D) of the branched fatty acid pathway are provided in Table 5, below.

One or more enzymes endogenous to the parental microbial cell may compete for substrate with enzymes of the engineered BCFA biosynthetic pathway in the recombinant microbial cell, or may break down or otherwise divert an intermediate in the BCFA biosynthetic pathway; genes encoding such undesired endogenous enzymes may be attenuated to increase the production of branched fatty acid derivatives by the recombinant microbial cell. For example, in *E. coli,* the endogenous β-ketoacyl-ACP synthase III (UniProtKB/Swiss-Prot Protein Accession Number P0A6R0), encoded by the *E. coli fabH* gene, primarily utilizes short straight-chain acyl-CoA molecules such as acetyl-CoA, but does not utilize branched acyl-CoA molecules, and thus competes with enzymes of the branched chain pathway for malonyl-ACP and other substrates and diverts metabolic flux away from the BCFA pathway. Deleting or otherwise reducing the expression of the *E. coli fabH* gene thus directs biosynthesis in recombinant *E. coli* away from straight-chain and more towards branched β-ketoacyl-ACP intermediates, and ultimately more towards branched-chain fatty acid production .

The branched β-ketoacyl-ACP intermediate generated in part (D) of the BCFA pathway (Figure 1) can undergo elongation by successive cycles of keto-reduction, dehydration and enoyl-reduction at the beta carbon and further condensation with malonyl-ACP molecules catalyzed by a fatty acid synthase (FAS) complex, such as for example a Type II FAS complex, adding 2-carbon units to the lengthening chain of the branched acyl-ACP intermediate as represented by part (E) of Figure 1. In one embodiment, an endogenous FAS complex native to the recombinant microbial cell catalyzes cycles of condensation with malonyl-ACP / keto-reduction / dehydration / enoyl-reduction to produce the branched acyl-ACP intermediate.

Branched fatty acid derivatives (such as branched fatty acids, branched fatty esters, branched fatty aldehydes, branched fatty alcohols, branched hydrocarbons, and branched ketones) can be produced from the branched acyl-ACP intermediate, as will be described in more detail below. Accordingly, in some embodiments, the recombinant microbial cell further comprises one or more polynucleotide sequences each encoding a polypeptide having fatty acid derivative enzyme activity, such as thioesterase (e.g., TesA), decarboxylase, carboxylic acid reductase (CAR; e.g., CarB), alcohol dehydrogenase / aldehyde reductase; aldehyde decarbonylase, fatty alcohol forming acyl-CoA reductase (FAR), acyl ACP reductase (AAR), ester synthase, or acyl-CoA reductase (ACR1), OleA, OleCD, or OleBCD, wherein the microbial cell produces a composition comprising a branched fatty acid, a branched fatty ester (such as a branched fatty methyl ester, branched fatty ethyl ester, a branched wax ester), a branched fatty aldehyde, a branched fatty alcohol, a branched hydrocarbon (such as a branched alkane, a branched terminal olefin, or a branched internal olefin), or a branched ketone, when the microbial cell is cultured in the presence of a carbon source under conditions effective to expresses the polynucleotides. The invention also includes methods for the production of a branched fatty acid derivative comprising culturing a recombinant microbial cell of the invention.

## Engineering Recombinant Microbial Cells to Produce Anteiso-Branched or Iso-Branched Fatty Acid Derivatives

The branching of the fatty acid derivative molecules can occur in the iso-configuration or the anteiso- configuration. Branched chain fatty acids and their derivatives, particularly in the anteiso-configuration, are preferred components of fuel compositions, due to lower melting points and higher oxidative stabilities compared to the non-branched (i.e., straight-chain) compounds.

To preferentially produce a particular branched fatty acid derivative, such as, an anteiso-branched fatty acid derivative or an iso-branched fatty acid derivative, the recombinant microbial cell can be modified to generate a particular branched acyl-CoA molecule, which serves as a primer for the initiation of chain elongation. The structure of the branched acyl-CoA primer molecule in part determines the structure of the final product.

For example, condensation of the branched acyl-CoA molecule 2-methyl-butyryl-CoA with malonyl-ACP catalyzed by a branched chain β-ketoacyl-ACP synthase III generates the anteiso-branched β-ketoacyl-ACP intermediate 4-methyl-3-oxo-hexanoyl-ACP, leading to production of anteiso-branched acyl-ACP intermediates and ultimately anteiso-branched fatty acid derivatives (Figure 2A). On the other hand, condensation of isobutyryl-CoA or isovaleryl-CoA with malonyl-ACP generates the iso-branched β-ketoacyl-ACP intermediates 4-methyl-3-oxo-pentanoyl-ACP or 5-methyl-3-oxo-hexanoyl-ACP, respectively, leading to production of iso-branched acyl-ACP intermediates and ultimately iso-branched fatty acid derivatives (Figure 2B).

Manipulation of various amino acid biosynthetic pathways has been shown to increase the production of those amino acids in microbial cells (Guillouet S., et al., Appl. Environ. Microbiol. 65:3100-3107 (1999); Lee K.H., et al., Mol. Syst. Biol. 3:149 (2007)). Amino acid biosynthetic pathways have been used in the production of short chain branched alcohols in *E. coli* (Atsumi S. and Liao J.C., Appl. Environ. Microbiol. 74(24): 7802-7808 (2008); Cann A.F. and Liao J.C., Appl Microbiol Biotechnol. 81(1):89-98(2008); Zhang K., et al., Proc. Natl. Acad. Sci. U S A. 105(52):20653-20658(2008)). The present invention is based in part on the discovery that directing the flux of certain amino acid biosynthetic metabolites to the production of branched α-keto acid intermediates, and diverting those branched α-keto acid intermediates to conversion into branched acyl-CoAs and entry into the fatty acid biosynthetic pathway optimizes the structures and improve the yields of branched chain fatty acid products.

Accordingly, in one aspect, the invention includes a recombinant microbial cell comprising polynucleotides encoding one or more enzymes (i.e., "BCFA pathway enzymes") which participate in the conversion of a sugar to the branched α-keto acid molecule 2-keto-3-methylvalerate (also known as α-keto-β-methylvalerate) when the microorganism is cultured in the presence of a carbon source under conditions sufficient to expresses the polynucleotides. The 2-keto-3-methylvalerate molecule is a branched α-keto acid intermediate in the microbial production of anteiso-branched fatty acid derivatives according to the BCFA pathway (see Figure 2A and Figure3B).

In another embodiment, the invention includes a recombinant microbial cell comprising polynucleotides encoding one or more BCFA pathway enzymes which participate in the biosynthesis of an anteiso-branched acyl-ACP intermediate when the microbial cell is cultured in the presence of a carbon source under conditions sufficient to expresses the polynucleotides. In another embodiment, the recombinant microbial cell further comprises one or more polynucleotides encoding one or more polypeptides each having a fatty acid derivative enzyme activity, wherein the recombinant microbial cell produces an anteiso-branched fatty acid derivative when cultured in the presence of a carbon source under conditions sufficient to expresses the polynucleotides.

In another aspect, the invention includes methods for the production of compositions comprising anteiso-branched fatty acid derivatives, comprising culturing a recombinant microbial cell of the invention.

Figures 3A and 3B show exemplary biosynthetic pathways for the conversion of a starting material (e.g., a sugar, such as glucose) to an anteiso-branched α-keto acid intermediate, 2-keto-3-methylvalerate, which is then converted to an anteiso-branched acyl-CoA primer, 2-methyl-butyryl-CoA. Condensation of the 2-methyl-butyryl-CoA primer with malonyl-ACP results in an anteiso-branched β-keto acyl-ACP intermediate, 4-methyl-3-oxo-hexanoyl-ACP. The anteiso-branched β-keto acyl-ACP intermediate is a starter unit for further cycles of FAS-catalyzed elongation by condensation with additional malonyl-ACP molecules to generate anteiso-branched acyl-ACP, according to the general pathway diagrammed in Figure 1. The anteiso-branched acyl-ACP is then converted in further biocatalytic steps, catalyzed by one or more fatty acid derivative enzymes, to produce an anteiso-branched fatty acid derivative.

## Pathway Part A: Sugar to 2-Ketobutyrate

To generate the anteiso-branched α-keto acid intermediate 2-keto-3-methylvalerate from the starting material, either or both of two pathways, each which produces a common α-ketobutyrate (2-ketobutyrate) intermediate that is subsequently converted to 2-keto-3-methylvalerate, can be engineered in the recombinant microbial cell. These pathways are diagramed in Figure 3A.

### *Pathway Part A.1 (Threonine Intermediate)*

The first pathway leading to the common α-ketobutyrate intermediate, as represented by part (A.1) of Figure 3A, involves production of the pathway intermediate threonine by threonine biosynthetic enzymes, followed by the deamination of threonine to α-ketobutyrate catalyzed by an enzyme with threonine dehydratase activity.

In part (A.1) of the pathway, increasing metabolic flux to the pathway intermediate threonine can be accomplished by expressing polynucleotides encoding enzymes involved in threonine biosynthesis, including enzymes with aspartate kinase activity (e.g., EC 2.7.2.4; also termed aspartokinase activity), which catalyzes the conversion of aspartate to aspartyl phosphate; aspartate-semialdehyde dehydrogenase activity (e.g., EC 1.2.1.11), which catalyzes the conversion of aspartyl phosphate to aspartate semialdehyde; homoserine dehydrogenase activity (e.g., EC 1.1.1.3), which catalyzes the conversion of aspartate semialdehyde to homoserine; homoserine kinase activity (e.g., EC 2.7.1.39), which catalyzes the conversion of homoserine to O-phospho-L-homoserine; and threonine synthase activity (e.g., EC 4.2.3.1), which catalyzes the conversion of O-phospho-L-homoserine to threonine. Not all of the activities listed above need be engineered in the recombinant microbial cell to increase metabolic flux through the threonine intermediate; in some instances, an activity already present in the parental microbial cell (for example, a polypeptide having that activity which is produced in the parental microbial cell by a non-recombinant native gene) will be sufficient to catalyze a step listed above. In one embodiment, the recombinant microbial cell is engineered to recombinantly express one or more polynucleotides selected from: a polynucleotide encoding a polypeptide having aspartate kinase activity, wherein the polypeptide catalyzes the conversion of aspartate to aspartyl phosphate; a polynucleotide encoding a polypeptide having aspartate-semialdehyde dehydrogenase activity, wherein the polypeptide catalyzes the conversion of aspartyl phosphate to aspartate semialdehyde; a polynucleotide encoding a polypeptide having homoserine dehydrogenase activity, wherein the polypeptide catalyzes the conversion of aspartate semialdehyde to homoserine; a polynucleotide encoding a polypeptide having homoserine kinase activity, wherein the polypeptide catalyzes the conversion of homoserine to O-phospho-L-homoserine; a polynucleotide encoding a polypeptide having threonine synthase activity, wherein the polypeptide catalyzes the conversion of O-phospho-L-homoserine to threonine; wherein the recombinant microbial cell has increased metabolic flux through the pathway intermediate threonine compared to the parental microbial cell. In some instances, the polypeptide encoded by recombinantly expressed polynucleotide is present in the recombinant microbial cell at a greater concentration compared to its concentration in the parent microbial cell when cultured under the same conditions, i.e., the polypeptide is "overexpressed" in the recombinant cell. For example, the recombinantly expressed polynucleotide can be operatively linked to a promoter which expresses the polynucleotide in the recombinant microbial cell at a greater concentration than is normally expressed in the parental microbial cell when cultured under the same conditions. In one embodiment, an *E. coli thrA* gene is used, which encodes a bifunctional ThrA with aspartate kinase and homoserine dehydrogenase activities. In another embodiment, a mutant *E. coli thrA* gene is used, encoding a variant enzyme with aspartate kinase and homoserine dehydrogenase activities and with reduced feedback inhibition relative to the parent ThrA enzyme (designated ThrA*; Ogawa-Miyata,Y., et al., Biosci. Biotechnol. Biochem. 65:1149-1154 (2001); Lee J.-H., et al., J. Bacteriol. 185: 5442-5451 (2003)).

Threonine can be deaminated to α-ketobutyrate (also known as 2-ketobutyrate, 2-oxobutanoate and 2-oxobutyrate) by an enzyme with threonine deaminase activity (e.g., EC 4.3.1.19; also known as threonine ammonia-lyase activity, and was previously classified as EC 4.2.1.16, threonine dehydratase), which catalyzes the conversion of threonine to α-ketobutyrate. In one embodiment, threonine deaminase activity already present in (i.e., endogenous to) the parental microbial cell is sufficient to catalyze the conversion of threonine to α-ketobutyrate. In another embodiment, the recombinant microbial cell is engineered to recombinantly express a polypeptide having threonine deaminase activity, wherein the polypeptide catalyzes the conversion of threonine to α-ketobutyrate. In some embodiments, the polypeptide having threonine deaminase activity is overexpressed in the recombinant microbial cell.

Non-limiting examples of BCFA pathway enzymes and polynucleotides encoding such enzymes for use in part (A.1) of the branched fatty acid pathway are provided in Table 1.

**Table 1.** Non-limiting examples of enzymes and nucleic acid coding sequences for use in Part A.1 of the anteiso-BCFA biosynthetic pathway shown in Figure 3A.

| EC Number | Organism | Gene symbol | UniProtKB (SwissProt) Accession Number, or literature reference | NCBI Protein Accession Number | SEQ ID NO: (pp, na) |
|---|---|---|---|---|---|
| EC 2.7.2.4 | aspartate kinase (aspartokinase) | | | | |
| | *E. coli K-12 MG1655* | *thrA* | P00561 | NP_414543 | 116,117 |
| | *E. coli* (mutant) | *thrA** | Ogawa-Miyata et al, 2001; Lee et al, 2003 | | 118,119 |
| | *B. subtilis 168* | *dapG* | Q04795 | ZP_03591402 | 120,121 |
| | *P. putida F1* | *Pput1442* | A5W0E0 | YP_001266784 | 122,123 |
| | *S. cerevisiae* | *hom3* | | NP_010972 | 124,125 |
| EC 1.1.1.3 | homoserine dehydrogenase | | | | |
| | *E. coli K12 MG1655* | *thrA* | P00561 | NP_414543 | 116,117 |
| | *E. coli* (mutant) | *thrA** | Ogawa-Miyata et al, 2001; Lee et al, 2003 | | 118,119 |
| | *B. subtilis 168* | *hom* | P19582 | NP_391106 | 126,127 |
| | *P. putida F1* | *Pput_4251* | A5W8B5 | YP_001269559 | 128,129 |
| | *S. cerevisiae* | *hom6* | P31116 | NP_012673 | 130,131 |
| EC 2.7.1.39 | homoserine kinase | | | | |
| | *E. coli K12 MG1655* | *thrB* | P00547 | NP_414544 | 132, 133 |

(continued)

| | | | | | |
|---|---|---|---|---|---|
| EC 2.7.1.39 | homoserine kinase | | | | |
| | *B. subtilis 168* | *thrB* | P04948 | NP_391104 | 134, 135 |
| | *P. putida F1* | *Pput_ 0138* | A5VWQ3 | YP_001265497 | 136,137 |
| | *S. cerevisiae* | *thr1* | P17423 | NP_011890 | 138,139 |
| EC 4.2.3.1 | threonine synthase | | | | |
| | *E. coli K12 MG1655* | *thrC* | P00934 | NP_414545 | 140,141 |
| EC Number | Organism | Gene symbol | UniProtKB (SwissProt) Accession Number, or literature reference | NCBI Protein Accession Number | SEQ ID NO: (pp, na) |
| | *B. subtilis 168* | *thrC* | P04990 | NP_391105 | 142,143 |
| | *C. glutamicum ATCC 13032* | *thrC* | P23669 | YP_226461 | 144,145 |
| EC 4.3.1.19 | threonine deaminase (threonine ammonia-lyase; previously termed threonine dehydratase) | | | | |
| | *E. coli K12 MG1655* | *tdcB* | P0AGF6 | NP_417587 | 146,147 |
| | *E. coli K12 MG1655* | *ilvA* | P04968 | NP_418220 | 148,149 |
| | *B. subtilis 168* | *ilvA* | P37946 | NP_390060 | 150,151 |
| | *C. glutamicum ATCC 13032* | *ilva* | Q04513 | YP_226365 | 152,153 |
| | *C. glutamicum ATCC 13032* | *tdcB* | Q8NRR7 | YP_225271 | 154,155 |

Additional polypeptides can be identified, for example, by searching a relevant database (such as the KEGG database (University of Tokyo), the PROTEIN or the GENE databases (Entrez databases; NCBI), the UNIPROTKB or ENZYME databases (ExPASy; Swiss Institute of Bioinformatics), and the BRENDA database (The Comprehensive Enzyme Information System; Technical University of Braunschweig)), all which are available on the World Wide Web, for polypeptides categorized by the above noted EC numbers. For example, additional aspartokinase polypeptides can be identified by searching for polypeptides categorized under EC 2.7.2.4; additional homoserine dehydrogenase polypeptides can be identified by searching for polypeptides categorized under EC 1.1.1.3; additional homoserine kinase polypeptides can be identified by searching for polypeptides categorized under EC 2.7.1.39; additional threonine synthase polypeptides can be identified by searching for polypeptides categorized under EC 4.2.3.1; and additional threonine deaminase polypeptides can be identified by searching for polypeptides categorized under EC 4.3.1.19.

In some embodiments, a polynucleotide encoding a parent fatty acid pathway polypeptide (such as a polypeptide described in Table 1 or identified by EC number or by homology to an exemplary polypeptide) is modified using methods well known in the art to generate a variant polypeptide having an enzymatic activity noted above (e.g., aspartokinase activity, homoserine dehydrogenase activity, homoserine kinase activity, threonine synthase activity, threonine deaminase activity) and an improved property, compared to that of the parent polypeptide, which is more suited to the microbial cell and/or to the pathway being engineered; such as, for example, increased catalytic activity or improved stability under conditions in which the recombinant microbial cell is cultured; reduced inhibition (e.g., reduced feedback inhibition) by a cellular metabolite or by a culture media component, and the like.

***Pathway Part A.2 (Citramalate Intermediate)***

The second pathway leading to the common 2-ketobutyrate intermediate, as represented by part (A.2) of Figure 3A, involves the production of the pathway intermediate citramalate (which is also known as 2-methylmalate) via an enzyme with citramalate synthase activity, and the conversion of citramalate to 2-ketobutyrate by the action of enzymes with isopropylmalate isomerase and alcohol dehydrogenase activities.

Citramalate synthase activity (e.g., EC 2.3.1.182), which catalyzes the reaction of acetyl-CoA and pyruvate to form (R)-citramalate, can be supplied by expression of a *cimA* gene from a bacterium such as *Methanococcus jannaschi or Leptospira interrogans* (Howell, D.M. et al., J. Bacteriol. 181(1):331-3 (1999); Xu, H., et al., J. Bacteriol. 186:5400-5409(2004)) which encodes a CimA polypeptide such as CimA from *M. jannaschii* (SEQ ID NO: 156) or *L. interrogans* (SEQ ID NO:160). Alternatively, a modified *cimA* nucleic acid sequence encoding a CimA variant with improved catalytic activity or stability in the recombinant microbial cell and/or reduced feedback inhibition can be used, such as, for example, a CimA variant described by Atsumi S. and Liao J.C. (Appl. Environ. Microbiol. 74(24): 7802-7808 (2008)), preferably the CimA3.7 variant (SEQ ID NO:158) encoded by the *cimA3.7* gene (SEQ ID NO:159). Alternatively, a *Leptospira interrogans* CimA variant (SEQ ID NO:162) can be used. Isopropylmalate isomerase activity (EC 4.2.1.33; also termed isopropylmalate dehydratase), which catalyzes the conversion of (R)-citramalate first to citraconate and then to beta-methyl-D-malate, can be provided, for example, by expression of a heterodimeric protein encoded by *E. coli* or *B. subtilis leuCD* genes. Alcohol dehydrogenase activity (EC 1.1.1.85; beta-isopropyl malate dehydrogenase), which catalyzes the conversion of beta-methyl-D-malate to 2-ketobutyrate (i.e., alpha-keto butyrate) can be provided, for example, by expression of an *E. coli* or *B. subtilis leuB* gene or a yeast *leu2* gene. Non-limiting examples of fatty acid pathway enzymes and polynucleotides encoding such enzymes for use in engineering part (A.2) of the branched fatty acid pathway are provided in Table 2.

**Table 2.** Non-limiting examples of enzymes and nucleic acid coding sequences for use in Part (A.2) of the anteiso-BCFA biosynthetic pathway shown in Figure 3A.

| EC number | Organism | Gene symbol | UniProtKB (Swiss-Prot) Protein Accession Number, or literature reference | NCBI Protein Accession Number | SEQ ID NO: (pp, na) |
|---|---|---|---|---|---|
| EC 2.3.1.182 | (R)-citramalate synthase | | | | |
| | *M. jannaschii* | *cimA* | Q58787 | NP_248395 | 156,157 |
| | *M.jannaschii* (mutant) | *cimA 3.7* | Atsumi and Liao (2008) | | 158,159 |
| | *Leptospira interrogans* | *cimA* | Q8F3Q1 | AAN49549 | 160,161 |
| | *Leptospira interrogans (mutant)* | *cimA** | (this disclosure) | | 162,163 |
| EC 4.2.1.33 | isopropylmalate isomerase (3-isopropylmalate dehydratase) | | | | |
| | *E. coli K12 MG1655* | *leuCD* | P0A6A6 (C, Lg subunit);<br>P30126 (D, Sm subunit) | (C) NP_414614<br>(D) NP_414613 | 164,165<br>166,167 |
| | *B. subtilis 168* | *leuCD* | P80858 (C, Lg subunit);<br>P94568 (D, Sm subunit) | (C) NP_390704<br>(D) NP_390703 | 168,169<br>170,171 |

(continued)

| | | | | | |
|---|---|---|---|---|---|
| EC 1.1.1.85 | beta-isopropylmalate dehydrogenase (3-isopropylmalate dehydrogenase) | | | | |
| | *E. coli K12 MG1655* | *leuB* | P30125 | NP_414615 | 172,173 |
| | *B. subtilis* | *leuB* | P05645 | NP_390705.2 | 174,175 |
| | *S. cerevisiae* | *leu2* | P04173 | NP_009911.2 | 176,177 |

Additional polypeptides can be identified, for example, by searching a relevant database (such as the KEGG database (University of Tokyo), the PROTEIN or the GENE databases (Entrez databases; NCBI), the UNIPROTKB or ENZYME databases (ExPASy; Swiss Institute of Bioinformatics), and the BRENDA database (The Comprehensive Enzyme Information System; Technical University of Braunschweig)), all which are available on the World Wide Web, for polypeptides categorized by the above noted EC numbers. For example, additional (R)-citramalate synthase polypeptides can be identified by searching for polypeptides categorized under EC 2.3.1.182; additional isopropyl malate isomerase polypeptides can be identified by searching for polypeptides categorized under EC 4.2.1.33; and additional beta-isopropyl malate dehydrogenase polypeptides can be identified by searching for polypeptides categorized under EC 1.1.1.85.

In some embodiments, a polynucleotide encoding a parent fatty acid pathway polypeptide (such as a polypeptide described in Table 2 or identified by EC number or by homology to an exemplary polypeptide) is modified using methods well known in the art to generate a variant polypeptide having an enzymatic activity noted above (e.g., (R)-citramalate synthase activity, isopropyl malate isomerase activity, beta-isopropyl malate dehydrogenase activity) and an improved property, compared to that of the parent polypeptide, which is more suited to the microbial cell and/or to the pathway being engineered; such as, for example, increased catalytic activity or improved stability under conditions in which the recombinant microbial cell is cultured; reduced inhibition (e.g., reduced feedback inhibition) by a cellular metabolite or by a culture media component, and the like.

**Pathway Part B: 2-Ketobutyrate to 2-Keto-3-Methylvalerate**

The α-ketobutyrate produced by the first and/or the second pathway can then be converted to the branched α-keto acid, 2-keto-3-methylvalerate, by the action of enzymes with acetohydroxyacid synthase activity (such as, an AHAS complex), acetohydroxyacid isomeroreductase activity, and dihydroxy acid dehydratase activity, as represented by part (B) of Figure 3B.

Condensation of α-ketobutyrate and pyruvate with concomitant decarboxylation to form the 2-aceto-2-hydroxybutyrate (α-aceto-α-hydroxybutyrate) intermediate can be accomplished by the action of an acetohydroxyacid synthase (AHAS; e.g., EC 2.2.1.6). AHAS (also called acetolactate synthase) is a multisubunit enzyme comprising a large subunit and a small subunit encoded by two genes. There are several AHAS isozymes present in bacteria, fungi and plants. *E. coli* and various other bacteria contain AHAS isozymes designated AHAS I (e.g., encoded by *ilvBN* genes), AHAS II (e.g., encoded by *ilvGM* genes) and AHAS III (e.g., encoded by *ilvIH* genes). In one embodiment, the acetohydroxyacid synthase activity present in the parental microbial cell is sufficient to catalyze the reaction of 2-ketobutyrate and pyruvate to 2-aceto-2-hydroxybutyrate. In another embodiment, the recombinant microbial cell is engineered to recombinantly express AHAS polypeptides having acetohydroxyacid synthase activity, wherein the AHAS polypeptides catalyze the reaction of 2-ketobutyrate and pyruvate to 2-aceto-2-hydroxybutyrate. In some embodiments, polypeptides having acetohydroxyacid synthase activity are overexpressed in the recombinant microbial cell. If the microbial cell being engineered is an *E. coli* K-12 strain, and, if *E. coli* AHAS II activity is desired, an *ilvG* gene (or an *ilvGM* gene cluster) must be introduced from a different strain of *E. coli,* or, the endogenous *ilvG* gene must be repaired by recombinant methods, since the *ilvG* gene endogenous to *E. coli* K-12 is inactive. Alternatively, AHAS I and AHAS III activities present in the parental *E. coli* K-12 cell (e.g., encoded by endogenous *ilvBN* and/or *ilvIH* genes) could be utilized.

Next, conversion of the 2-aceto-2-hydroxybutyrate intermediate to 2,3-dihydroxy-3-methylvalerate (i.e., α,β-dihydroxy-β-methylvalerate) and then to 2-keto-3-methylvalerate (i.e., α-keto-β-methylvalerate or 3-methyl-2-oxopentanoate), can be accomplished by expressing genes encoding enzymes with acetohydroxyacid isomeroreductase activity (e.g., EC 1.1.1.86, encoded by *ilvC* genes in bacteria and by *ilv5* genes in yeast and in plants), which catalyzes the conversion of 2-aceto-2-hydroxybutyrate to 2,3-dihydroxy-3-methylvalerate; and dihydroxy acid dehydratase activity (e.g., EC 4.2.1.9, encoded by *ilvD* in bacteria and by *ilv3* in yeast and in plants), which catalyzes the conversion of 2,3-dihydroxy-3-methylvalerate to 2-keto-3-methylvalerate. In one embodiment, genes endogenous to the parental microbial cell (that is, non-recombinant native host genes), and native enzymes encoded by those endogenous genes, could be utilized for the various steps of part (B) of the pathway. Non-limiting examples of fatty acid pathway enzymes and polynucleotides encoding such enzymes suitable for use in part (B) of the branched fatty acid pathway are provided in Table 3.

**Table 3.** Non-limiting examples of enzymes and coding sequences for use in Part (B) of the anteiso-BCFA biosynthetic pathway shown in Figure 3B.

| EC number | Organism | Gene symbol | UniProtKB (Swiss-Prot) Protein Accession Number, or literature reference | NCBI Protein Accession Number | SEQ ID NO: (pp, na) |
|---|---|---|---|---|---|
| EC 2.2.1.6 | acetohydroxyacid synthase (acetolactate synthase) | | | | |
| | *E. coli K-12 MG1655* | *ilvBN* | (AHAS I)<br>(IlvB) P08142<br>(IlvN) P0ADF8 | (B) NP_418127<br>(N) NP_418126 | 178,179<br>180,181 |
| | *E. coli B* | *ilvGM* | (AHAS II)<br>(IlvG) C6UI83<br>(IlvM) C6UI84 | (G) YP_ 003046821<br>(M) YP_ 003046822 | 182, 183<br>184, 185 |
| | *E. coli K-12 MG1655* | *ilvIH* | (AHAS III)<br>(IlvI) P00893<br>(IlvH) P00894 | (I) YP_025294.2<br>(H) NP_414620 | 186, 187<br>188, 189 |
| | *L. monocytogenes 08-5578* | *ilvBN* | (IlvB) D2NVG7<br>(IlvN) D2NVG8 | (B) YP_ 003414294<br>(N) YP_ 003414295 | 190, 191<br>192, 193 |
| EC 1.1.1.86 | acetohydroxyacid isomeroreductase (ketol-acid reductoisomerase) | | | | |
| | *E. coli K-12 MG1655* | *ilvC* | P05793 | NP _418222 | 194, 195 |
| | *B. subtilis 168* | *ilvC* | P37253 | NP_390707 | 196, 197 |
| EC 4.2.1.9 | dihydroxyacid dehydratase | | | | |
| | *E. coli K-12 MG1655* | *ilvD* | P05791 | YP_026248 | 198,199 |
| | *B. subtilis* 168 | *ilvD* | P51785 | NP_390070.2 | 200, 201 |

Additional polypeptides can be identified, for example, by searching a relevant database (such as the KEGG database (University of Tokyo), the PROTEIN or the GENE databases (Entrez databases; NCBI), the UNIPROTKB or ENZYME databases (ExPASy; Swiss Institute of Bioinformatics), and the BRENDA database (The Comprehensive Enzyme Information System; Technical University of Braunschweig)), all which are available on the World Wide Web, for polypeptides categorized by the above noted EC numbers. For example, additional acetohydroxyacid synthase polypeptides can be identified by searching for polypeptides categorized under EC 2.2.1.6; additional acetohydroxyacid isomeroreductase polypeptides can be identified by searching for polypeptides categorized under EC 1.1.1.86; and additional dihydroxyacid dehydratase polypeptides can be identified by searching for polypeptides categorized under EC 4.2.1.9.

In some embodiments, a polynucleotide encoding a parent fatty acid pathway polypeptide (such as a polypeptide described in Table 3 or identified by EC number or by homology to an exemplary polypeptide) is modified using methods well known in the art to generate a variant polypeptide having an enzymatic activity noted above (e.g., acetohydroxyacid synthase activity, acetohydroxyacid isomeroreductase activity, dihydroxyacid dehydratase activity) and an improved property, compared to that of the parent polypeptide, which is more suited to the microbial cell and/or to the pathway being engineered; such as, for example, increased catalytic activity or improved stability under conditions in which the recombinant microbial cell is cultured; reduced inhibition (e.g., reduced feedback inhibition) by a cellular metabolite or by a culture media component, and the like.

**Pathway Part C: Branched Alpha-Keto Acid to Branched Acyl-CoA**

The branched acyl-CoA molecule is generated from the branched α-keto acid, as represented by part (C) of Figures 1 and 3B, by the action of a multi-component branched chain alpha-keto acid dehydrogenase (BKD) complex. Polynucleotides encoding components of the BKD complex can be obtained from a microbial cell that normally produces branched chain fatty acids or can metabolize branched amino acids or branched α-ketoacids (including, but not limited to, strains of *Bacillus, Pseudomonas, Streptomyces, Listeria, Staphylococcus, and Streptococcus).*

The BKD complex comprises at least two components: an E1 component having alpha-keto acid dehydrogenase activity (e.g., EC 1.2.4.4), and which, depending on the source, may be a single polypeptide (that is, a monomer), or, a heterodimer denoted Elalpha and E1beta; and a E2 component having lipoamide acyltransferase activity (e.g., EC 2.3.1.168). Both the E1 (or E1alpha/E1beta) and E2 components utilize branched substrates. In some instances, the BDK complex comprises a third component, denoted E3, having dihydrolipoamide dehydrogenase activity (e.g., EC 1.8.1.4); in some instances, an enzyme having dihydrolipoamide dehydrogenase activity and which utilizes branched chain substrates can be obtained from a microbial cell which does not normally produce branched fatty acids (such as *E. coli*), which may be used in place of a BKD E3 component.

To engineer part (C) of the pathway, the branched chain alpha-keto acid dehydrogenase activity (E1 activity, e.g., EC 1.2.4.4) and the lipoamide acyltransferase activity (E2 activity, e.g., EC 2.3.1.168) of the BKD complex can be introduced by expression of polynucleotides encoding BKD E1 (or E1alpha/beta) and E2 component polypeptides from microorganisms that normally produce branched fatty acids or can metabolize branched amino acids or branched α-ketoacids, such as, for example, *Bacillus subtilis, Pseudomonas putida, Listeria monocytogenes, Micrococcus luteus, and Streptococcus mutans.* Dihydrolipoamide dehydrogenase activity (E3 activity, e.g., EC 1.8.1.4) can likewise be introduced by expression of a polynucleotide encoding an E3 component from a microorganism that normally produces branched chain fatty acids; alternatively, a polynucleotide encoding a polypeptide with dihydrolipoyl dehydrogenase activity from a microorganism that normally does not produce branched chain fatty acids, but which nevertheless utilizes branched chain substrates (for example, an *E. coli* dihydrolipoyl dehydrogenase), can be used. If the recombinant microbial cell being engineered is one that normally produces branched chain fatty acids (and, as such, produces an endogenous BKD complex), one or more endogenous BKD complex components can be overexpressed.

Non-limiting examples of fatty acid pathway enzymes and polynucleotides encoding such enzymes for use in engineering part (C) of the branched fatty acid pathway are provided in Table 4.

**Table 4.** Non-limiting examples of BKD complex polypeptides and coding sequences for use in Part C of the BCFA biosynthetic pathways shown in Figures 1 and 3B.

| EC number | Organism | Gene symbol | UniProtKB (Swiss-Prot) Protein Accession Number, or literature reference | NCBI Protein Accession Number | SEQ ID NO: pp,na |
|---|---|---|---|---|---|
| EC 1.2.4.4 | branched chain alpha-keto acid dehydrogenase (branched chain alpha-keto acid decarboxylase; 3-methyl-2-oxobutanoate dehydrogenase (2-methylpropanoyl-transferring); 2-oxoisovalerate dehydrogenase; BKD E1 complex component) | | | | |
| | *B. subtilis 168* | *bkdAA*<br>*bkdAB* | P37940 (E1a)<br>P37941 (E1b) | NP_390285 (E1a)<br>NP_390284 (E1b) | 1,2<br>22,23 |
| | *Streptomyces avermitilis MA-4860* | *bkdA*<br>*bkdB* | Q53592<br>Q82F97 | NP_825539<br>NP_825540 | 3,4<br>24,25 |
| | *Pseudomonas putida F1* | *Pput-1453*<br>*Pput_1452* | A5W0F1<br>A5W0F0 | YP_001266795<br>YP_001266794 | 5,6<br>26,27 |
| | *Listeria monocytogenes 08-5578* | *LM5578_1512*<br>*LM5578_1513* | D2P1Z6<br>D2P1Z7 | YP_003413622<br>YP_003413623 | 7,8<br>28,29 |
| | *Micrococcus luteus NCTC* | *Mlut_06800* | C5C9R0 | YP_002956766 | 9,10 |

(continued)

| EC number | Organism | Gene symbol | UniProtKB (Swiss-Prot) Protein Accession Number, or literature reference | NCBI Protein Accession Number | SEQ ID NO: pp,na |
|---|---|---|---|---|---|
| EC 1.2.4.4 | branched chain alpha-keto acid dehydrogenase (branched chain alpha-keto acid decarboxylase; 3-methyl-2-oxobutanoate dehydrogenase (2-methylpropanoyl-transferring); 2-oxoisovalerate dehydrogenase; BKD E1 complex component) | | | | |
| | *2655* | *Mlut_06810* | C5C9R1 | YP_002956767 | 30,31 |
| | *Staphylococcus aureus A8819* | | D4UFQ9<br>D4UFQ8 | ZP_06816445<br>ZP_06816444 | 11,12<br>32,33 |
| | *Streptococcus mutans UA159* | *adhA*<br>*adhB* | Q8DWD7<br>Q8DWD6 | NP_720600<br>NP_720601 | 13,14<br>34,35 |
| EC number | Organism | Gene symbol | UniProtKB (Swiss-Prot) Protein Accession Number, or literature reference | NCBI Protein Accession Number | SEQID NO: pp,na |
| EC 2.3.1.168 | lipoamide acyltransferase (dihydrolipoyl transacylase; dihydrolipoyllysine-residue (2-methylpropanoyl) transferase; BKD E2 complex component) | | | | |
| | *B. subtilis 168* | *bkdB* | P37942 | NP_390283 | 43,44 |
| | *Streptomyces avermitilis MA-4860* | *bkdC* | Q82F96 | NP_825541 | 45,46 |
| | *Pseudomonas putida F1* | *Pput_1451* | A5W0E9 | YP_001266793 | 47,48 |
| | *Listeria monocytogenes 08-5578* | *LM5578_1514* | D2P1Z8 | YP-003413624 | 49,50 |
| | *Micrococcus luteus NCTC 2655* | *Mlut_06810* | C5C9R1 | YP_002956767 | 51,52 |
| | *Staphylococcus aureus JH1* | *SaurJH1_1607* | A6U1Y7 | YP_001316742 | 53,54 |
| | *Streptococcus mutans UA159* | *adhC* | Q8DWD5 | NP_720602 | 55,56 |
| EC 1.8.1.4 | dihydrolipoamide dehydrogenase (dihydrolipoyl dehydrogenase; BKD E3 complex component) | | | | |
| | *Bacillus subtillis 168* | *IpdV* | P54533 | NP_390286.2 | 63,64 |
| | *Streptomyces avermitilis MA-4860* | *IpdA1* | Q82AN3 | NP_827200.2 | 65,66 |
| | *Pseudomonas putida F1* | *Pput_1450* | A5W0E8 | YP_001266792 | 67,68 |
| | *Listeria monocytogenes 08-5578* | *pdhD* | D2P0X6 | YP _003413252 | 69,70 |
| | *Micrococcus luteus NCTC 2655* | *Mlut_05640* | C5C9F0 | YP_002956656 | 71,72 |
| | *Staphylococcus aureus ED98* | *IpdA* | DOK5A1 | YP_003282417 | 73,74 |

(continued)

| | | | | | |
|---|---|---|---|---|---|
| EC 1.8.1.4 | dihydrolipoamide dehydrogenase (dihydrolipoyl dehydrogenase; BKD E3 complex component) | | | | |
| | *Streptococcus mutans UA159* | *adhD* | Q8DWD4 | NP_720603 | 75,76 |
| | *E. coli* | *lpdA* | P0A9P0 | NP_414658 | 77,78 |

Additional polypeptides can be identified, for example, by searching a relevant database (such as the KEGG database (University of Tokyo), the PROTEIN or the GENE databases (Entrez databases; NCBI), the UNIPROTKB or ENZYME databases (ExPASy; Swiss Institute of Bioinformatics), and the BRENDA database (The Comprehensive Enzyme Information System; Technical University of Braunschweig)), all which are available on the World Wide Web, for polypeptides categorized by the above noted EC numbers. For example, additional branched chain alpha-keto acid dehydrogenase polypeptides can be identified by searching for polypeptides categorized under EC 1.2.4.4; additional lipoamide acyltransferase polypeptides can be identified by searching for polypeptides categorized under EC 2.3.1.168; and additional dihydrolipoyl dehydrogenase polypeptides can be identified by searching for polypeptides categorized under EC 1.8.1.4.

BKD complex component polypeptides (such as, branched chain alpha-keto acid dehydrogenase polypeptides, lipoamide acyltransferase polypeptides, and dihydrolipoyl dehydrogenase polypeptides) can also be identified by searching a sequence pattern database, such as the Prosite database (ExPASy Proteomics Server, Swiss Institute of Bioinformatics) for a polypeptide comprising one or more of the sequence motifs listed below. This is readily accomplished, for example, by using the ScanProsite tool which is available on the World Wide Web site of the ExPASy Proteomics Server.

In one embodiment, a branched chain alpha-keto acid dehydrogenase (BKD E1-alpha subunit) polypeptide comprises one or more sequence motif selected from:

[S,Q]-x(2)-G-[Q,E]-E-A-x(3)-[G,A]-x-[G,A]-x-[V,A]-[L,T] (SEQ ID NO:15)
D-x(2)-[L,F]-P-x-Y-R (SEQ ID NO:16)
[S,T]-Q-x(2)-[H,Q]-A-[T,V]-G-x-A-[A,G] (SEQ ID NO:17)
[K,G]-x-[T,D]-x(2)-[A,V]-x-[A,V]-x(2)-G-[E,D]-G-x(4)-[G,S]-D-[F,V] (SEQ ID NO:18)
F-[A,S]-[H,A]-V-x(2)-[L,A]-P-V-x-[L,F]-x(3)-N-N-x(2)-A-I-S (SEQ ID NO:19)
[K,R]-[G,A]-x-G-[C,Y]-[F,G]-x-[A,P]-[S,G]-x(2)-V-D-G-N-D (SEQ ID NO:20)
[H,R]-A-R-[A,R]-G-x-G-P-x-L-x-E-x(2)-[S,T]-Y-R-x(3)-H-x(3)-D-D-x(3)-Y-R (SEQ ID NO:21)

wherein the amino acid residues in each of the brackets indicate alternative amino acid residues at the particular position, each x indicates any amino acid residue, and each n in "x(n)" indicates the number of x residues in a contiguous stretch of amino acid residues.

In another embodiment, a branched chain alpha-keto acid dehydrogenase (BKD E1-beta subunit) polypeptide comprises one or more sequence motif selected from:

V-x-[V,I]-x-G-[Q,E]-D-V-G-x(2)-G-G-V-F-[R,K]-x-T-x-G-I (SEQ ID NO:36)
[Y,F]-G-[E,K]-x-R-[C,V]-x-D-[A,T]-P-[L,I]-[A,S]-E-[A,S]-[A,G]-I (SEQ ID NO:37)
G-T-[A,E]-x-[R,Y]-G-x-R-P-[I,V]-[A,V]-E-x-Q-F (SEQ ID NO:38)
P-[C,Y]-G-G-[V,I]-x-[A,G]-x(3)-H-S-x-S-x-E-A-x-[F,Y] (SEQ ID NO:39)
[E,D]-D-P-V-x-[F,Y]-x-E-[H,P]-K-R-x-Y (SEQ ID NO:40)
[H,E]-V-[I,V]-D-L-R-[T,S]-x(2)-P-x-D (SEQ ID NO:41)
E-x-C-[L,F]-x-[D,H]-L-[D,E]-A-P-x(2)-R-[L,V]-x-G-x-[H,D]-P (SEQ ID NO:42)

wherein the amino acid residues in each of the brackets indicate alternative amino acid residues at the particular position, each x indicates any amino acid residue, and each n in "x(n)" indicates the number of x residues in a contiguous stretch of amino acid residues.

In another embodiment, a lipoamide acyltransferase (BKD E2 component) polypeptide comprises one or more sequence motif selected from:

P-x-V-[L,R]-x-[R,L]-A-x(3)-G-x-[D,E]-L (SEQ ID NO:57)
[G,P]-[S,T]-G-[A,P]-x-G-x-I (SEQ ID NO:58)

[V,I]-P-[L,V]-x-G-[L,V]-R-x-[A,K]A-x(2)-[L,M]-x(2)-[A,S] (SEQ ID NO:59)
G-[G,S]-T-x-T-x(2)-[N,S]-x-G-x-[F,L]-G (SEQ ID NO:60)
N-x-P-E-x-A-[I,M]-[L,V]-x-V-x(2)-[I,M]-x(3)-P-x-V (SEQ ID NO:61)
L-x-[L,S]-[S,T]-F-[D,L]-H-R-[V,L]-x-D-G (SEQ ID NO:62)

wherein the amino acid residues in each of the brackets indicate alternative amino acid residues at the particular position, each x indicates any amino acid residue, and each n in "x(n)" indicates the number of x residues in a contiguous stretch of amino acid residues.

In another embodiment, a dihydrolipoyl dehydrogenase (BKD E3 component) polypeptide comprises one or more sequence motif selected from:

[I,V]-G-G-[A,T]-[S,C]-[V,L]-x(2)-[G,D]-C-[V,I]-P-[T,S]-K-[A,T]-[M,L]-[I,L] (SEQ ID NO:79)
[L,I]-A-T-G-[G,S]-x-[S,P]-x(2)-L-[A,P]-[D,G]-x(3)-[D,L]-G (SEQ ID NO:80)
[V,I]-x-G-[G,S]-G-x-[I,T]-G-x-E-x-[A,G] (SEQ ID NO:81)
T-x(6)-[A,V]-x-G-D-x(2)-[P,G] (SEQ ID NO:82)
[I,V]-[GA]-x(2)-[F,I]-[T,H]-x-[Y,H]-P-[S,T]-[Q,L] (SEQ ID NO:83)

wherein the amino acid residues in each of the brackets indicate alternative amino acid residues at the particular position, each x indicates any amino acid residue, and each n in "x(n)" indicates the number of x residues in a contiguous stretch of amino acid residues.

In some embodiments, a polynucleotide encoding a parent fatty acid pathway polypeptide (such as a BKD complex polypeptide described in Table 4 or identified by EC number or by motif or by homology to an exemplary polypeptide) is modified using methods well known in the art to generate a variant polypeptide having BKD complex enzymatic activity and an improved property, compared to that of the parent polypeptide, which is more suited to the microbial cell and/or to the pathway being engineered; such as, for example, increased catalytic activity or improved stability under conditions in which the recombinant microbial cell is cultured; reduced inhibition (e.g., reduced feedback inhibition) by a cellular metabolite or by a culture media component, and the like.

BKD complex enzymatic activity can be measured according to various known protocols. For example, Sokatch *et al.* described an assay mixture containing: 100 mM potassium phosphate buffer (pH 7.0), 2 mM NAD+, 0.1 mM coenzyme A, 0.2 mM dithiothreitol, 0.2 mM thiamine pyrophosphate, 1 mM magnesium chloride, and 5 mM L-valine . The reaction can be initiated by adding 4.0 μmoL of the sodium salt of a branched-chain α-keto acid substrate, such as 2-keto-3-methylvalerate (Sokatch et al., J. Bacteriology 148(2):647-652 (1981)).

**Pathway Part D: Branched Acyl-CoA to Branched β-Ketoacyl-ACP**

As noted above, the branched acyl-CoA serves as a primer for subsequent FAS-catalyzed elongation steps. The initiation of this process involves condensation of the branched acyl-CoA with a malonyl-ACP molecule to form a branched β-ketoacyl-ACP intermediate. For example, the anteiso-branched acyl-CoA molecule 2-methylbutyryl-CoA can condense with malonyl-ACP to form an anteiso-branched β-ketoacyl-ACP intermediate 4-methyl-3-oxo-hexanoyl-ACP, while the iso-branched acyl-CoA molecule isobutyryl-CoA can condense with malonyl-ACP to form an iso-branched β-ketoacyl-ACP intermediate 4-methyl-3-oxo-pentanoyl-ACP (Figures 2A and 2B).

This initiation step, as represented by part (D) of Figures 1 and 3, is catalyzed in the recombinant microbial cell by an enzyme having beta-ketoacyl-ACP synthase activity (such as, a Type III beta-ketoacyl-ACP synthase (e.g., EC 2.3.1.180)) that utilizes branched acyl-CoA molecules (for example, anteiso-branched acyl-CoA molecules) as substrates. Such an enzyme is also referred to herein as an enzyme having "branched chain beta-ketoacyl-ACP synthase activity". A polynucleotide sequence encoding an polypeptide having branched chain beta-ketoacyl-ACP synthase activity can be obtained from a microbial cell that normally produces branched chain fatty acids (including, but not limited to, strains of *Bacillus, Streptomyces,, Stenotrophomonas, Listeria, Staphylococcus,* and *Streptococcus*), and expressed or overexpressed in the recombinant microbial cell.

In some instances, one or more enzymes endogenous to the parental microbial cell might compete for substrate with enzymes of the engineered branched fatty acid biosynthetic pathway in the recombinant microbial cell, or might break down or otherwise divert an intermediate in the biosynthetic pathway; genes encoding such undesired endogenous enzymes can be attenuated to increase branched fatty acid production in the recombinant microbial cell. For example, in *E. coli,* the endogenous β-ketoacyl-ACP synthase III (UniProtKB/Swiss-Prot Protein Accession Number P0A6R0), encoded by the *E. coli fabH* gene, primarily utilizes short straight-chain acyl-CoA molecules such as acetyl-CoA, but does not utilize branched acyl-CoA molecules, and thus competes with enzymes of the branched chain pathway for malonyl-ACP and other substrates and diverts flux away from the BCFA pathway. Deleting or otherwise attenuating the *E. coli fabH* gene thus directs fatty acid biosynthesis in a recombinant *E. coli* comprising a BCFA pathway more towards the production of branched fatty acids. Other endogenous enzymes that may be undesired and which may be attenuated in the recombinant microbial cell include, for example, the *E. coli fadE* gene encoding an acyl-CoA dehydrogenase which metabolizes acyl-CoA intermediates.

Non-limiting examples of fatty acid pathway enzymes and polynucleotides encoding such enzymes for use in engineering part D of the branched fatty acid pathway are provided in Table 5.

**Table 5.** Non-limiting examples of enzymes and coding sequences for use in Part D of the BCFA biosynthetic pathways shown in Figures 1 and 3B.

| EC number | Organism | Gene symbol | UniProtKB (Swiss-Prot) Protein Accession Number, or literature reference | NCBI Protein Accession Number | SEQ ID NO: pp, na |
|---|---|---|---|---|---|
| EC 2.3.1.180 | beta-ketoacyl-ACP synthase III | | | | |
| | *B. subtilis 168* | *fabH1* | O34746 | NP_389015 | 84,85 |
| | *B. subtilis 168* | *fabH2* | O07600 | NP_388898 | 86,87 |
| | *Staphylococcus aureus MW2* | *fabH* | Q8NXE2 | NP_645682 | 88,89 |
| | *Streptomyces avermitilis MA-4680* | *fabH3* | Q82KT2 | NP_823466 | 90,91 |
| | *Streptococcus mutans UA159* | *fabH* | Q8DSN2 | NP_722071 | 92,93 |
| | *Lactococcus lactis subsp. lactis* | *fabH* | Q9CHG0 | NP_266927 | 94,95 |
| | *Streptomyces coelicolor* | *fabH* | Q9K3G9 | CAB99151 | 96,97 |
| | *Listeria monocytogenes* | *fabH* | B8DFA8 | YP_ 002349314 | 98,99 |
| | *L. monocytogenes (mutant)* | *fabH2* | (this disclosure) | | 100,101 |
| | *Bacteroides vulgatus* | *fabH* | A6KXK3 | YP_ 001297789 | 102,103 |
| | *Clostridium acetobutyiicum* | *fabH* | Q97DA2 | NP_350161 | 104,105 |
| | *Flavobacterium johnsoniae* | *fabH2* | A5FM89 | YP_ 001193000 | 106,107 |
| | *Micrococcus luteus* | *fabH* | C5CAR9 | YP_ 002957006 | 108,109 |

Additional beta-ketoacyl-ACP III synthase polypeptides can be identified, for example, by searching a relevant database (such as the KEGG database (University of Tokyo), the PROTEIN or the GENE databases (Entrez databases; NCBI), the UNIPROTKB or ENZYME databases (ExPASy; Swiss Institute of Bioinformatics), and the BRENDA database (The Comprehensive Enzyme Information System; Technical University of Braunschweig)), all which are available on the World Wide Web, for polypeptides categorized under EC 2.3.1.180.

Additional beta-ketoacyl-ACP synthase III polypeptides can also be identified by searching a sequence pattern database, such as the Prosite database (ExPASy Proteomics Server, Swiss Institute of Bioinformatics) for a polypeptide comprising one or more of the sequence motifs listed below. This is readily accomplished, for example, by using the ScanProsite tool which is available on the World Wide Web site of the ExPASy Proteomics Server.

In one embodiment, a beta-ketoacyl-ACP synthase III polypeptide comprises one or more sequence motif selected from:

D-T-[N,S]D-[A,E]-W-I-x(2)-[M,R]-T-G-I-x-[N,E]-R-[R,H] (SEQ ID NO:110)
[S,A]-x-D-x(2)-A-[A,V]-C-[A,S]-G-F-x(3)-[M,L]-x(2)-A (SEQ ID NO:111)
D-P-x-T-[A,I]-[I,V]-x-F-[A,G]-D-G-A-[A,G]-[G,A]-[A,V] (SEQ ID NO:112)
H-Q-A-N-x-R-I-[M,L] (SEQ ID NO:113)
G-N-T-[G,S]-A-A-S-[V,I]-P-x(2)-[I,L]-x(6)-G (SEQ ID NO:114)
[I,V]-x-L-x(2)-F-G-G-G-[L,F]-[T,S]-W-G (SEQ ID NO:115)

wherein the amino acid residues in each of the brackets indicate alternative amino acid residues at the particular position, each x indicates any amino acid residue, and each n in "x(n)" indicates the number of x residues in a contiguous stretch of amino acid residues.

In some embodiments, a polynucleotide encoding a parent fatty acid pathway polypeptide (such as a polypeptide described in Table 5 or identified by EC number or by motif or by homology to an exemplary polypeptide) is modified using methods well known in the art to generate a variant polypeptide having branched chain beta-ketoacyl-ACP III synthase activity, and an improved property, compared to that of the parent polypeptide, which is more suited to the microorganism and/or to the pathway being engineered; such as, for example, increased catalytic activity or improved stability under conditions in which the recombinant microbial cell is cultured, reduced inhibition (e.g., reduced feedback inhibition) by a cellular metabolite or by a culture media component, and the like.

The invention includes an isolated polypeptide comprising a sequence having at least 80% identity to one of SEQ ID NOs:84, 86, 88, 90, 92, 94, 96, 98, 102, 104, 106, and 108, and comprising a substitution at position W310 or at an equivalent position thereto, wherein the polypeptide has beta-ketoacyl-ACP synthase activity. The invention also includes an isolated polynucleotide encoding any one of said polypeptides. In one embodiment, the polypeptide comprises a W310G substitution. In one embodiment, the polypeptide comprises a sequence having at least 80% identity to SEQ ID NO: 98 and comprises the substitution W310G. In another embodiment the polypeptide comprises the sequence SEQ ID NO:100. In some embodiments, the polynucleotide encodes the sequence SEQ ID NO:100, or comprises the sequence SEQ ID NO:101.

Enzymatic activity and specificity for branched substrates of beta ketoacyl-ACP synthases can be determined using known methods. For example, Choi et al. (J. Bacteriology 182(2):365-370 (2000)) described in detail a filtered disc assay suitable for determining β-ketoacyl-ACP synthase ("FabH") activity against acetyl-CoA substrates, which can be modified to use branched-chain acyl-CoA substrates. The assay contains 25 μM ACP, 1 mM β-mercaptoethanol 65 μM malonyl-CoA, 45 μM [1-$^{14}$C]acetyl-CoA (specificity activity about 45.8 Ci/mol), *E.coli* FadD (0.2 μg), and 0.1 M sodium phosphate buffer (pH 7.0) in a final volume of 40 μL. To assay branched-chain β-ketoacyl-ACP synthase activity, [1-$^{14}$C]acetyl-CoA can be substituted with a $^{14}$C labeled branched acyl-CoA. The reaction is initiated by the addition of FabH, and the mixture is incubated at 37°C for 12 minutes. A 35 mL aliquot is then removed and deposited on a Whatman 3 MM filter disc. The discs are then washed with three changes (20 mL/disc for 20 minutes each) of ice-cold trichloroacetic acid. The concentration of the trichloroacetic acid is then reduced from 10 to 5 to 1% in each successive wash. The filters are dried an counted in 3 mL of scintillation cocktail.

Alternatively, FabH activity can be determined using gel electrophoresis to separate and quantitate the products (Choi *et al., supra*). The assay mixture contains 25 μM ACP, 1 mM β-mercaptoethanol, 70 μM [2-$^{14}$C] malonyl-CoA (specific activity, ~ 9 Ci/mol), 45 μM of a CoA-substrate (such as acetyl-CoA; or, to assay branched-chain β-ketoacyl-ACP synthase, isobutyryl-CoA, isovaleryl-CoA, or 2-methylbutyryl-CoA), FadD (0.2 μg), 100 μM NADPH, FabG (0.2 μg) and 0.1 M sodium phosphate buffer (pH 7.0) in a final volume of 40 μL. The reaction can be initiated by the addition of FabH. The mixture is incubated at 37°C for 12 minutes and then placed in an ice slurry, gel loading buffer is then added, and the mixture is loaded onto a conformationally sensitive 13% polyacrylamide gel containing 0.5 to 2.0 M urea. Electrophoresis can be performed at 25°C at 32 mA/gel. The gels are then dried, and the bands quantitated by exposure of the gel to a PhosphoImager screen. Specific activity can be calculated from the slopes of the plot of product formation vs. FabH protein concentration in the assay.

**Pathway Part E: Branched β-Ketoacyl-ACP to Branched Fatty Acyl-ACP**

The branched β-ketoacyy-ACP intermediate generated in part (D) can undergo elongation by successive cycles of condensation with malonyl-ACP / keto-reduction / dehydration / enoyl-reduction, catalyzed by a fatty acid synthase (FAS) complex, such as, for example, a type II fatty acid synthase complex, thereby adding 2-carbon units to the lengthening fatty acid chain of the resulting branched acyl-ACP, as represented by part (E) of Figure 1. In one embodiment, an anteiso-branched β-ketoacyy-ACP intermediate produces an anteiso-branched acyl-ACP intermediate. In another embodiment, an iso-branched β-ketoacyl-ACP intermediate produces an iso-branched acyl-ACP intermediate. In one embodiment, a FAS enzyme complex (such as, for example, a Type II FAS complex) endogenous to the microbial cell is used to catalyze cycles of condensation with malonyl-ACP / keto-reduction / dehydration / enoyl-reduction to produce the branched acyl-ACP intermediate.

## Branched Fatty Acid Derivatives

Branched fatty acid derivatives (including branched fatty acids, branched fatty esters, branched fatty aldehydes, branched fatty alcohols, branched hydrocarbons, and branched ketones, in iso-branched or anteiso-branched form) can be produced by a recombinant microbial cell of the invention. The branched acyl-ACP intermediate is converted to a fatty acid derivative in a reaction catalyzed by an enzyme having fatty acid derivative activity (i.e., a fatty acid derivative enzyme). A fatty acid derivative enzyme can, for example, convert a branched acyl-ACP to an initial fatty acid derivative, or, can convert the initial fatty acid derivative to a second fatty acid derivative. In some instances, the initial fatty acid derivative is converted to a second fatty acid derivative by an enzyme having a different fatty acid derivative activity. In some instances, the second fatty acid derivative is further converted to a third fatty acid derivative by another fatty acid derivative enzyme, and so on.

Accordingly, in some embodiments, the recombinant microbial cell further comprises one or more polynucleotides, each polynucleotide encoding a polypeptide having a fatty acid derivative enzyme activity, wherein the recombinant microbial cell produces a branched fatty acid derivative when cultured in the presence of a carbon source under conditions effective to express the polynucleotides.

In various embodiments, the fatty acid derivative activity comprises thioesterase activity, wherein the recombinant microbial cell produces branched fatty acids; ester synthase activity, wherein the recombinant microbial cell produces branched fatty esters; fatty aldehyde biosynthesis activity, wherein the recombinant microbial cell produces branched fatty aldehydes; fatty alcohol biosynthesis activity, wherein the recombinant microbial cell produces branched fatty alcohols; ketone biosynthesis activity, wherein the recombinant microbial cell produces branched ketones; or hydrocarbon biosynthesis activity, wherein the recombinant microbial cell produces branched hydrocarbons. In some embodiments, the recombinant microbial cell comprises polynucleotides encoding two or more polypeptides, each polypeptide having fatty acid derivative enzyme activity.

In more particular embodiments, the recombinant microbial cell expresses or overexpresses one or more polypeptides having fatty acid derivative enzyme activity as described hereinabove, wherein the recombinant microbial cell produces a composition comprising branched fatty acids, branched fatty esters, branched wax esters, branched fatty aldehydes, branched fatty alcohols, branched alkanes, branched alkanes branched internal olefins, branched terminal olefins, or branched ketones.

The following are further examples of fatty acid derivative enzymes, and fatty acid derivatives produced by reactions catalyzed by such enzymes, in accordance with various embodiments of the invention.

### *Branched Fatty Acid*

In one embodiment, the recombinant microbial cell comprises a polynucleotide encoding a thioesterase, and the branched fatty acyl-ACP intermediate produced by the recombinant microbial cell is hydrolyzed by the thioesterase (e.g., 3.1.1.5, EC 3.1.2.-; such as, for example, EC 3.1.2.14) resulting in production of a branched fatty acid. In some embodiments, a composition comprising branched fatty acids (also referred to herein as a "branched fatty acid composition") is produced by culturing the recombinant cell in the presence of a carbon source under conditions effective to express the polynucleotides. In some embodiments, the composition is recovered from the cell culture. In some embodiments, the recombinant microbial cell comprises a polynucleotide encoding a polypeptide having thioesterase activity, and one or more additional polynucleotides encoding polypeptides having other fatty acid derivative enzyme activities. In some such instances, the branched fatty acid produced by the action of the thioesterase is converted by one or more enzymes having different fatty acid derivative enzyme activities to another branched fatty acid derivative, such as, for example, a branched fatty ester, a branched fatty aldehyde, a branched fatty alcohol, or a branched hydrocarbon.

In one embodiment, an anteiso-branched fatty acyl-ACP intermediate reacts with a thioesterase to form an anteiso-branched fatty acid. The anteiso-branched fatty acid can be recovered from the cell culture, or can be further converted to another anteiso-branched fatty acid derivative, such as an anteiso-branched fatty ester, an anteiso-branched fatty aldehyde, an anteiso-branched fatty alcohol, or an anteiso-branched hydrocarbon.

The chain length of a fatty acid, or a fatty acid derivative made therefrom, can be selected for by modifying the expression of certain thioesterases. Thioesterase influences the chain length of fatty acids produced as well as that of the derivatives made therefrom. Hence, the recombinant microbial cell can be engineered to express, overexpress, have attenuated expression, or not to express one or more selected thioesterases to increase the production of a preferred fatty acid or fatty acid derivative substrate. For example, $C_{10}$ fatty acids can be produced by expressing a thioesterase that has a preference for producing $C_{10}$ fatty acids and attenuating thioesterases that have a preference for producing fatty acids other than $C_{10}$ fatty acids (*e.g.*, a thioesterase which prefers to produce $C_{14}$ fatty acids). This would result in a relatively homogeneous population of fatty acids that have a carbon chain length of 10. In other instances, $C_{14}$ fatty acids can be produced by attenuating endogenous thioesterases that produce non-$C_{14}$ fatty acids and expressing thioesterases that use $C_{14}$-ACP. In some situations, $C_{12}$ fatty acids can be produced by expressing thioesterases that

use $C_{12}$-ACP and attenuating thioesterases that produce non-$C_{12}$ fatty acids. Fatty acid overproduction can be verified using methods known in the art, for example, by use of radioactive precursors, HPLC, or GC-MS subsequent to cell lysis.

Additional non-limiting examples of thioesterases and polynucleotides encoding them for use in the branched fatty acid pathway are provided in Table 6 and in PCT Publication No. WO 2010/075483 incorporated by reference herein.

**Table 6.** Non-limiting examples of thioesterases and coding sequences thereof for use in the BCFA pathway shown in Figure 1.

| EC number | Organism | Gene symbol | UniProtKB (Swiss-Prot) Protein Accession Number, or literature reference | NCBI Protein Accession Number | SEQ ID NO: pp, na |
|---|---|---|---|---|---|
| EC 3.1.2.-, | Thioesterase | | | | |
| | *E. coli K-12 MG1655* | *tesA* | P0ADA1 | AAC73596 | 202, 203 |
| | *E. coli* (without leader sequence) | *'tesA* | Cho et al, J. Biol. Chem., 270: 4216-4219 (1995) | | 204, 205 |
| | *E. coli K-12 MG1655* | *tesB* | P0AGG2 | AAC73555 | 206, 207 |
| | *Arabidopsis thaliana* | *fatA* | Q42561 | NP_189147 | 208, 209 |
| | *Arabidopsis thaliana* | *fatB* | Q9SJE2 | NP_172327 | 210, 211 |
| | *Umbellularia california* | *fatB* | Q41635 | AAA34215 | 212, 213 |
| | *Cuphea hookeriana* | *fatA1* | Q9ZTF7 | AAC72883 | 214, 215 |
| | *Cuphea hookeriana* | *fatB2* | Q39514 | AAC49269 | 216, 217 |
| | *Cuphea hookeriana* | *fatB3* | Q9ZTF9 | AAC72881 | 218, 219 |
| | *Cinnamonum camphorum* | *fatB* | Q39473 | AAC49151 | 220, 221 |
| | *Brassica juncea* | *fatA* | Q94IN9 | CAC39106 | 222, 223 |
| | *Helianthus annus* | *fatA1* | Q6K1M5 | AAL79361 | 224,225 |

***Branched Fatty Ester***

In one embodiment, the recombinant microbial cell produces a branched fatty ester (e.g., an anteiso-branched fatty ester or an iso-branched fatty ester), such as, for example, a branched fatty acid methyl ester or a branched fatty acid ethyl ester or a branched wax ester. In some embodiments, a branched fatty acid produced by the recombinant microbial cell is converted into the branched fatty ester.

In some embodiments, the recombinant microbial cell comprises a polynucleotide encoding a polypeptide (i.e., an enzyme) having ester synthase activity (also referred to herein as an "ester synthase polypeptide" or an "ester synthase enzyme"), and the branched fatty ester is produced by a reaction catalyzed by the ester synthase polypeptide expressed or overexpressed in the recombinant microbial cell. In some embodiments, a composition comprising branched fatty esters (also referred to herein as a "branched fatty ester composition"), produced by culturing the recombinant cell in the presence of a carbon source under conditions effective to express the polynucleotides, is recovered from the cell culture. In some embodiments, the recombinant cell produces a branched fatty ester composition comprising anteiso-branched fatty esters.

Ester synthase polypeptides include, for example, an ester synthase polypeptide classified as EC 2.3.1.75, or any other polypeptide which catalyzes the conversion of an acyl-thioester to a fatty ester, including, without limitation, a wax-ester synthase, an acyl-CoA:alcohol transacylase, an acyltransferase, or a fatty acyl-CoA:fatty alcohol acyltransferase. For example, the polynucleotide may encode wax/dgat, a bifunctonal ester synthase/acyl-CoA:diacylglycerol acyltransferase from *Simmondsia chinensis, Acinetobacter sp. Strain* ADP1, *Alcanivorax borkumensis, Pseudomonas aeruginosa, Fundibacter jadensis, Arabidopsis thaliana,* or *Alkaligenes eutrophus*. In a particular embodiment, the ester synthase polypeptide is an *Acinetobacter sp*. diacylglycerol O-acyltransferase (*wax-dgaT*; UniProtKB Q8GGG1, GenBank AAO17391) or *Simmondsia chinensis* wax synthase (UniProtKB Q9XGY6 , GenBank AAD38041). In a particular embodiment, the polynucleotide encoding the ester synthase polypeptide is overexpressed in the recombinant microbial cell. In some embodiments the recombinant microbial cell further comprises a polynucleotide encoding a thioesterase.

In another embodiment, the recombinant microbial cell produces a branched fatty ester, such as, for example, a branched fatty acid methyl ester or a branched fatty acid ethyl ester, wherein the recombinant microbial cell expresses a polynucleotide encoding an ester synthase / acyltransferase polypeptide classified as 2.3.1.20, such as AtfA1 (an acyltransferase derived from *Alcanivorax borkumensis* SK2, UniProtKB Q0VKV8, GenBank YP_694462) or AtfA2 (another acyltransferase derived from *Alcanivorax borkumensis* SK2, UniProtKB Q0VNJ6, GenBank YP_693524). In a particular embodiment, the polynucleotide encoding the ester synthase polypeptide is overexpressed in the recombinant microbial cell. In some embodiments the recombinant microbial cell further comprises a polynucleotide encoding a thioesterase.

In another embodiment, the recombinant microbial cell produces a branched fatty ester, such as, for example, a branched fatty acid methyl ester or a branched fatty acid ethyl ester, wherein the recombinant microbial cell expresses a polynucleotide encoding a ester synthase polypeptide, such as ES9 (a wax ester synthase from *Marinobacter hydrocarbonoclasticus* DSM 8798, UniProtKB A3RE51, GenBank ABO21021, encoded by the *ws2* gene), or ES376 (another wax ester synthase derived from *Marinobacter hydrocarbonoclasticus* DSM 8798, UniProtKB A3RE50, GenBank ABO21020, encoded by the *ws1* gene). In a particular embodiment, the polynucleotide encoding the ester synthase polypeptide is overexpressed in the recombinant microbial cell. In some embodiments the recombinant microbial cell further comprises a polynucleotide encoding a thioesterase.

Additional non-limiting examples of ester synthase polypeptides and polynucleotides encoding them suitable for use in these embodiments include those described in PCT Publication Nos. WO 2007/136762 and WO2008/119082 which are incorporated by reference herein.

***Branched Fatty Aldehyde***

In one embodiment, the recombinant microbial cell produces a branched fatty aldehyde. In some embodiments, a branched fatty acid produced by the recombinant microbial cell is converted into the branched fatty aldehyde. In some embodiments, the branched fatty aldehyde produced by the recombinant microbial cell is then converted into a branched fatty alcohol or a branched hydrocarbon.

In some embodiments, the recombinant microbial cell comprises a polynucleotide encoding a polypeptide (i.e., an enzyme) having fatty aldehyde biosynthesis activity (also referred to herein as a "fatty aldehyde biosynthesis polypeptide" or a "fatty aldehyde biosynthesis enzyme"), and the branched fatty aldehyde is produced by a reaction catalyzed by the fatty aldehyde biosynthesis polypeptide expressed or overexpressed in the recombinant microbial cell. In some embodiments, a composition comprising branched fatty aldehydes (also referred to herein as a "branched fatty aldehyde composition"), produced by culturing the recombinant cell in the presence of a carbon source under conditions effective to express the polynucleotides, is recovered from the cell culture. In some embodiments, the recombinant cell produces a branched fatty aldehyde composition comprising anteiso-branched fatty aldehydes.

In some embodiments, the branched fatty aldehyde is produced by expressing or overexpressing in the recombinant microbial cell a polynucleotide encoding a polypeptide having a fatty aldehyde biosynthesis activity such as carboxylic acid reductase (CAR) activity (encoded, for example, by a *car* gene). Examples of carboxylic acid reductase (CAR) polypeptides and polynucleotides encoding them useful in accordance with this embodiment include, but are not limited to, FadD9 (EC 6.2.1.-, UniProtKB Q50631, GenBank NP_217106), CarA (GenBank ABK75684), CarB (GenBank YP889972) and related polypeptides described in PCT Publication No. WO 2010/062480 which is incorporated by reference herein. In some embodiments the recombinant microbial cell further comprises a polynucleotide encoding a thioesterase.

In some embodiments, the branched fatty aldehyde is produced by expressing or overexpressing in the recombinant microbial cell a polynucleotide encoding a fatty aldehyde biosynthesis polypeptide, such as a polypeptide having acyl-ACP reductase (AAR) activity, encoded by, for example, an *aar* gene. Examples of acyl-ACP reductase polypeptides useful in accordance with this embodiment include, but are not limited to, acyl-ACP reductase from *Synechococcus elongatus* PCC 7942 (GenBank YP_400611) and related polypeptides described in PCT Publication No. WO 2010/042664 which is incorporated by reference herein.

In some embodiments, the branched fatty aldehyde is produced by expressing or overexpressing in the recombinant microbial cell a polynucleotide encoding a fatty aldehyde biosynthesis polypeptide, such as a polypeptide having acyl-CoA reductase activity (e.g., EC 1.2.1.x), encoded by, for example, an *acr1* gene. Examples of acyl-CoA reductase polypeptides useful in accordance with this embodiment include, but are not limited to, ACR1 from *Acinetobacter sp.* strain ADP1 (GenBank YP_047869) and related polypeptides described in PCT Publication No. WO 2010/042664 which is incorporated by reference herein. In some embodiments the recombinant microbial cell further comprises polynucleotides encoding a thioesterase and an acyl-CoA synthase.

*Branched Fatty Alcohol*

In one embodiment, the recombinant microbial cell produces a branched fatty alcohol (e.g., an anteiso-branched fatty alcohol or an iso-branched fatty alcohol). In some embodiments, a branched fatty aldehyde produced by the recombinant microbial cell is converted to the branched fatty alcohol.

In some embodiments, the recombinant microbial cell comprises a polynucleotide encoding a polypeptide (i.e., an enzyme) having fatty alcohol biosynthesis activity (also referred to herein as a "fatty alcohol biosynthesis polypeptide" or a "fatty alcohol biosynthesis enzyme"), and the branched fatty alcohol is produced by a reaction catalyzed by the fatty alcohol biosynthesis enzyme expressed or overexpressed in the recombinant microbial cell. In some embodiments, a composition comprising branched fatty alcohols (also referred to herein as a "branched fatty alcohol composition"), produced by culturing the recombinant cell in the presence of a carbon source under conditions effective to express the polynucleotides, is recovered from the cell culture. In some embodiments, the recombinant cell produces a branched fatty alcohol composition comprising anteiso-branched fatty alcohols.

In some embodiments, the branched fatty alcohol is produced by expressing or overexpressing in the recombinant microbial cell a polynucleotide encoding a polypeptide having fatty alcohol biosynthesis activity such as alcohol dehydrogenase (aldehyde reductase) activity, e.g., EC 1.1.1.1. Examples of alcohol dehydrogenase polypeptides useful in accordance with this embodiment include, but are not limited to, *E. coli* alcohol dehydrogenase YqhD (GenBank AP_003562) and related polypeptides described in PCT Publication Nos. WO 2007/136762 and WO2008/119082 which are incorporated by reference herein. In some embodiments the recombinant microbial cell further comprises a polynucleotide encoding a fatty aldehyde biosynthesis polypeptide. In some embodiments the recombinant microbial cell further comprises a polynucleotide encoding a thioesterase.

In some embodiments, the branched fatty alcohol is produced by expressing or overexpressing in the recombinant microbial cell a polynucleotide encoding a fatty alcohol biosynthesis polypeptide, such as a polypeptide having fatty alcohol forming acyl-CoA reductase (FAR) activity, e.g., EC 1.1.1.x. Examples of FAR polypeptides useful in accordance with this embodiment include, but are not limited to, those described in PCT Publication No. WO 2010/062480 which is incorporated by reference herein. In some embodiments the recombinant microbial cell further comprises polynucleotides encoding a thioesterase and an acyl-CoA synthase.

*Branched Hydrocarbon*

In one embodiment, the recombinant microbial cell produces a branched hydrocarbon (e.g., an anteiso-branched hydrocarbon or an iso-branched hydrocarbon), such as a branched alkane or a branched alkene (e.g., a branched terminal olefin or a branched internal olefin). In some embodiments, a branched fatty aldehyde produced by the recombinant microbial cell is converted into the branched hydrocarbon.

In some embodiments, the recombinant microbial cell comprises a polynucleotide encoding a polypeptide (i.e., an enzyme) having hydrocarbon biosynthesis activity (also referred to herein as a "hydrocarbon biosynthesis polypeptide" or a "hydrocarbon biosynthesis enzyme"), and the branched hydrocarbon is produced by a reaction catalyzed by the hydrocarbon biosynthesis enzyme expressed or overexpressed in the recombinant microbial cell. In some embodiments, a composition comprising branched hydrocarbons (also referred to herein as a "branched hydrocarbon composition"), produced by culturing the recombinant cell in the presence of a carbon source under conditions effective to express the polynucleotides, is recovered from the cell culture. In some embodiments, the recombinant cell produces a branched hydrocarbon composition comprising anteiso-branched fatty hydrocarbons.

In some embodiments, the branched hydrocarbon is produced by expressing or overexpressing in the recombinant microbial cell a polynucleotide encoding a polypeptide having hydrocarbon biosynthesis activity such as an aldehyde decarbonylase (ADC) activity (e.g., EC 4.1.99.5), for example, a polynucleotide encoding an aldehyde decarbonylase from *Prochlorococcus marinus* MIT9313 (GenBank NP_895059). Additional examples of aldehyde decarbonylase and related polypeptides useful in accordance with this embodiment include, but are not limited to, those described in PCT Publication Nos. WO 2007/136762 and WO2008/119082 which are incorporated by reference herein. In some embodiments the recombinant microbial cell further comprises a polynucleotide encoding a fatty aldehyde biosynthesis polypeptide. In some embodiments the recombinant microbial cell further comprises a polynucleotide encoding an acyl-ACP reductase .

In some embodiments, a branched terminal olefin is produced by expressing or overexpressing in the recombinant microbial cell a polynucleotide encoding a hydrocarbon biosynthesis polypeptide, such as a polypeptide having decarboxylase activity as described, for example, in PCT Publication No. 2009/085278 which is incorporated by reference herein. In some embodiments the recombinant microbial cell further comprises a polynucleotide encoding a thioesterase.

In some embodiments, a branched internal olefin is produced by expressing or overexpressing in the recombinant microbial cell a polynucleotide encoding a hydrocarbon biosynthesis polypeptide, such as a polypeptide having OleCD or OleBCD activity as described, for example, in PCT Publication No. WO 2008/147781 which is incorporated by reference herein. In some embodiments the recombinant microbial cell further comprises a polynucleotide encoding a thioesterase and an acyl-CoA synthase.

**Saturation Levels of Branched Fatty Acid Derivatives**

The degree of saturation of branched acyl-ACPs (which can then be converted into various branched fatty acid derivatives as described hereinabove) can be controlled by regulating the degree of saturation of fatty acid intermediates. For example, the *sfa, gns,* and *fab* families of genes can be expressed, overexpressed, or expressed at reduced levels (e.g., attenuated), to control the amount of saturation of a branched acyl-ACP.

**BCFA Pathway Polypeptides and Polynucleotides**

The disclosure identifies polynucleotides useful in the recombinant microbial cells, methods, and compositions of the invention; however it will be recognized that absolute sequence identity to such polynucleotides is not necessary. For example, changes in a particular polynucleotide sequence can be made and the encoded polypeptide screened for activity. Such changes typically comprise conservative mutations and silent mutations (such as, for example, codon optimization). Modified or mutated (i.e., mutant) polynucleotides and encoded variant polypeptides can be screened for a desired function, such as, an improved function compared to the parent polypeptide, including but not limited to increased catalytic activity, increased stability, or decreased inhibition (e.g., decreased feedback inhibition), using methods known in the art.

The disclosure identifies enzymatic activities involved in various steps (i.e., reactions) of the BCFA biosynthetic pathways described herein according to Enzyme Classification (EC) number, and provides exemplary polypeptides (i.e., enzymes) categorized by such EC numbers, and exemplary polynucleotides encoding such polypeptides. Such exemplary polypeptides and polynucleotides, which are identified herein by Accession Numbers and/or Sequence Identifier Numbers (SEQ ID NOs), are useful for engineering BCFA pathways in parental microbial cells to obtain the recombinant microbial cells described herein. It is to be understood, however, that polypeptides and polynucleotides described herein are exemplary and non-limiting. The sequences of homologues of representative polypeptides described herein are available to those of skill in the art using databases such as, for example, the Entrez databases provided by the National Center for Biotechnology Information (NCBI), the ExPasy databases provided by the Swiss Institute of Bioinformatics, and the KEGG database provided by the Bioinformatics Center of Kyoto University and University of Tokyo, all which are available on the World Wide Web.

It is to be further understood that a variety of microbial cells can be modified to contain a BCFA pathway described herein, resulting in recombinant microbial cells suitable for the production of branched chain fatty acid derivatives. It is also understood that a variety of cells can provide sources of genetic material, including sequences of polynucleotides encoding polypeptides suitable for use in a recombinant microbial cell provided herein.

The disclosure provides numerous examples of polypeptides (i.e., enzymes) having activities suitable for use in the BCFA biosynthetic pathways described herein. Such polypeptides are collectively referred to herein as "BCFA pathway polypeptides" (alternatively, "BCFA pathway enzymes"). Non-limiting examples of BCFA pathway polypeptides suitable for use in recombinant microbial cells of the invention are provided in the Tables and Description and in the Examples herein.

In some embodiments, the invention includes a recombinant microbial cell comprising a polynucleotide sequence (also referred to herein as a "BCFA pathway polynucleotide" sequence) which encodes a BCFA pathway polypeptide.

Additional BCFA pathway polypeptides and polynucleotides encoding them suitable for use in engineering a BCFA pathway in a recombinant microbial cell of the invention can be obtained by a number of methods.

For example, EC numbers classify enzymes according to the reaction catalyzed. Enzymes that catalyze a reaction in a biosynthetic pathway described herein can be identified by searching the EC number corresponding to that reaction in a database such as, for example: the KEGG database (Kyoto Encyclopedia of Genes and Genomes; Kyoto University and University of Tokyo); the UNIPROTKB database or the ENZYME database (ExPASy Proteomics Server; Swiss Institute of Bioinformatics); the PROTEIN database or the GENE database (Entrez databases; National Center for Biotechnology Information (NCBI)); or the BRENDA database (The Comprehensive Enzyme Information System; Technical University of Braunschweig); all of which are available on the World Wide Web. In one embodiment, a BCFA pathway polynucleotide encoding a BCFA pathway polypeptide having an enzymatic activity categorized by an EC number (such as, an EC number listed in the Description or in one of Tables herein), or a fragment or a variant thereof having that activity, is used in engineering the corresponding step of a BCFA pathway in a recombinant microbial cell.

In some embodiments, a BCFA pathway polynucleotide sequence encodes a polypeptide which is endogenous to the parental cell of the recombinant cell being engineered. Some such endogenous polypeptides are overexpressed in the recombinant microbial cell. An "endogenous polypeptide", as used herein, refers to a polypeptide which is encoded by the genome of the parental (e.g, wild-type) cell that is being engineered to produce the recombinant microbial cell.

A BCFA pathway polypeptide, such as for example an endogenous BCFA pathway polypeptide, can be overexpressed by any suitable means. As used herein, "overexpress" means to express or cause to be expressed a polynucleotide or polypeptide in a cell at a greater concentration than is normally expressed in a corresponding parental (for example, wild-type) cell under the same conditions. For example, a polypeptide is "overexpressed" in a recombinant microbial cell when it is present in a greater concentration in the recombinant cell as compared to its concentration in a non-recombinant host cell of the same species (e.g., the parental cell) when cultured under the same conditions.

In some embodiments, the BCFA pathway polynucleotide sequence encodes an exogenous or heterologous polypeptide. In other words, the polypeptide encoded by the polynucleotide is exogenous to the parental microbial cell. An "exogenous" (or "heterologous") polypeptide, as used herein, refers to a polypeptide not encoded by the genome of the parental (e.g, wild-type) microbial cell that is being engineered to produce the recombinant microbial cell. Such a polypeptide can also be referred to as a "non-native" polypeptide. A variant (that is, a mutant) polypeptide is an example of an exogenous polypeptide.

In certain embodiments, a BCFA pathway polypeptide comprises an amino acid sequence other than that of one of the exemplary polypeptides provided herein; for example, the BCFA pathway polypeptide can comprise a sequence which is a homologue, a fragment, or a variant of the sequence of the exemplary polypeptide.

The terms "homolog," "homologue," and "homologous" as used herein refer to a polynucleotide or a polypeptide comprising a sequence that is at least 50%, preferably at least 60%, more preferably at least 70% (e.g., at least 75%, at least 80%, at least 85%, at least 90%, at least 91%, at least 92%, at least 93%, at least 94%, at least 95%, at least 96%, at least 97%, at least 98%, or at least 99%) homologous to the corresponding polynucleotide or polypeptide sequence. One of ordinary skill in the art is well aware of methods to determine homology between two or more sequences. Briefly, calculations of "homology" between two sequences can be performed as follows. The sequences are aligned for optimal comparison purposes (e.g., gaps can be introduced in one or both of a first and a second amino acid or polynucleotide sequence for optimal alignment and non-homologous sequences can be disregarded for comparison purposes). In a preferred embodiment, the length of a first sequence that is aligned for comparison purposes is at least about 30%, preferably at least about 40%, more preferably at least about 50%, even more preferably at least about 60%, and even more preferably at least about 70%, at least about 80%, at least about 90%, or about 100% of the length of a second sequence. The amino acid residues or nucleotides at corresponding amino acid positions or nucleotide positions of the first and second sequences are then compared. When a position in the first sequence is occupied by the same amino acid residue or nucleotide as the corresponding position in the second sequence, then the molecules are identical at that position (as used herein, amino acid or nucleic acid "identity" is equivalent to amino acid or nucleic acid "homology"). The percent identity between the two sequences is a function of the number of identical positions shared by the sequences, taking into account the number of gaps and the length of each gap, which need to be introduced for optimal alignment of the two sequences.

The comparison of sequences and determination of percent homology (i.e., percent identity) between two sequences can be accomplished using a mathematical algorithm, such as BLAST (Altschul et al., J. Mol. Biol., 215(3): 403-410 (1990)). The percent homology between two amino acid sequences also can be determined using the Needleman and Wunsch algorithm that has been incorporated into the GAP program in the GCG software package, using either a Blossum 62 matrix or a PAM250 matrix, and a gap weight of 16, 14, 12, 10, 8, 6, or 4 and a length weight of 1, 2, 3,4, 5, or 6 (Needleman and Wunsch, J. Mol. Biol., 48: 444-453 (1970)). The percent homology between two nucleotide sequences also can be determined using the GAP program in the GCG software package, using a NWSgapdna.CMP matrix and a gap weight of 40, 50, 60, 70, or 80 and a length weight of 1, 2, 3, 4, 5, or 6. One of ordinary skill in the art can perform initial homology calculations and adjust the algorithm parameters accordingly. A preferred set of parameters (and the one that should be used if a practitioner is uncertain about which parameters should be applied to determine if a molecule is within a homology limitation of the claims) are a Blossum 62 scoring matrix with a gap penalty of 12, a gap extend penalty of 4, and a frameshift gap penalty of 5. Additional methods of sequence alignment are known in the biotechnology arts (see, e.g., Rosenberg, BMC Bioinformatics, 6: 278 (2005); Altschul et al., FEBSJ., 272(20): 5101-5109 (2005)).

An "equivalent position" (for example, an "equivalent amino acid position" or "equivalent nucleic acid position") is defined herein as a position (such as, an amino acid position or nucleic acid position) of a test polypeptide (or test polynucleotide) sequence which aligns with a corresponding position of a reference polypeptide (or reference polynucleotide) sequence, when optimally aligned using an alignment algorithm as described herein. The equivalent amino acid position of the test polypeptide need not have the same numerical position number as the corresponding position of the reference polypeptide; likewise, the equivalent nucleic acid position of the test polynucleotide need not have the same numerical position number as the corresponding position of the reference polynucleotide.

In some embodiments, the BCFA pathway polypeptide is a variant of a reference (e.g., a parent) polypeptide, such as a variant of an exemplary BCFA pathway polypeptide described herein. A "variant" (alternatively, "mutant") polypeptide as used herein refers to a polypeptide having an amino acid sequence that differs from that of a parent (e.g.,

wild-type) polypeptide by at least one amino acid. The variant can comprise one or more conservative amino acid substitutions, and/or can comprise one or more non-conservative substitutions, compared to the parent polypeptide sequence. In some embodiments, the variant polypeptide has 1, 2, 3, 4, 5, 6, 7, 8, 9, 10, 15, 20, 30, 40, 50, or more amino acid substitutions, additions, insertions, or deletions compared to the parent polypeptide sequence. In some embodiments, the sequence of the variant polypeptide is at least 80%, at least 85%, at least 90%, at least 91%, at least 92%, at least 93%, at least 94%, at least 95%, at least 96%, at least 97%, at least 98%, or at least 99% identical to the sequence of the parent polypeptide.

In some embodiments, the BCFA pathway polypeptide is a fragment of a reference (e.g., a parent) polypeptide, such as a fragment of an exemplary BCFA pathway polypeptide described herein. The term "fragment" refers to a shorter portion of a full-length polypeptide or protein ranging in size from four amino acid residues to the entire amino acid sequence minus one amino acid residue. In certain embodiments of the invention, a fragment refers to the entire amino acid sequence of a domain of a polypeptide or protein (e.g., a substrate binding domain or a catalytic domain).

In some embodiments, a homologue, a variant, or a fragment further comprises one or more sequence motifs as defined herein. In one embodiment, a homologue, a variant, or a fragment of a branched chain alpha-keto acid dehydrogenase E1-alpha subunit polypeptide further comprises one or more sequence motifs selected from SEQ ID NOs: 15-21. In another embodiment, a homologue, a variant, or a fragment of a branched chain alpha-keto acid dehydrogenase E1-beta subunit polypeptide further comprises one or more sequence motifs selected from SEQ ID NOs: 36-42. In another embodiment, a homologue, a variant, or a fragment of a lipoamide acyltransferase polypeptide further comprises one or more sequence motifs selected from SEQ ID NOs: 57-62. In another embodiment, a homologue, a variant, or a fragment of a dihydrolipoyl dehydrogenase polypeptide further comprises one or more sequence motifs selected from SEQ ID NOs: 79-83. In another embodiment, a homologue, a variant, or a fragment of a beta-ketoacyl-ACP synthase III polypeptide further comprises one or more sequence motifs selected from SEQ ID NOs:110-115. Determination that a sequence contains a particular sequence motif can be readily accomplished, for example, using the ScanProsite tool available on the World Wide Web site of the ExPASy Proteomics Server.

It is understood that a BCFA polypeptide may have conservative or non-essential amino acid substitutions, relative to a parent polypeptide, which does not have a substantial effect on a biological function or property of the BCFA polypeptide. Whether or not a particular substitution will be tolerated (i.e., will not adversely affect a desired biological function, such as enzymatic activity) can be determined, for example, as described in Bowie et al. (Science, 247: 1306-1310 (1990)).

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), and aromatic side chains (e.g., tyrosine, phenylalanine, tryptophan, histidine).

Variants can be naturally occurring or created *in vitro.* In particular, variants can be created using genetic engineering techniques, such as site directed mutagenesis, random chemical mutagenesis, exonuclease III deletion procedures, or standard cloning techniques. Alternatively, such variants, fragments, analogs, or derivatives can be created using chemical synthesis or modification procedures.

Methods of making variants are well known in the art. These include procedures in which nucleic acid sequences obtained from natural isolates are modified to generate nucleic acids that encode polypeptides having characteristics that enhance their value in industrial or laboratory applications (including, but not limited to, increased catalytic activity (turnover number), improved stability, and reduced feedback inhibition). In such procedures, a large number of modified nucleic acid sequences having one or more nucleotide differences with respect to the sequence obtained from the natural isolate are generated and characterized. Typically, these nucleotide differences result in amino acid changes with respect to the polypeptides encoded by the nucleic acids from the natural isolates. For example, variants can be prepared by using random or site-directed mutagenesis.

Variants can also be created by *in vivo* mutagenesis. In some embodiments, random mutations in a nucleic acid sequence are generated by propagating the sequence in a bacterial strain, such as an *E. coli* strain, which carries mutations in one or more of the DNA repair pathways. Such "mutator" strains have a higher random mutation rate than that of a wild-type strain. Propagating a DNA sequence in one of these strains will eventually generate random mutations within the DNA. Mutator strains suitable for use for *in vivo* mutagenesis are described in, for example, International Patent Application Publication No. WO 1991/016427.

Variants can also be generated using cassette mutagenesis. In cassette mutagenesis, a small region of a double-stranded DNA molecule is replaced with a synthetic oligonucleotide "cassette" that differs from the native sequence. The oligonucleotide often contains a completely and/or partially randomized native sequence.

Recursive ensemble mutagenesis can also be used to generate variants. Recursive ensemble mutagenesis is an algorithm for protein engineering (i.e., protein mutagenesis) developed to produce diverse populations of phenotypically related mutants whose members differ in amino acid sequence. This method uses a feedback mechanism to control successive rounds of combinatorial cassette mutagenesis. Recursive ensemble mutagenesis is described in, for example, Arkin et al., Proc. Natl. Acad. Sci., U.S.A., 89: 7811-7815 (1992).

In some embodiments, variants are created using exponential ensemble mutagenesis. Exponential ensemble mutagenesis is a process for generating combinatorial libraries with a high percentage of unique and functional mutants, wherein small groups of residues are randomized in parallel to identify, at each altered position, amino acids which lead to functional proteins. Exponential ensemble mutagenesis is described in, for example, Delegrave et al., Biotech. Res, 11: 1548-1552 (1993).

Preferred fragments or variants of a parent polypeptide (e.g, fragments or variants of a parent BCFA pathway polypeptide) retain some or all of a biological function or property (such as, enzymatic activity, thermal stability) of the parent polypeptide. In some embodiments, the fragment or variant retains at least 75% (e.g., at least 80%, at least 90%, or at least 95%) of a biological function or property of the parent polypeptide. In other embodiments, the fragment or variant retains about 100% of a biological function or property of the parent polypeptide.

In some embodiments, the fragment or variant of the parent polypeptide exhibits an increased catalytic activity (as reflected by, for example, a higher turnover number, an altered pH optimum, or a decreased $K_m$ for a desired substrate), relative to that of the parent polypeptide, under conditions in which the recombinant microbial cell is cultured. For example, if the parent polypeptide is endogenous to (that is, is derived from) a thermophilic cell, and if the recombinant microbial cell is generally cultured at a lower temperature than the thermophilic cell, the parent polypeptide may exhibit significantly reduced activity at the lower temperature; in which case, the variant polypeptide preferably exhibits an increased catalytic activity (such as, a higher turnover number), relative to that of the parent polypeptide, at that lower temperature.

In other embodiments, the fragment or variant of the parent polypeptide exhibits improved stability, relative to that of the parent polypeptide, under conditions in which the recombinant microbial cell is cultured. Such stability can include stability towards changes in temperature, ionic strength, pH, or any other differences in growth or media conditions between the recombinant microbial cell and the cell from which the parent polypeptide was derived. For example, if the parent polypeptide is derived from a psychrotrophic cell, and if the recombinant microbial cell is generally cultured at a higher temperature than the psychrotrophic cell, the parent polypeptide may be relatively unstable at the higher temperature ; in which case, the variant polypeptide preferably exhibits improved stability relative to that of the parent polypeptide at that higher temperature.

In other embodiments, the fragment or variant of the parent polypeptide exhibits reduced inhibition of catalytic activity (such as, reduced feedback inhibition) by a cellular metabolite or by a culture media component, relative to such inhibition exhibited by the parent polypeptide, under conditions in which the recombinant microbial cell is cultured.

In certain embodiments, a BCFA pathway polypeptide is a homologue, a fragment, or a variant of a parent polypeptide, wherein the BCFA pathway polypeptide is effective in carrying out a BCFA pathway reaction in a recombinant microbial cell. Such a BCFA pathway polypeptide is suitable for use in a recombinant microbial cell of the invention.

The effectiveness of a test polypeptide (such as, for example, a BCFA pathway polypeptide described herein, or a homologue, a fragment, or a variant thereof) in carrying out a reaction of a BCFA pathway can be determined by a number of methods. For example, to determine the effectiveness of a test polypeptide in catalyzing a specific reaction of a biochemical pathway, first a host cell is engineered to obtain a parental cell that comprises all the activities necessary to catalyze the reactions of the biochemical pathway in question, except for the specific pathway reaction being tested (although, in some instances, the parental cell may express endogenous polypeptide(s) that catalyze the specific pathway reaction being tested; in such instances the endogenous activity will preferably be low enough to readily detect an increase in product owing to the activity of the test polypeptide). A polynucleotide encoding the test polypeptide, operatively linked to a suitable promoter (e.g., in an expression vector), is then introduced into the parental cell, generating a test cell. The test cell and the parental cell are cultured separately under identical conditions which are sufficient for expression of the pathway polypeptides in the parental and test cell cultures and expression of the test polypeptide in the test cell culture. At various times during and/or after culturing, samples are obtained from the test cell culture and the parental cell culture. The samples are analyzed for the presence of a particular pathway intermediate or product. Presence of the pathway intermediate or product can be determined by methods including, but not limited to, gas chromatography (GC), mass spectroscopy (MS), thin layer chromatography (TLC), high-performance liquid chromatography (HPLC), liquid chromatography (LC), GC coupled with a flame ionization detector (FID), GC-MS, and LC-MS. Example 11 herein provides methods of analyzing culture samples for the presence of a BCFA pathway intermediate or product, such as a branched fatty acid, a branched fatty alcohol, a branched fatty ester or a branched hydrocarbon. The presence of a BCFA pathway intermediate or product in the test cell culture sample(s), and the absence (or a reduced amount) of the BCFA pathway intermediate or product in the parent cell culture sample(s), indicates that the test polypeptide is effective in carrying out a BCFA pathway reaction and is suitable for use in a recombinant microbial cell of the invention.

**Production of Branched Fatty Acid Derivatives in Recombinant Microbial Cells**

In one aspect, the invention includes a method of making a branched fatty acid derivative composition, the method comprising culturing a recombinant microbial cell of the invention in a culture medium containing a carbon source under conditions effective to express the recombinant polynucleotide sequences, and optionally isolating the produced branched fatty acid derivative composition.

A "branched fatty acid derivative composition" is a composition comprising a branched fatty acid derivative as defined herein, such as, for example, a branched fatty acid, a branched fatty ester (e.g., a branched fatty methyl ester, a branched fatty ethyl ester, a branched wax ester), a branched fatty aldehyde, a branched fatty alcohol, a branched hydrocarbon (such as a branched alkane, a branched alkene, a branched terminal olefin, a branched internal olefin), or a branched ketone. Similarly, a "branched fatty acid composition" is a composition comprising a branched fatty acid, and so on.

In one aspect, the invention includes a method of making a composition comprising a branched fatty acid derivative, the method comprising: obtaining a recombinant microbial cell (such as, a culture comprising a recombinant microbial cell) comprising : (a) polynucleotides encoding a branched chain alpha-keto acid dehydrogenase (BKD) complex, comprising polypeptides having branched-chain alpha-keto acid dehydrogenase activity, lipoamide acyltransferase activity, and dihydrolipoamide dehydrogenase activity, and (b) a polynucleotide encoding a polypeptide having beta-ketoacyl-ACP synthase activity that utilizes a branched acyl-CoA molecule as a substrate, wherein at least one polynucleotide according to (a) or (b) encodes a polypeptide that is exogenous to the parental microbial cell or expression of said polynucleotide is modulated in the recombinant microbial cell; the recombinant microbial cell further comprising one or more polynucleotides each which encodes a polypeptide having fatty acid derivative enzyme activity, wherein the recombinant microbial cell produces a branched chain fatty acid derivative when cultured in the presence of a carbon source under conditions effective to express the polynucleotides; culturing the recombinant microbial cell in a culture medium containing a carbon source under conditions effective to express the polynucleotides and produce a fatty acid derivative composition comprising straight-chain fatty acid derivatives and branched fatty acid derivatives, the branched fatty acid derivatives comprising iso-branched fatty acid derivatives and/or anteiso-branched fatty acid derivatives; and optionally recovering the composition from the culture medium.

In some embodiments, the fatty acid derivative composition produced by the recombinant cell comprises branched fatty acid derivatives, wherein at least 10%, at least 20%, at least 30%, at least 40%, at least 50%, at least 60%, at least 70%, at least 80% or at least 90% by weight of the fatty acid derivatives in the composition are branched fatty acid derivatives. In some embodiments, the fatty acid derivative composition comprises branched fatty acid derivatives in an amount (e.g., a titer) of at least 10 mg/L, at least 15 mg/L, at least 20 mg/L, at least 25 mg/L, at least 50 mg/L, at least 75 mg/L, at least 100 mg/L, at least 125 mg/L, at least 150 mg/L, at least 175 mg/L, at least 200 mg/L, at least 225 mg/L, at least 250 mg/L, at least 275 mg/L, at least 300 mg/L, at least 325 mg/L, at least 350 mg/L, at least 375 mg/L, at least 400 mg/L, at least 425 mg/L, at least 450 mg/L, at least 475 mg/L, at least 500 mg/L, at least 525 mg/L, at least 550 mg/L, at least 575 mg/L, at least 600 mg/L, at least 625 mg/L, at least 650 mg/L, at least 675 mg/L, at least 700 mg/L, at least 725 mg/L, at least 750 mg/L, at least 775 mg/L, at least 800 mg/L, at least 825 mg/L, at least 850 mg/L, at least 875 mg/L, at least 900 mg/L, at least 925 mg/L, at least 950 mg/L, at least 975 mg/L, at least 1000 mg/L, at least 1050 mg/L, at least 1075 mg/L, at least 1100 mg/L, at least 1125 mg/L, at least 1150 mg/L, at least 1175 mg/L, at least 1200 mg/L, at least 1225 mg/L, at least 1250 mg/L, at least 1275 mg/L, at least 1300 mg/L, at least 1325 mg/L, at least 1350 mg/L, at least 1375 mg/L, at least 1400 mg/L, at least 1425 mg/L, at least 1450 mg/L, at least 1475 mg/L, at least 1500 mg/L, at least 1525 mg/L, at least 1550 mg/L, at least 1575 mg/L, at least 1600 mg/L, at least 1625 mg/L, at least 1650 mg/L, at least 1675 mg/L, at least 1700 mg/L, at least 1725 mg/L, at least 1750 mg/L, at least 1775 mg/L, at least 1800 mg/L, at least 1825 mg/L, at least 1850 mg/L, at least 1875 mg/L, at least 1900 mg/L, at least 1925 mg/L, at least 1950 mg/L, at least 1975 mg/L, at least 2000 mg/L, or a range bounded by any two of the foregoing values.

In various embodiments, the fatty acid derivative enzyme activity comprises a thioesterase activity, an ester synthase activity, a fatty aldehyde biosynthesis activity, a fatty alcohol biosynthesis activity, a ketone biosynthesis activity, and/or a hydrocarbon biosynthesis activity. In some embodiments, the recombinant microbial cell comprises polynucleotides encoding two or more polypeptides, each polypeptide having a fatty acid derivative enzyme activity.

In various embodiments, the one or more polypeptides having fatty acid derivative enzyme activity as described hereinabove, wherein the recombinant microbial cell produces a composition comprising branched fatty acids, branched fatty esters, branched wax esters, branched fatty aldehydes, branched fatty alcohols, branched alkanes, branched alkenes, branched internal olefins, branched terminal olefins, or branched ketones.

In another aspect, the invention includes a method of making a composition comprising an anteiso-branched fatty acid derivative, the method comprising: obtaining a recombinant microbial cell (such as, a culture comprising a recombinant microbial cell) comprising : (a) polynucleotides encoding a branched chain alpha-keto acid dehydrogenase (BKD) complex, comprising polypeptides having branched-chain alpha-keto acid dehydrogenase activity, lipoamide acyltransferase activity, and dihydrolipoamide dehydrogenase activity, and (b) a polynucleotide encoding a polypeptide having beta-ketoacyl-ACP synthase activity that utilizes a branched acyl-CoA molecule as a substrate; and further comprising (c) polynucleotides encoding polypeptides having aspartokinase activity, homoserine dehydrogenase activity, homoserine kinase activity, threonine synthase activity, and threonine deaminase activity, or (d) polynucleotides encoding polypeptides having (R)-citramalate synthase activity, isopropylmalate isomerase activity, and beta-isopropyl malate dehydrogenase activity, or (c) and (d); and (e) polypeptides having acetohydroxyacid synthase activity, acetohydroxyacid isomeroreductase activity, and dihydroxy acid dehydratase activity; wherein at least one polynucleotide according to (a), (b), (c), (d), or (e) encodes a polypeptide that is exogenous to the recombinant microbial cell or expression of said polynucleotide is modulated in the recombinant microbial cell; the recombinant microbial cell further comprising one or more polynucleotides each which encodes a polypeptide having fatty acid derivative enzyme activity, wherein the recombinant microbial cell produces an anteiso-branched chain fatty acid derivative when cultured in the presence of a carbon source under conditions effective to express the polynucleotides; culturing the recombinant microbial cell in a culture medium containing a carbon source under conditions effective to express the polynucleotides and produce a fatty acid derivative composition comprising straight-chain fatty acid derivatives and branched fatty acid derivatives, the branched fatty acid derivatives comprising anteiso-branched fatty acid derivatives; and optionally recovering the composition from the culture medium.

In some embodiments, the fatty acid derivative composition produced by the recombinant microbial cell culture comprises anteiso-branched fatty acid derivatives, wherein at least 10%, at least 20%, at least 30%, at least 40%, at least 50%, at least 60%, at least 70%, or at least 80% by weight of the branched fatty acid derivatives in the composition are anteiso-branched fatty acid derivatives. In some embodiments, the fatty acid derivative composition comprises anteiso-branched fatty acid derivatives in an amount (e.g., a titer) of at least 10 mg/L, at least 15 mg/L, at least 20 mg/L, at least 25 mg/L, at least 50 mg/L, at least 75 mg/L, at least 100 mg/L, at least 125 mg/L, at least 150 mg/L, at least 175 mg/L, at least 200 mg/L, at least 225 mg/L, at least 250 mg/L, at least 275 mg/L, at least 300 mg/L, at least 325 mg/L, at least 350 mg/L, at least 375 mg/L, at least 400 mg/L, at least 425 mg/L, at least 450 mg/L, at least 475 mg/L, at least 500 mg/L, at least 525 mg/L, at least 550 mg/L, at least 575 mg/L, at least 600 mg/L, at least 625 mg/L, at least 650 mg/L, at least 675 mg/L, at least 700 mg/L, at least 725 mg/L, at least 750 mg/L, at least 775 mg/L, at least 800 mg/L, at least 825 mg/L, at least 850 mg/L, at least 875 mg/L, at least 900 mg/L, at least 925 mg/L, at least 950 mg/L, at least 975 mg/L, at least 1000 mg/L, at least 1050 mg/L, at least 1075 mg/L, at least 1100 mg/L, at least 1125 mg/L, at least 1150 mg/L, at least 1175 mg/L, at least 1200 mg/L, at least 1225 mg/L, at least 1250 mg/L, at least 1275 mg/L, at least 1300 mg/L, at least 1325 mg/L, at least 1350 mg/L, at least 1375 mg/L, at least 1400 mg/L, at least 1425 mg/L, at least 1450 mg/L, at least 1475 mg/L, at least 1500 mg/L, at least 1525 mg/L, at least 1550 mg/L, at least 1575 mg/L, at least 1600 mg/L, at least 1625 mg/L, at least 1650 mg/L, at least 1675 mg/L, at least 1700 mg/L, at least 1725 mg/L, at least 1750 mg/L, at least 1775 mg/L, at least 1800 mg/L, at least 1825 mg/L, at least 1850 mg/L, at least 1875 mg/L, at least 1900 mg/L, at least 1925 mg/L, at least 1950 mg/L, at least 1975 mg/L, at least 2000 mg/L, or a range bounded by any two of the foregoing values.

In various embodiments, the fatty acid derivative enzyme activity comprises a thioesterase activity, an ester synthase activity, a fatty aldehyde biosynthesis activity, a fatty alcohol biosynthesis activity, or a hydrocarbon biosynthesis activity. In some embodiments, the recombinant microbial cell comprises polynucleotides encoding two or more polypeptides, each polypeptide having a fatty acid derivative enzyme activity.

In more particular embodiments, the recombinant microbial cell expresses or overexpresses one or more polypeptides having fatty acid derivative enzyme activity, as described hereinabove, wherein the recombinant microbial cell produces a composition comprising anteiso-branched fatty acids, anteiso-branched fatty esters, anteiso-branched wax esters, anteiso-branched fatty aldehydes, anteiso-branched fatty alcohols, anteiso-branched alkanes, anteiso-branched alkenes, anteiso-branched internal olefins, anteiso-branched terminal olefins, or anteiso-branched ketones.

The branched fatty acid derivatives (including iso-branched fatty acid derivatives and anteiso-branched fatty acid derivatives) produced by the methods of invention may be recovered or isolated from the recombinant microbial cell culture. The term "isolated" as used herein with respect to products, such as fatty acids and derivatives thereof, refers to products that are separated from cellular components, cell culture media, or chemical or synthetic precursors. The branched fatty acids and derivatives thereof produced by the methods described herein can be relatively immiscible in the fermentation broth, as well as in the cytoplasm. Therefore, the branched fatty acids and derivatives thereof can collect in an organic phase either intracellularly or extracellularly. The collection of the products in the organic phase can lessen the impact of the branched fatty acid derivative, e.g., branched fatty aldehyde or branched fatty alcohol on cellular function and can allow the recombinant microbial cell to produce more product.

In some embodiments, the branched fatty acid derivatives produced by the methods of invention are purified. As used herein, the term "purify," "purified," or "purification" means the removal or isolation of a molecule from its environment by, for example, isolation or separation. "Substantially purified" molecules are at least about 60% free (e.g., at least about 70% free, at least about 75% free, at least about 85% free, at least about 90% free, at least about 95% free, at least about 97% free, at least about 99% free) from other components with which they are associated. As used herein, these terms also refer to the removal of contaminants from a sample. For example, the removal of contaminants can result in an increase in the percentage of a branched fatty acid derivative (such as, a branched fatty acid or a branched fatty aldehyde or a branched fatty alcohol or a branched fatty ester or a branched hydrocarbon) relative to other components in a sample. For example, when a branched fatty aldehyde or a branched fatty alcohol is produced in a recombinant microbial cell, the branched fatty aldehyde or branched fatty alcohol can be purified by the removal of recombinant microbial cell proteins. After purification, the percentage of the branched fatty aldehyde or branched fatty alcohol in the sample relative to other components is increased.

As used herein, the terms "purify," "purified," and "purification" are relative terms which do not require absolute purity. Thus, for example, when a branched fatty acid or branched fatty acid derivative (e.g., a branched fatty aldehyde, a branched fatty alcohol, and so forth) is produced in recombinant microbial cell s, a purified branched fatty acid or derivative is a branched fatty acid or derivative that is substantially separated from other cellular components (e.g., nucleic acids, polypeptides, lipids, carbohydrates, or other hydrocarbons).

The branched fatty acid derivative may be present in the extracellular environment, or it may be isolated from the extracellular environment of the recombinant microbial cell. In certain embodiments, a branched fatty derivative thereof is secreted from the recombinant microbial cell. In other embodiments, a branched fatty acid derivative is transported into the extracellular environment. In yet other embodiments, the branched fatty acid derivative is passively transported into the extracellular environment. A branched fatty acid derivative can be isolated from a recombinant microbial cell using methods known in the art.

Fatty acid derivatives (including branched fatty acid derivatives produced according to the methods of the present invention) can be distinguished from organic compounds derived from petrochemical carbon on the basis of dual carbon-isotopic fingerprinting or $^{14}C$ dating. Additionally, the specific source of biosourced carbon (e.g., glucose vs. glycerol) can be determined by dual carbon-isotopic fingerprinting (see, e.g., U.S. Patent 7,169,588).

The ability to distinguish fatty acid derivatives produced by recombinant microbial cells from petroleum-based organic compounds is beneficial in tracking these materials in commerce. For example, organic compounds or chemicals comprising both biologically-based and petroleum-based carbon isotope profiles may be distinguished from organic compounds and chemicals made only of petroleum-based materials. Hence, the materials prepared in accordance with the inventive methods may be followed in commerce on the basis of their unique carbon isotope profile.

Fatty acid derivatives produced by recombinant microbial cells can be distinguished from petroleum-based organic compounds by comparing the stable carbon isotope ratio ($^{13}C/^{12}C$) in each fuel. The $^{13}C$ /$^{12}C$ ratio in a given fatty acid derivative thereof produced according to the methods of the invention is a consequence of the $^{13}C/^{12}C$ ratio in atmospheric carbon dioxide at the time the carbon dioxide is fixed. It also reflects the precise metabolic pathway. Regional variations also occur. Petroleum, $C_3$ plants (the broadleaf), $C_4$ plants (the grasses), and marine carbonates all show significant differences in $^{13}C/^{12}C$ and the corresponding $\delta^{13}C$ values. Furthermore, lipid matter of $C_3$ and $C_4$ plants analyze differently than materials derived from the carbohydrate components of the same plants as a consequence of the metabolic pathway.

The $^{13}C$ measurement scale was originally defined by a zero set by Pee Dee Belemnite (PDB) limestone, where values are given in parts per thousand deviations from this material. The "$\delta^{13}C$" values are expressed in parts per thousand (per mil), abbreviated, %o, and are calculated as follows:

$$\delta^{13}C\ (\%o) = [(^{13}C/^{12}C)_{sample} - (^{13}C/^{12}C)_{standard}] / (^{13}C/^{12}C)_{standard} \times 1000$$

In some embodiments, a fatty acids or derivative thereof produced according to the methods of the invention has a $\delta^{13}C$ of about -30 or greater, about -28 or greater, about -27 or greater, about -20 or greater, about -18 or greater, about -15 or greater, about -13 or greater, or about -10 or greater. Alternatively, or in addition, a fatty acids or derivative thereof has a $\delta^{13}C$ of about -4 or less, about -5 or less, about -8 or less, about -10 or less, about -13 or less, about -15 or less, about -18 or less, or about -20 or less. Thus, the fatty acids or derivative thereof can have a $\delta^{13}C$ bounded by any two of the above endpoints. For example, a fatty acids or derivative thereof can have a $\delta^{13}C$ of about -30 to about -15, about -27 to about -19, about -25 to about -21, about -15 to about -5, about -13 to about -7, or about -13 to about -10. In some embodiments, a fatty acids or derivative thereof can have a $\delta^{13}C$ of about -10, -11, -12, or -12.3. In other embodiments, a fatty acids or derivative thereof has a $\delta^{13}C$ of about -15.4 or greater. In yet other embodiments, a fatty acids or derivative thereof has a $\delta^{13}C$ of about -15.4 to about -10.9, or a $\delta^{13}C$ of about -13.92 to about -13.84.

A fatty acid derivative produced by a recombinant microbial cell can also be distinguished from petroleum-based organic compounds by comparing the amount of $^{14}C$ in each compound. Because $^{14}C$ has a nuclear half life of 5730 years, petroleum based fuels containing "older" carbon can be distinguished from fatty acids or derivatives thereof which contain "newer" carbon (see, e.g., Currie, "Source Apportionment of Atmospheric Particles", Characterization of Environmental Particles, J. Buffle and H. P. van Leeuwen, Eds., Vol. I of the IUPAC Environmental Analytical Chemistry Series, Lewis Publishers, Inc., pp. 3-74 (1992)).

As used herein, "fraction of modern carbon" or $f_M$ has the same meaning as defined by National Institute of Standards and Technology (NIST) Standard Reference Materials (SRMs) 4990B and 4990C, known as oxalic acids standards HOxl and HOxll, respectively. The fundamental definition relates to 0.95 times the $^{14}C$ /$^{12}C$ isotope ratio HOxl (referenced to AD 1950). This is roughly equivalent to decay-corrected pre-Industrial Revolution wood. For the current living biosphere (plant material), $f_M$ is approximately 1.1.

In some embodiments, a fatty acid derivative produced according to the methods of the invention has a $f_M{}^{14}C$ of at least about 1, e.g., at least about 1.003, at least about 1.01, at least about 1.04, at least about 1.111, at least about 1.18, or at least about 1.124. Alternatively, or in addition, the fatty acid or derivative has an $f_M{}^{14}C$ of about 1.130 or less, e.g., about 1.124 or less, about 1.18 or less, about 1.111 or less, or about 1.04 or less. Thus, the fatty acid or derivative can have a $f_M{}^{14}C$ bounded by any two of the above endpoints. For example, the fatty acid or derivative can have a $f_m{}^{14}C$ of about 1.003 to about 1.124, a $f_M{}^{14}C$ of about 1.04 to about 1.18, or a $f_M{}^{14}C$ of about 1.111 to about 1.124.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language ("e.g.", "such as", "for example") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## EXAMPLES

### Media Compositions:

M9 minimal media: 6 g/L $Na_2HPO_4$, 3 g/L $KH_2PO_4$, 0.5 g/L NaCl, 1g/L $NH_4Cl$, 1 mg/L thiamine, 1 mM $MgSO_4$, 0.1 mM $CaCl_2$.

FA-2 media: M9 supplemented with Bis-Tris buffer (0.2 M), Triton X-100 (0.1% v/v), and trace minerals containing no iron (2mg/L ZnCl. $4H_2O$, 2mg/L $CaCl_2$. $6H_2O$, 2mg/L $Na_2MoO_4.2H_2O$, 1.9mg/L $CuSO_4.5H_2O$, 0.5mg/L $H_3BO_3$, 100 mL/L concentrated HCl), ferric citrate (10 mg/L), and 30 g/L glucose.

Che-9 media: M9 supplemented with extra $NH_4Cl$ (an additional 1 g/L), Bis-Tris buffer (0.2 M), Triton X-100 (0.1% v/v), and trace minerals (27mg/L $FeCl_3$.6 $H_2O$, 2mg/L $ZnCl.4H_2O$, 2mg/L $CaCl_2.6H_2O$, 2mg/L $Na_2MoO_4.2H_2O$, 1.9mg/L $CuSO_4.5H_2O$, 0.5mg/L $H_3BO_3$, 100 mL/L concentrated HCl).

V9-C media: Che-9 without $FeCl_3.6H_2O$

Che-9 2N-BT media: Che-9 supplemented with 20 g/L (2% w/v) glucose.

4NBT: Che-9 supplemented with 40 g/L (4% w/v) glucose.

### Example 1. Engineering Production Strains

*E.coli* MG1655 *ΔfadE* (Strain "D1")

This example describes the construction of a recombinant microbial cell in which the expression of a fatty acid degradation enzyme is attenuated. *The fadE* gene of *E.coli* (also known as *yafH*), which encodes an acyl coenzyme A dehydrogenase (GenBank Accession No. AAC73325) involved in fatty acid degradation, was deleted from *E. coli* strain MG1655 using the Red system described by Datsenko, K.A. et al. (Proc. Natl. Acad. Sci. USA 97: 6640-6645 (2000)), with the following modifications.

The following two primers were used to create the deletion of *fadE*:

Del-*fadE*-F 5' AAAAACAGCA ACAATGTGAG CTTTGTTGTAATTAT ATTGTAA ACATATT GATTCCGGGGATCCGTCGACC (SEQ ID NO:238); and
Del-*fadE*-R 5' AAACGGAGCCT TTCGGCTCCGTTATT CATTTACGCGGCTTCAAC TTTCCTG TAGGCTGGAGCTGCTTC (SEQ ID NO:239)

The Del-*fadE*-F and Del-*fadE*-R primers were used to amplify the kanamycin resistance ($Km^R$) cassette from plasmid pKD13 (Datsenko *et al., supra*) by PCR. The PCR product was then used to transform electrocompetent *E. coli* MG1655 cells containing plasmid pKD46, which expresses Red recombinase (Datsenko *et al., supra),* which had been previously induced with arabinose for 3-4 hours. Following a 3-hour outgrowth in SOC medium at 37°C, the cells were plated on Luria agar plates containing 50 μg/mL of kanamycin. Resistant colonies were identified and isolated after an overnight incubation at 37°C. Disruption of the *fadE* gene was confirmed in some of the colonies by PCR amplification using primers *fadE*-L2 and *fadE*-R1, which were designed to flank the *E.coli fadE* gene.
*fadE*-L2 5'-CGGGCAGGTGCTATGACCAGGAC (SEQ ID NO:240); and
*fadE*-R1 5'-CGCGGCGTTGACCGGCAGCCTGG (SEQ ID NO:241)

After the *fadE* deletion was confirmed, a single colony was used to remove the $Km^R$ marker using the pCP20 plasmid (Datsenko *et al., supra).* The resulting MG1655 *E.coli* strain with the *fadE* gene deleted and the $Km^R$ marker removed was designated *E.coli* MG1655 *ΔfadE*, or strain "D1".

<u>*E.coli* MG1655 *ΔfadE ΔtonA* (Strain "DV2")</u>

This example describes the construction of a recombinant microbial cell in which the expression of a fatty acid degradation enzyme and the expression of an outer membrane protein receptor are attenuated. The *tonA* (also known *as fhuA*) gene of *E.coli* MG1655 , which encodes a ferrichrome outer membrane transporter which also acts as a bacteriophage receptor (GenBank Accession No. NP_414692) was deleted from strain D1 (described above) using the Red system according to Datsenko *et al., supra,* with the following modifications:
The primers used to create the *tonA* deletion were:

Del-*tonA*-F 5'-ATCATTCTCGTTTACGTTATCATTCACTTTACATCAGAGATATAC CAATGATTCCGGGGATCCGTCGACC (SEQ ID NO:242); and
Del-*tonA*-R 5'-GCACGGAAATCCGTGCCCCAAAAGAGAAATTAGAAACGGAAG GTTGCGG TTGTAGGCTGGAGCTGCTTC (SEQ ID NO:243)

The Del-*tonA*-F and Del-*tonA*-R primers were used to amplify the kanamycin resistance ($Km^R$) cassette from plasmid pKD13 by PCR. The PCR product obtained in this way was used to transform electrocompetent *E. coli* MG1655 D1 cells containing pKD46 (Datsenko *et al., supra*), which cells had been previously induced with arabinose for 3-4 hours. Following a 3-hour outgrowth in SOC medium at 37°C, cells were plated on Luria agar plates containing 50 μg/mL of kanamycin. Resistant colonies were identified and isolated after an overnight incubation at 37°C. Disruption of the *tonA* gene was confirmed in some of the colonies by PCR amplification using primers flanking the *E.coli tonA* gene: ton*A*-verF and *tonA*-verR:.

ton*A*-verF 5'-CAACAGCAACCTGCTCAGCAA (SEQ ID NO:244); and
tonA-verR 5'-AAGCTGGAGCAGCAAAGCGTT (SEQ ID NO:245)

After the *tonA* deletion was confirmed, a single colony was used to remove the $Km^R$ marker using the pCP20 plasmid (Datsenko *et al., supra).* The resulting MG1655 *E.coli* strain having *fadE* and *tonA* gene deletions was designated *E.coli* MG1655 *ΔfadF ΔtonA,* or strain "DV2".

<u>*E. coli* MG1655 *ΔfadE ΔtonA lacl:tesA* (Strain "DV2 'tesA")</u>

This example describes the construction of a recombinant microbial cell comprising a polynucleotide encoding a polypeptide having a fatty acid derivative enzyme activity. The *tesA* polynucleotide sequence encoding *E.coli* acyl-CoA thioesterase I (EC 3.1.1.5, 3.1.2.-; *e.g.*, GenBank Accession AAC73596; SEQ ID NO:202) was modified to remove the leader sequence, such that the resulting *'tesA* gene product was truncated by 25 amino acids and the amino acid at the original position 26, alanine, was replaced with methionine, which then became the first amino acid of the 'TesA polypeptide sequence (SEQ ID NO:204; Cho et al., J. Biol. Chem., 270:4216-4219 (1995)).

An integration cassette containing the *'tesA* coding sequence operatively linked to the $P_{Trc}$ promoter plus a kanamycin resistance gene was PCR- amplified from plasmid pACYC-$P_{Trc}$-tesA (Example 2) using the primers lacl-forward: GGCTGGCTGGCATAAATATCTC (SEQ ID NO:313) and lacZ-reverse: GCGTTAAAGTTGTTCTGCTTCATCAGCAGGATATCCTGCACCATCGTCTGGATTTTGAACTTTTGCTTT GCCACGGAAC (SEQ ID NO:314), electroporated into strain DV2 and integrated into the chromosome using Red recombinase expressed from the pKD46 plasmid (Datsenko *et al., supra*). The transformants were selected on LB plates supplemented with kanamycin. Correct integration was assessed using diagnostic PCR.

**Example 2. Engineering Cells for Production of Branched Chain Fatty Acids**

The following examples describe the construction of recombinant microbial cells comprising polynucleotide sequences encoding branched chain alpha-ketoacid dehydrogenase (BKD) complexes according to part (C) of the BCFA pathway shown in Figure 1, and polynucleotide sequences encoding branched chain-specific β-ketoacyy-ACP synthases (i.e., FabH polypeptides) according to part (D) of the BCFA pathway of Figure 1. The strains exemplified herein also comprise polynucleotide sequences encoding fatty acid derivative enzymes, such as the modified *E. coli* 'tesA gene which expresses a thioesterase and generates fatty acids. This example demonstrates that recombinant *E. coli* strains engineered to express a BKD complex and a branched chain β-ketoacyy-ACP synthase produce branched chain fatty acids.

I. BDK plasmids

*Bacillus subtilis bkd* (pKZ2 plasmid)

*B. subtilis bkd* genes were amplified from *B. subtilis* 168 genomic DNA using the following primers:

B.s.BKD_R: 5'-GCTCTCGAGTTAGTAACAGATGTCTTC-3' (SEQ ID NO:246); and
B.s.BKD_F(4g): 5'-GCGGATCCATGGCAACTGAGTATGACG-3' (SEQ ID NO:247)

Primers B.s.BKD_F(4g) and B.s.BKD_R amplified genes *bkdAA* (encoding the alpha subunit of the E1 component, UniProtKB P37940, GenBank NP_390285; SEQ ID NO:1), *bkdAB* (encoding the beta subunit subunit of the E1 component, UniProtKB P37941, GenBank NP_390284 ; SEQ ID NO:22), *bkdB* (encoding the E2 component, UniProtKB P37942, GenBank NP_390283; SEQ ID NO:43), and *lpdV* (encoding the E3 component, UniProtKB P54533, GenBank NP_390286.2; SEQ ID NO:63). The PCR products were cloned into vector pGL10.173B (SEQ ID NO:228), a pBR322 based plasmid with a $P_{trc}$ promoter, to produce the pKZ2 plasmid. Correct insertion of the PCR products was verified using diagnostic restriction enzyme digests.

*Pseudomonas putida bkd* (pKZ4 plasmid)

*P. putida bkd* genes were amplified from *P. putida* F1 genomic DNA using the following primers:

P.p.BKDFusion_F: 5'-ATAAACCATGGATCCATGAACGAGTACGCCCC-3'(SEQ ID NO:248
P.pBKDFusion_R: 5'-CCAAGCTTCGAATTCTCAGATATGCAAGGCGTG-3'(SEQ ID NO:249

Primers P.p.BKDFusion_F and P.p.BKDFusion_R amplified *P. putida* genes *Pput_1450* (encoding the E3 component, UniProtKB Accession No. A5W0E08; SEQ ID NO:67), *Pput_1451* (encoding the E2 component, UniProtKB Accession No. A5W0E9; SEQ ID NO:47), *Pput_1452* and *Pput_1453* (encoding the E1 alpha and E1 beta subunits, UniProtKB A5W0F1 and A5W0F0, SEQ ID NOs:5 and 26, respectively). The PCR products were cloned into vector pGL10.173B (a pBR322 based plasmid with a Ptrc promoter; SEQ ID NO:228) to produce the pKZ4 plasmid (SEQ ID NO:231). Correct insertion of the PCR products was verified using diagnostic restriction enzyme digests.

*Listeria monocytogenes bkd* (pTB85 plasmid)

*L. monocytogenes bkd* genes were amplified from *L. monocytogenes* Li23 (ATCC 19114D-5) genomic DNA using the following primers:

primer 81 (BKD_forward) GAGGAATAAACCGTGGCAACAGAATATGATGTCGTTATTCT (SEQ ID NO:250)

primer 82 (BKD_reverse) CCCAAGCTTCGAATTTTAATACAATGCTGTATTTCTTTGGAAAT (SEQ ID NO:251)

The *L. monocytogenes bkd* operon (SEQ ID NO:232) generated by PCR was cloned into the NcoI and EcoRI sites of pGL10.173B (a pBR322 based plasmid with a Ptrc promoter; SEQ ID NO:228) to generate the plasmid pTB85.

II. FabH plasmids

pDG2 expression vector

The pDG2 expression vector was the base plasmid for may of the constructs described below. The pCDFDuet-1 vector (Novagen/EMD Biosciences) carries the CloDF13 replicon, *lacI* gene and streptomycin/spectinomycin resistance gene (*aadA*). To construct the pDG2 plasmid, the C-terminal portion of the *plsX* gene, which contains an internal promoter for the downstream *fabH* gene (Podkovyrov and Larson, Nucl. Acids Res. (1996) 24 (9): 1747-1752 (1996)) was amplified from *E. coli* MG1655 genomic DNA using primers 5'- TGAATTCCATGGCGCAACTCACTCTTCTTTTAGTCG-3' (SEQ ID NO:252) and 5'- CAGTACCTCGAGTCTTCGTATACATATGCGCT CAGTCAC-3' (SEQ ID NO:253) These primers introduced *NcoI* and *XhoI* restriction sites near the ends, as well as an internal *NdeI* site.

Both the *plsX* insert (containing the *EcfabH* promoter), and the pCDFDuet-1 vector, were digested with restriction enzymes *NcoI* and *XhoI.* The cut vector was treated with Antarctic phosphatase. The insert was ligated into the vector and transformed into transformation-competent *E. coli* cells. Clones were screened by DNA sequencing. The pDG2 plasmid sequence is provided herein as SEQ ID NO: 229.

*B. subtilis fabH1* (pDG6), *B. subtilis fabH2* (pDG7) and *Streptomyces coelicolor fabH* (pDG8)

The pDG6 plasmid was constructed using the pDG2 plasmid. The *fabH1* coding sequence was amplified from *Bacillus subtilis* strain 168 using primers
5'- CCTTGGGGCATATGAAAGCTG-3' (SEQ ID NO:254) and
5'- TTTAGTCATCTCGAGTGCACCTCACCTTT-3' (SEQ ID NO:255). These primers introduced *NdeI* and *XhoI* restriction sites at the ends of the amplification product.

Both the *fabH1* insert and the pDG2 vector were digested with restriction enzymes *NdeI* and *XhoI.* The cut vector was treated with Antarctic phosphatase. The insert was ligated into the vector and transformed into transformation-competent *E. coli* cells. Clones were screened by DNA sequencing. The pDG6 plasmid sequence is provided herein as SEQ ID NO: 230, and expresses the *B. subtilis* FabH1 polypeptide (SEQ ID NO:84) under the control of the *EcfabH* promoter.

Other plasmids based on pDG2 were prepared using a similar strategy as for the pDG6 plasmid. Plasmid pDG7 comprises a *Bacillus subtilis fabH2* insert which expresses the *B. subtilis* FabH2 polypeptide (SEQ ID NO:86). Plasmid pDG8 comprises a *Streptomyces coelicolor fabH* insert which expresses the *S. coelicolor* FabH polypeptide (SEQ ID NO:96).

*B. subtilis fabH1* (pKZ5 plasmid)

Plasmid pKZ5 was constructed by cloning the *NcoI-AvrII* fragment of pDG6, containing *BsFabH1* under control of the *EcfabH* promoter, into the *NcoI-AvrII* cut vector pACYCDuet-1 (Novagen). Plasmid pKZ5 carries a chloramophenicaol resistance gene and a streptomycin/spectinomycin resistance gene.

III. Other plasmids

pACYC-$P_{Trc}$-tesA and pACYC-$P_{Trc2}$-tesA plasmids

Plasmid pACYC-$P_{Trc}$ was constructed by PCR-amplifying the $lacI^q$, $P_{Trc}$ promoter and terminator region from pTrcHis2A (Invitrogen, Carlsbad, CA) using primers
pTrc_F TTTCGCGAGGCCGGCCCCGCCAACACCCGCTGACG (SEQ ID NO:258) and
pTrc_R AAGGACGTCTTAATTAATCAGGAGAGCGTTCACCGACAA (SEQ ID NO:259)

The PCR product was then digested with *AatII* and *NruI* and insterted into plasmid pACYC177 (Rose, R.E., Nucleic Acids Res., 16:356 (1988)) digested with *AatII* and *ScaI.* The nucleotide sequence of the pACYC-$P_{Trc}$ vector is provided herein as SEQ ID NO: 233.

To generate the pACYC-$P_{Trc2}$ vector, a single point mutation was introduced in the $P_{Trc}$ promoter of the pACYC-$P_{Trc}$ vector to generate the variant promoter $P_{Trc2}$ and the pACYC-$P_{Trc2}$ vector. The wild-type $P_{Trc}$ promoter sequence is provided herein as SEQ ID NO:234, and the $P_{Trc2}$ variant promoter is provided herein as SEQ ID NO:235.

The nucleotide sequence encoding *E.coli* acyl-CoA thioesterase I (TesA, EC 3.1.1.5, 3.1.2.- ; *e.g.*, GenBank Accession AAC73596; SEQ ID NO:202) was modified to remove the leader sequence, such that the resulting *'tesA* gene product was truncated by 25 amino acids and the amino acid at the original position 26, alanine, was replaced with methionine, which then became the first amino acid of the 'TesA polypeptide (SEQ ID NO:204; Cho et al., J. Biol. Chem., 270:4216-4219 (1995)). DNA encoding the 'TesA polypeptide was inserted into the *NcoI* and *EcoRI* sites of the pACYC-$P_{Trc}$ vector and the pACYC-$P_{Trc2}$ vector, producing the pACYC-$P_{Trc}$-tesA and pACYC-$P_{TrC2}$-tesA plasmids, respectively. Correct insertion of *'tesA* sequence into the plasmids was confirmed by restriction digestion.

*C. acetobutylicum* phosphotransbutyrylase-butyrate kinase (pDG10 plasmid)

The plasmid pDG10 was prepared using the PCR-Blunt vector (Invitrogen, Carlsbad, CA) and a *C. acetobutylicum ptb_buk* operon insert, wherein the *ptb* part represents the gene encoding *C. acetobutylicum* phosphotransbutyrylase (GenBank Accession AAA75486.1, SEQ ID NO:227), and the *buk* part represents the gene encoding *C. acetobutylicum* butyrate kinase (GenBank Accession JN0795, SEQ ID NO:226). The *buk_ptb* operon was amplified from *C. acetobutylicum* (ATCC 824) genomic DNA using primers 5'-CTTAACTTCATGTGAAAAGTTTGT-3' (SEQ ID NO:260) and 5'-ACAATACCCATGTTTATAGGGCAA-3' (SEQ ID NO:261). The PCR product was ligated into the PCR-Blunt vector following the manufacturer's instructions.

**Example 3: Production of Branched Fatty Acids in *E. coli* Engineered to Express Exogenous *bkd* and *fabH* Genes**

The following *E. coli* strains were prepared as described above:

DV2'tesA (MG1655 *ΔfadE AtonA lacI:tesA*) is *E. coli* strain DV2 which in addition expresses a leaderless *'tesA* gene for production of fatty acids.

DV2'tesA + *BsfabH1* is the DV2'tesA strain transformed with the pDG6 plasmid expressing the *B. subtilis fabH1* gene.

DV2'tesA + *BsfabH1* + *Bsbkd* is the DV2'tesA strain transformed with the pDG6 plasmid expressing the *B. subtilis fabH1* gene, and the pKZ2 plasmid expressing the *B. subtilis bkd* operon.

DV2'tesA + *BsfabH1* + *Ppbkd* is the DV2'tesA strain transformed with the pDG6 plasmid expressing the *B. subtilis fabH1* gene, and the pKZ4 plasmid expressing the *P. putida bkd* operon.

Seed cultures were grown in LB supplemented with the appropriate antibiotics. After 4 hours of growth, the cultures were diluted 1:25 in Che-9 2NBT medium (2% glucose, nitrogen limited medium, 0.2 M Bis-Tris, pH 7.0, 0.1% Triton) + appropriate antibiotics and grown overnight. The cultures were then diluted in 4NBT (4% glucose, nitrogen limited medium, 0.2M Bis-Tris, pH 7.0, 0.1% Triton) to a final $OD_{600}$ ~0.2. After 6 hours of growth, IPTG was added to a final concentration of 1 mM. At 24 hours post-induction, 1 ml of culture was extracted with 500 μl ethyl acetate (containing 1% HCl), derivatized with freshly prepared TMAH and subjected to GC/MS analysis.

**Table 7:** Production of Branched Fatty Acids

| Strain | Total FFA titer | Total BCFA titer | Total BCFA / Total FFA | Total Anteiso-BCFA titer | Anteiso / Total BCFA |
|---|---|---|---|---|---|
| DV2'tesA | ~ 2000 | 0 | 0 | 0 | 0 |
| DV2'tesA + *BsfabH1* (pDG6) | ~ 2000 | 3 | .0015 | 0 | 0 |
| DV2'tesA + *BsfabH1* (pDG6) + *Ppbkd* (pKZ4) | 2130 | 580 | .27 | 100 | 0.17 |
| all titers are in milligrams per liter<br>FFA = free fatty acid; BCFA = branched chain fatty acid | | | | | |

Results:

*E. coli* does not normally produce branched-chain fatty acids. Figure 4(b) is a GC/MS analysis of free fatty acids (FFA) produced by the control *E. coli* strain (DV2'tesA) which expresses a thioesterase gene but lacks enzymes of parts (C) and (D) of the BCFA pathway, and which shows no detectable production of branched chain fatty acids.

Engineering the *E. coli* strain to also express an exogenous *fabH* gene, encoding a polypeptide having beta-ketoacyl-ACP synthase III activity that utilizes a branched acyl-CoA molecule as a substrate corresponding to part (D) of the BCFA pathway, resulted in the production of a barely detectable amount of branched-chain fatty acids, corresponding to less than about 2% of the total FFA produced (Figure 4(a)). The *E. coli* DV2'tesA strain produced about 2000 mg/L free fatty acids, with no detectable branched chain fatty acids, while the DV2'tesA + *BsfabH1* strain expressing the *BsfabH1* gene likewise produced about 2000 mg/L FFA, approximately 3 mg/L (< 2%) of which was branched-chain fatty acids, essentially all of which were in the iso-branched configuration (Table 7).

Branched fatty acid production increased dramatically when the *E. coli* strain was engineered to express *bkd* genes encoding polypeptides having branched-chain alpha-keto acid dehydrogenase activity, lipoamide acyltransferase activity, and dihydrolipoamide dehydrogenase activity (corresponding to part (C) of the BCFA pathway), along with the exogenous *fabH* gene. As can be seen in Figure 5, expression of the *B. subtilis bkd* genes together with the *B. subtilis fabH1* gene produced a variety of branched fatty acid structures, including branched fatty acids with chain lengths from C13 to C17 in iso-branched (denoted "i-") and anteiso-branched (denoted "a-") forms.

When *P. putida bkd* genes were expressed together with the *B. subtilis fabH1* gene, the resulting DV2'tesA + *BsfabH1* + *Ppbkd* strain likewise produced branched fatty acids with chain lengths from C13 to C17 in iso-branched and anteiso-branched forms (Figure 6). Approximately 27% (by weight) of the FFA produced by this strain were branched fatty acids; approximately 83% of those branched fatty acids were in the iso-form and approximately 17% of those branched fatty acids were in the anteiso-form (Table 7).

**Example 4. Engineering *E. coli* for Production of Anteiso-Branched Fatty Acids by Pathway (A.1)**

The following example describes the construction of recombinant *E. coli* strains which express exogenous genes and/or overexpress endogenous genes encoding enzymes which serve to increase metabolic flux through the intermediates $\alpha$-ketobutyrate, the anteiso-branched $\alpha$-keto acid intermediate $\alpha$-keto-$\beta$-methylvalerate, and the anteiso-branched chain primer 2-methylbutyryl-CoA by the (A.1) part of the pathway of Figure 3A, leading to the increased production of anteiso-branched acyl-ACP, and ultimately anteiso-branched fatty acid derivatives, in these recombinant cells.

This example also describes the effect of attenuating expression of an undesired endogenous gene on BCFA production. In this example, the *fabH* gene of *E. coli* encoding a beta-ketoacyl-ACP synthase III, which utilizes straight-chain acyl-CoA molecules instead of branched-chain acyl-CoA molecules, was attenuated by deletion of that gene. This example also describes the effect on BCFA production of chromosomally integrating an exogenous BKD operon (corresponding to part (C) of the BCFA pathway of Figures 1 and 3B).

DV2 $P_L$ *thrA*BC*

This example describes the construction of a recombinant *E. coli* strain in which one of the chromosomal genes involved in threonine biosynthesis was mutated and was placed under control of a chromosomally-integrated lambda $P_L$ promoter.

To introduce a single mutation in the native aspartokinase I (*thrA*) gene, the gene was amplified from *E.coli* MG1655 DNA in two parts. The first part was amplified using primers TREE026 and TREE028 while the second part was amplified using TREE029 and TREE030 (Table 8). The primers used to amplify the two components contained overlapping sequences which were then used to "stitch" the individual pieces together. The two PCR products were combined in a single PCR reaction and primers TREE026 and TREE030 to amplify the entire *thrA* gene. Primers TREE028 and TREE029 were designed to create a mutation in the native *thrA* at codon 345, which resulted in an S345F variant of aspartokinase I (SEQ ID NO: 118). Previous work has shown that this mutation eliminates feedback inhibition by threonine in the host strain (Ogawa-Miyata,Y., et al., Biosci. Biotechnol. Biochem. 65:1149-1154 (2001); Lee J.-H., et al., J. Bacteriol. 185: 5442-5451 (2003)). The modified version of this gene was designated *"thrA*".*

The $P_L$ promoter was amplified using primers Km_trc_overF and TREE027 (Table 8) using plasmid pDS80 (Example 2) as a template. This fragment was then stitched to a kanamycin resistance cassette flanked by FRT sites, which was amplified from plasmid pKD13 using primers TREE025 and Km_trc_overR (Table 8). The resulting PCR product containing the KmFRT cassette and $P_L$ promoter was stitched to the thrA* PCR product. Primers TREE025 and TREE030 were used to amplify the entire KmFRT-$P_L$-*thrA** mutagenic cassette. These primers also contain approximately 50 bp of homology to the integration site at the 5' end and the entire *thrA* gene as homology on the 3' end, targeting the cassette to the native *thrA* site in *E. coli,* which is part of an operon comprising the *thrA, thrB* and *thrC* genes. This mutagenic cassette was electroporated into the parental strain, *E.coli* DV2 (Example 1) containing the helper plasmid pKD46 expressing Red recombinase (Datsenko et al., *supra*). Clones containing the chromosomal integration were selected in the presence of kanamycin, and verified by diagnostic PCR. The kanamycin marker was then removed by expression of the pCP20 plasmid (Datsenko et al., *supra*). Proper integration and marker removal were verified by PCR and sequencing. The resulting strain , in which the mutant *thrA** gene and the the endogenous *thrB* and *thrC* genes were overexpressed by the chromosomally-integrated lambda $P_L$ promoter, was designated DV2 $P_L$ *thrA*BC.*

**Table 8**: Primers

| Primer | Sequence (5' → 3') | SEQ ID No: |
|---|---|---|
| TREE025 | CCTGACAGTGCGGGCTTTTTTTTTCGACCAAAGGTAACGAGGTAACAACC GTGTAGGCTGGAGCTGCTTCG | 262 |
| TREE026 | GTATATATTAATGTATCGATTAAATAAGGAGGAATAAACCATGCGAGTGT TGAAGTTCGGCG | 263 |
| TREE027 | CTGATGTACCGCCGAACTTCAACACTCGCATGGTTTATTCCTCCTTATTTAA TCGATAC | 264 |
| TREE028 | GCGCCCGTATTTTCGTGGTGCTGATTAC | 265 |
| TREE029 | GTAATCAGCACCACGTAAATACGGGCGC | 266 |
| TREE030 | TCAGACTCCTAACTTCCATGAGAGG | 267 |
| Km_trc_ove rR | AATATTTGCCAGAACCGTTATGATGTCGGCATTCCGGGGATCCGTCGACC | 268 |
| Km_trc_ove rF | CTTCGAACTGCAGGTCGACGGATCCCCGGAATGCCGACATCATAACGGTT CTGGC | 269 |
| EG238 | GCTGATCATTAACTATCCGCTGGATGACC | 270 |
| TREE017 | ACTGGAAAGCGGGCAGTGAGCGCAACGCAATTAATGTAAG | 271 |
| TREE018 | TCACTGCCCGCTTTCC | 272 |
| TREE019 | ACCGGCAGATCGTATGTAATATGCATGGTTTATTCCTCCTTATTTAATCGAT ACA | 273 |
| TREE020 | ATGCATATTACATACGATCTGCC | 274 |
| TREE021 | GGTCGACGGATCCCCGGAATTAAGCGTCAACGAAACCG | 275 |
| TREE022 | GAAGCAGCTCCAGCCTACACCAGACGATGGTGCAGGAT | 276 |
| TREE023 | GCAAAGACCAGACCGTTCATA | 277 |
| Kan/Chlor 1 | ATTCCGGGGATCCGTCGACC | 278 |
| Kan/Chlor 4 | TGTAGGCTGGAGCTGCTTCG | 279 |
| TREE025 | CCTGACAGTGCGGGCTTTTTTTTTCGACCAAAGGTAACGAGGTAACAACC GTGTAGGCTGGAGCTGCTTCG | 280 |
| TREE026 | GTATATATTAATGTATCGATTAAATAAGGAGGAATAAACCATGCGAGTGT TGAAGTTCGGCG | 281 |

To evaluate the effect of $P_L$ *thrA*BC* overexpression in DV2, the following three plasmids (described in Example 2) were transformed into this strain: pKZ4, which expressed the *P. putida* BKD operon; pDG6, which expressed *B. subtilis fabH1*; and pACYC-$p_{trc2}$-tesA, which expressed a truncated form of *E. coli tesA.* Shake flask fermentation experiments were conducted, and the titers of free fatty acids (FFA), branched fatty acids (BCFA), and anteiso-branched fatty acids (anteiso-BCFA), along with the fraction of FFA produced as BCFA and the fraction of BCFA produced as anteiso-BCFA, is provided in Table 10.

DV2 $P_L$ *thrA*BC* $P_L$ *tdcB*

The native *E.coli* catabolic threonine deaminase (*tdcB*) gene (also known as threonine ammonia-lyase) was overexpressed by integrating an extra copy of the gene into the *lacZ* locus and placing it under the control of a strong non-inducible promoter.

Catabolic threonine deaminase catalyzes the degradation of threonine to α-ketobutyrate (2-oxobutanoate), the first reaction of the threonine degradation/ isoleucine production pathway. The reaction catalyzed probably involves initial elimination of water (hence the enzyme's earlier identification as a threonine dehydratase), followed by isomerization and hydrolysis of the product with C-N bond breakage. Increased expression of this gene has been shown to dramatically increase levels of isoleucine in heterologous organisms (Guillouet S. et al., Appl. Environ. Microbiol. 65:3100-3107 (1999)). Furthermore, threonine deaminase is relatively resistant to isoleucine feedback mechanisms (Guillouet et *al., supra*).

*E.coli* MG1655 genomic DNA was amplified using primers TREE020 and TREE021 (Table 8) to obtain the native *tdcB* gene. At the same time, primers Chlor 1 and Chlor 4 (Table 8) were used to amplify an FRT-Kanamycin resistance cassette to be used for integration selection/screening as previously described. Using *E.coli* MG1655 genomic DNA as template, primers EG238 and TREE018 (Table 8) were used to amplify a region of homology 3' to the lacZ integration site, while primers TREE022 and TREE023 (Table 8) were used to amplify a region of homology 5' to the lacZ site. The plasmid pDS80 (Example 2) was used as a template to amplify a fragment containing the $P_L$ promoter by using primers TREE017 and TREE018 (Table 8). Each of these fragments were designed with overlaps for corresponding adjacent piece and were stitched together using SOEing PCR techniques. The resulting $P_L$ *tdcB* mutagenic cassette (approx. 4.3kb) contained approximately 700bp of homology to the integration site at the 5' end and 750bp of homology to the integration site at the 3' end. The $P_L$ *tdcB* mutagenic cassette was electroporated into the host strain, *E.coli* DV2 $P_L$ *thrA*BC* containing the helper plasmid, pKD46 (Datsenko *et al.*, *supra*). Clones containing the chromosomal integration were selected for in the presence of kanamycin, and verified by PCR and sequencing analysis. The kanamycin marker was then removed using the pCP22 plasmid (Datsenko *et al.*, *supra*). The resulting strain was designated DV2 $P_L$ *thrA*BC* $P_L$ *tdcB.*

To evaluate the effect of $P_L$ *tdcB* integration into DV2 $P_L$ *thrA*BC,* the following three plasmids (described in Example 2) were transformed into this strain: pKZ4, which expressed the *P. putida* BKD operon; pDG6, which expressed *B. subtilis fabH1;* and pACYC-$p_{trc2}$-tesA, which expressed a truncated form of *E. coli tesA.* Shake flask fermentation experiments were conducted, and the titers of free fatty acids (FFA), branched fatty acids (BCFA), and anteiso-branched fatty acids (anteiso-BCFA), along with the fraction of FFA produced as BCFA and the fraction of BCFA produced as anteiso-BCFA, is provided in Table 10.

DV2 $P_L$-*thrA*BC* $P_{T5}$-*BsfabH1*

This example describes the construction of a recombinant microbial cell in which the *B. subtilis fabH1* gene was integrated into the chromosome and placed under transcriptional control of the strong constitutive T5 promoter.

First, a PCR product was generated for the chromosomal integration of a loxPcat integration cassette comprising a chloramphenicol resistance gene, a T5 promoter ($P_{T5}$), and *BsfabH1* coding sequence, at the site of the *fadE* deletion scar of DV2 $P_L$ *thrA*BC.* The individual components of the integration cassette were first PCR-amplified. The loxP-cat-loxP $P_{T5}$ component was amplified from plasmid p100.38 (SEQ ID NO:237) using primers TREE133 and TREE135 (Table 9). The *BsfabH1* gene was amplified from a plasmid carrying the *BsfabH1* gene using primers TREE134 and TREE136. Primers TREE133 and TREE136 contain the 5' and 3' 50 bp of homology sequence for integration. The primers used to amplify the components contain overlapping sequence which were then used to "stitch" the individual pieces together. The loxP-cat-$P_{T5}$ and *BsfabH1* PCR products were stitched together by combining both pieces in a single PCR reaction and using primers TREE133 and TREE136 to amplify the final loxPcat-$P_{T5}$-BsfabH1 integration cassette.

**Table 9:** Primers

| Primer Name | Sequence | Purpose | SEQ ID NO: |
|---|---|---|---|
| TREE133 | AAAAACAGCAACAATGTGAGCTTTGTTGTAATTATATTG TAAACATATTGTCCGCTGTTTCTGCATTCTTACgt | Amplify loxPcat-T5 cassette | 282 |
| TREE134 | GATGACGACGAACACGCATTaagGAGGTGAATAAGGAG GAATAAcatATGAAAGCTGGCATTCTTGGTGTTG | Amplify BsfabH1 | 283 |

(continued)

| Primer Name | Sequence | Purpose | SEQ ID NO: |
|---|---|---|---|
| TREE135 | GTAACGTCCAACACCAAGAATGCCAGCTTTCATatgTTAT TCCTCCTTATTCACCTCcttAATGCGTGTTCG | Amplify loxPcat-T5 cassette | 284 |
| TREE136 | AAACGGAGCCTTTCGGCTCCGTTATTCATTTACGCGGCTT CAACTTTCCGTTATCGGCCCCAGCGGATTG | Amplify BsfabH1 | 285 |
| TREE137 | CGCAGTTTGCAAGTGACGGTATATAACCGAAAAGTGACT GAGCGTACatgATTCCGGGGATCCGTCGACC | Amplify EcfabH deletion cassette | 286 |
| TREE138 | GCAAATTGCGTCATGTTTTAATCCTTATCCTAGAAACGAA CCAGCGCGGATGTAGGCTGGAGCTGCTTCG | Amplify EcfabH deletion cassette | 287 |
| TREE139 | GCAGCGACAAGTTCCTCAGC | Verify deletion of EcfabH | 288 |
| TREE140 | CCGCAGAAGCTTCAGCAAACG | Verify deletion of EcfabH | 289 |
| fadE-L2 | CGGGCAGGTGCTATGACCAGGAC | Verify integration of BsfabH1 | 290 |
| fadE-R2 | GGGCAGGATAAGCTCGGGAGG | Verify integration of BsfabH1 | 291 |

The loxP-cat-$P_{T5}$-*BsfabH1* cassette was integrated using the Red recombinase system (Datsenko, *et al.*, *supra*). The loxP-cat-$P_{T5}$-*BsfabH1* PCR product was used to transform electrocompetent DV2 $P_L$-*thrA*BC* cells containing plasmid pKD46, which had been previously induced with arabinose for 3 - 4 hours at 30°C. Following a 3 hour 37°C outgrowth in SOC medium, cells were plated on Luria agar plates containing 17μg/mL chloramphenicol and incubated overnight at 37°C. Chloramphenicol-resistent colonies were screened by PCR for proper integration of loxP-cat-$P_{T5}$-*BsfabH1*. Primers fadE-L2 and fadE-R2 (Table 9) which flank the chromosomal integration site, were used to confirm the integration. Upon verification of integration, the chloramphenicol marker gene was removed by expressing a Cre recombinase which promotes recombination between the two loxP sites that flank the chloramphenicol resistance gene. The plasmid pJW168, which harbors the *cre* recombinase gene, was transformed into strain DV2 $P_L$-*thrA*BC* loxP-cat-$P_{T5}$-*BsfabH1* and the marker was removed according to the method described by Palmeros et al. (Gene 247:255-264 (2000)). The resulting strain DV2 $P_L$-*thrA*BC* $P_{T5}$-*BsfabH1* was verified by sequencing.

DV2 $P_L$-*thrA*BC* $P_{T5}$-*BsfabH1* Δ*EcfabH*

This example describes the construction of a recombinant *E. coli* cell in which the expression of an undesired endogenous gene (in this instance, the *fabH* gene of *E. coli,* encoding a beta-ketoacyl-ACP synthase III which utilizes straight-chain acyl-CoA molecules instead of branched-chain acyl-CoA molecules) was attenuated by deletion of that gene.

The *fabH* gene of *E.coli* was deleted from DV2 $P_L$-*thrA*BC* $P_{T5}$-*BsfcbH1* using the Red recombinase system (Datsenko *et al*., *supra*). Primers TREE137 and TREE138 (Table 9), were used to amplify the kanamycin resistance cassette from plasmid pKD13 by PCR. The PCR product was then used to transform electrocompetent DV2 $P_L$-*thrA*BC* $P_{T5}$-*BsfabH1* cells containing plasmid pKD46. Deletion of *EcfabH* and removal of the kanamycin marker were carried out according to the method described by Wanner and Datsenko, *supra.* Primers TREE139 and TREE140 were used to confirm the deletion of *EcfabH.* The final markerless strain was named DV2 $P_L$-*thrA*BC* $P_{T5}$-*BsfabH1* Δ*EcfabH.*

DV2 $P_L$-*thrA*BC* $P_L$-*tdcB* $P_{T5}$-*BsfabH1* Δ*EcfabH*

A recombinant *E. coli* strain was constructed containing chromosomally-integrated genes overexpressing enzymes of parts (A.1) and (D) of the anteiso-BCFA biosynthetic pathway of Figures 3A and 3B. The $P_L$-*tdcB* mutagenic cassette (prepared as described above) was integrated into strain DV2 $P_L$-*thrA*BC* $P_{T5}$-*BsfabH1* Δ*EcfabH* to generate the strain DV2 $P_L$-*thrA*BC* $P_L$-*tdcB* $P_{T5}$-*BsfabH1* Δ*EcfabH.* In this strain, the integrated *E. coli thrA*BC* genes and the integrated *E. coli tdcB* gene are both under the control of strong lambda $P_L$ promoters, and the integrated *B. subtilis*

*fabH1* gene is under the control of the strong T5 promoter. The endogenous *E. coli fadH* gene was deleted from this strain.

pOP80 plasmid

The pOP80 plasmid was constructed by digesting the cloning vector pCL1920 (GenBank AB236930; Lerner C.G. and Inouye M., Nucleic Acids Res. 18:4631 (1990)) with the restriction enzymes *AflII* and *SfoI.* Three DNA fragments were produced by this digestion. The 3737 bp fragment was gel-purified using a gel-purification kit (Qiagen, Inc., Valencia, CA). In parallel, a DNA sequence fragment containing the $P_{Trc}$ promoter and *lacI* region from the commercial plasmid pTrcHis2 (Invitrogen, Carlsbad, CA) was amplified by PCR using primers LF302 (5'-atatgacgtcGGCATCCGCTTACAGACA-3', SEQ ID NO:292) and LF303 (5'-aattcttaagTCAGGAGAGCGTTCACCGACAA-3', SEQ ID NO:293) introducing the recognition sites for the *ZraI* and *AflII* enzymes, respectively. After amplification, the PCR products were purified using a PCR-purification kit (Qiagen, Inc. Valencia, CA) and digested with *ZraI* and *AflII* following the recommendations of the supplier (New England BioLabs Inc., Ipswich, MA). After digestion, the PCR product was gel-purified and ligated with the 3737 bp DNA sequence fragment derived from pCL1920 to generate the expression plasmid pOP80 containing the $P_{Trc}$ promoter.

*C. glutamicum ilvA* plasmid

A plasmid was constructed which expresses the *ilvA* gene encoding a threonine deaminase from *Corynebacterium glutamicum,* and was tested for its suitability for use in part (A.1) of the anteiso-BCFA biosynthetic pathway of Figure 3A. The genomic DNA of *Corynebacterium glutamicum* was used to amplify the *ilvA* gene using the following primers:

ilvA_F TAAGGAGGAATAAACCATGAGTGAAACATACGTGTCTGAGA (SEQ ID NO:294)
ilvA_R CGGGCCCAAGCTTCGAATTTTATTAGGTCAAGTATTCGTACTCAGGG (SEQ ID NO:295)

The gene was inserted into the *NcoI* and *EcoRI* sites of plasmid OP80 (above). The plasmid was sequence verified, then transformed into DV2 $P_L$-*thrA*BC* $P_{T5}$-*BsfabH1* Δ*EcfabH*.

This strain was tested for branched fatty acid production against DV2 $P_L$-*thrA*BC* $P_L$-*tdcB* $P_{T5}$-*BsfabH1* Δ*EcfabH,* which has an integrated *tdcB* gene under control of the $P_L$ promoter. The strains were grown in FA-2 media following the protocol outlined below.

Shake flask fermentation and extraction (FA-2 media protocol)

Strains were evaluated for branched chain fatty acid production through shake flask fermentation. The standard FA-2 media protocol was generally used. In short, three individual colonies from a transformation were used to inoculate an LB + appropriate antibiotics overnight culture. The following morning, 50μL of the overnight cultures was used to inoculate 2mL LB + antibiotics seed cultures. After 3 - 4 hours of growth, the entire 2mL LB + antibiotics seed culture was transferred to 18mL of FA-2 media in 125mL baffled shake flasks. Cultures were induced with 1mM IPTG once the $OD_{600}$ reached 1.5 and samples were taken for extraction 20 - 22 hours post-induction. 400μL culture samples were acidified with 40μL 1N HCl and then extracted with 400μL of butyl acetate spiked with a 500mg/L C24 alkane internal standard. Extracts were derivatized with an equal volume of N,O-bis(trimethylsilyl)trifluoroacetamide (BSTFA) before being analyzed by GC/MS. The C24 alkane internal standard was used to quantify the free fatty acids (FFA) present in the samples.

**Table 10:** Production of Branched Fatty Acids

| | Strain | *Pp bkd* pKZ4 | *Bs fabH1* | *Ec fabH* | **Total FFA titer** | **Total BCFA titer** | **BCFA / Total FFA** | **Anteiso-BCFA titer** | **Anteiso / total BCFA** |
|---|---|---|---|---|---|---|---|---|---|
| 1 | DV2 | + | *p* | | 2008 | 533 | .27 | 66 | .12 |
| 2 | DV2 *thrA*BC* | + | *p* | | 1955 | 535 | .27 | 214 | .40 |
| 3 | DV2 *thrA*BC* | + | *int* | | 1908 | 651 | .34 | 245 | .38 |

(continued)

| | Strain | *Pp bkd* pKZ4 | *Bs fabH1* | *Ec fabH* | **Total FFA titer** | **Total BCFA titer** | **BCFA / Total FFA** | **Anteiso-BCFA titer** | **Anteiso / total BCFA** |
|---|---|---|---|---|---|---|---|---|---|
| 4 | DV2 *thrA*BC* | + | *int* | Δ | 1563 | 705 | .45 | 255 | .36 |
| 5 | DV2 *thrA*BC tdcB* | + | *p* | | 2012 | 589 | .29 | 334 | .57 |
| 6 | DV2 *thrA*BC tdcB* | + | *int* | Δ | 1470 | 918 | .62 | 609 | .66 |
| 7 | DV2 *thrA*BC Cg ilva* | + | *int* | Δ | 1257 | 704 | .56 | 513 | .73 |

all titers are in milligrams per liter
FFA = free fatty acid; BCFA = branched chain fatty acid
all strains also express the *'tesA* gene on plasmid pACYC-pTrc2-tesA *p* = plasmid-expressed *BsfabH1* (pDG6)
*int* = chromosomally integrated *BsfabH1* gene
*Δ* = deleted *E. coli* chromosomal *fabH* gene

Results:

Comparing strains 1 and 2, increasing the production of the anteiso-BCFA pathway intermediate threonine by overexpressing the *thrA*BC* genes significantly increased the proportion of anteiso-BCFA produced by the cells; about 12% (by weight) of the BCFA produced by strain 1 were in the anteiso-form, which increased in strain 2 to about 40% of the BCFA produced. On the other hand, the proportion of total BCFA produced by these cells remained fairly constant; about 27% of the FFA produced by each strain was in the branched-chain (BCFA) form.

Comparing strains 2 and 3 shows a slight improvement was obtained by chromosomally integrating a BCFA pathway gene. This example shows an increase in the amount and the proportion of BCFA produced by strain 3, in which the *BsfabH1* gene (encoding a polypeptide having branched chain beta-ketoacyl-ACP synthase III activity) was chromosomally integrated, compared to that produced by strain 2 containing the plasmid-expressed *BsfabH1* gene. The proportion of anteiso-BCFA produced by the strains containing chromosomally integrated and plasmid-expressed *BsFabH1* was relatively unchanged; about 38 to 40% of the total BCFA produced by these strains were anteiso-BCFA.

Comparing strains 3 and 4 demonstrates that attenuating an undesired endogenous gene that directs flux away from the BCFA pathway increases BCFA production. Strain 3 contained the endogenous *E. coli fabH* gene involved in straight-chain fatty acid production. Deletion of that gene from strain 4 significantly increased the amount of BCFA produced by that strain, increasing from about 34% of the FFA produced in branched form in strain 3, to about 45% of the FFA produced in branched form in strain 4. On the other hand, the proportion of anteiso-BCFA produced by these strains was relatively unchanged; in both of these strains, between 36% to 38% of the total BCFA was produced in the anteiso-form.

Comparing strains 1, 2, 5 and 6 shows that the proportion of anteiso-BCFA produced by a recombinant microbial cell is dramatically increased when cells are engineered to overexpress one more genes encoding endogenous or exogenous polypeptides having activities corresponding to the (A1) part of the anteiso-BCFA pathway. For instance, strain 1 (DV2), which did not overexpress any of the (A1) pathway activities, produced about 12% of BCFA in the anteiso-BCFA form. On the other hand, strain 2 (DV2 *thrA*BC*), which overexpressed polypeptides having aspartokinase activity, homoserine dehydrogenase activity, homoserine kinase activity, and threonine synthase activity, produced about 40% of BCFA in the anteiso-BCFA form. Strain 2 also shows that the native threonine deaminase activity present in the parental microbial cell was sufficient for production of anteiso-branched chain fatty acids by the anteiso-BCFA pathway shown in Fig. 3A.

Comparing strain 2 and strain 5 demonstrates that, although a native (i.e., unmodified) level of *E. coli* threonine deaminase activity was sufficient for anteiso-BCFA production in strain 2, increasing that activity by overexpressing an endogenous threonine deaminase enzyme further increased anteiso-BCFA production. While strain 2 produced about 40% of BCFA in the anteiso-form, Strain 5. (DV2 *thrA*BC tdcB*), which was identical to strain 2 except it also overexpressed a polypeptide having threonine deaminase activity encoded by the *E. coli tdcB* gene, produced about 57% of BCFA in the anteiso-form.

Comparing strains 5 and 6 further demonstrates the effect of manipulating beta-ketoacyl-ACP synthase III activity (step (D) of the BCFA pathway) on BCFA production. Both strains 5 and 6 overexpress the *thrA*BC* and *tdcB* genes. By deleting the endogenous *E.coli FabH* gene (described above in the context of strain 4) and chromosomally integrating the exogenous *BsFabH1* gene (described above in the context of strain 3) the proportion of BCFA produced was nearly doubled, from about 30% of the FFA by strain 5 to over 60% of the FFA by strain 6. In this instance strain 6 also showed an increase in the proportion of anteiso-BCFA produced (from about 57% of BCFA by strain 5, to about 66% of BCFA by strain 6), which was albeit a less dramatic relative increase than in the proportion of BCFA produced.

Comparing strains 6 and 7 demonstrates that an enzyme that catalyzes a particular pathway reaction can be substituted by a different enzyme which catalyzes the same pathway reaction. In this example, an endogenous enzyme encoded by the *E. coli tdcB* gene that was overexpressed in strain 6 was substituted in strain 7 by an exogenous enzyme encoded by the *C. glutamicum ilvA* gene. The *E. coli tdcB* gene encodes a catabolic threonine deaminase, while the *C. glutamicum ilvA* gene encodes an anabolic threonine deaminase. Both of these enzymes catalyze the conversion of threonine to $\alpha$-ketobutyrate (i.e., 2-oxobutanoate) and both are classified under EC 4.3.1.19. Although these enzymes are derived from different sources and are encoded by different genes, Table 10 shows that both of these enzymes are suitable for use in a recombinant microbial cell to carry out the conversion of threonine to $\alpha$-ketobutyrate in the anteiso-BCFA pathway described herein: strain 6 produced about 66% of BCFA in the anteiso-form, while strain 7 produced over 70% of BCFA in the anteiso-form. This result not only confirms that the *C. glutamicum* anabolic threonine deaminase is suitable for use in a recombinant microbial cell to catalyze the conversion of threonine to $\alpha$-ketobutyrate according to the anteiso-BCFA pathway, it demonstrates that a pathway enzyme (such as, a pathway enzyme described herein) which catalyzes a particular pathway reaction can be "functionally replaced" in the recombinant microbial cell by a different enzyme which catalyzes the same reaction.

**Example 5. Engineering *E. coli* for Production of Anteiso-Branched Fatty Acids by Pathway (A.2).**

The following example describes the construction of recombinant *E. coli* strains which express exogenous genes and/or overexpress endogenous genes encoding enzymes which serve to increase metabolic flux through the intermediates $\alpha$-ketobutyrate, the anteiso-branched $\alpha$-keto acid intermediate $\alpha$-keto-$\beta$-methylvalerate, and the anteiso-branched chain primer 2-methylbutyryl-CoA by the (A.2) part of the BCFA pathway of Figure 3A, leading to the increased production of anteiso-branched acyl-ACP, and ultimately anteiso-branched fatty acid derivatives, in these recombinant cells.

This example also describes the construction of plasmids which express a *fabH* gene from *Listeria monocytogenes* and a novel mutant *L. monocytogenes fabH* gene, which provide alternative beta-ketoacyl-ACP synthase III enzymes for part (D) of the BCFA biosynthetic pathways of Figures 1 and 3.

DV2 $P_{Trc}$-*cimA3.7 leuBCD*

To prepare an *E. coli* strain overexpressing endogenous *leuBCD* genes and an exogenous *cimA3.7* gene, a PCR product was generated for the chromosomal integration of a KmFRT cassette, a $P_{Trc}$ promoter, and *cimA3.7* between the endogenous chromosomal *E. coli leuA* and *leuB* genes. This integration disrupted the native *leuABCD* operon, placing *cimA3.7* and *leuBCD* in an operon under control of the strong IPTG-inducible promoter, $P_{Trc}$.

DNA encoding CimA3.7 was synthesized by Geneart AG (Regensburg, Germany). The DNA was cloned into the SfiI site of plasmid pMK-RQ (kanR) (Geneart AG, Regensburg, Germany). Flanking the coding sequence, a 5' KpnI restriction site and a 3' SacI restriction site were introduced directly upstream of the ATG start codon and immediately downstream of the TAA stop codon respectively. The cimA 3.7 cloning vector was verified by sequencing.

The individual components of the integration cassette were PCR-amplified as follows. The KmFRT component was amplified from plasmid pKD13 using primers TREE146 and Km_trc_overR (Table 11). The $P_{Trc}$ promoter was amplified from pOP80 (Example 4) using primers Km_trc_overF and TREE033.

The *cimA3.7* coding sequence was amplified from the cimA 3.7 cloning vector described above using primers TREE032 and TREE035. To provide the 3' homology sequence for integration, *E. coli* native *leuBC* genes were amplified using *E. coli* genomic DNA and primers TREE034 and TREE104. The forward primer TREE146, which was used to amplify the KmFRT cassette, included the 5' 50bp of homology sequence for integration. Each of the primers used to amplify the components contained overlapping sequence which were used to "stitch" the individual pieces together. First, KmFRT and $P_{Trc}$ were stitched together by combining both pieces in a single PCR reaction and using primers TREE146 and TREE033 to amplify the KmFRT-$P_{Trc}$ product. KmFRT-$P_{Trc}$ was then stitched with *cimA3.7* using primers TREE146 and TREE035 to generate KmFRT-$P_{Trc}$-*cimA3.7.* The final piece, *leuBC* was stitched to KmFRT-$P_{Trc}$-*cimA3.7* using primers TREE146 and TREE104 to generate the final integration cassette: KmFRT-$P_{Trc}$-*cimA3.7 leuBC.*

**Table 11:** Primers

| Primer Name | Primer Sequence (5' --> 3') | Purpose | SEQ ID NO: |
|---|---|---|---|
| Km_trc_overF | CTTCGAACTGCAGGTCGACGGATCCCCGGAATGCCG ACATCATAACGGTTCTGGC | Amplify pTrc promoter | 296 |
| Km_trc_overR | AATATTTGCCAGAACCGTTATGATGTCGGCATTCCG GGGATCCGTCGACC | Amplify KmFRT cassette | 297 |
| TREE032 | GTATATATTAATGTATCGATTAAATAAGGAGGAATA AACCatgatggtaaggatatttgatacaacac | Amplify cimA3.7 | 298 |
| TREE033 | ctaagtgttgtatcaaatatccttaccatcatGGTTTATTCCTCC TTATTTAATCGATAC | Amplify pTrc promoter | 299 |
| TREE034 | gatttgttggctatagttagagaagttactggaaaattgTAACAAG GAAACCGTGTGATGTCGAAG | Amplify leuBC | 300 |
| TREE035 | GTAATTCTTCGACATCACACGGTTTCCTTGTTAcaattt tccagtaacttctctaactatag | Amplify cimA3.7 | 301 |
| TREE104 | GGTAGCGAAGGTTTTGCCCGGC | Amplify leuBC | 302 |
| TREE106 | GATTGGTGCCCCAGGTGACCTG | Verify integration | 303 |
| TREE146 | GAGTTGCAACGCAAAGCTCAACACAACGAAAACAAC AAGGAAACCGTGTGaGTGTAGGCTGGAGCTGCTTCG | Amplify KmFRT cassette | 304 |
| TREE151 | CTTCCACGGCGTCGGCCTG | Verify integration | 305 |

The KmFRT-$P_{Trc}$-*cimA3.7 leuBC* cassette was integrated into the *E. coli* genome using the Red recombinase system (Datsenko *et al., supra*). The KmFRT-$P_{Trc}$-*cimA3.7 leuBC* PCR product was used to transform electrocompetent *E. coli* MG1655 DV2 cells containing plasmid pKD46, which had been previously induced with arabinose for 3 - 4 hours at 30°C. Following a 3-hour 37°C outgrowth in SOC medium, cells were plated on Luria agar plates containing 50μg/mL kanamycin and incubated overnight at 37°C. Kanamycin-resistant colonies were screened by PCR for proper integration of KmFRT-$P_{Trc}$-*cimA3.7.* Primers TREE151 and TREE106, which flank the chromosomal integration site, were used to confirm the integration. Upon verification of integration, the kanamycin marker gene was removed in accordance with the method described by Datsenko *et al., supra.* Successful integration of $P_{Trc}$-*cimA3.7* and removal of the kanamycin marker gene in the final strain, DV2 $P_{Trc}$ *cimA3.7 leuBCD,* was verified by sequencing.

Strains were transformed with the plasmids pDG6, which expressed *BsfabH1;* pKZ4, which expressed *PpBKD;* and pACYC-$P_{Trc2}$-*tesA* which expressed the leaderless *E. coli 'tesA,* as indicated, and tested for branched chain fatty acid production.

*L. monocytogenes fabH1 and fabH2* (pTB.079 and pTB.081 plasmids)

The genomic DNA of *Listeria monocytogenes* Li23 (ATCC 19114D-5) was used as template to amplify the *fabH* gene using the following primers:

TREE044 (fabH_forward) GAGGAATAAACCATGAACGCAGGAATTTTAGGAGTAG (SEQ ID NO:256); primer 61 (fabH_reverse)

CCCAAGCTTCGAATTCTTACTTACCCCAACGAATGATTAGG (SEQ ID NO:257)

The PCR product was then cloned into the NcoI/EcoRI sites of pDS80 (a pCL1920-based vector carrying the phage lambda $P_L$ promoter; SEQ ID NO:236) and transformed into transformation-competent *E. coli* cells. Individual colonies were picked for sequence verification of cloned inserts. The nucleic acid sequence of wild type *L. monocytogenes fabH* (SEQ ID NO:99) encodes the wild type LmFabH1 protein (SEQ ID NO:98), and the plasmid containing this sequence was designated pTB.079.

A mutant *L. monocytogenes fabH* gene was discovered containing a T to G change at position 928, resulting in a change in the expressed protein at amino acid position 310 from Tryptophan (W) to Glycine (G), i.e., a W310G variant. The novel mutant *L. monocytogenes fabH* gene (SEQ ID NO:101) encoding the FabH W310G variant was designated LmFabH2 (SEQ ID NO:100), and the plasmid containing this sequence pTB.081.

Plasmids containing the wild type *LmfabH1* gene (pTB.079) and the mutant *LmfabH2* gene (pTB.081) were transformed into the DV2 $P_{Trc}$-*cimA3.7_leuBCD*/pACYCtrc2_tesA strain. The strains were transformed with pKZ4 (*P. putida* BKD) and pDG6 (*B subtilis* fabH1) plasmids and evaluated for BCFA production.

**Table 12:** Production of Branched Fatty Acids

| | Strains | *bkd* | *fabH* | **Total FFA titer** | **Total BCFA titer** | **BCFA / Total FFA** | **Anteiso-BCFA titer** | **Anteiso / total BCFA** |
|---|---|---|---|---|---|---|---|---|
| 1 | DV2 | *Pp* | *BsH1* | 2008 | 533 | .27 | 66 | .12 |
| 2 | DV2 *cimA3.7 leuBCD* | (-) | (-) | 3764 | 0 | 0 | 0 | 0 |
| 3 | DV2 *cimA3.7 leuBCD* | *Pp* | (-) | 2691 | 6 | .002 | 0 | 0 |
| 4 | DV2 *cimA3.7 leuBCD* | *Pp* | *BsH1* | 1945 | 522 | .27 | 362 | .69 |
| 5 | DV2 *cimA3.7 leuBCD* | *Pp* | *LmH* | 322 | 122 | .38 | 91 | .75 |
| 6 | DV2 *cimA3.7 leuBCD* | *Pp* | *LmH2* | 1597 | 419 | .26 | 385 | .92 |

all titers are in milligrams per liter
all strains also express the *'tesA* gene on plasmid pACYC-$p_{Trc2}$-tesA
FFA = free fatty acid; BCFA = branched chain fatty acid
Pp = plasmid-expressed BKD operon from *P. putida* (pKZ4)
*BsH1* = plasmid-expressed *B. subtilis* FabH1 (pDG6)
*LmH1* = plasmid-expressed *L. monocytogenes* FabH1 (pTB.079)
*LmH2* = plasmid-expressed *L. monocytogenes* FabH2 W310G (pTB.081)

Results:

Together with the data described in Example 3 above, strains 2 and 3 of Table 12 demonstrate that little if any BCFA is produced in microbial cells lacking branched chain alpha-keto acid dehydrogenase (BKD) activities and/or beta-ketoacyl-ACP synthase III (e.g., FabH) activity specific for branched-chain substrates, corresponding to steps (C) and (D) of the BCFA pathway of Figure 1.

Comparing strains 1 and 4 shows the effect of engineering activities corresponding to part (A.2) of the BCFA pathway in recombinant microbial cells on the production of anteiso-BCFA . Strains 1 and 4 produced nearly identical amounts and proportions of BFCA (about 27% of the total FFA produced in these cells were branched fatty acids), however, strain 1 produced primarily iso-BCFA, with only about 12% of the total BCFA being in the anteiso-form. On the other hand, strain 4, expressing genes encoding polypeptides having (R)-citramalate synthase activity, isopropylmalate isomerase activity, and beta-isopropyl malate dehydrogenase activity (corresponding to part (A.2) of the BCFA pathway) produced substantially more anteiso-BCFA, with nearly 70% of the total BCFA in the anteiso-form.

Comparing strains 4, 5 and 6 shows that a variety of polypeptides having branched chain beta-ketoacyl-ACP synthase III activity can be utilized for producing branched chain fatty acids in recombinant microbial cells. More particularly, this example shows that the *L. monocytogenes* wild type FabH and a novel W310G variant of *L. monocytogenes* FabH have activities suitable for use in the BCFA pathway. Comparing FFA produced by cultures of these three strains, which are identical except for the *fabH* genes employed, shows that strain 5 expressing *B. subtilis* FabH1 produced about 27% of total FFA in branched form, with almost 70% of those branched fatty acids in the anteiso-form; strain 6 expressing wild-type *L. monocytogenes* FabH produced about 38% of total FFA in branched form, with about 75% of of those branched fatty acids in anteiso-form; and strain 7 expressing a *L. monocytogenes* W310G variant FabH (designated FabH2) produced about 26% of total FFA in branched form, with, remarkably, over 90% of those branched fatty acids in the anteiso-form.

**Example 6. Production of Anteiso-Branched Fatty Acids in *E. coli* by Pathways A.1 and A.2. Combined**

The following example describes the construction of recombinant *E. coli* strains which express exogenous genes and/or overexpress endogenous genes encoding enzymes which serve to increase metabolic flux through the intermediates α-ketobutyrate, the anteiso-branched α-keto acid intermediate α-keto-β-methylvalerate, and the anteiso-branched chain primer 2-methylbutyryl-CoA by the combined (A.1) and (A.2) parts of the pathway of Figure 3A, leading to even greater production of anteiso-branched acyl-ACP, and ultimately anteiso-branched fatty acid derivatives, in these recombinant cells.

This example also describes the construction of a plasmid which expresses *bkd* genes from *Listeria monocytogenes,* which provides another example of branched-chain alpha-keto acid dehydrogenase (BKD) complex enzymes suitable for use in part (C) of the BCFA biosynthetic pathway of Figure 1.

DV2 $P_L$-*thrA*BC* $P_{Trc}$-*cimA3.7 leuBCD* $P_{T5}$-*BsfabH1* Δ*EcfabH* (strain "G1")

To begin combining the (A.1) and (A.2) parts of the anteiso-BCFA pathway of Figure 3A, the $P_{Trc}$-*cimA3.7_leuBCD* cassette (Example 5) was integrated into strain DV2 $P_L$-*thrA*BC* $P_{T5}$-*BsfabH1* Δ*EcfabH* (Example 4) to generate the strain DV2 $P_L$-*thrA*BC* $P_{Trc}$-*cimA3.7_leuBCD* $P_{T5}$-*BsfabH1* Δ*EcfabH,* which was also called strain G1. This strain overexpressed polypeptides having (R)-citramalate synthase activity, isopropylmalate isomerase activity, and beta-isopropyl malate dehydrogenase activity according to the (A.2) part of the anteiso-BCFA pathway, and overexpressed polypeptides having aspartokinase activity, homoserine dehydrogenase activity, homoserine kinase activity, and threonine synthase activity according to the (A.1) part of the part of the anteiso-BCFA pathway.

DV2 $P_L$-*thrA*BC* $P_L$-*tdcB* $P_{Trc}$-*cimA3.7 leuBCD* $P_{T5}$-*BsfabH1* Δ*EcfabH* (strain "G2")

To create a strain engineered to overexpress polypeptides having activities corresponding to the combined (A.1) and (A.2) parts of the anteiso-BCFA pathway, the $P_L$-*tdcB* cassette (Example 4) was integrated into strain G1, to generate strain DV2 $P_L$-*thrA*BC* $P_L$-*tdcB* $P_{Trc}$-*cimA3.7_leuBCD* $P_{T5}$-*BsfabH1* Δ*EcfabH,* which was also called strain G2. In this strain, the integrated *E. coli thrA*BC* genes and the integrated *E. coli tdcB* gene (encoding polypeptides having aspartokinase activity, homoserine dehydrogenase activity, homoserine kinase activity, threonine synthase activity, and threonine deaminase activity, corresponding to the (A.1) part of the BCFA pathway) were placed under the control of strong lambda $P_L$ promoters, and were as such overexpressed. The exogenous *cimA3.7* gene and the native *E. coli leuBCD* genes (encoding polypeptides having (R)-citramalate synthase activity, isopropylmalate isomerase activity, and beta-isopropyl malate dehydrogenase activity corresponding to the (A.2) part of the BCFA pathway), were also integrated into the *E. coli* chromosome under control of the strong IPTG-inducible promoter $P_{Trc}$ and therefore were also overexpressed. The integrated *B. subtilis fabH1* gene, encoding a branched chain beta ketoacyl-ACP synthase III corresponding to part (C) the pathway, was under the control of the strong T5 promoter. The endogenous *E. coli fadH* gene was deleted from this strain.

Plasmid pTB85 (expressing the *L. monocytogenes* BKD complex)

The genomic DNA of *Listeria monocytogenes* Li23 (ATCC 19114D-5) was used for amplification of the *bkd* genes using the following primers:

primer81 (BKD_for) GAGGAATAAACCGTGGCAACAGAATATGATGTCGTTATTCT (SEQ ID NO:306)
primer82 (BKD_rev) CCCAAGCTTCGAATTTTAATACAATGCTGTATTTTCTTTGGAAAT (SEQ ID NO:307)

The *Lmbkd* PCR product was cloned into the NcoI and EcoRI sites of pGL10.173B (SEQ ID NO:228) under the control of the $P_{trc}$ promoter. The sequence-verified plasmid was transformed into strain G1 (above). The strains were also transformed with pACYC-$P_{Trc2}$-tesA (leaderless *E. coli* TesA) pKZ4 (*P. putida* BKD) and pDG6 (*B subtilis fabH1*) plasmids and evaluated for BCFA production using the FA-2 media protocol as described in Example 4.

Evaluation of BCFA production

To test for BCFA production, strains DV2 $P_L$-*thrA*BC* $P_{T5}$-*BsfabH1,* DV2 $P_L$-*thrA*BC* $P_{T5}$-*BsfabH1* Δ*EcfabH,* DV2 $P_L$-*thrA*BC* $P_L$-*tdcB* $P_{T5}$-*BsfabH1* Δ*EcfabH,* G1, and G2 were transformed with plasmids pKZ4, which expresses *PpBKD,* and pACYC-$P_{Trc2}$-*tesA*, which expresses the leaderless *E. coli 'tesA.* Strain DV2 $P_L$-*thrA*BC* transformed with plasmids pKZ4, pACYC-$P_{Trc2}$-*tesA*, and pDG6 (which expresses *BsfabH1*) served as a control for these experiments. For comparison, fatty acid titers and compositions produced by production strain DV2 and strains engineered to overexpress polypeptides having activities corresponding to the (A.1) pathway or the (A.2) pathway can be found in **Tables 10** and **12**, above.

**Table 13:** Production of Branched Fatty Acids

| | Strain | *bkd* | *fabH* | **Total FFA titer** | **Total BCFA titer** | **BCFA / Total FFA** | **Anteiso-BCFA titer** | **Anteiso / total BCFA** |
|---|---|---|---|---|---|---|---|---|
| 1 | DV2 *thrA*BC* | *Pp* | *Int BsH1 ΔEc* | 1563 | 705 | .45 | 255 | .36 |
| 2 | DV2 *thrA*BC tdcB* | *Pp* | *Int BsH1 ΔEc* | 1470 | 918 | .62 | 609 | .66 |
| 3 | DV2 *thrA*BC cimA3.7 leuBCD (G1)* | *Pp* | *Int BsH1 ΔEc* | 1483 | 880 | .59 | 741 | .84 |
| 4 | DV2 *thrA*BC cimA3.7 leuBCD* | *Lm* | *Int BsH1 ΔEc* | 830 | 95 | .11 | 83 | .87 |
| 5 | DV2 *thrA*BC tdcB cimA3.7 leuBCD (G2)* | *Pp* | *Int BsH1 ΔEc* | 1429 | 702 | .49 | 633 | .90 |

all titers are in milligrams per liter
all strains also express the *'tesA* gene on plasmid pACYC-$p_{Trc2}$-tesA
FFA = free fatty acid; BCFA = branched chain fatty acid
Pp = plasmid-expressed *P. putida* BKD operon
Lm = plasmid-expressed *L. monocytogenes* BKD operon
*int BsH1* = chromosomally integrated *BsfabH1* gene
*ΔEc* = deleted *E. coli* chromosomal *fabH* gene

Results:

As was previously noted in Example 4, strain DV2 *thrA*BC tdcB* (strain 2 in Table 13 above), which overexpressed polypeptides having aspartokinase activity, homoserine dehydrogenase activity, homoserine kinase activity, threonine synthase activity and threonine deaminase activity (according to the (A.1) part of the anteiso-BCFA pathway) and a polypeptide having branched chain beta-ketoacyl-ACP synthase III activity by a chromosomally integrated *BsfabH1* gene, produced about two-thirds (66%) of its branched chain fatty acids in the anteiso-form.

Comparing strain 2 to strain 3 (DV2 *thrA*BC cimA3.7 leuBCD,* also denoted "strain G1"), which overexpressed polypeptides having (R)-citramalate synthase activity, isopropylmalate isomerase activity, and beta-isopropyl malate dehydrogenase activity (according to the (A.2) part of the anteiso-BCFA pathway) in addition to polypeptides having aspartokinase activity, homoserine dehydrogenase activity, homoserine kinase activity, and threonine synthase activity, the amount and proportion of BCFA produced was comparable (about 59% of FFA produced as BCFA in strain 2, compared to about 62% of FFA produced as BCFA in strain 3), but the proportion of anteiso-BCFA was much greater in strain 3 (G1) than in strain 2, such that about 84% of the BCFA produced by strain 3 was in the anteiso-form compared to 66% in strain 2.

Comparing strains 3 and 4 shows the effect of different BKD enzyme complexes on BCFA and anteiso-BCFA production. Strain 3 expressed *bkd* genes from *P. putida* while strain 4 expressed *bkd* genes from *L. monocytogenes.* Although the strain expressing the *L. monocytogenes bkd* genes showed a lower overall production (titer) of both FFA and BCFA than the strain expressing the *P. putida bkd* genes, the proportions of anteiso-branched fatty acids produced by these strains were remarkably consistent, with each strain producing about 85% of the branched-chain fatty acids in the anteiso-form.

Comparing strain 3 to strain 5 (DV2 *thrA*BC tdcB cimA3.7 leuBCD*, also denoted "strain G2"), which is identical to strain 3 except for also overexpressing threonine deaminase, about 90% of the BCFA produced by strain 5 was in the anteiso-form, compared to about 84% in strain 3, which utilized the host cell's native threonine deaminase activity.

Taken together, the data obtained from strain 3 (G1) and strain 5 (G2) indicates that engineering a microbial cell which is capable of producing branched chain fatty acids (owing to the presence of BKD and branched chain beta-ketoacyl-ACP synthase activities) to express or overexpress polypeptides having (R)-citramalate synthase activity, isopropylmalate isomerase activity, and beta-isopropyl malate dehydrogenase activity (according to the (A.2) part of the anteiso-BCFA pathway) together with polypeptides having aspartokinase activity, homoserine dehydrogenase activity, homoserine kinase activity, threonine synthase activity, and optionally threonine deaminase activity (according to the (A.1) part of the anteiso-BCFA pathway) not only results in the production of compositions comprising anteiso-branched chain fatty acids, but compositions in which over 80% of the branched fatty acids produced are in the anteiso-form.

**Example 7: Production of Branched Fatty Esters in *E. coli***

To produce branched chain fatty methyl esters and branched chain fatty ethyl esters, *E. coli* strain DV2 (Example 1) is transformed with plasmids pKZ4 (expressing *P. putida bkd* genes), pDG6 (expressing *B. subtilis fabH1*), and a plasmid which expresses the ester synthase polypeptide *Marinobacter hydrocarbonoclasticus* DSM 8798 ester synthase ES9 (GenBank Accession No. ABO21021; SEQ ID NO:308)

A polynucleotide encoding ES9 is synthesized by DNA2.0 (Menlo Park, CA), is subjected to restriction digestion with *BspHI and XhoI,* and cloned into plasmid pOP80 (Example 4) also digested with *BspHI and XhoI,* resulting in a plasmid expressing ES9 under the control of the $P_{Trc}$ promoter.

Individual colonies of DV2 transformed with plasmids pKZ4, pDG6, and the ES9 plasmid are used to inoculate an overnight culture of LB + appropriate antibiotics. The following morning, 50μL of the overnight cultures are used to inoculate 2mL LB + antibiotics seed cultures. After 4 h of growth, the cultures are diluted 1 :25 in Che-9 2NBT media containing the appropriate antibiotics and grown overnight. The cultures are diluted in 4NBT to a final OD600 (opticalal density at 600 nm) of about 0.2. After 6 h of growth, IPTG is added to the culture at a final concentration of 1 mM, and methanol or ethanol to 2% (v/v). At 24 h post-induction, 1 ml of culture is extracted with 500 μl ethyl acetate (containing 1% HCl), derivatized with freshly prepared TMAH and subjected to GC-MS analysis.

An *E. coli* DV2 strain expressing an ES9 ester synthase polypeptide and transformed with plasmids pKZ4 and pDG6, which was cultured essentially as described above and was supplemented with methanol, produced a variety of straight-chain and branched fatty acid methyl esters (FAME). The branched chain FAME detected included iso-$C_1C_{12:0}$, iso-$C_1C_{13:0}$, anteiso-$C_1C_{13:0,}$ iso-$C_1C_{14:0}$, iso-$C_1C_{15:0}$, anteiso-$C_1C_{15:0}$, iso-$C_1C_{16:0}$, iso-$C_1C_{17:0}$ and anteiso-$C_1C_{17}$:0 methyl esters. About 31% of the FAME produced were branched FAME. About 74% of the branched FAME were iso-branched FAME and about 26% were anteiso-branched FAME (Table 14).

When the culture was supplemented with ethanol, a variety of straight chain and branched chain fatty acid ethyl esters (FAEE) were produced. The branched chain FAEE detected included iso-$C_2C_{12:0}$, iso-$C_2C_{13:0}$, anteiso-$C_2C_{13:0}$, iso-$C_2C_{14:0}$, iso-$C_2C_{15:0}$, anteiso-$C_1C_{15:0}$, iso-$C_2C_{16:0}$, iso-$C_2C_{17:0}$ and anteiso-$C_2C_{17:0}$ ethyl esters. About 22% of the FAEE produced were branched FAEE. About 81% of those branched FAEE were iso-branched FAEE, and about 26% were anteiso-branched FAEE (Table 14).

**Table 14:** Production of Branched Fatty Esters

| | Fatty Ester (FE) produced | Total FE titer | Total Branched Fatty Ester (BFE) titer | BFE / total FE | Anteiso-BFE titer | Anteiso-BFE / total BFE |
|---|---|---|---|---|---|---|
| 1 | Fatty acid methyl esters (FAME) | 232 | 73 | 0.31 | 19 | 0.26 |
| 2 | Fatty acid ethyl esters (FAEE) | 325 | 72 | 0.22 | 14 | 0.19 |
| all titers are in milligrams per liter | | | | | | |

**Example 8. Production of Branched Fatty Esters in *Bacillus***

*B. subtilis* cells expressing an ester synthase from *Marinobacter hydrocarbonoclasticus* DSM 8798 ester synthase ES9 (GenBank Accession No. ABO21021) produce branched fatty esters.

A polynucleotide sequence encoding the ES9 ester synthase polypeptide (SEQ ID NO:308) is cloned into *B. subtilis* expression vector pHT01 (MoBiTec GmbH, Goettingen, Germany). Vector pHT01 is an *Escherichia coli - Bacillus subtilis* shuttle vector that carries the strong promoter Pgrac for protein expression in *B. subtilis.* The ES9 coding sequence is inserted between the *BamHI* and *XbaI* cloning sites. A *B. subtilis* strain 1HA01 (*lacA::spec leuB8 metB5 r(-)m(+) Sp ;* obtained from Bacillus Genetic Stock Center, Columbus, OH, Strain Number BGSC 1A785) is transformed with pHT01_ES9 according to the protocol of Anagnostopoloulos and Spizizen (J. Bacteriol. 1961, 81:741) with the following modifications:

*B. subtilis* 1HA01 cells are grown at 37°C in the miminal medium as described in Anagnostopoloulos and Spizizen (*supra*), supplemented with 50 μg/mL methionine (auxotrophic requirements) for 5 hours, until the OD600 reaches 0.6 to 1.0. To each 1 mL culture, 15 μL of plasmid (1-2 μg of DNA) is added and cells are allowed to grow for another 90 minutes at 37°C. Cells are pelleted by centrifugation. The supernatant is removed and discarded and the cells are resuspended in 100 μL LB and plated onto LB agar plates containing 10 μg/mL chloramphenicol. Single colonies are picked from the resulting transformants and used to prepare freezer stocks, and tested for branched fatty ester production.

*B. subtilis* transformed with the pHT01_ES9 vector produces branched methyl esters (when the culture is supplemented with methanol) or branched ethyl esters (when the culture is supplemented with ethanol), including branched esters of C13, C15 and C17 chain lengths.

**Example 9: Production of Branched Alkanes in *E. coli***

Branched alkanes are produced by a recombinant microbial cell of the invention which expresses polynucleotides encoding polypeptides having fatty acid derivative enzyme activity, wherein the fatty acid derivative enzyme activity is hydrocarbon biosynthesis activity. The following example demonstrates the production of branched alkanes by a strain which expresses a polypeptide having acyl-ACP reductase (AAR) activity and a polypeptide having aldehyde decarbonylase (ADC) activity. The AAR activity converts the branched acyl-ACP intermediate to a branched aldehyde and the ADC activity converts the branched aldehyde to a branched alkane.

To produce branched alkanes, the *Synechococcus elongatus* PCC7942 *aar gene,* which encodes a fatty acyl-ACP reductase (GenBank Accession No. YP_400611; SEQ ID NO: 309) was integrated into *E.coli* strain MG1655 Δ*fadE* Δ*tonA* (strain DV2; Example 1) to produce strain MG1655 Δ*fadE* Δ*tonA AAR:kan* as follows: A polynucleotide encoding the *aar* gene controlled by a $P_{trc}$ promoter and flanked by a partial *lacI* gene and a kanamycin resistance cassette was amplified from plasmid pSL67-78A (SEQ ID NO:315) using primers AAR_F (5'-GGCT GGCTGG CATAAAT ATCTC-3'; SEQ ID NO:310) and AAR_R (5'- GTTATGATAT GTTGGTCGGATA AGCGTCGCGCCGCA TCCGACATTGATTGC GAG AGC GTT CAC CGA CAA-3'; SEQ ID NO:311) and integrated between the *lacI* and *lacA* genes using the Red recombinase system (Datsenko, *et al.*, *supra*). The resulting strain was named SL106A. Strain SL106A was transformed with plasmid pTB38, which encodes aldehyde decarbonylase (ADC) from *Nostoc punctiforme* PCC73102 (GenBank Accession No. YP_001865325; SEQ ID NO: 312) under the control of the $P_{trc}$ promoter and contains a spectinomycin resistance cassette.

The strain was then transformed with plasmids pKZ4 (expressing *P. putida bkd* genes) and pKZ5 (expressing *B. subtilis fabH1*) and evaluated for branched alkane production. The shake flask protocol using Che-9 media (Example 7) was followed. At 24 hour post induction, 1 mL of culture was extracted with 0.5 mL ethyl acetate (containing 1% HCl) and subjected to GC/MS analysis.

A variety of straight chain and branched alkanes were produced (Table 15). The branched alkanes detected included iso-C14:0, anteiso-C14:0, iso-C16:0, and anteiso-C16:0 alkanes. About 14% of the alkanes produced were branched alkanes. About 54% of the branched alkanes were iso-branched alkanes, and about 46% were anteiso-branched alkanes (Table 15).

**Table 15:** Production of Branched Alkanes

| | Strain | Total alkane titer | Total branched chain (BC) alkane titer | BC alkane / total alkane | Anteiso-BC alkane titer | Anteiso-BC / total BC alkane titer |
|---|---|---|---|---|---|---|
| 1 | AAR, ADC, BsfabH1, PpBKD | 109 | 15 | 0.14 | 6.9 | 0.46 |

**Example 10: Production of Branched Fatty Alcohols in *E. coli***

The *Synechococcus elongatus* PCC7942 *aar* gene, which encodes a fatty acyl-ACP reductase (GenBank Accession No. YP_400611; SEQ ID NO: 309) was integrated into the chromosome of *E.coli* strain MG1655 *ΔfadE_ΔtonA* as described in Example 9. The resulting *E.coli* strain MG1655 *ΔfadE_ΔtonA AAR:kan* was transformed with with plasmids pKZ4 (expressing *P. putida bkd* genes) and pDG6 (expressing *B. subtilis fabH1*). The strain was evaluated for production of branched chain alcohols using shake flask fermentation.

Cultures of *E. coli* MG1655 *ΔfadE_ΔtonA AAR:kan* without plasmids, or carrying individual plasmids, were used as controls. Seed cultures were grown in LB broth supplemented with the appropriate antibiotics. After 4 hours of growth, the cultures were diluted 1:25 in Che-9 2NBT medium + appropriate selection marker and grown overnight. The cultures are then diluted in 4NBT to a final OD600 ~0.2. After 6 hours of growth, IPTG was added to a final concentration of 1 mM. At 24 hours post-induction, 1 ml of culture was extracted with 0.5 mL of methyl tert-butyl ether (MTBE) and subjected to GC/MS analysis. Figure 7(A) shows that iso-branched and anteiso-branched C14-C17 fatty alcohols were produced by the recombinant microbial strain expressing a fatty acyl-ACP reductase (AAR), a branched chain alpha-keto acid dehydrogenase (BKD) complex, and a branched chain-specific β-ketoacyl-ACP synthase III (FabH). Figure 7(B) shows that branched fatty alcohols were not produced by the recombinant microbial strain expressing AAR but not BDK nor the branched chain-specific FabH.

**Example 11: Identification and Quantification of Branched Fatty Acid Derivatives**

*Instrumentation:*

The instrument is an Agilent 5975B MSD system equipped with a 30 mx0.25 mm (0.10 μm film) DB-5 column. The mass spectrometer is equipped with an electron impact ionization source. Two GC/MS programs were utilized.

GC/MS program #1: The temperature of the column is held isothermal at 90ºC for 5 min, then is raised to 300ºC with a 25ºC/min ramp, and finally stays at 300ºC for 1.6 min. The total run time is 15 min. With this program, the inlet temperature is hold at 300ºC. The injector is set at splitless mode. 1 μL of sample is injected for every injection. The carrier gas (helium) is released at 1.0 mL/min. The source temperature of the mass spectrometer is held at 230ºC.

GC/MS program #2: The temperature of the column is held isothermal at 100ºC for 3 min, then is raised to 320ºC with 20ºC/min, and finally stays isothermal at 320ºC for 5 min. The total run time is 19 min. The injector is set at splitless mode. 1 μL of sample is injected for every injection. The carrier gas (helium) is released at 1.2 mL/min. The ionization source temperature is set at 230ºC.

*Samples:*

Extracts containing branched fatty acids, branched fatty acid derivatives, and/or branched alkanes produced by the engineered E.coli strains were analyzed on GC/MS. As described in Example 7 above, various branched-chain fatty acids, fatty acid derivatives, such as fatty esters, and branched alkanes were detected.

GC/MS semi-quantitative analysis:

In addition to the qualitative analysis, semi-quantitative analysis was performed to obtain the ratio between the branched chain compounds and the straight chain isomers.

*Standards:*

A mixture of bacterial acid methyl ester (BAME, Sigma-Aldrich, Cat #: 47080-U 10 mg/mL total concentration) contains the following 26 compounds:

Methyl undecanoate
Methyl (±)-2-hydroxydecanoate
Methyl dodecanoate
Methyl tridecanoate
Methyl 2-hydroxydodecanoate
Methyl (±)-3-hydroxydodecanoate
Methyl myristate
Methyl 13-methyltetradecanoate
Methyl 12-methyltetradecanoate
Methyl pentadecanoate
Methyl 2-hydroxytetradecanoate
Methyl 3-hydroxytetradecanoate
Methyl 14-methylpentadecanoate
Methyl cis-9-hexadecenoate

Methyl palmitate
Methyl 15-methylhexadecanoate
Methyl *cis*-9,10-methylenehexadecanoate
Methyl heptadecanoate
Methyl 2-hydroxyhexadecanoate
Methyl linoleate
Methyl oleate
Methyl trans-9-octadecenoate
Methyl stearate
Methyl cis-9,10-methyleneoctadecanoate
Methyl nonadecanoate
Methyl eicosenoate

Among these compounds, there are 4 branched FAMEs along with their straight chain isomers: *iso*-$C_1C_{15:0}$, *anteiso*-$C_1C_{15:0}$ and *n*-$C_1C_{15:0}$; *iso*-$C_1C_{16:0}$ and *n*-$C_1C_{16:0}$; *iso*-$C_1C_{17:0}$ and *n*-$C_1C_{17:0}$. This mixture was diluted 4 fold with ethyl acetate so that each compound in the mixture has a concentration at around 100 mg/L. The diluted mixture was then analyzed by GC/MS to provide qualitative information for all the branched chain acyl compounds produced.

BAME standards were analyzed using GC/MS. The data sheet which provides the GC eluting sequence of all 26 components in the BAME mixture was obtain from a SPB-1 phase column. The retention time (RT) of these compounds analyzed with these two GC programs are listed in the table below. These retention times were used to identify the branched compounds produced by the recombinant microbial strains.

**Table 16: Retention Times of BAME Standards**

| Compounds | RT at GC program #1, min | RT at GC program #2, min |
|---|---|---|
| *iso*-$C_1C_{15:0}$ | 11.37 | 9.73 |
| *anteiso*-$C_1C_{15:0}$ | 11.41 | 9.77 |
| *n*-$C_1C_{15:0}$ | 11.53 | 9.94 |
| *iso*-$C_1C_{16:0}$ | 11.8 | 10.27 |
| *n*-$C_1C_{16:0}$ | 12.0 | 10.46 |
| *iso*-$C_1C_{17:0}$ | 12.21 | 10.79 |
| *n*-$C_1C_{17:0}$ | 12.36 | 10.97 |

With these retention times, the identification and quantification of the exact compounds measured above were possible. However, the engineered *E. coli* strains were expected to produce branched compounds with chain lengths other than those listed, including, for example, $C_7$, $C_8$, $C_9$, $C_{10}$, $C_{11}$, $C_{12}$, $C_{13}$, or $C_{14}$ compounds. Without commercially available standards, the identification of their structures would have been problematic.

Two approaches were taken. In the first approach, the relative RT of branched chain compounds vs. the straight chain isomers were determined. It was found that the straight chain *n*-C1C15:0 compound was retained in the column with the longest time, and the *iso*-C1C15:0 compound was retained in the column with the shortest time among the three isomers. This trend was consistent with the fact that the DB-5 column used in the GC separates volatile compounds based on the boiling point of the compounds. Compounds with higher boiling points typically have longer retention time than compounds with lower boiling points. It was known that the boiling points of branched chain compounds are lower than those of their straight-chain isomer counterparts. This information was used as a qualitative tool to assign the structure of isomers with different chain lengths.

In the second approach, mass spectra of *iso*-$C_1C_{15:0}$, *anteiso*-$C_1C_{15:0}$ and *n*-$C_1C_{15:0}$ isomers were obtained. Because the radical formed by the fragmentation between C12 and C11 is very stable, the spectra of *iso*- and *n*- $C_1C_{15:0}$ appeared nearly identical, wheras the spectrum of *anteiso*-$C_1C_{15:0}$ was substantially different at 199 m/z. Combining the information obtained from the two approaches, the structure of the branched chain products could be reliably predicated.

Using these methods, it was found that the following branched fatty acid branched fatty acid derivatives (e.g., branched fatty acids, alcohols, esters and hydrocarbons) could be detected using GC/MS and the methods described herein (Table 17).

**Table 17**

| | |
|---|---|
| Fatty acid | iso-$C_{12:0}$, iso-$C_{13:0}$, anteiso-$C_{13:0}$, iso-$C_{14:0}$, iso-$C_{15:0}$, anteiso-$C_{15:0}$, iso-$C_{16:0}$, iso-$C_{17:0}$, anteiso-$C_{17:0}$ |
| Fatty alcohol | iso-$C_{14:0}$, iso-$C_{15:0}$, anteiso-$C_{15:0}$, iso-$C_{16:0}$, iso-$C_{17:0}$, anteiso-$C_{17:0}$ |
| FAME | iso-$C_{12:0}$, iso-$C_{130}$, anteiso-$C_{13:0,}$ iso-$C_{14:0}$, iso-$C_{15:0}$, anteiso-$C_{15:0}$, iso-$C_{16:0}$, iso-$C_{17:0}$, anteiso-$C_{17:0}$ |
| FAEE | iso-$C_{12:0}$, iso-$C_{13:0}$, anteiso-$C_{13:0}$, iso-$C_{14:0}$, iso-$C_{15:0}$, anteiso-$C_{15:0}$, iso-$C_{16:0}$, iso-$C_{17:0}$, anteiso-$C_{17:0}$ |
| 3-OH-FAEE | iso-$C_{13:0}$, anteiso-$C_{13:0}$, iSO-$C_{15:0}$, anteiso-$C_{15:0}$, |
| alkane | iso-$C_{14:0}$, anteiso-$C_{14:0}$, iso-$C_{16:0}$, anteiso-$C_{16:0}$ |

*Semiquantitative measurements of yield*

Due to the often lack of commercially available standards for various branched fatty acids, branched fatty acid derivatives and/or branched hydrocarbons, accurate quantitation for the branched chain compounds was challenging. However, by using straight chain standard with the same functional group, the relative quantity or yield of branched-chan compounds in relation to the yield of their straight-chain counterpart (isomers) were estimated semi-quantitatively.

Standard curve quantitation method was applied, wherein standard mixtures with different concentrations were analyzed by the same GC/MS program as the samples. After data acquisition, the instrument response (total ion current) was plotted against the concentrations of the standards. Linear calibration curves were obtained. The concentration of branched alcohols in a given sample was calculated according to the equation y = ax+b, wherein y is the instrument response for a particular compound in a sample. Accordingly, the relative concentration of branched compounds in the production mixture was calculated.

Table 18 lists the fatty methyl esters used as standards to quantify various branched fatty acid methyl esters.

**Table 18**

| FAME compound in sample | Standard used for quantitation |
|---|---|
| iso-$C_1C_{12:0}$ | $C_1C_{12:0}$ |
| $C_1C_{12:0}$ | $C_1C_{12:0}$ |
| Iso-$C_1C_{13:0}$ | $C_1C_{13:0}$ |
| Anteiso-$C_1C_{13:0}$ | $C_1C_{13:0}$ |
| $C_1C_{13:0}$ | $C_1C_{13:0}$ |
| Iso-$C_1C_{14:0}$ | $C_1C_{14:0}$ |
| $C_1C_{14:1}$ | $C_1C_{14:1}$ |
| $C_1C_{14:0}$ | $C_1C_{14:0}$ |
| Iso-$C_1C_{15:0}$ | $C_1C_{15:0}$ |
| Anteiso-$C_1C_{15:0}$ | $C_1C_{15:0}$ |
| $C_1C_{15:0}$ | $C_1C_{15:0}$ |
| Iso-$C_1C_{16:0}$ | $C_1C_{16:0}$ |
| $C_1C_{16:1}$ | $C_1C_{16:1}$ |
| $C_1C_{16:0}$ | $C_1C_{16:0}$ |
| Iso-$C_1C_{17:0}$ | $C_1C_{16:0}$ |
| Anteiso-$C_1C_{17:0}$ | $C_1C_{16:0}$ |
| $C_1C_{18:1}$ | $C_1C_{18:1}$ |
| $C_1C_{18:0}$ | $C_1C_{18:0}$ |

Table 19 lists fatty ethyl esters used as standards to quantify various branched fatty acid ethyl esters.

**Table 19**

| FAEE compound in sample | Standard used for quantitation |
|---|---|
| $C_2C_{8:0}$ | $C_2C_{8:0}$ |
| $C_2C_{10:0}$ | $C_2C_{10:0}$ |
| Iso- $C_2C_{12:0}$ | $C_2C_{12:0}$ |
| $C_2C_{12:1}$ | $C_2C_{12:0}$ |
| $C_2C_{12:0}$ | $C_2C_{12:0}$ |
| Iso- $C_2C_{13:0}$ | $C_2C_{12:0}$ |
| Anteiso-$C_2C_{13:0}$ | $C_2C_{12:0}$ |
| Iso-$C_2C_{14:0}$ | $C_2C_{14:0}$ |
| $C_2C_{14:1}$ | $C_2C_{14:0}$ |
| $C_2C_{14:0}$ | $C_2C_{14:0}$ |
| Iso-$C_2C_{15:0}$ | $C_2C_{14:0}$ |
| Anteiso-$C_2C_{15:0}$ | $C_2C_{14:0}$ |
| Iso- $C_2C_{16:0}$ | $C_2C_{16:0}$ |
| $C_2C_{16:1}$ | $C_2C_{16:0}$ |
| $C_2C_{16:0}$ | $C_2C_{16:0}$ |
| Iso- $C_2C_{17:0}$ | $C_2C_{16:0}$ |
| Anteiso-$C_2C_{17:0}$ | $C_2C_{16:0}$ |
| $C_2C_{18:1}$ | $C_2C_{18:0}$ |
| $C_2C_{18:0}$ | $C_2C_{18:0}$ |

Branched free fatty acids and various other fatty acid derivatives were analyzed using the standard listed below (Table 20):

**Table 20**

| Acyl compounds in sample | Standard used for quantitation |
|---|---|
| straight chain alcohol | $C_{15:0}$ alcohol |
| branched chain alcohol | |
| aldehyde | |
| Free fatty acid | |

Alternatively, a $C_1C_{14:0}$ fatty acid methyl ester was used as a standard for quantitating the derivitized branched free fatty acids in the extract from any production strain (Table 21). The measured concentrations were then converted back to branched free fatty acid concentrations based on their molecular weights.

**Table 21**

| FFA compound in sample (derivatized into FAME) | FAME Standard used for quantitation |
|---|---|
| staight chain FFA | $C_1C_{14:0}$ |
| branched chain FFA | |

Branched alkanes were measured using the following standards (Table 22), which were also used to verify the amount of branched fatty aldehydes or branched fatty alcohols.

**Table 22**

| Alkane, aldehyde and alcohol in sample | Standard used for quantitation |
|---|---|
| Alk $C_{13:0}$ | Alk $C_{12:0}$ |
| Iso-Alk $C_{14:0}$ | Alk $C_{15:1}$ |
| Anteiso-Alk $C_{14:0}$ | Alk $C_{15:1}$ |
| Alk $C_{14:0}$ | Alk $C_{15:1}$ |
| Alk $C_{15:0}$ | Alk $C_{16:1}$ |
| Iso-Alk $C_{16:0}$ | Alk $C_{16:1}$ |
| Anteiso-Alk $C_{16:0}$ | Alk $C_{16:1}$ |
| Ald $C_{14:0}$ | Alc $C_{15:0}$ |
| Alk $C_{16:0}$ | Alk $C_{16:1}$ |
| Alc $C_{16:0}$ | Alc $C_{15:0}$ |
| Alk $C_{17:1}$ | Alk $C_{17:0}$ |
| Iso-Alc $C_{15:0}$ | Alc $C_{15:0}$ |
| Anteiso-Alc $C_{15:0}$ | Alc $C_{15:0}$ |
| Alc $C_{15:0}$ | Alc $C_{15:0}$ |
| Aid $C_{16:0}$ | Alc $C_{15:0}$ |
| Alc $C_{16:0}$ | Alc $C_{15:0}$ |

For a given composition of fatty acid derivative produced, the percentage of the derivative that was produced in the branched chain form was determined according to the equation:

$$\text{Percentage of branched derivative} = 100 \times \frac{\text{(Total branched derivative product in mg/L)}}{\text{(Total branched + straight-chain derivative product in mg/L)}}$$

Likewise, for a given composition of fatty acid derivative produced, the percentage of the branched-chain derivative that was produced in the anteiso-branched chain form was determined according to the equation:

$$\text{Percentage of anteiso-branched derivative} = 100 \times \frac{\text{(Total anteiso-branched derivative product in mg/L)}}{\text{(Total branched derivative product in mg/L)}}$$

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Further aspects and embodiments of the invention are:**

1. A recombinant microbial cell comprising:

(a) polynucleotides encoding a branched chain alpha-keto acid dehydrogenase (BKD) complex which comprises polypeptides having branched-chain alpha-keto acid dehydrogenase activity, lipoamide acyltransferase activity, and dihydrolipoamide dehydrogenase activity,
(b) a polynucleotide encoding a polypeptide having beta-ketoacyl-ACP synthase activity that utilizes a branched acyl-CoA molecule as a substrate, and
(c) one or more polynucleotides encoding a polypeptide having fatty acid derivative enzyme activity,

wherein at least one polynucleotide according to (a) or (b) encodes a polypeptide that is exogenous to the recombinant microbial cell or wherein expression of at least one polynucleotide according to (a) or (b) is modulated in the recombinant microbial cell as compared to the expression in a corresponding wild-type microbial cell, and
wherein the recombinant microbial cell produces a branched fatty acid derivative when cultured in the presence of a carbon source under conditions effective to express the polynucleotides.
2. The recombinant microbial cell of item 1, wherein expression of the at least one polynucleotide according to (a) or (b) is modulated by overexpression of the at least one polynucleotide in the recombinant microbial cell.
3. The recombinant microbial cell of item 1, which produces a fatty acid derivative composition comprising straight-chain fatty acid derivatives and branched fatty acid derivatives, wherein at least 5% of the fatty acid derivatives in the composition are branched fatty acid derivatives, when cultured in the presence of a carbon source under conditions effective to express the polynucleotides.
4. The recombinant microbial cell of item 3, wherein at least 50% of the branched fatty acid derivatives in the composition are iso-branched fatty acid derivatives.
5. The recombinant microbial cell of item 1, which produces at least 25 mg/L branched fatty acid derivatives when cultured in the presence of a carbon source under conditions effective to express the polynucleotides.
6. The recombinant microbial cell of item 1, further comprising :

(d) polynucleotides encoding polypeptides having aspartokinase activity, homoserine dehydrogenase activity, homoserine kinase activity, threonine synthase activity, and threonine deaminase activity, and/or
(e) polynucleotides encoding polypeptides having (R)-citramalate synthase activity, isopropylmalate isomerase activity, and beta-isopropylmalate dehydrogenase activity, and further comprising
(f) polynucleotides encoding polypeptides having acetohydroxyacid synthase activity, acetohydroxyacid isomeroreductase activity, and dihydroxy acid dehydratase activity;

wherein at least one polynucleotide according to (d), (e), and/or (f) encodes a polypeptide that is exogenous to the recombinant microbial cell or wherein expression of at least one polynucleotide according to (d), (e), and/or (f) is modulated in the recombinant microbial cell as compared to the expression in a corresponding wild-type microbial cell, and
wherein the recombinant microbial cell produces an anteiso-branched fatty acid derivative when cultured in the presence of a carbon source under conditions effective to express the polynucleotides.
7. The recombinant microbial cell of item 6, wherein expression of the at least one polynucleotide according to (c), (d), and/or (e) is modulated by overexpression of the at least one polynucleotide in the recombinant microbial cell.
8. The recombinant microbial cell of item 6, wherein the recombinant microbial cell produces a fatty acid derivative composition comprising straight-chain fatty acid derivatives and branched fatty acid derivatives, wherein at least 10% of the branched fatty acid derivatives in the composition are anteiso-branched fatty acid derivatives.
9. The recombinant microbial cell of item 1, which comprises an endogenous polynucleotide sequence encoding a polypeptide having beta-ketoacyl-ACP synthase activity that does not utilize a branched acyl-CoA molecule as a substrate, wherein expression of the endogenous polynucleotide sequence encoding a polypeptide having beta-ketoacyl-ACP synthase activity in the recombinant microbial cell is attenuated.
10. The recombinant microbial cell of item 1, wherein the recombinant microbial cell is a member of the genus *Escherichia, Bacillus, Lactobacillus, Pantoea, Zymomonas, Rhodococcus, Pseudomonas, Aspergillus, Trichoderma, Neurospora, Fusarium, Humicola, Rhizomucor, Kluyveromyces, Pichia, Mucor, Myceliophtora, Penicillium, Phanerochaete, Pleurotus, Trametes, Chrysosporium, Saccharomyces, Stenotrophamonas, Schizosaccharomyces, Yarrowia, Streptomyces, Synechococcus, Chlorella,* or *Prototheca.*
11. A cell culture comprising the recombinant microbial cell of item 1.
12. The recombinant microbial cell of item 1, wherein the fatty acid derivative enzyme activity comprises thioesterase activity and the branched fatty acid derivative is a branched fatty acid, and
wherein the recombinant microbial cell produces a fatty acid composition comprising straight-chain fatty acids and branched fatty acids, wherein at least 5% of the fatty acids in the composition are branched fatty acids, when cultured in the presence of a carbon source under conditions effective to express the polynucleotides.
13. The recombinant microbial cell of item 12, which produces at least 25 mg/L branched fatty acids when cultured in the presence of a carbon source under conditions effective to express the polynucleotides.

14. The recombinant microbial cell of item 6, wherein the fatty acid derivative enzyme activity comprises thioesterase activity and the anteiso-branched fatty acid derivative is an anteiso-branched fatty acid, and
wherein the recombinant microbial cell produces a fatty acid composition comprising straight-chain fatty acids and branched fatty acids, wherein at least 10% of the branched fatty acids in the composition are anteiso-branched fatty acids, when cultured in the presence of a carbon source under conditions effective to express the polynucleotides.
15. The recombinant microbial cell of item 14, which produces at least 10 mg/L anteiso-branched fatty acids when cultured in the presence of a carbon source under conditions effective to express the polynucleotides.
16. The recombinant microbial cell of item 1, wherein the fatty acid derivative enzyme activity comprises ester synthase activity and the branched fatty acid derivative is a branched fatty ester, and
wherein the recombinant microbial cell produces a fatty ester composition comprising straight-chain fatty esters and branched fatty esters, wherein at least 5% of the fatty esters in the composition are branched fatty esters, when cultured in the presence of a carbon source under conditions effective to express the polynucleotides.
17. The recombinant microbial cell of item 6, wherein the fatty acid derivative enzyme activity comprises ester synthase activity and the anteiso-branched fatty acid derivative is an anteiso-branched fatty ester, and
wherein the recombinant microbial cell produces a fatty ester composition comprising straight-chain fatty esters and branched fatty esters, wherein at least 10% of the branched fatty esters in the composition are anteiso-branched fatty esters, when cultured in the presence of a carbon source under conditions effective to express the polynucleotides.
18. The recombinant microbial cell of item 1, wherein the fatty acid derivative enzyme activity comprises fatty aldehyde biosynthesis activity and the branched fatty acid derivative is a branched fatty aldehyde, and
wherein the recombinant microbial cell produces a fatty aldehyde composition comprising straight-chain fatty aldehydes and branched fatty aldehydes, wherein at least 5% of the fatty aldehydes in the composition are branched fatty aldehydes, when cultured in the presence of a carbon source under conditions effective to express the polynucleotides.
19. The recombinant microbial cell of item 6, wherein the fatty acid derivative enzyme activity comprises fatty aldehyde biosynthesis activity and the anteiso-branched fatty acid derivative is an anteiso-branched fatty aldehyde, and
wherein the recombinant microbial cell produces a fatty aldehyde composition comprising straight-chain fatty esters and branched fatty esters, wherein at least 10% of the branched fatty esters in the composition are anteiso-branched fatty aldehydes, when cultured in the presence of a carbon source under conditions effective to express the polynucleotides.
20. The recombinant microbial cell of item 1, wherein the fatty acid derivative enzyme activity comprises fatty alcohol biosynthesis activity and the branched fatty acid derivative is a branched fatty alcohol, and
wherein the recombinant microbial cell produces a fatty alcohol composition comprising straight-chain fatty alcohols and branched fatty alcohols, wherein at least 5% of the fatty alcohols in the composition are branched fatty alcohols, when cultured in the presence of a carbon source under conditions effective to express the polynucleotides.
21. The recombinant microbial cell of item 6, wherein the fatty acid derivative enzyme activity comprises fatty alcohol biosynthesis activity and the anteiso-branched fatty acid derivative is an anteiso-branched fatty alcohol, and
wherein the recombinant microbial cell produces a fatty alcohol composition comprising straight-chain fatty alcohols and branched fatty alcohols, wherein at least 10% of the branched fatty alcohols in the composition are anteiso-branched fatty alcohols, when cultured in the presence of a carbon source under conditions effective to express the polynucleotides.
22. The recombinant microbial cell of item 1, wherein the fatty acid derivative enzyme activity comprises hydrocarbon biosynthesis activity and the branched fatty acid derivative is a branched hydrocarbon, and
wherein the recombinant microbial cell produces a hydrocarbon composition comprising straight-chain hydrocarbons and branched hydrocarbons, wherein at least 5% of the hydrocarbons in the composition are branched hydrocarbons, when cultured in the presence of a carbon source under conditions effective to express the polynucleotides.
23. The recombinant microbial cell of item 6, wherein the fatty acid derivative enzyme activity comprises hydrocarbon biosynthesis activity and the anteiso-branched fatty acid derivative is an anteiso-branched hydrocarbon, and
wherein the recombinant microbial cell produces a hydrocarbon composition comprising straight-chain hydrocarbons and branched hydrocarbons, wherein at least 10% of the branched hydrocarbons in the composition are anteiso-branched hydrocarbons, when cultured in the presence of a carbon source under conditions effective to express the polynucleotides.
24. A method of making a composition comprising a branched fatty acid derivative, the method comprising:

obtaining the recombinant microbial cell of item 1,
culturing the recombinant microbial cell in a culture medium containing a carbon source under conditions effective to express the polynucleotides and produce a fatty acid derivative composition comprising straight-chain fatty acid derivatives and branched fatty acid derivatives wherein at least 5% of the fatty acid derivatives in the composition are branched fatty acid derivatives, and
optionally recovering the composition from the culture medium.

25. The method of item 24, wherein the fatty acid derivative composition produced in the culture medium comprises at least 25 mg/L branched fatty acid derivatives.
26. The method of item 24, wherein the recombinant microbial cell expresses one or more polypeptides having a fatty acid derivative enzyme activity selected from the group consisting of:

(1) a polypeptide having thioesterase activity;
(2) a polypeptide having decarboxylase activity;
(3) a polypeptide having carboxylic acid reductase activity;
(4) a polypeptide having alcohol dehydrogenase activity (EC 1.1.1.1);
(5) a polypeptide having aldehyde decarbonylase activity (EC 4.1.99.5);
(6) a polypeptide having acyl-CoA reductase activity (EC 1.2.1.50);
(7) a polypeptide having acyl-ACP reductase activity;
(8) a polypeptide having ester synthase activity (EC 3.1.1.67);
(9) a polypeptide having OleA activity; and
(10) a polypeptide having OleCD or OleBCD activity;

wherein the recombinant microbial cell produces a composition comprising branched fatty acids, branched fatty esters, branched fatty aldehydes, branched fatty alcohols, branched alkanes, branched alkenes, branched alkenes, branched terminal olefins, branched internal olefins, or branched ketones.
27. A method of making a composition comprising an anteiso-branched fatty acid derivative, the method comprising:

obtaining the recombinant microbial cell of item 3,
culturing the recombinant microbial cell in a culture medium containing a carbon source under conditions effective to express the polynucleotides and produce a fatty acid derivative composition comprising straight-chain fatty acid derivatives and branched fatty acid derivatives wherein at least 10 % of the branched fatty acid derivatives in the composition are anteiso-branched fatty acid derivatives, and
optionally recovering the composition from the culture medium.

28. The method of item 27, wherein the branched fatty acid derivative composition produced in the culture medium comprises at least 10 mg/L anteiso-branched fatty acid derivatives.
29. The method of item 27, wherein the recombinant microbial cell expresses one or more polypeptides having a fatty acid derivative enzyme activity selected from the group consisting of:

(1) a polypeptide having thioesterase activity;
(2) a polypeptide having decarboxylase activity;
(3) a polypeptide having carboxylic acid reductase activity;
(4) a polypeptide having alcohol dehydrogenase activity (EC 1.1.1.1);
(5) a polypeptide having aldehyde decarbonylase activity (EC 4.1.99.5);
(6) a polypeptide having acyl-CoA reductase activity (EC 1.2.1.50);
(7) a polypeptide having acyl-ACP reductase activity;
(8) a polypeptide having ester synthase activity (EC 3.1.1.67);
(9) a polypeptide having OleA activity; and
(10) a polypeptide having OleCD or OleBCD activity;

wherein the recombinant microbial cell produces a composition comprising anteiso-branched fatty acids, anteiso-branched fatty esters, anteiso-branched wax esters, anteiso-branched fatty aldehydes, anteiso-branched fatty alcohols, anteiso-branched alkanes, anteiso-branched alkenes, anteiso-branched internal olefins, anteiso-branched terminal olefins, or anteiso-branched ketones.

**Claims**

**1.** A recombinant microbial cell comprising:

(a) polynucleotides encoding a branched chain alpha-keto acid dehydrogenase (BKD) complex which comprises polypeptides having branched-chain alpha-keto acid dehydrogenase activity, lipoamide acyltransferase activity, and dihydrolipoamide dehydrogenase activity;
(b) a polynucleotide encoding a polypeptide having beta-ketoacyl-ACP synthase activity that utilizes a branched acyl-CoA molecule as a substrate;
(c) one or more polynucleotides encoding a polypeptide having fatty acid derivative enzyme activity, wherein the fatty acid derivative enzyme activity comprises one or more of thioesterase activity, fatty alcohol biosynthetic activity, ester synthase activity, and hydrocarbon biosynthetic activity; and
(d) a polynucleotide encoding a polypeptide having aspartokinase activity, homoserine dehydrogenase activity, homoserine kinase activity, threonine synthase activity or threonine deaminase activity;

wherein at least one polynucleotide according to (a), (b) or (d) encodes a polypeptide that is heterologous to the recombinant microbial cell.

**2.** The recombinant microbial cell of claim 1, wherein the recombinant microbial cell produces a branched fatty acid derivative when cultured in the presence of a carbon source under conditions effective to express the polynucleotides.

**3.** The recombinant microbial cell of claim 1, further comprising

(e) one or more polynucleotides encoding a polypeptide having (R)-citramalate synthase activity, isopropylmalate isomerase activity, or beta-isopropylmalate dehydrogenase activity.

**4.** The recombinant microbial cell of claim 1, which produces a fatty acid derivative composition comprising straight-chain fatty acid derivatives and branched fatty acid derivatives, wherein at least 5% of the fatty acid derivatives in the composition are branched fatty acid derivatives, when cultured in the presence of a carbon source under conditions effective to express the polynucleotides.

**5.** The recombinant microbial cell of claim 1, which produces at least 25 mg/L branched fatty acid derivatives when cultured in the presence of a carbon source under conditions effective to express the polynucleotides.

**6.** The recombinant microbial cell of claim 1, wherein the recombinant microbial cell expresses one or more polypeptides having a fatty acid derivative activity selected from the group consisting of:

(i) a polypeptide having thioesterase activity;
(ii) a polypeptide having decarboxylase activity;
(iii) a polypeptide having carboxylic acid reductase activity;
(iv) a polypeptide having alcohol dehydrogenase activity (EC 1.1.1.1);
(v) a polypeptide having aldehyde decarbonylase activity (EC 4.1.99.5);
(vi) a polypeptide having acyl-CoA reductase activity (EC 1.2.1.50);
(vii) a polypeptide having acyl-ACP reductase activity;
(viii) a polypeptide having ester synthase activity (EC 3.1.1.67);
(ix) a polypeptide having OleA activity; and
(x) a polypeptide having OleCD or OleBCD activity.

**7.** The recombinant microbial cell of claim 1, which comprises an endogenous polynucleotide sequence encoding a polypeptide having beta-ketoacyl-ACP synthase activity that does not utilize a branched acyl-CoA molecule as a substrate, wherein expression of the endogenous polynucleotide sequence encoding a polypeptide having beta-ketoacyl-ACP synthase activity in the recombinant microbial cell is attenuated.

**8.** The recombinant microbial cell of claim 1, wherein the recombinant microbial cell is a member of the genus *Escherichia, Bacillus, Lactobacillus, Pantoea, Zymomonas, Rhodococcus, Pseudomonas, Aspergillus, Trichoderma, Neurospora, Fusarium, Humicola, Rhizomucor, Kluyveromyces, Pichia, Mucor, Myceliophtora, Penicillium, Phanerochaete, Pleurotus, Trametes, Chrysosporium, Saccharomyces, Stenotrophamonas, Schizosaccharomyces, Yarrowia, Streptomyces, Synechococcus, Chlorella,* or *Prototheca.*

**9.** A cell culture comprising the recombinant microbial cell of claim 1.

**10.** The recombinant microbial cell of claim 1, which produces at least 10 mg/L of a branched fatty acid derivative.

**11.** A method of making a composition comprising a branched fatty acid derivative, the method comprising:

obtaining the recombinant microbial cell of claim 1;
culturing the recombinant microbial cell in a culture medium containing a carbon source under conditions effective to express the polynucleotides and produce a fatty acid derivative composition comprising straight-chain fatty acid derivatives and branched fatty acid derivatives, wherein at least 10% of the fatty acid derivatives in the composition are branched fatty acid derivatives; and
optionally recovering the composition from the culture medium.

**12.** The method of claim 11, wherein the fatty acid derivative composition produced in the culture medium comprises at least 10 mg/L branched fatty acid derivatives.

**13.** The method of claim 11, wherein the recombinant microbial cell expresses one or more polypeptides having a fatty acid derivative enzyme activity selected from the group consisting of:

(i) a polypeptide having thioesterase activity;
(ii) a polypeptide having decarboxylase activity;
(iii) a polypeptide having carboxylic acid reductase activity;

(iv) a polypeptide having alcohol dehydrogenase activity (EC 1.1.1.1);
(v) a polypeptide having aldehyde decarbonylase activity (EC 4.1.99.5);
(vi) a polypeptide having acyl-CoA reductase activity (EC 1.2.1.50);
(vii) a polypeptide having acyl-ACP reductase activity;
(viii) a polypeptide having ester synthase activity (EC 3.1.1.67);
(ix) a polypeptide having OleA activity; and
(x) a polypeptide having OleCD or OleBCD activity.

**14.** The recombinant microbial cell of claim 1, wherein the polypeptide having aspartokinase activity further has homoserine dehydrogenase activity.

**15.** The recombinant microbial cell of claim 1, wherein the polypeptide having aspartokinase activity is ThrA.

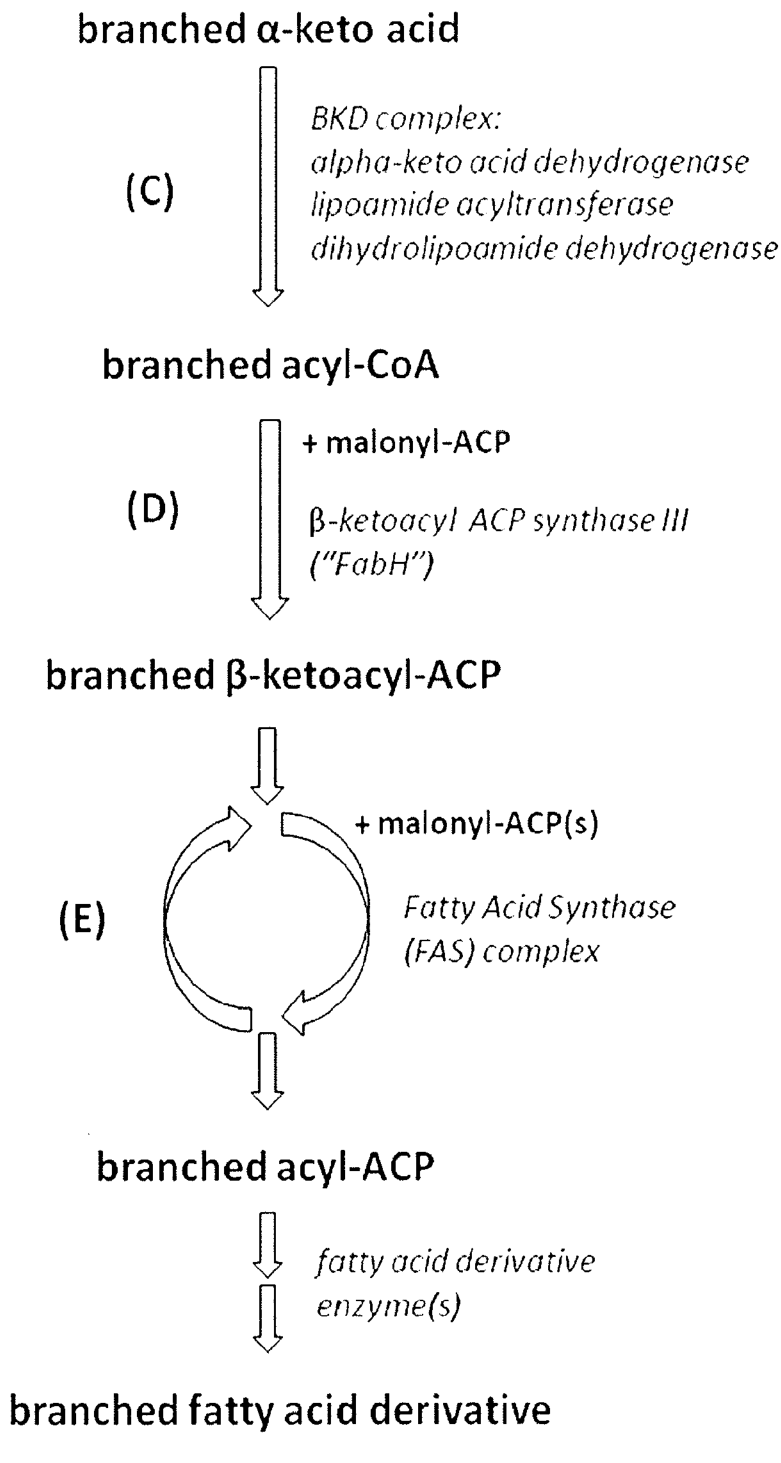
branched α-keto acid
(C)
BKD complex:
alpha-keto acid dehydrogenase
lipoamide acyltransferase
dihydrolipoamide dehydrogenase
branched acyl-CoA
(D)
+ malonyl-ACP
β-ketoacyl ACP synthase III
("FabH")
branched β-ketoacyl-ACP
(E)
+ malonyl-ACP(s)
Fatty Acid Synthase
(FAS) complex
branched acyl-ACP
fatty acid derivative
enzyme(s)
branched fatty acid derivative

Fig. 1

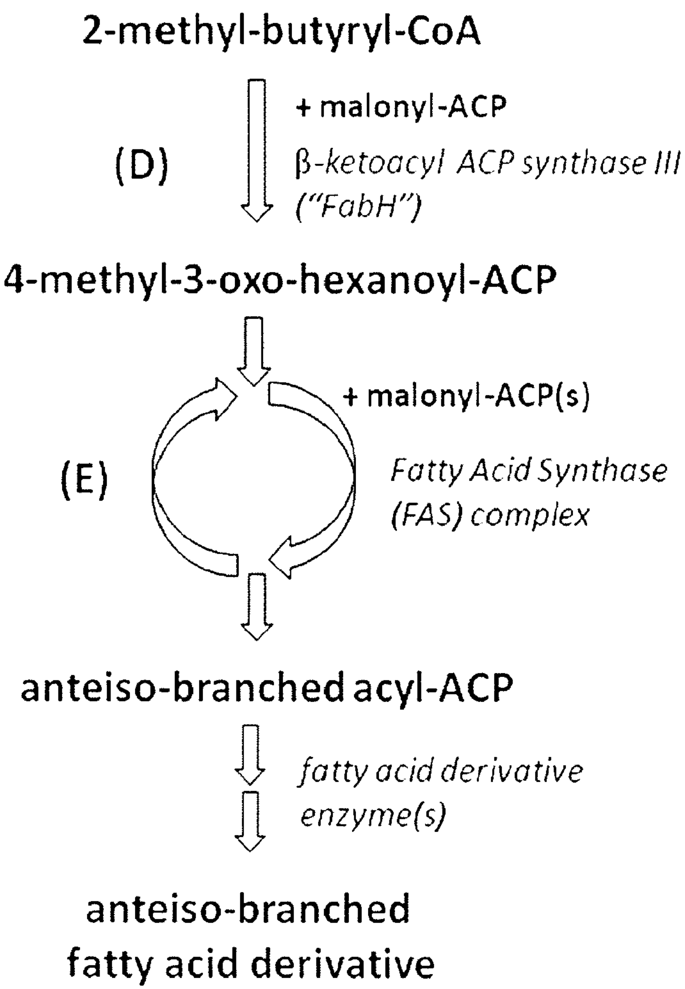
2-methyl-butyryl-CoA
(D)
+ malonyl-ACP
β-ketoacyl ACP synthase III
("FabH")
4-methyl-3-oxo-hexanoyl-ACP
(E)
+ malonyl-ACP(s)
Fatty Acid Synthase
(FAS) complex
anteiso-branched acyl-ACP
fatty acid derivative
enzyme(s)
anteiso-branched
fatty acid derivative

Fig. 2A

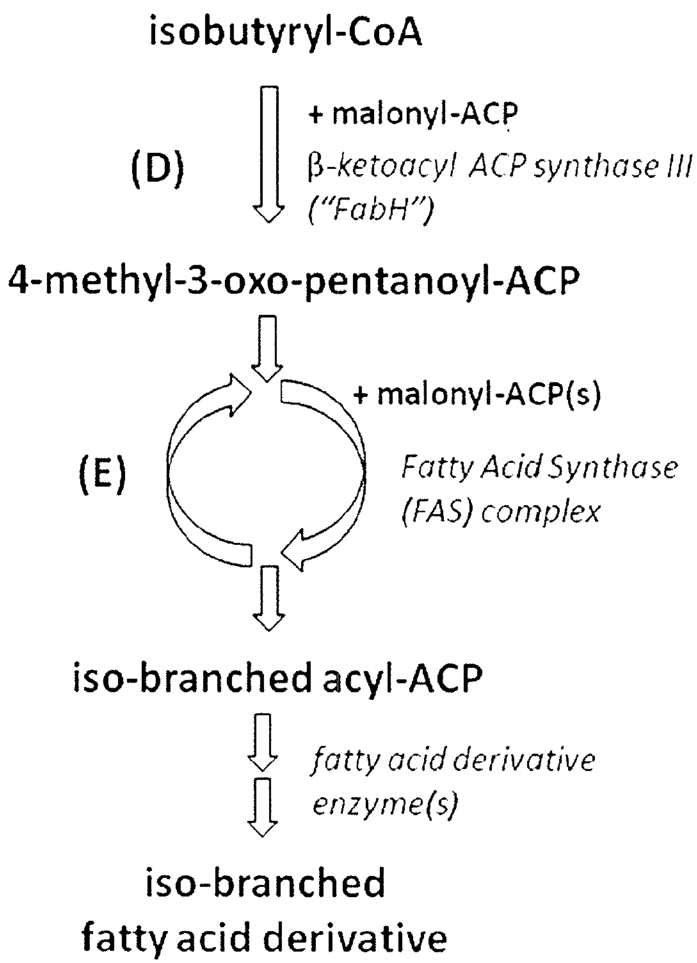
isobutyryl-CoA
(D)
+ malonyl-ACP
β-ketoacyl ACP synthase III
("FabH")
4-methyl-3-oxo-pentanoyl-ACP
(E)
+ malonyl-ACP(s)
Fatty Acid Synthase
(FAS) complex
iso-branched acyl-ACP
fatty acid derivative
enzyme(s)
iso-branched
fatty acid derivative

Fig. 2B

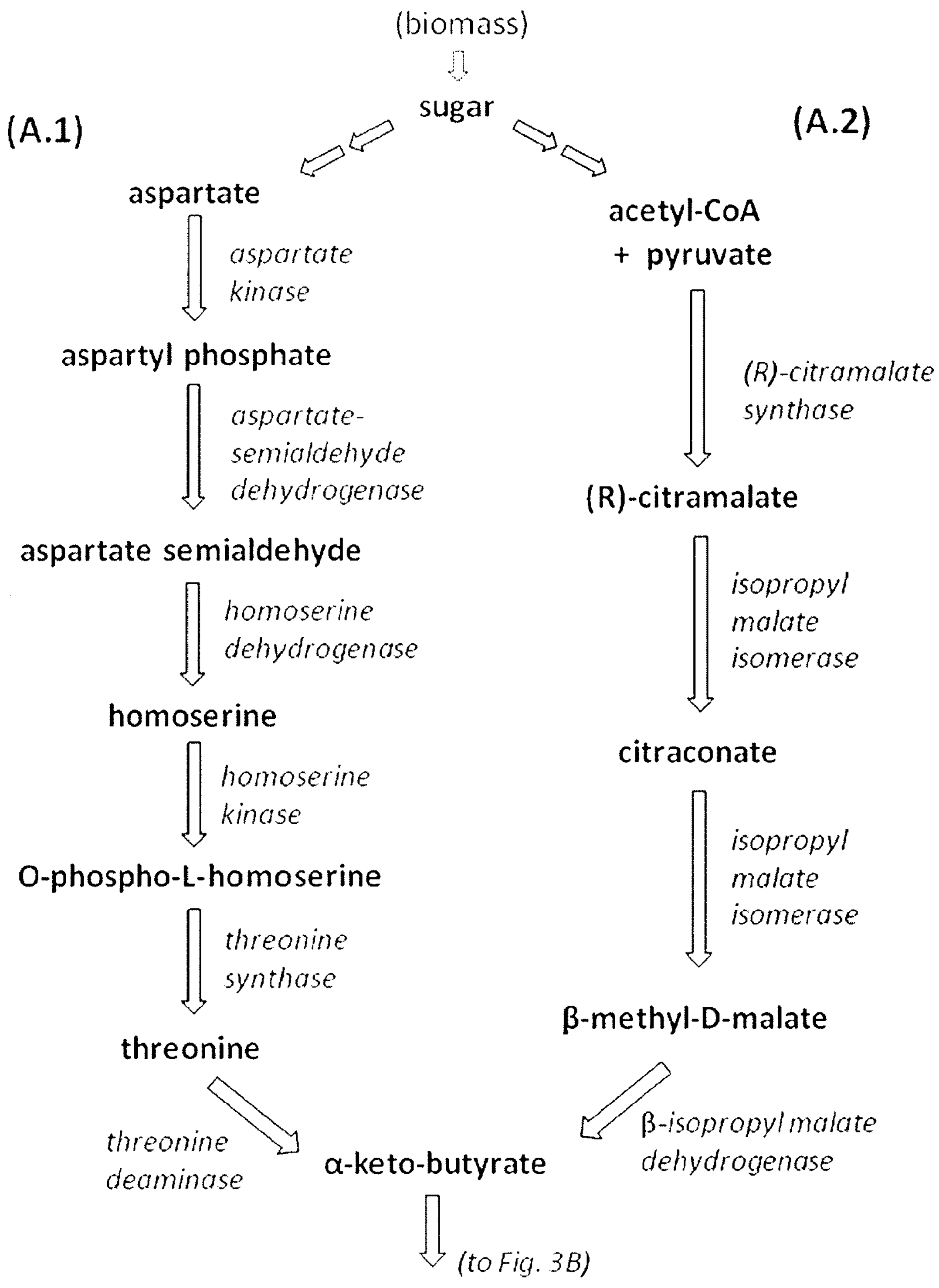
(biomass)
sugar
(A.1)
(A.2)
aspartate
aspartate kinase
aspartyl phosphate
aspartate-semialdehyde dehydrogenase
aspartate semialdehyde
homoserine dehydrogenase
homoserine
homoserine kinase
O-phospho-L-homoserine
threonine synthase
threonine
threonine deaminase
acetyl-CoA + pyruvate
(R)-citramalate synthase
(R)-citramalate
isopropyl malate isomerase
citraconate
isopropyl malate isomerase
β-methyl-D-malate
β-isopropylmalate dehydrogenase
α-keto-butyrate
(to Fig. 3B)

Fig. 3A

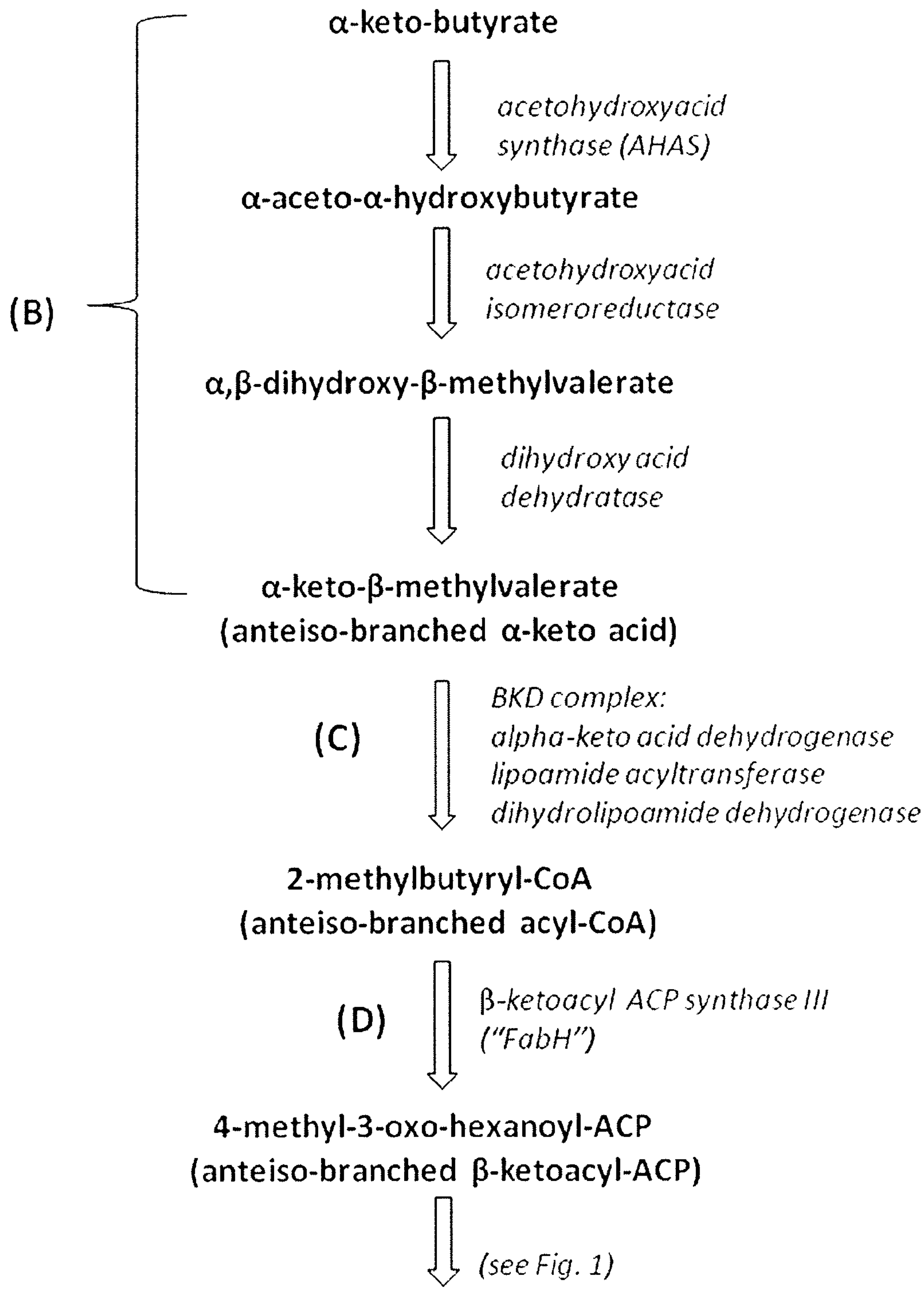
α-keto-butyrate
acetohydroxyacid synthase (AHAS)
α-aceto-α-hydroxybutyrate
acetohydroxyacid isomeroreductase
(B)
α,β-dihydroxy-β-methylvalerate
dihydroxy acid dehydratase
α-keto-β-methylvalerate
(anteiso-branched α-keto acid)
(C)
BKD complex:
alpha-keto acid dehydrogenase
lipoamide acyltransferase
dihydrolipoamide dehydrogenase
2-methylbutyryl-CoA
(anteiso-branched acyl-CoA)
(D)
β-ketoacyl ACP synthase III
("FabH")
4-methyl-3-oxo-hexanoyl-ACP
(anteiso-branched β-ketoacyl-ACP)
(see Fig. 1)

Fig. 3B

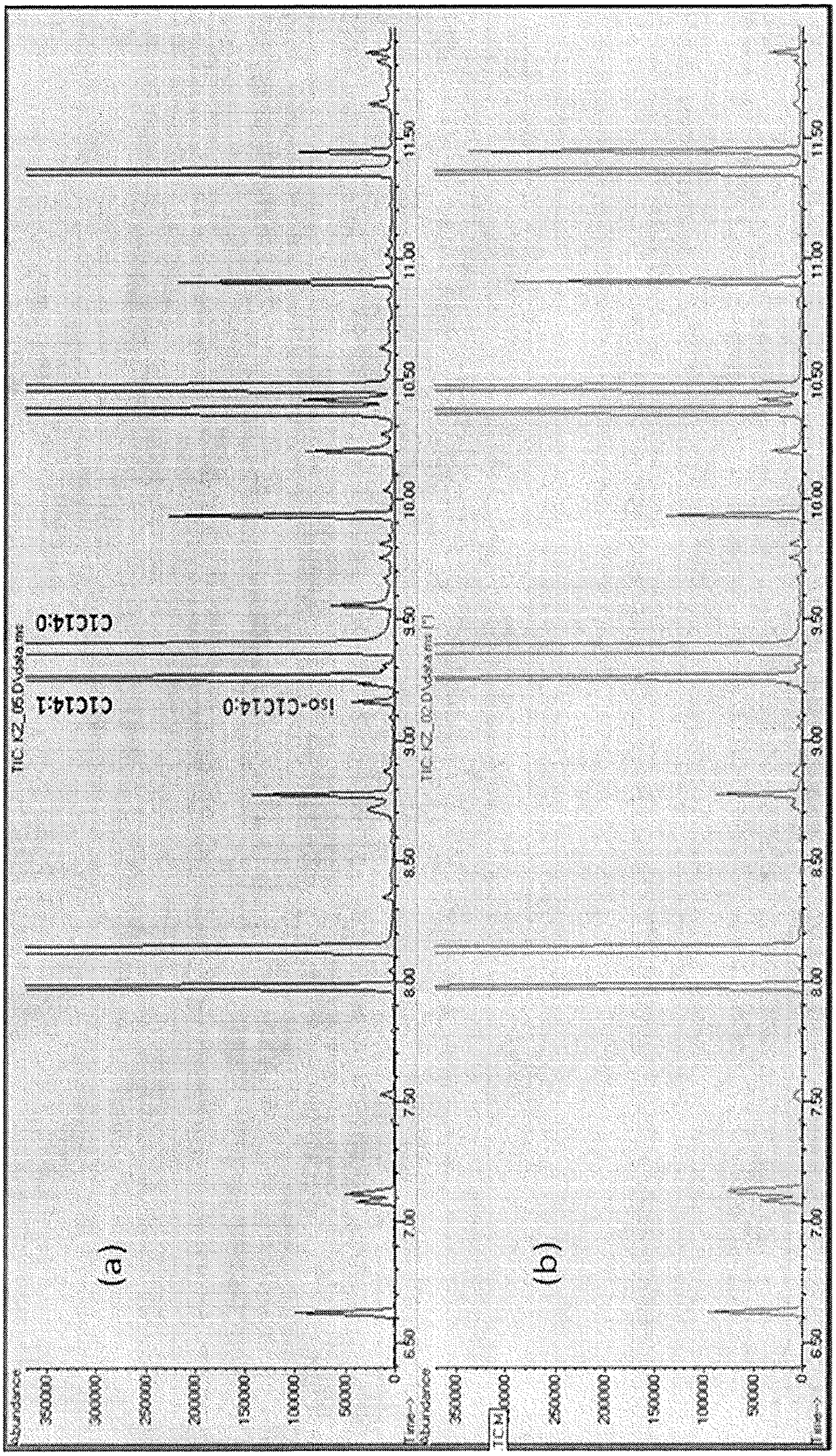
(a)
(b)
C1C14:1
C1C14:0
iso-C1C14:0
Abundance
Time-->
6.50
7.00
7.50
8.00
8.50
9.00
9.50
10.00
10.50
11.00
11.50

Fig. 4

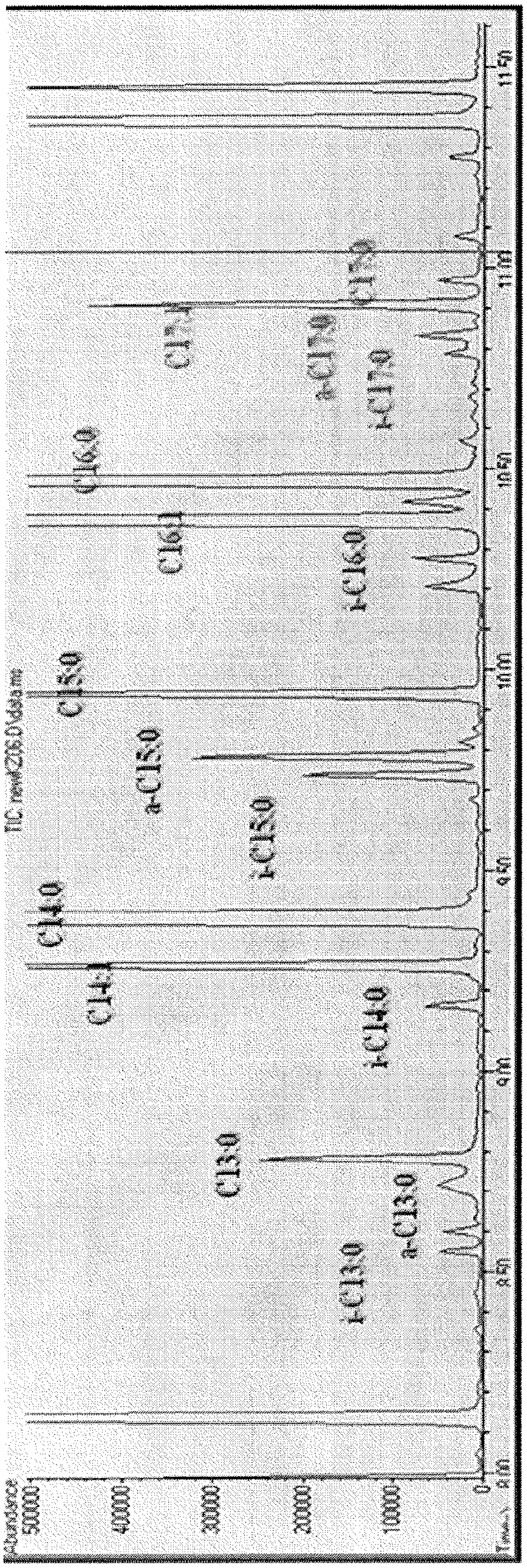

Abundance
500000
400000
300000
200000
100000
0
Time
TIC: new\Z16.D\data.ms
i-C13:0
a-C13:0
C13:0
i-C14:0
C14:1
C14:0
i-C15:0
a-C15:0
C15:0
i-C16:0
C16:1
C16:0
i-C17:0
a-C17:0
C17:1
C17:0


Fig. 5

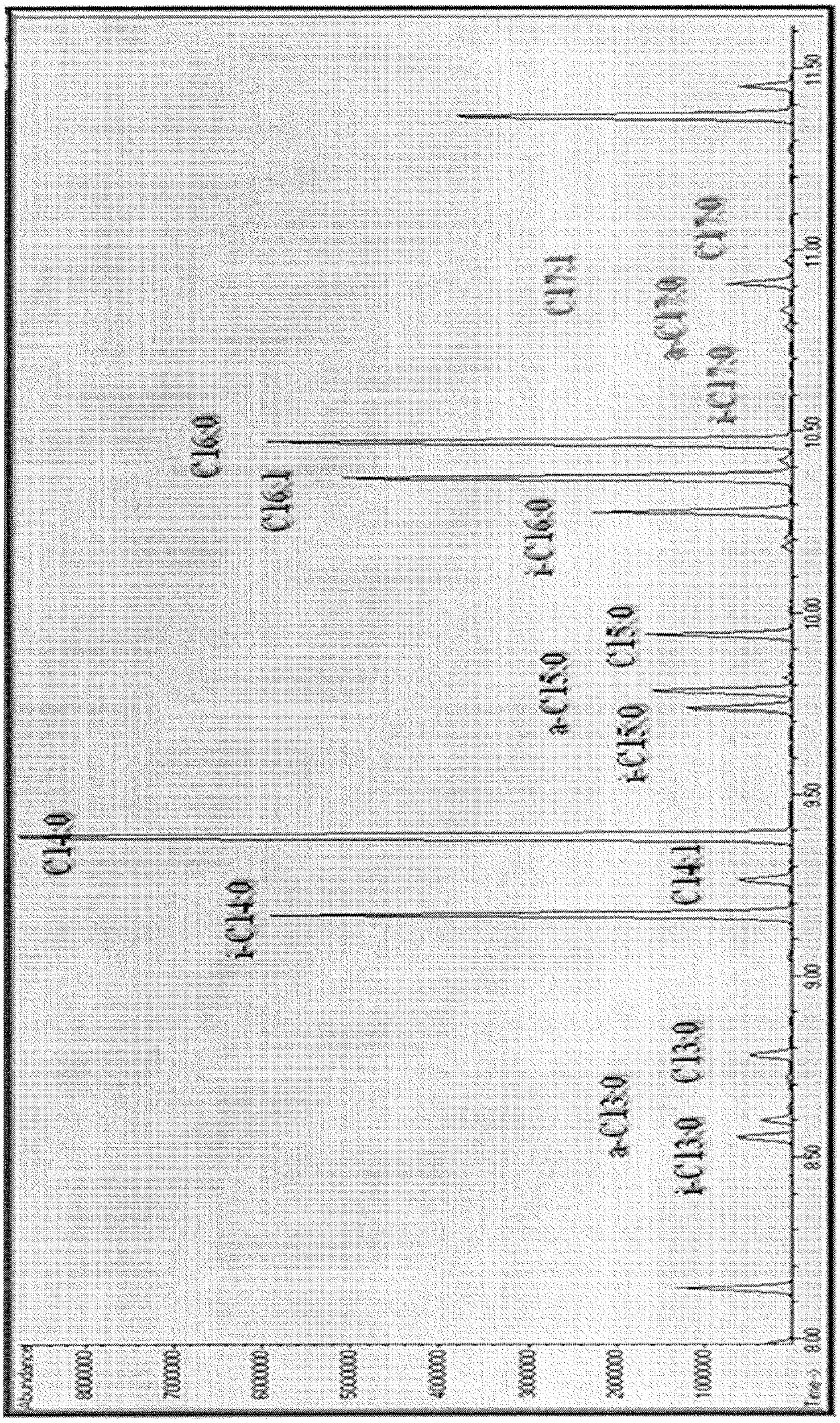
Abundance
Time
C14:0
i-C14:0
C14:1
i-C13:0
a-C13:0
C13:0
i-C15:0
a-C15:0
C15:0
i-C16:0
C16:1
C16:0
i-C17:0
a-C17:0
C17:1
C17:0

Fig. 6

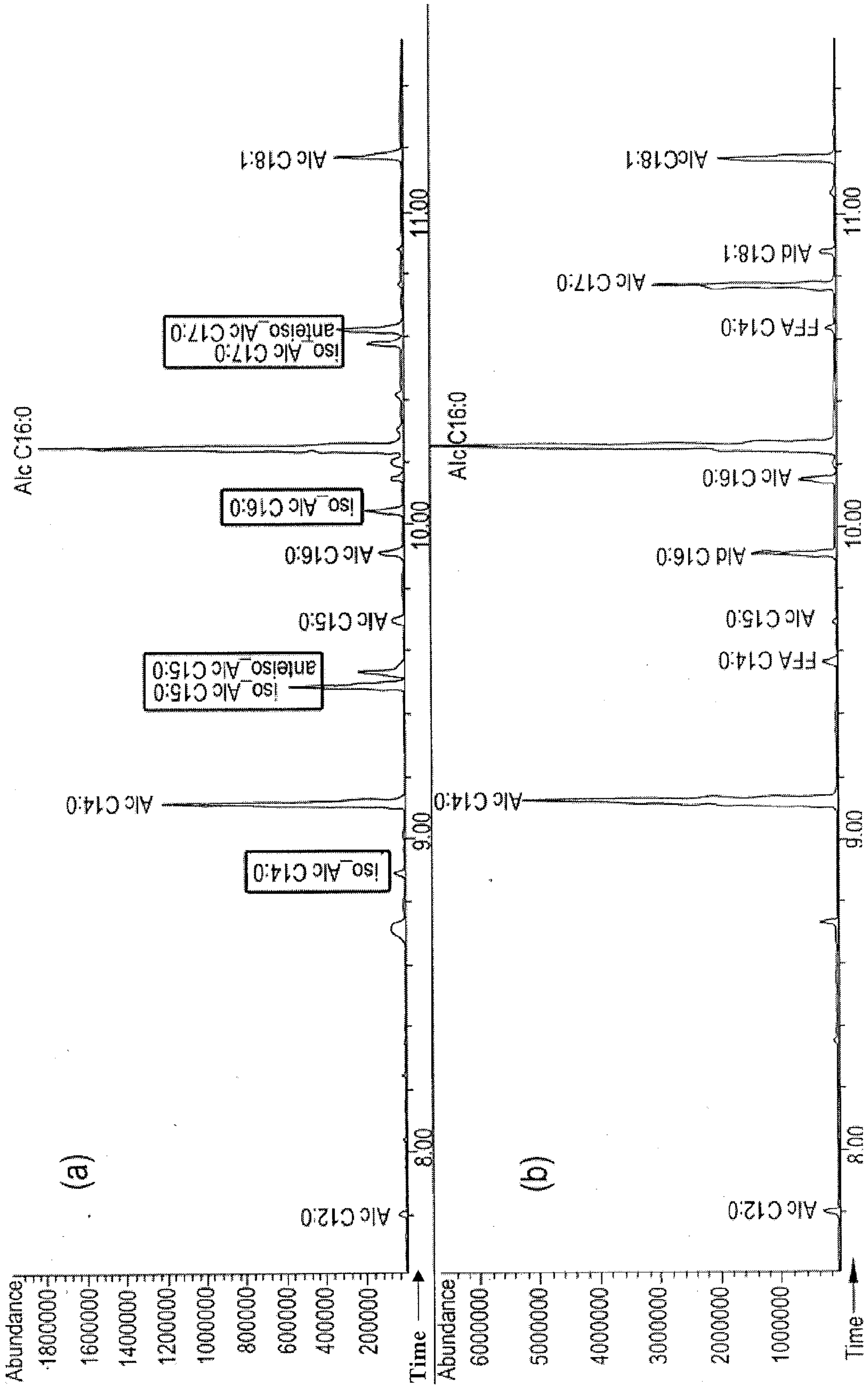
(a)
Abundance
Time
Alc C12:0
iso_Alc C14:0
Alc C14:0
iso_Alc C15:0
anteiso_Alc C15:0
Alc C15:0
Alc C16:0
iso_Alc C16:0
Alc C16:0
iso_Alc C17:0
anteiso_Alc C17:0
Alc C18:1
(b)
Abundance
Time
Alc C12:0
Alc C14:0
FFA C14:0
Alc C15:0
Ald C16:0
Alc C16:0
Alc C16:0
FFA C14:0
Alc C17:0
Ald C18:1
AlcC18:1

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 00693311 A
- US 00710011 A
- WO 2010075483 A
- WO 2007136762 A
- WO 2008119082 A
- WO 2010062480 A
- WO 2010042664 A
- WO 2009085278 A
- WO 2008147781 A
- WO 1991016427 A
- US 7169588 B


### Non-patent literature cited in the description


- **KNOTHE G.** *Fuel Process Technol.,* 2005, vol. 86, 1059-1070
- **DATSENKO et al.** *Proc. Natl. Acad. Sci. U.S.A.,* 2000, vol. 97 (12), 6640-6645
- **MANIATIS et al.** *Science,* 1987, vol. 236, 1237-1245
- **GOEDDEL.** Gene Expression Technology: Methods in Enzymology. Academic Press, 1990, vol. 185
- **SMITH et al.** Gene. Pharmacia Biotech, Inc, 1988, vol. 67, 31-40
- **DATSENKO et al.** *Proc. Natl. Acad. Sci. USA,* 2000, vol. 97, 6640-45
- **KANEDA.** *Microbiol. Rev.,* 1991, vol. 55, 288-302
- **CROPP et al.** *Can J Microbiol,* 2000, vol. 46, 506-14
- **SOKATCH et al.** *J. Bacteriol.,* 1981, vol. 148, 647-652
- **HAN ; REYNOLDS.** *J. Bacteriol.,* 1997, vol. 179, 5157
- **GUILLOUET S. et al.** *Appl. Environ. Microbiol.,* 1999, vol. 65, 3100-3107
- **LEE K.H. et al.** *Mol. Syst. Biol.,* 2007, vol. 3, 149
- **ATSUMI S. ; LIAO J.C.** *Appl. Environ. Microbiol.,* 2008, vol. 74 (24), 7802-7808
- **CANN A.F. ; LIAO J.C.** *Appl Microbiol Biotechnol.,* 2008, vol. 81 (1), 89-98
- **ZHANG K. et al.** *Proc. Natl. Acad. Sci. U S A.,* 2008, vol. 105 (52), 20653-20658
- **OGAWA-MIYATA,Y. et al.** *Biosci. Biotechnol. Biochem.,* 2001, vol. 65, 1149-1154
- **LEE J.-H. et al.** *J. Bacteriol.,* 2003, vol. 185, 5442-5451
- **HOWELL, D.M. et al.** *J. Bacteriol.,* 1999, vol. 181 (1), 331-3
- **XU, H. et al.** *J. Bacteriol.,* 2004, vol. 186, 5400-5409
- **SOKATCH et al.** *J. Bacteriology,* 1981, vol. 148 (2), 647-652
- **CHOI et al.** *J. Bacteriology,* 2000, vol. 182 (2), 365-370
- **CHO et al.** *J. Biol. Chem.,* 1995, vol. 270, 4216-4219
- **ALTSCHUL et al.** *J. Mol. Biol.,* 1990, vol. 215 (3), 403-410
- **NEEDLEMAN ; WUNSCH.** *J. Mol. Biol.,* 1970, vol. 48, 444-453
- **ROSENBERG.** *BMC Bioinformatics,* 2005, vol. 6, 278
- **ALTSCHUL et al.** *FEBSJ.,* 2005, vol. 272 (20), 5101-5109
- **BOWIE et al.** *Science,* 1990, vol. 247, 1306-1310
- **ARKIN et al.** *Proc. Natl. Acad. Sci., U.S.A.,* 1992, vol. 89, 7811-7815
- **DELEGRAVE et al.** *Biotech. Res,* 1993, vol. 11, 1548-1552
- Source Apportionment of Atmospheric Particles. **CURRIE.** Characterization of Environmental Particles. IUPAC Environmental Analytical Chemistry Series, Lewis Publishers, Inc, 1992, vol. I, 3-74
- **DATSENKO, K.A. et al.** *Proc. Natl. Acad. Sci. USA,* 2000, vol. 97, 6640-6645
- **PODKOVYROV ; LARSON.** *Nucl. Acids Res.,* 1996, vol. 24 (9), 1747-1752
- **ROSE, R.E.** *Nucleic Acids Res.,* 1988, vol. 16, 356
- **PALMEROS et al.** *Gene,* 2000, vol. 247, 255-264
- **LERNER C.G. ; INOUYE M.** *Nucleic Acids Res.,* 1990, vol. 18, 4631
- **SPIZIZEN.** *J. Bacteriol.,* 1961, vol. 81, 741

SEQUENCE LISTING

<110> LS9, Inc.

<120> PRODUCTION OF BRANCHED CHAIN FATTY ACIDS AND DERIVATIVES THEREOF IN RECOMBINANT MICROBIAL CELLS

<130> EP88675IFZRPpau

<140> not yet assigned
<141> 2011-05-10

<150> EP11722657.1
<151> 2011-05-10

<150> PCT/US2011/035979
<151> 2011-05-10

<150> 13/006,933
<151> 2011-01-14

<150> 13/007,100
<151> 2011-01-14

<160> 315

<170> PatentIn version 3.5

<210> 1
<211> 330
<212> PRT
<213> Bacillus subtilis

<220>
<221> MISC_FEATURE
<223> BKD E1alpha subunit

<400> 1

```
Met Ser Thr Asn Arg His Gln Ala Leu Gly Leu Thr Asp Gln Glu Ala
1               5                   10                  15

Val Asp Met Tyr Arg Thr Met Leu Leu Ala Arg Lys Ile Asp Glu Arg
            20                  25                  30

Met Trp Leu Leu Asn Arg Ser Gly Lys Ile Pro Phe Val Ile Ser Cys
        35                  40                  45

Gln Gly Gln Glu Ala Ala Gln Val Gly Ala Ala Phe Ala Leu Asp Arg
    50                  55                  60

Glu Met Asp Tyr Val Leu Pro Tyr Tyr Arg Asp Met Gly Val Val Leu
65                  70                  75                  80

Ala Phe Gly Met Thr Ala Lys Asp Leu Met Met Ser Gly Phe Ala Lys
                85                  90                  95
```

```
Ala Ala Asp Pro Asn Ser Gly Gly Arg Gln Met Pro Gly His Phe Gly
            100                 105                 110

Gln Lys Lys Asn Arg Ile Val Thr Gly Ser Ser Pro Val Thr Thr Gln
        115                 120                 125

Val Pro His Ala Val Gly Ile Ala Leu Ala Gly Arg Met Glu Lys Lys
    130                 135                 140

Asp Ile Ala Ala Phe Val Thr Phe Gly Glu Gly Ser Ser Asn Gln Gly
145                 150                 155                 160

Asp Phe His Glu Gly Ala Asn Phe Ala Ala Val His Lys Leu Pro Val
                165                 170                 175

Ile Phe Met Cys Glu Asn Asn Lys Tyr Ala Ile Ser Val Pro Tyr Asp
            180                 185                 190

Lys Gln Val Ala Cys Glu Asn Ile Ser Asp Arg Ala Ile Gly Tyr Gly
        195                 200                 205

Met Pro Gly Val Thr Val Asn Gly Asn Asp Pro Leu Glu Val Tyr Gln
    210                 215                 220

Ala Val Lys Glu Ala Arg Glu Arg Ala Arg Arg Gly Glu Gly Pro Thr
225                 230                 235                 240

Leu Ile Glu Thr Ile Ser Tyr Arg Leu Thr Pro His Ser Ser Asp Asp
                245                 250                 255

Asp Asp Ser Ser Tyr Arg Gly Arg Glu Glu Val Glu Glu Ala Lys Lys
            260                 265                 270

Ser Asp Pro Leu Leu Thr Tyr Gln Ala Tyr Leu Lys Glu Thr Gly Leu
        275                 280                 285

Leu Ser Asp Glu Ile Glu Gln Thr Met Leu Asp Glu Ile Met Ala Ile
    290                 295                 300

Val Asn Glu Ala Thr Asp Glu Ala Glu Asn Ala Pro Tyr Ala Ala Pro
305                 310                 315                 320

Glu Ser Ala Leu Asp Tyr Val Tyr Ala Lys
                325                 330

<210>  2
<211>  993
```

<212> DNA
<213> Bacillus subtilis

<220>
<221> misc_feature
<223> Encodes BKD E1alpha subunit

<400> 2
ctacttcgca taaacataat caagcgctga ctcaggagct gcatatgggg cgttctccgc 60

ttcatccgtc gcttcattta cgattgccat aatttcatcc agcatggttt gttctatctc 120

atcggacagc aggcctgttt cctttaagta agcttgataa gtaagcaggg gatcactttt 180

tttcgcttcc tctacttctt cacggcctct gtagctgctg tcatcgtcat cactggaatg 240

tggtgtaagg cggtaagaaa tcgtttcaat taatgtcggg ccttctcctc tgcgtgccct 300

ttcgcgtgct tctttaaccg cttgataaac ttccagcgga tcatttccat tcacagttac 360

gccaggcatc ccatagccta tggcacggtc ggaaatgttc tcacatgcga cttgcttatc 420

gtaaggcact gagattgcgt atttgttgtt ttcacacatg aaaataaccg gcagcttatg 480

gacagcggca aagtttgccc cttcatggaa atcgccttgg tttgaagacc cttccccgaa 540

tgtaacaaag gctgcgatat cctttttctc catacgtccc gcaagcgcaa taccgactgc 600

gtgcggcact tgcgttgtaa ccggagatga tcccgtcaca atgcggtttt tcttttgtcc 660

gaaatgtccc ggcatctggc ggcctcctga gttcggatct gctgcttttg caaacccgga 720

catcattaag tcctttgctg tcatgccaaa cgcgagcacg acacccatgt ctctgtagta 780

cggcaataca taatccattt cacggtcaag tgcgaaagcc gctcctacct gtgctgcttc 840

ctgtccttga caagagatta caaatggaat tttgccagaa cggtttaaca gccacattct 900

ttcatcgatt tttcttgcta acagcatggt tctatacata tcaacggctt cctgatcagt 960

cagccctagt gcttgatgtc ggtttgtact cat 993

<210> 3
<211> 381
<212> PRT
<213> Streptomyces avermitilis

<220>
<221> MISC_FEATURE
<223> BKD E1alpha subunit

<400> 3

Met Thr Val Met Glu Gln Arg Gly Ala Tyr Arg Pro Thr Pro Pro Pro
1 5 10 15

Ala Trp Gln Pro Arg Thr Asp Pro Ala Pro Leu Leu Pro Asp Ala Leu
20 25 30

```
Pro His Arg Val Leu Gly Thr Glu Ala Ala Ala Glu Ala Asp Pro Leu
        35                  40                  45

Leu Leu Arg Arg Leu Tyr Ala Glu Leu Val Arg Gly Arg Arg Tyr Asn
    50                  55                  60

Thr Gln Ala Thr Ala Leu Thr Lys Gln Gly Arg Leu Ala Val Tyr Pro
65                  70                  75                  80

Ser Ser Thr Gly Gln Glu Ala Cys Glu Val Ala Ala Ala Leu Val Leu
                85                  90                  95

Glu Glu Arg Asp Trp Leu Phe Pro Ser Tyr Arg Asp Thr Leu Ala Ala
            100                 105                 110

Val Ala Arg Gly Leu Asp Pro Val Gln Ala Leu Thr Leu Leu Arg Gly
        115                 120                 125

Asp Trp His Thr Gly Tyr Asp Pro Arg Glu His Arg Ile Ala Pro Leu
    130                 135                 140

Cys Thr Pro Leu Ala Thr Gln Leu Pro His Ala Val Gly Leu Ala His
145                 150                 155                 160

Ala Ala Arg Leu Lys Gly Asp Asp Val Val Ala Leu Ala Leu Val Gly
                165                 170                 175

Asp Gly Gly Thr Ser Glu Gly Asp Phe His Glu Ala Leu Asn Phe Ala
            180                 185                 190

Ala Val Trp Gln Ala Pro Val Val Phe Leu Val Gln Asn Asn Gly Phe
        195                 200                 205

Ala Ile Ser Val Pro Leu Ala Lys Gln Thr Ala Ala Pro Ser Leu Ala
    210                 215                 220

His Lys Ala Val Gly Tyr Gly Met Pro Gly Arg Leu Val Asp Gly Asn
225                 230                 235                 240

Asp Ala Ala Ala Val His Glu Val Leu Ser Asp Ala Val Ala His Ala
                245                 250                 255

Arg Ala Gly Gly Gly Pro Thr Leu Val Glu Ala Val Thr Tyr Arg Ile
            260                 265                 270

Asp Ala His Thr Asn Ala Asp Asp Ala Thr Arg Tyr Arg Gly Asp Ser
        275                 280                 285
```

Glu Val Glu Ala Trp Arg Ala His Asp Pro Ile Ala Leu Leu Glu His
    290                 295                 300

Glu Leu Thr Glu Arg Gly Leu Leu Asp Glu Asp Gly Ile Arg Ala Ala
305                 310                 315                 320

Arg Glu Asp Ala Glu Ala Met Ala Ala Asp Leu Arg Ala Arg Met Asn
                325                 330                 335

Gln Asp Pro Ala Leu Asp Pro Met Asp Leu Phe Ala His Val Tyr Ala
            340                 345                 350

Glu Pro Thr Pro Gln Leu Arg Glu Gln Glu Ala Gln Leu Arg Ala Glu
        355                 360                 365

Leu Ala Ala Glu Ala Asp Gly Pro Gln Gly Val Gly Arg
    370                 375                 380

<210> 4
<211> 1146
<212> DNA
<213> Streptomyces avermitilis

<220>
<221> misc_feature
<223> Encodes BKD E1alpha subunit

<400> 4
atgacggtca tggagcagcg gggcgcttac cggcccacac cgccgcccgc ctggcagccc 60

cgcaccgacc ccgcgccact gctgcccgac gcgctgcccc accgcgtcct gggcaccgag 120

gcggccgcgg aggccgaccc gctactgctg cgccgcctgt acgcggagct ggtgcgcggc 180

cgccgctaca acacgcaggc cacggctctc accaagcagg gccggctcgc cgtctacccg 240

tcgagcacgg gccaggaggc ctgcgaggtc gccgccgcgc tcgtgctgga ggagcgcgac 300

tggctcttcc ccagctaccg ggacaccctc gccgccgtcg cccgcggcct cgatcccgtc 360

caggcgctca ccctcctgcg cggcgactgg cacaccgggt acgacccccg tgagcaccgc 420

atcgcgcccc tgtgcacccc tctcgcgacc cagctcccgc acgccgtcgg cctcgcgcac 480

gccgcccgcc tcaagggcga cgacgtggtc gcgctcgccc tggtcggcga cggcggcacc 540

agcgagggcg acttccacga ggcactgaac ttcgccgccg tctggcaggc gccggtcgtc 600

ttcctcgtgc agaacaacgg cttcgccatc tccgtcccgc tcgccaagca gaccgccgcc 660

ccgtcgctgg cccacaaggc cgtcggctac gggatgccgg gccgcctggt cgacggcaac 720

gacgcggcgg ccgtgcacga ggtcctcagc gacgccgtgg cccacgcgcg cgcgggaggg 780

gggccgacgc tcgtggaggc ggtgacctac cgcatcgacg cccacaccaa cgccgacgac 840

gcgacgcgct accgggggga ctccgaggtg gaggcctggc gcgcgcacga cccgatcgcg 900

ctcctggagc acgagttgac cgaacgcggg ctgctcgacg aggacggcat ccgggccgcc 960

cgcgaggacg ccgaggcgat ggccgcggac ctgcgcgcac gcatgaacca ggatccggcc 1020

ctggacccca tggacctgtt cgcccatgtg tatgccgagc ccacccccca gctgcgggag 1080

caggaagccc agttgcgggc cgagctggca gcggaggccg acgggcccca aggagtcggc 1140

cgatga 1146

<210> 5
<211> 410
<212> PRT
<213> Pseudomonas putida

<220>
<221> MISC_FEATURE
<223> BKD E1alpha subunit

<400> 5

Met Asn Glu Tyr Ala Pro Leu Arg Leu His Val Pro Glu Pro Thr Gly
1               5                   10                  15

Arg Pro Gly Cys Gln Thr Asp Phe Ser Tyr Leu Arg Leu Asn Asp Ala
            20                  25                  30

Gly Gln Ala Arg Lys Pro Ala Ile Asp Val Asp Ala Ala Asp Thr Ala
        35                  40                  45

Asp Leu Ser Tyr Ser Leu Val Arg Val Leu Asp Glu Gln Gly Asp Ala
    50                  55                  60

Gln Gly Pro Trp Ala Glu Asp Ile Asp Pro Gln Ile Leu Arg Gln Gly
65                  70                  75                  80

Met Arg Ala Met Leu Lys Thr Arg Ile Phe Asp Ser Arg Met Val Val
                85                  90                  95

Ala Gln Arg Gln Lys Lys Met Ser Phe Tyr Met Gln Ser Leu Gly Glu
            100                 105                 110

Glu Ala Ile Gly Ser Gly Gln Ala Leu Ala Leu Asn Arg Thr Asp Met
        115                 120                 125

Cys Phe Pro Thr Tyr Arg Gln Gln Ser Ile Leu Met Ala Arg Asp Val
    130                 135                 140

```
Ser Leu Val Glu Met Ile Cys Gln Leu Leu Ser Asn Glu Arg Asp Pro
145                 150                 155                 160

Leu Lys Gly Arg Gln Leu Pro Ile Met Tyr Ser Val Arg Glu Ala Gly
                165                 170                 175

Phe Phe Thr Ile Ser Gly Asn Leu Ala Thr Gln Phe Val Gln Ala Val
            180                 185                 190

Gly Trp Ala Met Ala Ser Ala Ile Lys Gly Asp Thr Lys Ile Ala Ser
        195                 200                 205

Ala Trp Ile Gly Asp Gly Ala Thr Ala Glu Ser Asp Phe His Thr Ala
    210                 215                 220

Leu Thr Phe Ala His Val Tyr Arg Ala Pro Val Ile Leu Asn Val Val
225                 230                 235                 240

Asn Asn Gln Trp Ala Ile Ser Thr Phe Gln Ala Ile Ala Gly Gly Glu
                245                 250                 255

Ser Thr Thr Phe Ala Gly Arg Gly Val Gly Cys Gly Ile Ala Ser Leu
            260                 265                 270

Arg Val Asp Gly Asn Asp Phe Val Ala Val Tyr Ala Ala Ser Arg Trp
        275                 280                 285

Ala Ala Glu Arg Ala Arg Arg Gly Leu Gly Pro Ser Leu Ile Glu Trp
    290                 295                 300

Val Thr Tyr Arg Ala Gly Pro His Ser Thr Ser Asp Asp Pro Ser Lys
305                 310                 315                 320

Tyr Arg Pro Ala Asp Asp Trp Ser His Phe Pro Leu Gly Asp Pro Ile
                325                 330                 335

Ala Arg Leu Lys Gln His Leu Ile Lys Ile Gly His Trp Ser Glu Glu
            340                 345                 350

Glu His Gln Ala Val Thr Ala Glu Leu Glu Ala Ala Val Ile Ala Ala
        355                 360                 365

Gln Lys Glu Ala Glu Gln Tyr Gly Thr Leu Ala Asn Gly His Ile Pro
    370                 375                 380

Ser Ala Ala Ser Met Phe Glu Asp Val Tyr Lys Glu Met Pro Glu His
385                 390                 395                 400
```

```
Leu Arg Arg Gln Arg Gln Glu Leu Gly Val
                405                 410


<210>  6
<211>  1233
<212>  DNA
<213>  Pseudomonas putida


<220>
<221>  misc_feature
<223>  Encodes BKD E1alpha subunit

<400>  6
tcaaaccccc agttcctggc gttgacggcg caggtgttcg ggcatctcct tgtacacatc      60

ctcgaacatc gaggcggcgc tcgggatgtg cccgttagcc agggtgccgt actgctcggc     120

ttctttctgt gcggcaatca ccgcagcttc gagctcggcc gtgacggctt ggtgttcttc     180

ttcggaccag tggccgatct tgatcaggtg ctgcttcagg cgggcgatcg ggtcacccag     240

cgggaagtgg ctccagtcat cggcagggcg gtacttggag gggtcgtccg acgtcgagtg     300

cgggccggca cggtaggtga cccactcgat caggcttggg cccaggccgc ggcgggcgcg     360

ctcggcagcc cagcgcgagg cggcgtacac ggcgacgaag tcgttgccgt caacccgcag     420

cgaggcaatg ccgcagccca cgccacggcc ggcgaaggtg gtcgactcgc caccggcgat     480

ggcctggaag gtagaaatcg cccactggtt gttgaccaca ttgaggatca ccggggcgcg     540

gtaaacgtgg gcaaaggtga gggcggtgtg gaagtccgac tcggcggtgg ctccgtcacc     600

gatccacgcc gaagcaatct tggtatcgcc cttgatcgcc gaggccatgg cccagccgac     660

tgcctgcacg aactgggtcg ccaggttgcc gctgatggtg aagaagccgg cttcgcgcac     720

cgagtacatg atcggcaact ggcggccctt gaggggggtcg cgctcgttgg acagcagttg     780

gcagatcatc tcgaccagcg atacgtcgcg ggccatcagg atgctttgct ggcggtaggt     840

cgggaagcac atgtcggtgc ggttcagcgc cagcgcctgg ccactgccga tggcttcttc     900

gcccaggctt tgcatgtaga aggacatctt cttctggcgc tgggcaacca ccatgcggct     960

gtcgaagatc cgcgtcttga gcatggcgcg catgccttga cgaaggatct gtgggtcgat    1020

gtcttcggcc caggggcctt gcgcatcacc ttgctcgtcg agcacgcgga ccaggctgta    1080

ggacaggtcg gcagtgtcgg cagcatcgac atcgatcgcg ggtttacggg cttgacctgc    1140

atcgttgagg cgcaggtagg aaaaatcggt ctggcagcct ggccggccgg tgggctcggg    1200

cacatgcaaa cgcagggggg cgtactcgtt cat                                  1233


<210>  7
<211>  331
<212>  PRT
<213>  Listeria monocytogenes
```

<220>
<221> MISC_FEATURE
<223> BKD E1alpha subunit

<400> 7

```
Met Thr Leu Lys Glu Ala Gly Leu Thr Glu Asp Lys Leu Ile Lys Met
1               5                   10                  15

Tyr Glu Thr Met Leu Met Ala Arg Arg Leu Asp Glu Arg Met Trp Leu
            20                  25                  30

Leu Asn Arg Ser Gly Lys Ile Pro Phe Thr Ile Ser Gly Gln Gly Gln
        35                  40                  45

Glu Thr Ala Gln Ile Gly Ala Ala Phe Ala Phe Asp Leu Asp Lys Asp
    50                  55                  60

Tyr Ala Leu Pro Tyr Tyr Arg Asp Leu Ala Val Val Leu Ala Phe Gly
65                  70                  75                  80

Met Thr Ala Lys Asp Ile Met Leu Ser Ala Phe Ala Lys Ala Glu Asp
                85                  90                  95

Pro Asn Ser Gly Gly Arg Gln Met Pro Ala His Phe Gly Gln Lys Ser
            100                 105                 110

Asn Arg Ile Val Thr Gln Ser Ser Pro Val Thr Thr Gln Phe Pro His
        115                 120                 125

Ala Ala Gly Ile Gly Leu Ala Ala Lys Met Ala Gly Asp Glu Ile Ala
    130                 135                 140

Ile Tyr Ala Ser Thr Gly Glu Gly Ser Ser Asn Gln Gly Asp Phe His
145                 150                 155                 160

Glu Gly Ile Asn Phe Ala Ser Val His Lys Leu Pro Val Val Phe Val
                165                 170                 175

Ile His Asn Asn Gln Tyr Ala Ile Ser Val Pro Ala Ser Lys Gln Tyr
            180                 185                 190

Ala Ala Glu Lys Leu Ser Asp Arg Ala Ile Gly Tyr Gly Ile Pro Gly
        195                 200                 205

Glu Arg Val Asp Gly Thr Asn Met Gly Glu Val Tyr Ala Ala Phe Lys
    210                 215                 220
```

Arg Ala Ala Asp Arg Ala Arg Asn Gly Glu Gly Pro Thr Leu Ile Glu
225 230 235 240

Thr Val Ser Tyr Arg Phe Thr Pro His Ser Ser Asp Asp Asp Asp Ser
245 250 255

Ser Tyr Arg Ser Arg Glu Glu Val Asn Glu Ala Lys Gly Lys Asp Pro
260 265 270

Leu Thr Ile Phe Gln Thr Glu Leu Leu Glu Glu Gly Tyr Leu Thr Glu
275 280 285

Glu Lys Ile Ala Glu Ile Glu Lys Asn Ile Ala Lys Glu Val Asn Glu
290 295 300

Ala Thr Asp Tyr Ala Glu Ser Ala Ala Tyr Ala Glu Pro Glu Ser Ser
305 310 315 320

Leu Leu Tyr Val Tyr Asp Glu Glu Ala Asn Ser
325 330

<210> 8
<211> 996
<212> DNA
<213> Listeria monocytogenes

<220>
<221> misc_feature
<223> Encodes BKD E1alpha subunit

<400> 8
atgactttaa aagaagcagg tttaacagaa gataaattaa ttaaaatgta tgaaacaatg 60

ctaatggcaa gaagactaga cgagcgtatg tggttgctga accgttctgg gaaaattcct 120

ttcaccattt ctggacaagg acaagaaacg gcacaaattg gcgcagcgtt tgcctttgat 180

ttagataaag attacgcatt accatattac cgtgatttag cggtggtgtt agcatttggg 240

atgacagcga aagatattat gttatccgcg ttcgctaaag cagaggatcc aaactctggt 300

ggacgtcaaa tgccagctca ttttggtcaa aaatcaaatc gcatcgtgac acaaagttca 360

ccagtaacaa cgcagttccc gcatgcagca ggtattggtc ttgcagcgaa aatggccggt 420

gatgagattg caatttatgc ttcaacgggt gaaggatctt ctaaccaagg agatttccat 480

gaaggaatca acttcgcatc tgtacataag ttgccagttg ttttcgtgat tcacaataac 540

caatatgcca tttccgttcc agcatcgaaa caatatgctg cagaaaaact atccgaccga 600

gcaatcggtt atggtatccc aggggaacgt gtggatggca caaatatggg tgaagtatac 660

gcggcattta aacgtgcagc agatcgtgca agaaacggcg agggccccac tttaattgaa 720

acagtttctt accgattcac accgcactct tctgatgatg atgacagcag ttatcgttcc 780

agagaagaag tgaacgaagc aaaaggaaaa gatccactga caattttcca aacagaatta 840

ctcgaagaag gttacttaac agaagaaaaa atcgctgaaa tcgaaaaaaa tattgcaaaa 900

gaagttaacg aagcaaccga ttacgcggaa agtgcagcat acgctgaacc agaatcatct 960

ttactttatg tatatgatga agaagcgaat agctga 996

<210> 9
<211> 384
<212> PRT
<213> Micrococcus luteus

<220>
<221> MISC_FEATURE
<223> BKD E1alpha subunit

<400> 9

Met Thr Leu Val Asp His Thr Arg Pro Thr Gly Gly Gln Ser Ala Gly
1 5 10 15

Ser Pro Pro Pro Ala Gly Pro Ala Glu Ala Val Met Leu Gln Val Leu
20 25 30

Asp Thr Glu Gly Arg Arg Arg Pro Gln Pro Glu Leu Asp Pro Trp Ile
35 40 45

Glu Asp Val Asp Ala Ala Ala Leu Ala Ala Leu Tyr Arg Gln Met Ala
50 55 60

Val Val Arg Arg Leu Asp Val Glu Ala Thr His Leu Gln Arg Gln Gly
65 70 75 80

Glu Leu Ala Leu Trp Pro Pro Leu Leu Gly Gln Glu Ala Ala Gln Val
85 90 95

Gly Ser Ala Val Ala Leu Arg Pro Asp Asp Phe Val Phe Pro Ser Tyr
100 105 110

Arg Glu Asn Gly Val Ala Leu Leu Arg Gly Val Pro Ala Leu Asp Leu
115 120 125

Leu Arg Val Trp Arg Gly Ser Thr Phe Ser Ser Trp Asp Pro Asn Glu
130 135 140

Thr Arg Val Ala Thr Gln Gln Ile Ile Ile Gly Ala Gln Ala Leu His
145 150 155 160

Ala Val Gly Tyr Ala Met Gly Val Gln Arg Asp Gln Ala Asp Val Ala
165 170 175

Thr Ile Val Tyr Phe Gly Asp Gly Ala Thr Ser Gln Gly Asp Val Asn
180 185 190

Glu Ala Met Val Phe Ser Ala Ser Tyr Gln Ala Pro Val Val Phe Phe
195 200 205

Cys Gln Asn Asn His Trp Ala Ile Ser Glu Pro Val Arg Leu Gln Thr
210 215 220

Arg Arg Ser Ile Ala Asp Arg Pro Trp Gly Phe Gly Ile Pro Ser Met
225 230 235 240

Arg Val Asp Gly Asn Asp Val Leu Ala Val Leu Ala Ala Thr Arg Ala
245 250 255

Ala Val Glu Arg Ala Ala Asp Gly Gly Gly Pro Thr Phe Val Glu Ala
260 265 270

Val Thr Tyr Arg Met Gly Pro His Thr Thr Ala Asp Asp Pro Thr Arg
275 280 285

Tyr Arg Asp Asp Ala Glu Leu Glu Ala Trp Lys Ala Arg Asp Pro Leu
290 295 300

Thr Arg Val Glu Ala His Leu Arg Thr Leu Asp Val Asp Val Asp Ala
305 310 315 320

Val Leu Ala Gln Ala Gln Ala Glu Ala Asp Glu Leu Ala Ala Glu Val
325 330 335

Arg Arg Ala Leu Glu Ala Leu Glu Glu Asp Gly Ala Asp Arg Leu Phe
340 345 350

Asp Glu Ile Tyr Ala Glu Pro His Gln Glu Leu Glu Arg Gln Arg Arg
355 360 365

Glu His Ala Leu Tyr Leu Gln Gln Phe Asp Asp Glu Glu Ala Gly Ala
370 375 380

<210> 10
<211> 1155
<212> DNA
<213> Micrococcus luteus

<220>
<221> misc_feature
<223> Encodes BKD E1alpha subunit

<400> 10
gtgaccctcg tggaccacac ccgtcccacc ggcggacagt ccgccggctc tccgcccccg 60

gcgggcccgg ccgaggccgt gatgctccag gtgctcgaca cggagggccg ccgccgtccg 120

cagccggagc tcgacccgtg gatcgaggac gtcgacgccg ccgccctcgc cgcgctgtac 180

cgccagatgg ccgtggtccg tcgcctcgac gtcgaggcca cgcacctgca gcgtcagggc 240

gagctggccc tgtggccgcc gctgctgggc caggaggccg cccaggtggg ctccgccgtc 300

gcgctgcgcc cggacgactt cgtcttcccg tcctaccgcg agaacggcgt ggccctgctg 360

cgcggcgtcc ccgcgctgga cctgctgcgg gtgtggcgcg gctccacgtt ctcgagctgg 420

gacccgaacg agacgcgggt ggccacccag cagatcatca tcggcgcgca ggccctgcac 480

gccgtcggct acgcgatggg cgtccagcgg gaccaggcgg acgtcgccac gatcgtctac 540

ttcggcgacg gcgccacgag ccagggcgac gtcaacgagg ccatggtctt cagcgcctcc 600

taccaggcgc ccgtggtgtt cttctgccag aacaaccact gggccatctc cgagcccgtg 660

cgcctgcaga cccgccgcag catcgcggac cgcccgtggg gcttcggcat cccgtcgatg 720

cgcgtggacg gcaacgacgt cctggccgtg ctcgccgcaa cccgcgccgc cgtcgagcgc 780

gcggccgacg gggcggcccc cacgttcgtc gaggccgtca cctaccgcat gggtccacac 840

accaccgcgg acgaccccac ccgctaccgg gacgacgccg agctcgaggc ctggaaggcc 900

cgtgacccgc tgacccgcgt ggaggcgcac ctgcgcaccc tcgacgtgga cgtggacgcc 960

gtgcttgcac aggcccaggc cgaggccgac gagctggcag cggaggtccg ccgtgccctc 1020

gaggcgctcg aggaggacgg cgcggacagg ctcttcgacg agatctacgc ggagccccac 1080

caggagctcg agcggcagcg ccgcgagcac gccctctacc tgcagcagtt cgacgacgag 1140

gaggcgggcg cgtga 1155

<210> 11
<211> 330
<212> PRT
<213> Staphylococcus aureus

<220>
<221> MISC_FEATURE
<223> BKD E1alpha subunit

<400> 11

Met Ile Asp Tyr Lys Ser Leu Gly Leu Ser Glu Glu Asp Leu Lys Val
1 5 10 15

Ile Tyr Lys Trp Met Asp Leu Gly Arg Lys Ile Asp Glu Arg Leu Trp

```
            20                  25                  30

Leu Leu Asn Arg Ala Gly Lys Ile Pro Phe Val Val Ser Gly Gln Gly
        35                  40                  45

Gln Glu Ala Thr Gln Ile Gly Met Ala Tyr Ala Leu Glu Glu Gly Asp
    50                  55                  60

Ile Thr Ala Pro Tyr Tyr Arg Asp Leu Ala Phe Val Thr Tyr Met Gly
65                  70                  75                  80

Ile Ser Ala Tyr Asp Thr Phe Leu Ser Ala Phe Gly Lys Lys Asp Asp
                85                  90                  95

Val Asn Ser Gly Gly Lys Gln Met Pro Ser His Phe Ser Ser Arg Ala
            100                 105                 110

Lys Asn Ile Leu Ser Gln Ser Ser Pro Val Ala Thr Gln Ile Pro His
        115                 120                 125

Ala Val Gly Ala Ala Leu Ala Leu Lys Met Asp Gly Lys Lys Lys Ile
    130                 135                 140

Ala Thr Ala Thr Val Gly Glu Gly Ser Ser Asn Gln Gly Asp Phe His
145                 150                 155                 160

Glu Gly Leu Asn Phe Ala Gly Val His Lys Leu Pro Phe Val Cys Val
                165                 170                 175

Ile Ile Asn Asn Lys Tyr Ala Ile Ser Val Pro Asp Ser Leu Gln Tyr
            180                 185                 190

Ala Ala Glu Lys Leu Ser Asp Arg Ala Leu Gly Tyr Gly Ile His Gly
        195                 200                 205

Glu Gln Val Asp Gly Asn Asp Pro Leu Ala Met Tyr Lys Ala Met Lys
    210                 215                 220

Glu Ala Arg Asp Arg Ala Ile Ser Gly Gln Gly Ser Thr Leu Ile Glu
225                 230                 235                 240

Ala Val Thr Ser Arg Met Thr Ala His Ser Ser Asp Asp Asp Asp Gln
                245                 250                 255

Tyr Arg Thr Lys Glu Glu Arg Glu Ala Leu Lys Lys Ala Asp Cys Asn
            260                 265                 270
```

Glu Lys Phe Lys Lys Glu Leu Leu Ser Ala Gly Ile Ile Asp Asp Ala
275 280 285

Trp Leu Ala Glu Ile Glu Ala Glu His Lys Asp Ile Ile Asn Lys Ala
290 295 300

Thr Lys Ala Ala Glu Asp Ala Pro Tyr Pro Ser Val Glu Glu Ala Tyr
305 310 315 320

Ala Phe Val Tyr Glu Glu Gly Ser Leu Asn
325 330

<210> 12
<211> 993
<212> DNA
<213> Staphylococcus aureus

<220>
<221> misc_feature
<223> Encodes BKD E1alpha subunit

<400> 12
ttagttaaga ctcccttctt cgtacacaaa tgcataggct tcttcgacac ttggatatgg 60

cgcgtcttca gcagcctttg tcgctttatt gatgatgtct ttatgctccg cttctatttc 120

tgccaaccaa gcatcatcga taatgccagc tgaaagcaac tctttttga acttttcatt 180

gcagtcagct tttttaagcg cttcacgctc ttctttcgta cgatattggt cgtcatcatc 240

tgatgaatga gctgtcatac gacttgttac tgcttcaatc aaagttgaac cttgaccaga 300

aatagctcga tctcttgctt ctttcatcgc tttatacatt gctaatggat cattaccatc 360

tacttgttca ccatgtatac cgtaaccaag tgctctatcc gataattttt cagctgcgta 420

ttgtaatgaa tcaggtactg aaattgcata tttattattt ataatgacac atacaaaagg 480

aagtttgtgt acacccgcga agtttaaacc ttcatggaag tcaccttggt ttgagctacc 540

ttcaccaaca gttgctgttg caattttctt cttaccatcc atttttaaag ctaaagcagc 600

accaacagca tggggtattt gagttgctac cggtgaactt tgagacaaaa tattcttagc 660

tctactacta aagtgtgatg gcatttgttt tccaccagag ttaacatcgt ctttctttcc 720

aaacgctgat aaaaacgtat catacgctga gatacccata taagtaacga aagctagatc 780

tctataataa ggcgctgtaa tatcaccttc ttctaatgcg tatgccatcc caatctgagt 840

tgcttcttgt ccttgaccac ttacaacaaa tggaatttta cctgcacggt tcaataacca 900

cagtctttca tctatttttc tacctaaatc catccattta tatattactt ttaggtcttc 960

ttcgctaagg cctaatgatt tataatcaat cat 993

<210> 13

<211> 331
<212> PRT
<213> Streptococcus mutans

<220>
<221> MISC_FEATURE
<223> BKD E1alpha subunit

<400> 13

Met Ala Arg Lys Ile Leu Glu Val Ile Ile Ala Met Leu Ser Lys Lys
1               5                   10                  15

Gln Tyr Leu Asp Met Phe Leu Lys Met Gln Arg Ile Arg Asp Val Asp
            20                  25                  30

Thr Lys Leu Asn Lys Leu Val Arg Arg Gly Phe Val Gln Gly Met Thr
        35                  40                  45

His Phe Ser Val Gly Glu Glu Ala Ala Ser Val Gly Ala Ile Gln Gly
    50                  55                  60

Leu Thr Asp Gln Asp Ile Ile Phe Ser Asn His Arg Gly His Gly Gln
65                  70                  75                  80

Thr Ile Ala Lys Gly Ile Asp Ile Pro Ala Met Phe Ala Glu Leu Ala
                85                  90                  95

Gly Lys Ala Thr Gly Ser Ser Lys Gly Arg Gly Gly Ser Met His Leu
            100                 105                 110

Ala Asn Leu Glu Lys Gly Asn Tyr Gly Thr Asn Gly Ile Val Gly Gly
        115                 120                 125

Gly Tyr Ala Leu Ala Val Gly Ala Ala Leu Thr Gln Gln Tyr Asp Asn
    130                 135                 140

Thr Gly Asn Ile Val Val Ala Phe Ser Gly Asp Ser Ala Thr Asn Glu
145                 150                 155                 160

Gly Ser Phe His Glu Ser Val Asn Leu Ala Ala Val Trp Asn Leu Pro
                165                 170                 175

Val Ile Phe Phe Ile Ile Asn Asn Arg Tyr Gly Ile Ser Thr Asp Ile
            180                 185                 190

Asn Tyr Ser Thr Lys Ile Ser His Leu Tyr Leu Arg Ala Asp Ala Tyr
        195                 200                 205

```
Gly Ile Pro Gly His Tyr Val Glu Asp Gly Asn Asp Val Ile Ala Val
    210                 215                 220

Tyr Glu Lys Met Gln Glu Val Ile Asp Tyr Val Arg Ser Gly Asn Gly
225                 230                 235                 240

Pro Ala Leu Val Glu Val Glu Ser Tyr Arg Trp Phe Gly His Ser Thr
                245                 250                 255

Ala Asp Ala Gly Ala Tyr Arg Thr Lys Glu Glu Val Asp Ala Trp Lys
            260                 265                 270

Ala Lys Asp Pro Leu Lys Lys Tyr Arg Thr Tyr Leu Thr Glu Asn Lys
        275                 280                 285

Ile Ala Thr Asp Glu Glu Leu Asp Met Ile Glu Lys Glu Val Ala Gln
    290                 295                 300

Glu Ile Glu Asp Ala Val Lys Phe Ala Gln Asp Ser Pro Glu Pro Glu
305                 310                 315                 320

Leu Ser Val Ala Phe Glu Asp Val Trp Val Asp
                325                 330


<210>  14
<211>  996
<212>  DNA
<213>  Streptococcus mutans


<220>
<221>  misc_feature
<223>  Encodes BKD E1alpha subunit

<400>  14
atggcaagaa aaattttgga ggtcattata gcaatgttat ctaaaaaaca atatttggat      60

atgtttttaa aaatgcagcg tatccgtgat gtcgatacaa aactcaataa attagttcgt     120

cgtggtttcg tacaaggtat gacacacttt tcagtaggag aagaggcggc ttcggttggt     180

gcgattcaag gcttgactga tcaggatatt atcttttcaa atcaccgtgg acatggtcaa     240

accattgcaa aagggattga cattcctgct atgtttgcag aattagccgg taaggcaacg     300

ggttcttcaa aaggtcgtgg tggttctatg cacttggcaa atcttgaaaa aggaaactat     360

gggaccaatg gtattgttgg cgggggttat gccttagcag tcggtgctgc tttgacacag     420

caatatgaca atacgggaaa tattgttgtc gccttttcag gagactcggc aactaatgaa     480

ggctctttcc atgagtctgt taatttggca gctgtctgga atttaccggt tatcttcttt     540

attattaata atcgttatgg tatctcaaca gatatcaatt attctactaa gatttcacat     600
```

```
ctttatttac gtgctgatgc ttatggtatt cctggacatt atgttgaaga tggtaatgat      660

gtcattgcag tttatgaaaa aatgcaggaa gtcattgatt atgtgcgttc aggaaatggg      720

ccagctcttg ttgaagtgga atcttatcgt tggttcggac attctactgc tgatgcagga      780

gcttaccgta caaaagaaga agtagatgct tggaaagcta aagatcctct caagaaatac      840

cgcacttatc taacagaaaa taagattgca acagatgagg aacttgatat gattgaaaaa      900

gaagtcgcac aggaaattga ggatgcagtg aaatttgccc aagatagccc tgaaccagag      960

ctttctgtag cttttgaaga tgtttgggta gattag                                996
```

<210> 15
<211> 16
<212> PRT
<213> Artificial sequence

<220>
<223> Synthetic polypeptide

<220>
<221> MISC_FEATURE
<223> BKD E1alpha motif

<220>
<221> VARIANT
<222> (1)..(1)
<223> Xaa is Ser or Gln

<220>
<221> VARIANT
<222> (2)..(3)
<223> Xaa is any amino acid

<220>
<221> VARIANT
<222> (5)..(5)
<223> Xaa is Gln or Glu

<220>
<221> VARIANT
<222> (8)..(10)
<223> Xaa is any amino acid

<220>
<221> VARIANT
<222> (11)..(11)
<223> Xaa is Gly or Ala

<220>
<221> VARIANT
<222> (12)..(12)
<223> Xaa is any amino acid

<220>
<221> VARIANT
<222> (13)..(13)
<223> Xaa is Gly or Ala

<220>
<221> VARIANT
<222> (14)..(14)
<223> Xaa is any amino acid

<220>
<221> VARIANT
<222> (15)..(15)
<223> Xaa is Val or Ala

<220>
<221> VARIANT
<222> (16)..(16)
<223> Xaa is Leu or Thr

<400> 15

Xaa Xaa Xaa Gly Xaa Glu Ala Xaa Xaa Xaa Xaa Xaa Xaa Xaa Xaa Xaa
1               5                   10                  15

<210> 16
<211> 8
<212> PRT
<213> Artificial sequence

<220>
<223> Synthetic polypeptide

<220>
<221> MISC_FEATURE
<223> BKD E1alpha motif

<220>
<221> VARIANT
<222> (2)..(3)
<223> Xaa is any amino acid

<220>
<221> VARIANT
<222> (4)..(4)
<223> Xaa is Leu or Phe

<220>
<221> VARIANT
<222> (6)..(6)
<223> Xaa is any amino acid

<400> 16

Asp Xaa Xaa Xaa Pro Xaa Tyr Arg
1               5

<210> 17
<211> 11
<212> PRT
<213> Artificial sequence

<220>
<223> Synthetic polypeptide

<220>
<221> MISC_FEATURE
<223> BKD E1alpha motif

<220>
<221> VARIANT
<222> (1)..(1)
<223> Xaa is Ser or Thr

<220>
<221> VARIANT
<222> (3)..(4)
<223> Xaa is any amino acid

<220>
<221> VARIANT
<222> (5)..(5)
<223> Xaa is His or Gln

<220>
<221> VARIANT
<222> (7)..(7)
<223> Xaa is Thr or Val

<220>
<221> VARIANT
<222> (9)..(9)
<223> Xaa is any amino acid

<220>
<221> VARIANT
<222> (11)..(11)
<223> Xaa is Ala or Gly

<400> 17

```
Xaa Gln Xaa Xaa Xaa Ala Xaa Gly Xaa Ala Xaa
1               5                   10
```

<210> 18
<211> 20
<212> PRT
<213> Artificial sequence

<220>
<223> Synthetic polypeptide

<220>
<221> MISC_FEATURE
<223> BKD E1alpha motif

<220>
<221> VARIANT
<222> (1)..(1)
<223> Xaa is Lys or Gly

<220>
<221> VARIANT
<222> (2)..(2)
<223> Xaa is any amino acid

```
<220>
<221>  VARIANT
<222>  (3)..(3)
<223>  Xaa is Thr or Asp

<220>
<221>  VARIANT
<222>  (4)..(5)
<223>  Xaa is any amino acid

<220>
<221>  VARIANT
<222>  (6)..(6)
<223>  Xaa is Ala or Val

<220>
<221>  VARIANT
<222>  (7)..(7)
<223>  Xaa is any amino acid

<220>
<221>  VARIANT
<222>  (8)..(8)
<223>  Xaa is Ala or Val

<220>
<221>  VARIANT
<222>  (9)..(10)
<223>  Xaa is any amino acid

<220>
<221>  VARIANT
<222>  (12)..(12)
<223>  Xaa is Glu or Asp

<220>
<221>  VARIANT
<222>  (14)..(17)
<223>  Xaa is any amino acid

<220>
<221>  VARIANT
<222>  (18)..(18)
<223>  Xaa is Gly or Ser

<220>
<221>  VARIANT
<222>  (20)..(20)
<223>  Xaa is Phe or Val

<400>  18

Xaa Xaa Xaa Xaa Xaa Xaa Xaa Xaa Xaa Xaa Gly Xaa Gly Xaa Xaa Xaa
1               5                   10                  15

Xaa Xaa Asp Xaa
            20

<210>  19
<211>  21
```

<212> PRT
<213> Artificial sequence

<220>
<223> Synthetic polypeptide

<220>
<221> MISC_FEATURE
<223> BKD E1alpha motif

<220>
<221> VARIANT
<222> (2)..(2)
<223> Xaa is Ala or Ser

<220>
<221> VARIANT
<222> (3)..(3)
<223> Xaa is His or Ala

<220>
<221> VARIANT
<222> (5)..(6)
<223> Xaa is any amino acid

<220>
<221> VARIANT
<222> (7)..(7)
<223> Xaa is Leu or Ala

<220>
<221> VARIANT
<222> (10)..(10)
<223> Xaa is any amino acid

<220>
<221> VARIANT
<222> (11)..(11)
<223> Xaa is Leu or Phe

<220>
<221> VARIANT
<222> (12)..(14)
<223> Xaa is any amino acid

<220>
<221> VARIANT
<222> (17)..(18)
<223> Xaa is any amino acid

<400> 19

```
Phe Xaa Xaa Val Xaa Xaa Xaa Pro Val Xaa Xaa Xaa Xaa Xaa Asn Asn
1               5                   10                  15


Xaa Xaa Ala Ile Ser
            20
```

<210> 20
<211> 16

<212> PRT
<213> Artificial sequence

<220>
<223> Synthetic polypeptide

<220>
<221> MISC_FEATURE
<223> BKD E1alpha motif

<220>
<221> VARIANT
<222> (1)..(1)
<223> Xaa is Lys or Arg

<220>
<221> VARIANT
<222> (2)..(2)
<223> Xaa is Gly or Ala

<220>
<221> VARIANT
<222> (3)..(3)
<223> Xaa is any amino acid

<220>
<221> VARIANT
<222> (5)..(5)
<223> Xaa is Cys or Tyr

<220>
<221> VARIANT
<222> (6)..(6)
<223> Xaa is Phe or Gly

<220>
<221> VARIANT
<222> (7)..(7)
<223> Xaa is any amino acid

<220>
<221> VARIANT
<222> (8)..(8)
<223> Xaa is Ala or Pro

<220>
<221> VARIANT
<222> (9)..(9)
<223> Xaa is Ser or Gly

<220>
<221> VARIANT
<222> (10)..(11)
<223> Xaa is any amino acid

<400> 20

```
Xaa Xaa Xaa Gly Xaa Xaa Xaa Xaa Xaa Xaa Xaa Val Asp Gly Asn Asp
1               5                   10                  15
```

<210> 21

<211> 31
<212> PRT
<213> Artificial sequence

<220>
<223> Synthetic polypeptide

<220>
<221> MISC_FEATURE
<223> BKD E1alpha motif

<220>
<221> VARIANT
<222> (1)..(1)
<223> Xaa is His or Arg

<220>
<221> VARIANT
<222> (4)..(4)
<223> Xaa is Ala or Arg

<220>
<221> VARIANT
<222> (6)..(6)
<223> Xaa is any amino acid

<220>
<221> VARIANT
<222> (9)..(9)
<223> Xaa is any amino acid

<220>
<221> VARIANT
<222> (11)..(11)
<223> Xaa is any amino acid

<220>
<221> VARIANT
<222> (13)..(14)
<223> Xaa is any amino acid

<220>
<221> VARIANT
<222> (15)..(15)
<223> Xaa is Ser or Thr

<220>
<221> VARIANT
<222> (18)..(20)
<223> Xaa is any amino acid

<220>
<221> VARIANT
<222> (22)..(24)
<223> Xaa is any amino acid

<220>
<221> VARIANT
<222> (27)..(29)
<223> Xaa is any amino acid

<400> 21

Xaa Ala Arg Xaa Gly Xaa Gly Pro Xaa Leu Xaa Glu Xaa Xaa Xaa Tyr
1 5 10 15

Arg Xaa Xaa Xaa His Xaa Xaa Xaa Asp Asp Xaa Xaa Xaa Tyr Arg
20 25 30

<210> 22
<211> 327
<212> PRT
<213> Bacillus subtilis

<220>
<221> MISC_FEATURE
<223> BKD E1beta subunit

<400> 22

Met Ser Val Met Ser Tyr Ile Asp Ala Ile Asn Leu Ala Met Lys Glu
1 5 10 15

Glu Met Glu Arg Asp Ser Arg Val Phe Val Leu Gly Glu Asp Val Gly
20 25 30

Arg Lys Gly Gly Val Phe Lys Ala Thr Ala Gly Leu Tyr Glu Gln Phe
35 40 45

Gly Glu Glu Arg Val Met Asp Thr Pro Leu Ala Glu Ser Ala Ile Ala
50 55 60

Gly Val Gly Ile Gly Ala Ala Met Tyr Gly Met Arg Pro Ile Ala Glu
65 70 75 80

Met Gln Phe Ala Asp Phe Ile Met Pro Ala Val Asn Gln Ile Ile Ser
85 90 95

Glu Ala Ala Lys Ile Arg Tyr Arg Ser Asn Asn Asp Trp Ser Cys Pro
100 105 110

Ile Val Val Arg Ala Pro Tyr Gly Gly Gly Val His Gly Ala Leu Tyr
115 120 125

His Ser Gln Ser Val Glu Ala Ile Phe Ala Asn Gln Pro Gly Leu Lys
130 135 140

Ile Val Met Pro Ser Thr Pro Tyr Asp Ala Lys Gly Leu Leu Lys Ala
145 150 155 160

Ala Val Arg Asp Glu Asp Pro Val Leu Phe Phe Glu His Lys Arg Ala
165 170 175

Tyr Arg Leu Ile Lys Gly Glu Val Pro Ala Asp Asp Tyr Val Leu Pro
180 185 190

Ile Gly Lys Ala Asp Val Lys Arg Glu Gly Asp Asp Ile Thr Val Ile
195 200 205

Thr Tyr Gly Leu Cys Val His Phe Ala Leu Gln Ala Ala Glu Arg Leu
210 215 220

Glu Lys Asp Gly Ile Ser Ala His Val Val Asp Leu Arg Thr Val Tyr
225 230 235 240

Pro Leu Asp Lys Glu Ala Ile Ile Glu Ala Ala Ser Lys Thr Gly Lys
245 250 255

Val Leu Leu Val Thr Glu Asp Thr Lys Glu Gly Ser Ile Met Ser Glu
260 265 270

Val Ala Ala Ile Ile Ser Glu His Cys Leu Phe Asp Leu Asp Ala Pro
275 280 285

Ile Lys Arg Leu Ala Gly Pro Asp Ile Pro Ala Met Pro Tyr Ala Pro
290 295 300

Thr Met Glu Lys Tyr Phe Met Val Asn Pro Asp Lys Val Glu Ala Ala
305 310 315 320

Met Arg Glu Leu Ala Glu Phe
325

<210> 23
<211> 984
<212> DNA
<213> Bacillus subtilis

<220>
<221> misc_feature
<223> Encodes BKD E1beta subunit

<400> 23
ttaaaactcc gctaattctc tcatcgccgc ttccacttta tcagggttga ccataaagta 60

tttttccatt gtcggcgcat aaggcatagc cggaatatca ggacctgcaa gccgtttgat 120

cggcgcgtct aagtcgaaca gacaatgctc ggatataatt gcggctactt cgctcatgat 180

gctgccttct tttgtatctt ctgtgaccaa aagaaccttt ccagttttgg acgcagcttc 240

gatgatggct tctttatcaa gcgggtaaac tgttcttaaa tccaccacat gcgctgaaat 300

gccatctttt tcgagacgtt ctgcagcttg taaggcgaag tggacacaca ggccgtatgt 360

gatcactgtg atgtcgtcgc cttccctttt tacgtccgcc ttgccgattg gcaggacata 420

atcatcagcc ggaacctcgc cctttatcag acggtatgcc cgcttgtgct caaaaaacag 480

cacggggtct tcgtcacgaa ctgcggcttt taagagccct ttcgcgtcat atggtgttga 540

tggcatgaca attttcagtc cgggctggtt ggcgaaaatt gcttcgactg attgagaatg 600

atacagggct ccgtgcacgc ctccgccgta tggcgctctg acgacaatcg gacagctcca 660

gtcattgttg ctgcggtagc ggattttagc cgcttcagaa ataatttggt tgactgccgg 720

cataatgaaa tcagcaaact gcatttcagc aatcggtctc attccgtaca ttgccgctcc 780

gataccgact cctgcgattg cagattcagc aagcggcgta tccataacgc gctcttcccc 840

aaattgttca tagagtcccg ctgtcgcttt aaacacaccg ccttttcttc ctacatcttc 900

cccaaggacg aaaacgcgag aatctcgttc catttcttct ttcatcgcca aattgattgc 960

atcaatatat gacattactg acat 984

<210> 24
<211> 334
<212> PRT
<213> Streptomyces avermitilis

<220>
<221> MISC_FEATURE
<223> BKD E1beta subunit

<400> 24

Met Thr Thr Val Ala Leu Lys Pro Ala Thr Met Ala Gln Ala Leu Thr
1 5 10 15

Arg Ala Leu Arg Asp Ala Met Ala Ala Asp Pro Ala Val His Val Met
20 25 30

Gly Glu Asp Val Gly Thr Leu Gly Gly Val Phe Arg Val Thr Asp Gly
35 40 45

Leu Ala Lys Glu Phe Gly Glu Asp Arg Cys Thr Asp Thr Pro Leu Ala
50 55 60

Glu Ala Gly Ile Leu Gly Thr Ala Val Gly Met Ala Met Tyr Gly Leu
65 70 75 80

Arg Pro Val Val Glu Met Gln Phe Asp Ala Phe Ala Tyr Pro Ala Phe
85 90 95

Glu Gln Leu Ile Ser His Val Ala Arg Met Arg Asn Arg Thr Arg Gly
100 105 110

```
Ala Met Pro Leu Pro Ile Thr Ile Arg Val Pro Tyr Gly Gly Gly Ile
        115                 120                 125

Gly Gly Val Glu His His Ser Asp Ser Ser Glu Ala Tyr Tyr Met Ala
    130                 135                 140

Thr Pro Gly Leu His Val Val Thr Pro Ala Thr Val Ala Asp Ala Tyr
145                 150                 155                 160

Gly Leu Leu Arg Ala Ala Ile Ala Ser Asp Asp Pro Val Val Phe Leu
                165                 170                 175

Glu Pro Lys Arg Leu Tyr Trp Ser Lys Asp Ser Trp Asn Pro Asp Glu
            180                 185                 190

Pro Gly Thr Val Glu Pro Ile Gly Arg Ala Val Val Arg Arg Ser Gly
        195                 200                 205

Arg Ser Ala Thr Leu Ile Thr Tyr Gly Pro Ser Leu Pro Val Cys Leu
    210                 215                 220

Glu Ala Ala Glu Ala Ala Arg Ala Glu Gly Trp Asp Leu Glu Val Val
225                 230                 235                 240

Asp Leu Arg Ser Leu Val Pro Phe Asp Asp Glu Thr Val Cys Ala Ser
                245                 250                 255

Val Arg Arg Thr Gly Arg Ala Val Val Val His Glu Ser Gly Gly Tyr
            260                 265                 270

Gly Gly Pro Gly Gly Glu Ile Ala Ala Arg Ile Thr Glu Arg Cys Phe
        275                 280                 285

His His Leu Glu Ala Pro Val Leu Arg Val Ala Gly Phe Asp Ile Pro
    290                 295                 300

Tyr Pro Pro Pro Met Leu Glu Arg His His Leu Pro Gly Val Asp Arg
305                 310                 315                 320

Ile Leu Asp Ala Val Gly Arg Leu Gln Trp Glu Ala Gly Ser
                325                 330
```

<210> 25
<211> 1005
<212> DNA
<213> Streptomyces avermitilis

<220>
<221> misc_feature
<223> Encodes BKD E1beta subunit

<400> 25
atgaccaccg ttgccctcaa gccggccacc atggcgcagg cactcacacg cgcgttgcgt 60

gacgccatgg ccgccgaccc cgccgtccac gtgatgggcg aggacgtcgg cacgctcggc 120

ggggtcttcc gggtcaccga cgggctcgcc aaggagttcg gcgaggaccg ctgcacggac 180

acgccgctcg ccgaggcagg catcctcggc acggccgtcg gcatggcgat gtacgggctg 240

cggccggtcg tcgagatgca gttcgacgcg ttcgcgtacc cggcgttcga gcagctcatc 300

agccatgtcg cgcggatgcg caaccgcacc cgcggggcga tgccgctgcc gatcaccatc 360

cgtgtcccct acggcggcgg aatcggcgga gtcgaacacc acagcgactc ctccgaggcg 420

tactacatgg cgactccggg gctccatgtc gtcacgcccg ccacggtcgc cgacgcgtac 480

gggctgctgc gcgccgccat cgcctccgac gacccggtcg tcttcctgga gcccaagcgg 540

ctgtactggt cgaaggactc ctggaacccg gacgagccgg ggaccgttga accgataggc 600

cgcgcggtgg tgcggcgctc gggccggagc gccacgctca tcacgtacgg gccttccctg 660

cccgtctgcc tggaggcggc cgaggcggcc cgggccgagg gctgggacct cgaagtcgtc 720

gatctgcgct ccctggtgcc cttcgacgac gagacggtgt gcgcgtcggt gcgccggacc 780

ggacgcgccg tcgtcgtgca cgagtcgggt ggttacggcg gcccgggcgg ggagatcgcc 840

gcgcggatca ccgagcgctg cttccaccat ctggaggcgc cggtgctgcg cgtcgccggg 900

ttcgacatcc cgtatccgcc gccgatgctg gagcgccatc atctgcccgg tgtcgaccgg 960

atcctggacg cggtggggcg gcttcagtgg gaggcgggga gctga 1005

<210> 26
<211> 352
<212> PRT
<213> Pseudomonas putida

<220>
<221> MISC_FEATURE
<223> BKD E1beta subunit

<400> 26

Met Asn Asp His Asn Asn Ser Ile Asn Pro Glu Thr Ala Met Ala Thr
1 5 10 15

Thr Thr Met Thr Met Ile Gln Ala Leu Arg Ser Ala Met Asp Val Met
20 25 30

Leu Glu Arg Asp Asp Asn Val Val Val Tyr Gly Gln Asp Val Gly Tyr
35 40 45

```
Phe Gly Gly Val Phe Arg Cys Thr Glu Gly Leu Gln Asn Lys Tyr Gly
    50                  55                  60

Lys Ser Arg Val Phe Asp Ala Pro Ile Ser Glu Ser Gly Ile Val Gly
65                  70                  75                  80

Thr Ala Val Gly Met Gly Ala Tyr Gly Leu Arg Pro Val Val Glu Ile
                85                  90                  95

Gln Phe Ala Asp Tyr Phe Tyr Pro Ala Ser Asp Gln Ile Val Ser Glu
            100                 105                 110

Leu Ala Arg Leu Arg Tyr Arg Ser Ala Gly Glu Phe Ile Ala Pro Leu
        115                 120                 125

Thr Leu Arg Met Pro Cys Gly Gly Gly Ile Tyr Gly Gly Gln Thr His
    130                 135                 140

Ser Gln Ser Pro Glu Ala Met Phe Thr Gln Val Cys Gly Leu Arg Thr
145                 150                 155                 160

Val Met Pro Ser Asn Pro Tyr Asp Ala Lys Gly Leu Leu Ile Ala Ser
                165                 170                 175

Ile Glu Cys Asp Asp Pro Val Ile Phe Leu Glu Pro Lys Arg Leu Tyr
            180                 185                 190

Asn Gly Pro Phe Asp Gly His His Asp Arg Pro Val Thr Pro Trp Ser
        195                 200                 205

Lys His Pro His Ser Ala Val Pro Asp Gly Tyr Tyr Thr Val Pro Leu
    210                 215                 220

Asp Lys Ala Ala Ile Thr Arg Pro Gly Asn Asp Val Thr Val Leu Thr
225                 230                 235                 240

Tyr Gly Thr Thr Val Tyr Val Ala Gln Val Ala Ala Glu Glu Ser Gly
                245                 250                 255

Val Asp Ala Glu Val Ile Asp Leu Arg Ser Leu Trp Pro Leu Asp Leu
            260                 265                 270

Asp Thr Ile Val Glu Ser Val Lys Lys Thr Gly Arg Cys Val Val Val
        275                 280                 285

His Glu Ala Thr Arg Thr Cys Gly Phe Gly Ala Glu Leu Val Ser Leu
```

290 295 300

Val Gln Glu His Cys Phe His His Leu Glu Ala Pro Ile Glu Arg Val
305 310 315 320

Thr Gly Trp Asp Thr Pro Tyr Pro His Ala Gln Glu Trp Ala Tyr Phe
325 330 335

Pro Gly Pro Ser Arg Val Gly Ala Ala Leu Lys Lys Val Met Glu Val
340 345 350

<210> 27
<211> 1059
<212> DNA
<213> Pseudomonas putida

<220>
<221> misc_feature
<223> Encodes BKD E1beta subunit

<400> 27
tcagacctcc atgacctttt tcagtgccgc acctacccgc gaagggccgg ggaagtaagc 60

ccattcctgt gcgtgagggt agggggtgtc ccagccggtg acgcgctcga tcggcgcctc 120

caggtggtgg aagcagtgct cctgcaccag cgacaccagc tcggcaccga agccgcaggt 180

gcgggtggcc tcgtgcacca ccacgcaacg gccagtcttt ttcaccgact cgacgatagt 240

gtccaggtcc agcggccaca ggctgcgcag gtcgatcact tcggcatcga cgccgctttc 300

ttcggcggcc acctgggcca cgtacaccgt ggtgccgtaa gtcagtacgg tcacgtcatt 360

gccagggcgg gtaatggcgg ccttgtccag cggtacggtg taatagccgt cgggcacggc 420

gctgtgcggg tgcttcgacc atggggttac agggcggtcg tggtggccat cgaacgggcc 480

gttgtacaga cgtttgggct ccaggaagat taccgggtcg tcgcattcga tcgaggcaat 540

cagcaggcct ttggcgtcat aagggttgga cggcatcacg gtgcgcaggc cgcagacctg 600

ggtgaacatc gcttccgggc tctggctgtg agtctggccg ccatagatgc cgccgccgca 660

aggcatgcgc agggtcagcg gggcaatgaa ctcgccggcc gaccggtaac gcaggcgggc 720

cagctcggag acgatctggt cggaggccgg gtagaagtag tcggcgaact ggatctccac 780

caccgggcgc aggccatagg cgcccatgcc tacggcggta ccgacgatgc cgctctcgga 840

gatgggcgcg tcgaacacgc gcgatttgcc gtacttgttc tgcaggcctt cggtgcagcg 900

gaacacgccg ccgaagtaac cgacgtcctg gccgtacacc accacattgt cgtcgcgctc 960

aagcatgaca tccatggccg agcgcagggc ctggatcatg gtcatggtag tggtggccat 1020

ggcggtttcc gggttgatgc tgttgttgtg gtcgttcat 1059

<210> 28
<211> 327
<212> PRT
<213> Listeria monocytogenes

<220>
<221> MISC_FEATURE
<223> BKD E1beta subunit

<400> 28

Met Pro Val Ile Ser Tyr Ile Asp Ala Ile Thr Met Ala Leu Lys Glu
1               5                   10                  15

Glu Met Glu Arg Asp Asp Lys Val Phe Ile Leu Gly Glu Asp Val Gly
            20                  25                  30

Lys Lys Gly Gly Val Phe Lys Ala Thr Ala Gly Leu Tyr Asp Glu Phe
        35                  40                  45

Gly Glu Asp Arg Val Leu Asp Thr Pro Leu Ala Glu Ser Ala Ile Ala
    50                  55                  60

Gly Val Gly Ile Gly Ala Ala Met Tyr Gly Tyr Arg Pro Val Ala Glu
65                  70                  75                  80

Met Gln Phe Ala Asp Phe Ile Met Pro Ala Val Asn Gln Ile Ile Ser
                85                  90                  95

Glu Ala Ala Arg Ile Arg Tyr Arg Ser Asn Asn Asp Trp Ser Cys Pro
            100                 105                 110

Met Val Ile Arg Ala Pro Phe Gly Gly Gly Val His Gly Ala Leu Tyr
        115                 120                 125

His Ser Gln Ser Val Glu Lys Val Phe Phe Gly Gln Pro Gly Leu Lys
    130                 135                 140

Ile Val Val Pro Ser Ser Pro Tyr Asp Ala Lys Gly Leu Leu Lys Ala
145                 150                 155                 160

Ala Ile Arg Asp Asn Asp Pro Val Leu Phe Phe Glu His Lys Arg Ala
                165                 170                 175

Tyr Arg Leu Leu Lys Gly Glu Val Pro Glu Thr Asp Tyr Ile Val Pro
            180                 185                 190

Ile Gly Glu Ala Asn Val Val Arg Glu Gly Asp Asp Ile Thr Val Ile
        195                 200                 205

Thr Tyr Gly Leu Ala Val Gln Phe Ala Gln Gln Ala Ala Glu Arg Leu
    210                 215                 220

Ala Ala Glu Gly Val Glu Ala His Ile Leu Asp Leu Arg Thr Ile Tyr
225                 230                 235                 240

Pro Leu Asp Gln Glu Ala Ile Ile Glu Ala Thr Lys Lys Thr Gly Lys
                245                 250                 255

Val Leu Leu Val Thr Glu Asp Asn Lys Gln Gly Ser Ile Ile Ser Glu
            260                 265                 270

Val Ala Ala Ile Ile Ser Glu His Cys Leu Phe Asp Leu Asp Ala Pro
        275                 280                 285

Ile Ala Arg Leu Ala Gly Pro Asp Thr Pro Ala Met Pro Phe Ala Pro
    290                 295                 300

Thr Met Glu Lys His Phe Met Ile Asn Pro Asp Lys Val Ala Asp Ala
305                 310                 315                 320

Met Lys Glu Leu Ala Glu Phe
                325

<210> 29
<211> 984
<212> DNA
<213> Listeria monocytogenes

<220>
<221> misc_feature
<223> Encodes BKD E1beta subunit

<400> 29
atgccagtca tttcatatat tgatgcaata accatggcgc ttaaagaaga aatggagcgc 60

gatgataaag tatttatttt aggagaagat gttgggaaaa aaggtggcgt atttaaagcg 120

actgctggtc tatatgacga atttggtgaa gacagagtac ttgatacacc acttgctgaa 180

tctgccattg ccggagttgg aattggcgcg gcgatgtatg gctaccgccc agttgcagaa 240

atgcaatttg ctgactttat tatgccagct gtcaaccaaa tcatttcaga agctgccaga 300

attcggtacc gttctaataa cgattggtct tgtccaatgg ttattcgcgc acctttttggc 360

ggcggggtac acggggcact ttaccattca caatctgttg aaaaagtgtt tttcggacaa 420

cctggtttga aaatcgttgt tccttcttca ccatatgatg caaaagggct tttaaaagcg 480

gcgattcgcg ataatgatcc agtgcttttc tttgagcata aacgtgcgta ccgcttgcta 540

aaaggcgaag tgccagaaac tgattatatc gttccaatcg gcgaagcaaa tgttgttcgt 600

gaaggtgatg atattacagt aattacttac ggacttgcgg ttcaatttgc ccaacaagca 660

gcagaacgtt tagcagcgga aggcgtagaa gcacatattc ttgatttacg gacaatctat 720

ccactagacc aagaagcaat tattgaagca acgaaaaaaa caggtaaagt acttcttgta 780

acggaagata acaaacaagg aagtattatc agtgaagtgg cagcaatcat ttcggagcat 840

tgtttatttg acttagacgc accgattgct agactcgcag gacctgatac cccagcgatg 900

ccttttgctc caacaatgga aaaacatttt atgatcaatc cagataaagt ggcggatgca 960

atgaaagaat tagcggaatt ttag 984

<210> 30
<211> 355
<212> PRT
<213> Micrococcus luteus

<220>
<221> MISC_FEATURE
<223> BKD E1beta subunit

<400> 30

Met Ser Glu Arg Met Thr Phe Gly Arg Ala Ile Asn Arg Gly Leu His
1 5 10 15

Arg Ala Leu Ala Asp Asp Pro Lys Val Leu Leu Met Gly Glu Asp Ile
20 25 30

Gly Ala Leu Gly Gly Val Phe Arg Ile Thr Asp Gly Leu Gln Ala Glu
35 40 45

Phe Gly Glu Asp Arg Val Leu Asp Thr Pro Leu Ala Glu Ser Gly Ile
50 55 60

Val Gly Thr Ala Ile Gly Leu Ala Met Arg Gly Tyr Arg Pro Val Val
65 70 75 80

Glu Ile Gln Phe Asp Gly Phe Val Tyr Pro Ala Phe Asp Gln Ile Val
85 90 95

Ala Asn Leu Ala Lys Leu Arg Ala Arg Thr Arg Gly Ala Val Pro Met
100 105 110

Pro Val Thr Ile Arg Ile Pro Phe Gly Gly Gly Ile Gly Ser Pro Glu
115 120 125

His His Ser Glu Ser Pro Glu Ala Tyr Phe Leu His Thr Ala Gly Leu
130 135 140

```
Arg Val Val Ser Pro Ser Ser Pro Gln Glu Gly Tyr Asp Leu Ile Arg
145                 150                 155                 160

Ala Ala Ile Ala Ser Glu Asp Pro Val Val Tyr Leu Glu Pro Lys Arg
                165                 170                 175

Arg Tyr His Asp Lys Gly Asp Val Asp Leu Gly Val Ala Ile Pro Pro
            180                 185                 190

Met Ser Pro Ala Arg Ile Leu Arg Glu Gly Arg Asp Ala Thr Leu Val
        195                 200                 205

Ala Tyr Gly Pro Leu Val Lys Thr Ala Leu Gln Ala Ala Glu Val Ala
    210                 215                 220

Ala Glu Glu Gly Val Glu Val Glu Val Val Asp Leu Arg Ser Leu Ser
225                 230                 235                 240

Pro Leu Asp Thr Gly Leu Val Glu Ser Ser Val Arg Arg Thr Gly Arg
                245                 250                 255

Leu Val Val Ala His Glu Ala Ser Arg Thr Gly Gly Leu Gly Ala Glu
            260                 265                 270

Leu Val Ala Thr Val Ala Glu Arg Ala Phe His Trp Leu Glu Ala Pro
        275                 280                 285

Pro Val Arg Val Thr Gly Met Asp Val Pro Tyr Pro Pro Ser Lys Leu
    290                 295                 300

Glu His Leu His Leu Pro Asp Leu Asp Arg Ile Leu Asp Gly Leu Asp
305                 310                 315                 320

Arg Ala Leu Gly Arg Pro Asn Ser Leu Asp Ser Val Asp Ala Phe Ala
                325                 330                 335

Ala Pro Glu Thr Ala Glu Gln Phe Leu Ala Ala Gln Asn Ala Gly Glu
            340                 345                 350

Glu Thr Arg
        355


<210>  31
<211>  1068
<212>  DNA
<213>  Micrococcus luteus
```

<220>
<221> misc_feature
<223> Encodes BKD E1beta subunit

<400> 31
gtgagcgagc gcatgacctt cggccgtgcg atcaaccgcg gcctgcaccg tgccctggcc 60

gacgacccca aggtcctgct catgggcgag gacatcggcg ccctcggcgg cgtgttccgc 120

atcaccgacg gcctgcaggc cgagttcggc gaggaccggg tgctcgacac cccgctggcc 180

gagtccggca tcgtgggcac ggccatcggc ctggcgatgc gcggctaccg gcccgtcgtc 240

gagatccagt tcgacggctt cgtgtacccg gcgttcgacc agatcgtggc gaacctggcc 300

aagctgcgcg cccgcacccg cggcgccgtg ccgatgccgg tgaccatccg catccccttc 360

ggcggcggca tcggctcccc ggagcaccac tccgagtcgc ccgaggccta cttcctgcac 420

accgcgggtc tgcgcgtggt ctccccgtcc tccccgcagg aggggtacga cctcatccgc 480

gccgcgatcg cctcggagga cccggtggtc tacctcgagc ccaagcgtcg ctaccacgac 540

aagggcgacg tggacctggg cgtcgcgatc ccgccgatga gcccggcccg catcctgcgc 600

gagggccgtg acgccacgct cgtggcctac ggcccgctcg tgaagaccgc cctgcaggcc 660

gccgaggtgg cggccgagga gggtgtcgag gtcgaggtgg tcgacctgcg cagcctgtcc 720

ccgctggaca ccggcctcgt cgagtcctcg gtgcggcgca ccggtcggct cgtcgtggcg 780

cacgaggcct cccgcacggg cggcctcggc gccgagctcg tggccacggt ggccgagcgc 840

gcgttccatt ggctcgaggc cccgccggtg cgcgtcaccg gcatggacgt gccctacccg 900

ccgtccaagc tcgagcacct gcacctgccg gacctcgacc gcatcctcga cggcctggac 960

cgtgctctgg gccggccgaa ttcgctggac tccgtggacg cgttcgccgc ccccgagacc 1020

gccgagcagt tcctcgccgc ccagaacgcc ggggaggaga cccgatga 1068

<210> 32
<211> 327
<212> PRT
<213> Staphylococcus aureus

<220>
<221> MISC_FEATURE
<223> BKD E1beta subunit

<400> 32

Met Ala Lys Leu Ser Tyr Leu Glu Ala Ile Arg Gln Ala Gln Asp Leu
1 5 10 15

Ala Leu Gln Gln Asn Lys Asp Val Phe Ile Leu Gly Glu Asp Val Gly
20 25 30

Lys Lys Gly Gly Val Phe Gly Thr Thr Gln Gly Leu Gln Gln Gln Tyr

35 40 45

Gly Glu Asp Arg Val Ile Asp Thr Pro Leu Ala Glu Ser Asn Ile Val
50 55 60

Gly Thr Ala Ile Gly Ala Ala Met Val Gly Lys Arg Pro Ile Ala Glu
65 70 75 80

Ile Gln Phe Ala Asp Phe Ile Leu Pro Ala Thr Asn Gln Ile Ile Ser
85 90 95

Glu Ala Ala Lys Met Arg Tyr Arg Ser Asn Asn Asp Trp Gln Cys Pro
100 105 110

Leu Thr Ile Arg Ala Pro Phe Gly Gly Gly Val His Gly Gly Leu Tyr
115 120 125

His Ser Gln Ser Ile Glu Ser Ile Phe Ala Ser Ser Pro Gly Leu Thr
130 135 140

Ile Val Ile Pro Ser Thr Pro Tyr Asp Ala Lys Gly Leu Leu Leu Ser
145 150 155 160

Ser Ile Glu Ser Asn Asp Pro Val Leu Tyr Phe Glu His Lys Lys Ala
165 170 175

Tyr Arg Phe Leu Lys Glu Glu Val Pro Glu Glu Tyr Tyr Thr Val Pro
180 185 190

Leu Gly Lys Ala Asp Val Lys Arg Glu Gly Glu Asp Leu Thr Val Phe
195 200 205

Cys Tyr Gly Leu Met Val Asn Tyr Cys Leu Gln Ala Ala Asp Ile Leu
210 215 220

Ala Ala Asp Gly Ile Asn Val Glu Val Val Asp Leu Arg Thr Val Tyr
225 230 235 240

Pro Leu Asp Lys Glu Thr Ile Ile Asp Arg Ala Lys Asn Thr Gly Lys
245 250 255

Val Leu Leu Val Thr Glu Asp Asn Leu Glu Gly Ser Ile Met Ser Glu
260 265 270

Val Ser Ala Ile Ile Ala Glu His Cys Leu Phe Asp Leu Asp Thr Pro
275 280 285

Ile Met Arg Leu Ala Ala Pro Asp Val Pro Ser Met Pro Phe Ser Pro
    290                 295                 300

Val Leu Glu Asn Glu Ile Met Met Asn Pro Glu Lys Ile Leu Asn Lys
305                 310                 315                 320

Met Arg Glu Leu Ala Glu Phe
                325

<210> 33
<211> 984
<212> DNA
<213> Staphylococcus aureus

<220>
<221> misc_feature
<223> Encodes BKD E1beta subunit

<400> 33
ctagaattct gctaattcac gcattttatt taagattttt tctggattca tcataatttc 60

attttctaat acaggagaaa atggcataga tggtacatct ggagcagcta aacgcatgat 120

tggtgtatct aaatcgaaca agcaatgctc tgcaataatc gctgacactt ctgacataat 180

actaccttct aaattatctt cagttacaag taaaacttta cctgtatttt tagcacgatc 240

aataattgtt tctttatcta atggataaac agttcgtaaa tcaacgactt caacgttgat 300

accgtctgca gctaaaatat ccgctgcttg taaacaataa ttgaccatta atccataaca 360

aaatactgtt aaatcttcac cttcacgttt aacatctgct tttcctaaag gtacagtgta 420

atattcttct ggcacttctt cctttaagaa acgataagct tttttatgct caaagtacaa 480

tactggatca tttgattcga tagatgataa taaaagccct ttagcatcat acggtgtgga 540

aggaataaca attgttaaac ctggtgatga agcaaatata ctttcaatac tttgtgaatg 600

atatagtcct ccgtgaacac cgccaccaaa tggtgcacga atcgttaatg ggcattgcca 660

atcattattt gaacgataac gcattttcgc agcttcacta ataatttgat ttgtcgcagg 720

taaaataaaa tctgcaaatt gaatttctgc aattggtctt ttacctacca tagctgcacc 780

aatggcagtt ccaacaatat ttgactcagc taatggcgta tcgataactc tgtcttcacc 840

atattgttgt tgtagtcctt gagtagtacc aaatacgcca cctttttac caacatcttc 900

accaagaata aacacatctt tattttgttg taatgctaag tcttgtgcct ggcgtatcgc 960

ctctaaataa gataatttag ccat 984

<210> 34
<211> 337
<212> PRT
<213> Streptococcus mutans

<220>
<221>  MISC_FEATURE
<223>  BKD E1beta subunit

<400>  34

```
Met Arg Arg Lys Arg Tyr Met Ser Glu Thr Lys Val Val Ala Leu Arg
1               5                   10                  15

Glu Ala Ile Asn Leu Ala Met Ser Glu Glu Met Arg Lys Asp Glu Lys
            20                  25                  30

Ile Ile Leu Met Gly Glu Asp Val Gly Ile Tyr Gly Gly Asp Phe Gly
        35                  40                  45

Thr Ser Val Gly Met Leu Ala Glu Phe Gly Glu Lys Arg Val Lys Asp
    50                  55                  60

Thr Pro Ile Ser Glu Ala Ala Ile Ala Gly Ser Ala Val Gly Ala Ala
65                  70                  75                  80

Gln Thr Gly Leu Arg Pro Ile Val Asp Leu Thr Phe Met Asp Phe Val
                85                  90                  95

Thr Ile Ala Met Asp Ala Ile Val Asn Gln Gly Ala Lys Ala Asn Tyr
            100                 105                 110

Met Phe Gly Gly Gly Leu Lys Thr Pro Val Thr Phe Arg Val Ala Ser
        115                 120                 125

Gly Ser Gly Ile Gly Ser Ala Ala Gln His Ser Gln Ser Leu Glu Ala
    130                 135                 140

Trp Leu Thr His Ile Pro Gly Ile Lys Val Val Ala Pro Gly Thr Val
145                 150                 155                 160

Asn Asp Ala Lys Ala Leu Leu Lys Ser Ala Ile Arg Asp Asn Asn Ile
                165                 170                 175

Val Ile Phe Met Glu Pro Lys Ala Leu Tyr Gly Lys Lys Glu Glu Val
            180                 185                 190

Asn Leu Asp Pro Asp Phe Tyr Ile Pro Leu Gly Lys Gly Glu Ile Lys
        195                 200                 205

Arg Glu Gly Thr Asp Val Thr Ile Val Ser Tyr Gly Arg Met Leu Glu
    210                 215                 220
```

```
Arg Val Leu Lys Ala Ala Glu Glu Val Ala Ala Glu Asp Ile Ser Val
225                 230                 235                 240

Glu Val Val Asp Pro Arg Thr Leu Ile Pro Leu Asp Lys Asp Leu Ile
                245                 250                 255

Ile Asn Ser Val Lys Lys Thr Gly Lys Val Ile Leu Val Asn Asp Ala
            260                 265                 270

Tyr Lys Thr Gly Gly Phe Ile Gly Glu Ile Ala Ser Val Ile Thr Glu
        275                 280                 285

Ser Glu Ala Phe Asp Tyr Leu Asp Ala Pro Val Leu Arg Leu Ala Ser
    290                 295                 300

Glu Asp Val Pro Val Pro Tyr Ser His Val Leu Glu Thr Ala Ile Leu
305                 310                 315                 320

Pro Asp Val Ala Lys Ile Lys Glu Ala Ile Tyr Lys Gln Val Arg Lys
                325                 330                 335

Arg


<210>  35
<211>  1014
<212>  DNA
<213>  Streptococcus mutans


<220>
<221>  misc_feature
<223>  Encodes BKD E1beta subunit

<400>  35
atgaggagaa agagatatat gtcagaaaca aaagtagtag ccttacggga agctatcaat      60

cttgctatga gcgaggaaat gcgtaaggac gaaaaaatta ttttgatggg tgaagatgtc     120

ggtatttatg gtggtgactt tggaacttct gttggtatgc tggctgaatt tggtgaaaag     180

cgtgttaaag atacccctat ttcagaagca gccattgcag gatctgcagt aggtgccgct     240

caaactggac ttcgtcctat tgttgatttg acctttatgg actttgtgac tattgccatg     300

gatgctattg ttaatcaagg tgctaaagcc aattatatgt ttggcggcgg acttaaaacg     360

cctgtaacct ttcgtgtggc ctcaggctca ggtatcggct cagcagcgca gcattctcag     420

tcactagaag cttggttaac tcatattccg ggaatcaagg tggttgcgcc tggcacagtc     480

aatgatgcta aagccttgct caaatctgct attcgtgata ataatatcgt tattttcatg     540

gaaccaaaag cgctttatgg caaaaaagaa gaggtcaatt tagatcctga tttttatatt     600
```

```
ccgcttggta aaggcgaaat taagcgcgag ggaacagatg ttaccattgt gtcttatggt      660

cgtatgctgg aacgcgttct caaagccgct gaggaagtgg cggctgaaga tatcagtgtt      720

gaagttgttg acccgcgtac ccttattccg cttgataaag acttaattat taattctgtg      780

aaaaagacgg gtaaggttat cctagttaat gatgcttata aaacaggtgg tttcattggt      840

gaaatagcat cagtgattac tgaaagcgaa gcatttgatt atttagatgc accagtgctt      900

cgtctcgctt ctgaggatgt gcctgttccc tattctcatg ttctcgaaac agccatttta      960

ccagatgtgg caaaaattaa agaagctatc tataaacaag tcaggaaaag atag           1014


<210>  36
<211>  21
<212>  PRT
<213>  Artificial sequence

<220>
<223>  Synthetic polypeptide


<220>
<221>  MISC_FEATURE
<223>  BKD E1beta motif

<220>
<221>  VARIANT
<222>  (2)..(2)
<223>  Xaa is any amino acid

<220>
<221>  VARIANT
<222>  (3)..(3)
<223>  Xaa is Val or Ile

<220>
<221>  VARIANT
<222>  (4)..(4)
<223>  Xaa is any amino acid

<220>
<221>  VARIANT
<222>  (6)..(6)
<223>  Xaa is Gln or Glu

<220>
<221>  VARIANT
<222>  (10)..(11)
<223>  Xaa is any amino acid

<220>
<221>  VARIANT
<222>  (16)..(16)
<223>  Xaa is Arg or Lys

<220>
<221>  VARIANT
<222>  (17)..(17)
<223>  Xaa is any amino acid
```

<220>
<221> VARIANT
<222> (19)..(19)
<223> Xaa is any amino acid

<400> 36

Val Xaa Xaa Xaa Gly Xaa Asp Val Gly Xaa Xaa Gly Gly Val Phe Xaa
1 5 10 15

Xaa Thr Xaa Gly Ile
20

<210> 37
<211> 16
<212> PRT
<213> Artificial sequence

<220>
<223> Synthetic polypeptide

<220>
<221> MISC_FEATURE
<223> BKD E1beta motif

<220>
<221> VARIANT
<222> (1)..(1)
<223> Xaa is Tyr or Phe

<220>
<221> VARIANT
<222> (3)..(3)
<223> Xaa is Glu or Lys

<220>
<221> VARIANT
<222> (4)..(4)
<223> Xaa is any amino acid

<220>
<221> VARIANT
<222> (6)..(6)
<223> Xaa is Cys or Val

<220>
<221> VARIANT
<222> (7)..(7)
<223> Xaa is any amino acid

<220>
<221> VARIANT
<222> (9)..(9)
<223> Xaa is Ala or Thr

<220>
<221> VARIANT
<222> (11)..(11)
<223> Xaa is Leu or Ile

<220>
<221> VARIANT
<222> (12)..(12)
<223> Xaa is Ala or Ser

<220>
<221> VARIANT
<222> (14)..(14)
<223> Xaa is Ala or Ser

<220>
<221> VARIANT
<222> (15)..(15)
<223> Xaa is Ala or Gly

<400> 37

Xaa Gly Xaa Xaa Arg Xaa Xaa Asp Xaa Pro Xaa Xaa Glu Xaa Xaa Ile
1 5 10 15

<210> 38
<211> 15
<212> PRT
<213> Artificial sequence

<220>
<223> Synthetic polypeptide

<220>
<221> MISC_FEATURE
<223> BKD E1beta motif

<220>
<221> VARIANT
<222> (3)..(3)
<223> Xaa is Ala or Glu

<220>
<221> VARIANT
<222> (4)..(4)
<223> Xaa is any amino acid

<220>
<221> VARIANT
<222> (5)..(5)
<223> Xaa is Arg or Tyr

<220>
<221> VARIANT
<222> (7)..(7)
<223> Xaa is any amino acid

<220>
<221> VARIANT
<222> (10)..(10)
<223> Xaa is Ile or Val

<220>
<221> VARIANT
<222> (11)..(11)
<223> Xaa is Ala or Val

```
<220>
<221>  VARIANT
<222>  (13)..(13)
<223>  Xaa is any amino acid

<400>  38

Gly Thr Xaa Xaa Xaa Gly Xaa Arg Pro Xaa Xaa Glu Xaa Gln Phe
1               5                   10                  15


<210>  39
<211>  19
<212>  PRT
<213>  Artificial sequence

<220>
<223>  Synthetic polypeptide


<220>
<221>  MISC_FEATURE
<223>  BKD E1beta motif

<220>
<221>  VARIANT
<222>  (2)..(2)
<223>  Xaa is Cys or Tyr

<220>
<221>  VARIANT
<222>  (5)..(5)
<223>  Xaa is Val or Ile

<220>
<221>  VARIANT
<222>  (6)..(6)
<223>  Xaa is any amino acid

<220>
<221>  VARIANT
<222>  (7)..(7)
<223>  Xaa is Ala or Gly

<220>
<221>  VARIANT
<222>  (8)..(10)
<223>  Xaa is any amino acid

<220>
<221>  VARIANT
<222>  (13)..(13)
<223>  Xaa is any amino acid

<220>
<221>  VARIANT
<222>  (15)..(15)
<223>  Xaa is any amino acid

<220>
<221>  VARIANT
<222>  (18)..(18)
```

<223> Xaa is any amino acid

<220>
<221> VARIANT
<222> (19)..(19)
<223> Xaa is Phe or Tyr

<400> 39

Pro Xaa Gly Gly Xaa Xaa Xaa Xaa Xaa Xaa His Ser Xaa Ser Xaa Glu
1               5                   10                  15

Ala Xaa Xaa

<210> 40
<211> 13
<212> PRT
<213> Artificial sequence

<220>
<223> Synthetic polypeptide

<220>
<221> MISC_FEATURE
<223> BKD E1beta motif

<220>
<221> VARIANT
<222> (1)..(1)
<223> Xaa is Glu or Asp

<220>
<221> VARIANT
<222> (5)..(5)
<223> Xaa is any amino acid

<220>
<221> VARIANT
<222> (6)..(6)
<223> Xaa is Phe or Tyr

<220>
<221> VARIANT
<222> (7)..(7)
<223> Xaa is any amino acid

<220>
<221> VARIANT
<222> (9)..(9)
<223> Xaa is His or Pro

<220>
<221> VARIANT
<222> (12)..(12)
<223> Xaa is any amino acid

<400> 40

Xaa Asp Pro Val Xaa Xaa Xaa Glu Xaa Lys Arg Xaa Tyr

1 5 10

<210> 41
<211> 12
<212> PRT
<213> Artificial sequence

<220>
<223> Synthetic polypeptide

<220>
<221> MISC_FEATURE
<223> BKD E1beta motif

<220>
<221> VARIANT
<222> (1)..(1)
<223> Xaa is His or Glu

<220>
<221> VARIANT
<222> (3)..(3)
<223> Xaa is Ile or Val

<220>
<221> VARIANT
<222> (7)..(7)
<223> Xaa is Thr or Ser

<220>
<221> VARIANT
<222> (8)..(9)
<223> Xaa is any amino acid

<220>
<221> VARIANT
<222> (11)..(11)
<223> Xaa is any amino acid

<400> 41

Xaa Val Xaa Asp Leu Arg Xaa Xaa Xaa Pro Xaa Asp
1 5 10

<210> 42
<211> 19
<212> PRT
<213> Artificial sequence

<220>
<223> Synthetic polypeptide

<220>
<221> MISC_FEATURE
<223> BKD E1beta motif

<220>
<221> VARIANT
<222> (2)..(2)

<223> Xaa is any amino acid

<220>
<221> VARIANT
<222> (4)..(4)
<223> Xaa is Leu or Phe

<220>
<221> VARIANT
<222> (5)..(5)
<223> Xaa is any amino acid

<220>
<221> VARIANT
<222> (6)..(6)
<223> Xaa is Asp or His

<220>
<221> VARIANT
<222> (8)..(8)
<223> Xaa is Asp or Glu

<220>
<221> VARIANT
<222> (11)..(12)
<223> Xaa is any amino acid

<220>
<221> VARIANT
<222> (14)..(14)
<223> Xaa is Leu or Val

<220>
<221> VARIANT
<222> (15)..(15)
<223> Xaa is any amino acid

<220>
<221> VARIANT
<222> (17)..(17)
<223> Xaa is any amino acid

<220>
<221> VARIANT
<222> (18)..(18)
<223> Xaa is His or Asp

<400> 42

```
Glu Xaa Cys Xaa Xaa Xaa Leu Xaa Ala Pro Xaa Xaa Arg Xaa Xaa Gly
1               5                   10                  15

Xaa Xaa Pro
```

<210> 43
<211> 424
<212> PRT
<213> Bacillus subtilis

<220>
<221> MISC_FEATURE
<223> BKD E2 component

<400> 43

```
Met Ala Ile Glu Gln Met Thr Met Pro Gln Leu Gly Glu Ser Val Thr
1               5                   10                  15

Glu Gly Thr Ile Ser Lys Trp Leu Val Ala Pro Gly Asp Lys Val Asn
            20                  25                  30

Lys Tyr Asp Pro Ile Ala Glu Val Met Thr Asp Lys Val Asn Ala Glu
        35                  40                  45

Val Pro Ser Ser Phe Thr Gly Thr Ile Thr Glu Leu Val Gly Glu Glu
    50                  55                  60

Gly Gln Thr Leu Gln Val Gly Glu Met Ile Cys Lys Ile Glu Thr Glu
65                  70                  75                  80

Gly Ala Asn Pro Ala Glu Gln Lys Gln Glu Gln Pro Ala Ala Ser Glu
                85                  90                  95

Ala Ala Glu Asn Pro Val Ala Lys Ser Ala Gly Ala Ala Asp Gln Pro
            100                 105                 110

Asn Lys Lys Arg Tyr Ser Pro Ala Val Leu Arg Leu Ala Gly Glu His
        115                 120                 125

Gly Ile Asp Leu Asp Gln Val Thr Gly Thr Gly Ala Gly Gly Arg Ile
    130                 135                 140

Thr Arg Lys Asp Ile Gln Arg Leu Ile Glu Thr Gly Gly Val Gln Glu
145                 150                 155                 160

Gln Asn Pro Glu Glu Leu Lys Thr Ala Ala Pro Ala Pro Lys Ser Ala
                165                 170                 175

Ser Lys Pro Glu Pro Lys Glu Glu Thr Ser Tyr Pro Ala Ser Ala Ala
            180                 185                 190

Gly Asp Lys Glu Ile Pro Val Thr Gly Val Arg Lys Ala Ile Ala Ser
        195                 200                 205

Asn Met Lys Arg Ser Lys Thr Glu Ile Pro His Ala Trp Thr Met Met
    210                 215                 220

Glu Val Asp Val Thr Asn Met Val Ala Tyr Arg Asn Ser Ile Lys Asp
```

225 230 235 240

Ser Phe Lys Lys Thr Glu Gly Phe Asn Leu Thr Phe Phe Ala Phe Phe
245 250 255

Val Lys Ala Val Ala Gln Ala Leu Lys Glu Phe Pro Gln Met Asn Ser
260 265 270

Met Trp Ala Gly Asp Lys Ile Ile Gln Lys Lys Asp Ile Asn Ile Ser
275 280 285

Ile Ala Val Ala Thr Glu Asp Ser Leu Phe Val Pro Val Ile Lys Asn
290 295 300

Ala Asp Glu Lys Thr Ile Lys Gly Ile Ala Lys Asp Ile Thr Gly Leu
305 310 315 320

Ala Lys Lys Val Arg Asp Gly Lys Leu Thr Ala Asp Asp Met Gln Gly
325 330 335

Gly Thr Phe Thr Val Asn Asn Thr Gly Ser Phe Gly Ser Val Gln Ser
340 345 350

Met Gly Ile Ile Asn Tyr Pro Gln Ala Ala Ile Leu Gln Val Glu Ser
355 360 365

Ile Val Lys Arg Pro Val Val Met Asp Asn Gly Met Ile Ala Val Arg
370 375 380

Asp Met Val Asn Leu Cys Leu Ser Leu Asp His Arg Val Leu Asp Gly
385 390 395 400

Leu Val Cys Gly Arg Phe Leu Gly Arg Val Lys Gln Ile Leu Glu Ser
405 410 415

Ile Asp Glu Lys Thr Ser Val Tyr
420

<210> 44
<211> 1275
<212> DNA
<213> Bacillus subtilis

<220>
<221> misc_feature
<223> Encodes BKD E2 component

<400> 44
ttagtaaaca gatgtcttct cgtcaatcga ttctaaaatt tgtttcactc gtccgaggaa 60

```
tcgtccgcac acgagaccgt caagcactct gtgatctaat gacaggcaca gattaaccat      120
gtctctgaca gcaatcatgc cattgtccat gacaaccggg cgtttgacga tggattctac      180
ttgaagaatc gcagcctgag ggtagttgat aatgcccatc gactgaacag acccgaacga      240
acctgtgttg ttgacggtaa acgtgcctcc ctgcatgtca tctgcagtga gttttccgtc      300
tcttactttt ttagctaggc cggtaatgtc tttcgcaatg cctttaattg tttttttcatc     360
agcgttttta atcaccggaa caaataaaga atcctctgtg gcaactgcaa ttgaaatatt      420
gatatccttt ttctgaataa ttttgtcccc cgcccacatg ctattcattt gcgggaattc      480
ttttaacgcc tgagcgaccg cttttacaaa aaaggcgaag aacgttaaat taaagccttc      540
tgtcttctta aaagaatctt ttatactgtt gcgatatgca accatatttg tgacgtcgac      600
ttccatcatc gtccaagcat gcggaatttc tgttttgctt cgcttcatat tggaagcaat      660
tgcttttctt acacctgtga cagggatttc tttatcaccg gctgcagacg caggatatga      720
cgtctcttct tttggctcag gttttgatgc agacttcggt gcaggagctg ctgttttcag      780
ctcctcagga ttctgttctt gcacgccgcc tgtttcaatt aagcgctgaa tatcttttcg      840
tgtgatgcgc ccgccggcac cagttcctgt cacttgatcg aggtcaatgc cgtgctctcc      900
ggccaaacgg agaacagctg gcgagtagcg ctttttattg ggctgatcgg ctgctccagc      960
actttttgca acagggttct cagcggcttc tgatgctgct ggctgttctt gtttttgttc     1020
agccggattc gcgccttctg tttcaatttt gcaaatcatt tctccgactt gcagggtttg     1080
gccttcttct cccacaagct ctgttatcgt accagtaaaa gaagacggaa cctctgcatt     1140
taccttatct gtcatgactt ccgcgatcgg atcgtatttg ttcactttat caccgggggc     1200
gacaagccat ttgctgatcg tcccctctgt tacgctttct ccaagctgcg gcatcgtcat     1260
ttgttcaatt gccat                                                      1275


<210>  45
<211>  455
<212>  PRT
<213>  Streptomyces avermitilis


<220>
<221>  MISC_FEATURE
<223>  BKD E2 component

<400>  45

Met Ala Gln Val Leu Glu Phe Lys Leu Pro Asp Leu Gly Glu Gly Leu
1               5                   10                  15


Thr Glu Ala Glu Ile Val Arg Trp Leu Val Gln Val Gly Asp Val Val
            20                  25                  30
```

```
Ala Ile Asp Gln Pro Val Val Glu Val Glu Thr Ala Lys Ala Met Val
        35                  40                  45

Glu Val Pro Cys Pro Tyr Gly Gly Val Val Thr Ala Arg Phe Gly Glu
    50                  55                  60

Glu Gly Thr Glu Leu Pro Val Gly Ser Pro Leu Leu Thr Val Ala Val
65                  70                  75                  80

Gly Ala Pro Ser Ser Val Pro Ala Ala Ser Ser Leu Ser Gly Ala Thr
                85                  90                  95

Ser Ala Ser Ser Ala Ser Ser Val Ser Ser Asp Asp Gly Glu Ser Ser
            100                 105                 110

Gly Asn Val Leu Val Gly Tyr Gly Thr Ser Ala Ala Pro Ala Arg Arg
        115                 120                 125

Arg Arg Val Arg Pro Gly Gln Ala Ala Pro Val Val Thr Ala Thr Ala
    130                 135                 140

Ala Ala Ala Ala Thr Arg Val Ala Ala Pro Glu Arg Ser Asp Gly Pro
145                 150                 155                 160

Val Pro Val Ile Ser Pro Leu Val Arg Arg Leu Ala Arg Glu Asn Gly
                165                 170                 175

Leu Asp Leu Arg Ala Leu Ala Gly Ser Gly Pro Asp Gly Leu Ile Leu
            180                 185                 190

Arg Ser Asp Val Glu Gln Ala Leu Arg Ala Ala Pro Thr Pro Ala Pro
        195                 200                 205

Thr Pro Thr Met Pro Pro Ala Pro Thr Pro Ala Pro Thr Pro Ala Ala
    210                 215                 220

Ala Pro Arg Gly Thr Arg Ile Pro Leu Arg Gly Val Arg Gly Ala Val
225                 230                 235                 240

Ala Asp Lys Leu Ser Arg Ser Arg Arg Glu Ile Pro Asp Ala Thr Cys
                245                 250                 255

Trp Val Asp Ala Asp Ala Thr Ala Leu Met His Ala Arg Val Ala Met
            260                 265                 270

Asn Ala Thr Gly Gly Pro Lys Ile Ser Leu Ile Ala Leu Leu Ala Arg
        275                 280                 285
```

Ile Cys Thr Ala Ala Leu Ala Arg Phe Pro Glu Leu Asn Ser Thr Val
    290                 295                 300

Asp Met Asp Ala Arg Glu Val Val Arg Leu Asp Gln Val His Leu Gly
305                 310                 315                 320

Phe Ala Ala Gln Thr Glu Arg Gly Leu Val Val Pro Val Val Arg Asp
                325                 330                 335

Ala His Ala Arg Asp Ala Glu Ser Leu Ser Ala Glu Phe Ala Arg Leu
            340                 345                 350

Thr Glu Ala Ala Arg Thr Gly Thr Leu Thr Pro Gly Glu Leu Thr Gly
        355                 360                 365

Gly Thr Phe Thr Leu Asn Asn Tyr Gly Val Phe Gly Val Asp Gly Ser
    370                 375                 380

Thr Pro Ile Ile Asn His Pro Glu Ala Ala Met Leu Gly Val Gly Arg
385                 390                 395                 400

Ile Ile Pro Lys Pro Trp Val His Glu Gly Glu Leu Ala Val Arg Gln
                405                 410                 415

Val Val Gln Leu Ser Leu Thr Phe Asp His Arg Val Cys Asp Gly Gly
            420                 425                 430

Thr Ala Gly Gly Phe Leu Arg Tyr Val Ala Asp Cys Val Glu Gln Pro
        435                 440                 445

Ala Val Leu Leu Arg Thr Leu
    450                 455

<210> 46
<211> 1368
<212> DNA
<213> Streptomyces avermitilis

<220>
<221> misc_feature
<223> Encodes BKD E2 component

<400> 46
atggcccagg tgctcgagtt caagctcccc gacctcgggg agggcctgac cgaggccgag 60

atcgtccgct ggctggtgca ggtcggcgac gtcgtggcga tcgaccagcc ggtcgtcgag 120

gtggagacgg ccaaggcgat ggtcgaggtg ccgtgcccct acgggggcgt ggtcaccgcc 180

```
cgcttcggcg aggagggcac ggaactgccc gtgggctcac cgctgttgac ggtggctgtc      240

ggagctccgt cctcggtgcc cgcggcgtcc tcgctgtccg gggcgacatc ggcgtcctcc      300

gcgtcctcgg tgtcatcgga cgacggcgag tcgtccggca acgtcctggt cggatacggc      360

acgtcggccg cgcccgcgcg ccggcggagg gtgcggccgg gccaggcggc acccgtggtg      420

acggcaactg ccgccgcggc cgccacgcgc gtggcggctc ccgagcggag cgacggcccc      480

gtgcccgtga tctccccgct ggtccgcagg ctcgcccggg agaacggcct ggatctgcgg      540

gcgctggcgg gctccgggcc cgacgggctg atcctgaggt cggacgtcga gcaggcgctg      600

cgcgccgcgc ccactcctgc ccccaccccg accatgcctc cggctcccac tcctgccccc      660

acccccgccg cggcaccccg cggcacccgc atccccctcc gaggggtccg cggtgccgtc      720

gccgacaaac tctcccgcag ccggcgtgag atccccgacg cgacctgctg ggtggacgcc      780

gacgccacgg cactcatgca cgcgcgcgtg gcgatgaacg cgaccggcgg cccgaagatc      840

tccctcatcg cgctgctcgc caggatctgc accgccgcac tggcccgctt ccccgagctc      900

aactccaccg tcgacatgga cgcccgcgag gtcgtacggc tcgaccaggt gcacctgggc      960

ttcgccgcgc agaccgaacg gggctcgtc gtcccggtcg tgcgggacgc gcacgcgcgg      1020

gacgccgagt cgctcagcgc cgagttcgcg cggctgaccg aggccgcccg gaccggcacc      1080

ctcacacccg gggaactgac cggcggcacc ttcacgttga acaactacgg ggtgttcggc      1140

gtcgacggtt ccacgccgat catcaaccac cccgaggcgg ccatgctggg cgtcggccgc      1200

atcatcccca agccgtgggt gcacgagggc gagctggcgg tgcggcaggt cgtccagctc      1260

tcgctcacct tcgaccaccg ggtgtgcgac ggcggcacgg caggcggttt cctgcgctac      1320

gtggcggact gcgtggaaca gccggcggtg ctgctgcgca ccctgtag                   1368
```

<210>  47
<211>  423
<212>  PRT
<213>  Pseudomonas putida

<220>
<221>  MISC_FEATURE
<223>  BKD E2 component

<400>  47

```
Met Gly Thr His Val Ile Lys Met Pro Asp Ile Gly Glu Gly Ile Ala
1               5                   10                  15

Gln Val Glu Leu Val Glu Trp Phe Val Lys Val Gly Asp Ile Ile Ala
            20                  25                  30

Glu Asp Gln Val Val Ala Asp Val Met Thr Asp Lys Ala Thr Val Glu
        35                  40                  45
```

```
Ile Pro Ser Pro Val Ser Gly Lys Val Leu Ala Leu Gly Gly Gln Pro
    50                  55                  60

Gly Glu Val Met Ala Val Gly Ser Glu Leu Ile Arg Ile Glu Val Glu
65                  70                  75                  80

Gly Ser Gly Asn His Val Asp Val Pro Gln Pro Lys Pro Val Glu Ala
                85                  90                  95

Pro Ala Ala Pro Ile Ala Ala Lys Pro Glu Pro Gln Lys Asp Val Lys
            100                 105                 110

Pro Ala Val Tyr Gln Ala Pro Ala Asn His Glu Ala Ala Pro Ile Val
        115                 120                 125

Pro Arg Gln Pro Gly Asp Lys Pro Leu Ala Ser Pro Ala Val Arg Lys
    130                 135                 140

Arg Ala Leu Asp Ala Gly Ile Glu Leu Arg Tyr Val His Gly Ser Gly
145                 150                 155                 160

Pro Ala Gly Arg Ile Leu His Glu Asp Leu Asp Ala Phe Met Ser Lys
                165                 170                 175

Pro Gln Ser Asn Ala Gly Gln Ala Pro Asp Gly Tyr Ala Lys Arg Thr
            180                 185                 190

Asp Ser Glu Gln Val Pro Val Ile Gly Leu Arg Arg Lys Ile Ala Gln
        195                 200                 205

Arg Met Gln Asp Ala Lys Arg Arg Val Ala His Phe Ser Tyr Val Glu
    210                 215                 220

Glu Ile Asp Val Thr Ala Leu Glu Ala Leu Arg Gln Gln Leu Asn Ser
225                 230                 235                 240

Lys His Gly Asp Ser Arg Gly Lys Leu Thr Leu Leu Pro Phe Leu Val
                245                 250                 255

Arg Ala Leu Val Val Ala Leu Arg Asp Phe Pro Gln Ile Asn Ala Thr
            260                 265                 270

Tyr Asp Asp Glu Ala Gln Ile Ile Thr Arg His Gly Ala Val His Val
        275                 280                 285

Gly Ile Ala Thr Gln Gly Asp Asn Gly Leu Met Val Pro Val Leu Arg
```

290 295 300

His Ala Glu Ala Gly Ser Leu Trp Ala Asn Ala Gly Glu Ile Ser Arg
305 310 315 320

Leu Ala Asn Ala Ala Arg Asn Asn Lys Ala Ser Arg Glu Glu Leu Ser
325 330 335

Gly Ser Thr Ile Thr Leu Thr Ser Leu Gly Ala Leu Gly Gly Ile Val
340 345 350

Ser Thr Pro Val Val Asn Thr Pro Glu Val Ala Ile Val Gly Val Asn
355 360 365

Arg Met Val Glu Arg Pro Val Val Ile Asp Gly Gln Ile Val Val Arg
370 375 380

Lys Met Met Asn Leu Ser Ser Ser Phe Asp His Arg Val Val Asp Gly
385 390 395 400

Met Asp Ala Ala Leu Phe Ile Gln Ala Val Arg Gly Leu Leu Glu Gln
405 410 415

Pro Ala Cys Leu Phe Val Glu
420

<210> 48
<211> 1272
<212> DNA
<213> Pseudomonas putida

<220>
<221> misc_feature
<223> Encodes BKD E2 component

<400> 48
tcactccacg aacaggcagg cgggttgttc gagcaggcca cgcacggcct ggatgaacag 60

ggcggcgtcc atgccatcga ccacgcggtg gtcgaacgag ctggacaggt tcatcatctt 120

gcgcacgacg atctggccat caatcaccac cggtcgttcg accatgcggt tgaccccgac 180

gattgccact tccggggtgt tgaccaccgg cgtgctgaca atgccaccca aggcgccgag 240

gctggtcagg gtgatggtcg agccggacag ctcctcgcgg ctggccttgt tgttacgtgc 300

agcgttggcc aggcgcgaaa tctcgccggc attggcccac aggctgcccg cttcggcgtg 360

gcgcagcacg ggtaccatca ggccgttgtc accctgggtg gcaatgccca catgcaccgc 420

gccatggcgg gtgatgatct gcgcttcgtc gtcgtaggtc gcgttgatct gcgggaagtc 480

acgcagcgcc acgacgaggg cgcgcaccag gaatggcagc aaggtcagtt tgccgcggct 540

gtcgccgtgc ttgctgttga gttgctggcg cagggcttcc agggcggtga cgtcgatttc 600

ctcgacataa ctgaagtgcg cgacccggcg tttggcgtcc tgcatgcgct gggcgatctt 660

gcggcgcagg ccgatcaccg gcacctgctc gctgtcggtg cgcttggcat aaccatcagg 720

tgcttgcccg gcattgcttt gcggcttgct catgaaggcg tcgaggtctt cgtgcagaat 780

gcgcccggcc gggccgctac catgcacata acgcagttcg ataccggcgt ccagggcgcg 840

tttgcgcacg gccggcgagg ccagcggctt gtcgcccggc tggcgcggca cgatgggcgc 900

agcttcgtgg ttggcgggcg cctggtacac ggcgggtttt acgtctttct gcggttccgg 960

cttggctgca atcggggcgg ccggggcctc taccggtttt ggctgaggca cgtccacatg 1020

gttgccgctg ccttccactt cgatgcggat cagttcgcta ccgaccgcca tcacttcccc 1080

gggctggcca cccagggcca acaccttgcc gctgaccggc gaggggattt ccacggtggc 1140

cttgtcggtc atgacgtcgg ccaccacctg gtcctcggcg atgatgtcgc cgaccttgac 1200

gaaccattcc accaactcga cctgcgcgat gccttcgcca atgtccggca tcttgatgac 1260

gtgcgtgccc at 1272

<210> 49
<211> 416
<212> PRT
<213> Listeria monocytogenes

<220>
<221> MISC_FEATURE
<223> BKD E2 component

<400> 49

Met Ala Val Glu Lys Ile Thr Met Pro Lys Leu Gly Glu Ser Val Thr
1               5                   10                  15

Glu Gly Thr Ile Ser Ser Trp Leu Val Lys Pro Gly Asp Thr Val Glu
            20                  25                  30

Lys Tyr Asp Ala Ile Ala Glu Val Leu Thr Asp Lys Val Thr Ala Glu
        35                  40                  45

Ile Pro Ser Ser Phe Ser Gly Thr Ile Lys Glu Ile Leu Ala Glu Glu
    50                  55                  60

Asp Glu Thr Leu Glu Val Gly Glu Val Ile Cys Thr Ile Glu Thr Glu
65                  70                  75                  80

Glu Ala Ser Ser Ser Glu Pro Val Val Glu Ala Glu Gln Thr Glu Pro
                85                  90                  95

```
Lys Thr Pro Glu Lys Gln Glu Thr Lys Gln Val Lys Leu Ala Glu Ala
            100                 105                 110

Pro Ala Ser Gly Arg Phe Ser Pro Ala Val Leu Arg Ile Ala Gly Glu
        115                 120                 125

Asn Asn Ile Asp Leu Ser Thr Val Glu Gly Thr Gly Lys Gly Gly Arg
    130                 135                 140

Ile Thr Arg Lys Asp Leu Leu Gln Val Ile Glu Asn Gly Pro Val Ala
145                 150                 155                 160

Pro Lys Arg Glu Glu Val Lys Ser Ala Pro Gln Glu Lys Glu Ala Thr
                165                 170                 175

Pro Asn Pro Val Arg Ser Ala Ala Gly Asp Arg Glu Ile Pro Ile Asn
            180                 185                 190

Gly Val Arg Lys Ala Ile Ala Lys His Met Ser Val Ser Lys Gln Glu
        195                 200                 205

Ile Pro His Ala Trp Met Met Val Glu Val Asp Ala Thr Gly Leu Val
    210                 215                 220

Arg Tyr Arg Asn Thr Val Lys Asp Ser Phe Lys Lys Glu Glu Gly Tyr
225                 230                 235                 240

Ser Leu Thr Tyr Phe Ala Phe Phe Ile Lys Ala Val Ala Gln Ala Leu
                245                 250                 255

Lys Glu Phe Pro Gln Leu Asn Ser Thr Trp Ala Gly Asp Lys Ile Ile
            260                 265                 270

Glu His Ala Asn Ile Asn Ile Ser Ile Ala Ile Ala Ala Gly Asp Leu
        275                 280                 285

Leu Tyr Val Pro Val Ile Lys Asn Ala Asp Glu Lys Ser Ile Lys Gly
    290                 295                 300

Ile Ala Arg Glu Ile Ser Glu Leu Ala Gly Lys Ala Arg Asn Gly Lys
305                 310                 315                 320

Leu Ser Gln Ala Asp Met Glu Gly Gly Thr Phe Thr Val Asn Ser Thr
                325                 330                 335

Gly Ser Phe Gly Ser Val Gln Ser Met Gly Ile Ile Asn His Pro Gln
            340                 345                 350
```

```
Ala Ala Ile Leu Gln Val Glu Ser Ile Val Lys Arg Pro Val Ile Ile
        355                 360                 365

Asp Asp Met Ile Ala Val Arg Asp Met Val Asn Leu Cys Leu Ser Ile
    370                 375                 380

Asp His Arg Ile Leu Asp Gly Leu Leu Ala Gly Lys Phe Leu Gln Ala
385                 390                 395                 400

Ile Lys Ala Asn Val Glu Lys Ile Ser Lys Glu Asn Thr Ala Leu Tyr
                405                 410                 415
```

<210> 50
<211> 1251
<212> DNA
<213> Listeria monocytogenes

<220>
<221> misc_feature
<223> Encodes BKD E2 component

<400> 50

```
gtggcagttg aaaaaatcac catgcccaaa ttaggggaaa gtgtaacaga aggaacgatt      60
agttcatggt tagttaaacc aggcgataca gtagaaaaat atgatgctat cgcggaagtt     120
ttaacagata aagtaacagc tgaaatccca tcatccttta gtggcactat caaagaaatt     180
ttagcagagg aagatgaaac actagaagta ggcgaagtta tttgtaccat cgaaacagaa     240
gaggctagta gttcagagcc tgtagttgaa gcagaacaaa cagaaccaaa aactccagaa     300
aaacaagaaa caaaacaagt gaaattagca gaagcaccag ccagtggaag attttcacca     360
gcggtactgc gtattgctgg agaaaacaat attgatttat caaccgtaga aggcacaggt     420
aaaggtggcc gaattacaag aaaagattta cttcaagtaa ttgaaaatgg tccagtagct     480
ccgaaacgcg aggaagtgaa gtctgctcca caagaaaaag aagcgacgcc aaatcctgta     540
cgttcagcag caggtgacag agaaatccca atcaatggtg taagaaaagc gattgctaaa     600
catatgagcg tgagtaaaca agaaattccg catgcttgga tgatggtgga agtagatgca     660
actggtcttg ttcgctatcg taatacagtt aaagacagct ttaaaaaaga agaaggttat     720
tcattaactt atttcgcctt tttcatcaaa gccgttgcac aagcattgaa agaattcccg     780
caacttaaca gcacgtgggc aggcgataaa attattgagc atgcgaatat caatatttcg     840
attgcgattg cagctggcga tttattgtat gtgccagtta ttaaaaatgc ggacgaaaaa     900
tccattaaag gtattgctcg cgaaataagt gaactagctg gaaaagcgcg taatggtaaa     960
ctgagccaag ccgatatgga aggtgggact ttcactgtaa atagtactgg ttcatttggc    1020
```

tctgttcaat caatggggat tattaaccac ccacaagccg ctattcttca agtggaatcc 1080

attgttaagc gcccagtcat tattgacgat atgattgctg tacgagatat ggtcaaccta 1140

tgtctatcca tcgatcatcg tattttagac ggcttactag caggtaaatt cttacaagca 1200

attaaagcca atgtcgaaaa gatttctaaa gaaaatacag cgttgtatta a 1251

<210> 51
<211> 355
<212> PRT
<213> Micrococcus luteus

<220>
<221> MISC_FEATURE
<223> BKD E2 component

<400> 51

Met Ser Glu Arg Met Thr Phe Gly Arg Ala Ile Asn Arg Gly Leu His
1 5 10 15

Arg Ala Leu Ala Asp Asp Pro Lys Val Leu Leu Met Gly Glu Asp Ile
20 25 30

Gly Ala Leu Gly Gly Val Phe Arg Ile Thr Asp Gly Leu Gln Ala Glu
35 40 45

Phe Gly Glu Asp Arg Val Leu Asp Thr Pro Leu Ala Glu Ser Gly Ile
50 55 60

Val Gly Thr Ala Ile Gly Leu Ala Met Arg Gly Tyr Arg Pro Val Val
65 70 75 80

Glu Ile Gln Phe Asp Gly Phe Val Tyr Pro Ala Phe Asp Gln Ile Val
85 90 95

Ala Asn Leu Ala Lys Leu Arg Ala Arg Thr Arg Gly Ala Val Pro Met
100 105 110

Pro Val Thr Ile Arg Ile Pro Phe Gly Gly Gly Ile Gly Ser Pro Glu
115 120 125

His His Ser Glu Ser Pro Glu Ala Tyr Phe Leu His Thr Ala Gly Leu
130 135 140

Arg Val Val Ser Pro Ser Ser Pro Gln Glu Gly Tyr Asp Leu Ile Arg
145 150 155 160

Ala Ala Ile Ala Ser Glu Asp Pro Val Val Tyr Leu Glu Pro Lys Arg
165 170 175

Arg Tyr His Asp Lys Gly Asp Val Asp Leu Gly Val Ala Ile Pro Pro
180 185 190

Met Ser Pro Ala Arg Ile Leu Arg Glu Gly Arg Asp Ala Thr Leu Val
195 200 205

Ala Tyr Gly Pro Leu Val Lys Thr Ala Leu Gln Ala Ala Glu Val Ala
210 215 220

Ala Glu Glu Gly Val Glu Val Glu Val Val Asp Leu Arg Ser Leu Ser
225 230 235 240

Pro Leu Asp Thr Gly Leu Val Glu Ser Ser Val Arg Arg Thr Gly Arg
245 250 255

Leu Val Val Ala His Glu Ala Ser Arg Thr Gly Gly Leu Gly Ala Glu
260 265 270

Leu Val Ala Thr Val Ala Glu Arg Ala Phe His Trp Leu Glu Ala Pro
275 280 285

Pro Val Arg Val Thr Gly Met Asp Val Pro Tyr Pro Pro Ser Lys Leu
290 295 300

Glu His Leu His Leu Pro Asp Leu Asp Arg Ile Leu Asp Gly Leu Asp
305 310 315 320

Arg Ala Leu Gly Arg Pro Asn Ser Leu Asp Ser Val Asp Ala Phe Ala
325 330 335

Ala Pro Glu Thr Ala Glu Gln Phe Leu Ala Ala Gln Asn Ala Gly Glu
340 345 350

Glu Thr Arg
355

<210> 52
<211> 1068
<212> DNA
<213> Micrococcus luteus

<220>
<221> misc_feature
<223> Encodes BKD E2 component

<400> 52
gtgagcgagc gcatgacctt cggccgtgcg atcaaccgcg gcctgcaccg tgccctggcc 60

gacgacccca aggtcctgct catgggcgag gacatcggcg ccctcggcgg cgtgttccgc 120

atcaccgacg gcctgcaggc cgagttcggc gaggaccggg tgctcgacac cccgctggcc 180

gagtccggca tcgtgggcac ggccatcggc ctggcgatgc gcggctaccg gcccgtcgtc 240

gagatccagt tcgacggctt cgtgtacccg gcgttcgacc agatcgtggc gaacctggcc 300

aagctgcgcg cccgcacccg cggcgccgtg ccgatgccgg tgaccatccg catccccttc 360

ggcggcggca tcggctcccc ggagcaccac tccgagtcgc ccgaggccta cttcctgcac 420

accgcgggtc tgcgcgtggt ctccccgtcc tccccgcagg aggggtacga cctcatccgc 480

gccgcgatcg cctcggagga cccggtggtc tacctcgagc ccaagcgtcg ctaccacgac 540

aagggcgacg tggacctggg cgtcgcgatc ccgccgatga gcccggcccg catcctgcgc 600

gagggccgtg acgccacgct cgtggcctac ggcccgctcg tgaagaccgc cctgcaggcc 660

gccgaggtgg cggccgagga gggtgtcgag gtcgaggtgg tcgacctgcg cagcctgtcc 720

ccgctggaca ccggcctcgt cgagtcctcg gtgcggcgca ccggtcggct cgtcgtggcg 780

cacgaggcct cccgcacggg cggcctcggc gccgagctcg tggccacggt ggccgagcgc 840

gcgttccatt ggctcgaggc cccgccggtg cgcgtcaccg gcatggacgt gccctacccg 900

ccgtccaagc tcgagcacct gcacctgccg gacctcgacc gcatcctcga cggcctggac 960

cgtgctctgg gccggccgaa ttcgctggac tccgtggacg cgttcgccgc ccccgagacc 1020

gccgagcagt tcctcgccgc ccagaacgcc ggggaggaga cccgatga 1068

<210> 53
<211> 424
<212> PRT
<213> Staphylococcus aureus

<220>
<221> MISC_FEATURE
<223> BKD E2 component

<400> 53

Met Glu Ile Thr Met Pro Lys Leu Gly Glu Ser Val His Glu Gly Thr
1               5                   10                  15

Ile Glu Gln Trp Leu Val Ser Val Gly Asp His Ile Asp Glu Tyr Glu
            20                  25                  30

Pro Leu Cys Glu Val Ile Thr Asp Lys Val Thr Ala Glu Val Pro Ser
        35                  40                  45

Thr Ile Ser Gly Thr Ile Thr Glu Ile Leu Val Glu Ala Gly Gln Thr
    50                  55                  60

```
Val Ala Ile Asp Thr Ile Ile Cys Lys Ile Glu Thr Ala Asp Glu Lys
65                  70                  75                  80

Thr Asn Glu Thr Thr Glu Glu Ile Gln Ala Lys Val Asp Glu His Thr
                85                  90                  95

Gln Lys Ser Thr Lys Lys Ala Ser Ala Thr Val Glu Gln Thr Phe Thr
            100                 105                 110

Ala Lys Gln Asn Gln Pro Arg Asn Asn Gly Arg Phe Ser Pro Val Val
        115                 120                 125

Phe Lys Leu Ala Ser Glu His Asp Ile Asp Leu Ser Gln Val Val Gly
    130                 135                 140

Ser Gly Phe Glu Gly Arg Val Thr Lys Lys Asp Ile Met Ser Val Ile
145                 150                 155                 160

Glu Asn Gly Gly Thr Thr Ala Gln Ser Asp Lys Gln Val Gln Thr Lys
                165                 170                 175

Ser Thr Ser Val Asp Thr Ser Ser Asn Gln Ser Ser Glu Asp Asn Ser
            180                 185                 190

Glu Asn Ser Thr Ile Pro Val Asn Gly Val Arg Lys Ala Ile Ala Gln
        195                 200                 205

Asn Met Val Asn Ser Val Thr Glu Ile Pro His Ala Trp Met Met Ile
    210                 215                 220

Glu Val Asp Ala Thr Asn Leu Val Asn Thr Arg Asn His Tyr Lys Asn
225                 230                 235                 240

Ser Phe Lys Asn Lys Glu Gly Tyr Asn Leu Thr Phe Phe Ala Phe Phe
                245                 250                 255

Val Lys Ala Val Ala Asp Ala Leu Lys Ala Tyr Pro Leu Leu Asn Ser
            260                 265                 270

Ser Trp Gln Gly Asn Glu Ile Val Leu His Lys Asp Ile Asn Ile Ser
        275                 280                 285

Ile Ala Val Ala Asp Glu Asn Lys Leu Tyr Val Pro Val Ile Lys His
    290                 295                 300

Ala Asp Glu Lys Ser Ile Lys Gly Ile Ala Arg Glu Ile Asn Thr Leu
305                 310                 315                 320
```

```
Ala Thr Lys Ala Arg Asn Lys Gln Leu Thr Thr Glu Asp Met Gln Gly
                325                 330                 335

Gly Thr Phe Thr Val Asn Asn Thr Gly Thr Phe Gly Ser Val Ser Ser
            340                 345                 350

Met Gly Ile Ile Asn His Pro Gln Ala Ala Ile Leu Gln Val Glu Ser
        355                 360                 365

Ile Val Lys Lys Pro Val Val Ile Asn Asp Met Ile Ala Ile Arg Ser
    370                 375                 380

Met Val Asn Leu Cys Ile Ser Ile Asp His Arg Ile Leu Asp Gly Leu
385                 390                 395                 400

Gln Thr Gly Lys Phe Met Asn His Ile Lys Gln Arg Ile Glu Gln Tyr
                405                 410                 415

Thr Leu Glu Asn Thr Asn Ile Tyr
            420


<210>  54
<211>  1275
<212>  DNA
<213>  Staphylococcus aureus


<220>
<221>  misc_feature
<223>  Encodes BKD E2 component

<400>  54
ctaatatata tttgtatttt ctaaagtata ctgttcgata cgctgtttaa tatgattcat        60

aaatttacca gtttgtaaac catctaaaat gcgatgatct attgaaatac ataaatttac       120

catacttcga attgcaatca tatcattaat tactactggc tttttaacga ttgattctac       180

ttgtaatatc gctgcttgag ggtgatttat aatccccatt gatgatactg aaccaaatgt       240

accagtatta ttaactgtaa atgttccacc ttgcatatct tcagttgtca attgcttatt       300

acgcgctttt gttgctaaag tattaatttc tctagctata cctttgattg acttttcgtc       360

tgcatgctta atcacaggta cgtataattt attttcatca gcaacagcaa ttgaaatatt       420

aatgtcttta tgtaagacaa tttcatttcc ttgccagcta ctatttaata aaggatatgc       480

ttttaaagca tctgctacag cttttacaaa gaaagcaaag aacgttagat tatatccttc       540

tttattttta aagctgtttt tataatgatt tctcgtattc acaagatttg tagcatctac       600

ttcaatcatc atccatgcat gtggaatctc tgttacacta ttaaccatat tttgcgcaat       660

tgctttacgc acaccattta ctggtattgt gctgttttca ctattgtctt cagatgattg       720
```

gttacttgat gtatctactg atgttgattt tgtttgaact tgtttgtcag attgagctgt 780

ggtaccacca ttttcaataa ctgacattat atccttctta gttacacgac cttcaaatcc 840

actacctaca acttgtgata aatcaatgtc atgctctgaa gcgagtttaa atacaacagg 900

tgaaaagcga ccattattac gaggttgatt ttgtttagca gtaaatgtct gttccactgt 960

tgcactagct tttttagtag atttctgagt atgctcatcc acttttgctt gtatctcttc 1020

agttgtttca tttgtctttt catcagcagt ttcaatttta cagataattg tatcaatagc 1080

tactgtctgc cccgcttcaa ctaaaatttc tgtaattgtt cctgatatcg tggaagggac 1140

ttcagctgtc actttatctg taataacttc acataatggt tcatattcat caatatgatc 1200

accaacagaa actaaccatt gttcaatggt accttcatga acactctcac ctaacttagg 1260

cattgttatt tccat 1275

<210> 55
<211> 455
<212> PRT
<213> Streptococcus mutans

<220>
<221> MISC_FEATURE
<223> BKD E2 component

<400> 55

Met Ala Val Glu Ile Ile Met Pro Lys Leu Gly Val Asp Met Gln Glu
1 5 10 15

Gly Glu Ile Ile Glu Trp Lys Lys Gln Glu Gly Asp Glu Val Lys Glu
20 25 30

Gly Glu Ile Leu Leu Glu Ile Met Ser Asp Lys Thr Asn Met Glu Ile
35 40 45

Glu Ala Glu Asp Ser Gly Val Leu Leu Lys Ile Val Lys Gly Asn Gly
50 55 60

Gln Val Val Pro Val Thr Glu Val Ile Gly Tyr Ile Gly Gln Ala Gly
65 70 75 80

Glu Val Leu Glu Ile Ala Asp Val Pro Ala Ser Thr Val Pro Lys Glu
85 90 95

Asn Ser Ala Ala Pro Ala Glu Lys Thr Lys Ala Met Ser Ser Pro Thr
100 105 110

Val Ala Ala Ala Pro Gln Gly Lys Ile Arg Ala Thr Pro Ala Ala Arg

115 120 125

Lys Ala Ala Arg Asp Leu Gly Val Asn Leu Asn Gln Val Ser Gly Thr
130 135 140

Gly Ala Lys Gly Arg Val His Lys Glu Asp Val Glu Ser Phe Lys Ala
145 150 155 160

Ala Gln Pro Lys Ala Thr Pro Leu Ala Arg Lys Ile Ala Ile Asp Lys
165 170 175

Gly Ile Asp Leu Ala Ser Val Ser Gly Thr Gly Phe Gly Gly Lys Ile
180 185 190

Ile Lys Glu Asp Ile Leu Asn Leu Phe Glu Ala Ala Gln Pro Val Asn
195 200 205

Asp Val Ser Asp Pro Ala Lys Glu Ala Ala Ala Leu Pro Glu Gly Val
210 215 220

Glu Val Ile Lys Met Ser Ala Met Arg Lys Ala Val Ala Lys Ser Met
225 230 235 240

Val Asn Ser Tyr Leu Thr Ala Pro Thr Phe Thr Leu Asn Tyr Asp Ile
245 250 255

Asp Met Thr Glu Met Ile Ala Leu Arg Lys Lys Leu Ile Asp Pro Ile
260 265 270

Met Glu Lys Thr Gly Phe Lys Val Ser Phe Thr Asp Leu Ile Gly Leu
275 280 285

Ala Val Val Lys Thr Leu Met Lys Pro Glu His Arg Tyr Leu Asn Ala
290 295 300

Ser Leu Ile Asn Asp Ala Thr Glu Ile Glu Leu His Gln Phe Val Asn
305 310 315 320

Leu Gly Ile Ala Val Gly Leu Asp Glu Gly Leu Leu Val Pro Val Val
325 330 335

His Gly Ala Asp Lys Met Ser Leu Ser Asp Phe Val Ile Ala Ser Lys
340 345 350

Asp Val Ile Lys Lys Ala Gln Thr Gly Lys Leu Lys Ala Thr Glu Met
355 360 365

Ser Gly Ser Thr Phe Ser Ile Thr Asn Leu Gly Met Phe Gly Thr Lys
    370                 375                 380

Thr Phe Asn Pro Ile Ile Asn Gln Pro Asn Ser Ala Ile Leu Gly Val
385                 390                 395                 400

Gly Ala Thr Ile Gln Thr Pro Thr Val Val Asp Gly Glu Ile Lys Ile
                405                 410                 415

Arg Pro Ile Met Ala Leu Cys Leu Thr Ile Asp His Arg Leu Val Asp
            420                 425                 430

Gly Met Asn Gly Ala Lys Phe Met Val Asp Leu Lys Lys Leu Met Glu
        435                 440                 445

Asn Pro Phe Thr Leu Leu Ile
    450                 455

<210>  56
<211>  1368
<212>  DNA
<213>  Streptococcus mutans

<220>
<221>  misc_feature
<223>  Encodes BKD E2 component

<400>  56
atggcagtcg aaattattat gcctaaactt ggtgttgata tgcaggaagg cgaaatcatc    60

gagtggaaaa aacaagaagg tgatgaggtc aaagaagggg agatcctcct tgagattatg    120

tctgacaaga ccaatatgga aatcgaagct gaggattcag gtgtcctgct taagattgtt    180

aaaggaaatg gtcaagttgt tcctgtaact gaggtcattg gttatattgg tcaagcaggt    240

gaagttcttg aaatagctga tgttcctgca agtacagttc ctaaagaaaa tagtgcagca    300

cctgctgaaa aaacaaaagc aatgtcttct ccgacagttg cagcagcccc tcaaggaaag    360

attcgagcaa caccagcagc tcgtaaggcg gctcgtgatc tgggagttaa cctgaatcag    420

gtttcaggga caggcgctaa aggccgtgtt cacaaggaag atgttgaaag ctttaaagca    480

gctcagccta aagcaacacc attagctagg aaaattgcta tagataaagg tattgatcta    540

gccagtgtct caggaacagg ttttggcggc aaaattatca aggaagatat tttaaatctg    600

tttgaggcag ctcagcctgt taatgatgtg tcagatcctg ctaaagaagc agctgcctta    660

ccagagggtg ttgaagtcat taagatgtct gccatgcgta aggcagtggc taaaagcatg    720

gtcaattctt acctgacagc tccaactttt actctcaatt atgacattga catgactgag    780

atgattgcgt tgcgtaaaaa gttaattgat cctatcatgg aaaaaacagg ttttaaagtt    840

agcttcacag atttgattgg tctggcagtc gtaaaaacct taatgaaacc agaacatcgt 900

tacctcaatg cttcactcat taatgacgcg actgagattg aacttcatca atttgttaac 960

cttggtatcg ccgttggact tgatgaagga ctgttagtac ctgttgttca tggtgcagat 1020

aagatgagct tgtcagattt tgttatagct tcaaaggatg tcattaagaa agctcagacc 1080

ggtaaattaa aagccactga aatgtctggt tcaacctttt ccattacaaa cttggggatg 1140

tttggcacta agactttcaa ccccattatc aatcagccaa attcggctat tttgggtgta 1200

ggagcaacta tccaaacgcc aactgttgtg gatggtgaaa ttaagattcg tccaatcatg 1260

gcactgtgct tgaccatcga tcaccgcttg gttgatggca tgaacggcgc taagttcatg 1320

gttgatctta aaaaactgat ggaaaatcca tttacattat tgatttga 1368

<210> 57
<211> 14
<212> PRT
<213> Artificial sequence

<220>
<223> Synthetic polypeptide

<220>
<221> MISC_FEATURE
<223> BKD E2 motif

<220>
<221> VARIANT
<222> (2)..(2)
<223> Xaa is any amino acid

<220>
<221> VARIANT
<222> (4)..(4)
<223> Xaa is Leu or Arg

<220>
<221> VARIANT
<222> (5)..(5)
<223> Xaa is any amino acid

<220>
<221> VARIANT
<222> (6)..(6)
<223> Xaa is Arg or Leu

<220>
<221> VARIANT
<222> (8)..(10)
<223> Xaa is any amino acid

<220>
<221> VARIANT
<222> (12)..(12)
<223> Xaa is any amino acid

<220>

<221> VARIANT
<222> (13)..(13)
<223> Xaa is Asp or Glu

<400> 57

Pro Xaa Val Xaa Xaa Xaa Ala Xaa Xaa Xaa Gly Xaa Xaa Leu
1 5 10

<210> 58
<211> 8
<212> PRT
<213> Artificial sequence

<220>
<223> Synthetic polypeptide

<220>
<221> MISC_FEATURE
<223> BKD E2 motif

<220>
<221> VARIANT
<222> (1)..(1)
<223> Xaa is Gly or Pro

<220>
<221> VARIANT
<222> (2)..(2)
<223> Xaa is Ser or Thr

<220>
<221> VARIANT
<222> (4)..(4)
<223> Xaa is Ala or Pro

<220>
<221> VARIANT
<222> (5)..(5)
<223> Xaa is any amino acid

<220>
<221> VARIANT
<222> (7)..(7)
<223> Xaa is any amino acid

<400> 58

Xaa Xaa Gly Xaa Xaa Gly Xaa Ile
1 5

<210> 59
<211> 16
<212> PRT
<213> Artificial sequence

<220>
<223> Synthetic polypeptide

<220>
<221> MISC_FEATURE
<223> BKD E2 motif

<220>
<221> VARIANT
<222> (1)..(1)
<223> Xaa is Val or Ile

<220>
<221> VARIANT
<222> (3)..(3)
<223> Xaa is Leu or Val

<220>
<221> VARIANT
<222> (4)..(4)
<223> Xaa is any amino acid

<220>
<221> VARIANT
<222> (6)..(6)
<223> Xaa is Leu or Val

<220>
<221> VARIANT
<222> (8)..(8)
<223> Xaa is any amino acid

<220>
<221> VARIANT
<222> (9)..(9)
<223> Xaa is Ala or Lys

<220>
<221> VARIANT
<222> (11)..(12)
<223> Xaa is any amino acid

<220>
<221> VARIANT
<222> (13)..(13)
<223> Xaa is Leu or Met

<220>
<221> VARIANT
<222> (14)..(15)
<223> Xaa is any amino acid

<220>
<221> VARIANT
<222> (16)..(16)
<223> Xaa is Ala or Ser

<400> 59

```
Xaa Pro Xaa Xaa Gly Xaa Arg Xaa Xaa Ala Xaa Xaa Xaa Xaa Xaa Xaa
1               5                   10                  15
```

<210> 60
<211> 13
<212> PRT

<213> Artificial sequence

<220>
<223> Synthetic polypeptide

<220>
<221> MISC_FEATURE
<223> BKD E2 motif

<220>
<221> VARIANT
<222> (2)..(2)
<223> Xaa is Gly or Ser

<220>
<221> VARIANT
<222> (4)..(4)
<223> Xaa is any amino acid

<220>
<221> VARIANT
<222> (6)..(7)
<223> Xaa is any amino acid

<220>
<221> VARIANT
<222> (8)..(8)
<223> Xaa is Asn or Ser

<220>
<221> VARIANT
<222> (9)..(9)
<223> Xaa is any amino acid

<220>
<221> VARIANT
<222> (11)..(11)
<223> Xaa is any amino acid

<220>
<221> VARIANT
<222> (12)..(12)
<223> Xaa is Phe or Leu

<400> 60

```
Gly Xaa Thr Xaa Thr Xaa Xaa Xaa Xaa Gly Xaa Xaa Gly
1               5                   10
```

<210> 61
<211> 19
<212> PRT
<213> Artificial sequence

<220>
<223> Synthetic polypeptide

<220>
<221> MISC_FEATURE
<223> BKD E2 motif

<220>
<221> VARIANT
<222> (2)..(2)
<223> Xaa is any amino acid

<220>
<221> VARIANT
<222> (5)..(5)
<223> Xaa is any amino acid

<220>
<221> VARIANT
<222> (7)..(7)
<223> Xaa is Ile or Met

<220>
<221> VARIANT
<222> (8)..(8)
<223> Xaa is Leu or Val

<220>
<221> VARIANT
<222> (9)..(9)
<223> Xaa is any amino acid

<220>
<221> VARIANT
<222> (11)..(12)
<223> Xaa is any amino acid

<220>
<221> VARIANT
<222> (13)..(13)
<223> Xaa is Ile or Met

<220>
<221> VARIANT
<222> (14)..(16)
<223> Xaa is any amino acid

<220>
<221> VARIANT
<222> (18)..(18)
<223> Xaa is any amino acid

<400> 61

```
Asn Xaa Pro Glu Xaa Ala Xaa Xaa Xaa Val Xaa Xaa Xaa Xaa Xaa Xaa
1               5                   10                  15


Pro Xaa Val
```

<210> 62
<211> 12
<212> PRT
<213> Artificial sequence

<220>
<223> Synthetic polypeptide

<220>
<221> MISC_FEATURE
<223> BKD E2 motif

<220>
<221> VARIANT
<222> (2)..(2)
<223> Xaa is any amino acid

<220>
<221> VARIANT
<222> (3)..(3)
<223> Xaa is Leu or Ser

<220>
<221> VARIANT
<222> (4)..(4)
<223> Xaa is Ser or Thr

<220>
<221> VARIANT
<222> (6)..(6)
<223> Xaa is Asp or Leu

<220>
<221> VARIANT
<222> (9)..(9)
<223> Xaa is Val or Leu

<220>
<221> VARIANT
<222> (10)..(10)
<223> Xaa is any amino acid

<400> 62

```
Leu Xaa Xaa Xaa Phe Xaa His Arg Xaa Xaa Asp Gly
1               5                   10
```

<210> 63
<211> 474
<212> PRT
<213> Bacillus subtilis

<220>
<221> MISC_FEATURE
<223> BKD E3 component

<400> 63

```
Met Ala Thr Glu Tyr Asp Val Val Ile Leu Gly Gly Gly Thr Gly Gly
1               5                   10                  15

Tyr Val Ala Ala Ile Arg Ala Ala Gln Leu Gly Leu Lys Thr Ala Val
            20                  25                  30

Val Glu Lys Glu Lys Leu Gly Gly Thr Cys Leu His Lys Gly Cys Ile
```

35 40 45

Pro Ser Lys Ala Leu Leu Arg Ser Ala Glu Val Tyr Arg Thr Ala Arg
50 55 60

Glu Ala Asp Gln Phe Gly Val Glu Thr Ala Gly Val Ser Leu Asn Phe
65 70 75 80

Glu Lys Val Gln Gln Arg Lys Gln Ala Val Val Asp Lys Leu Ala Ala
85 90 95

Gly Val Asn His Leu Met Lys Lys Gly Lys Ile Asp Val Tyr Thr Gly
100 105 110

Tyr Gly Arg Ile Leu Gly Pro Ser Ile Phe Ser Pro Leu Pro Gly Thr
115 120 125

Ile Ser Val Glu Arg Gly Asn Gly Glu Glu Asn Asp Met Leu Ile Pro
130 135 140

Lys Gln Val Ile Ile Ala Thr Gly Ser Arg Pro Arg Met Leu Pro Gly
145 150 155 160

Leu Glu Val Asp Gly Lys Ser Val Leu Thr Ser Asp Glu Ala Leu Gln
165 170 175

Met Glu Glu Leu Pro Gln Ser Ile Ile Ile Val Gly Gly Gly Val Ile
180 185 190

Gly Ile Glu Trp Ala Ser Met Leu His Asp Phe Gly Val Lys Val Thr
195 200 205

Val Ile Glu Tyr Ala Asp Arg Ile Leu Pro Thr Glu Asp Leu Glu Ile
210 215 220

Ser Lys Glu Met Glu Ser Leu Leu Lys Lys Lys Gly Ile Gln Phe Ile
225 230 235 240

Thr Gly Ala Lys Val Leu Pro Asp Thr Met Thr Lys Thr Ser Asp Asp
245 250 255

Ile Ser Ile Gln Ala Glu Lys Asp Gly Glu Thr Val Thr Tyr Ser Ala
260 265 270

Glu Lys Met Leu Val Ser Ile Gly Arg Gln Ala Asn Ile Glu Gly Ile
275 280 285

```
Gly Leu Glu Asn Thr Asp Ile Val Thr Glu Asn Gly Met Ile Ser Val
    290                 295                 300

Asn Glu Ser Cys Gln Thr Lys Glu Ser His Ile Tyr Ala Ile Gly Asp
305                 310                 315                 320

Val Ile Gly Gly Leu Gln Leu Ala His Val Ala Ser His Glu Gly Ile
                325                 330                 335

Ile Ala Val Glu His Phe Ala Gly Leu Asn Pro His Pro Leu Asp Pro
            340                 345                 350

Thr Leu Val Pro Lys Cys Ile Tyr Ser Ser Pro Glu Ala Ala Ser Val
        355                 360                 365

Gly Leu Thr Glu Asp Glu Ala Lys Ala Asn Gly His Asn Val Lys Ile
    370                 375                 380

Gly Lys Phe Pro Phe Met Ala Ile Gly Lys Ala Leu Val Tyr Gly Glu
385                 390                 395                 400

Ser Asp Gly Phe Val Lys Ile Val Ala Asp Arg Asp Thr Asp Asp Ile
                405                 410                 415

Leu Gly Val His Met Ile Gly Pro His Val Thr Asp Met Ile Ser Glu
            420                 425                 430

Ala Gly Leu Ala Lys Val Leu Asp Ala Thr Pro Trp Glu Val Gly Gln
        435                 440                 445

Thr Ile His Pro His Pro Thr Leu Ser Glu Ala Ile Gly Glu Ala Ala
    450                 455                 460

Leu Ala Ala Asp Gly Lys Ala Ile His Phe
465                 470
```

<210> 64
<211> 1425
<212> DNA
<213> Bacillus subtilis

<220>
<221> misc_feature
<223> Encodes BKD E3 component

<400> 64

```
ttaaaaatga atggctttgc catctgcggc aagcgcagct tctccaattg cttcagaaag        60

cgttggatgc gggtgaatcg tttgcccgac ctcccacggt gttgcgtcca gcactttggc       120
```

aagacccgct tcagaaatca tgtcggtgac atgcgggcca atcatatgaa cgccgagaat 180

atcatctgta tctcggtcag ccacgatttt gacaaaaccg tcgctttcac cgtatacaag 240

cgcttttcca atcgccataa atgggaactt gccgattttg acattatgcc cgttcgcctt 300

tgcttcgtct tcggttaagc cgacactggc agcttcaggg cttgagtaaa tgcacttcgg 360

cacaagcgtc ggatcaagcg gatgcggatt gagacctgca aaatgctcaa cagcaataat 420

tccctcatgt gaagcaacgt gagctaactg caggccaccg attacgtctc cgattgcata 480

aatatgagat tccttcgttt ggcagctttc attgactgaa atcatgccat tttcagtaac 540

aatatcggtg ttctctaggc cgatgccttc gatatttgcc tgtctgccga tggaaacaag 600

cattttctca gcagaatagg taacggtttc tccgtctttt tccgcttgta tgctgatatc 660

gtctgatgtt tttgtcattg tgtcaggcag cacttttgcc cctgttatga actggatgcc 720

ttttttctta agaagacttt ccatttcttt tgaaatctct agatcttcag tcggcaatat 780

gcgatccgcg tattcaataa ccgttacctt aacgccaaaa tcatgaagca tagacgccca 840

ttcgataccg ataacccctc cgccgacaat gatgattgac tgtggcagct cctccatttg 900

gagcgcctca tctgaagtca gtacagactt accgtccact tcaagacccg gaagcattct 960

cggtcttgat cctgttgcaa tgatcacttg tttcgggatc agcatgtcat tttcttcgcc 1020

atttccccgc tcaacagaaa ttgttcccgg cagcggagag aagattgacg gtccaaggat 1080

acgtccatat ccggtgtaca cgtcaatttt tcctttttc attaaatgat ttacacccgc 1140

tgcaagctta tcaacaacgg cttgcttacg ctgctgcact ttttcaaagt tgagggacac 1200

gccagccgtt tccactccga attgatcggc ttcacgagct gtccggtata cctctgcgct 1260

tctaagcagc gctttactcg ggatacagcc tttatgcaga catgttcccc cgagtttttc 1320

cttttccaca acggctgttt ttaagccgag ctgagcggct ctgatggccg caacataacc 1380

gccggtaccg ccgcccagaa tgactacgtc atactcagtt gccat 1425

<210> 65
<211> 462
<212> PRT
<213> Streptomyces avermitilis

<220>
<221> MISC_FEATURE
<223> BKD E3 component

<400> 65

Met Ala Asn Asp Ala Ser Thr Val Phe Asp Leu Val Ile Leu Gly Gly
1 5 10 15

Gly Ser Gly Gly Tyr Ala Ala Ala Leu Arg Gly Ala Gln Leu Gly Leu
20 25 30

```
Asp Val Ala Leu Ile Glu Lys Asp Lys Val Gly Gly Thr Cys Leu His
        35                  40                  45

Arg Gly Cys Ile Pro Thr Lys Ala Leu Leu His Ala Gly Glu Ile Ala
    50                  55                  60

Asp Gln Ala Arg Glu Ser Glu Gln Phe Gly Val Lys Ala Thr Phe Glu
65                  70                  75                  80

Gly Ile Asp Val Pro Ala Val His Lys Tyr Lys Asp Gly Val Ile Ser
                85                  90                  95

Gly Leu Tyr Lys Gly Leu Gln Gly Leu Ile Ala Ser Arg Lys Val Thr
            100                 105                 110

Tyr Ile Glu Gly Glu Gly Arg Leu Ser Ser Pro Thr Ser Val Asp Val
        115                 120                 125

Asn Gly Gln Arg Val Gln Gly Arg His Val Leu Leu Ala Thr Gly Ser
    130                 135                 140

Val Pro Lys Ser Leu Pro Gly Leu Ala Ile Asp Gly Asn Arg Ile Ile
145                 150                 155                 160

Ser Ser Asp His Ala Leu Val Leu Asp Arg Val Pro Glu Ser Ala Ile
                165                 170                 175

Val Leu Gly Gly Gly Val Ile Gly Val Glu Phe Ala Ser Ala Trp Lys
            180                 185                 190

Ser Phe Gly Ala Asp Val Thr Val Ile Glu Gly Leu Lys His Leu Val
        195                 200                 205

Pro Val Glu Asp Glu Asn Ser Ser Lys Leu Leu Glu Arg Ala Phe Arg
    210                 215                 220

Lys Arg Gly Ile Lys Phe Asn Leu Gly Thr Phe Phe Ser Lys Ala Glu
225                 230                 235                 240

Tyr Thr Gln Asn Gly Val Lys Val Thr Leu Ala Asp Gly Lys Glu Phe
                245                 250                 255

Glu Ala Glu Val Leu Leu Val Ala Val Gly Arg Gly Pro Val Ser Gln
            260                 265                 270

Gly Leu Gly Tyr Glu Glu Gln Gly Val Ala Met Asp Arg Gly Tyr Val
```

275 280 285

Leu Val Asp Glu Tyr Met Arg Thr Asn Val Pro Thr Ile Ser Ala Val
290 295 300

Gly Asp Leu Val Pro Thr Leu Gln Leu Ala His Val Gly Phe Ala Glu
305 310 315 320

Gly Ile Leu Val Ala Glu Arg Leu Ala Gly Leu Lys Thr Val Pro Ile
325 330 335

Asp Tyr Asp Gly Val Pro Arg Val Thr Tyr Cys His Pro Glu Val Ala
340 345 350

Ser Val Gly Ile Thr Glu Ala Lys Ala Lys Glu Ile Tyr Gly Ala Asp
355 360 365

Lys Val Val Ala Leu Lys Tyr Asn Leu Ala Gly Asn Gly Lys Ser Lys
370 375 380

Ile Leu Asn Thr Ala Gly Glu Ile Lys Leu Val Gln Val Lys Asp Gly
385 390 395 400

Ala Val Val Gly Val His Met Val Gly Asp Arg Met Gly Glu Gln Val
405 410 415

Gly Glu Ala Gln Leu Ile Tyr Asn Trp Glu Ala Leu Pro Ala Glu Val
420 425 430

Ala Gln Leu Ile His Ala His Pro Thr Gln Asn Glu Ala Met Gly Glu
435 440 445

Ala His Leu Ala Leu Ala Gly Lys Pro Leu His Ser His Asp
450 455 460

<210> 66
<211> 1389
<212> DNA
<213> Streptomyces avermitilis

<220>
<221> misc_feature
<223> Encodes BKD E3 component

<400> 66
tcagtcgtgc gagtgcagcg gcttgcccgc gagggccagg tgggcctcgc ccatcgcttc 60

gttctgcgtc gggtgggcgt ggatgagctg ggcgacctcg gccggcagcg cctcccagtt 120

gtagatcagc tgggcttcgc cgacctgctc gcccatacgg tcaccgacca tgtggacgcc 180

gaccacggca ccgtccttca cctggacgag cttgatctcg cccgcggtgt tgaggatctt 240

gctcttgccg ttgcccgcca ggttgtactt cagagcgacg accttgtccg cgccgtagat 300

ctccttggcc ttggcctcgg tgatgcccac ggaggcgacc tcggggtggc agtacgtcac 360

ccgcggcacg ccgtcgtagt cgatcgggac ggtcttcaga ccggccagac gctccgccac 420

caggatgccc tcggcgaagc cgacgtgcgc gagctggagc gtcgggacca ggtcaccgac 480

ggcggagatg gtcgggacgt tcgtccgcat gtactcgtcg accaggacgt agccgcggtc 540

catggcgacg ccctgctcct cgtagccgag gccctgcgag accgggccgc ggccgacggc 600

gacgagcagg acctcggcct cgaactcctt gccgtcggcg agggtgacct tgacaccgtt 660

ctgggtgtac tcggccttcg agaagaaggt gcccaggttg aacttgatgc cgcgcttgcg 720

gaacgcgcgc tcaagaagct tggaggagtt ctcgtcctcg accgggacga ggtgcttgag 780

gccctcgatc accgtcacgt cggctccgaa ggacttccac gcggaggcga actcgacgcc 840

gatgacgccg ccgccgagca cgatcgcgga ctccgggacg cggtccagga ccagcgcgtg 900

gtcggaggag atgatgcggt tgccgtcgat cgccaggccc ggcagcgact tcggcacgga 960

gccggtcgcc aggagcacgt ggcggccctg gacgcgctgg ccgttcacgt cgacggaggt 1020

cggggaggac agacggccct caccctcgat gtacgtcacc ttgcgggagg cgatcagccc 1080

ctgcagaccc ttgtacaggc ccgagatgac cccgtccttg tacttgtgga cggccggtac 1140

gtcgatgccc tcgaaggtgg ccttgacgcc gaactgctcg ctctcgcggg cctggtcggc 1200

gatctcgccc gcgtgcagca gcgccttggt ggggatgcac ccacggtgca ggcaggtacc 1260

gccgaccttg tccttctcga tcagggcgac gtccaggccc agctgcgctc cgcgcagggc 1320

cgcggcgtaa ccaccgctac caccgccgag gatcactagg tcgaaaacgg tgctggcgtc 1380

gttcgccac 1389

<210> 67
<211> 459
<212> PRT
<213> Pseudomonas putida

<220>
<221> MISC_FEATURE
<223> BKD E3 component

<400> 67

Met Gln Gln Ile Ile Gln Thr Thr Leu Leu Ile Ile Gly Gly Gly Pro
1               5                   10                  15

Gly Gly Tyr Val Ala Ala Ile Arg Ala Gly Gln Leu Gly Ile Pro Thr
            20                  25                  30

```
Val Leu Val Glu Gly Gln Ala Leu Gly Gly Thr Cys Leu Asn Ile Gly
        35                  40                  45

Cys Ile Pro Ser Lys Ala Leu Ile His Val Ala Glu Gln Phe His Gln
    50                  55                  60

Ala Ser Arg Phe Thr Glu Pro Ser Pro Leu Gly Ile Ser Val Ala Ser
65                  70                  75                  80

Pro Arg Leu Asp Ile Gly Gln Ser Val Thr Trp Lys Asp Gly Ile Val
                85                  90                  95

Asp Arg Leu Thr Thr Gly Val Ala Ala Leu Leu Lys Lys His Gly Val
            100                 105                 110

Lys Val Val His Gly Trp Ala Lys Val Leu Asp Gly Lys Gln Val Glu
        115                 120                 125

Val Asp Gly Gln Arg Ile Gln Cys Glu His Leu Leu Leu Ala Thr Gly
    130                 135                 140

Ser Thr Ser Val Glu Leu Pro Met Leu Pro Leu Gly Gly Pro Val Ile
145                 150                 155                 160

Ser Ser Thr Glu Ala Leu Ala Pro Lys Ala Leu Pro Gln His Leu Val
                165                 170                 175

Val Val Gly Gly Gly Tyr Ile Gly Leu Glu Leu Gly Ile Ala Tyr Arg
            180                 185                 190

Lys Leu Gly Ala Gln Val Ser Val Val Glu Ala Arg Glu Arg Ile Leu
        195                 200                 205

Pro Thr Tyr Asp Ser Glu Leu Thr Ala Pro Val Ala Glu Ser Leu Lys
    210                 215                 220

Lys Leu Gly Ile Ala Leu His Leu Gly His Ser Val Glu Gly Tyr Glu
225                 230                 235                 240

Asn Gly Cys Leu Leu Ala Ser Asp Gly Lys Gly Gly Gln Leu Arg Leu
                245                 250                 255

Glu Ala Asp Gln Val Leu Val Ala Val Gly Arg Arg Pro Arg Thr Lys
            260                 265                 270

Gly Phe Asn Leu Glu Cys Leu Asp Leu Lys Met Asn Gly Thr Ala Ile
        275                 280                 285
```

Ala Ile Asp Glu Arg Cys His Thr Ser Met His Asn Val Trp Ala Ile
    290                 295                 300

Gly Asp Val Ala Gly Glu Pro Met Leu Ala His Arg Ala Met Ala Gln
305                 310                 315                 320

Gly Glu Met Val Ala Glu Ile Ile Ala Gly Lys Ala Arg Arg Phe Glu
                325                 330                 335

Pro Ala Ala Ile Ala Ala Val Cys Phe Thr Asp Pro Glu Val Val Val
            340                 345                 350

Val Gly Lys Thr Pro Glu Gln Ala Ser Gln Gln Ala Leu Asp Cys Ile
        355                 360                 365

Val Ala Gln Phe Pro Phe Ala Ala Asn Gly Arg Ala Met Ser Leu Glu
    370                 375                 380

Ser Lys Ser Gly Phe Val Arg Val Val Ala Arg Arg Asp Asn His Leu
385                 390                 395                 400

Ile Val Gly Trp Gln Ala Val Gly Val Ala Val Ser Glu Leu Ser Thr
                405                 410                 415

Ala Phe Ala Gln Ser Leu Glu Met Gly Ala Cys Leu Glu Asp Val Ala
            420                 425                 430

Gly Thr Ile His Ala His Pro Thr Leu Gly Glu Ala Val Gln Glu Ala
        435                 440                 445

Ala Leu Arg Ala Leu Gly His Ala Leu His Ile
    450                 455

<210> 68
<211> 1380
<212> DNA
<213> Pseudomonas putida

<220>
<221> misc_feature
<223> Encodes BKD E3 component

<400> 68
tcagatatgc aaggcgtggc ccaacgcgcg tagggcggct tcttgcaccg cttcacccaa 60

cgtggggtgg gcatgaatgg tgccggccac atcttccagg cacgcgccca tctccagcga 120

ttgggcaaac gcggtggaca gctcggagac cgccacgcca accgcctgcc aacccacgat 180

```
caggtggttg tcacggcgcg ccaccacccg cacgaaaccg cttttcgact ccaggctcat      240
ggcccggcca ttggcggcaa acgggaactg cgcgacgatg cagtccaggg cctgctggct      300
ggcttgttcc ggggtcttgc cgaccaccac cacttccggg tcggtaaagc acacggcggc      360
aatcgctgcc ggctcgaagc gtcgggcctt gccggcgatg atttcggcga ccatctcgcc      420
ttgggccatg gcccggtgcg ccagcatcgg ttcgccagcg acgtcgccaa tggcccagac      480
gttgtgcatg ctggtatgac agcgctcgtc gatggcaatg gcggtgccgt tcatcttcag      540
gtccaggcat tccaggttga agcccttggt gcgtggccgg cggcccacgg ccaccagtac      600
ctgatcggct tcaagacgca gttgcccacc cttgccgtcg ctggccagca ggcagccatt      660
ttcgtagccc tcgacgctgt ggcccaggtg caacgcgatg cccagtttct tcagcgactc      720
ggccaccggg gcggtcaatt cgctgtcgta ggtcggcagg atgcgttcgc gcgcttccac      780
cacactcacc tgtgcaccca gcttgcgata ggcaatgccc agctccaggc cgatatagcc      840
accgccgacc accaccaggt gttgcggcag ggctttcggc gccagggctt cggtcgagga      900
aatcaccggg ccacccagcg gcagcatcgg cagttcgaca ctggtggaac cggtcgccag      960
caacagatgc tcgcactgga tacgctggcc atcgacctcg acctgcttgc cgtccagtac     1020
cttggcccag ccatgcacca ctttcacccc gtgctttttc agcaaggcgg caacaccggt     1080
ggtcagacgg tcgacaatgc cgtccttcca ggtgacgctc tggccgatgt ccaggcgcgg     1140
cgaagccacg ctgatgccca gcggcgaggg ttcggtaaag cgcgaggctt ggtgaaactg     1200
ctcggccacg tggatcagcg ccttggacgg gatgcagccg atgttcaggc aggtgccgcc     1260
cagtgcctgg ccttccacca gtacggtagg aatgcccagt tgcccggcgc ggatggctgc     1320
tacatagccg ccagggccgc cgccgatgat caacagggta gtctggataa tctgttgcat     1380
```

<210> 69
<211> 467
<212> PRT
<213> Listeria monocytogenes

<220>
<221> MISC_FEATURE
<223> BKD E3 component

<400> 69

```
Met Val Val Gly Asp Phe Pro Glu Glu Arg Asp Thr Ile Val Ile Gly
1               5                   10                  15

Ala Gly Pro Gly Gly Tyr Val Ala Ala Ile Arg Ala Ala Gln Leu Gly
            20                  25                  30

Gln Lys Val Thr Ile Ile Glu Lys Glu Tyr Tyr Gly Gly Val Cys Leu
        35                  40                  45
```

```
Asn Val Gly Cys Ile Pro Ser Lys Ala Leu Ile Thr Ile Gly His Arg
    50                  55                  60

Phe Lys Glu Ala Gly His Ser Asp Asn Met Gly Ile Thr Ala Asp Asn
65                  70                  75                  80

Val Asn Leu Asp Phe Thr Lys Ala Gln Glu Trp Lys Gly Gly Val Val
                85                  90                  95

Asn Lys Leu Thr Ser Gly Val Lys Gly Leu Leu Lys Lys Asn Lys Val
            100                 105                 110

Glu Met Leu Glu Gly Glu Ala Phe Phe Val Asp Asp His Ser Leu Arg
        115                 120                 125

Val Ile His Pro Asp Ser Ala Gln Thr Tyr Thr Phe Asn Asn Val Ile
    130                 135                 140

Ile Ala Thr Gly Ser Arg Pro Ile Glu Ile Pro Gly Phe Lys Tyr Gly
145                 150                 155                 160

Lys Arg Val Leu Ser Ser Thr Gly Ala Leu Ala Leu Thr Glu Val Pro
                165                 170                 175

Lys Lys Leu Val Val Ile Gly Gly Gly Tyr Ile Gly Thr Glu Leu Gly
            180                 185                 190

Gly Ala Phe Ala Asn Leu Gly Thr Glu Leu Thr Ile Leu Glu Gly Gly
        195                 200                 205

Pro Glu Ile Leu Pro Thr Tyr Glu Lys Asp Met Val Ser Leu Val Lys
    210                 215                 220

Arg Asn Leu Lys Ser Lys Asn Val Glu Met Val Thr Lys Ala Leu Ala
225                 230                 235                 240

Lys Ser Ala Glu Glu Thr Glu Asn Gly Val Lys Val Thr Tyr Glu Ala
                245                 250                 255

Asn Gly Glu Thr Lys Thr Ile Glu Ala Asp Tyr Val Leu Val Thr Val
            260                 265                 270

Gly Arg Arg Pro Asn Thr Asp Glu Ile Gly Leu Glu Gln Ala Gly Val
        275                 280                 285

Lys Val Thr Glu Arg Gly Leu Val Glu Val Asp Lys Gln Gly Arg Ser
```

290 295 300

Asn Val Ser Asn Ile Phe Ala Ile Gly Asp Ile Val Pro Gly Val Pro
305 310 315 320

Leu Ala His Lys Ala Ser Tyr Glu Ala Lys Ile Ala Ala Glu Ala Ile
325 330 335

Ala Gly Glu Lys Ser Glu Asn Asp Tyr Thr Ala Leu Pro Ala Val Val
340 345 350

Phe Ser Asp Pro Glu Leu Ala Thr Val Gly Leu Thr Glu Lys Glu Ala
355 360 365

Lys Glu Lys Gly Phe Asp Val Lys Ala Ala Lys Phe Pro Phe Gly Gly
370 375 380

Asn Gly Arg Ala Leu Ser Leu Asp Ala Pro Glu Gly Phe Val Arg Leu
385 390 395 400

Val Thr Arg Lys Glu Asp Gly Leu Val Ile Gly Ala Gln Val Ala Gly
405 410 415

Met Asn Ala Ser Asp Ile Ile Ser Glu Ile Gly Leu Ala Ile Glu Ser
420 425 430

Gly Ile Thr Ala Glu Asp Ile Ala Leu Thr Ile His Ala His Pro Ser
435 440 445

Leu Gly Glu Leu Thr Met Glu Ala Ala Glu Leu Ala Leu Gly Arg Pro
450 455 460

Ile His Met
465

<210> 70
<211> 1404
<212> DNA
<213> Listeria monocytogenes

<220>
<221> misc_feature
<223> Encodes BKD E3 component

<400> 70
atggtagtag gcgattttcc agaagaaaga gacaccatag tcatcggtgc aggcccaggt 60

gggtatgtag ccgcaatccg agctgcacaa ctcggacaaa aagttaccat tattgaaaaa 120

gaatattacg gcggtgtgtg cttaaacgtg ggatgtattc cttccaaagc acttatcaca 180

atcggtcacc gttttaaaga agctggacac tctgataaca tgggtattac agcggacaac 240
gtgaacttag atttcacaaa agcacaagaa tggaaaggcg gcgtagttaa caagcttaca 300
tcaggtgtta aaggccttct taagaaaaat aaagtagaaa tgttagaagg agaagcgttc 360
ttcgtggatg atcattcctt acgtgtgatt caccctgatt ccgctcaaac ttatacattc 420
aataacgtta tcattgcaac aggatctcgt ccaatcgaaa tcccaggttt caaatatggt 480
aaacgtgtat taagctcaac tggtgcactt gcactaacag aagttccgaa aaaattagtc 540
gtaattggcg gcggatatat cggaacagaa ctaggtggag cattcgctaa ccttggtaca 600
gaactaacta tccttgaggg tggtccagaa atcttaccaa catacgaaaa agacatggtt 660
tccctagtta aacgtaatct gaaaagcaaa aacgttgaaa tggttactaa agcacttgca 720
aaatctgctg aagaaactga aaacggcgtt aaagtaacgt atgaagctaa cggtgaaaca 780
aaaactatcg aagctgatta tgttttagta acagtaggtc gtcgtccaaa tacagacgaa 840
atcggtttag aacaagctgg cgttaaagta acagaacgcg gtttagtaga agttgataaa 900
caaggtcgct cgaacgtttc taatattttc gcaattggtg acatcgttcc tggtgttcct 960
ctagctcaca aagctagcta tgaagcaaaa atcgctgctg aagcaattgc tggcgaaaaa 1020
tccgaaaacg attatacagc acttccagct gtcgttttca gtgatccaga acttgctaca 1080
gttggtttaa cagaaaaaga agcaaaagaa aaaggctttg atgtaaaagc tgctaaattc 1140
ccattcggcg gtaacggtcg tgcgctttcc ttagatgcac ctgaaggatt tgttcgtcta 1200
gttactcgta aagaagatgg cctagttatc ggcgcacaag ttgccggaat gaacgcttct 1260
gatattattt cagaaatcgg cttagcaatc gaatcaggca ttacagcaga agacatcgcg 1320
cttactatcc acgctcaccc atcactagga gagcttacaa tggaagcagc cgaacttgct 1380
ttaggtcgcc caattcatat gtaa 1404

<210> 71
<211> 476
<212> PRT
<213> Micrococcus luteus

<220>
<221> MISC_FEATURE
<223> BKD E3 component

<400> 71

Met Thr Glu Glu Asn Ser Thr Phe Ile Pro Ser Leu Thr Ile Ile Gly
1               5                   10                  15

Gly Gly Pro Gly Gly Tyr Glu Ala Ala Met Val Ala Ala Lys Leu Gly
            20                  25                  30

```
Ala Arg Val Thr Leu Val Glu Arg Gln Gly Val Gly Gly Ala Ala Val
        35                  40                  45

Leu Thr Asp Val Val Pro Ser Lys Thr Leu Ile Ala Ala Ala Asp Ser
    50                  55                  60

Met Arg Arg Val Gly Ala Ser Val Asp Leu Gly Val Asp Leu Gly Gly
65                  70                  75                  80

Ala Glu Val His Ala Asp Met Gly Arg Val Gly His Arg Ile Leu Asn
                85                  90                  95

Leu Ala His Glu Gln Ser Ser Asp Ile Arg Ala Gly Leu Glu Arg Val
            100                 105                 110

Gly Val Arg Val Ile Asp Gly Val Gly Arg Val Val Gly Pro His Glu
        115                 120                 125

Val Ser Val Arg Ala Leu Asp Asp Ala Asp Ala Gly Ala Glu Pro Glu
    130                 135                 140

Ile Ile Thr Ser Asp Ala Ile Leu Val Ala Val Gly Ala Ser Pro Arg
145                 150                 155                 160

Glu Leu Pro Thr Ala Val Pro Asp Gly Glu Arg Ile Phe Asn Trp Lys
                165                 170                 175

Gln Val Tyr Asn Leu Lys Glu Leu Pro Glu His Leu Ile Val Val Gly
            180                 185                 190

Ser Gly Val Thr Gly Ala Glu Phe Ala Ser Ala Tyr Asn Arg Leu Gly
        195                 200                 205

Ala Lys Val Thr Leu Val Ser Ser Arg Asp Arg Val Leu Pro Gly Glu
    210                 215                 220

Asp Ala Asp Ala Ala Glu Leu Leu Glu Lys Val Phe Glu Gly Asn Gly
225                 230                 235                 240

Leu Arg Val Val Ser Arg Ser Arg Ala Glu Ser Val Glu Arg Thr Glu
                245                 250                 255

Thr Gly Val Arg Val His Leu Ser Gly Glu Gly Ala Glu Asp Thr Pro
            260                 265                 270

Ser Ile Glu Gly Ser His Ala Leu Val Ala Val Gly Gly Val Pro Asn
        275                 280                 285
```

Thr Ala Gly Leu Gly Leu Asp Asp Val Gly Val Lys Leu Ala Asp Ser
    290                 295                 300

Gly His Val Leu Val Asp Gly Val Ser Arg Thr Ser Val Pro Ser Ile
305                 310                 315                 320

Tyr Ala Ala Gly Asp Cys Thr Gly Lys Leu Ala Leu Ala Ser Val Ala
                325                 330                 335

Ala Met Gln Gly Arg Ile Ala Val Ala His Leu Leu Gly Asp Ala Leu
            340                 345                 350

Lys Pro Leu Arg Pro His Leu Leu Ala Ser Asn Ile Phe Thr Ser Pro
        355                 360                 365

Glu Ile Ala Thr Val Gly Val Ser Gln Ala Gln Val Asp Ser Gly Gln
    370                 375                 380

Tyr Gln Ala Asp Val Leu Arg Leu Asp Phe His Thr Asn Pro Arg Ala
385                 390                 395                 400

Lys Met Ser Gly Ala Glu Glu Gly Phe Val Lys Ile Phe Ala Arg Gln
                405                 410                 415

Gly Ser Gly Thr Val Ile Gly Gly Val Val Val Ser Pro Arg Ala Ser
            420                 425                 430

Glu Leu Ile Tyr Ala Leu Ala Leu Ala Val Thr His Lys Leu His Val
        435                 440                 445

Asp Asp Leu Ala Asp Thr Phe Thr Val Tyr Pro Ser Met Ser Gly Ser
    450                 455                 460

Ile Ala Glu Ala Ala Arg Arg Leu His Val Arg Val
465                 470                 475

<210> 72
<211> 1431
<212> DNA
<213> Micrococcus luteus

<220>
<221> misc_feature
<223> Encodes BKD E3 component

<400> 72
gtgaccgagg aaaacagcac cttcatcccg tccctgacca tcatcggcgg cggccccggc 60

```
ggctacgagg ccgccatggt ggccgcgaag ctgggcgccc gcgtgaccct ggtcgagcgc      120
caggggggtgg gcggcgcggc cgtcctcacg gacgtggtcc cctccaagac gctgatcgcc      180
gccgccgact cgatgcgccg cgtgggcgcc tccgtggacc tgggggtcga cctcggcggg      240
gccgaggtcc acgcggacat gggccgggtc ggccaccgca tcctgaacct ggcccacgag      300
cagtcctcgg acatccgcgc gggcctcgag cgggtcggtg tccgggtgat cgacggcgtg      360
ggccgcgtcg tcggccccca cgaggtgtcc gtccgcgccc tcgacgacgc cgacgccggc      420
gccgagcccg agatcatcac ctcggacgcg atcctcgtgg ccgtcggcgc gagtccccgg      480
gagctgccca ccgccgtccc ggacggcgag cggatcttca actggaagca ggtctacaac      540
ctcaaggagc tgcccgagca cctgatcgtc gtgggctccg gcgtcaccgg cgccgagttc      600
gcctcggcct acaaccgcct cggcgccaag gtcaccctcg tctcctcgcg cgaccgcgtg      660
ctccccggcg aggacgccga cgccgcagag ctgctcgaga aggtcttcga gggcaacggc      720
ctcagggttg tctcccgctc ccgggccgag tcggtcgagc ggaccgagac cggcgtgcgc      780
gtgcacctct ccggcgaggg ggccgaagac accccgtcga tcgagggctc ccacgcgctg      840
gtggccgtcg gcggcgtgcc gaacacggcg ggcctcggcc tcgacgacgt gggcgtgaag      900
ctggccgact ccggccacgt gctcgtggac ggcgtctccc gcacgtccgt gccgagcatc      960
tacgcggcgg gcgactgcac gggcaagctc gccctcgcct cggtggcggc catgcagggg     1020
cgcatcgccg tggcccacct gctcggcgac gccctcaagc cgctgcgccc gcacctgctg     1080
gcctcgaaca tcttcacctc gccggagatc gccaccgtgg gcgtctcgca ggcgcaggtg     1140
gactccggcc agtaccaggc ggacgtgctg cgactggact tccacaccaa cccccgcgcc     1200
aagatgtccg gcgcggagga ggggttcgtg aagatcttcg cgcgtcaggg ctccggcacc     1260
gtgatcggcg gcgtggtggt ctcgccgcgc gcctccgagc tgatctacgc gctcgcgctc     1320
gcggtcacgc acaagttgca cgtggacgac ctcgcggaca ccttcaccgt gtacccgtcc     1380
atgtccgggt cgatcgcgga ggcggcgcgc cgcctccatg tgcgggtgtg a              1431
```

<210>  73
<211>  473
<212>  PRT
<213>  Staphylococcus aureus

<220>
<221>  MISC_FEATURE
<223>  BKD E3 component

<400>  73

```
Met Ser Glu Lys Gln Tyr Asp Leu Val Val Leu Gly Gly Gly Thr Ala
1               5                   10                  15
```

```
Gly Tyr Val Ala Ala Ile Arg Ala Ser Gln Leu Gly Lys Lys Val Ala
            20                  25                  30

Ile Val Glu Arg Gln Leu Leu Gly Gly Thr Cys Leu His Lys Gly Cys
        35                  40                  45

Ile Pro Thr Lys Ser Leu Leu Lys Ser Ala Glu Val Phe Gln Thr Val
    50                  55                  60

Lys Gln Ala Ala Met Phe Gly Val Asp Val Lys Asp Ala Asn Val Asn
65                  70                  75                  80

Phe Glu Asn Met Leu Ala Arg Lys Glu Asp Ile Ile Asn Gln Met Tyr
                85                  90                  95

Gln Gly Val Lys His Leu Met Gln His Asn His Ile Asp Ile Tyr Asn
            100                 105                 110

Gly Thr Gly Arg Ile Leu Gly Thr Ser Ile Phe Ser Pro Gln Ser Gly
        115                 120                 125

Thr Ile Ser Val Glu Tyr Glu Asp Gly Glu Ser Asp Leu Leu Pro Asn
    130                 135                 140

Gln Phe Val Leu Ile Ala Thr Gly Ser Ser Pro Ala Glu Leu Pro Phe
145                 150                 155                 160

Leu Ser Phe Asp His Asp Lys Ile Leu Ser Ser Asp Asp Ile Leu Ser
                165                 170                 175

Leu Lys Thr Leu Pro Ser Ser Ile Gly Ile Ile Gly Gly Gly Val Ile
            180                 185                 190

Gly Met Glu Phe Ala Ser Leu Met Ile Asp Leu Gly Val Asp Val Thr
        195                 200                 205

Val Ile Glu Ala Gly Glu Arg Ile Leu Pro Thr Glu Ser Lys Gln Ala
    210                 215                 220

Ser Gln Leu Leu Lys Lys Ser Leu Ser Ala Arg Gly Val Lys Phe Tyr
225                 230                 235                 240

Glu Gly Ile Lys Leu Ser Glu Asn Asp Ile Asn Val Asn Glu Asp Gly
                245                 250                 255

Val Thr Phe Glu Ile Ser Ser Asp Ile Ile Lys Val Asp Lys Val Leu
            260                 265                 270
```

Leu Ser Ile Gly Arg Lys Pro Asn Thr Ser Asp Ile Gly Leu Asn Asn
275 280 285

Thr Lys Ile Lys Leu Ser Thr Ser Gly His Ile Leu Thr Asn Glu Phe
290 295 300

Gln Gln Thr Glu Asp Lys His Ile Tyr Ala Ala Gly Asp Cys Ile Gly
305 310 315 320

Lys Leu Gln Leu Ala His Val Gly Ser Lys Glu Gly Val Val Ala Val
325 330 335

Asp His Met Phe Glu Gly Asn Pro Ile Pro Val Asn Tyr Asn Met Met
340 345 350

Pro Lys Cys Ile Tyr Ser Gln Pro Glu Ile Ala Ser Ile Gly Leu Asn
355 360 365

Ile Glu Gln Ala Lys Ala Glu Gly Met Lys Val Lys Ser Phe Lys Val
370 375 380

Pro Phe Lys Ala Ile Gly Lys Ala Val Ile Asp Ser His Asp Ala Asn
385 390 395 400

Glu Gly Tyr Ser Glu Met Val Ile Asp Gln Ser Thr Glu Glu Ile Val
405 410 415

Gly Ile Asn Met Ile Gly Gln His Val Thr Glu Leu Ile Asn Glu Ala
420 425 430

Ser Leu Leu Gln Phe Met Asn Gly Ser Ala Leu Glu Leu Gly Leu Thr
435 440 445

Thr His Ala His Pro Ser Ile Ser Glu Val Leu Met Glu Leu Gly Leu
450 455 460

Lys Ala Glu Ser Arg Ala Ile His Val
465 470

<210> 74
<211> 1422
<212> DNA
<213> Staphylococcus aureus

<220>
<221> misc_feature
<223> Encodes BKD E3 component

<400> 74
ttatacgtga atagctctac tttctgcttt caatcctaat tccatcaaca cttcagagat 60

ggaaggatgt gcgtgtgttg ttagtcctaa ttctaatgcc gagccattca tgaactgtaa 120

cagtgatgcc tcattaatca attctgttac atgttgacca atcatattaa tacccacaat 180

ttcttcagtt gattgatcaa tcaccatttc gctataccct tcgtttgcgt catggctatc 240

aatcactgct ttaccaattg ctttaaatgg tactttaaaa cttttaactt tcattccctc 300

tgcctttgct tgttcaatgt ttaaaccgat agaagcaatt tcaggttgtg aataaataca 360

cttaggcatc atgttatagt ttactgggat tgggttcccc tcaaacatat gatcaacagc 420

cacaacacct tcttttgatc caacatgtgc caattgtaat tttcctatac aatcaccagc 480

tgcataaata tgtttatctt cagtttgttg aaattcgttc gttaaaatat gtcctgatgt 540

agaaagtttt attttagtgt tgtttaaacc aatatctgat gtgttaggtt ttctaccaat 600

cgatagcaac actttatcta ctttaattat gtctgaagaa atttcaaacg taacaccatc 660

ttcgttaaca tttatatcat tttcagaaag ttttattccc tcatagaatt taacaccacg 720

tgctgacaat gattttttta atagttgtga agcttgttta ctttcagttg gtaaaattct 780

ttcacctgct tctataactg ttacgtcaac acctaaatct atcatcaatg atgcaaattc 840

cattccgata acaccaccac caataatacc aatacttgat ggtaacgtct ttaatgataa 900

tatatcatcg ctagataaaa ttttatcatg atcaaatgat aagaatggca actctgcagg 960

cgaagaacca gttgcaatta atacaaattg gttgggtaat aagtctgatt caccatcttc 1020

atattcgaca gaaattgtgc cactttgagg tgaaaatata gatgtaccta gaatacgtcc 1080

cgtgccatta taaatgtcaa tgtgattgtg ttgcattaaa tgctttacac cttgatacat 1140

ttgattaata atgtcttctt ttcgtgccaa catattttca aaattaacat tagcatcttt 1200

gacatcaacg ccaaacattg ctgcctgttt tactgtttga aatacttcag cagatttaag 1260

cagcgattta gtaggaatac aacctttatg gagacaagta cctcctaata gttgtcgttc 1320

tactattgcc actttcttac ctaattgaga cgcacgtatc gcagcaacat atcctgcagt 1380

acctccaccg agaacgacta aatcatattg tttctctgac at 1422

<210> 75
<211> 581
<212> PRT
<213> Streptococcus mutans

<220>
<221> MISC_FEATURE
<223> BKD E3 component

<400> 75

Met Ala Val Glu Ile Ile Met Pro Lys Leu Gly Val Asp Met Gln Glu

```
1               5                   10                  15

Gly Glu Ile Ile Glu Trp Lys Lys Gln Glu Gly Asp Glu Val Lys Glu
            20                  25                  30

Gly Asp Ile Leu Leu Glu Ile Met Ser Asp Lys Thr Asn Met Glu Ile
        35                  40                  45

Glu Ala Glu Asp Ser Gly Val Leu Leu Lys Ile Val Lys Gly Asn Gly
    50                  55                  60

Gln Val Val Pro Val Thr Glu Val Ile Gly Tyr Ile Gly Ser Ala Gly
65                  70                  75                  80

Glu Thr Ile Glu Thr Asn Ala Ala Pro Ala Ala Ser Ala Asp Asp Leu
                85                  90                  95

Lys Ala Ala Gly Leu Glu Val Pro Asp Thr Leu Gly Glu Ser Ala Ala
            100                 105                 110

Pro Ala Ala Gln Lys Thr Pro Leu Ala Asp Asp Glu Tyr Asp Met Ile
        115                 120                 125

Val Val Gly Gly Gly Pro Ala Gly Tyr Tyr Ala Ala Ile Arg Gly Ala
    130                 135                 140

Gln Leu Gly Gly Lys Val Ala Ile Val Glu Lys Ser Glu Phe Gly Gly
145                 150                 155                 160

Thr Cys Leu Asn Lys Gly Cys Ile Pro Thr Lys Thr Tyr Leu Lys Asn
                165                 170                 175

Ala Glu Ile Leu Asp Gly Ile Lys Ile Ala Ala Gly Arg Gly Ile Asn
            180                 185                 190

Phe Ala Ser Thr Asn Tyr Thr Ile Asp Met Asp Lys Thr Val Ala Phe
        195                 200                 205

Lys Asp Thr Val Val Lys Thr Leu Thr Ser Gly Val Gln Gly Leu Leu
    210                 215                 220

Lys Ala Asn Lys Val Thr Ile Phe Asn Gly Leu Gly Gln Val Asn Pro
225                 230                 235                 240

Asp Lys Thr Val Thr Val Gly Ser Glu Thr Ile Lys Gly His Asn Ile
                245                 250                 255
```

```
Ile Leu Ala Thr Gly Ser Lys Val Ser Arg Ile Asn Ile Pro Gly Ile
            260                 265                 270

Asp Ser Pro Leu Val Leu Thr Ser Asp Asp Ile Leu Asp Leu Arg Glu
        275                 280                 285

Ile Pro Lys Ser Leu Ala Val Met Gly Gly Gly Val Val Gly Ile Glu
    290                 295                 300

Leu Gly Leu Val Tyr Ala Ser Tyr Gly Thr Glu Val Thr Val Ile Glu
305                 310                 315                 320

Met Ala Asp Arg Ile Ile Pro Ala Met Asp Lys Glu Val Ser Leu Glu
                325                 330                 335

Leu Gln Lys Ile Leu Ser Lys Lys Gly Met Asn Ile Lys Thr Ser Val
            340                 345                 350

Gly Val Ala Glu Ile Val Glu Ala Asn Asn Gln Leu Thr Leu Lys Leu
        355                 360                 365

Asn Asp Gly Ser Glu Val Val Ala Glu Lys Ala Leu Leu Ser Ile Gly
    370                 375                 380

Arg Val Pro Gln Leu Ser Gly Leu Glu Asn Leu Asn Leu Glu Leu Glu
385                 390                 395                 400

Arg Gly Arg Ile Lys Val Asp Asp Tyr Gln Glu Thr Ser Ile Ser Gly
                405                 410                 415

Ile Tyr Ala Pro Gly Asp Val Asn Gly Arg Lys Met Leu Ala His Ala
            420                 425                 430

Ala Tyr Arg Met Gly Glu Val Ala Ala Glu Asn Ala Ile Trp Gly Asn
        435                 440                 445

Val Arg Lys Ala Asn Leu Lys Tyr Thr Pro Ala Ala Val Tyr Thr His
    450                 455                 460

Pro Glu Val Ala Met Cys Gly Ile Thr Glu Glu Gln Ala Arg Gln Glu
465                 470                 475                 480

Tyr Gly Asn Val Leu Val Gly Lys Ser Ser Phe Ser Gly Asn Gly Arg
                485                 490                 495

Ala Ile Ala Ser Asn Glu Ala Gln Gly Phe Val Lys Val Val Ala Asp
            500                 505                 510
```

```
Ala Lys Tyr His Glu Ile Leu Gly Val His Ile Ile Gly Pro Ala Ala
        515                 520                 525

Ala Glu Met Ile Asn Glu Ala Ser Thr Ile Met Glu Asn Glu Leu Thr
    530                 535                 540

Val Asp Glu Leu Leu Arg Ser Ile His Gly His Pro Thr Phe Ser Glu
545                 550                 555                 560

Val Met Tyr Glu Ala Phe Ala Asp Val Leu Gly Glu Ala Ile His Asn
                565                 570                 575

Pro Pro Lys Arg Arg
            580


<210>  76
<211>  1746
<212>  DNA
<213>  Streptococcus mutans


<220>
<221>  misc_feature
<223>  Encodes BKD E3 component

<400>  76
atggcagtcg aaattattat gcctaaactc ggtgttgata tgcaggaagg cgaaatcatc      60

gagtggaaaa aacaagaagg tgatgaggtc aaagaagggg atatcctcct tgaaatcatg     120

tctgacaaga ccaatatgga aattgaagct gaggattcag gtgtcctgct caaaattgtt     180

aaaggaaatg gtcaagttgt ccctgtgact gaggtcattg gttatattgg ttctgctggt     240

gaaacgattg aaacaaatgc agcgccagca gcttcagctg atgatctcaa agcagcgggt     300

cttgaagttc ctgatacttt aggcgagtca gcagcaccag cagctcaaaa aactccgctt     360

gctgatgatg agtatgatat gattgtcgtt ggtggtggtc ctgctggtta ttatgctgct     420

attcgcggtg cacaattggg cggcaaggtt gctatcgtcg aaaaatcaga atttggaggg     480

acttgtttaa ataaaggctg cattccaact aaaacttatc ttaagaatgc tgaaatcctt     540

gatggcatca aaattgcagc gggtcgcggt attaattttg cttcaaccaa ctataccatt     600

gacatggaca aaacggttgc ctttaaagat accgttgtta aaacattgac aagtggggtt     660

cagggtcttc ttaaagccaa taaagtgact attttcaatg gtctcggtca ggttaatcct     720

gataagacag tgactgtcgg ttcggaaacg attaaaggac ataatattat ccttgcaaca     780

ggttcaaaag tgtctcgtat taatattccg ggaattgatt cacctcttgt tttaacatcg     840

gatgatattc ttgatcttcg tgaaattcca aagtcacttg ctgttatggg cggtggtgtt     900

gtcggcattg aactcggtct tgtttacgct tcctatggta cagaagtgac tgttattgaa     960
```

atggctgatc gcattattcc tgctatggac aaggaagtat cgcttgaact gcaaaaaatt 1020

ctatccaaga aaggaatgaa cattaagact tctgttggtg tggctgaaat tgttgaagct 1080

aacaatcaat taacgctgaa actcaatgac ggctctgaag ttgtggctga aaaggccctg 1140

ctttctattg gtcgtgtccc acaattaagc ggtttagaaa atcttaatct ggaacttgaa 1200

cgcggtcgca tcaaagtgga cgattatcag gaaacctcta tttcaggtat ttatgccccg 1260

ggtgatgtta atggaagaaa gatgttagcg catgctgcct atcgtatggg tgaagtagct 1320

gccgaaaatg ctatctgggg aaatgttcgt aaggctaacc tgaaatatac accagcagct 1380

gtttacaccc atccagaggt tgctatgtgc ggtattactg aagaacaagc ccgtcaagaa 1440

tatggaaacg tcttagttgg gaaatcctct tttcaggaa atggacgtgc gatcgcttct 1500

aatgaagcac aaggatttgt caaagttgtc gcagatgcta aataccatga aattcttgga 1560

gtccatatta ttggaccagc agctgctgag atgattaatg aagcctcaac gattatggaa 1620

aatgagttga cggttgatga gctgctacgt tctattcatg gccatcctac cttctcggag 1680

gttatgtatg aagcctttgc agacgtcctt ggcgaagcta tccataaccc gccaaagcgt 1740

cgttaa 1746

<210> 77
<211> 474
<212> PRT
<213> Escherichia coli

<220>
<221> MISC_FEATURE
<223> E3 component

<400> 77

Met Ser Thr Glu Ile Lys Thr Gln Val Val Val Leu Gly Ala Gly Pro
1 5 10 15

Ala Gly Tyr Ser Ala Ala Phe Arg Cys Ala Asp Leu Gly Leu Glu Thr
20 25 30

Val Ile Val Glu Arg Tyr Asn Thr Leu Gly Gly Val Cys Leu Asn Val
35 40 45

Gly Cys Ile Pro Ser Lys Ala Leu Leu His Val Ala Lys Val Ile Glu
50 55 60

Glu Ala Lys Ala Leu Ala Glu His Gly Ile Val Phe Gly Glu Pro Lys
65 70 75 80

Thr Asp Ile Asp Lys Ile Arg Thr Trp Lys Glu Lys Val Ile Asn Gln

85 90 95

Leu Thr Gly Gly Leu Ala Gly Met Ala Lys Gly Arg Lys Val Lys Val
100 105 110

Val Asn Gly Leu Gly Lys Phe Thr Gly Ala Asn Thr Leu Glu Val Glu
115 120 125

Gly Glu Asn Gly Lys Thr Val Ile Asn Phe Asp Asn Ala Ile Ile Ala
130 135 140

Ala Gly Ser Arg Pro Ile Gln Leu Pro Phe Ile Pro His Glu Asp Pro
145 150 155 160

Arg Ile Trp Asp Ser Thr Asp Ala Leu Glu Leu Lys Glu Val Pro Glu
165 170 175

Arg Leu Leu Val Met Gly Gly Gly Ile Ile Gly Leu Glu Met Gly Thr
180 185 190

Val Tyr His Ala Leu Gly Ser Gln Ile Asp Val Val Glu Met Phe Asp
195 200 205

Gln Val Ile Pro Ala Ala Asp Lys Asp Ile Val Lys Val Phe Thr Lys
210 215 220

Arg Ile Ser Lys Lys Phe Asn Leu Met Leu Glu Thr Lys Val Thr Ala
225 230 235 240

Val Glu Ala Lys Glu Asp Gly Ile Tyr Val Thr Met Glu Gly Lys Lys
245 250 255

Ala Pro Ala Glu Pro Gln Arg Tyr Asp Ala Val Leu Val Ala Ile Gly
260 265 270

Arg Val Pro Asn Gly Lys Asn Leu Asp Ala Gly Lys Ala Gly Val Glu
275 280 285

Val Asp Asp Arg Gly Phe Ile Arg Val Asp Lys Gln Leu Arg Thr Asn
290 295 300

Val Pro His Ile Phe Ala Ile Gly Asp Ile Val Gly Gln Pro Met Leu
305 310 315 320

Ala His Lys Gly Val His Glu Gly His Val Ala Ala Glu Val Ile Ala
325 330 335

Gly Lys Lys His Tyr Phe Asp Pro Lys Val Ile Pro Ser Ile Ala Tyr
340 345 350

Thr Glu Pro Glu Val Ala Trp Val Gly Leu Thr Glu Lys Glu Ala Lys
355 360 365

Glu Lys Gly Ile Ser Tyr Glu Thr Ala Thr Phe Pro Trp Ala Ala Ser
370 375 380

Gly Arg Ala Ile Ala Ser Asp Cys Ala Asp Gly Met Thr Lys Leu Ile
385 390 395 400

Phe Asp Lys Glu Ser His Arg Val Ile Gly Gly Ala Ile Val Gly Thr
405 410 415

Asn Gly Gly Glu Leu Leu Gly Glu Ile Gly Leu Ala Ile Glu Met Gly
420 425 430

Cys Asp Ala Glu Asp Ile Ala Leu Thr Ile His Ala His Pro Thr Leu
435 440 445

His Glu Ser Val Gly Leu Ala Ala Glu Val Phe Glu Gly Ser Ile Thr
450 455 460

Asp Leu Pro Asn Pro Lys Ala Lys Lys Lys
465 470

<210> 78
<211> 1425
<212> DNA
<213> Escherichia coli

<220>
<221> misc_feature
<223> Encodes E3 component

<400> 78
atgagtactg aaatcaaaac tcaggtcgtg gtacttgggg caggccccgc aggttactcc 60

gctgccttcc gttgcgctga tttaggtctg gaaaccgtaa tcgtagaacg ttacaacacc 120

cttggcggtg tttgcctgaa cgtcggctgt atcccttcta aagcactgct gcacgtagca 180

aaagttatcg aagaagccaa agcgctggct gaacacggta tcgtcttcgg cgaaccgaaa 240

accgatatcg acaagattcg tacctggaaa gagaaagtga tcaatcagct gaccggtggt 300

ctggctggta tggcgaaagg ccgcaaagtc aaagtggtca acggtctggg taaattcacc 360

ggggctaaca ccctggaagt tgaaggtgag aacggcaaaa ccgtgatcaa cttcgacaac 420

gcgatcattg cagcgggttc tcgcccgatc caactgccgt ttattccgca tgaagatccg 480

```
cgtatctggg actccactga cgcgctggaa ctgaaagaag taccagaacg cctgctggta      540

atgggtggcg gtatcatcgg tctggaaatg ggcaccgttt accacgcgct gggttcacag      600

attgacgtgg ttgaaatgtt cgaccaggtt atcccggcag ctgacaaaga catcgttaaa      660

gtcttcacca agcgtatcag caagaaattc aacctgatgc tggaaaccaa agttaccgcc      720

gttgaagcga aagaagacgg catttatgtg acgatggaag gcaaaaaagc acccgctgaa      780

ccgcagcgtt acgacgccgt gctggtagcg attggtcgtg tgccgaacgg taaaaacctc      840

gacgcaggca aagcaggcgt ggaagttgac gaccgtggtt tcatccgcgt tgacaaacag      900

ctgcgtacca acgtaccgca catctttgct atcggcgata tcgtcggtca accgatgctg      960

gcacacaaag gtgttcacga aggtcacgtt gccgctgaag ttatcgccgg taagaaacac     1020

tacttcgatc cgaaagttat cccgtccatc gcctataccg aaccagaagt tgcatgggtg     1080

ggtctgactg agaaagaagc gaaagagaaa ggcatcagct atgaaaccgc caccttcccg     1140

tgggctgctt ctggtcgtgc tatcgcttcc gactgcgcag acggtatgac caagctgatt     1200

ttcgacaaag aatctcaccg tgtgatcggt ggtgcgattg tcggtactaa cggcggcgag     1260

ctgctgggtg aaatcggcct ggcaatcgaa atgggttgtg atgctgaaga catcgcactg     1320

accatccacg cgcacccgac tctgcacgag tctgtgggcc tggcggcaga agtgttcgaa     1380

ggtagcatta ccgacctgcc gaacccgaaa gcgaagaaga agtaa                     1425
```

```
<210>  79
<211>  17
<212>  PRT
<213>  Artificial sequence

<220>
<223>  Synthetic polypeptide


<220>
<221>  MISC_FEATURE
<223>  BKD E3 motif

<220>
<221>  VARIANT
<222>  (1)..(1)
<223>  Xaa is Ile or Val

<220>
<221>  VARIANT
<222>  (4)..(4)
<223>  Xaa is Ala or Thr

<220>
<221>  VARIANT
<222>  (5)..(5)
<223>  Xaa is Ser or Cys

<220>
<221>  VARIANT
```

<222> (6)..(6)
<223> Xaa is Val or Leu

<220>
<221> VARIANT
<222> (7)..(8)
<223> Xaa is any amino acid

<220>
<221> VARIANT
<222> (9)..(9)
<223> Xaa is Gly or Asp

<220>
<221> VARIANT
<222> (11)..(11)
<223> Xaa is Val or Ile

<220>
<221> VARIANT
<222> (13)..(13)
<223> Xaa is Thr or Ser

<220>
<221> VARIANT
<222> (15)..(15)
<223> Xaa is Ala or Thr

<220>
<221> VARIANT
<222> (16)..(16)
<223> Xaa is Met or Leu

<220>
<221> VARIANT
<222> (17)..(17)
<223> Xaa is Ile or Leu

<400> 79

Xaa Gly Gly Xaa Xaa Xaa Xaa Xaa Xaa Cys Xaa Pro Xaa Lys Xaa Xaa
1               5                   10                  15

Xaa

<210> 80
<211> 17
<212> PRT
<213> Artificial sequence

<220>
<223> Synthetic polypeptide

<220>
<221> MISC_FEATURE
<223> BKD E3 motif

<220>
<221> VARIANT

<222> (1)..(1)
<223> Xaa is Leu or Ile

<220>
<221> VARIANT
<222> (5)..(5)
<223> Xaa is Gly or Ser

<220>
<221> VARIANT
<222> (6)..(6)
<223> Xaa is any amino acid

<220>
<221> VARIANT
<222> (7)..(7)
<223> Xaa is Ser or Pro

<220>
<221> VARIANT
<222> (8)..(9)
<223> Xaa is any amino acid

<220>
<221> VARIANT
<222> (11)..(11)
<223> Xaa is Ala or Pro

<220>
<221> VARIANT
<222> (12)..(12)
<223> Xaa is Asp or Gly

<220>
<221> VARIANT
<222> (13)..(15)
<223> Xaa is any amino acid

<220>
<221> VARIANT
<222> (16)..(16)
<223> Xaa is Asp or Leu

<400> 80

```
Xaa Ala Thr Gly Xaa Xaa Xaa Xaa Xaa Leu Xaa Xaa Xaa Xaa Xaa Xaa
1               5                   10                  15


Gly
```

<210> 81
<211> 12
<212> PRT
<213> Artificial sequence

<220>
<223> Synthetic polypeptide

<220>

<221> MISC_FEATURE
<223> BKD E3 motif

<220>
<221> VARIANT
<222> (1)..(1)
<223> Xaa is Val or Ile

<220>
<221> VARIANT
<222> (2)..(2)
<223> Xaa is any amino acid

<220>
<221> VARIANT
<222> (4)..(4)
<223> Xaa is Gly or Ser

<220>
<221> VARIANT
<222> (6)..(6)
<223> Xaa is any amino acid

<220>
<221> VARIANT
<222> (7)..(7)
<223> Xaa is Ile or Thr

<220>
<221> VARIANT
<222> (9)..(9)
<223> Xaa is any amino acid

<220>
<221> VARIANT
<222> (11)..(11)
<223> Xaa is any amino acid

<220>
<221> VARIANT
<222> (12)..(12)
<223> Xaa is Ala or Gly

<400> 81

```
Xaa Xaa Gly Xaa Gly Xaa Xaa Gly Xaa Glu Xaa Xaa
1               5                   10
```

<210> 82
<211> 14
<212> PRT
<213> Artificial sequence

<220>
<223> Synthetic polypeptide

<220>
<221> MISC_FEATURE
<223> BKD E3 motif

<220>

<221> VARIANT
<222> (2)..(7)
<223> Xaa is any amino acid

<220>
<221> VARIANT
<222> (8)..(8)
<223> Xaa is Ala or Val

<220>
<221> VARIANT
<222> (9)..(9)
<223> Xaa is any amino acid

<220>
<221> VARIANT
<222> (12)..(13)
<223> Xaa is any amino acid

<220>
<221> VARIANT
<222> (14)..(14)
<223> Xaa is Pro or Gly

<400> 82

```
Thr Xaa Xaa Xaa Xaa Xaa Xaa Xaa Xaa Gly Asp Xaa Xaa Xaa
1               5                   10
```

<210> 83
<211> 11
<212> PRT
<213> Artificial sequence

<220>
<223> Synthetic polypeptide

<220>
<221> MISC_FEATURE
<223> BKD E3 motif

<220>
<221> VARIANT
<222> (1)..(1)
<223> Xaa is Ile or Val

<220>
<221> VARIANT
<222> (2)..(2)
<223> Xaa is Gly or Ala

<220>
<221> VARIANT
<222> (3)..(4)
<223> Xaa is any amino acid

<220>
<221> VARIANT
<222> (5)..(5)
<223> Xaa is Phe or Ile

<220>
<221> VARIANT
<222> (6)..(6)
<223> Xaa is Thr or His

<220>
<221> VARIANT
<222> (7)..(7)
<223> Xaa is any amino acid

<220>
<221> VARIANT
<222> (8)..(8)
<223> Xaa is Tyr or His

<220>
<221> VARIANT
<222> (10)..(10)
<223> Xaa is Ser or Thr

<220>
<221> VARIANT
<222> (11)..(11)
<223> Xaa is Gln or Leu

<400> 83

```
Xaa Xaa Xaa Xaa Xaa Xaa Xaa Xaa Pro Xaa Xaa
1               5                   10
```

<210> 84
<211> 312
<212> PRT
<213> Bacillus subtilis

<220>
<221> MISC_FEATURE
<223> beta ketoacyl-ACP synthase III (FabH1)

<400> 84

```
Met Lys Ala Gly Ile Leu Gly Val Gly Arg Tyr Ile Pro Glu Lys Val
1               5                   10                  15

Leu Thr Asn His Asp Leu Glu Lys Met Val Glu Thr Ser Asp Glu Trp
            20                  25                  30

Ile Arg Thr Arg Thr Gly Ile Glu Glu Arg Arg Ile Ala Ala Asp Asp
        35                  40                  45

Val Phe Ser Ser His Met Ala Val Ala Ala Ala Lys Asn Ala Leu Glu
    50                  55                  60

Gln Ala Glu Val Ala Ala Glu Asp Leu Asp Met Ile Leu Val Ala Thr
65                  70                  75                  80
```

```
Val Thr Pro Asp Gln Ser Phe Pro Thr Val Ser Cys Met Ile Gln Glu
                85                  90                  95

Gln Leu Gly Ala Lys Lys Ala Cys Ala Met Asp Ile Ser Ala Ala Cys
            100                 105                 110

Ala Gly Phe Met Tyr Gly Val Val Thr Gly Lys Gln Phe Ile Glu Ser
        115                 120                 125

Gly Thr Tyr Lys His Val Leu Val Val Gly Val Glu Lys Leu Ser Ser
    130                 135                 140

Ile Thr Asp Trp Glu Asp Arg Asn Thr Ala Val Leu Phe Gly Asp Gly
145                 150                 155                 160

Ala Gly Ala Ala Val Val Gly Pro Val Ser Asp Asp Arg Gly Ile Leu
                165                 170                 175

Ser Phe Glu Leu Gly Ala Asp Gly Thr Gly Gly Gln His Leu Tyr Leu
            180                 185                 190

Asn Glu Lys Arg His Thr Ile Met Asn Gly Arg Glu Val Phe Lys Phe
        195                 200                 205

Ala Val Arg Gln Met Gly Glu Ser Cys Val Asn Val Ile Glu Lys Ala
    210                 215                 220

Gly Leu Ser Lys Glu Asp Val Asp Phe Leu Ile Pro His Gln Ala Asn
225                 230                 235                 240

Ile Arg Ile Met Glu Ala Ala Arg Glu Arg Leu Glu Leu Pro Val Glu
                245                 250                 255

Lys Met Ser Lys Thr Val His Lys Tyr Gly Asn Thr Ser Ala Ala Ser
            260                 265                 270

Ile Pro Ile Ser Leu Val Glu Glu Leu Glu Ala Gly Lys Ile Lys Asp
        275                 280                 285

Gly Asp Val Val Val Met Val Gly Phe Gly Gly Gly Leu Thr Trp Gly
    290                 295                 300

Ala Ile Ala Ile Arg Trp Gly Arg
305                 310
```

<210> 85
<211> 939
<212> DNA

<213> Bacillus subtilis

<220>
<221> misc_feature
<223> Encodes beta ketoacyl-ACP synthase III (FabH1)

<400> 85
atgaaagctg gaatacttgg tgttggacgt tacattcctg agaaggtttt aacaaatcat 60

gatcttgaaa aaatggttga aacttctgac gagtggattc gtacaagaac aggaatagaa 120

gaaagaagaa tcgcagcaga tgatgtgttt tcatcacata tggctgttgc agcagcgaaa 180

aatgcgctgg aacaagctga agtggctgct gaggatctgg atatgatctt ggttgcaact 240

gttacacctg atcagtcatt ccctacggtc tcttgtatga ttcaagaaca actcggcgcg 300

aagaaagcgt gtgctatgga tatcagcgcg gcttgtgcgg gcttcatgta cggggttgta 360

accggtaaac aatttattga atccggaacc tacaagcatg ttctagttgt tggtgtagag 420

aagctctcaa gcattaccga ctgggaagac cgcaatacag ccgttctgtt tggagacgga 480

gcaggcgctg cggtagtcgg gccagtcagt gatgacagag gaatcctttc atttgaacta 540

ggagccgacg gcacaggcgg tcagcacttg tatctgaatg aaaaacgaca tacaatcatg 600

aatggacgag aagttttcaa atttgcagtc cgccaaatgg gagaatcatg cgtaaatgtc 660

attgaaaaag ccggactttc aaaagaggat gtcgactttt tgattccgca tcaggcgaac 720

atccgtatca tggaagctgc tcgcgagcgt ttagagcttc ctgtcgaaaa gatgtctaaa 780

actgttcata aatatggaaa tacttctgcc gcatccattc cgatctctct tgtagaagaa 840

ttggaagccg gtaaaatcaa agacggcgat gtggtcgtta tggtagggtt cggcggagga 900

ctaacatggg gcgccattgc aatccgctgg ggccgataa 939

<210> 86
<211> 325
<212> PRT
<213> Bacillus subtilis

<220>
<221> MISC_FEATURE
<223> beta ketoacyl-ACP synthase III (FabH2)

<400> 86

Met Ser Lys Ala Lys Ile Thr Ala Ile Gly Thr Tyr Ala Pro Ser Arg
1               5                   10                  15

Arg Leu Thr Asn Ala Asp Leu Glu Lys Ile Val Asp Thr Ser Asp Glu
            20                  25                  30

Trp Ile Val Gln Arg Thr Gly Met Arg Glu Arg Arg Ile Ala Asp Glu
        35                  40                  45

His Gln Phe Thr Ser Asp Leu Cys Ile Glu Ala Val Lys Asn Leu Lys
50 55 60

Ser Arg Tyr Lys Gly Thr Leu Asp Asp Val Asp Met Ile Leu Val Ala
65 70 75 80

Thr Thr Thr Ser Asp Tyr Ala Phe Pro Ser Thr Ala Cys Arg Val Gln
85 90 95

Glu Tyr Phe Gly Trp Glu Ser Thr Gly Ala Leu Asp Ile Asn Ala Thr
100 105 110

Cys Ala Gly Leu Thr Tyr Gly Leu His Leu Ala Asn Gly Leu Ile Thr
115 120 125

Ser Gly Leu His Gln Lys Ile Leu Val Ile Ala Gly Glu Thr Leu Ser
130 135 140

Lys Val Thr Asp Tyr Thr Asp Arg Thr Thr Cys Val Leu Phe Gly Asp
145 150 155 160

Ala Ala Gly Ala Leu Leu Val Glu Arg Asp Glu Glu Thr Pro Gly Phe
165 170 175

Leu Ala Ser Val Gln Gly Thr Ser Gly Asn Gly Gly Asp Ile Leu Tyr
180 185 190

Arg Ala Gly Leu Arg Asn Glu Ile Asn Gly Val Gln Leu Val Gly Ser
195 200 205

Gly Lys Met Val Gln Asn Gly Arg Glu Val Tyr Lys Trp Ala Ala Arg
210 215 220

Thr Val Pro Gly Glu Phe Glu Arg Leu Leu His Lys Ala Gly Leu Ser
225 230 235 240

Ser Asp Asp Leu Asp Trp Phe Val Pro His Ser Ala Asn Leu Arg Met
245 250 255

Ile Glu Ser Ile Cys Glu Lys Thr Pro Phe Pro Ile Glu Lys Thr Leu
260 265 270

Thr Ser Val Glu His Tyr Gly Asn Thr Ser Ser Val Ser Ile Val Leu
275 280 285

Ala Leu Asp Leu Ala Val Lys Ala Gly Lys Leu Lys Lys Asp Gln Ile

290 295 300

Val Leu Leu Phe Gly Phe Gly Gly Gly Leu Thr Tyr Thr Gly Leu Leu
305 310 315 320

Ile Lys Trp Gly Met
325

<210> 87
<211> 978
<212> DNA
<213> Bacillus subtilis

<220>
<221> misc_feature
<223> Encodes beta ketoacyl-ACP synthase III

<400> 87
ttacatcccc catttaataa gcaatcctgt ataggttaat ccgccgccaa acccgaaaag 60

caaaacgatt tgatcttttt tcagcttccc ggctttcact gcgagatcga gcgccaaaac 120

aattgaaacc gaagacgtgt ttccgtagtg ctcaacacta gtgagcgttt tttcaatcgg 180

gaacggtgtt ttttcacaaa ttgactcgat catgcgcaag ttggcgctgt gaggaacaaa 240

ccaatcgaga tcatcggagc tgagtcctgc tttatgtaaa agccgttcaa attcgccagg 300

gacggttctt gcggcccatt tatatacctc gcgtccgttt tgcaccattt ttccggaacc 360

gacaagctgc acaccgttta tttcatttcg cagtccggca cgatacaaaa tatcgccgcc 420

gttcccgctt gttccttgta cagacgcaag aaatcccggc gtctcttcat ctcgttctac 480

taacagcgca cccgcggcat cgccgaacag tacgcatgtc gttcgatcgg tataatcggt 540

tacctttgat aacgtctctc cggcgatgac gagaattttt tgatgaaggc cagatgtgat 600

caatccattt gccaaatgga ggccgtatgt cagcccggcg catgtcgcat taatatccag 660

cgcgccggtg ctttcccagc cgaaatattc ctgtacgcgg catgccgtac tcggaaaggc 720

gtaatcggat gtggttgtgg caacgaggat catatcgaca tcatcaagcg ttcctttata 780

acggctcttg agattcttca ccgcttctat gcataaatca gaggtaaatt gatgttcatc 840

cgcaatccgg cgttctctca ttcctgtgcg ctgaacgatc cattcatcag aggtatcaac 900

gatcttttct aaatctgcat tggttaaacg tctgctcggc gcataggtgc cgatagctgt 960

aatttttgct tttgacat 978

<210> 88
<211> 313
<212> PRT
<213> Staphylococcus aureus

<220>
<221> MISC_FEATURE
<223> beta ketoacyl-ACP synthase III

<400> 88

Met Asn Val Gly Ile Lys Gly Phe Gly Ala Tyr Ala Pro Glu Lys Ile
1               5                   10                  15

Ile Asp Asn Ala Tyr Phe Glu Gln Phe Leu Asp Thr Ser Asp Glu Trp
            20                  25                  30

Ile Ser Lys Met Thr Gly Ile Lys Glu Arg His Trp Ala Asp Asp Asp
        35                  40                  45

Gln Asp Thr Ser Asp Leu Ala Tyr Glu Ala Ser Leu Lys Ala Ile Ala
    50                  55                  60

Asp Ala Gly Ile Gln Pro Glu Asp Ile Asp Met Ile Ile Val Ala Thr
65                  70                  75                  80

Ala Thr Gly Asp Met Pro Phe Pro Thr Val Ala Asn Met Leu Gln Glu
                85                  90                  95

Arg Leu Gly Thr Gly Lys Val Ala Ser Met Asp Gln Leu Ala Ala Cys
            100                 105                 110

Ser Gly Phe Met Tyr Ser Met Ile Thr Ala Lys Gln Tyr Val Gln Ser
        115                 120                 125

Gly Asp Tyr His Asn Ile Leu Val Val Gly Ala Asp Lys Leu Ser Lys
    130                 135                 140

Ile Thr Asp Leu Thr Asp Arg Ser Thr Ala Val Leu Phe Gly Asp Gly
145                 150                 155                 160

Ala Gly Ala Val Ile Ile Gly Glu Val Ser Asp Gly Arg Gly Ile Ile
                165                 170                 175

Ser Tyr Glu Met Gly Ser Asp Gly Thr Gly Gly Lys His Leu Tyr Leu
            180                 185                 190

Asp Lys Asp Thr Gly Lys Leu Lys Met Asn Gly Arg Glu Val Phe Lys
        195                 200                 205

Phe Ala Val Arg Ile Met Gly Asp Ala Ser Thr Arg Val Val Glu Lys
    210                 215                 220

Ala Asn Leu Thr Ser Asp Asp Ile Asp Leu Phe Ile Pro His Gln Ala

225 230 235 240

Asn Ile Arg Ile Met Glu Ser Ala Arg Glu Arg Leu Gly Ile Ser Lys
245 250 255

Asp Lys Met Ser Val Ser Val Asn Lys Tyr Gly Asn Thr Ser Ala Ala
260 265 270

Ser Ile Pro Leu Ser Ile Asp Gln Glu Leu Lys Asn Gly Lys Ile Lys
275 280 285

Asp Asp Asp Thr Ile Val Leu Val Gly Phe Gly Gly Gly Leu Thr Trp
290 295 300

Gly Ala Met Thr Ile Lys Trp Gly Lys
305 310

<210> 89
<211> 942
<212> DNA
<213> Staphylococcus aureus

<220>
<221> misc_feature
<223> Encodes beta ketoacyl-ACP synthase III

<400> 89
atgaacgtgg gtattaaagg ttttggtgca tatgcgccag aaaagattat tgacaatgcc 60

tattttgagc aatttttaga tacatctgat gaatggattt ctaagatgac tggaattaaa 120

gaaagacatt gggcagatga tgatcaagat acttcagatt tagcatatga agcaagttta 180

aaagcaatcg ctgacgctgg tattcagccc gaagatatag atatgataat tgttgccaca 240

gcaactggag atatgccatt tccaactgtc gcaaatatgt tgcaagaacg tttagggacg 300

ggcaaagttg cctctatgga tcaacttgca gcatgttctg gatttatgta ttcaatgatt 360

acagctaaac aatatgttca atctggagat tatcataaca ttttagttgt cggtgcagat 420

aaattatcta aaataacaga tttaactgac cgttctactg cagttctatt tggagatggt 480

gcaggtgcgg ttatcatcgg tgaagtttca gatggcagag gtattataag ttatgaaatg 540

ggttctgatg gcacaggtgg taaacattta tatttagata aagatactgg taaactgaaa 600

atgaatggtc gagaagtatt taaatttgct gttagaatta tgggtgatgc atcaacacgt 660

gtagttgaaa aagcgaattt aacatcagat gatatagatt tatttattcc tcatcaagct 720

aatattagaa ttatggaatc agctagagaa cgcttaggta tttcaaaaga caaaatgagt 780

gtttctgtaa ataaatatgg aaatacttca gctgcgtcaa taccttaag tatcgatcaa 840

gaattaaaaa atggtaaaat caaagatgat gatacaattg ttcttgtcgg attcggtggc 900

ggcctaactt ggggcgcaat gacaataaaa tggggaaaat ag 942

<210> 90
<211> 335
<212> PRT
<213> Streptomyces avermitilis

<220>
<221> MISC_FEATURE
<223> beta ketoacyl-ACP synthase III

<400> 90

Met Ser Gly Gly Arg Ala Ala Val Ile Thr Gly Ile Gly Gly Tyr Val
1 5 10 15

Pro Pro Asp Leu Val Thr Asn Asp Asp Leu Ala Gln Arg Leu Asp Thr
20 25 30

Ser Asp Ala Trp Ile Arg Ser Arg Thr Gly Ile Ala Glu Arg His Val
35 40 45

Ile Ala Pro Gly Thr Ala Thr Ser Asp Leu Ala Val Glu Ala Gly Leu
50 55 60

Arg Ala Leu Lys Ser Ala Gly Asp Glu His Val Asp Ala Val Val Leu
65 70 75 80

Ala Thr Thr Thr Pro Asp Gln Pro Cys Pro Ala Thr Ala Pro Gln Val
85 90 95

Ala Ala Arg Leu Gly Leu Gly Gln Val Pro Ala Phe Asp Val Ala Ala
100 105 110

Val Cys Ser Gly Phe Leu Phe Gly Leu Ala Thr Ala Ser Gly Leu Ile
115 120 125

Ala Ala Gly Val Ala Asp Lys Val Leu Leu Val Ala Ala Asp Ala Phe
130 135 140

Thr Thr Ile Ile Asn Pro Glu Asp Arg Thr Thr Ala Val Ile Phe Ala
145 150 155 160

Asp Gly Ala Gly Ala Val Val Leu Arg Ala Gly Ala Ala Asp Glu Pro
165 170 175

Gly Ala Val Gly Pro Leu Val Leu Gly Ser Asp Gly Glu Leu Ser His
180 185 190

Leu Ile Glu Val Pro Ala Gly Gly Ser Arg Gln Arg Ser Ser Gly Pro
195 200 205

Thr Thr Asp Pro Asp Asp Gln Tyr Phe Arg Met Leu Gly Arg Asp Thr
210 215 220

Tyr Arg His Ala Val Glu Arg Met Thr Asp Ala Ser Gln Arg Ala Ala
225 230 235 240

Glu Leu Ala Asp Trp Arg Ile Asp Asp Val Asp Arg Phe Ala Ala His
245 250 255

Gln Ala Asn Ala Arg Ile Leu Asp Ser Val Ala Glu Arg Leu Gly Val
260 265 270

Pro Ala Glu Arg Gln Leu Thr Asn Ile Ala Arg Val Gly Asn Thr Gly
275 280 285

Ala Ala Ser Ile Pro Leu Leu Leu Ser Gln Ala Ala Ala Ala Gly Arg
290 295 300

Leu Gly Ala Gly His Arg Val Leu Leu Thr Ala Phe Gly Gly Gly Leu
305 310 315 320

Ser Trp Gly Ala Gly Thr Leu Val Trp Pro Glu Val Gln Pro Val
325 330 335

<210> 91
<211> 1008
<212> DNA
<213> Streptomyces avermitilis

<220>
<221> misc_feature
<223> Encodes beta ketoacyl-ACP synthase III

<400> 91
atgagcggcg gacgcgcggc ggtgatcacc gggatcgggg gctatgtgcc tcccgatctg 60

gtgaccaacg acgatctggc ccagcggctc gacacctccg acgcgtggat ccgctcgcgc 120

accgggatcg ccgagcggca tgtgatcgcg cccggcaccg cgacctccga cctggcggtg 180

gaggccggac tgcgggccct gaagtcggcg ggcgacgagc acgtggacgc ggtcgtcctg 240

gccaccacga cgcccgacca gccctgcccg gcgaccgccc cgcaggtggc cgcacggctg 300

ggactcgggc aggtgccggc gttcgacgtg gccgccgtct gctccggctt cctgttcggc 360

ctcgccaccg cgtccgggct gatcgcggcc ggggtggcgg acaaggtcct gctggtcgcc 420

gccgacgcgt tcaccacgat catcaacccc gaggaccgca ccacggccgt catcttcgcg 480

gacggcgcgg gcgcggtggt gctgcgcgcg ggcgccgccg acgagccggg ggccgtcggc 540

ccgctggtgc tcggcagcga cggcgagctg agccatctca tcgaggtgcc ggcgggcggc 600

tcgcgccagc gctcgtccgg ccccacgacc gacccggacg accagtactt ccggatgctc 660

ggccgggaca cctaccggca cgcggtggag cggatgaccg atgcgtccca gcgggcggcc 720

gaactggccg actggcggat cgacgacgtc gaccggttcg cggcgcacca ggccaacgcc 780

cgcatcctcg actcggtcgc ggaacgtctc ggggtccccg ccgaacggca gttgaccaac 840

atcgcccggg tcggcaacac cggcgccgcc tcgatcccgc tgcttctgtc gcaggcggcc 900

gcggccggcc ggctcggcgc cgggcaccgg gtgctcctga ccgcgttcgg cgggggcctg 960

tcctggggcg cggggactct ggtctggccg gaggtccagc cggtctga 1008

<210> 92
<211> 325
<212> PRT
<213> Streptococcus mutans

<220>
<221> MISC_FEATURE
<223> beta ketoacyl-ACP synthase III

<400> 92

Met Thr Phe Ala Lys Ile Ser Gln Ala Ala Tyr Tyr Val Pro Ser Gln
1               5                   10                  15

Val Val Thr Asn Asp Asp Leu Ser Lys Ile Met Asp Thr Ser Asp Glu
            20                  25                  30

Trp Ile Thr Ser Arg Thr Gly Ile Arg Glu Arg Arg Ile Ser Gln Ser
        35                  40                  45

Glu Asp Thr Ser Asp Leu Ala Ser Gln Val Ala Lys Glu Leu Leu Lys
    50                  55                  60

Lys Ala Ser Leu Lys Ala Lys Glu Ile Asp Phe Ile Ile Val Ala Thr
65                  70                  75                  80

Ile Thr Pro Asp Ala Met Met Pro Ser Thr Ala Ala Cys Val Gln Ala
                85                  90                  95

Lys Ile Gly Ala Val Asn Ala Phe Ala Phe Asp Leu Thr Ala Ala Cys
            100                 105                 110

Ser Gly Phe Ile Phe Ala Leu Ser Ala Ala Glu Lys Met Ile Lys Ser
        115                 120                 125

```
Gly Gln Tyr Gln Lys Gly Leu Val Ile Gly Ala Glu Val Leu Ser Lys
    130                 135                 140

Ile Ile Asp Trp Ser Asp Arg Thr Thr Ala Val Leu Phe Gly Asp Gly
145                 150                 155                 160

Ala Gly Gly Val Leu Leu Glu Ala Asp Ser Ser Glu His Phe Leu Phe
                165                 170                 175

Glu Ser Ile His Ser Asp Gly Ser Arg Gly Glu Ser Leu Thr Ser Gly
            180                 185                 190

Glu His Ala Val Ser Ser Pro Phe Ser Gln Val Asp Lys Lys Asp Asn
        195                 200                 205

Cys Phe Leu Lys Met Asp Gly Arg Ala Ile Phe Asp Phe Ala Ile Arg
    210                 215                 220

Asp Val Ser Lys Ser Ile Ser Met Leu Ile Arg Lys Ser Asp Met Pro
225                 230                 235                 240

Val Glu Ala Ile Asp Tyr Phe Leu Leu His Gln Ala Asn Ile Arg Ile
                245                 250                 255

Leu Asp Lys Met Ala Lys Lys Ile Gly Ala Asp Arg Glu Lys Phe Pro
            260                 265                 270

Ala Asn Met Met Lys Tyr Gly Asn Thr Ser Ala Ala Ser Ile Pro Ile
        275                 280                 285

Leu Leu Ala Glu Cys Val Glu Asn Gly Thr Ile Glu Leu Asn Gly Ser
    290                 295                 300

His Thr Val Leu Leu Ser Gly Phe Gly Gly Gly Leu Thr Trp Gly Ser
305                 310                 315                 320

Leu Ile Val Lys Ile
                325


<210>  93
<211>  978
<212>  DNA
<213>  Streptococcus mutans


<220>
<221>  misc_feature
<223>  Encodes beta ketoacyl-ACP synthase III
```

<400> 93
ctaaatttta acaattaaac tgccccatgt caaaccccca ccgaacccgc tcaggagaac 60

agtgtgtgaa ccatttagct ctatagttcc attttcgaca cattcggcta ataaaatagg 120

aatacttgct gcactggtat taccatactt catcatatta gcaggaaatt tttctctatc 180

agcgccaatt tttttagcca ttttatccaa aatacgaata ttagcctgat gtaataagaa 240

ataatcaatc gcttctacag gcatatctga cttcctaatg agcatcgaaa tactttttga 300

cacatcacga atagcaaagt caaatatagc tcgaccatcc atttttagaa aacagttatc 360

tttttatca acctgtgaaa agggtgacga aacagcgtgt tcacctgatg tcaaactttc 420

accacgactg ccatctgaat gaatagattc aaataaaaaa tgttcagaag aatctgcttc 480

taaaagaaca ccgccagctc catctccaaa aagaacagct gttgttcgat ccgaccaatc 540

gatgatttta gatagaactt ctgcaccgat aactaaacct ttctggtact gaccggattt 600

aatcattttt tccgcagctg aaagtgcaaa aataaatcca ctgcaggcgg cagttaaatc 660

gaaagcaaaa gcattcactg caccaatttt cgcttggaca caagcagctg ttgatggcat 720

cattgcatcc ggagtaattg tagcaacaat aataaaatca atctctttcg cctttaatga 780

ggcttttttt aaaagttctt tggccacctg actggctaag tcactggtat cttcggattg 840

actaatacgg cgctctctta ttcccgtacg acttgtaatc cattcatcac tggtatccat 900

tattttagat aaatcatcat tggtgacaac ctgtgatggt acataatatg ctgcttgact 960

aatctttgca aaagtcat 978

<210> 94
<211> 325
<212> PRT
<213> Lactococcus lactis

<220>
<221> MISC_FEATURE
<223> beta ketoacyl-ACP synthase III

<400> 94

Met Thr Phe Ala Lys Ile Thr Gln Val Ala His Tyr Val Pro Glu Asn
1 5 10 15

Val Val Ser Asn Asp Asp Leu Ser Lys Ile Met Asp Thr Asn Asp Glu
20 25 30

Trp Ile Tyr Ser Arg Thr Gly Ile Lys Asn Arg His Ile Ser Thr Gly
35 40 45

Glu Asn Thr Ser Asp Leu Ala Ala Lys Val Ala Lys Gln Leu Ile Ser
50 55 60

Asp Ser Asn Leu Ser Pro Glu Thr Ile Asp Phe Ile Ile Val Ala Thr
65 70 75 80

Val Thr Pro Asp Ser Leu Met Pro Ser Thr Ala Ala Arg Val Gln Ala
85 90 95

Gln Val Gly Ala Val Asn Ala Phe Ala Tyr Asp Leu Thr Ala Ala Cys
100 105 110

Ser Gly Phe Val Phe Ala Leu Ser Thr Ala Glu Lys Leu Ile Ser Ser
115 120 125

Gly Ala Tyr Gln Arg Gly Leu Val Ile Gly Ala Glu Val Phe Ser Lys
130 135 140

Val Ile Asp Trp Ser Asp Arg Ser Thr Ala Val Leu Phe Gly Asp Gly
145 150 155 160

Ala Ala Gly Val Leu Ile Glu Ala Gly Ala Ser Gln Pro Leu Ile Ile
165 170 175

Ala Glu Lys Met Gln Thr Asp Gly Ser Arg Gly Asn Ser Leu Leu Ser
180 185 190

Ser Tyr Ala Asp Ile Gln Thr Pro Phe Ala Ser Val Ser Tyr Glu Ser
195 200 205

Ser Asn Leu Ser Met Glu Gly Arg Ala Ile Phe Asp Phe Ala Val Arg
210 215 220

Asp Val Pro Lys Asn Ile Gln Ala Thr Leu Glu Lys Ala Asn Leu Ser
225 230 235 240

Ala Glu Glu Val Asp Tyr Tyr Leu Leu His Gln Ala Asn Ser Arg Ile
245 250 255

Leu Asp Lys Met Ala Lys Lys Leu Gly Val Thr Arg Gln Lys Phe Leu
260 265 270

Gln Asn Met Gln Glu Tyr Gly Asn Thr Ser Ala Ala Ser Ile Pro Ile
275 280 285

Leu Leu Ser Glu Ser Val Lys Asn Gly Ile Phe Ser Leu Asp Gly Gln
290 295 300

Thr Lys Val Val Leu Thr Gly Phe Gly Gly Gly Leu Thr Trp Gly Thr
305 310 315 320

```
Ala Ile Ile Asn Leu
                325


<210>  95
<211>  978
<212>  DNA
<213>  Lactococcus lactis


<220>
<221>  misc_feature
<223>  Encodes beta ketoacyl-ACP synthase III

<400>  95
atgacttttg cgaaaattac gcaagtggca cactatgtgc ctgaaaatgt ggtatctaat      60

gatgacttgt ccaaaataat ggatactaat gatgaatgga tttacagtcg gacagggatt     120

aaaaatcgcc atatttcaac tggagagaac acctcagact tagcagctaa agttgctaag     180

cagttgatta gcgattcaaa tttaagccca gaaacgattg acttcatcat tgttgctaca     240

gtaactccgg actcattgat gccttcaacc gcggcacggg ttcaagctca agtaggagca     300

gttaatgctt ttgcttacga tttgactgcg gcttgttcag gctttgtctt tgctctatca     360

acagcggaaa aattaatttc ctcaggagca tatcaacgag ggcttgtcat tggcgcagaa     420

gtcttttcaa aagtaattga ttggtcagac cgatcaactg ctgttctttt cggagatgga     480

gctgctggtg tgcttattga agctggcgcg agtcaacctc tgattattgc tgaaaaaatg     540

caaacagatg gaagtcgtgg gaacagttta ctttctagtt atgctgacat ccaaactcca     600

tttgcctctg tttcatacga aagttcaaac ttgagtatgg aagggcgagc aatttttgat     660

tttgccgtac gtgatgttcc taaaaatatc caggcaactt tagaaaaagc taatttgtct     720

gctgaagaag tagattatta tctccttcat caagcgaatt caagaatcct tgataaaatg     780

gctaaaaagc ttggtgtgac gcgccaaaag ttccttcaaa atatgcaaga atatggtaac     840

acatcggcag caagtatccc tatattgttg tcagaatccg taaaaaatgg tatatttagt     900

ttggacggtc aaacaaaagt cgtcttgaca ggatttggcg gtggcctcac ttggggtaca     960

gcaattatta atttataa                                                   978


<210>  96
<211>  320
<212>  PRT
<213>  Streptomyces coelicolor


<220>
<221>  MISC_FEATURE
<223>  beta ketoacyl-ACP synthase III

<400>  96
```

```
Met Ala Arg Gly Ala Gly Arg Leu Thr Gly Ile Gly Val Tyr Arg Pro
1               5                   10                  15

Gly Gly Leu Leu Thr Ser Ala Glu Leu Asp Thr Arg Phe Gly His Glu
            20                  25                  30

Asp Gly Tyr Ile Glu Gln Ile Thr Gly Ile Arg Thr Arg Leu Lys Ala
        35                  40                  45

Asp Pro Asp Asp Thr Phe Val Glu Met Ala Ala Gln Ala Ala Asp Lys
    50                  55                  60

Ala Leu Ala His Ala Gly Val Leu Ala Glu Asp Leu Asp Cys Val Leu
65                  70                  75                  80

Phe Ser Ser Ala Ser Ser Val Gly Gln Ala Ser Cys Arg Ala Ala Ser
                85                  90                  95

Leu Thr His Arg Ile Gly Ala Gly Arg Ala Gly Gly Phe Asp Leu Asn
            100                 105                 110

Gly Gly Cys Ala Gly Phe Gly Tyr Gly Leu Thr Leu Ala Ser Gly Leu
        115                 120                 125

Ile Ala Ala Gln Gln Ala Arg Gln Ile Leu Val Val Ala Ala Glu Arg
    130                 135                 140

Leu Ser Asp Ile Thr Asp Pro Asp Asp Cys Gly Thr Val Met Val Phe
145                 150                 155                 160

Gly Asp Ala Ala Gly Ala Ala Val Val Ser Ala Ala Glu His Pro Gly
                165                 170                 175

Ile Gly Pro Ala Val Trp Gly Thr His Gly Pro Gly Glu Pro Trp Met
            180                 185                 190

Thr Ser Ala Pro Pro Lys Pro Gly Ala Ala Arg Pro Tyr Met His Met
        195                 200                 205

Asp Gly Thr Arg Val Val Arg Trp Phe Gly Ser Gln Met Pro Gln Val
    210                 215                 220

Ala Arg Asp Ala Leu Glu Ala Ala Gly Leu Thr Trp Asp Asp Ile Gly
225                 230                 235                 240

Ala Phe Val Pro His Gln Cys Asn Gly Arg Leu Ile Asp Ala Met Val
                245                 250                 255
```

Arg Arg Leu Arg Pro Pro Glu His Val Ala Ile Ala Arg Ser Ile Val
            260                 265                 270

Thr Asp Gly Asn Thr Ser Ser Ala Ser Ile Pro Leu Ala Leu Glu Ser
        275                 280                 285

Leu Leu Ala Ser Ala Thr Val Arg Pro Gly Asp Lys Ala Leu Leu Leu
    290                 295                 300

Gly Phe Gly Ala Gly Leu Thr Trp Cys Ala Gln Val Val Glu Leu Pro
305                 310                 315                 320

<210> 97
<211> 963
<212> DNA
<213> Streptomyces coelicolor

<220>
<221> misc_feature
<223> Encodes beta ketoacyl-ACP synthase III

<400> 97
ttacggaagt tcgaccacct gggcgcacca ggtcagcccg gcgccgaagc ccaggagcag 60

cgccttgtcg ccgggccgta cggtggccga ggcgagcagc gactccaggg ccagcgggat 120

ggacgccgaa ctggtgttgc cgtcggtgac gatgctgcgg gcgatcgcca cgtgctcggg 180

cgggcgcagt cggcgcacca tcgcgtcgat cagccggccg ttgcactggt gcggcacgaa 240

ggcgccgatg tcgtcccagg tgagcccggc cgcctcgagt gcgtcccggg ccacttgcgg 300

catctgcgaa ccgaaccagc ggaccacccg ggtgccgtcc atgtgcatgt acgggcgggc 360

ggcaccgggt ttgggcgggg cgctggtcat ccagggctca ccggggccgt gggttcccca 420

caccgccggt ccgatcccgg ggtgctcggc tgcgctgacc accgcggccc cggcggcatc 480

gccgaagacc atgacggtcc cgcagtcgtc ggggtcggtg atgtcgctca gccgctcggc 540

ggcgacgacc aggatctgac gcgcctgctg tgcggcgatg agtccggacg ccagggtcag 600

cccgtaaccg aacccggcgc acccgccgtt gaggtcgaac ccgccggccc ggccggcgcc 660

gatgcggtgg gtgagactgg cggcccggca cgacgcctgg ccgacgctgc tggcggagga 720

gaacaggacg cagtccaggt cctcggcgag cacgcccgcg tgggccaggg ccttgtccgc 780

cgcctgcgcg gccatctcga cgaaggtgtc gtcggggtcg gccttcagcc tggtccggat 840

tccggtgatc tgctcgatat agccgtcctc gtgaccgaac ctggtgtcga gttcggcgct 900

ggtgagcagg ccaccggggc ggtagacgcc gatgccggtc agccgccccg cgccccgggc 960

cat 963

<210> 98
<211> 312
<212> PRT
<213> Listeria monocytogenes

<220>
<221> MISC_FEATURE
<223> beta ketoacyl-ACP synthase III

<400> 98

```
Met Asn Ala Gly Ile Leu Gly Val Gly Lys Tyr Val Pro Glu Lys Ile
1               5                   10                  15

Val Thr Asn Phe Asp Leu Glu Lys Ile Met Asp Thr Ser Asp Glu Trp
            20                  25                  30

Ile Arg Thr Arg Thr Gly Ile Glu Glu Arg Arg Ile Ala Arg Asp Asp
        35                  40                  45

Glu Tyr Thr His Asp Leu Ala Tyr Glu Ala Ala Lys Val Ala Ile Glu
    50                  55                  60

Asn Ala Gly Leu Thr Pro Asp Asp Ile Asp Leu Phe Ile Val Ala Thr
65                  70                  75                  80

Val Thr Gln Glu Ala Thr Phe Pro Ser Val Ala Asn Ile Ile Gln Asp
                85                  90                  95

Arg Leu Gly Ala Thr Asn Ala Ala Gly Met Asp Val Glu Ala Ala Cys
            100                 105                 110

Ala Gly Phe Thr Phe Gly Val Val Thr Ala Ala Gln Phe Ile Lys Thr
        115                 120                 125

Gly Ala Tyr Lys Asn Ile Val Val Val Gly Ala Asp Lys Leu Ser Lys
    130                 135                 140

Ile Thr Asn Trp Asp Asp Arg Ala Thr Ala Val Leu Phe Gly Asp Gly
145                 150                 155                 160

Ala Gly Ala Val Val Met Gly Pro Val Ser Asp Asp His Gly Leu Leu
                165                 170                 175

Ser Phe Asp Leu Gly Ser Asp Gly Ser Gly Gly Lys Tyr Leu Asn Leu
            180                 185                 190

Asp Glu Asn Lys Lys Ile Tyr Met Asn Gly Arg Glu Val Phe Arg Phe
        195                 200                 205
```

Ala Val Arg Gln Met Gly Glu Ala Ser Leu Arg Val Leu Glu Arg Ala
210 215 220

Gly Leu Glu Lys Glu Glu Leu Asp Leu Leu Ile Pro His Gln Ala Asn
225 230 235 240

Ile Arg Ile Met Glu Ala Ser Arg Glu Arg Leu Asn Leu Pro Glu Glu
245 250 255

Lys Leu Met Lys Thr Val His Lys Tyr Gly Asn Thr Ser Ser Ser Ser
260 265 270

Ile Ala Leu Ala Leu Val Asp Ala Val Glu Glu Gly Arg Ile Lys Asp
275 280 285

Asn Asp Asn Val Leu Leu Val Gly Phe Gly Gly Gly Leu Thr Trp Gly
290 295 300

Ala Leu Ile Ile Arg Trp Gly Lys
305 310

<210> 99
<211> 939
<212> DNA
<213> Listeria monocytogenes

<220>
<221> misc_feature
<223> Encodes beta ketoacyl-ACP synthase III

<400> 99
atgaacgcag gaattttagg agtaggtaaa tacgtacctg aaaaaatagt aacaaatttt 60

gatttagaaa aaataatgga tacatccgat gagtggattc gtactcgaac tggtattgaa 120

gaaagaagaa ttgctcgtga tgacgaatat acgcacgact tagcatacga agcagcaaag 180

gtagctattg agaatgctgg gcttacacca gatgacattg acttatttat tgttgccact 240

gtgacgcagg aagcgacttt tccatccgtt gcgaatatta ttcaagaccg tttaggagca 300

acaaatgctg cgggtatgga cgtggaagcg gcatgtgccg gttttacttt tggcgtagta 360

actgcagcac aatttattaa aacaggggca tacaagaata tcgtcgtagt tggtgcggat 420

aaattatcta aaatcactaa ctgggatgat cgcgcaacag ccgtattatt tggtgatgga 480

gcgggagccg ttgttatggg tccggtttct gatgaccatg gactactttc gtttgactta 540

ggctcagatg gatctggcgg caaatacttg aacttagatg aaaataagaa gatttatatg 600

aatggacgtg aagtgttccg ttttgcagtt cgccaaatgg gagaagcttc gttacgagta 660

cttgaacgtg ctggacttga aaaagaagaa ttggatttac taattcctca ccaagcaaat 720

atccgtatca tggaagcttc tcgcgagcgt ttgaatttac cggaagaaaa actgatgaaa 780

acagtgcata aatacggtaa tacttcgtca tcttctattg ctcttgcgct agttgatgca 840

gtcgaagaag gacgcattaa agataatgac aatgtcctgc ttgttggctt tggcggcgga 900

ctaacatggg gcgccctaat cattcgttgg ggtaagtaa 939

<210> 100
<211> 312
<212> PRT
<213> Listeria monocytogenes W310G variant

<220>
<221> MISC_FEATURE
<223> L. monocytogenes beta ketoacyl-ACP synthase III W310G variant

<400> 100

Met Asn Ala Gly Ile Leu Gly Val Gly Lys Tyr Val Pro Glu Lys Ile
1 5 10 15

Val Thr Asn Phe Asp Leu Glu Lys Ile Met Asp Thr Ser Asp Glu Trp
20 25 30

Ile Arg Thr Arg Thr Gly Ile Glu Glu Arg Arg Ile Ala Arg Asp Asp
35 40 45

Glu Tyr Thr His Asp Leu Ala Tyr Glu Ala Ala Lys Val Ala Ile Glu
50 55 60

Asn Ala Gly Leu Thr Pro Asp Asp Ile Asp Leu Phe Ile Val Ala Thr
65 70 75 80

Val Thr Gln Glu Ala Thr Phe Pro Ser Val Ala Asn Ile Ile Gln Asp
85 90 95

Arg Leu Gly Ala Thr Asn Ala Ala Gly Met Asp Val Glu Ala Ala Cys
100 105 110

Ala Gly Phe Thr Phe Gly Val Val Thr Ala Ala Gln Phe Ile Lys Thr
115 120 125

Gly Ala Tyr Lys Asn Ile Val Val Val Gly Ala Asp Lys Leu Ser Lys
130 135 140

Ile Thr Asn Trp Asp Asp Arg Ala Thr Ala Val Leu Phe Gly Asp Gly
145 150 155 160

Ala Gly Ala Val Val Met Gly Pro Val Ser Asp Asp His Gly Leu Leu
165 170 175

Ser Phe Asp Leu Gly Ser Asp Gly Ser Gly Gly Lys Tyr Leu Asn Leu
180 185 190

Asp Glu Asn Lys Lys Ile Tyr Met Asn Gly Arg Glu Val Phe Arg Phe
195 200 205

Ala Val Arg Gln Met Gly Glu Ala Ser Leu Arg Val Leu Glu Arg Ala
210 215 220

Gly Leu Glu Lys Glu Glu Leu Asp Leu Leu Ile Pro His Gln Ala Asn
225 230 235 240

Ile Arg Ile Met Glu Ala Ser Arg Glu Arg Leu Asn Leu Pro Glu Glu
245 250 255

Lys Leu Met Lys Thr Val His Lys Tyr Gly Asn Thr Ser Ser Ser Ser
260 265 270

Ile Ala Leu Ala Leu Val Asp Ala Val Glu Glu Gly Arg Ile Lys Asp
275 280 285

Asn Asp Asn Val Leu Leu Val Gly Phe Gly Gly Gly Leu Thr Trp Gly
290 295 300

Ala Leu Ile Ile Arg Gly Gly Lys
305 310

<210> 101
<211> 939
<212> DNA
<213> Listeria monocytogenes W310G variant

<220>
<221> misc_feature
<223> Encodes L. monocytogenes beta ketoacyl-ACP synthase III W310G variant

<400> 101
atgaacgcag gaattttagg agtaggtaaa tacgtacctg aaaaaatagt aacaaatttt 60

gatttagaaa aaataatgga tacatccgat gagtggattc gtactcgaac tggtattgaa 120

gaaagaagaa ttgctcgtga tgacgaatat acgcacgact tagcatacga agcagcaaag 180

gtagctattg agaatgctgg gcttacacca gatgacattg acttatttat tgttgccact 240

gtgacgcagg aagcgacttt tccatccgtt gcgaatatta ttcaagaccg tttaggagca 300

acaaatgctg cgggtatgga cgtggaagcg gcatgtgccg gttttacttt tggcgtagta 360

```
actgcagcac aatttattaa aacaggggca tacaagaata tcgtcgtagt tggtgcggat      420

aaattatcta aaatcactaa ctgggatgat cgcgcaacag ccgtattatt tggtgatgga      480

gcgggagccg ttgttatggg tccggtttct gatgaccatg gactactttc gtttgactta      540

ggctcagatg gatctggcgg caaatacttg aacttagatg aaaataagaa gatttatatg      600

aatggacgtg aagtgttccg ttttgcagtt cgccaaatgg gagaagcttc gttacgagta      660

cttgaacgtg ctggacttga aaaagaagaa ttggatttac taattcctca ccaagcaaat      720

atccgtatca tggaagcttc tcgcgagcgt ttgaatttac cggaagaaaa actgatgaaa      780

acagtgcata aatacggtaa tacttcgtca tcttctattg ctcttgcgct agttgatgca      840

gtcgaagaag gacgcattaa agataatgac aatgtcctgc ttgttggctt tggcggcgga      900

ctaacatggg gcgccctaat cattcgtggg ggtaagtaa                             939


<210>  102
<211>  334
<212>  PRT
<213>  Bacteroides vulgatus


<220>
<221>  MISC_FEATURE
<223>  beta ketoacyl-ACP synthase III

<400>  102

Met Glu Lys Ile Asn Ala Val Ile Thr Gly Val Gly Gly Tyr Val Pro
1               5                   10                  15


Asp Tyr Val Leu Thr Asn Glu Glu Ile Ser Arg Met Val Asp Thr Asn
            20                  25                  30


Asp Glu Trp Ile Met Thr Arg Ile Gly Val Lys Glu Arg Arg Ile Leu
        35                  40                  45


Asn Glu Glu Gly Leu Gly Thr Ser Tyr Met Ala Arg Lys Ala Ala Lys
    50                  55                  60


Gln Leu Met Gln Lys Thr Ala Ser Asn Pro Asp Asp Ile Asp Ala Val
65                  70                  75                  80


Ile Val Ala Thr Thr Thr Pro Asp Tyr His Phe Pro Ser Thr Ala Ser
                85                  90                  95


Ile Leu Cys Asp Lys Leu Gly Leu Lys Asn Ala Phe Ala Phe Asp Leu
            100                 105                 110


Gln Ala Ala Cys Cys Gly Phe Leu Tyr Leu Met Glu Thr Ala Ala Ser
```

115 120 125

Leu Ile Ala Ser Gly Arg His Lys Lys Ile Ile Ile Val Gly Ala Asp
130 135 140

Lys Met Ser Ser Met Val Asn Tyr Gln Asp Arg Ala Thr Cys Pro Ile
145 150 155 160

Phe Gly Asp Gly Ala Ala Ala Cys Met Val Glu Ala Thr Thr Glu Asp
165 170 175

Tyr Gly Ile Met Asp Ser Ile Leu Arg Thr Asp Gly Lys Gly Leu Pro
180 185 190

Phe Leu His Met Lys Ala Gly Gly Ser Val Cys Pro Pro Ser Tyr Phe
195 200 205

Thr Val Asp His Lys Met His Tyr Leu Tyr Gln Glu Gly Arg Thr Val
210 215 220

Phe Lys Tyr Ala Val Ser Asn Met Ser Asp Ile Thr Ala Thr Ile Ala
225 230 235 240

Glu Lys Asn Gly Leu Asn Lys Asp Asn Ile Asp Trp Val Ile Pro His
245 250 255

Gln Ala Asn Leu Arg Ile Ile Asp Ala Val Ala Ser Arg Leu Glu Val
260 265 270

Pro Leu Glu Lys Val Met Ile Asn Ile Gln Arg Tyr Gly Asn Thr Ser
275 280 285

Gly Ala Thr Leu Pro Leu Cys Leu Trp Asp Tyr Glu Lys Gln Leu Lys
290 295 300

Lys Gly Asp Asn Leu Ile Phe Thr Ala Phe Gly Ala Gly Phe Thr Tyr
305 310 315 320

Gly Ala Val Tyr Val Lys Trp Gly Tyr Asp Gly Ser Lys Arg
325 330

<210> 103
<211> 1005
<212> DNA
<213> Bacteroides vulgatus

<220>
<221> misc_feature

<223> Encodes beta ketoacyl-ACP synthase III

<400> 103
ttatctctta ctaccatcgt aaccccattt cacataaacg gctccatagg taaaacctgc 60
gccgaaagct gtaaatatca ggttatctcc tttcttcagc tgcttttcgt aatcccaaag 120
acacaacgga agtgtagcac cactggtatt accatatcgc tgaatattaa tcattacctt 180
ttccaaggga acttccaagc gagaggctac cgcatcaata atacgcagat tagcctgatg 240
aggaattacc cagtcgatat tatctttatt caaaccattc ttttcggcaa tagtcgctgt 300
aatatccgac atattggaaa cagcatattt aaatactgtt cttccttcct gataaagata 360
atgcatctta tgatcaacag tgaaataaga aggaggacat acagaaccac cggctttcat 420
gtgaagaaaa ggaagtccct taccatctgt acgaagaata gaatccataa taccataatc 480
ttctgtagta gcttccacca tacatgctgc tgcaccatca ccaaagatag ggcaagttgc 540
acgatcctgg tagtttacca tagatgacat cttatctgca ccgacaataa taatcttttt 600
atgtcttccc gatgcgataa gtgaagcagc agtttccatt aaatacaaaa agccgcagca 660
ggcagcctgc aaatcaaatg caaatgcatt tttcaatccc agcttatcac acaggataga 720
agcagtggaa gggaaatgat agtcaggagt agtagttgct acgattactg catcaatgtc 780
atccggatta gaagctgttt tctgcatcag ttgtttggca gccttacgcg ccatatacga 840
tgtacctaat ccttcttcat tcagaatacg tctttcttta actccgattc gagtcataat 900
ccattcatca ttggtatcta ccattcttga aatctcttcg ttagtcaaga cataatctgg 960
tacatatcca ccgactcctg ttattactgc atttattttt tccat 1005

<210> 104
<211> 325
<212> PRT
<213> Clostridium acetobutylicum

<220>
<221> MISC_FEATURE
<223> beta ketoacyl-ACP synthase III

<400> 104

Met Asn Ser Val Glu Ile Ile Gly Thr Gly Ser Tyr Val Pro Glu Lys
1 5 10 15

Ile Val Thr Asn Glu Asp Met Ser Lys Ile Val Asp Thr Ser Asp Glu
20 25 30

Trp Ile Ser Ser Arg Thr Gly Ile Lys Glu Arg Arg Ile Ser Ile Asn
35 40 45

Glu Asn Thr Ser Asp Leu Gly Ala Lys Ala Ala Leu Arg Ala Ile Glu

50 55 60

Asp Ser Asn Ile Lys Pro Glu Glu Ile Asp Leu Ile Ile Val Ala Thr
65 70 75 80

Thr Ser Pro Asp Ser Tyr Thr Pro Ser Val Ala Cys Ile Val Gln Glu
85 90 95

Lys Ile Gly Ala Lys Asn Ala Ala Cys Phe Asp Leu Asn Ala Ala Cys
100 105 110

Thr Gly Phe Ile Phe Ala Leu Asn Thr Ala Ser Gln Phe Ile Lys Thr
115 120 125

Gly Glu Tyr Lys Thr Ala Leu Val Val Gly Thr Glu Val Leu Ser Lys
130 135 140

Ile Leu Asp Trp Gln Asp Arg Gly Thr Cys Val Leu Phe Gly Asp Gly
145 150 155 160

Ala Gly Ala Val Ile Ile Arg Gly Gly Asp Glu Asn Gly Ile Ile Lys
165 170 175

Ala Cys Leu Gly Ser Asp Gly Thr Gly Lys Asp Phe Leu His Cys Pro
180 185 190

Ala Thr Asn Val Ile Asn Pro Phe Ser Asp Glu Lys Gly Leu Ala Ser
195 200 205

Ser Lys Ile Ser Met Asn Gly Arg Glu Val Phe Lys Phe Ala Val Lys
210 215 220

Val Met Val Ser Ser Val Lys Lys Val Ile Glu Asp Ser Gly Leu Asn
225 230 235 240

Ile Glu Asp Ile Asp Tyr Ile Val Pro His Gln Ala Asn Ile Arg Ile
245 250 255

Ile Glu Phe Ala Ala Lys Lys Leu Gly Leu Ser Met Asp Lys Phe Phe
260 265 270

Ile Asn Leu Gln Asn Tyr Gly Asn Thr Ser Gly Ala Thr Ile Pro Leu
275 280 285

Ala Ile Asp Glu Met Asn Lys Lys Gly Leu Leu Lys Arg Gly Ala Lys
290 295 300

Ile Val Val Val Gly Phe Gly Gly Gly Leu Thr Trp Gly Ser Met Val
305 310 315 320

Leu Lys Trp Thr Lys
325

<210> 105
<211> 978
<212> DNA
<213> Clostridium acetobutylicum

<220>
<221> misc_feature
<223> Encodes beta ketoacyl-ACP synthase III

<400> 105
ttatttagtc catttaagaa ccatggaacc ccaagtaagt cctccaccaa aaccaactac 60

aactatttta gcacctcttt taagcaagcc ttttttattc atttcatcta ttgccagtgg 120

tatagtcgct ccagatgtat ttccatagtt ttgtaggttt ataaaaaatt tgtccatact 180

taatccaagt tttttagctg caaactctat tattctaatg ttagcctgat gaggtactat 240

ataatcaatg tcttctatat ttagtccact atcttctata acctttttaa ctgagcttac 300

cattacctta actgcaaatt taaagacttc tcttccattc atagaaatct tactgcttgc 360

taaacctttt tcatccgaaa atggatttat cacattagtc gctggacaat gcaagaagtc 420

ttttcccgta ccatctgaac caagacatgc tttaataatt ccgttttcat ctccgcctct 480

tataattacc gcacctgcac catctccaaa aagtacacat gtacctctat cttgccaatc 540

aagtatcttt gatagtacct ctgttcctac tacaagagct gttttatact ctcctgtttt 600

tataaactga gatgccgtat taagagcaaa tataaatcca gtacatgccg cattcaaatc 660

aaaacaggca gcatttttgg cacctatctt ctcctgaaca atacaagcta cggatggagt 720

atatgagtct ggacttgtag ttgcaactat tattaaatct atttcttctg gttttatgtt 780

tgagtcctct attgccctta aggcagcttt agcacctaaa tctgatgtat tttcgtttat 840

agatattctt ctttccttta tacctgttct tgatgatatc cactcatcac tagtatcaac 900

tatcttagac atatcttcat tagtaactat tttttctggg acatagcttc cagtccctat 960

aatctcaaca ctattcac 978

<210> 106
<211> 332
<212> PRT
<213> Flavobacterium johnsoniae

<220>
<221> MISC_FEATURE
<223> beta ketoacyl-ACP synthase III

<400> 106

```
Met Asn Thr Ile Thr Ala Ala Ile Thr Ala Val Gly Gly Tyr Val Pro
1               5                   10                  15

Asp Phe Val Leu Ser Asn Lys Val Leu Glu Thr Met Val Asp Thr Asn
            20                  25                  30

Asp Glu Trp Ile Thr Thr Arg Thr Gly Ile Lys Glu Arg Arg Ile Leu
        35                  40                  45

Lys Asp Ala Asp Lys Gly Thr Ser Tyr Leu Ala Ile Gln Ala Ala Gln
    50                  55                  60

Asp Leu Ile Ala Lys Ala Asn Ile Asp Pro Leu Glu Ile Asp Met Val
65                  70                  75                  80

Ile Met Ala Thr Ala Thr Pro Asp Met Met Val Ala Ser Thr Gly Val
                85                  90                  95

Tyr Val Ala Thr Glu Ile Gly Ala Val Asn Ala Phe Ala Tyr Asp Leu
            100                 105                 110

Gln Ala Ala Cys Ser Ser Phe Leu Tyr Gly Met Ser Thr Ala Ala Ala
        115                 120                 125

Tyr Val Gln Ser Gly Arg Tyr Lys Lys Val Leu Leu Ile Gly Ala Asp
    130                 135                 140

Lys Met Ser Ser Ile Val Asp Tyr Thr Asp Arg Ala Thr Cys Ile Ile
145                 150                 155                 160

Phe Gly Asp Gly Ala Gly Ala Val Leu Phe Glu Pro Asn Tyr Glu Gly
                165                 170                 175

Leu Gly Leu Gln Asp Glu Tyr Leu Arg Ser Asp Gly Val Gly Arg Asp
            180                 185                 190

Phe Leu Lys Ile Pro Ala Gly Gly Ser Leu Ile Pro Ala Ser Glu Asp
        195                 200                 205

Thr Val Lys Asn Arg Gln His Asn Ile Met Gln Asp Gly Lys Thr Val
    210                 215                 220

Phe Lys Tyr Ala Val Thr Asn Met Ala Asp Ala Ser Glu Leu Ile Leu
225                 230                 235                 240
```

Gln Arg Asn Asn Leu Thr Asn Gln Asp Val Asp Trp Leu Val Pro His
245 250 255

Gln Ala Asn Lys Arg Ile Ile Asp Ala Thr Ala Gly Arg Leu Glu Leu
260 265 270

Glu Glu Ser Lys Val Leu Val Asn Ile Glu Arg Tyr Gly Asn Thr Thr
275 280 285

Ser Gly Thr Leu Pro Leu Val Leu Ser Asp Phe Glu Asn Gln Phe Lys
290 295 300

Lys Gly Asp Asn Ile Ile Leu Ala Ala Phe Gly Gly Gly Phe Thr Trp
305 310 315 320

Gly Ser Ile Tyr Leu Lys Trp Ala Tyr Asp Lys Lys
325 330

<210> 107
<211> 999
<212> DNA
<213> Flavobacterium johnsoniae

<220>
<221> misc_feature
<223> Encodes beta ketoacyl-ACP synthase III

<400> 107
ttatttctta tcgtaagccc attttaggta aatagatccc caagtgaatc cacctccaaa 60

tgctgctaaa ataatattat ctcctttttt gaattgattt tcaaaatcgc ttaataccaa 120

aggtaatgtt cctgaagttg tattaccata tctttcgata ttaactagta ctttagactc 180

ttctaactct agtcttcctg cagttgcatc gatgatgcgt ttgtttgcct ggtgaggcac 240

taaccaatca acatcctgat tagttaaatt gtttctttgc aagattagtt cgctggcatc 300

agccatattg gttacagcat atttaaaaac tgttttacca tcctgcataa tattgtgttg 360

tctgtttttt acagtatctt ctgaagctgg aattaaagat cctccagctg gtattttaag 420

aaaatcgcgt cctacaccat cacttcttaa atattcgtct tgcagaccaa gaccttcgta 480

atttggctca aacaaaactg cccctgctcc atcaccaaaa ataatacaag ttgctctgtc 540

tgtgtaatct acaattgatg acattttatc ggcaccaatt aaaagaactt ttttatatct 600

tccagattgt acataagccg cagcagtaga cattccgtat aagaaacttg aacaagctgc 660

ctgcaaatcg tatgcaaatg cattaacagc tccaatttct gttgcaacat aaactcctgt 720

tgaagctacc atcatatctg gtgttgcagt tgccataata accatatcaa tttcaagagg 780

atcaatatta gcttttgcta ttaaatcctg tgctgcttgt atggcaaggt aagatgtacc 840

tttatcagca tctttaagaa ttcttctttc tttaattcct gtacgagtgg taatccattc 900

gtcattggta tctaccattg tttccaacac tttgtttgaa agcacaaagt ctggaacgta 960

gcctccaaca gcggtaattg cggctgtgat tgtattcat 999

<210> 108
<211> 350
<212> PRT
<213> Micrococcus luteus

<220>
<221> MISC_FEATURE
<223> beta ketoacyl-ACP synthase III

<400> 108

Met Thr Val Thr Leu Lys Gln His Glu Arg Pro Ala Ala Ser Arg Ile
1 5 10 15

Val Ala Val Gly Ala Tyr Arg Pro Ala Asn Leu Val Pro Asn Glu Asp
20 25 30

Leu Ile Gly Pro Ile Asp Ser Ser Asp Glu Trp Ile Arg Gln Arg Thr
35 40 45

Gly Ile Val Thr Arg Gln Arg Ala Thr Ala Glu Glu Thr Val Pro Val
50 55 60

Met Ala Val Gly Ala Ala Arg Glu Ala Leu Glu Arg Ala Gly Leu Gln
65 70 75 80

Gly Ser Asp Leu Asp Ala Val Ile Val Ser Thr Val Thr Phe Pro His
85 90 95

Ala Thr Pro Ser Ala Ala Ala Leu Val Ala His Glu Ile Gly Ala Thr
100 105 110

Pro Ala Pro Ala Tyr Asp Val Ser Ala Ala Cys Ala Gly Tyr Cys Tyr
115 120 125

Gly Val Ala Gln Ala Asp Ala Leu Val Arg Ser Gly Thr Ala Arg His
130 135 140

Val Leu Val Val Gly Val Glu Arg Leu Ser Asp Val Val Asp Pro Thr
145 150 155 160

Asp Arg Ser Ile Ser Phe Leu Leu Gly Asp Gly Ala Gly Ala Val Ile
165 170 175

Val Ala Ala Ser Asp Glu Pro Gly Ile Ser Pro Ser Val Trp Gly Ser
180 185 190

Asp Gly Glu Arg Trp Ser Thr Ile Ser Met Thr His Ser Gln Leu Glu
195 200 205

Leu Arg Asp Ala Val Glu His Ala Arg Thr Thr Gly Asp Ala Ser Ala
210 215 220

Ile Thr Gly Ala Glu Gly Met Leu Trp Pro Thr Leu Arg Gln Asp Gly
225 230 235 240

Pro Ser Val Phe Arg Trp Ala Val Trp Ser Met Ala Lys Val Ala Arg
245 250 255

Glu Ala Leu Asp Ala Ala Gly Val Glu Pro Glu Asp Leu Ala Ala Phe
260 265 270

Ile Pro His Gln Ala Asn Met Arg Ile Ile Asp Glu Phe Ala Lys Gln
275 280 285

Leu Lys Leu Pro Glu Ser Val Val Val Ala Arg Asp Ile Ala Asp Ala
290 295 300

Gly Asn Thr Ser Ala Ala Ser Ile Pro Leu Ala Met His Arg Leu Leu
305 310 315 320

Glu Glu Asn Pro Glu Leu Ser Gly Gly Leu Ala Leu Gln Ile Gly Phe
325 330 335

Gly Ala Gly Leu Val Tyr Gly Ala Gln Val Val Arg Leu Pro
340 345 350

<210> 109
<211> 1053
<212> DNA
<213> Micrococcus luteus

<220>
<221> misc_feature
<223> Encodes beta ketoacyl-ACP synthase III

<400> 109
tcaggggagg cggaccacct gggcgccgta gaccagcccg gcaccgaagc cgatctgcag 60

ggccagaccg ccggagagct cggggttctc ctccagcagc cggtgcatgg ccagcgggat 120

ggacgcggcc gacgtgttgc cggcgtccgc gatgtcccgg gccacgacga cggactccgg 180

cagcttcagc tgcttggcga actcgtcgat gatccgcatg ttggcctggt gcgggatgaa 240

```
cgcggcgagg tcctcgggct ccacgcccgc ggcgtcaagg gcctcgcggg ccaccttcgc      300
catcgaccac acggcccaac ggaagacgga gggcccgtcc tggcgcagcg tgggccagag      360
catcccctct gcgccggtga tcgccgaggc gtcgcccgtg gtgcgggcgt gctccacggc      420
atcgcgcagc tccagctgcg agtgcgtcat ggagatcgtg gaccagcgct ccccgtccga      480
gccccacacc gagggggaga tgcccggctc gtccgaggcc gcgacgatca cggcgcccgc      540
gccgtcgccc agcaggaagg agatggagcg gtccgtggga tccacgacgt cggagaggcg      600
ctcgacgccg accacgagca cgtgccgcgc ggtgccggag cgcacgagcg cgtcggcctg      660
ggccacgccg tagcagtagc cggcgcacgc ggcggagacg tcgtaggcgg gcgccggggt      720
ggcgccgatc tcgtgcgcca cgagggccgc ggccgagggg gtggcgtgcg ggaaggtgac      780
ggtcgagacg atcacggcgt ccaggtccga gccctgcagg ccggcccgct cgagggcctc      840
ccgggcggcg cccacggcca tgacgggcac ggtctcctcc gccgtggcgc gctggcgtgt      900
gacgatgccg gtgcgctggc ggatccactc gtccgacgag tcgatggggc cgatgaggtc      960
ctcgttcggg accaggttcg ccgggcggta ggcgcccacg gccacgatgc ggctggccgc     1020
ggggcgctcg tgctgcttca gggtgacggt cat                                  1053
```

<210>  110
<211>  17
<212>  PRT
<213>  Artificial sequence

<220>
<223>  Synthetic polypeptide

<220>
<221>  MISC_FEATURE
<223>  FabH motif

<220>
<221>  VARIANT
<222>  (3)..(3)
<223>  Xaa is Asn or Ser

<220>
<221>  VARIANT
<222>  (5)..(5)
<223>  Xaa is Ala or Glu

<220>
<221>  VARIANT
<222>  (8)..(9)
<223>  Xaa is any amino acid

<220>
<221>  VARIANT
<222>  (10)..(10)
<223>  Xaa is Met or Arg

<220>

<221> VARIANT
<222> (14)..(14)
<223> Xaa is any amino acid

<220>
<221> VARIANT
<222> (15)..(15)
<223> Xaa is Asn or Glu

<220>
<221> VARIANT
<222> (17)..(17)
<223> Xaa is Arg or His

<400> 110

Asp Thr Xaa Asp Xaa Trp Ile Xaa Xaa Xaa Thr Gly Ile Xaa Xaa Arg
1               5                   10                  15

Xaa

<210> 111
<211> 18
<212> PRT
<213> Artificial sequence

<220>
<223> Synthetic polypeptide

<220>
<221> MISC_FEATURE
<223> FabH motif

<220>
<221> VARIANT
<222> (1)..(1)
<223> Xaa is Ser or Ala

<220>
<221> VARIANT
<222> (2)..(2)
<223> Xaa is any amino acid

<220>
<221> VARIANT
<222> (4)..(5)
<223> Xaa is any amino acid

<220>
<221> VARIANT
<222> (7)..(7)
<223> Xaa is Ala or Val

<220>
<221> VARIANT
<222> (9)..(9)
<223> Xaa is Ala or Ser

<220>

<221> VARIANT
<222> (12)..(14)
<223> Xaa is any amino acid

<220>
<221> VARIANT
<222> (15)..(15)
<223> Xaa is Met or Leu

<220>
<221> VARIANT
<222> (16)..(17)
<223> Xaa is any amino acid

<400> 111

Xaa Xaa Asp Xaa Xaa Ala Xaa Cys Xaa Gly Phe Xaa Xaa Xaa Xaa Xaa
1 5 10 15

Xaa Ala

<210> 112
<211> 15
<212> PRT
<213> Artificial sequence

<220>
<223> Synthetic polypeptide

<220>
<221> MISC_FEATURE
<223> FabH motif

<220>
<221> VARIANT
<222> (3)..(3)
<223> Xaa is any amino acid

<220>
<221> VARIANT
<222> (5)..(5)
<223> Xaa is Ala or Ile

<220>
<221> VARIANT
<222> (6)..(6)
<223> Xaa is Ile or Val

<220>
<221> VARIANT
<222> (7)..(7)
<223> Xaa is any amino acid

<220>
<221> VARIANT
<222> (9)..(9)
<223> Xaa is Ala or Gly

<220>

<221> VARIANT
<222> (13)..(13)
<223> Xaa is Ala or Gly

<220>
<221> VARIANT
<222> (14)..(14)
<223> Xaa is Gly or Ala

<220>
<221> VARIANT
<222> (15)..(15)
<223> Xaa is Ala or Val

<400> 112

Asp Arg Xaa Thr Xaa Xaa Xaa Phe Xaa Asp Gly Ala Xaa Xaa Xaa
1 5 10 15

<210> 113
<211> 8
<212> PRT
<213> Artificial sequence

<220>
<223> Synthetic polypeptide

<220>
<221> MISC_FEATURE
<223> FabH motif

<220>
<221> VARIANT
<222> (5)..(5)
<223> Xaa is any amino acid

<220>
<221> VARIANT
<222> (8)..(8)
<223> Xaa is Met or Leu

<400> 113

His Gln Ala Asn Xaa Arg Ile Xaa
1 5

<210> 114
<211> 19
<212> PRT
<213> Artificial sequence

<220>
<223> Synthetic polypeptide

<220>
<221> MISC_FEATURE
<223> FabH motif

<220>

<221> VARIANT
<222> (4)..(4)
<223> Xaa is Gly or Ser

<220>
<221> VARIANT
<222> (8)..(8)
<223> Xaa is Val or Ile

<220>
<221> VARIANT
<222> (10)..(11)
<223> Xaa is any amino acid

<220>
<221> VARIANT
<222> (12)..(12)
<223> Xaa is Ile or Leu

<220>
<221> VARIANT
<222> (13)..(18)
<223> Xaa is any amino acid

<400> 114

Gly Asn Thr Xaa Ala Ala Ser Xaa Pro Xaa Xaa Xaa Xaa Xaa Xaa Xaa
1               5                   10                  15

Xaa Xaa Gly

<210> 115
<211> 13
<212> PRT
<213> Artificial sequence

<220>
<223> Synthetic polypeptide

<220>
<221> MISC_FEATURE
<223> FabH motif

<220>
<221> VARIANT
<222> (1)..(1)
<223> Xaa is Ile or Val

<220>
<221> VARIANT
<222> (2)..(2)
<223> Xaa is any amino acid

<220>
<221> VARIANT
<222> (4)..(5)
<223> Xaa is any amino acid

<220>

<221> VARIANT
<222> (10)..(10)
<223> Xaa is Leu or Phe

<220>
<221> VARIANT
<222> (11)..(11)
<223> Xaa is Thr or Ser

<400> 115

Xaa Xaa Leu Xaa Xaa Phe Gly Gly Gly Xaa Xaa Trp Gly
1 5 10

<210> 116
<211> 820
<212> PRT
<213> Escherichia coli

<220>
<221> MISC_FEATURE
<223> aspartokinase I and homoserine dehydrogenase I (EcThrA)

<400> 116

Met Arg Val Leu Lys Phe Gly Gly Thr Ser Val Ala Asn Ala Glu Arg
1 5 10 15

Phe Leu Arg Val Ala Asp Ile Leu Glu Ser Asn Ala Arg Gln Gly Gln
20 25 30

Val Ala Thr Val Leu Ser Ala Pro Ala Lys Ile Thr Asn His Leu Val
35 40 45

Ala Met Ile Glu Lys Thr Ile Ser Gly Gln Asp Ala Leu Pro Asn Ile
50 55 60

Ser Asp Ala Glu Arg Ile Phe Ala Glu Leu Leu Thr Gly Leu Ala Ala
65 70 75 80

Ala Gln Pro Gly Phe Pro Leu Ala Gln Leu Lys Thr Phe Val Asp Gln
85 90 95

Glu Phe Ala Gln Ile Lys His Val Leu His Gly Ile Ser Leu Leu Gly
100 105 110

Gln Cys Pro Asp Ser Ile Asn Ala Ala Leu Ile Cys Arg Gly Glu Lys
115 120 125

Met Ser Ile Ala Ile Met Ala Gly Val Leu Glu Ala Arg Gly His Asn
130 135 140

Val Thr Val Ile Asp Pro Val Glu Lys Leu Leu Ala Val Gly His Tyr
145 150 155 160

Leu Glu Ser Thr Val Asp Ile Ala Glu Ser Thr Arg Arg Ile Ala Ala
165 170 175

Ser Arg Ile Pro Ala Asp His Met Val Leu Met Ala Gly Phe Thr Ala
180 185 190

Gly Asn Glu Lys Gly Glu Leu Val Val Leu Gly Arg Asn Gly Ser Asp
195 200 205

Tyr Ser Ala Ala Val Leu Ala Ala Cys Leu Arg Ala Asp Cys Cys Glu
210 215 220

Ile Trp Thr Asp Val Asp Gly Val Tyr Thr Cys Asp Pro Arg Gln Val
225 230 235 240

Pro Asp Ala Arg Leu Leu Lys Ser Met Ser Tyr Gln Glu Ala Met Glu
245 250 255

Leu Ser Tyr Phe Gly Ala Lys Val Leu His Pro Arg Thr Ile Thr Pro
260 265 270

Ile Ala Gln Phe Gln Ile Pro Cys Leu Ile Lys Asn Thr Gly Asn Pro
275 280 285

Gln Ala Pro Gly Thr Leu Ile Gly Ala Ser Arg Asp Glu Asp Glu Leu
290 295 300

Pro Val Lys Gly Ile Ser Asn Leu Asn Asn Met Ala Met Phe Ser Val
305 310 315 320

Ser Gly Pro Gly Met Lys Gly Met Val Gly Met Ala Ala Arg Val Phe
325 330 335

Ala Ala Met Ser Arg Ala Arg Ile Ser Val Val Leu Ile Thr Gln Ser
340 345 350

Ser Ser Glu Tyr Ser Ile Ser Phe Cys Val Pro Gln Ser Asp Cys Val
355 360 365

Arg Ala Glu Arg Ala Met Gln Glu Glu Phe Tyr Leu Glu Leu Lys Glu
370 375 380

Gly Leu Leu Glu Pro Leu Ala Val Thr Glu Arg Leu Ala Ile Ile Ser
385 390 395 400

```
Val Val Gly Asp Gly Met Arg Thr Leu Arg Gly Ile Ser Ala Lys Phe
                405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
        435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655
```

Thr Thr Leu Ala Arg Glu Met Gly Tyr Thr Glu Pro Asp Pro Arg Asp
660 665 670

Asp Leu Ser Gly Met Asp Val Ala Arg Lys Leu Leu Ile Leu Ala Arg
675 680 685

Glu Thr Gly Arg Glu Leu Glu Leu Ala Asp Ile Glu Ile Glu Pro Val
690 695 700

Leu Pro Ala Glu Phe Asn Ala Glu Gly Asp Val Ala Ala Phe Met Ala
705 710 715 720

Asn Leu Ser Gln Leu Asp Asp Leu Phe Ala Ala Arg Val Ala Lys Ala
725 730 735

Arg Asp Glu Gly Lys Val Leu Arg Tyr Val Gly Asn Ile Asp Glu Asp
740 745 750

Gly Val Cys Arg Val Lys Ile Ala Glu Val Asp Gly Asn Asp Pro Leu
755 760 765

Phe Lys Val Lys Asn Gly Glu Asn Ala Leu Ala Phe Tyr Ser His Tyr
770 775 780

Tyr Gln Pro Leu Pro Leu Val Leu Arg Gly Tyr Gly Ala Gly Asn Asp
785 790 795 800

Val Thr Ala Ala Gly Val Phe Ala Asp Leu Leu Arg Thr Leu Ser Trp
805 810 815

Lys Leu Gly Val
820

<210> 117
<211> 2463
<212> DNA
<213> Escherichia coli

<220>
<221> misc_feature
<223> Encodes aspartokinase I and homoserine dehydrogenase I (EcThrA)

<400> 117
atgcgagtgt tgaagttcgg cggtacatca gtggcaaatg cagaacgttt tctgcgtgtt 60

gccgatattc tggaaagcaa tgccaggcag gggcaggtgg ccaccgtcct ctctgccccc 120

gccaaaatca ccaaccacct ggtggcgatg attgaaaaaa ccattagcgg ccaggatgct 180

ttacccaata tcagcgatgc cgaacgtatt tttgccgaac ttttgacggg actcgccgcc 240
gcccagccgg ggttcccgct ggcgcaattg aaaactttcg tcgatcagga atttgcccaa 300
ataaaacatg tcctgcatgg cattagtttg ttggggcagt gcccggatag catcaacgct 360
gcgctgattt gccgtggcga gaaaatgtcg atcgccatta tggccggcgt attagaagcg 420
cgcggtcaca acgttactgt tatcgatccg gtcgaaaaac tgctggcagt ggggcattac 480
ctcgaatcta ccgtcgatat tgctgagtcc acccgccgta ttgcggcaag ccgcattccg 540
gctgatcaca tggtgctgat ggcaggtttc accgccggta atgaaaaagg cgaactggtg 600
gtgcttggac gcaacggttc cgactactct gctgcggtgc tggctgcctg tttacgcgcc 660
gattgttgcg agatttggac ggacgttgac ggggtctata cctgcgaccc gcgtcaggtg 720
cccgatgcga ggttgttgaa gtcgatgtcc taccaggaag cgatggagct ttcctacttc 780
ggcgctaaag ttcttcaccc ccgcaccatt acccccatcg cccagttcca gatcccttgc 840
ctgattaaaa ataccggaaa tcctcaagca ccaggtacgc tcattggtgc cagccgtgat 900
gaagacgaat taccggtcaa gggcatttcc aatctgaata acatggcaat gttcagcgtt 960
tctggtccgg ggatgaaagg gatggtcggc atggcggcgc gcgtctttgc agcgatgtca 1020
cgcgcccgta tttccgtggt gctgattacg caatcatctt ccgaatacag catcagtttc 1080
tgcgttccac aaagcgactg tgtgcgagct gaacgggcaa tgcaggaaga gttctacctg 1140
gaactgaaag aaggcttact ggagccgctg gcagtgacgg aacggctggc cattatctcg 1200
gtggtaggtg atggtatgcg caccttgcgt gggatctcgg cgaaattctt tgccgcactg 1260
gcccgcgcca atatcaacat tgtcgccatt gctcagggat cttctgaacg ctcaatctct 1320
gtcgtggtaa ataacgatga tgcgaccact ggcgtgcgcg ttactcatca gatgctgttc 1380
aataccgatc aggttatcga agtgtttgtg attggcgtcg gtggcgttgg cggtgcgctg 1440
ctggagcaac tgaagcgtca gcaaagctgg ctgaagaata aacatatcga cttacgtgtc 1500
tgcggtgttg ccaactcgaa ggctctgctc accaatgtac atggccttaa tctggaaaac 1560
tggcaggaag aactggcgca agccaaagag ccgtttaatc tcgggcgctt aattcgcctc 1620
gtgaaagaat atcatctgct gaacccggtc attgttgact gcacttccag ccaggcagtg 1680
gcggatcaat atgccgactt cctgcgcgaa ggtttccacg ttgtcacgcc gaacaaaaag 1740
gccaacacct cgtcgatgga ttactaccat cagttgcgtt atgcggcgga aaaatcgcgg 1800
cgtaaattcc tctatgacac caacgttggg gctggattac cggttattga gaacctgcaa 1860
aatctgctca atgcaggtga tgaattgatg aagttctccg gcattctttc tggttcgctt 1920
tcttatatct tcggcaagtt agacgaaggc atgagtttct ccgaggcgac cacgctggcg 1980
cgggaaatgg gttataccga accggacccg cgagatgatc tttctggtat ggatgtggcg 2040
cgtaaactat tgattctcgc tcgtgaaacg ggacgtgaac tggagctggc ggatattgaa 2100

```
attgaacctg tgctgccccgc agagtttaac gccgagggtg atgttgccgc ttttatggcg     2160

aatctgtcac aactcgacga tctctttgcc gcgcgcgtgg cgaaggcccg tgatgaagga     2220

aaagttttgc gctatgttgg caatattgat gaagatggcg tctgccgcgt gaagattgcc     2280

gaagtggatg gtaatgatcc gctgttcaaa gtgaaaaatg gcgaaaacgc cctggccttc     2340

tatagccact attatcagcc gctgccgttg gtactgcgcg gatatggtgc gggcaatgac     2400

gttacagctg ccggtgtctt tgctgatctg ctacgtaccc tctcatggaa gttaggagtc     2460

tga                                                                   2463
```

<210>  118
<211>  820
<212>  PRT
<213>  Escherichia coli S345F variant

<220>
<221>  MISC_FEATURE
<223>  Escherichia coli  ThrA S345F variant

<400>  118

```
Met Arg Val Leu Lys Phe Gly Gly Thr Ser Val Ala Asn Ala Glu Arg
1               5                   10                  15

Phe Leu Arg Val Ala Asp Ile Leu Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Glu Lys Thr Ile Ser Gly Gln Asp Ala Leu Pro Asn Ile
    50                  55                  60

Ser Asp Ala Glu Arg Ile Phe Ala Glu Leu Leu Thr Gly Leu Ala Ala
65                  70                  75                  80

Ala Gln Pro Gly Phe Pro Leu Ala Gln Leu Lys Thr Phe Val Asp Gln
                85                  90                  95

Glu Phe Ala Gln Ile Lys His Val Leu His Gly Ile Ser Leu Leu Gly
            100                 105                 110

Gln Cys Pro Asp Ser Ile Asn Ala Ala Leu Ile Cys Arg Gly Glu Lys
        115                 120                 125

Met Ser Ile Ala Ile Met Ala Gly Val Leu Glu Ala Arg Gly His Asn
    130                 135                 140
```

Val Thr Val Ile Asp Pro Val Glu Lys Leu Leu Ala Val Gly His Tyr
145 150 155 160

Leu Glu Ser Thr Val Asp Ile Ala Glu Ser Thr Arg Arg Ile Ala Ala
165 170 175

Ser Arg Ile Pro Ala Asp His Met Val Leu Met Ala Gly Phe Thr Ala
180 185 190

Gly Asn Glu Lys Gly Glu Leu Val Val Leu Gly Arg Asn Gly Ser Asp
195 200 205

Tyr Ser Ala Ala Val Leu Ala Ala Cys Leu Arg Ala Asp Cys Cys Glu
210 215 220

Ile Trp Thr Asp Val Asp Gly Val Tyr Thr Cys Asp Pro Arg Gln Val
225 230 235 240

Pro Asp Ala Arg Leu Leu Lys Ser Met Ser Tyr Gln Glu Ala Met Glu
245 250 255

Leu Ser Tyr Phe Gly Ala Lys Val Leu His Pro Arg Thr Ile Thr Pro
260 265 270

Ile Ala Gln Phe Gln Ile Pro Cys Leu Ile Lys Asn Thr Gly Asn Pro
275 280 285

Gln Ala Pro Gly Thr Leu Ile Gly Ala Ser Arg Asp Glu Asp Glu Leu
290 295 300

Pro Val Lys Gly Ile Ser Asn Leu Asn Asn Met Ala Met Phe Ser Val
305 310 315 320

Ser Gly Pro Gly Met Lys Gly Met Val Gly Met Ala Ala Arg Val Phe
325 330 335

Ala Ala Met Ser Arg Ala Arg Ile Phe Val Val Leu Ile Thr Gln Ser
340 345 350

Ser Ser Glu Tyr Ser Ile Ser Phe Cys Val Pro Gln Ser Asp Cys Val
355 360 365

Arg Ala Glu Arg Ala Met Gln Glu Glu Phe Tyr Leu Glu Leu Lys Glu
370 375 380

Gly Leu Leu Glu Pro Leu Ala Val Thr Glu Arg Leu Ala Ile Ile Ser
385 390 395 400

Val Val Gly Asp Gly Met Arg Thr Leu Arg Gly Ile Ser Ala Lys Phe
405 410 415

Phe Ala Ala Leu Ala Arg Ala Asn Ile Asn Ile Val Ala Ile Ala Gln
420 425 430

Gly Ser Ser Glu Arg Ser Ile Ser Val Val Val Asn Asn Asp Asp Ala
435 440 445

Thr Thr Gly Val Arg Val Thr His Gln Met Leu Phe Asn Thr Asp Gln
450 455 460

Val Ile Glu Val Phe Val Ile Gly Val Gly Gly Val Gly Gly Ala Leu
465 470 475 480

Leu Glu Gln Leu Lys Arg Gln Gln Ser Trp Leu Lys Asn Lys His Ile
485 490 495

Asp Leu Arg Val Cys Gly Val Ala Asn Ser Lys Ala Leu Leu Thr Asn
500 505 510

Val His Gly Leu Asn Leu Glu Asn Trp Gln Glu Glu Leu Ala Gln Ala
515 520 525

Lys Glu Pro Phe Asn Leu Gly Arg Leu Ile Arg Leu Val Lys Glu Tyr
530 535 540

His Leu Leu Asn Pro Val Ile Val Asp Cys Thr Ser Ser Gln Ala Val
545 550 555 560

Ala Asp Gln Tyr Ala Asp Phe Leu Arg Glu Gly Phe His Val Val Thr
565 570 575

Pro Asn Lys Lys Ala Asn Thr Ser Ser Met Asp Tyr Tyr His Gln Leu
580 585 590

Arg Tyr Ala Ala Glu Lys Ser Arg Arg Lys Phe Leu Tyr Asp Thr Asn
595 600 605

Val Gly Ala Gly Leu Pro Val Ile Glu Asn Leu Gln Asn Leu Leu Asn
610 615 620

Ala Gly Asp Glu Leu Met Lys Phe Ser Gly Ile Leu Ser Gly Ser Leu
625 630 635 640

Ser Tyr Ile Phe Gly Lys Leu Asp Glu Gly Met Ser Phe Ser Glu Ala

645 650 655

Thr Thr Leu Ala Arg Glu Met Gly Tyr Thr Glu Pro Asp Pro Arg Asp
660 665 670

Asp Leu Ser Gly Met Asp Val Ala Arg Lys Leu Leu Ile Leu Ala Arg
675 680 685

Glu Thr Gly Arg Glu Leu Glu Leu Ala Asp Ile Glu Ile Glu Pro Val
690 695 700

Leu Pro Ala Glu Phe Asn Ala Glu Gly Asp Val Ala Ala Phe Met Ala
705 710 715 720

Asn Leu Ser Gln Leu Asp Asp Leu Phe Ala Ala Arg Val Ala Lys Ala
725 730 735

Arg Asp Glu Gly Lys Val Leu Arg Tyr Val Gly Asn Ile Asp Glu Asp
740 745 750

Gly Val Cys Arg Val Lys Ile Ala Glu Val Asp Gly Asn Asp Pro Leu
755 760 765

Phe Lys Val Lys Asn Gly Glu Asn Ala Leu Ala Phe Tyr Ser His Tyr
770 775 780

Tyr Gln Pro Leu Pro Leu Val Leu Arg Gly Tyr Gly Ala Gly Asn Asp
785 790 795 800

Val Thr Ala Ala Gly Val Phe Ala Asp Leu Leu Arg Thr Leu Ser Trp
805 810 815

Lys Leu Gly Val
820

<210> 119
<211> 2463
<212> DNA
<213> Escherichia coli S345F variant

<220>
<221> misc_feature
<223> Encodes Escherichia coli ThrA S345F variant

<400> 119
atgcgagtgt tgaagttcgg cggtacatca gtggcaaatg cagaacgttt tctgcgtgtt 60

gccgatattc tggaaagcaa tgccaggcag gggcaggtgg ccaccgtcct ctctgccccc 120

gccaaaatca ccaaccacct ggtggcgatg attgaaaaaa ccattagcgg ccaggatgct 180

ttacccaata tcagcgatgc cgaacgtatt tttgccgaac ttttgacggg actcgccgcc 240

gcccagccgg ggttcccgct ggcgcaattg aaaactttcg tcgatcagga atttgcccaa 300

ataaaacatg tcctgcatgg cattagtttg ttggggcagt gcccggatag catcaacgct 360

gcgctgattt gccgtggcga gaaaatgtcg atcgccatta tggccggcgt attagaagcg 420

cgcggtcaca acgttactgt tatcgatccg gtcgaaaaac tgctggcagt ggggcattac 480

ctcgaatcta ccgtcgatat tgctgagtcc acccgccgta ttgcggcaag ccgcattccg 540

gctgatcaca tggtgctgat ggcaggtttc accgccggta atgaaaaagg cgaactggtg 600

gtgcttggac gcaacggttc cgactactct gctgcggtgc tggctgcctg tttacgcgcc 660

gattgttgcg agatttggac ggacgttgac ggggtctata cctgcgaccc gcgtcaggtg 720

cccgatgcga ggttgttgaa gtcgatgtcc taccaggaag cgatggagct ttcctacttc 780

ggcgctaaag ttcttcaccc ccgcaccatt acccccatcg cccagttcca gatcccttgc 840

ctgattaaaa ataccggaaa tcctcaagca ccaggtacgc tcattggtgc cagccgtgat 900

gaagacgaat taccggtcaa gggcatttcc aatctgaata acatggcaat gttcagcgtt 960

tctggtccgg ggatgaaagg gatggtcggc atggcggcgc gcgtctttgc agcgatgtca 1020

cgcgcccgta ttttcgtggt gctgattacg caatcatctt ccgaatacag catcagtttc 1080

tgcgttccac aaagcgactg tgtgcgagct gaacgggcaa tgcaggaaga gttctacctg 1140

gaactgaaag aaggcttact ggagccgctg gcagtgacgg aacggctggc cattatctcg 1200

gtggtaggtg atggtatgcg caccttgcgt gggatctcgg cgaaattctt tgccgcactg 1260

gcccgcgcca atatcaacat tgtcgccatt gctcagggat cttctgaacg ctcaatctct 1320

gtcgtggtaa ataacgatga tgcgaccact ggcgtgcgcg ttactcatca gatgctgttc 1380

aataccgatc aggttatcga agtgtttgtg attggcgtcg gtggcgttgg cggtgcgctg 1440

ctggagcaac tgaagcgtca gcaaagctgg ctgaagaata aacatatcga cttacgtgtc 1500

tgcggtgttg ccaactcgaa ggctctgctc accaatgtac atggccttaa tctggaaaac 1560

tggcaggaag aactggcgca agccaaagag ccgtttaatc tcgggcgctt aattcgcctc 1620

gtgaaagaat atcatctgct gaacccggtc attgttgact gcacttccag ccaggcagtg 1680

gcggatcaat atgccgactt cctgcgcgaa ggtttccacg ttgtcacgcc gaacaaaaag 1740

gccaacacct cgtcgatgga ttactaccat cagttgcgtt atgcggcgga aaaatcgcgg 1800

cgtaaattcc tctatgacac caacgttggg gctggattac cggttattga gaacctgcaa 1860

aatctgctca atgcaggtga tgaattgatg aagttctccg gcattctttc tggttcgctt 1920

tcttatatct tcggcaagtt agacgaaggc atgagtttct ccgaggcgac cacgctggcg 1980

cgggaaatgg gttataccga accggacccg cgagatgatc tttctggtat ggatgtggcg 2040

cgtaaactat tgattctcgc tcgtgaaacg ggacgtgaac tggagctggc ggatattgaa 2100

attgaacctg tgctgcccgc agagtttaac gccgagggtg atgttgccgc ttttatggcg 2160

aatctgtcac aactcgacga tctctttgcc gcgcgcgtgg cgaaggcccg tgatgaagga 2220

aaagttttgc gctatgttgg caatattgat gaagatggcg tctgccgcgt gaagattgcc 2280

gaagtggatg gtaatgatcc gctgttcaaa gtgaaaaatg gcgaaaacgc cctggccttc 2340

tatagccact attatcagcc gctgccgttg gtactgcgcg gatatggtgc gggcaatgac 2400

gttacagctg ccggtgtctt tgctgatctg ctacgtaccc tctcatggaa gttaggagtc 2460

tga 2463

<210> 120
<211> 404
<212> PRT
<213> Bacillus subtilis

<220>
<221> MISC_FEATURE
<223> aspartate kinase

<400> 120

Met Lys Ile Ile Val Gln Lys Phe Gly Gly Thr Ser Val Lys Asp Asp
1 5 10 15

Lys Gly Arg Lys Leu Ala Leu Gly His Ile Lys Glu Ala Ile Ser Glu
20 25 30

Gly Tyr Lys Val Val Val Val Val Ser Ala Met Gly Arg Lys Gly Asp
35 40 45

Pro Tyr Ala Thr Asp Ser Leu Leu Gly Leu Leu Tyr Gly Asp Gln Ser
50 55 60

Ala Ile Ser Pro Arg Glu Gln Asp Leu Leu Leu Ser Cys Gly Glu Thr
65 70 75 80

Ile Ser Ser Val Val Phe Thr Ser Met Leu Leu Asp Asn Gly Val Lys
85 90 95

Ala Ala Ala Leu Thr Gly Ala Gln Ala Gly Phe Leu Thr Asn Asp Gln
100 105 110

His Thr Asn Ala Lys Ile Ile Glu Met Lys Pro Glu Arg Leu Phe Ser
115 120 125

Val Leu Ala Asn His Asp Ala Val Val Val Ala Gly Phe Gln Gly Ala
130 135 140

```
Thr Glu Lys Gly Asp Thr Thr Thr Ile Gly Arg Gly Gly Ser Asp Thr
145                 150                 155                 160

Ser Ala Ala Ala Leu Gly Ala Ala Val Asp Ala Glu Tyr Ile Asp Ile
                165                 170                 175

Phe Thr Asp Val Glu Gly Val Met Thr Ala Asp Pro Arg Val Val Glu
            180                 185                 190

Asn Ala Lys Pro Leu Pro Val Val Thr Tyr Thr Glu Ile Cys Asn Leu
        195                 200                 205

Ala Tyr Gln Gly Ala Lys Val Ile Ser Pro Arg Ala Val Glu Ile Ala
    210                 215                 220

Met Gln Ala Lys Val Pro Ile Arg Val Arg Ser Thr Tyr Ser Asn Asp
225                 230                 235                 240

Lys Gly Thr Leu Val Thr Ser His His Ser Ser Lys Val Gly Ser Asp
                245                 250                 255

Val Phe Glu Arg Leu Ile Thr Gly Ile Ala His Val Lys Asp Val Thr
            260                 265                 270

Gln Phe Lys Val Pro Ala Lys Ile Gly Gln Tyr Asn Val Gln Thr Glu
        275                 280                 285

Val Phe Lys Ala Met Ala Asn Ala Gly Ile Ser Val Asp Phe Phe Asn
    290                 295                 300

Ile Thr Pro Ser Glu Ile Val Tyr Thr Val Ala Gly Asn Lys Thr Glu
305                 310                 315                 320

Thr Ala Gln Arg Ile Leu Met Asp Met Gly Tyr Asp Pro Met Val Thr
                325                 330                 335

Arg Asn Cys Ala Lys Val Ser Ala Val Gly Ala Gly Ile Met Gly Val
            340                 345                 350

Pro Gly Val Thr Ser Lys Ile Val Ser Ala Leu Ser Glu Lys Glu Ile
        355                 360                 365

Pro Ile Leu Gln Ser Ala Asp Ser His Thr Thr Ile Trp Val Leu Val
    370                 375                 380

His Glu Ala Asp Met Val Pro Ala Val Asn Ala Leu His Glu Val Phe
```

385 390 395 400

Glu Leu Ser Lys

<210> 121
<211> 1215
<212> DNA
<213> Bacillus subtilis

<220>
<221> misc_feature
<223> Encodes aspartate kinase

<400> 121
gtgaagataa ttgttcaaaa gttcggcgga acatcagtaa aagatgataa aggccgcaaa 60

ctggctttag ggcatattaa agaagcaatc agtgaaggat ataaggttgt cgttgtcgtt 120

tcagcgatgg gccgaaaagg ggacccgtat gcaacagatt ctttgctcgg gctgctttac 180

ggcgatcagt cagcgatttc tcctagggag caggatttgc ttttatcttg cggagagact 240

atttcctctg tcgtctttac aagcatgctg cttgataacg gtgtgaaagc agctgcgctg 300

acaggggctc aggctggctt tttaacgaat gatcagcata ctaatgcgaa aatcattgaa 360

atgaaaccgg agcgtttatt cagtgtgctc gccaatcatg atgcggtagt cgtcgcaggc 420

ttccagggcg caactgaaaa gggtgataca acaacaatcg gcagaggggg cagcgatacg 480

tctgcagccg cactcggagc agctgttgat gcggaataca tcgatatttt caccgatgta 540

gaaggcgtga tgactgctga cccaagagtg gtagaaaatg caaaaccgct tcctgttgtg 600

acgtatacgg aaatctgcaa cctggcgtat caggggggcca aggtcatttc tccgcgtgct 660

gtggaaatcg caatgcaggc caaggttccg atccgcgtcc gttcgactta ttcgaatgat 720

aaaggcacat tggtgacatc acatcactct tccaaggtcg gcagtgatgt ttttgaaagg 780

ctgattacgg gaatcgccca tgtcaaagat gtcacgcagt tcaaagtgcc tgcgaaaatc 840

ggccagtata acgttcagac tgaagtgttc aaggctatgg caaacgctgg aatcagtgtc 900

gatttcttta acattacacc tagtgaaatc gtctatacgg tagccggaaa taaaacagaa 960

acagctcagc gtattttaat ggatatgggc tatgatccaa tggtgacgag aaactgcgcg 1020

aaggtatcgg cggtcggcgc gggcattatg ggtgttcccg gtgttacgtc caaaattgta 1080

tcggctcttt cagaaaaaga aattccgatc cttcagtcgg ctgacagcca tacgacgata 1140

tgggtgctcg tccatgaagc ggatatggtt cctgctgtaa acgctttgca tgaagtgttt 1200

gaactttcga agtaa 1215

<210> 122
<211> 411

<212> PRT
<213> Pseudomonas putida

<220>
<221> MISC_FEATURE
<223> aspartate kinase

<400> 122

Met Ala Leu Ile Val Gln Lys Phe Gly Gly Thr Ser Val Gly Ser Ile
1 5 10 15

Glu Arg Ile Glu Gln Val Ala Glu Lys Val Lys Lys His Arg Glu Ala
20 25 30

Gly Asp Asp Leu Val Val Val Leu Ser Ala Met Ser Gly Glu Thr Asn
35 40 45

Arg Leu Ile Asp Leu Ala Lys Gln Ile Thr Asp Gln Pro Val Pro Arg
50 55 60

Glu Leu Asp Val Ile Val Ser Thr Gly Glu Gln Val Thr Ile Ala Leu
65 70 75 80

Leu Thr Met Ala Leu Ile Lys Arg Gly Val Pro Ala Val Ser Tyr Thr
85 90 95

Gly Asn Gln Val Arg Ile Leu Thr Asp Ser Ser His Asn Lys Ala Arg
100 105 110

Ile Leu Gln Ile Asp Asp Gln Lys Ile Arg Ala Asp Leu Lys Glu Gly
115 120 125

Arg Val Val Val Val Ala Gly Phe Gln Gly Val Asp Glu His Gly Ser
130 135 140

Ile Thr Thr Leu Gly Arg Gly Gly Ser Asp Thr Thr Gly Val Ala Leu
145 150 155 160

Ala Ala Ala Leu Lys Ala Asp Glu Cys Gln Ile Tyr Thr Asp Val Asp
165 170 175

Gly Val Tyr Thr Thr Asp Pro Arg Val Val Pro Gln Ala Arg Arg Leu
180 185 190

Glu Lys Ile Thr Phe Glu Glu Met Leu Glu Met Ala Ser Leu Gly Ser
195 200 205

Lys Val Leu Gln Ile Arg Ser Val Glu Phe Ala Gly Lys Tyr Asn Val

```
    210                 215                 220

Pro Leu Arg Val Leu His Ser Phe Lys Glu Gly Pro Gly Thr Leu Ile
225                 230                 235                 240

Thr Ile Asp Glu Glu Glu Ser Met Glu Gln Pro Ile Ile Ser Gly Ile
                245                 250                 255

Ala Phe Asn Arg Asp Glu Ala Lys Leu Thr Ile Arg Gly Val Pro Asp
            260                 265                 270

Thr Pro Gly Val Ala Phe Lys Ile Leu Gly Pro Ile Ser Ala Ser Asn
        275                 280                 285

Ile Glu Val Asp Met Ile Val Gln Asn Val Ala His Asp Asn Thr Thr
    290                 295                 300

Asp Phe Thr Phe Thr Val His Arg Asn Glu Tyr Glu Lys Ala Gln Ser
305                 310                 315                 320

Val Leu Glu Asn Thr Ala Arg Glu Ile Gly Ala Arg Glu Val Ile Gly
                325                 330                 335

Asp Thr Lys Ile Ala Lys Val Ser Ile Val Gly Val Gly Met Arg Ser
            340                 345                 350

His Ala Gly Val Ala Ser Cys Met Phe Glu Ala Leu Ala Lys Glu Ser
        355                 360                 365

Ile Asn Ile Gln Met Ile Ser Thr Ser Glu Ile Lys Val Ser Val Val
    370                 375                 380

Leu Glu Glu Lys Tyr Leu Glu Leu Ala Val Arg Ala Leu His Thr Ala
385                 390                 395                 400

Phe Asp Leu Asp Ala Pro Ala Arg Gln Gly Glu
                405                 410


<210>  123
<211>  1236
<212>  DNA
<213>  Pseudomonas putida


<220>
<221>  misc_feature
<223>  Encodes aspartate kinase

<400>  123
atggcgttga tcgtacagaa atttggcggc acctctgtcg gttccatcga gcggatcgag        60
```

caggtagccg aaaaggtcaa gaaacaccgt gaagcgggcg acgacctggt ggttgtgctg 120

tcggccatga gcggtgaaac caatcgcctg atcgacttgg ccaagcagat caccgatcag 180

ccggttcctc gtgaactgga cgtgatcgtg tcgacgggtg agcaggtcac cattgccctg 240

ctgaccatgg ccttgatcaa gcgtggtgtg ccagcggtgt cctacaccgg caaccaggtg 300

cgtattctca ccgacagctc gcacaacaag gcgcgcattc tgcaaatcga cgaccagaag 360

attcgtgccg acctcaagga aggtcgcgtg gtcgtggttg caggcttcca gggtgtcgac 420

gagcacggca gcatcactac cctgggtcgt ggtggctctg ataccaccgg cgtggccctg 480

gcggcagcgc tgaaggctga tgagtgccag atctacactg atgttgatgg cgtctacacc 540

accgacccgc gcgtcgtgcc acaggctcgt cgcctggaga agatcacctt cgaagagatg 600

ctggaaatgg ccagcctcgg ttccaaggtg ctgcagatcc gttcggtgga gttcgccggc 660

aagtacaacg ttccgctgcg cgtgctgcac agcttcaagg agggtccggg taccctcatt 720

accattgatg aagaggaatc catggaacag ccgatcattt ccggtatcgc cttcaaccgt 780

gatgaagcaa agctgaccat tcgcggcgtg ccggataccc cgggcgtggc cttcaagatc 840

ctcggcccga tcagcgcttc gaacatcgaa gtcgacatga tcgtgcagaa cgttgctcac 900

gataacacca ccgacttcac cttcaccgtg caccgcaatg agtacgagaa ggcgcagagc 960

gtgctggaaa acactgcgcg cgaaatcggt gcccgtgaag tgatcggcga caccaagatc 1020

gccaaggtct cgatcgttgg cgtcggcatg cgttcgcacg ccggtgttgc cagctgcatg 1080

ttcgaagccc tggccaagga gagcatcaac atccagatga tctccacttc cgagatcaag 1140

gtttcggtgg tgctcgaaga gaagtacctg gagctggccg tgcgcgcgct gcataccgcg 1200

ttcgatctgg atgctcctgc ccgacagggc gagtaa 1236

<210> 124
<211> 527
<212> PRT
<213> Saccharomyces cerevisiae

<220>
<221> MISC_FEATURE
<223> aspartate kinase

<400> 124

Met Pro Met Asp Phe Gln Pro Thr Ser Ser His Ser Asn Trp Val Val
1               5                   10                  15

Gln Lys Phe Gly Gly Thr Ser Val Gly Lys Phe Pro Val Gln Ile Val
            20                  25                  30

Asp Asp Ile Val Lys His Tyr Ser Lys Pro Asp Gly Pro Asn Asn Asn

35 40 45

Val Ala Val Val Cys Ser Ala Arg Ser Ser Tyr Thr Lys Ala Glu Gly
50 55 60

Thr Thr Ser Arg Leu Leu Lys Cys Cys Asp Leu Ala Ser Gln Glu Ser
65 70 75 80

Glu Phe Gln Asp Ile Ile Glu Val Ile Arg Gln Asp His Ile Asp Asn
85 90 95

Ala Asp Arg Phe Ile Leu Asn Pro Ala Leu Gln Ala Lys Leu Val Asp
100 105 110

Asp Thr Asn Lys Glu Leu Glu Leu Val Lys Lys Tyr Leu Asn Ala Ser
115 120 125

Lys Val Leu Gly Glu Val Ser Ser Arg Thr Val Asp Leu Val Met Ser
130 135 140

Cys Gly Glu Lys Leu Ser Cys Leu Phe Met Thr Ala Leu Cys Asn Asp
145 150 155 160

Arg Gly Cys Lys Ala Lys Tyr Val Asp Leu Ser His Ile Val Pro Ser
165 170 175

Asp Phe Ser Ala Ser Ala Leu Asp Asn Ser Phe Tyr Thr Phe Leu Val
180 185 190

Gln Ala Leu Lys Glu Lys Leu Ala Pro Phe Val Ser Ala Lys Glu Arg
195 200 205

Ile Val Pro Val Phe Thr Gly Phe Phe Gly Leu Val Pro Thr Gly Leu
210 215 220

Leu Asn Gly Val Gly Arg Gly Tyr Thr Asp Leu Cys Ala Ala Leu Ile
225 230 235 240

Ala Val Ala Val Asn Ala Asp Glu Leu Gln Val Trp Lys Glu Val Asp
245 250 255

Gly Ile Phe Thr Ala Asp Pro Arg Lys Val Pro Glu Ala Arg Leu Leu
260 265 270

Asp Ser Val Thr Pro Glu Glu Ala Ser Glu Leu Thr Tyr Tyr Gly Ser
275 280 285

```
Glu Val Ile His Pro Phe Thr Met Glu Gln Val Ile Arg Ala Lys Ile
    290                 295                 300

Pro Ile Arg Ile Lys Asn Val Gln Asn Pro Leu Gly Asn Gly Thr Ile
305                 310                 315                 320

Ile Tyr Pro Asp Asn Val Ala Lys Lys Gly Glu Ser Thr Pro Pro His
                325                 330                 335

Pro Pro Glu Asn Leu Ser Ser Ser Phe Tyr Glu Lys Arg Lys Arg Gly
            340                 345                 350

Ala Thr Ala Ile Thr Thr Lys Asn Asp Ile Phe Val Ile Asn Ile His
        355                 360                 365

Ser Asn Lys Lys Thr Leu Ser His Gly Phe Leu Ala Gln Ile Phe Thr
    370                 375                 380

Ile Leu Asp Lys Tyr Lys Leu Val Val Asp Leu Ile Ser Thr Ser Glu
385                 390                 395                 400

Val His Val Ser Met Ala Leu Pro Ile Pro Asp Ala Asp Ser Leu Lys
                405                 410                 415

Ser Leu Arg Gln Ala Glu Glu Lys Leu Arg Ile Leu Gly Ser Val Asp
            420                 425                 430

Ile Thr Lys Lys Leu Ser Ile Val Ser Leu Val Gly Lys His Met Lys
        435                 440                 445

Gln Tyr Ile Gly Ile Ala Gly Thr Met Phe Thr Thr Leu Ala Glu Glu
    450                 455                 460

Gly Ile Asn Ile Glu Met Ile Ser Gln Gly Ala Asn Glu Ile Asn Ile
465                 470                 475                 480

Ser Cys Val Ile Asn Glu Ser Asp Ser Ile Lys Ala Leu Gln Cys Ile
                485                 490                 495

His Ala Lys Leu Leu Ser Glu Arg Thr Asn Thr Ser Asn Gln Phe Glu
            500                 505                 510

His Ala Ile Asp Glu Arg Leu Glu Gln Leu Lys Arg Leu Gly Ile
        515                 520                 525

<210>  125
<211>  1584
<212>  DNA
```

<213> Saccharomyces cerevisiae

<220>
<221> misc_feature
<223> Encodes aspartate kinase

<400> 125
atgccaatgg atttccaacc tacatcaagt cattcgaact gggtcgtgca aaagttcggt 60

ggtacatctg tcggtaaatt tcccgtccaa atagtggatg acattgtgaa gcactattct 120

aaacctgacg gcccaaacaa taatgtcgct gtcgtttgtt ccgcccgttc ttcatacacc 180

aaggctgaag gtaccacttc tcgtcttttg aaatgttgtg atttggcttc gcaagaatct 240

gaatttcaag acattatcga agttatcaga caagaccata tcgataatgc cgaccgcttc 300

attctcaatc ctgccttgca agccaagtta gtggatgata ccaataaaga acttgaactg 360

gtcaagaaat atttaaatgc ttcaaaagtt ttgggtgaag tgagttcacg tacagtagat 420

ctggtgatgt catgtggtga gaagttgagt tgtttgttca tgactgcttt atgtaatgac 480

cgtggctgta aggccaaata tgtggatttg agccacattg ttccctctga tttcagtgcc 540

agcgctttgg ataacagttt ctacactttc ctggttcaag cattgaaaga aaaattggcc 600

ccctttgtaa gtgctaaaga gcgtatcgtt ccagtcttta cagggttttt tggtttagtt 660

ccaactggtc ttctgaatgg tgttggtcgt ggctataccg atttatgtgc cgctttgata 720

gcagttgctg taaatgctga tgaactacaa gtttggaagg aagttgatgg tatatttact 780

gctgatcctc gtaaggttcc tgaagcacgt ttgctagaca gtgttactcc agaagaagct 840

tctgaattaa catattatgg ttccgaagtt atacatcctt ttacgatgga acaagttatt 900

agggctaaga ttcctattag aatcaagaat gttcaaaatc cattaggtaa cggtaccatt 960

atctacccag ataatgtagc aaagaagggt gaatctactc caccacatcc tcctgagaac 1020

ttatcctcat ctttctatga aaagagaaag agaggtgcca ctgctatcac caccaaaaat 1080

gacattttcg tcatcaacat tcattccaat aagaaaaccc tatcccatgg tttcctagct 1140

caaatattta ccatcctgga taagtacaag ttagtcgtag atttaatatc tacttctgaa 1200

gttcatgttt cgatggcttt gcccattcca gatgcagact cattaaaatc tctgagacaa 1260

gctgaggaaa aattgagaat tttaggttct gttgatatca caaagaagtt gtctattgtt 1320

tcattagttg gtaaacatat gaaacaatac atcggcattg ctggtaccat gtttactact 1380

cttgctgaag aaggcatcaa cattgaaatg atttctcaag gggcaaatga aataaacata 1440

tcctgcgtta tcaatgaatc tgactccata aaagcgctac aatgtattca tgccaagtta 1500

ctaagtgagc ggacaaatac ttcaaaccaa tttgaacatg ccattgatga acgtttagaa 1560

caattgaaaa gacttggaat ttaa 1584

<210> 126
<211> 433
<212> PRT
<213> Bacillus subtilis

<220>
<221> MISC_FEATURE
<223> homoserine dehydrogenase

<400> 126

```
Met Lys Ala Ile Arg Val Gly Leu Leu Gly Leu Gly Thr Val Gly Ser
1               5                   10                  15

Gly Val Val Lys Ile Ile Gln Asp His Gln Asp Lys Leu Met His Gln
            20                  25                  30

Val Gly Cys Pro Val Thr Ile Lys Lys Val Leu Val Lys Asp Leu Glu
        35                  40                  45

Lys Lys Arg Glu Val Asp Leu Pro Lys Glu Val Leu Thr Thr Glu Val
    50                  55                  60

Tyr Asp Val Ile Asp Asp Pro Asp Val Asp Val Val Ile Glu Val Ile
65                  70                  75                  80

Gly Gly Val Glu Gln Thr Lys Gln Tyr Leu Val Asp Ala Leu Arg Ser
                85                  90                  95

Lys Lys His Val Val Thr Ala Asn Lys Asp Leu Met Ala Val Tyr Gly
            100                 105                 110

Ser Glu Leu Leu Ala Glu Ala Lys Glu Asn Gly Cys Asp Ile Tyr Phe
        115                 120                 125

Glu Ala Ser Val Ala Gly Gly Ile Pro Ile Leu Arg Thr Leu Glu Glu
    130                 135                 140

Gly Leu Ser Ser Asp Arg Ile Thr Lys Met Met Gly Ile Val Asn Gly
145                 150                 155                 160

Thr Thr Asn Phe Ile Leu Thr Lys Met Ile Lys Glu Lys Ser Pro Tyr
                165                 170                 175

Glu Glu Val Leu Lys Glu Ala Gln Asp Leu Gly Phe Ala Glu Ala Asp
            180                 185                 190

Pro Thr Ser Asp Val Glu Gly Leu Asp Ala Ala Arg Lys Met Ala Ile
        195                 200                 205
```

```
Leu Ala Arg Leu Gly Phe Ser Met Asn Val Asp Leu Glu Asp Val Lys
    210                 215                 220

Val Lys Gly Ile Ser Gln Ile Thr Asp Glu Asp Ile Ser Phe Ser Lys
225                 230                 235                 240

Arg Leu Gly Tyr Thr Met Lys Leu Ile Gly Ile Ala Gln Arg Asp Gly
                245                 250                 255

Ser Lys Ile Glu Val Ser Val Gln Pro Thr Leu Leu Pro Asp His His
            260                 265                 270

Pro Leu Ser Ala Val His Asn Glu Phe Asn Ala Val Tyr Val Tyr Gly
        275                 280                 285

Glu Ala Val Gly Glu Thr Met Phe Tyr Gly Pro Gly Ala Gly Ser Met
    290                 295                 300

Pro Thr Ala Thr Ser Val Val Ser Asp Leu Val Ala Val Met Lys Asn
305                 310                 315                 320

Met Arg Leu Gly Val Thr Gly Asn Ser Phe Val Gly Pro Gln Tyr Glu
                325                 330                 335

Lys Asn Met Lys Ser Pro Ser Asp Ile Tyr Ala Gln Gln Phe Leu Arg
            340                 345                 350

Ile His Val Lys Asp Glu Val Gly Ser Phe Ser Lys Ile Thr Ser Val
        355                 360                 365

Phe Ser Glu Arg Gly Val Ser Phe Glu Lys Ile Leu Gln Leu Pro Ile
    370                 375                 380

Lys Gly His Asp Glu Leu Ala Glu Ile Val Ile Val Thr His His Thr
385                 390                 395                 400

Ser Glu Ala Asp Phe Ser Asp Ile Leu Gln Asn Leu Asn Asp Leu Glu
                405                 410                 415

Val Val Gln Glu Val Lys Ser Thr Tyr Arg Val Glu Gly Asn Gly Trp
            420                 425                 430

Ser
```

<210>  127
<211>  1302

<212> DNA
<213> Bacillus subtilis

<220>
<221> misc_feature
<223> Encodes homoserine dehydrogenase

<400> 127
ttagctccaa ccgttccctt ctacacgata tgtgcttttg acttcttgaa cgacttccaa 60

atcatttagg ttttgcagga tatcactgaa atcagcttct gatgtatgat gtgtgacaat 120

tacgatttca gctaactcat catggccttt aattggcagc tgaaggattt tttcaaagct 180

cacgccccgc tctgagaaca cagatgtaat tttcgagaat gaaccaacct catcttttac 240

atgaattctt aaaaactgct gtgcataaat gtcagacggc gatttcatgt ttttctcata 300

ttgcggtccg acaaagctgt tgccggttac acctaggcgc atatttttca tgacagcgac 360

gaggtcagaa acaacggatg tcgctgtcgg catgcttccg gctcccggcc cgtagaacat 420

cgtctcaccg acagcctcgc cgtatacata aacagcgtta aactcattat gaacagcaga 480

aagcggatga tggtcaggaa gcagtgtcgg ctgtacgctg acctcgattt tgctgccgtc 540

acgctgagca atcccgatca gcttcattgt atagccgagg cgtttgctga agctgatgtc 600

ctcgtctgta atttgggaga tcccctttac tttgacgtct tccagatcca cgttcatcga 660

gaagccgagg cgcgccaata tcgccatttt ccgtgcggcg tcaagccctt ccacgtcaga 720

agtcggatca gcttcggcaa aaccgagatc ctgcgcttcc ttgagcactt cctcgtatgg 780

gcttttctct ttgatcattt tggttaagat aaagtttgtt gtgccgttca caattcccat 840

catttttgta attcgatctg atgagaggcc ttcctctaac gtgcgcagaa tcggaatccc 900

gccggcaaca ctggcttcaa agtagatatc gcatccattt tccttcgctt cggcaagcag 960

ctcggagccg tacacagcca ttaagtcctt gtttgctgtg acaacatgct tttcgatct 1020

cagcgcgtcg accaaatatt gttttgtctg ttcaacgccg ccgatcacct cgatgacgac 1080

atcaacatct ggatcatcaa tgacatcata cacttctgtc gtgagcactt ccttcggcaa 1140

atctacttct ctctttttct ctaaatcttt tacaagcact ttttttatcg taaccggaca 1200

gccgacctga tgcataagct tatcctgatg gtcttgaata attttaacga caccgcttcc 1260

gacggtacct aaacctaaaa gccctacacg aatcgctttc aa 1302

<210> 128
<211> 434
<212> PRT
<213> Pseudomonas putida

<220>
<221> MISC_FEATURE
<223> homoserine dehydrogenase

<400> 128

Met Lys Pro Val Lys Val Gly Ile Cys Gly Leu Gly Thr Val Gly Gly
1 5 10 15

Gly Thr Phe Asn Val Leu Gln Arg Asn Ala Glu Glu Ile Ala Arg Arg
20 25 30

Ala Gly Arg Gly Ile Glu Val Ala Gln Ile Ala Met Arg Ser Gln Asn
35 40 45

Pro Asn Cys Gln Ile Thr Gly Thr Pro Ile Thr Ala Asp Val Phe Glu
50 55 60

Val Ala Ser Asn Pro Glu Ile Asp Ile Val Ile Glu Leu Ile Gly Gly
65 70 75 80

Tyr Thr Ile Ala Arg Asp Leu Val Leu Lys Ala Ile Glu Asn Gly Lys
85 90 95

His Val Val Thr Ala Asn Lys Ala Leu Ile Ala Val His Gly Asn Glu
100 105 110

Ile Phe Ala Lys Ala Arg Glu Lys Gly Val Ile Val Ala Phe Glu Ala
115 120 125

Ala Val Ala Gly Gly Ile Pro Val Ile Lys Ala Ile Arg Glu Gly Leu
130 135 140

Ser Ala Asn Arg Ile Asn Trp Leu Ala Gly Ile Ile Asn Gly Thr Gly
145 150 155 160

Asn Phe Ile Leu Thr Glu Met Arg Glu Lys Gly Arg Ala Phe Pro Asp
165 170 175

Val Leu Ala Glu Ala Gln Ala Leu Gly Tyr Ala Glu Ala Asp Pro Thr
180 185 190

Phe Asp Val Glu Gly Ile Asp Ala Ala His Lys Leu Thr Ile Leu Ala
195 200 205

Ser Ile Ala Phe Gly Ile Pro Leu Gln Phe Asp Lys Ala Tyr Thr Glu
210 215 220

Gly Ile Thr Gln Leu Thr Thr Ala Asp Val Asn Tyr Ala Glu Ala Leu
225 230 235 240

```
Gly Tyr Arg Ile Lys His Leu Gly Val Ala Arg Arg Thr Ala Glu Gly
                245                 250                 255

Ile Glu Leu Arg Val His Pro Thr Leu Ile Pro Ala Asp Arg Leu Ile
            260                 265                 270

Ala Asn Val Asn Gly Val Met Asn Ala Val Met Val Asn Gly Asp Ala
        275                 280                 285

Ala Gly Ser Thr Leu Tyr Tyr Gly Ala Gly Ala Gly Met Glu Pro Thr
    290                 295                 300

Ala Ser Ser Val Val Gly Asp Leu Val Asp Val Val Arg Ala Met Thr
305                 310                 315                 320

Ser Asp Pro Glu Asn Arg Val Pro His Leu Ala Phe Gln Pro Asp Ser
                325                 330                 335

Leu Ser Ala His Pro Ile Leu Pro Ile Glu Ala Cys Glu Ser Ala Tyr
            340                 345                 350

Tyr Leu Arg Ile Gln Ala Lys Asp His Pro Gly Val Leu Ala Gln Val
        355                 360                 365

Ala Ser Ile Leu Ser Glu Arg Gly Ile Asn Ile Glu Ser Ile Met Gln
    370                 375                 380

Lys Glu Ala Glu Glu Gln Asp Gly Leu Val Pro Met Ile Leu Val Thr
385                 390                 395                 400

His Gly Val Val Glu Gln Arg Ile Asn Asp Ala Ile Val Ala Leu Glu
                405                 410                 415

Ala Leu Gln Asp Val Val Gly Lys Val Val Arg Ile Arg Val Glu Gln
            420                 425                 430

Leu Asn


<210>  129
<211>  1305
<212>  DNA
<213>  Pseudomonas putida


<220>
<221>  misc_feature
<223>  Encodes homoserine dehydrogenase

<400>  129
```

```
ttagttgagc tgctcgacgc gaatgcgcac aaccttgccg accacgtcct gcagggcttc        60
cagggcgacg atggcgtcat tgatacgctg ctcgaccacg ccatgggtca ccaggatcat       120
tggcaccagg ccatcctgtt cctcggcttc cttctgcatg atcgactcga tgttgatacc       180
gcgctccgag aggatgctgg cgacctgggc cagtacgccc gggtgatcct tggcctggat       240
acgcaggtag taggcgcttt cgcaggcttc gatcggcagg atcgggtggg ccgacagcga       300
gtctggctgg aaggccaggt gcggcacacg gttttccggg tcggaggtca tggcacggac       360
cacgtcgacc aggtcgccga ccactgacga agcggtaggc tccatacccg cgccggcacc       420
gtagtacagg gtggagccgg cagcatcacc gttgaccatg acagcgttca tcacgccgtt       480
gacgttggcg atcagacggt cggccgggat cagcgtcggg tgcacgcgca gctcgatacc       540
ttcggcggtg cgacgcgcca cgcccaggtg cttgatgcgg tagcccaacg cctcggcgta       600
gttcacgtct gccgttgtca gctgggtgat accttcggtg taggccttgt cgaactgcag       660
cgggataccg aacgcgatgg acgccaggat cgtcagcttg tgcgcagcgt caatgccttc       720
tacgtcaaag gtcgggtcgg cttcggcgta gcccagcgct tgagcttcgg ccagcacgtc       780
cgggaaggca cggcccttct cacgcatttc ggtgaggatg aagttgccag tgccgttgat       840
gatgccagcc agccagttga tgcggttggc cgacaggcct tcgcggatgg ccttgatcac       900
cgggatgcca ccggctacgg ccgcctcgaa agcgacaatc acgcccttct cgcgagcttt       960
ggcgaagatt tcgttgccgt gcacggcaat cagcgccttg ttggcagtga ccacgtgctt      1020
gccgttttcg atggccttca gcaccaggtc gcgggcgatg gtatagccac cgatcagctc      1080
gatgacaatg tcgatctccg ggttgcttgc aacttcgaac acgtcagcgg taatgggggt      1140
accggtaatc tggcagttcg ggttctgcga gcgcatggcg atctgtgcca cttcaatacc      1200
gcgcccggca cggcgggcaa tctcctcggc gttgcgctga agtacattga aggttccgcc      1260
accgacggtc cccaacccac agatgcctac tttgaccggt ttcac                      1305
```

<210> 130
<211> 359
<212> PRT
<213> Saccharomyces cerevisiae

<220>
<221> MISC_FEATURE
<223> homoserine dehydrogenase

<400> 130

```
Met Ser Thr Lys Val Val Asn Val Ala Val Ile Gly Ala Gly Val Val
1               5                   10                  15

Gly Ser Ala Phe Leu Asp Gln Leu Leu Ala Met Lys Ser Thr Ile Thr
            20                  25                  30
```

```
Tyr Asn Leu Val Leu Leu Ala Glu Ala Glu Arg Ser Leu Ile Ser Lys
        35                  40                  45

Asp Phe Ser Pro Leu Asn Val Gly Ser Asp Trp Lys Ala Ala Leu Ala
    50                  55                  60

Ala Ser Thr Thr Lys Thr Leu Pro Leu Asp Asp Leu Ile Ala His Leu
65                  70                  75                  80

Lys Thr Ser Pro Lys Pro Val Ile Leu Val Asp Asn Thr Ser Ser Ala
                85                  90                  95

Tyr Ile Ala Gly Phe Tyr Thr Lys Phe Val Glu Asn Gly Ile Ser Ile
            100                 105                 110

Ala Thr Pro Asn Lys Lys Ala Phe Ser Ser Asp Leu Ala Thr Trp Lys
        115                 120                 125

Ala Leu Phe Ser Asn Lys Pro Thr Asn Gly Phe Val Tyr His Glu Ala
    130                 135                 140

Thr Val Gly Ala Gly Leu Pro Ile Ile Ser Phe Leu Arg Glu Ile Ile
145                 150                 155                 160

Gln Thr Gly Asp Glu Val Glu Lys Ile Glu Gly Ile Phe Ser Gly Thr
                165                 170                 175

Leu Ser Tyr Ile Phe Asn Glu Phe Ser Thr Ser Gln Ala Asn Asp Val
            180                 185                 190

Lys Phe Ser Asp Val Val Lys Val Ala Lys Lys Leu Gly Tyr Thr Glu
        195                 200                 205

Pro Asp Pro Arg Asp Asp Leu Asn Gly Leu Asp Val Ala Arg Lys Val
    210                 215                 220

Thr Ile Val Gly Arg Ile Ser Gly Val Glu Val Glu Ser Pro Thr Ser
225                 230                 235                 240

Phe Pro Val Gln Ser Leu Ile Pro Lys Pro Leu Glu Ser Val Lys Ser
                245                 250                 255

Ala Asp Glu Phe Leu Glu Lys Leu Ser Asp Tyr Asp Lys Asp Leu Thr
            260                 265                 270

Gln Leu Lys Lys Glu Ala Ala Thr Glu Asn Lys Val Leu Arg Phe Ile
```

            275                 280                 285

Gly Lys Val Asp Val Ala Thr Lys Ser Val Ser Val Gly Ile Glu Lys
        290                 295                 300

Tyr Asp Tyr Ser His Pro Phe Ala Ser Leu Lys Gly Ser Asp Asn Val
305                 310                 315                 320

Ile Ser Ile Lys Thr Lys Arg Tyr Thr Asn Pro Val Val Ile Gln Gly
                325                 330                 335

Ala Gly Ala Gly Ala Ala Val Thr Ala Ala Gly Val Leu Gly Asp Val
            340                 345                 350

Ile Lys Ile Ala Gln Arg Leu
        355

<210>  131
<211>  1080
<212>  DNA
<213>  Saccharomyces cerevisiae

<220>
<221>  misc_feature
<223>  Encodes homoserine dehydrogenase

<400>  131
atgagcacta aagttgttaa tgttgccgtt atcggtgccg gtgttgttgg ttcagctttc      60

ttggatcaat tgttagccat gaagtctacc attacttaca atctagttct tttggctgaa     120

gctgagcgtt ctttaatctc caaggacttt tctccattaa atgttggttc tgattggaag     180

gctgctttag cagcctccac tactaaaacg ttgcctttgg atgatttaat tgctcatttg     240

aagacttcac ctaagccagt cattttggtt gataacactt ccagcgctta cattgctggt     300

ttttacacta agtttgtcga aaatggtatt tccattgcta ctccaaacaa gaaggccttt     360

tcctctgatt tggctacctg gaaggctctt ttctcaaata agccaactaa cggttttgtc     420

tatcatgaag ctaccgtcgg tgctggtttg cctatcatca gtttcttaag agaaattatt     480

caaaccggtg acgaagttga aaaaattgaa ggtatcttct ctggtactct atcttatatt     540

ttcaacgagt tctccactag tcaagctaac gacgtcaaat tctctgatgt tgtcaaagtt     600

gctaaaaaat tgggttatac tgaaccagat ccaagagatg atttgaatgg gttggatgtt     660

gctagaaagg ttaccattgt tggtaggata tctggtgtgg aagttgaatc tccaacttcc     720

ttccctgtcc agtctttgat tccaaaacca ttggaatctg tcaagtctgc tgatgaattc     780

ttggaaaaat tatctgatta cgataaagat ttgactcaat tgaagaagga agctgccact     840

gaaaataagg tattgagatt cattggtaaa gtcgatgttg ccaccaaatc tgtgtctgta     900

ggaattgaaa agtacgatta ctcacaccca ttcgcatcat tgaagggatc agataacgtt 960

atttccatca agactaagcg ttacaccaat cctgttgtca ttcaaggtgc cggtgccggt 1020

gctgccgtta ctgccgctgg tgttttgggt gatgttatca agattgctca aagactttag 1080

<210> 132
<211> 310
<212> PRT
<213> Escherichia coli

<220>
<221> MISC_FEATURE
<223> homoserine kinase

<400> 132

Met Val Lys Val Tyr Ala Pro Ala Ser Ser Ala Asn Met Ser Val Gly
1 5 10 15

Phe Asp Val Leu Gly Ala Ala Val Thr Pro Val Asp Gly Ala Leu Leu
20 25 30

Gly Asp Val Val Thr Val Glu Ala Ala Glu Thr Phe Ser Leu Asn Asn
35 40 45

Leu Gly Arg Phe Ala Asp Lys Leu Pro Ser Glu Pro Arg Glu Asn Ile
50 55 60

Val Tyr Gln Cys Trp Glu Arg Phe Cys Gln Glu Leu Gly Lys Gln Ile
65 70 75 80

Pro Val Ala Met Thr Leu Glu Lys Asn Met Pro Ile Gly Ser Gly Leu
85 90 95

Gly Ser Ser Ala Cys Ser Val Val Ala Ala Leu Met Ala Met Asn Glu
100 105 110

His Cys Gly Lys Pro Leu Asn Asp Thr Arg Leu Leu Ala Leu Met Gly
115 120 125

Glu Leu Glu Gly Arg Ile Ser Gly Ser Ile His Tyr Asp Asn Val Ala
130 135 140

Pro Cys Phe Leu Gly Gly Met Gln Leu Met Ile Glu Glu Asn Asp Ile
145 150 155 160

Ile Ser Gln Gln Val Pro Gly Phe Asp Glu Trp Leu Trp Val Leu Ala
165 170 175

```
Tyr Pro Gly Ile Lys Val Ser Thr Ala Glu Ala Arg Ala Ile Leu Pro
            180                 185                 190

Ala Gln Tyr Arg Arg Gln Asp Cys Ile Ala His Gly Arg His Leu Ala
        195                 200                 205

Gly Phe Ile His Ala Cys Tyr Ser Arg Gln Pro Glu Leu Ala Ala Lys
    210                 215                 220

Leu Met Lys Asp Val Ile Ala Glu Pro Tyr Arg Glu Arg Leu Leu Pro
225                 230                 235                 240

Gly Phe Arg Gln Ala Arg Gln Ala Val Ala Glu Ile Gly Ala Val Ala
                245                 250                 255

Ser Gly Ile Ser Gly Ser Gly Pro Thr Leu Phe Ala Leu Cys Asp Lys
            260                 265                 270

Pro Glu Thr Ala Gln Arg Val Ala Asp Trp Leu Gly Lys Asn Tyr Leu
        275                 280                 285

Gln Asn Gln Glu Gly Phe Val His Ile Cys Arg Leu Asp Thr Ala Gly
    290                 295                 300

Ala Arg Val Leu Glu Asn
305                 310
```

```
<210>  133
<211>  933
<212>  DNA
<213>  Escherichia coli

<220>
<221>  misc_feature
<223>  Encodes homoserine kinase

<400>  133
atggttaaag tttatgcccc ggcttccagt gccaatatga gcgtcgggtt tgatgtgctc      60

ggggcggcgg tgacacctgt tgatggtgca ttgctcggag atgtagtcac ggttgaggcg     120

gcagagacat tcagtctcaa caacctcgga cgctttgccg ataagctgcc gtcagaacca     180

cgggaaaata tcgtttatca gtgctgggag cgtttttgcc aggaactggg taagcaaatt     240

ccagtggcga tgaccctgga aaagaatatg ccgatcggtt cgggcttagg ctccagtgcc     300

tgttcggtgg tcgcggcgct gatggcgatg aatgaacact gcggcaagcc gcttaatgac     360

actcgtttgc tggctttgat gggcgagctg gaaggccgta tctccggcag cattcattac     420

gacaacgtgg caccgtgttt tctcggtggt atgcagttga tgatcgaaga aaacgacatc     480
```

atcagccagc aagtgccagg gtttgatgag tggctgtggg tgctggcgta tccggggatt 540

aaagtctcga cggcagaagc cagggctatt ttaccggcgc agtatcgccg ccaggattgc 600

attgcgcacg ggcgacatct ggcaggcttc attcacgcct gctattcccg tcagcctgag 660

cttgccgcga agctgatgaa agatgttatc gctgaaccct accgtgaacg gttactgcca 720

ggcttccggc aggcgcggca ggcggtcgcg gaaatcggcg cggtagcgag cggtatctcc 780

ggctccggcc cgaccttgtt cgctctgtgt gacaagccgg aaaccgccca gcgcgttgcc 840

gactggttgg gtaagaacta cctgcaaaat caggaaggtt ttgttcatat ttgccggctg 900

gatacggcgg gcgcacgagt actggaaaac taa 933

<210> 134
<211> 309
<212> PRT
<213> Bacillus subtilis

<220>
<221> MISC_FEATURE
<223> homoserine kinase

<400> 134

Met Asn Glu Ala Asp Met Leu Phe Ser Val Thr Val Pro Gly Ser Thr
1 5 10 15

Ala Asn Leu Gly Pro Gly Phe Asp Ser Val Gly Met Ala Leu Ser Arg
20 25 30

Tyr Leu Lys Leu Thr Val Phe Glu Ser Asp Lys Trp Ser Phe Glu Ala
35 40 45

Glu Thr Glu Thr Val Ala Gly Ile Pro Ala Gly Thr Asp Asn Leu Ile
50 55 60

Tyr Gln Val Ala Lys Arg Thr Ala Asp Leu Tyr Gly Lys Glu Met Pro
65 70 75 80

Pro Val His Val Lys Val Trp Ser Asp Ile Pro Leu Ala Arg Gly Leu
85 90 95

Gly Ser Ser Ala Ala Ala Ile Val Ala Ala Ile Glu Leu Ala Asp Glu
100 105 110

Leu Cys Gly Leu Lys Leu Ser Glu Ala Asp Lys Leu His Leu Ala Ser
115 120 125

Leu Glu Glu Gly His Pro Asp Asn Ala Gly Ala Ser Leu Val Gly Gly

```
    130                 135                 140

Leu Val Ile Gly Leu His Glu Asp Asp Glu Thr Gln Met Ile Arg Val
145                 150                 155                 160

Pro Asn Ala Asp Ile Asp Val Val Val Val Ile Pro Phe Tyr Glu Val
                165                 170                 175

Leu Thr Arg Asp Ala Arg Asp Val Leu Pro Lys Glu Phe Pro Tyr Ala
            180                 185                 190

Asp Ala Val Lys Ala Ser Ala Val Ser Asn Ile Leu Ile Ala Ala Ile
        195                 200                 205

Met Ser Lys Asp Trp Pro Leu Val Gly Lys Ile Met Lys Lys Asp Met
    210                 215                 220

Phe His Gln Pro Tyr Arg Ala Met Leu Val Pro Glu Leu Ser Lys Val
225                 230                 235                 240

Glu His Val Ala Glu Met Lys Gly Ala Tyr Gly Thr Ala Leu Ser Gly
                245                 250                 255

Ala Gly Pro Thr Ile Leu Val Met Thr Glu Lys Gly Lys Gly Glu Glu
            260                 265                 270

Leu Lys Glu Gln Leu Ala Leu His Phe Pro His Cys Glu Val Asp Ala
        275                 280                 285

Leu Thr Val Pro Lys Glu Gly Ser Ile Ile Glu Arg Asn Pro Leu Tyr
    290                 295                 300

Gln Val Lys Ser Val
305


<210>  135
<211>  930
<212>  DNA
<213>  Bacillus subtilis


<220>
<221>  misc_feature
<223>  Encodes homoserine kinase

<400>  135
ctatacactt tttacttgat ataaaggatt tcgctctatt atacttccct ctttcggaac      60

ggtcaaagcg tctacttcac aatgagggaa atgaagcgcg agctgttctt ttagctcttc     120

tccctttcct ttttcggtca tgacgagaat cgttgggcct gctccgctga gagccgttcc     180
```

atatgcgccc ttcatctcgg cgacgtgctc tacttttgac agctcaggca caagcatcgc 240

ccggtacggc tgatggaaca tatccttctt catgattttc ccgacaagcg gccaatcctt 300

ggacatgatc gcagcaatga ggatattgct gacagcactt gcttttacgg catcggcata 360

tggaaactcc ttcggaagca cgtctctcgc gtctcttgtc agcacttcat aaaaaggaat 420

gacaacgact acgtcaatgt cagcattcgg gacgcggatc atttgggtct cgtcatcctc 480

atgcaggccg atcacaagtc cgccgacgag agaagcgcca gcattgtccg ggtgtccttc 540

ttctagactc gctaaatgca gcttgtccgc ttcagacagc tttaagccgc ataattcatc 600

agccagttca atggccgcta caatcgctgc ggcgctgctg ccaaggccgc gtgcaagcgg 660

gatgtcgctc cacaccttca catggacagg aggcatttct tttccgtaca aatctgcggt 720

ccgtttagcc acttggtaga tcaggttatc tgtacccgcc ggaattccgg cgactgtttc 780

tgtttcagcc tcaaaagacc atttgtcgct ttcaaagacg gtcagcttca aatatctgct 840

gagcgccatt ccgactgaat caaagccggg gcctaggtta gctgtgcttc cgggaacagt 900

gacagagaac agcatgtcgg cttcgttcat 930

<210> 136
<211> 316
<212> PRT
<213> Pseudomonas putida

<220>
<221> MISC_FEATURE
<223> homoserine kinase

<400> 136

Met Ser Val Phe Thr Pro Val Thr Arg Pro Glu Leu Glu Thr Phe Leu
1 5 10 15

Ala Pro Tyr Glu Leu Gly Arg Leu Leu Asp Phe Gln Gly Ile Ala Ala
20 25 30

Gly Thr Glu Asn Ser Asn Phe Phe Val Ser Leu Glu Gln Gly Glu Phe
35 40 45

Val Leu Thr Leu Ile Glu Arg Gly Pro Ser Glu Asp Met Pro Phe Phe
50 55 60

Ile Glu Leu Leu Asp Thr Leu His Gly Ala Asp Met Pro Val Pro Tyr
65 70 75 80

Ala Ile Arg Asp Arg Asp Gly Asn Gly Leu Arg Glu Leu Cys Gly Lys
85 90 95

```
Pro Ala Leu Leu Gln Pro Arg Leu Ser Gly Lys His Ile Lys Ala Pro
            100                 105                 110

Asn Asn Gln His Cys Ala Gln Val Gly Glu Leu Leu Ala His Ile His
        115                 120                 125

Leu Ala Thr Arg Glu His Ile Ile Glu Arg Arg Thr Asp Arg Gly Leu
    130                 135                 140

Asp Trp Met Leu Ala Ser Gly Val Glu Leu Leu Pro Arg Leu Thr Ala
145                 150                 155                 160

Glu Gln Ala Ala Leu Leu Gln Pro Ala Leu Asp Glu Ile Ser Ala His
                165                 170                 175

Lys Ala Gln Ile Leu Ala Leu Pro Arg Ala Asn Leu His Ala Asp Leu
            180                 185                 190

Phe Arg Asp Asn Val Met Phe Glu Gly Thr His Leu Thr Gly Val Ile
        195                 200                 205

Asp Phe Tyr Asn Ala Cys Ser Gly Pro Met Leu Tyr Asp Ile Ala Ile
    210                 215                 220

Thr Val Asn Asp Trp Cys Leu Asp Glu Gln Gly Ala Val Asp Val Pro
225                 230                 235                 240

Arg Ala Gln Ala Leu Leu Ala Ala Tyr Ala Ala Leu Arg Pro Phe Thr
                245                 250                 255

Ala Ala Glu Ala Glu Leu Trp Pro Glu Met Leu Arg Val Gly Cys Val
            260                 265                 270

Arg Phe Trp Leu Ser Arg Leu Ile Ala Ala Glu Ser Phe Ala Gly Met
        275                 280                 285

Asp Val Met Ile His Asp Pro Ser Glu Phe Glu Val Arg Leu Ala Gln
    290                 295                 300

Arg Gln Gln Val Ala Leu His Leu Pro Phe Ala Leu
305                 310                 315
```

<210> 137
<211> 951
<212> DNA
<213> Pseudomonas putida

<220>
<221> misc_feature
<223> Encodes homoserine kinase

<400> 137
ctacagcgcg aacggcaggt gcaaagccac ctgctggcgc tgcgccagac gtacttcaaa 60

ctcgctcgga tcgtggatca tcacatccat cccggcaaac gattcggcgg caatcaggcg 120

cgacagccag aaacgcacgc agcctaccct caacatttcc ggccacagct cggcttcggc 180

tgcagtgaac ggccgtagcg ccgcataggc tgccagcagc gcctgggcgc gtggtacatc 240

caccgctccc tgctcatcca ggcaccagtc gttcacggtg atggcaatgt catacagcat 300

cggcccggag caggcattgt agaagtcgat cacaccggtc aggtgggtgc cttcgaacat 360

cacgttgtcg cggaacaggt cggcatgcag gttggcccgc ggcaaggcca ggatctgcgc 420

cttatgtgcg ctgatttcgt ccagcgccgg ctgcagcaat gcggcttgct ccgcagtcag 480

gcgtggcagt agctcgacac ccgaggccag catccagtcc aggccgcggt cggtgcggcg 540

ctcgatgatg tgctctcggg tggccaggtg aatgtgtgcc agtagctcgc cgacctgggc 600

gcagtgctgg ttgttgggtg ccttgatgtg tttgcccgac agcctgggct gcagcaatgc 660

cggcttgccg cacagctcgc gcaggccgtt gccgtcgcga tcacgaatgg catagggcac 720

cggcatgtcg gcaccgtgca gggtatcgag cagctcgatg aagaacggca tgtcctcgct 780

gggcccacgc tcgatcagcg tcaggacgaa ctccccctgt tcgaggctga cgaagaaatt 840

gctgttctcg gtaccggcgg caatgccctg gaagtcgagc agacggccca gctcgtacgg 900

cgccagaaag gtttccagct caggccgggt cacgggggtg aagactgaca t 951

<210> 138
<211> 357
<212> PRT
<213> Saccharomyces cerevisiae

<220>
<221> MISC_FEATURE
<223> homoserine kinase

<400> 138

Met Val Arg Ala Phe Lys Ile Lys Val Pro Ala Ser Ser Ala Asn Ile
1 5 10 15

Gly Pro Gly Tyr Asp Val Leu Gly Val Gly Leu Ser Leu Phe Leu Glu
20 25 30

Leu Asp Val Thr Ile Asp Ser Ser Gln Ala Gln Glu Thr Asn Asp Asp
35 40 45

Pro Asn Asn Cys Lys Leu Ser Tyr Thr Lys Glu Ser Glu Gly Tyr Ser

```
    50                  55                  60

Thr Val Pro Leu Arg Ser Asp Ala Asn Leu Ile Thr Arg Thr Ala Leu
65                  70                  75                  80

Tyr Val Leu Arg Cys Asn Asn Ile Arg Asn Phe Pro Ser Gly Thr Lys
                85                  90                  95

Val His Val Ser Asn Pro Ile Pro Leu Gly Arg Gly Leu Gly Ser Ser
            100                 105                 110

Gly Ala Ala Val Val Ala Gly Val Ile Leu Gly Asn Glu Val Ala Gln
        115                 120                 125

Leu Gly Phe Ser Lys Gln Arg Met Leu Asp Tyr Cys Leu Met Ile Glu
    130                 135                 140

Arg His Pro Asp Asn Ile Thr Ala Ala Met Met Gly Gly Phe Cys Gly
145                 150                 155                 160

Ser Phe Leu Arg Asp Leu Thr Pro Gln Glu Val Glu Arg Arg Glu Ile
                165                 170                 175

Pro Leu Ala Glu Val Leu Pro Glu Pro Ser Gly Gly Glu Asp Thr Gly
            180                 185                 190

Leu Val Pro Pro Leu Pro Pro Thr Asp Ile Gly Arg His Val Lys Tyr
        195                 200                 205

Gln Trp Asn Pro Ala Ile Lys Cys Ile Ala Ile Ile Pro Gln Phe Glu
    210                 215                 220

Leu Ser Thr Ala Asp Ser Arg Gly Val Leu Pro Lys Ala Tyr Pro Thr
225                 230                 235                 240

Gln Asp Leu Val Phe Asn Leu Gln Arg Leu Ala Val Leu Thr Thr Ala
                245                 250                 255

Leu Thr Met Asp Pro Pro Asn Ala Asp Leu Ile Tyr Pro Ala Met Gln
            260                 265                 270

Asp Arg Val His Gln Pro Tyr Arg Lys Thr Leu Ile Pro Gly Leu Thr
        275                 280                 285

Glu Ile Leu Ser Cys Val Thr Pro Ser Thr Tyr Pro Gly Leu Leu Gly
    290                 295                 300
```

Ile Cys Leu Ser Gly Ala Gly Pro Thr Ile Leu Ala Leu Ala Thr Glu
305                 310                 315                 320

Asn Phe Glu Glu Ile Ser Gln Glu Ile Ile Asn Arg Phe Ala Lys Asn
                325                 330                 335

Gly Ile Lys Cys Ser Trp Lys Leu Leu Glu Pro Ala Tyr Asp Gly Ala
            340                 345                 350

Ser Val Glu Gln Gln
        355

<210> 139
<211> 1074
<212> DNA
<213> Saccharomyces cerevisiae

<220>
<221> misc_feature
<223> Encodes homoserine kinase

<400> 139
atggttcgtg ccttcaaaat taaagttcca gcttcttccg ccaatatcgg ccctggttat 60

gatgttcttg gtgtcggtct ttctctattc ttggagttag atgtcaccat cgactccagc 120

caagctcagg aaacaaacga cgatcccaac aactgcaagc tgtcttacac taaagaaagt 180

gaaggctatt ctacggtccc attgcgttct gatgctaacc tgattaccag gactgcgtta 240

tatgtgttgc gttgcaacaa tattagaaac ttcccctccg gaaccaaagt tcacgtctcc 300

aacccaatcc cacttggccg tggattgggt tcctctggtg cagcagttgt ggcaggtgtt 360

attttaggta acgaagtggc ccaattgggt ttctctaagc aacgtatgtt ggactactgt 420

ttgatgattg aacgtcatcc agacaacata accgctgcta tgatgggagg cttttgcggt 480

tcattcctaa gggacttgac cccacaagag gtggaaagac gtgagattcc attggctgag 540

gtgcttccag aaccttctgg tggtgaagat accggtctgg ttcccccatt acctcccacc 600

gatatcggta gacatgtcaa ataccaatgg aaccccgcca ttaaatgtat tgcgatcatc 660

ccacagttcg agttgtccac cgccgactcc agaggcgttc ttccaaaagc ctacccaacc 720

caggacttgg ttttcaatct acaaagattg gccgtcttga ccacagcttt gaccatggac 780

ccacctaatg ccgacttaat ctaccctgct atgcaagatc gtgtccacca accttataga 840

aagacattga tcccaggtct cacggaaatc ttatcatgtg tcaccccatc cacataccct 900

ggcctattgg gtatctgctt gtcaggtgca ggcccaacta tcttggcttt ggccactgag 960

aatttcgaag aaatctctca agaaattatc aacaggttcg ccaaaaacgg catcaagtgc 1020

tcctggaaac tactggagcc tgcctacgat ggtgctagcg tcgaacagca atga 1074

<210> 140
<211> 428
<212> PRT
<213> Escherichia coli

<220>
<221> MISC_FEATURE
<223> threonine synthase

<400> 140

```
Met Lys Leu Tyr Asn Leu Lys Asp His Asn Glu Gln Val Ser Phe Ala
1               5                   10                  15

Gln Ala Val Thr Gln Gly Leu Gly Lys Asn Gln Gly Leu Phe Phe Pro
            20                  25                  30

His Asp Leu Pro Glu Phe Ser Leu Thr Glu Ile Asp Glu Met Leu Lys
        35                  40                  45

Leu Asp Phe Val Thr Arg Ser Ala Lys Ile Leu Ser Ala Phe Ile Gly
    50                  55                  60

Asp Glu Ile Pro Gln Glu Ile Leu Glu Glu Arg Val Arg Ala Ala Phe
65                  70                  75                  80

Ala Phe Pro Ala Pro Val Ala Asn Val Glu Ser Asp Val Gly Cys Leu
                85                  90                  95

Glu Leu Phe His Gly Pro Thr Leu Ala Phe Lys Asp Phe Gly Gly Arg
            100                 105                 110

Phe Met Ala Gln Met Leu Thr His Ile Ala Gly Asp Lys Pro Val Thr
        115                 120                 125

Ile Leu Thr Ala Thr Ser Gly Asp Thr Gly Ala Ala Val Ala His Ala
    130                 135                 140

Phe Tyr Gly Leu Pro Asn Val Lys Val Val Ile Leu Tyr Pro Arg Gly
145                 150                 155                 160

Lys Ile Ser Pro Leu Gln Glu Lys Leu Phe Cys Thr Leu Gly Gly Asn
                165                 170                 175

Ile Glu Thr Val Ala Ile Asp Gly Asp Phe Asp Ala Cys Gln Ala Leu
            180                 185                 190

Val Lys Gln Ala Phe Asp Asp Glu Glu Leu Lys Val Ala Leu Gly Leu
        195                 200                 205
```

```
Asn Ser Ala Asn Ser Ile Asn Ile Ser Arg Leu Leu Ala Gln Ile Cys
    210                 215                 220

Tyr Tyr Phe Glu Ala Val Ala Gln Leu Pro Gln Glu Thr Arg Asn Gln
225                 230                 235                 240

Leu Val Val Ser Val Pro Ser Gly Asn Phe Gly Asp Leu Thr Ala Gly
                245                 250                 255

Leu Leu Ala Lys Ser Leu Gly Leu Pro Val Lys Arg Phe Ile Ala Ala
            260                 265                 270

Thr Asn Val Asn Asp Thr Val Pro Arg Phe Leu His Asp Gly Gln Trp
        275                 280                 285

Ser Pro Lys Ala Thr Gln Ala Thr Leu Ser Asn Ala Met Asp Val Ser
    290                 295                 300

Gln Pro Asn Asn Trp Pro Arg Val Glu Glu Leu Phe Arg Arg Lys Ile
305                 310                 315                 320

Trp Gln Leu Lys Glu Leu Gly Tyr Ala Ala Val Asp Asp Glu Thr Thr
                325                 330                 335

Gln Gln Thr Met Arg Glu Leu Lys Glu Leu Gly Tyr Thr Ser Glu Pro
            340                 345                 350

His Ala Ala Val Ala Tyr Arg Ala Leu Arg Asp Gln Leu Asn Pro Gly
        355                 360                 365

Glu Tyr Gly Leu Phe Leu Gly Thr Ala His Pro Ala Lys Phe Lys Glu
    370                 375                 380

Ser Val Glu Ala Ile Leu Gly Glu Thr Leu Asp Leu Pro Lys Glu Leu
385                 390                 395                 400

Ala Glu Arg Ala Asp Leu Pro Leu Leu Ser His Asn Leu Pro Ala Asp
                405                 410                 415

Phe Ala Ala Leu Arg Lys Leu Met Met Asn His Gln
            420                 425
```

<210> 141
<211> 1287
<212> DNA
<213> Escherichia coli

<220>
<221> misc_feature
<223> Encodes threonine synthase

<400> 141
atgaaactct acaatctgaa agatcacaac gagcaggtca gctttgcgca agccgtaacc 60

caggggttgg gcaaaaatca ggggctgttt tttccgcacg acctgccgga attcagcctg 120

actgaaattg atgagatgct gaagctggat tttgtcaccc gcagtgcgaa gatcctctcg 180

gcgtttattg gtgatgaaat cccacaggaa atcctggaag agcgcgtgcg cgcggcgttt 240

gccttcccgg ctccggtcgc caatgttgaa agcgatgtcg gttgtctgga attgttccac 300

gggccaacgc tggcatttaa agatttcggc ggtcgcttta tggcacaaat gctgacccat 360

attgcgggtg ataagccagt gaccattctg accgcgacct ccggtgatac cggagcggca 420

gtggctcatg ctttctacgg tttaccgaat gtgaaagtgg ttatcctcta tccacgaggc 480

aaaatcagtc cactgcaaga aaaactgttc tgtacattgg gcggcaatat cgaaactgtt 540

gccatcgacg gcgatttcga tgcctgtcag gcgctggtga agcaggcgtt tgatgatgaa 600

gaactgaaag tggcgctagg gttaaactcg gctaactcga ttaacatcag ccgtttgctg 660

gcgcagattt gctactactt tgaagctgtt gcgcagctgc cgcaggagac gcgcaaccag 720

ctggttgtct cggtgccaag cggaaacttc ggcgatttga cggcgggtct gctggcgaag 780

tcactcggtc tgccggtgaa acgttttatt gctgcgacca acgtgaacga taccgtgcca 840

cgtttcctgc acgacggtca gtggtcaccc aaagcgactc aggcgacgtt atccaacgcg 900

atggacgtga gtcagccgaa caactggccg cgtgtggaag agttgttccg ccgcaaaatc 960

tggcaactga aagagctggg ttatgcagcc gtggatgatg aaaccacgca acagacaatg 1020

cgtgagttaa aagaactggg ctacacttcg gagccgcacg ctgccgtagc ttatcgtgcg 1080

ctgcgtgatc agttgaatcc aggcgaatat ggcttgttcc tcggcaccgc gcatccggcg 1140

aaatttaaag agagcgtgga agcgattctc ggtgaaacgt tggatctgcc aaaagagctg 1200

gcagaacgtg ctgatttacc cttgctttca cataatctgc ccgccgattt tgctgcgttg 1260

cgtaaattga tgatgaatca tcagtaa 1287

<210> 142
<211> 352
<212> PRT
<213> Bacillus subtilis

<220>
<221> MISC_FEATURE
<223> threonine synthase

<400> 142

```
Met Trp Lys Gly Leu Ile His Gln Tyr Lys Glu Phe Leu Pro Val Thr
1               5                   10                  15

Asp Gln Thr Pro Ala Leu Thr Leu His Glu Gly Asn Thr Pro Leu Ile
            20                  25                  30

His Leu Pro Lys Leu Ser Glu Gln Leu Gly Ile Glu Leu His Val Lys
        35                  40                  45

Thr Glu Gly Val Asn Pro Thr Gly Ser Phe Lys Asp Arg Gly Met Val
    50                  55                  60

Met Ala Val Ala Lys Ala Lys Glu Glu Gly Asn Asp Thr Ile Met Cys
65                  70                  75                  80

Ala Ser Thr Gly Asn Thr Ser Ala Ala Ala Ala Ala Tyr Ala Ala Arg
                85                  90                  95

Ala Asn Met Lys Cys Ile Val Ile Ile Pro Asn Gly Lys Ile Ala Phe
            100                 105                 110

Gly Lys Leu Ala Gln Ala Val Met Tyr Gly Ala Glu Ile Ile Ala Ile
        115                 120                 125

Asp Gly Asn Phe Asp Asp Ala Leu Lys Ile Val Arg Ser Ile Cys Glu
    130                 135                 140

Lys Ser Pro Ile Ala Leu Val Asn Ser Val Asn Pro Tyr Arg Ile Glu
145                 150                 155                 160

Gly Gln Lys Thr Ala Ala Phe Glu Val Cys Glu Gln Leu Gly Glu Ala
                165                 170                 175

Pro Asp Val Leu Ala Ile Pro Val Gly Asn Ala Gly Asn Ile Thr Ala
            180                 185                 190

Tyr Trp Lys Gly Phe Lys Glu Tyr His Glu Lys Asn Gly Thr Gly Leu
        195                 200                 205

Pro Lys Met Arg Gly Phe Glu Ala Glu Gly Ala Ala Ala Ile Val Arg
    210                 215                 220

Asn Glu Val Ile Glu Asn Pro Glu Thr Ile Ala Thr Ala Ile Arg Ile
225                 230                 235                 240

Gly Asn Pro Ala Ser Trp Asp Lys Ala Val Lys Ala Ala Glu Glu Ser
                245                 250                 255
```

Asn Gly Lys Ile Asp Glu Val Thr Asp Asp Glu Ile Leu His Ala Tyr
260 265 270

Gln Leu Ile Ala Arg Val Glu Gly Val Phe Ala Glu Pro Gly Ser Cys
275 280 285

Ala Ser Ile Ala Gly Val Leu Lys Gln Val Lys Ser Gly Glu Ile Pro
290 295 300

Lys Gly Ser Lys Val Val Ala Val Leu Thr Gly Asn Gly Leu Lys Asp
305 310 315 320

Pro Asn Thr Ala Val Asp Ile Ser Glu Ile Lys Pro Val Thr Leu Pro
325 330 335

Thr Asp Glu Asp Ser Ile Leu Glu Tyr Val Lys Gly Ala Ala Arg Val
340 345 350

<210> 143
<211> 1059
<212> DNA
<213> Bacillus subtilis

<220>
<221> misc_feature
<223> Encodes threonine synthase

<400> 143
tcatacacgg gccgctcctt ttacatattc aaggatgctg tcttcatcag tcggcaatgt 60

gacaggcttg atttctgaaa tgtcgaccgc tgtgttcgga tctttcagtc cgtttcctgt 120

taacacagct acgaccttgc tgcctttcgg aatttctccg gatttcacct gtttcagcac 180

tcctgcgata gacgcgcaag aacctggttc tgcaaacacg ccttctacac gggcgatcag 240

ctgatatgcg tgaaggattt catcatcagt gacttcgtca attttcccat tggattcctc 300

ggctgccttt acagctttgt cccagcttgc cgggtttccg atacgaatgg ctgtcgctat 360

tgtttccgga ttttcaatca cttcattgcg cacgattgcc gccgcaccct cagcttcaaa 420

gccgcgcatt ttcggaaggc ctgtgccgtt tttctcatga tattccttga agcccttcca 480

gtacgcagtg atgtttccag catttccgac cgggatcgct aaaacatcag gcgcttcccc 540

cagctgttcg cacacttcga aggcagcagt tttttggcct tcaatgcggt aagggttgac 600

tgagttgaca agggcaatcg gtgatttctc acagatggaa cggacaattt taagcgcatc 660

gtcaaagttt ccgtcaattg cgataatctc ggctccgtac atgacagctt gagcgagttt 720

tccaaatgca atttttccgt tcgggatgat gacaatgcat ttcatgttag cacgggctgc 780

atatgctgcc gcagcagcgg aagtgttacc tgttgacgcg cacataatcg tgtcattgcc 840

ttcttctttt gcctttgcca cagccataac cattccgcga tctttaaatg atcccgtagg 900

attgacgcct tccgttttga catgaagctc aattccgagc tgctcagaca gcttcggcag 960

gtgaataaga ggtgtgtttc cttcatgtaa agttagcgcc ggtgtttgat ctgttacagg 1020

taaaaattct ttatattgat ggataagtcc tttccacat 1059

<210> 144
<211> 481
<212> PRT
<213> Corynebacterium glutamicum

<220>
<221> MISC_FEATURE
<223> threonine synthase

<400> 144

Met Asp Tyr Ile Ser Thr Arg Asp Ala Ser Arg Thr Pro Ala Arg Phe
1 5 10 15

Ser Asp Ile Leu Leu Gly Gly Leu Ala Pro Asp Gly Gly Leu Tyr Leu
20 25 30

Pro Ala Thr Tyr Pro Gln Leu Asp Asp Ala Gln Leu Ser Lys Trp Arg
35 40 45

Glu Val Leu Ala Asn Glu Gly Tyr Ala Ala Leu Ala Ala Glu Val Ile
50 55 60

Ser Leu Phe Val Asp Asp Ile Pro Val Glu Asp Ile Lys Ala Ile Thr
65 70 75 80

Ala Arg Ala Tyr Thr Tyr Pro Lys Phe Asn Ser Glu Asp Ile Val Pro
85 90 95

Val Thr Glu Leu Glu Asp Asn Ile Tyr Leu Gly His Leu Ser Glu Gly
100 105 110

Pro Thr Ala Ala Phe Lys Asp Met Ala Met Gln Leu Leu Gly Glu Leu
115 120 125

Phe Glu Tyr Glu Leu Arg Arg Arg Asn Glu Thr Ile Asn Ile Leu Gly
130 135 140

Ala Thr Ser Gly Asp Thr Gly Ser Ser Ala Glu Tyr Ala Met Arg Gly
145 150 155 160

Arg Glu Gly Ile Arg Val Phe Met Leu Thr Pro Ala Gly Arg Met Thr

165 170 175

Pro Phe Gln Gln Ala Gln Met Phe Gly Leu Asp Asp Pro Asn Ile Phe
180 185 190

Asn Ile Ala Leu Asp Gly Val Phe Asp Asp Cys Gln Asp Val Val Lys
195 200 205

Ala Val Ser Ala Asp Ala Glu Phe Lys Lys Asp Asn Arg Ile Gly Ala
210 215 220

Val Asn Ser Ile Asn Trp Ala Arg Leu Met Ala Gln Val Val Tyr Tyr
225 230 235 240

Val Ser Ser Trp Ile Arg Thr Thr Thr Ser Asn Asp Gln Lys Val Ser
245 250 255

Phe Ser Val Pro Thr Gly Asn Phe Gly Asp Ile Cys Ala Gly His Ile
260 265 270

Ala Arg Gln Met Gly Leu Pro Ile Asp Arg Leu Ile Val Ala Thr Asn
275 280 285

Glu Asn Asp Val Leu Asp Glu Phe Phe Arg Thr Gly Asp Tyr Arg Val
290 295 300

Arg Ser Ser Ala Asp Thr His Glu Thr Ser Ser Pro Ser Met Asp Ile
305 310 315 320

Ser Arg Ala Ser Asn Phe Glu Arg Phe Ile Phe Asp Leu Leu Gly Arg
325 330 335

Asp Ala Thr Arg Val Asn Asp Leu Phe Gly Thr Gln Val Arg Gln Gly
340 345 350

Gly Phe Ser Leu Ala Asp Asp Ala Asn Phe Glu Lys Ala Ala Ala Glu
355 360 365

Tyr Gly Phe Ala Ser Gly Arg Ser Thr His Ala Asp Arg Val Ala Thr
370 375 380

Ile Ala Asp Val His Ser Arg Leu Asp Val Leu Ile Asp Pro His Thr
385 390 395 400

Ala Asp Gly Val His Val Ala Arg Gln Trp Arg Asp Glu Val Asn Thr
405 410 415

Pro Ile Ile Val Leu Glu Thr Ala Leu Pro Val Lys Phe Ala Asp Thr
420 425 430

Ile Val Glu Ala Ile Gly Glu Ala Pro Gln Thr Pro Glu Arg Phe Ala
435 440 445

Ala Ile Met Asp Ala Pro Phe Lys Val Ser Asp Leu Pro Asn Asp Thr
450 455 460

Asp Ala Val Lys Gln Tyr Ile Val Asp Ala Ile Ala Asn Thr Ser Val
465 470 475 480

Lys

<210> 145
<211> 1446
<212> DNA
<213> Corynebacterium glutamicum

<220>
<221> misc_feature
<223> Encodes threonine synthase

<400> 145
ttacttcacg gaagtgtttg caatcgcatc gactatgtac tgcttaactg catcggtgtc 60

gtttggtagg tcggaaacct tgaatggagc atccatgatc gcggcgaaac gctctggagt 120

ttgaggtgct tcaccaattg cttcgacgat ggtgtcggca aatttcactg ggagtgcagt 180

ttctaggacg atgattgggg tgttgacctc gtccctccac tggcgtgcca cgtgaacgcc 240

gtcggcggtg tggggatcga ttagtacgtc gaggcgggaa tgcacgtcag cgatggttgc 300

cacacggtca gcatgggtgg atcgtccgga ggcgaaaccg tattctgctg cagccttctc 360

aaagttggcg tcatcagcca gtgagaatcc gccttggcga acctgggtac caaatagatc 420

gttgacgcgg gtggcgtcgc ggccgagcag gtcgaagatg aaacgctcga agttggaggc 480

gcgggagata tccatcgaag gtgaggaggt ctcgtgggtg tctgcggagc tgcggactcg 540

gtagtcgccg gtacggaaga actcgtcgag cacatcgttt tcgttggtgg ccacgatgag 600

gcgatcgatg ggaagtccca tttggcgggc gatgtggcct gcgcaaatgt caccgaagtt 660

gccggttggt acggagaagc tgaccttttg gtcattgctg gttgtggtgc ggatccatga 720

ggaaacgtag tacacaacct gtgccataag gcgtgcccag ttgatggagt tcacggcacc 780

gatgcggttg tctttttga attctgcgtc ggcggagaca gccttgacta cgtcttggca 840

atcgtcgaaa acgccgtcga gggcgatgtt gaagatgttt ggatcgtcaa ggccaaacat 900

ctgtgcttgc tggaatgggg tcatgcggcc agctggggtc agcatgaata cgcggattcc 960

ctcgcggccg cgcatggcgt attccgcaga ggagccggta tcgccagagg tagcgcccag 1020

gatgttgatg gtttcgttgc ggcggcgaag ctcgtattcg aaaagttcgc cgagcagctg 1080

catggccatg tctttgaatg cagcggttgg gccttcggaa aggtggccca ggtaaatgtt 1140

gtcctcgagt tcggtgacag gaacgatgtc ttcgctgttg aacttcgggt aggtgtaggc 1200

gcgtgcggtg atcgccttga tgtcttctac tgggatgtca tcaacaaaca gggagataac 1260

ttcagcagcc aaagctgcgt atccttcgtt ggctaatacc tcacgccatt tactcagctg 1320

ggcatcatct agttgagggt aggttgcagg caggtacagg ccgccgtctg gtgctagacc 1380

gcccagcaaa atatcactga agcgggcagg ggtacggctg gcatcacgcg tcgaaatgta 1440

gtccac 1446

<210> 146
<211> 329
<212> PRT
<213> Escherichia coli

<220>
<221> MISC_FEATURE
<223> threonine deaminase (TdcB)

<400> 146

Met His Ile Thr Tyr Asp Leu Pro Val Ala Ile Asp Asp Ile Ile Glu
1 5 10 15

Ala Lys Gln Arg Leu Ala Gly Arg Ile Tyr Lys Thr Gly Met Pro Arg
20 25 30

Ser Asn Tyr Phe Ser Glu Arg Cys Lys Gly Glu Ile Phe Leu Lys Phe
35 40 45

Glu Asn Met Gln Arg Thr Gly Ser Phe Lys Ile Arg Gly Ala Phe Asn
50 55 60

Lys Leu Ser Ser Leu Thr Asp Ala Glu Lys Arg Lys Gly Val Val Ala
65 70 75 80

Cys Ser Ala Gly Asn His Ala Gln Gly Val Ser Leu Ser Cys Ala Met
85 90 95

Leu Gly Ile Asp Gly Lys Val Val Met Pro Lys Gly Ala Pro Lys Ser
100 105 110

Lys Val Ala Ala Thr Cys Asp Tyr Ser Ala Glu Val Val Leu His Gly
115 120 125

```
Asp Asn Phe Asn Asp Thr Ile Ala Lys Val Ser Glu Ile Val Glu Met
    130                 135                 140

Glu Gly Arg Ile Phe Ile Pro Pro Tyr Asp Asp Pro Lys Val Ile Ala
145                 150                 155                 160

Gly Gln Gly Thr Ile Gly Leu Glu Ile Met Glu Asp Leu Tyr Asp Val
                165                 170                 175

Asp Asn Val Ile Val Pro Ile Gly Gly Gly Gly Leu Ile Ala Gly Ile
            180                 185                 190

Ala Val Ala Ile Lys Ser Ile Asn Pro Thr Ile Arg Val Ile Gly Val
        195                 200                 205

Gln Ser Glu Asn Val His Gly Met Ala Ala Ser Phe His Ser Gly Glu
    210                 215                 220

Ile Thr Thr His Arg Thr Thr Gly Thr Leu Ala Asp Gly Cys Asp Val
225                 230                 235                 240

Ser Arg Pro Gly Asn Leu Thr Tyr Glu Ile Val Arg Glu Leu Val Asp
                245                 250                 255

Asp Ile Val Leu Val Ser Glu Asp Glu Ile Arg Asn Ser Met Ile Ala
            260                 265                 270

Leu Ile Gln Arg Asn Lys Val Val Thr Glu Gly Ala Gly Ala Leu Ala
        275                 280                 285

Cys Ala Ala Leu Leu Ser Gly Lys Leu Asp Gln Tyr Ile Gln Asn Arg
    290                 295                 300

Lys Thr Val Ser Ile Ile Ser Gly Gly Asn Ile Asp Leu Ser Arg Val
305                 310                 315                 320

Ser Gln Ile Thr Gly Phe Val Asp Ala
                325
```

<210> 147
<211> 990
<212> DNA
<213> Escherichia coli

<220>
<221> misc_feature
<223> Encodes threonine deaminase (TdcB)

<400> 147

ttaagcgtca acgaaaccgg tgatttgaga gacgcgagaa agatcgatat tgccgccgga 60

aataatactg acggtttttc tgttttgaat atattggtct aatttaccgc ttaataatgc 120

agcacatgcc agagcgcctg cgccttcggt gacgacttta ttgcgctgaa ttaaggcaat 180

catactgttt ctgatttcgt cttcgctgac cagcacgatg tcatcgacta attcacgaac 240

gatttcgtaa gttaaattac ccgggcggga gacatcacaa ccatccgcca gggtgccggt 300

agttcggtgc gtggttattt ctccggagtg gaaagaagcc gccatgccgt gaacgttttc 360

agactgtacg ccaataacac gaatggtcgg gttaatagat ttaattgcca ccgcaatacc 420

agcaattaaa ccgccaccac caattggcac aatcacgtta tcgacatcat agagatcttc 480

cataatttcc agaccaatcg ttccctggcc agcaatcact ttcggatcat cgtaaggtgg 540

gataaaaata cggccttcca tttcgacaat ttcgctcact ttagcgatag tgtcgttgaa 600

gttatcacca tgcagaacga cttctgcgga gtagtcgcac gttgccgcta ctttggattt 660

tggcgcacct tttggcatca ccactttacc gtcgataccc agcatcgcgc aggagaggga 720

aacccccttgc gcatggttgc ccgcagaaca ggccaccacg cctttgcgtt tttccgcatc 780

ggtcagtgaa cttaatttat taaatgcgcc acgaatttta aatgaacccg tacgctgcat 840

attttcaaac ttcaggaata tttcaccttt gcaacgttca ctaaaatagt tggagcgagg 900

catgcctgtt ttataaattc gcccagccag tcgttgtttc gcttcaataa tgtcatcaat 960

agcaaccggc agatcgtatg taatatgcat 990

<210> 148
<211> 514
<212> PRT
<213> Escherichia coli

<220>
<221> MISC_FEATURE
<223> threonine deaminase (IlvA)

<400> 148

Met Ala Asp Ser Gln Pro Leu Ser Gly Ala Pro Glu Gly Ala Glu Tyr
1               5                   10                  15

Leu Arg Ala Val Leu Arg Ala Pro Val Tyr Glu Ala Ala Gln Val Thr
            20                  25                  30

Pro Leu Gln Lys Met Glu Lys Leu Ser Ser Arg Leu Asp Asn Val Ile
        35                  40                  45

Leu Val Lys Arg Glu Asp Arg Gln Pro Val His Ser Phe Lys Leu Arg
    50                  55                  60

```
Gly Ala Tyr Ala Met Met Ala Gly Leu Thr Glu Glu Gln Lys Ala His
65                  70                  75                  80

Gly Val Ile Thr Ala Ser Ala Gly Asn His Ala Gln Gly Val Ala Phe
                85                  90                  95

Ser Ser Ala Arg Leu Gly Val Lys Ala Leu Ile Val Met Pro Thr Ala
            100                 105                 110

Thr Ala Asp Ile Lys Val Asp Ala Val Arg Gly Phe Gly Gly Glu Val
        115                 120                 125

Leu Leu His Gly Ala Asn Phe Asp Glu Ala Lys Ala Lys Ala Ile Glu
    130                 135                 140

Leu Ser Gln Gln Gln Gly Phe Thr Trp Val Pro Pro Phe Asp His Pro
145                 150                 155                 160

Met Val Ile Ala Gly Gln Gly Thr Leu Ala Leu Glu Leu Leu Gln Gln
                165                 170                 175

Asp Ala His Leu Asp Arg Val Phe Val Pro Val Gly Gly Gly Gly Leu
            180                 185                 190

Ala Ala Gly Val Ala Val Leu Ile Lys Gln Leu Met Pro Gln Ile Lys
        195                 200                 205

Val Ile Ala Val Glu Ala Glu Asp Ser Ala Cys Leu Lys Ala Ala Leu
    210                 215                 220

Asp Ala Gly His Pro Val Asp Leu Pro Arg Val Gly Leu Phe Ala Glu
225                 230                 235                 240

Gly Val Ala Val Lys Arg Ile Gly Asp Glu Thr Phe Arg Leu Cys Gln
                245                 250                 255

Glu Tyr Leu Asp Asp Ile Ile Thr Val Asp Ser Asp Ala Ile Cys Ala
            260                 265                 270

Ala Met Lys Asp Leu Phe Glu Asp Val Arg Ala Val Ala Glu Pro Ser
        275                 280                 285

Gly Ala Leu Ala Leu Ala Gly Met Lys Lys Tyr Ile Ala Leu His Asn
    290                 295                 300

Ile Arg Gly Glu Arg Leu Ala His Ile Leu Ser Gly Ala Asn Val Asn
305                 310                 315                 320
```

```
Phe His Gly Leu Arg Tyr Val Ser Glu Arg Cys Glu Leu Gly Glu Gln
                325                 330                 335

Arg Glu Ala Leu Leu Ala Val Thr Ile Pro Glu Glu Lys Gly Ser Phe
            340                 345                 350

Leu Lys Phe Cys Gln Leu Leu Gly Gly Arg Ser Val Thr Glu Phe Asn
        355                 360                 365

Tyr Arg Phe Ala Asp Ala Lys Asn Ala Cys Ile Phe Val Gly Val Arg
    370                 375                 380

Leu Ser Arg Gly Leu Glu Glu Arg Lys Glu Ile Leu Gln Met Leu Asn
385                 390                 395                 400

Asp Gly Gly Tyr Ser Val Val Asp Leu Ser Asp Asp Glu Met Ala Lys
                405                 410                 415

Leu His Val Arg Tyr Met Val Gly Gly Arg Pro Ser His Pro Leu Gln
            420                 425                 430

Glu Arg Leu Tyr Ser Phe Glu Phe Pro Glu Ser Pro Gly Ala Leu Leu
        435                 440                 445

Arg Phe Leu Asn Thr Leu Gly Thr Tyr Trp Asn Ile Ser Leu Phe His
    450                 455                 460

Tyr Arg Ser His Gly Thr Asp Tyr Gly Arg Val Leu Ala Ala Phe Glu
465                 470                 475                 480

Leu Gly Asp His Glu Pro Asp Phe Glu Thr Arg Leu Asn Glu Leu Gly
                485                 490                 495

Tyr Asp Cys His Asp Glu Thr Asn Asn Pro Ala Phe Arg Phe Phe Leu
            500                 505                 510

Ala Gly


<210>  149
<211>  1545
<212>  DNA
<213>  Escherichia coli


<220>
<221>  misc_feature
<223>  Encodes threonine deaminase (ilvA)
```

<400> 149
atggctgact cgcaacccct gtccggtgct ccggaaggtg ccgaatattt aagagcagtg 60
ctgcgcgcgc cggtttacga ggcggcgcag gttacgccgc tacaaaaaat ggaaaaactg 120
tcgtcgcgtc ttgataacgt cattctggtg aagcgcgaag atcgccagcc agtgcacagc 180
tttaagctgc gcggcgcata cgccatgatg gcgggcctga cggaagaaca gaaagcgcac 240
ggcgtgatca ctgcttctgc gggtaaccac gcgcagggcg tcgcgttttc ttctgcgcgg 300
ttaggcgtga aggccctgat cgttatgcca accgccaccg ccgacatcaa agtcgacgcg 360
gtgcgcggct tcggcggcga agtgctgctc cacggcgcga actttgatga agcgaaagcc 420
aaagcgatcg aactgtcaca gcagcagggg ttcacctggg tgccgccgtt cgaccatccg 480
atggtgattg ccgggcaagg cacgctggcg ctggaactgc tccagcagga cgcccatctc 540
gaccgcgtat ttgtgccagt cggcggcggc ggtctggctg ctggcgtggc ggtgctgatc 600
aaacaactga tgccgcaaat caaagtgatc gccgtagaag cggaagactc cgcctgcctg 660
aaagcagcgc tggatgcggg tcatccggtt gatctgccgc gcgtagggct atttgctgaa 720
ggcgtagcgg taaaacgcat cggtgacgaa accttccgtt tatgccagga gtatctcgac 780
gacatcatca ccgtcgatag cgatgcgatc tgtgcggcga tgaaggattt attcgaagat 840
gtgcgcgcgg tggcggaacc ctctggcgcg ctggcgctgg cgggaatgaa aaaatatatc 900
gccctgcaca acattcgcgg cgaacggctg gcgcatattc tttccggtgc caacgtgaac 960
ttccacggcc tgcgctacgt ctcagaacgc tgcgaactgg gcgaacagcg tgaagcgttg 1020
ttggcggtga ccattccgga agaaaaaggc agcttcctca aattctgcca actgcttggc 1080
gggcgttcgg tcaccgagtt caactaccgt tttgccgatg ccaaaaacgc ctgcatcttt 1140
gtcggtgtgc gcctgagccg cggcctcgaa gagcgcaaag aaattttgca gatgctcaac 1200
gacggcggct acagcgtggt tgatctctcc gacgacgaaa tggcgaagct acacgtgcgc 1260
tatatggtcg gcggacgtcc atcgcatccg ttgcaggaac gcctctacag cttcgaattc 1320
ccggaatcac cgggcgcgct gctgcgcttc ctcaacacgc tgggtacgta ctggaacatt 1380
tctttgttcc actatcgcag ccatggcacc gactacgggc gcgtactggc ggcgttcgaa 1440
cttggcgacc atgaaccgga tttcgaaacc cggctgaatg agctgggcta cgattgccac 1500
gacgaaacca ataacccggc gttcaggttc tttttggcgg gttag 1545

<210> 150
<211> 422
<212> PRT
<213> Bacillus subtilis

<220>
<221> MISC_FEATURE
<223> threonine deaminase (IlvA)

<400> 150

Met Lys Pro Leu Leu Lys Glu Asn Ser Leu Ile Gln Val Lys Asp Ile
1               5                   10                  15

Leu Lys Ala His Gln Asn Val Lys Asp Val Val Ile His Thr Pro Leu
            20                  25                  30

Gln Arg Asn Asp Arg Leu Ser Glu Arg Tyr Glu Cys Asn Ile Tyr Leu
        35                  40                  45

Lys Arg Glu Asp Leu Gln Val Val Arg Ser Phe Lys Leu Arg Gly Ala
    50                  55                  60

Tyr His Lys Met Lys Gln Leu Ser Ser Glu Gln Thr Glu Asn Gly Val
65                  70                  75                  80

Val Cys Ala Ser Ala Gly Asn His Ala Gln Gly Val Ala Phe Ser Cys
                85                  90                  95

Lys His Leu Gly Ile His Gly Lys Ile Phe Met Pro Ser Thr Thr Pro
            100                 105                 110

Arg Gln Lys Val Ser Gln Val Glu Leu Phe Gly Lys Gly Phe Ile Asp
        115                 120                 125

Ile Ile Leu Thr Gly Asp Thr Phe Asp Asp Ala Tyr Lys Ser Ala Ala
    130                 135                 140

Glu Cys Cys Glu Ala Glu Ser Arg Thr Phe Ile His Pro Phe Asp Asp
145                 150                 155                 160

Pro Asp Val Met Ala Gly Gln Gly Thr Leu Ala Val Glu Ile Leu Asn
                165                 170                 175

Asp Ile Asp Thr Glu Pro His Phe Leu Phe Ala Ser Val Gly Gly Gly
            180                 185                 190

Gly Leu Leu Ser Gly Val Gly Thr Tyr Leu Lys Asn Val Ser Pro Asp
        195                 200                 205

Thr Lys Val Ile Ala Val Glu Pro Ala Gly Ala Ala Ser Tyr Phe Glu
    210                 215                 220

Ser Asn Lys Ala Gly His Val Val Thr Leu Asp Lys Ile Asp Lys Phe
225                 230                 235                 240

Val Asp Gly Ala Ala Val Lys Lys Ile Gly Glu Glu Thr Phe Arg Thr
245 250 255

Leu Glu Thr Val Val Asp Asp Ile Leu Leu Val Pro Glu Gly Lys Val
260 265 270

Cys Thr Ser Ile Leu Glu Leu Tyr Asn Glu Cys Ala Val Val Ala Glu
275 280 285

Pro Ala Gly Ala Leu Ser Val Ala Ala Leu Asp Leu Tyr Lys Asp Gln
290 295 300

Ile Lys Gly Lys Asn Val Val Cys Val Val Ser Gly Gly Asn Asn Asp
305 310 315 320

Ile Gly Arg Met Gln Glu Met Lys Glu Arg Ser Leu Ile Phe Glu Gly
325 330 335

Leu Gln His Tyr Phe Ile Val Asn Phe Pro Gln Arg Ala Gly Ala Leu
340 345 350

Arg Glu Phe Leu Asp Glu Val Leu Gly Pro Asn Asp Asp Ile Thr Arg
355 360 365

Phe Glu Tyr Thr Lys Lys Asn Asn Lys Ser Asn Gly Pro Ala Leu Val
370 375 380

Gly Ile Glu Leu Gln Asn Lys Ala Asp Tyr Gly Pro Leu Ile Glu Arg
385 390 395 400

Met Asn Lys Lys Pro Phe His Tyr Val Glu Val Asn Lys Asp Glu Asp
405 410 415

Leu Phe His Leu Leu Ile
420

<210> 151
<211> 1269
<212> DNA
<213> Bacillus subtilis

<220>
<221> misc_feature
<223> Encodes threonine deaminase (IlvA)

<400> 151
ttagattagc aaatggaaca agtcctcatc cttgttcacc tctacataat gaaacggttt 60

tttgttcatc cgttcaatca gcggcccata atcggctttg ttctgcagct cgatgccgac 120

aagagcaggt ccattgcttt tattattttt cttcgtatat tcaaaccttg taatgtcatc 180

attcggcccg aggacttcgt caagaaattc gcggagcgcc cccgctcttt gtggaaaatt 240

gacgataaaa taatgctgaa ggccttcaaa aatcaaggaa cgctccttca tttcctgcat 300

tcttccgata tcgttattgc ccccgcttac cacacacacc acgtttttcc ctttaatttg 360

atccttgtac aaatcaagcg ctgcgacaga aagagctcca gccggttcag caacaaccgc 420

acattcgtta tataattcaa gaatggatgt acatactttt ccttcaggca caaggagaat 480

gtcatcaacg acagtttcca gcgtccggaa ggtttcctcg ccaatttttt taacagccgc 540

cccatctaca aacttatcaa ttttatcaag tgtgacgaca tgcccagctt tgtttgattc 600

aaagtaggat gccgctcccg ccggctcaac agcaattacc tttgtatcag gagacacgtt 660

tttcaaatat gtacctaccc cagagagaag gccgccgcct ccgacacttg caaaaagaaa 720

atgcggttct gtatcaatgt cgtttaaaat ctcgactgcg agcgttccct gtccggccat 780

cacatccgga tcatcgaacg gatggataaa cgtccgagat tccgcctcac agcattcagc 840

agcgctttta tacgcgtcgt caaacgtatc gcctgtcaaa ataatatcga taaatccttt 900

tccgaatagt tcaacttgag atactttttg tctcggtgta gtagacggca taaaaatttt 960

cccgtgaatg ccaagatgtt tacacgaaaa tgccacacct tgagcgtggt ttccggcact 1020

tgcacacaca actccgttct ctgtctgctc gctggaaagc tgtttcattt tatgataagc 1080

ccctctgagc ttaaaagatc tgacaacttg caagtcttcg cgttttaaat agatattgca 1140

ttcatatctc tcagaaagtc tgtcattcct ctgcaaaggt gtgtgaatga caacgtcctt 1200

tacgttttgg tgagccttca aaatgtcttt cacttggatg agagagtttt ctttaagcaa 1260

cggtttcat 1269

<210> 152
<211> 436
<212> PRT
<213> Corynebacterium glutamicum

<220>
<221> MISC_FEATURE
<223> threonine deaminase (IlvA)

<400> 152

Met Ser Glu Thr Tyr Val Ser Glu Lys Ser Pro Gly Val Met Ala Ser
1 5 10 15

Gly Ala Glu Leu Ile Arg Ala Ala Asp Ile Gln Thr Ala Gln Ala Arg
20 25 30

Ile Ser Ser Val Ile Ala Pro Thr Pro Leu Gln Tyr Cys Pro Arg Leu
35 40 45

```
Ser Glu Glu Thr Gly Ala Glu Ile Tyr Leu Lys Arg Glu Asp Leu Gln
    50                  55                  60

Asp Val Arg Ser Tyr Lys Ile Arg Gly Ala Leu Asn Ser Gly Ala Gln
65                  70                  75                  80

Leu Thr Gln Glu Gln Arg Asp Ala Gly Ile Val Ala Ala Ser Ala Gly
                85                  90                  95

Asn His Ala Gln Gly Val Ala Tyr Val Cys Lys Ser Leu Gly Val Gln
            100                 105                 110

Gly Arg Ile Tyr Val Pro Val Gln Thr Pro Lys Gln Lys Arg Asp Arg
        115                 120                 125

Ile Met Val His Gly Gly Glu Phe Val Ser Leu Val Val Thr Gly Asn
    130                 135                 140

Asn Phe Asp Glu Ala Ser Ala Ala Ala His Glu Asp Ala Glu Arg Thr
145                 150                 155                 160

Gly Ala Thr Leu Ile Glu Pro Phe Asp Ala Arg Asn Thr Val Ile Gly
                165                 170                 175

Gln Gly Thr Val Ala Ala Glu Ile Leu Ser Gln Leu Thr Ser Met Gly
            180                 185                 190

Lys Ser Ala Asp His Val Met Val Pro Val Gly Gly Gly Gly Leu Leu
        195                 200                 205

Ala Gly Val Val Ser Tyr Met Ala Asp Met Ala Pro Arg Thr Ala Ile
    210                 215                 220

Val Gly Ile Glu Pro Ala Gly Ala Ala Ser Met Gln Ala Ala Leu His
225                 230                 235                 240

Asn Gly Gly Pro Ile Thr Leu Glu Thr Val Asp Pro Phe Val Asp Gly
                245                 250                 255

Ala Ala Val Lys Arg Val Gly Asp Leu Asn Tyr Thr Ile Val Glu Lys
            260                 265                 270

Asn Gln Gly Arg Val His Met Met Ser Ala Thr Glu Gly Ala Val Cys
        275                 280                 285

Thr Glu Met Leu Asp Leu Tyr Gln Asn Glu Gly Ile Ile Ala Glu Pro
```

290 295 300

Ala Gly Ala Leu Ser Ile Ala Gly Leu Lys Glu Met Ser Phe Ala Pro
305 310 315 320

Gly Ser Val Val Val Cys Ile Ile Ser Gly Gly Asn Asn Asp Val Leu
325 330 335

Arg Tyr Ala Glu Ile Ala Glu Arg Ser Leu Val His Arg Gly Leu Lys
340 345 350

His Tyr Phe Leu Val Asn Phe Pro Gln Lys Pro Gly Gln Leu Arg His
355 360 365

Phe Leu Glu Asp Ile Leu Gly Pro Asp Asp Asp Ile Thr Leu Phe Glu
370 375 380

Tyr Leu Lys Arg Asn Asn Arg Glu Thr Gly Thr Ala Leu Val Gly Ile
385 390 395 400

His Leu Ser Glu Ala Ser Gly Leu Asp Ser Leu Leu Glu Arg Met Glu
405 410 415

Glu Ser Ala Ile Asp Ser Arg Arg Leu Glu Pro Gly Thr Pro Glu Tyr
420 425 430

Glu Tyr Leu Thr
435

<210> 153
<211> 1311
<212> DNA
<213> Corynebacterium glutamicum

<220>
<221> misc_feature
<223> Encodes threonine deaminase (IlvA)

<400> 153
ttaggtcaag tattcgtact caggggtgcc cggctcgagg cgacgggaat caattgccga 60

ttcctccata cgttccagca aagaatccaa tcctgatgct tcactcaagt gaatacccac 120

caacgcagta ccggtctcac ggttgttgcg cttgaggtac tcaaacagcg tgatgtcatc 180

atccggtccc aggatatctt ccaggaagtg acgcaactga ccaggctttt gcgggaagtt 240

caccaagaag tagtgcttca aaccgcggtg caccaaggag cgctcagcga tttccgcata 300

acgcagcaca tcgttgttgc caccagagat gatgcacacc acgacagaac caggtgcaaa 360

ggacatttcc ttcaacccag cgatagacag cgcgccagca ggctccgcga tgatgccttc 420

gttttggtaa agatcgagca tctcagtaca cacagcgccc tcggtcgcgc tcatcatgtg 480

cacgcgaccc tggttcttct ccacgatggt gtagttgaga tctccgacac gtttgactgc 540

tgcgccgtcc acaaagggat caacagtctc caaagtgatt ggtccaccat tgtgcaatgc 600

agcctgcatg gatgctgctc ccgctggttc gataccaacg atcgcagtgc gaggtgccat 660

atcagccatg tagctgacca cacctgcaag aagtccgcca ccgccgactg gaaccatcac 720

gtgatctgca ctcttgccca tggaagtcag ctgcgacaag atctcagcag ccacggtgcc 780

ctgaccgatg acggtgttgc gagcatcgaa aggctcgatc agcgttgcgc cggtgcgctc 840

tgcatcttca tgcgctgcag ccgatgcttc gtcgaagtta ttgccagtga ccaccaagga 900

gacaaactct ccgccgtgaa ccatgatgcg gtcacgcttt tgctttggag tctgcacagg 960

aacatagatg cgtccctgaa cgcccaagga cttgcacaca taggccacgc cctgggcatg 1020

gttacctgca gatgcggcaa cgatacctgc atcgcgctgc tcttgggtga gctgcgctcc 1080

agagttcagc gcaccgcgga tcttgtagga acgaacatcc tgcagatcct cacgcttaag 1140

gtagatttcc gctccggttt cctcagaaag acgagggcaa tactgcaatg gagttggtgc 1200

aatgacggag gaaattcgtg cctgcgccgt ttgaatgtcg gcggcacgaa tcagctccgc 1260

tccgctagcc atcactcctg gacttttctc agacacgtat gtttcactca t 1311

<210> 154
<211> 310
<212> PRT
<213> Corynebacterium glutamicum

<220>
<221> MISC_FEATURE
<223> threonine deaminase (TdcB)

<400> 154

Met Leu Thr Leu Asn Asp Val Ile Thr Ala Gln Gln Arg Thr Ala Pro
1 5 10 15

His Val Arg Arg Thr Pro Leu Phe Glu Ala Asp Pro Ile Asp Gly Thr
20 25 30

Gln Ile Trp Ile Lys Ala Glu Phe Leu Gln Lys Cys Gly Val Phe Lys
35 40 45

Thr Arg Gly Ala Phe Asn Arg Gln Leu Ala Ala Ser Glu Asn Gly Leu
50 55 60

Leu Asp Pro Thr Val Gly Ile Val Ala Ala Ser Gly Gly Asn Ala Gly
65 70 75 80

```
Leu Ala Asn Ala Phe Ala Ala Ala Ser Leu Ser Val Pro Ala Thr Val
                85                  90                  95

Leu Val Pro Glu Thr Ala Pro Gln Val Lys Val Asp Arg Leu Lys Gln
            100                 105                 110

Tyr Gly Ala Thr Val Gln Gln Ile Gly Ser Glu Tyr Ala Glu Ala Phe
        115                 120                 125

Glu Ala Ala Gln Thr Phe Glu Ser Glu Thr Gly Ala Leu Phe Cys His
    130                 135                 140

Ala Tyr Asp Gln Pro Asp Ile Ala Ala Gly Ala Gly Val Ile Gly Leu
145                 150                 155                 160

Glu Ile Val Glu Asp Leu Pro Asp Val Asp Thr Ile Val Val Ala Val
                165                 170                 175

Gly Gly Gly Gly Leu Tyr Ala Gly Ile Ala Ala Val Val Ala Ala His
            180                 185                 190

Asp Ile Lys Val Val Ala Val Glu Pro Ser Lys Ile Pro Thr Leu His
        195                 200                 205

Asn Ser Leu Ile Ala Gly Gln Pro Val Asp Val Asn Val Ser Gly Ile
    210                 215                 220

Ala Ala Asp Ser Leu Gly Ala Arg Gln Ile Gly Arg Glu Ala Phe Asp
225                 230                 235                 240

Ile Ala Thr Ala His Pro Pro Ile Gly Val Leu Val Asp Asp Glu Ala
                245                 250                 255

Ile Ile Ala Ala Arg Arg His Leu Trp Asp Asn Tyr Arg Ile Pro Ala
            260                 265                 270

Glu His Gly Ala Ala Ala Ala Leu Ala Ser Leu Thr Ser Gly Ala Tyr
        275                 280                 285

Lys Pro Ala Ala Asp Glu Lys Val Ala Val Ile Val Cys Gly Ala Asn
    290                 295                 300

Thr Asp Leu Thr Thr Leu
305                 310
```

<210> 155
<211> 933

<212> DNA
<213> Corynebacterium glutamicum

<220>
<221> misc_feature
<223> Encodes threonine deaminase (TdcB)

<400> 155
tcacagtgtt gtgaggtcag tgttcgctcc gcacacaatg actgccactt tttcatctgc 60

tgcaggtttg tatgctccac tggtaagaga ggcgagtgct gcggcagcgc catgctcggc 120

agggatgcgg tagttgtccc agaggtggcg tcgagctgcg atgattgctt catcgtccac 180

taggacgcct attgggggat gggcagttgc gatgtcaaag gcttctcgtc caatttggcg 240

agccccccaaa gaatctgccg cgataccaga aacgttcaca tcgactggtt ggccggcaat 300

gagtgagttg tgcagggttg gaattttgga gggttcaacg gccaccactt tgatgtcgtg 360

ggctgctacg acggctgcga ttcctgcata gagtccaccg ccaccgacag caaccacgat 420

ggtgtcaacg tcgggaagat cttcgacaat ttctagccca atgacgcctg ctccagctgc 480

gatgtcgggc tggtcgtagg cgtggcaaaa cagagcacca gtttccgact caaaggtttg 540

agctgcctca aatgcttccg catattcaga tccgatttgt tgcacggttg caccgtattg 600

cttgaggcga tcaactttta cttgtggggc agtttcgggc accaataccg tggcgggaac 660

gcttaaggat gctgcggcaa aagcatttgc gagtcctgcg tttccgcctg atgccgcgac 720

gatgccaacc gttgggtcga gtagtccgtt ttccgaagct gcgagctggc ggttgaatgc 780

tccacgcgtt ttgaacacgc cgcacttttg gaggaactct gctttgatcc agatttgtgt 840

gccgtcgatg ggtctgctt cgaaaagtgg cgttcgtcga acatgagggg cggttcgttg 900

ttgggcggtg atgacatcgt tgagggtgag cat 933

<210> 156
<211> 491
<212> PRT
<213> Methanocaldococcus jannaschii

<220>
<221> MISC_FEATURE
<223> (R)-citramalate synthase

<400> 156

Met Met Val Arg Ile Phe Asp Thr Thr Leu Arg Asp Gly Glu Gln Thr
1 5 10 15

Pro Gly Val Ser Leu Thr Pro Asn Asp Lys Leu Glu Ile Ala Lys Lys
20 25 30

Leu Asp Glu Leu Gly Val Asp Val Ile Glu Ala Gly Ser Ala Ile Thr

35 40 45

Ser Lys Gly Glu Arg Glu Gly Ile Lys Leu Ile Thr Lys Glu Gly Leu
50 55 60

Asn Ala Glu Ile Cys Ser Phe Val Arg Ala Leu Pro Val Asp Ile Asp
65 70 75 80

Ala Ala Leu Glu Cys Asp Val Asp Ser Val His Leu Val Val Pro Thr
85 90 95

Ser Pro Ile His Met Lys Tyr Lys Leu Arg Lys Thr Glu Asp Glu Val
100 105 110

Leu Glu Thr Ala Leu Lys Ala Val Glu Tyr Ala Lys Glu His Gly Leu
115 120 125

Ile Val Glu Leu Ser Ala Glu Asp Ala Thr Arg Ser Asp Val Asn Phe
130 135 140

Leu Ile Lys Leu Phe Asn Glu Gly Glu Lys Val Gly Ala Asp Arg Val
145 150 155 160

Cys Val Cys Asp Thr Val Gly Val Leu Thr Pro Gln Lys Ser Gln Glu
165 170 175

Leu Phe Lys Lys Ile Thr Glu Asn Val Asn Leu Pro Val Ser Val His
180 185 190

Cys His Asn Asp Phe Gly Met Ala Thr Ala Asn Thr Cys Ser Ala Val
195 200 205

Leu Gly Gly Ala Val Gln Cys His Val Thr Val Asn Gly Ile Gly Glu
210 215 220

Arg Ala Gly Asn Ala Ser Leu Glu Glu Val Val Ala Ala Leu Lys Ile
225 230 235 240

Leu Tyr Gly Tyr Asp Thr Lys Ile Lys Met Glu Lys Leu Tyr Glu Val
245 250 255

Ser Arg Ile Val Ser Arg Leu Met Lys Leu Pro Val Pro Pro Asn Lys
260 265 270

Ala Ile Val Gly Asp Asn Ala Phe Ala His Glu Ala Gly Ile His Val
275 280 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Val Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490

<210> 157
<211> 1476
<212> DNA
<213> Methanocaldococcus jannaschii

<220>
<221> misc_feature
<223> Encodes (R)-citramalate synthase

<400> 157

```
atgatggtaa ggatatttga tacaacactt agagatggag agcaaacacc aggagtttct      60
ttaacaccaa atgataagtt agagatagca aaaaaattgg atgagcttgg agttgatgtt     120
atagaggcag gttcagctat aacttcaaaa ggagagagag aaggaataaa attaataaca     180
aaagaaggtt taaatgcaga aatctgctca tttgttagag ctttacctgt agatattgat     240
gctgccttag aatgtgatgt agatagtgtc catttagtag tgccaacatc tccaatacac     300
atgaaatata agcttagaaa aacagaagat gaggttttag agacagcttt aaaggctgta     360
gagtatgcta aagaacatgg attgattgtt gagttatctg cagaggatgc aacaagaagt     420
gatgtaaatt tcttaataaa actatttaat gaaggggaaa aggttggagc agacagagtt     480
tgtgtttgtg acacagtagg agttttaact ccacaaaaga gtcaggaatt atttaaaaaa     540
ataactgaaa atgttaattt accggtctca gttcattgcc acaacgactt tggaatggct     600
actgctaata cttgctcagc agttttaggt ggagctgttc agtgccacgt aacagttaat     660
ggtattggag agagagcagg aaatgcctca ttggaagagg ttgttgctgc tttaaaaata     720
ctctatggct atgatactaa gataaagatg gaaaagttat atgaggtttc aagaattgtc     780
tcaagattga tgaaacttcc tgttccacca aataaagcaa ttgttgggga caatgcattt     840
gctcatgaag caggaataca tgttgatgga ttaataaaaa atactgaaac ctatgagcca     900
ataaaaccag aaatggttgg gaatagaaga agaattattt tgggtaagca ttctggtaga     960
aaagctttaa aatacaaact tgatttgatg ggcataaacg ttagtgatga gcaattaaat    1020
aaaatatatg aaagagttaa agaatttggg gatttgggta aatacatttc agacgctgat    1080
ttgttggcta tagttagaga agttactgga aaattggtag aagagaaaat caaattagat    1140
gaattaactg ttgtatctgg aaataaaata acaccaattg catctgttaa actccattat    1200
aaaggagaag atataacttt aatagaaact gcttatggtg ttggaccggt agatgcagca    1260
ataaatgctg tgagaaaggc aataagtgga gttgcagata ttaagttggt agagtataga    1320
gttgaagcaa ttggtggagg aactgatgcg ttaatagagg ttgttgttaa attaagaaaa    1380
ggaactgaaa ttgttgaagt tagaaaatca gacgctgata taataagggc ttctgtagat    1440
gctgtaatgg aaggaatcaa tatgttattg aattaa                              1476
```

<210> 158
<211> 372
<212> PRT
<213> Methanocaldococcus jannaschii variant

<220>
<221> MISC_FEATURE
<223> Methanocaldococcus jannaschii (R)-citramalate synthase variant

<400> 158

```
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Val Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Ser Lys Ile
225                 230                 235                 240

Leu Tyr Gly Tyr Asp Thr Lys Ile Lys Met Glu Lys Leu Tyr Glu Val
                245                 250                 255
```

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu
    370

<210> 159
<211> 1119
<212> DNA
<213> Methanocaldococcus jannaschii variant

<220>
<221> misc_feature
<223> Encodes Methanocaldococcus jannaschii (R)-citramalate synthase variant

<400> 159
atgatggtaa ggatatttga tacaacactt agagatggag agcaaacacc aggagtttct 60

ttaacaccaa atgataagtt agagatagca aaaaaattgg atgagcttgg agttgatgtt 120

atagaggcag gttcagctgt aacttcaaaa ggagagagag aaggaataaa attaataaca 180

aaagaaggtt taaatgcaga aatctgctca tttgttagag ctttacctgt agatattgat 240

gctgccttag aatgtgatgt agatagtgtc catttagtag tgccaacatc tccaatacac 300

atgaaatata agcttagaaa aacagaagat gaggttttag tgacagcttt aaaggctgta 360

gagtatgcta aagaacaggg attgattgtt gagttatctg cagaggatgc aacaagaagt 420

gatgtaaatt tcttaataaa actatttaat gaaggggaaa aggttggagc agacagagtt 480

tgtgtttgtg acacagtagg agttttaact ccacaaaaga gtcaggaatt atttaaaaaa 540

ataactgaaa atgttaattt accggtctca gttcattgcc acaacgactt tggaatggct 600

actgctaatg cttgctcagc agttttaggt ggagctgttc agtgccacgt aacagttaat 660

ggtattggag agagagcagg aaatgcctca ttggaagagg ttgttgctgc ttcaaaaata 720

ctctatggct atgatactaa gataaagatg gaaaagttat atgaggtttc aagaattgtc 780

tcaagattga tgaaacttcc tgttccacca aataaagcaa ttgttgggga caatgcattt 840

gctcatgaag caggaataca tgttgatgga ttaataaaaa atactgaaac ctatgagcca 900

ataaaaccag aaatggttgg gaatagaaga agaattattt tgggtaagca ttctggtaga 960

aaagctttaa aatacaaact tgatttgatg ggcataaacg ttagtgatga gcaattaaat 1020

aaaatatatg aaagagttaa agaatttggg gatttgggta aatacatttc agacgctgat 1080

ttgttggcta tagttagaga agttactgga aaattgtaa 1119

<210> 160
<211> 516
<212> PRT
<213> Leptospira interrogans

<220>
<221> MISC_FEATURE
<223> (R)-citramalate synthase

<400> 160

Met Thr Lys Val Glu Thr Arg Leu Glu Ile Leu Asp Val Thr Leu Arg
1 5 10 15

Asp Gly Glu Gln Thr Arg Gly Val Ser Phe Ser Thr Ser Glu Lys Leu
20 25 30

Asn Ile Ala Lys Phe Leu Leu Gln Lys Leu Asn Val Asp Arg Val Glu
35 40 45

Ile Ala Ser Ala Arg Val Ser Lys Gly Glu Leu Glu Thr Val Gln Lys
50 55 60

Ile Met Glu Trp Ala Ala Thr Glu Gln Leu Thr Glu Arg Ile Glu Ile
65 70 75 80

Leu Gly Phe Val Asp Gly Asn Lys Thr Val Asp Trp Ile Lys Asp Ser
85 90 95

Gly Ala Lys Val Leu Asn Leu Leu Thr Lys Gly Ser Leu His His Leu
100 105 110

Glu Lys Gln Leu Gly Lys Thr Pro Lys Glu Phe Phe Thr Asp Val Ser
115 120 125

```
Phe Val Ile Glu Tyr Ala Ile Lys Ser Gly Leu Lys Ile Asn Val Tyr
    130                 135                 140

Leu Glu Asp Trp Ser Asn Gly Phe Arg Asn Ser Pro Asp Tyr Val Lys
145                 150                 155                 160

Ser Leu Val Glu His Leu Ser Lys Glu His Ile Glu Arg Ile Phe Leu
                165                 170                 175

Pro Asp Thr Leu Gly Val Leu Ser Pro Glu Glu Thr Phe Gln Gly Val
            180                 185                 190

Asp Ser Leu Ile Gln Lys Tyr Pro Asp Ile His Phe Glu Phe His Gly
        195                 200                 205

His Asn Asp Tyr Asp Leu Ser Val Ala Asn Ser Leu Gln Ala Ile Arg
    210                 215                 220

Ala Gly Val Lys Gly Leu His Ala Ser Ile Asn Gly Leu Gly Glu Arg
225                 230                 235                 240

Ala Gly Asn Thr Pro Leu Glu Ala Leu Val Thr Thr Ile His Asp Lys
                245                 250                 255

Ser Asn Ser Lys Thr Asn Ile Asn Glu Ile Ala Ile Thr Glu Ala Ser
            260                 265                 270

Arg Leu Val Glu Val Phe Ser Gly Lys Arg Ile Ser Ala Asn Arg Pro
        275                 280                 285

Ile Val Gly Glu Asp Val Phe Thr Gln Thr Ala Gly Val His Ala Asp
    290                 295                 300

Gly Asp Lys Lys Gly Asn Leu Tyr Ala Asn Pro Ile Leu Pro Glu Arg
305                 310                 315                 320

Phe Gly Arg Lys Arg Ser Tyr Ala Leu Gly Lys Leu Ala Gly Lys Ala
                325                 330                 335

Ser Ile Ser Glu Asn Val Lys Gln Leu Gly Met Val Leu Ser Glu Val
            340                 345                 350

Val Leu Gln Lys Val Leu Glu Arg Val Ile Glu Leu Gly Asp Gln Asn
        355                 360                 365

Lys Leu Val Thr Pro Glu Asp Leu Pro Phe Ile Ile Ala Asp Val Ser
```

370 375 380

Gly Arg Thr Gly Glu Lys Val Leu Thr Ile Lys Ser Cys Asn Ile His
385 390 395 400

Ser Gly Ile Gly Ile Arg Pro His Ala Gln Ile Glu Leu Glu Tyr Gln
405 410 415

Gly Lys Ile His Lys Glu Ile Ser Glu Gly Asp Gly Gly Tyr Asp Ala
420 425 430

Phe Met Asn Ala Leu Thr Lys Ile Thr Asn Arg Leu Gly Ile Ser Ile
435 440 445

Pro Lys Leu Ile Asp Tyr Glu Val Arg Ile Pro Pro Gly Gly Lys Thr
450 455 460

Asp Ala Leu Val Glu Thr Arg Ile Thr Trp Asn Lys Ser Leu Asp Leu
465 470 475 480

Glu Glu Asp Gln Thr Phe Lys Thr Met Gly Val His Pro Asp Gln Thr
485 490 495

Val Ala Ala Val His Ala Thr Glu Lys Met Leu Asn Gln Ile Leu Gln
500 505 510

Pro Trp Gln Ile
515

<210> 161
<211> 1551
<212> DNA
<213> Leptospira interrogans

<220>
<221> misc_feature
<223> Encodes (R)-citramalate synthase

<400> 161
atgacaaaag tagaaactcg attggaaatt ttagacgtaa ctttgagaga cggggagcag 60

accagagggg tcagtttttc cacttccgaa aaactaaata tcgcaaaatt tctattacaa 120

aaactaaatg tagatcgggt agagattgcg tctgcaagag tttctaaagg ggaattggaa 180

acggtccaaa aaatcatgga atgggctgca acagaacagc ttacggaaag aatcgaaatc 240

ttaggttttg tagacgggaa taaaaccgta gattggatca aagatagtgg ggctaaggtt 300

ttaaatcttt tgactaaggg atcgcttcat catttagaaa aacaattagg caaaactccg 360

aaagaattct ttacagacgt ttcttttgta atagaatacg cgatcaaaag cggacttaaa 420

```
ataaacgtat atttagaaga ttggtccaac ggtttcagaa acagtccaga ttacgtcaaa      480
tcgctcgtag aacatctaag taaagaacat atagaaagaa tttttcttcc agacacgtta      540
ggcgttcttt cgccagaaga gacgtttcaa ggagtggatt cactcattca aaaatacccg      600
gatattcatt ttgaatttca cggacataac gactacgatc tttccgtggc aaatagtctt      660
caagcgattc gtgccggagt caaaggtctt cacgcttcta taaatggtct cggagaaaga      720
gccggaaata ctccgttgga agcactcgta accacgattc atgataagtc taactctaaa      780
acgaacataa acgaaattgc aattacggaa gcaagccgtc ttgtagaagt attcagcgga      840
aaaagaattt ctgcaaatag accgatcgta ggagaagacg tgtttactca gaccgcggga      900
gtacacgcag acggagacaa aaaaggaaat ttatacgcaa atcctatttt accggaaaga      960
tttggtagga aaagaagtta cgcgttaggc aaacttgcag gtaaggcgag tatctccgaa     1020
aatgtaaaac aactcggaat ggttttaagt gaagtggttt tacaaaaggt tttagaaagg     1080
gtgatcgaat taggagatca gaataaacta gtgacacctg aagatcttcc atttatcatt     1140
gcggacgttt ctggaagaac cggagaaaag gtacttacaa tcaaatcttg taatattcat     1200
tccggaattg gaattcgtcc tcacgcacaa attgaattgg aatatcaggg aaagattcat     1260
aaggaaattt ctgaaggaga cggagggtat gatgcgttta tgaatgcact tactaaaatt     1320
acgaatcgcc tcggtattag tattcctaaa ttgatagatt acgaagtaag gattcctcct     1380
ggtggaaaaa cagatgcact tgtagaaact aggatcacct ggaacaagtc cttagattta     1440
gaagaggacc agactttcaa aacgatggga gttcatccgg atcaaacggt tgcagcggtt     1500
catgcaactg aaaagatgct caatcaaatt ctacaaccat ggcaaatcta a              1551
```

```
<210>  162
<211>  386
<212>  PRT
<213>  Leptospira interrogans mutant


<220>
<221>  MISC_FEATURE
<223>  Leptospira interrogans (R)-citramalate synthase mutant

<400>  162

Met Thr Lys Val Glu Thr Arg Leu Glu Ile Leu Asp Val Thr Leu Arg
1               5                   10                  15


Asp Gly Glu Gln Thr Arg Gly Val Ser Phe Ser Thr Ser Glu Lys Leu
            20                  25                  30


Asn Ile Ala Lys Phe Leu Leu Gln Lys Leu Asn Val Asp Arg Val Glu
        35                  40                  45
```

```
Ile Ala Ser Ala Arg Val Ser Lys Gly Glu Leu Glu Thr Val Gln Lys
    50                  55                  60

Ile Met Glu Trp Ala Ala Thr Glu Gln Leu Thr Glu Arg Ile Glu Ile
65                  70                  75                  80

Leu Gly Phe Val Asp Gly Asn Lys Thr Val Asp Trp Ile Lys Asp Ser
                85                  90                  95

Gly Ala Lys Val Leu Asn Leu Leu Thr Lys Gly Ser Leu His His Leu
            100                 105                 110

Glu Lys Gln Leu Gly Lys Thr Pro Lys Glu Phe Phe Thr Asp Val Ser
        115                 120                 125

Phe Val Ile Glu Tyr Ala Ile Lys Ser Gly Leu Lys Ile Asn Val Tyr
    130                 135                 140

Leu Glu Asp Trp Ser Asn Gly Phe Arg Asn Ser Pro Asp Tyr Val Lys
145                 150                 155                 160

Ser Leu Val Glu His Leu Ser Lys Glu His Ile Glu Arg Ile Phe Leu
                165                 170                 175

Pro Asp Thr Leu Gly Val Leu Ser Pro Glu Glu Thr Phe Gln Gly Val
            180                 185                 190

Asp Ser Leu Ile Gln Lys Tyr Pro Asp Ile His Phe Glu Phe His Gly
        195                 200                 205

His Asn Asp Tyr Asp Leu Ser Val Ala Asn Ser Leu Gln Ala Ile Arg
    210                 215                 220

Ala Gly Val Lys Gly Leu His Ala Ser Ile Asn Gly Leu Gly Glu Arg
225                 230                 235                 240

Ala Gly Asn Thr Pro Leu Glu Ala Leu Val Thr Thr Ile His Asp Lys
                245                 250                 255

Ser Asn Ser Lys Thr Asn Ile Asn Glu Ile Ala Ile Thr Glu Ala Ser
            260                 265                 270

Arg Leu Val Glu Val Phe Ser Gly Lys Arg Ile Ser Ala Asn Arg Pro
        275                 280                 285

Ile Val Gly Glu Asp Val Phe Thr Gln Thr Ala Gly Val His Ala Asp
    290                 295                 300
```

Gly Asp Lys Lys Gly Asn Leu Tyr Ala Asn Pro Ile Leu Pro Glu Arg
305 310 315 320

Phe Gly Arg Lys Arg Ser Tyr Ala Leu Gly Lys Leu Ala Gly Lys Ala
325 330 335

Ser Ile Ser Glu Asn Val Lys Gln Leu Gly Met Val Leu Ser Glu Val
340 345 350

Val Leu Gln Lys Val Leu Glu Arg Val Ile Glu Leu Gly Asp Gln Asn
355 360 365

Lys Leu Val Thr Pro Glu Asp Leu Pro Phe Ile Ile Ala Asp Val Ser
370 375 380

Gly Arg
385

<210> 163
<211> 1161
<212> DNA
<213> Leptospira interrogans mutant

<220>
<221> misc_feature
<223> Encodes Leptospira interrogans (R)-citramalate synthase mutant

<400> 163
atgacaaaag tagaaactcg attggaaatt ttagacgtaa ctttgagaga cggggagcag 60
accagagggg tcagtttttc cacttccgaa aaactaaata tcgcaaaatt tctattacaa 120
aaactaaatg tagatcgggt agagattgcg tctgcaagag tttctaaagg ggaattggaa 180
acggtccaaa aaatcatgga atgggctgca acagaacagc ttacggaaag aatcgaaatc 240
ttaggttttg tagacgggaa taaaaccgta gattggatca aagatagtgg ggctaaggtt 300
ttaaatcttt tgactaaggg atcgcttcat catttagaaa aacaattagg caaaactccg 360
aaagaattct ttacagacgt ttcttttgta atagaatacg cgatcaaaag cggacttaaa 420
ataaacgtat atttagaaga ttggtccaac ggtttcagaa acagtccaga ttacgtcaaa 480
tcgctcgtag aacatctaag taaagaacat atagaaagaa tttttcttcc agacacgtta 540
ggcgttcttt cgccagaaga gacgtttcaa ggagtggatt cactcattca aaaataccccg 600
gatattcatt ttgaatttca cggacataac gactacgatc tttccgtggc aaatagtctt 660
caagcgattc gtgccggagt caaaggtctt cacgcttcta taaatggtct cggagaaaga 720
gccggaaata ctccgttgga agcactcgta accacgattc atgataagtc taactctaaa 780

acgaacataa acgaaattgc aattacggaa gcaagccgtc ttgtagaagt attcagcgga 840

aaaagaattt ctgcaaatag accgatcgta ggagaagacg tgtttactca gaccgcggga 900

gtacacgcag acggagacaa aaaaggaaat ttatacgcaa atcctatttt accggaaaga 960

tttggtagga aaagaagtta cgcgttaggc aaacttgcag gtaaggcgag tatctccgaa 1020

aatgtaaaac aactcggaat ggttttaagt gaagtggttt tacaaaaggt tttagaaagg 1080

gtgatcgaat taggagatca gaataaacta gtgacacctg aagatcttcc atttatcatt 1140

gcggacgttt ctggaagata a 1161

<210> 164
<211> 466
<212> PRT
<213> Escherichia coli

<220>
<221> MISC_FEATURE
<223> isopropylmalate isomerase large subunit

<400> 164

```
Met Ala Lys Thr Leu Tyr Glu Lys Leu Phe Asp Ala His Val Val Tyr
1               5                   10                  15

Glu Ala Glu Asn Glu Thr Pro Leu Leu Tyr Ile Asp Arg His Leu Val
            20                  25                  30

His Glu Val Thr Ser Pro Gln Ala Phe Asp Gly Leu Arg Ala His Gly
        35                  40                  45

Arg Pro Val Arg Gln Pro Gly Lys Thr Phe Ala Thr Met Asp His Asn
    50                  55                  60

Val Ser Thr Gln Thr Lys Asp Ile Asn Ala Cys Gly Glu Met Ala Arg
65                  70                  75                  80

Ile Gln Met Gln Glu Leu Ile Lys Asn Cys Lys Glu Phe Gly Val Glu
                85                  90                  95

Leu Tyr Asp Leu Asn His Pro Tyr Gln Gly Ile Val His Val Met Gly
            100                 105                 110

Pro Glu Gln Gly Val Thr Leu Pro Gly Met Thr Ile Val Cys Gly Asp
        115                 120                 125

Ser His Thr Ala Thr His Gly Ala Phe Gly Ala Leu Ala Phe Gly Ile
    130                 135                 140
```

```
Gly Thr Ser Glu Val Glu His Val Leu Ala Thr Gln Thr Leu Lys Gln
145                 150                 155                 160

Gly Arg Ala Lys Thr Met Lys Ile Glu Val Gln Gly Lys Ala Ala Pro
                165                 170                 175

Gly Ile Thr Ala Lys Asp Ile Val Leu Ala Ile Ile Gly Lys Thr Gly
            180                 185                 190

Ser Ala Gly Gly Thr Gly His Val Val Glu Phe Cys Gly Glu Ala Ile
        195                 200                 205

Arg Asp Leu Ser Met Glu Gly Arg Met Thr Leu Cys Asn Met Ala Ile
    210                 215                 220

Glu Met Gly Ala Lys Ala Gly Leu Val Ala Pro Asp Glu Thr Thr Phe
225                 230                 235                 240

Asn Tyr Val Lys Gly Arg Leu His Ala Pro Lys Gly Lys Asp Phe Asp
                245                 250                 255

Asp Ala Val Ala Tyr Trp Lys Thr Leu Gln Thr Asp Glu Gly Ala Thr
            260                 265                 270

Phe Asp Thr Val Val Thr Leu Gln Ala Glu Glu Ile Ser Pro Gln Val
        275                 280                 285

Thr Trp Gly Thr Asn Pro Gly Gln Val Ile Ser Val Asn Asp Asn Ile
    290                 295                 300

Pro Asp Pro Ala Ser Phe Ala Asp Pro Val Glu Arg Ala Ser Ala Glu
305                 310                 315                 320

Lys Ala Leu Ala Tyr Met Gly Leu Lys Pro Gly Ile Pro Leu Thr Glu
                325                 330                 335

Val Ala Ile Asp Lys Val Phe Ile Gly Ser Cys Thr Asn Ser Arg Ile
            340                 345                 350

Glu Asp Leu Arg Ala Ala Ala Glu Ile Ala Lys Gly Arg Lys Val Ala
        355                 360                 365

Pro Gly Val Gln Ala Leu Val Val Pro Gly Ser Gly Pro Val Lys Ala
    370                 375                 380

Gln Ala Glu Ala Glu Gly Leu Asp Lys Ile Phe Ile Glu Ala Gly Phe
385                 390                 395                 400
```

Glu Trp Arg Leu Pro Gly Cys Ser Met Cys Leu Ala Met Asn Asn Asp
405 410 415

Arg Leu Asn Pro Gly Glu Arg Cys Ala Ser Thr Ser Asn Arg Asn Phe
420 425 430

Glu Gly Arg Gln Gly Arg Gly Gly Arg Thr His Leu Val Ser Pro Ala
435 440 445

Met Ala Ala Ala Ala Ala Val Thr Gly His Phe Ala Asp Ile Arg Asn
450 455 460

Ile Lys
465

<210> 165
<211> 1401
<212> DNA
<213> Escherichia coli

<220>
<221> misc_feature
<223> Encodes isopropylmalate isomerase large subunit

<400> 165
ttatttaatg ttgcgaatgt cggcgaaatg tccggtcaca gcagcagcgg cagccattgc 60

cgggctgacc agatgcgtgc gcccgccgcg cccctggcgg ccttcaaagt tacggttgct 120

ggtggaggca caacgttcgc ccggattcag acggtcgttg ttcatcgcca gacacattga 180

gcagccaggc aagcgccatt caaaaccggc ttcaataaag attttatcca gaccttccgc 240

ttccgcctgg gcttttaccg ggccagagcc gggaaccacc agtgcctgca cgcctggcgc 300

gacttttcgc cctttggcga tctccgctgc cgcgcgtaaa tcttcaatgc gcgagttggt 360

acaggaaccg ataaacactt tgtcgatagc cacttcggtc agcggaatac ccggtttcag 420

ccccatatag gccagcgctt tttctgccga cgcgcgttca accggatcgg caaacgaagc 480

cggatcggga atattgtcgt tcacggaaat cacctggccg ggattggtgc cccaggtgac 540

ctgcggtgaa atttcttctg cttgcagagt gacaacggta tcgaaagttg cgccttcgtc 600

ggtttgcagg gttttccagt aggcaacggc gtcgtcgaaa tctttgcctt tcggcgcatg 660

cagacggcct ttgacatagt taaaggtggt ttcgtccggt gcaaccagac cggcttttgc 720

gcccatttcg attgccatat tgcacagggt catacgacct tccatgctta aatcacggat 780

tgcttcgccg caaaactcca ccacatgccc ggtgccgcct gcgctaccgg ttttaccgat 840

aattgccagc acgatatctt ttgcggtaat gcccggcgcg gctttgccct ggacttcaat 900

tttcatggtt tttgcgcggc cctgtttcag ggtttgcgtt gccagtacgt gttcaacttc 960

ggaagtgccg ataccaaagg ccagtgcgcc aaacgcgccg tgggtggcgg tatgcgagtc 1020

gccgcagaca atggtcatcc ccggcaaggt gacgccctgt tccggcccca ttacgtggac 1080

gatcccctga tacgggtgat tcaggtcata cagttcgacg ccaaattctt tgcagttttt 1140

gatcagttcc tgcatctgga tacgcgccat ttcaccgcag gcattaatgt ctttggtctg 1200

ggtagagacg ttgtgatcca tggtagcgaa ggttttgccc ggctgacgta ccgggcgacc 1260

gtgggcgcgc agaccatcga acgcctgcgg tgaggtcact tcatgcacca ggtggcggtc 1320

gatatataac agtggggttt cgttttcggc ttcgtacaca acgtgagcgt cgaacaattt 1380

ttcgtataac gtcttagcca t 1401

<210> 166
<211> 201
<212> PRT
<213> Escherichia coli

<220>
<221> MISC_FEATURE
<223> isopropylmalate isomerase small subunit

<400> 166

Met Ala Glu Lys Phe Ile Lys His Thr Gly Leu Val Val Pro Leu Asp
1 5 10 15

Ala Ala Asn Val Asp Thr Asp Ala Ile Ile Pro Lys Gln Phe Leu Gln
20 25 30

Lys Val Thr Arg Thr Gly Phe Gly Ala His Leu Phe Asn Asp Trp Arg
35 40 45

Phe Leu Asp Glu Lys Gly Gln Gln Pro Asn Pro Asp Phe Val Leu Asn
50 55 60

Phe Pro Gln Tyr Gln Gly Ala Ser Ile Leu Leu Ala Arg Glu Asn Phe
65 70 75 80

Gly Cys Gly Ser Ser Arg Glu His Ala Pro Trp Ala Leu Thr Asp Tyr
85 90 95

Gly Phe Lys Val Val Ile Ala Pro Ser Phe Ala Asp Ile Phe Tyr Gly
100 105 110

Asn Ser Phe Asn Asn Gln Leu Leu Pro Val Lys Leu Ser Asp Ala Glu
115 120 125

Val Asp Glu Leu Phe Ala Leu Val Lys Ala Asn Pro Gly Ile His Phe

130 135 140

Asp Val Asp Leu Glu Ala Gln Glu Val Lys Ala Gly Glu Lys Thr Tyr
145 150 155 160

Arg Phe Thr Ile Asp Ala Phe Arg Arg His Cys Met Met Asn Gly Leu
165 170 175

Asp Ser Ile Gly Leu Thr Leu Gln His Asp Asp Ala Ile Ala Ala Tyr
180 185 190

Glu Ala Lys Gln Pro Ala Phe Met Asn
195 200

<210> 167
<211> 606
<212> DNA
<213> Escherichia coli

<220>
<221> misc_feature
<223> Encodes isopropylmalate isomerase small subunit

<400> 167
ttaattcata aacgcaggtt gttttgcttc ataagcggca atggcgtcgt cgtgctgcaa 60

ggtaagccca atactgtcca gaccgttcat catgcagtgg cggcggaagg catcgatggt 120

aaagcgatag gttttctctc ccgctttcac ctcttgcgct tccagatcca cgtcgaaatg 180

gatccccgga ttagctttca ccagcgcaaa cagttcgtcc acttctgcat cgcttaattt 240

caccggcagc agctggttgt taaagctatt gccgtagaag atgtcagcaa aactcggcgc 300

aatcaccact ttaaaaccgt agtcggtcaa tgcccagggc gcgtgctcac gcgaagagcc 360

acagccgaag ttttctcgtg ccagcaaaat ggaagcgccc tgatactgcg ggaagttcag 420

cacgaagtcc gggtttggct gttggccttt ttcatccaga aaacgccagt cgttaaacag 480

atgcgcgcca aaacccgtac gggtcacttt ctgcaaaaac tgtttcggga tgattgcatc 540

ggtatcgaca ttggcggcat ccagcggaac caccaggcct gtgtgtttga taaatttctc 600

tgccat 606

<210> 168
<211> 472
<212> PRT
<213> Bacillus subtilis

<220>
<221> MISC_FEATURE
<223> isopropylmalate isomerase large subunit

<400> 168

Met Met Pro Arg Thr Ile Ile Glu Lys Ile Trp Asp Gln His Ile Val
1 5 10 15

Lys His Gly Glu Gly Lys Pro Asp Leu Leu Tyr Ile Asp Leu His Leu
20 25 30

Ile His Glu Val Thr Ser Pro Gln Ala Phe Glu Gly Leu Arg Gln Lys
35 40 45

Gly Arg Lys Val Arg Arg Pro Gln Asn Thr Phe Ala Thr Met Asp His
50 55 60

Asn Ile Pro Thr Val Asn Arg Phe Glu Ile Lys Asp Glu Val Ala Lys
65 70 75 80

Arg Gln Val Thr Ala Leu Glu Arg Asn Cys Glu Glu Phe Gly Val Arg
85 90 95

Leu Ala Asp Leu His Ser Val Asp Gln Gly Ile Val His Val Val Gly
100 105 110

Pro Glu Leu Gly Leu Thr Leu Pro Gly Lys Thr Ile Val Cys Gly Asp
115 120 125

Ser His Thr Ser Thr His Gly Ala Phe Gly Ala Leu Ala Phe Gly Ile
130 135 140

Gly Thr Ser Glu Val Glu His Val Leu Ser Thr Gln Thr Leu Trp Gln
145 150 155 160

Gln Arg Pro Lys Thr Leu Glu Val Arg Val Asp Gly Thr Leu Gln Lys
165 170 175

Gly Val Thr Ala Lys Asp Val Ile Leu Ala Val Ile Gly Lys Tyr Gly
180 185 190

Val Lys Phe Gly Thr Gly Tyr Val Ile Glu Tyr Thr Gly Glu Val Phe
195 200 205

Arg Asn Met Thr Met Asp Glu Arg Met Thr Val Cys Asn Met Ser Ile
210 215 220

Glu Ala Gly Ala Arg Ala Gly Leu Ile Ala Pro Asp Glu Val Thr Phe
225 230 235 240

Glu Tyr Cys Lys Asn Arg Lys Tyr Thr Pro Lys Gly Glu Glu Phe Asp

```
                245                 250                 255

Lys Ala Val Glu Glu Trp Lys Ala Leu Arg Thr Asp Pro Gly Ala Val
            260                 265                 270

Tyr Asp Lys Ser Ile Val Leu Asp Gly Asn Lys Ile Ser Pro Met Val
        275                 280                 285

Thr Trp Gly Ile Asn Pro Gly Met Val Leu Pro Val Asp Ser Glu Val
    290                 295                 300

Pro Ala Pro Glu Ser Phe Ser Ala Glu Asp Asp Lys Lys Glu Ala Ile
305                 310                 315                 320

Arg Ala Tyr Glu Tyr Met Gly Leu Thr Pro His Gln Lys Ile Glu Asp
                325                 330                 335

Ile Lys Val Glu His Val Phe Ile Gly Ser Cys Thr Asn Ser Arg Met
            340                 345                 350

Thr Asp Leu Arg Gln Ala Ala Asp Met Ile Lys Gly Lys Lys Val Ala
        355                 360                 365

Asp Ser Val Arg Ala Ile Val Val Pro Gly Ser Gln Ser Val Lys Leu
    370                 375                 380

Gln Ala Glu Lys Glu Gly Leu Asp Gln Ile Phe Leu Glu Ala Gly Phe
385                 390                 395                 400

Glu Trp Arg Glu Ser Gly Cys Ser Met Cys Leu Ser Met Asn Asn Asp
                405                 410                 415

Val Val Pro Glu Gly Glu Arg Cys Ala Ser Thr Ser Asn Arg Asn Phe
            420                 425                 430

Glu Gly Arg Gln Gly Lys Gly Ala Arg Thr His Leu Val Ser Pro Ala
        435                 440                 445

Met Ala Ala Met Ala Ala Ile His Gly His Phe Val Asp Val Arg Lys
    450                 455                 460

Phe Tyr Gln Glu Lys Thr Val Val
465                 470
```

<210>  169
<211>  1419
<212>  DNA
<213>  Bacillus subtilis

<220>
<221> misc_feature
<223> Encodes isopropylmalate isomerase large subunit

<400> 169
ttacacaact gtttttcct gataaaactt tctgacatca acgaagtgtc cgtgaatggc 60

agccatcgca gccattgccg ggctgacgag atgtgttctt gcacctttc cttgtctgcc 120

ctcgaagttg cggttagagg ttgatgcaca gcgctctccc tcaggaacaa catcattatt 180

catactcaaa cacatgctgc agcctgactc tctccattca aatccagctt ccaagaaaat 240

ctggtcaagc ccttcttttt cagcctgaag cttcacgctt tgggatccgg gcacgacgat 300

ggcccttacg ctgtcagcta ccttcttccc tttgatcatg tcagcagcct gtcgaaggtc 360

agtcatgcgg gaatttgtgc aggaaccgat aaatacgtgc tccactttaa tgtcttcaat 420

tttttggtga ggagtcaatc ccatatattc ataagcacga atcgcttctt ttttatcgtc 480

ttctgcagaa aagctttccg gcgcaggaac ttcagaatcg acaggaagaa ccattcccgg 540

gttaatgccc catgtcacca taggggaaat tttgttgccg tcaaggacga tagatttatc 600

gtaaacagcg cccgggtctg tgcgcagcgc cttccattcc tctacggcct tgtcaaattc 660

ttcgcctttt ggcgtgtact tgcgattttt gcaatattca aacgtcacct cgtcaggtgc 720

aatcaaacct gctcttgctc ctgcttcaat tgacatgtta caaacagtca ttcgttcatc 780

catcgtcata tttctgaata cttccccagt gtattcaatg acgtagcctg tgccgaattt 840

cacaccgtat ttgccgatga cagcaaggat gacatccttt gccgttaccc ctttttgaag 900

cgttccatct acgcgcactt caagtgtttt tggacgctgc tgccaaagtg tctgtgtgga 960

aaggacatgt tcaacttcac tcgtcccgat tccaaatgca agagcgccga aagcgccatg 1020

tgttgatgta tgactgtcac cgcacacaat cgttttccct ggaagcgtta agcctagttc 1080

aggtccgacg acatggacaa tcccttgatc cacactgtga agatcggcaa ggcgcacgcc 1140

aaattcctca cagtttcttt caagcgccgt tacctggcgt ttcgcaactt catcctttat 1200

ctcaaaacga ttgacagtcg ggatgttgtg gtccattgtc gcaaatgtgt tttggggtct 1260

tctgaccttt cttccctttt gtctcaagcc ttcaaatgcc tgaggagacg tcacctcatg 1320

aatgaggtgc aaatcaatat agagaagatc cggctttccc tcaccatgtt ttacaatatg 1380

ctgatcccaa atcttttcga tgattgttcg aggcatcat 1419

<210> 170
<211> 199
<212> PRT
<213> Bacillus subtilis

<220>

<221> MISC_FEATURE
<223> isopropylmalate isomerase small subunit

<400> 170

Met Glu Pro Leu Lys Ser His Thr Gly Lys Ala Ala Val Leu Asn Arg
1 5 10 15

Ile Asn Val Asp Thr Asp Gln Ile Ile Pro Lys Gln Phe Leu Lys Arg
20 25 30

Ile Glu Arg Thr Gly Tyr Gly Arg Phe Ala Phe Phe Asp Trp Arg Tyr
35 40 45

Asp Ala Asn Gly Glu Pro Asn Pro Glu Phe Glu Leu Asn Gln Pro Val
50 55 60

Tyr Gln Gly Ala Ser Ile Leu Ile Ala Gly Glu Asn Phe Gly Cys Gly
65 70 75 80

Ser Ser Arg Glu His Ala Pro Trp Ala Leu Asp Asp Tyr Gly Phe Lys
85 90 95

Ile Ile Ile Ala Pro Ser Phe Ala Asp Ile Phe His Gln Asn Cys Phe
100 105 110

Lys Asn Gly Met Leu Pro Ile Arg Met Pro Tyr Asp Asn Trp Lys Gln
115 120 125

Leu Val Gly Gln Tyr Glu Asn Gln Ser Leu Gln Met Thr Val Asp Leu
130 135 140

Glu Asn Gln Leu Ile His Asp Ser Glu Gly Asn Gln Ile Ser Phe Glu
145 150 155 160

Val Asp Pro His Trp Lys Glu Met Leu Ile Asn Gly Tyr Asp Glu Ile
165 170 175

Ser Leu Thr Leu Leu Leu Glu Asp Glu Ile Lys Gln Phe Glu Ser Gln
180 185 190

Arg Ser Ser Trp Leu Gln Ala
195

<210> 171
<211> 600
<212> DNA
<213> Bacillus subtilis

<220>
<221> misc_feature
<223> Encodes isopropylmalate isomerase small subunit

<400> 171
tcaggcttga agccaagagc ttctttgtga ttcaaattgc ttgatttcat cttccagcag 60

cagcgttaat gaaatttcat catatccgtt gatcagcatc tctttccaat gcggatcaac 120

ttcaaatgaa atttgattgc cttcactgtc atgaatcagc tgattttcaa ggtcaacagt 180

catttgcaat gactggtttt catactggcc gacaagctgt ttccaattgt catatggcat 240

gcggatcgga agcatgccgt ttttaaagca gttctgatgg aaaatatcag cgaatgacgg 300

cgcaatgata attttaaacc cataatcatc aagtgcccac ggagcgtgtt cacgcgatga 360

cccgcagccg aagttttctc ctgctattaa aatggaagct ccttgataaa caggctggtt 420

taattcaaat tcagggttcg gttcaccatt cgcatcatat ctccagtcaa agaatgcaaa 480

acgtccgtag cctgttcttt caatcctctt caaaaattgc ttaggaataa tctggtctgt 540

atccacattg atccgattta atacggctgc tttccccgta tgtgatttca aaggttccat 600

<210> 172
<211> 363
<212> PRT
<213> Escherichia coli

<220>
<221> MISC_FEATURE
<223> 3-isopropylmalate dehydrogenase

<400> 172

Met Ser Lys Asn Tyr His Ile Ala Val Leu Pro Gly Asp Gly Ile Gly
1               5                   10                  15

Pro Glu Val Met Thr Gln Ala Leu Lys Val Leu Asp Ala Val Arg Asn
            20                  25                  30

Arg Phe Ala Met Arg Ile Thr Thr Ser His Tyr Asp Val Gly Gly Ala
        35                  40                  45

Ala Ile Asp Asn His Gly Gln Pro Leu Pro Pro Ala Thr Val Glu Gly
    50                  55                  60

Cys Glu Gln Ala Asp Ala Val Leu Phe Gly Ser Val Gly Gly Pro Lys
65                  70                  75                  80

Trp Glu His Leu Pro Pro Asp Gln Gln Pro Glu Arg Gly Ala Leu Leu
                85                  90                  95

Pro Leu Arg Lys His Phe Lys Leu Phe Ser Asn Leu Arg Pro Ala Lys

100 105 110

Leu Tyr Gln Gly Leu Glu Ala Phe Cys Pro Leu Arg Ala Asp Ile Ala
115 120 125

Ala Asn Gly Phe Asp Ile Leu Cys Val Arg Glu Leu Thr Gly Gly Ile
130 135 140

Tyr Phe Gly Gln Pro Lys Gly Arg Glu Gly Ser Gly Gln Tyr Glu Lys
145 150 155 160

Ala Phe Asp Thr Glu Val Tyr His Arg Phe Glu Ile Glu Arg Ile Ala
165 170 175

Arg Ile Ala Phe Glu Ser Ala Arg Lys Arg Arg His Lys Val Thr Ser
180 185 190

Ile Asp Lys Ala Asn Val Leu Gln Ser Ser Ile Leu Trp Arg Glu Ile
195 200 205

Val Asn Glu Ile Ala Thr Glu Tyr Pro Asp Val Glu Leu Ala His Met
210 215 220

Tyr Ile Asp Asn Ala Thr Met Gln Leu Ile Lys Asp Pro Ser Gln Phe
225 230 235 240

Asp Val Leu Leu Cys Ser Asn Leu Phe Gly Asp Ile Leu Ser Asp Glu
245 250 255

Cys Ala Met Ile Thr Gly Ser Met Gly Met Leu Pro Ser Ala Ser Leu
260 265 270

Asn Glu Gln Gly Phe Gly Leu Tyr Glu Pro Ala Gly Gly Ser Ala Pro
275 280 285

Asp Ile Ala Gly Lys Asn Ile Ala Asn Pro Ile Ala Gln Ile Leu Ser
290 295 300

Leu Ala Leu Leu Leu Arg Tyr Ser Leu Asp Ala Asp Asp Ala Ala Cys
305 310 315 320

Ala Ile Glu Arg Ala Ile Asn Arg Ala Leu Glu Glu Gly Ile Arg Thr
325 330 335

Gly Asp Leu Ala Arg Gly Ala Ala Ala Val Ser Thr Asp Glu Met Gly
340 345 350

```
Asp Ile Ile Ala Arg Tyr Val Ala Glu Gly Val
        355                 360


<210>  173
<211>  1092
<212>  DNA
<213>  Escherichia coli


<220>
<221>  misc_feature
<223>  Encodes 3-isopropylmalate dehydrogenase

<400>  173
ttacacccct tctgctacat agcgggcaat gatatcgccc atttcatcgg tactaacggc      60

ggcagcgcca cgggctaaat ccccggtgcg aatgccttct tctaatgcgc ggttaatggc     120

gcgttcaatg gcgcaagccg catcatcggc atccaggctg taacgcagca gcagtgccag     180

cgaaaggatt tgtgcaatcg ggttggcgat gtttttgcct gcgatatctg gtgccgagcc     240

gcccgccggt tcatacagtc caaaaccttg ctcgttcagg ctggcggaag gcaacatccc     300

catcgagcca gtgatcattg cgcactcgtc agacagaatg tcgccaaaca ggttggagca     360

cagcagaacg tcaaactgtg atggatcttt aatcagctgc atggtggcgt tgtcgatgta     420

catatgcgcc agttcgacat ccgggtattc cgtggcgatc tcgttaacga tctcccgcca     480

taaaatagag gattgcagca cgttggcttt atcgatcgac gtcactttgt ggcgacgctt     540

gcgagcagat tcaaacgcga tgcgggcgat acgttcgatc tcaaaacggt gatacacctc     600

ggtatcaaag gctttttcat attgtccgct accttcgcgg ccttttggct gaccgaaata     660

gatgccgccg gtcagttcgc gcacacacag gatgtcgaag ccgtttgcgg caatgtctgc     720

acgcagcgga cagaatgctt ccagcccctg atacagtttt gccgggcgca ggttgctgaa     780

taatttgaag tgcttacgca gaggcagcag cgcgccgcgt tctggttgct ggtctggtgg     840

taaatgttcc cacttcgggc cgcctaccga gccaaacagc acggcatcgg cttgctcaca     900

accttcaacc gtcgcaggcg gcagtggttg cccgtggtta tcaatggctg cgccgcctac     960

atcgtaatgg ctggtggtga tgcgcatcgc aaagcggttg cgcacggcat ccagcacttt    1020

cagcgcctgg gtcatcactt ccggaccaat accgtccccc ggcaatacgg caatatggta    1080

attcttcgac at                                                        1092


<210>  174
<211>  365
<212>  PRT
<213>  Bacillus subtilis


<220>
<221>  MISC_FEATURE
<223>  3-isopropylmalate dehydrogenase
```

<400> 174

```
Met Lys Lys Arg Ile Ala Leu Leu Pro Gly Asp Gly Ile Gly Pro Glu
1               5                   10                  15

Val Leu Glu Ser Ala Thr Asp Val Leu Lys Ser Val Ala Glu Arg Phe
            20                  25                  30

Asn His Glu Phe Glu Phe Glu Tyr Gly Leu Ile Gly Gly Ala Ala Ile
        35                  40                  45

Asp Glu His His Asn Pro Leu Pro Glu Glu Thr Val Ala Ala Cys Lys
    50                  55                  60

Asn Ala Asp Ala Ile Leu Leu Gly Ala Val Gly Gly Pro Lys Trp Asp
65                  70                  75                  80

Gln Asn Pro Ser Glu Leu Arg Pro Glu Lys Gly Leu Leu Ser Ile Arg
                85                  90                  95

Lys Gln Leu Asp Leu Phe Ala Asn Leu Arg Pro Val Lys Val Phe Glu
            100                 105                 110

Ser Leu Ser Asp Ala Ser Pro Leu Lys Lys Glu Tyr Ile Asp Asn Val
        115                 120                 125

Asp Phe Val Ile Val Arg Glu Leu Thr Gly Gly Leu Tyr Phe Gly Gln
    130                 135                 140

Pro Ser Lys Arg Tyr Val Asn Thr Glu Gly Glu Gln Glu Ala Val Asp
145                 150                 155                 160

Thr Leu Phe Tyr Lys Arg Thr Glu Ile Glu Arg Val Ile Arg Glu Gly
                165                 170                 175

Phe Lys Met Ala Ala Ala Arg Lys Gly Lys Val Thr Ser Val Asp Lys
            180                 185                 190

Ala Asn Val Leu Glu Ser Ser Arg Leu Trp Arg Glu Val Ala Glu Asp
        195                 200                 205

Val Ala Gln Glu Phe Pro Asp Val Lys Leu Glu His Met Leu Val Asp
    210                 215                 220

Asn Ala Ala Met Gln Leu Ile Tyr Ala Pro Asn Gln Phe Asp Val Val
225                 230                 235                 240
```

Val Thr Glu Asn Met Phe Gly Asp Ile Leu Ser Asp Glu Ala Ser Met
245 250 255

Leu Thr Gly Ser Leu Gly Met Leu Pro Ser Ala Ser Leu Ser Ser Ser
260 265 270

Gly Leu His Leu Phe Glu Pro Val His Gly Ser Ala Pro Asp Ile Ala
275 280 285

Gly Lys Gly Met Ala Asn Pro Phe Ala Ala Ile Leu Ser Ala Ala Met
290 295 300

Leu Leu Arg Thr Ser Phe Gly Leu Glu Glu Glu Ala Lys Ala Val Glu
305 310 315 320

Asp Ala Val Asn Lys Val Leu Ala Ser Gly Lys Arg Thr Arg Asp Leu
325 330 335

Ala Arg Ser Glu Glu Phe Ser Ser Thr Gln Ala Ile Thr Glu Glu Val
340 345 350

Lys Ala Ala Ile Met Ser Glu Asn Thr Ile Ser Asn Val
355 360 365

<210> 175
<211> 1098
<212> DNA
<213> Bacillus subtilis

<220>
<221> misc_feature
<223> Encodes 3-isopropylmalate dehydrogenase

<400> 175
tcacacatta gaaattgtat tttcactcat gattgctgcc ttaacttcct ctgtaatggc 60

ctgagtgctg ctgaactctt cactccgtgc caagtctctt gttctttttc cagaagccaa 120

gactttgttt accgcatctt ctacagcttt cgcttcctct tcaagcccga aagatgttct 180

caaaagcatt gccgctgaca atatggctgc gaacggattt gccatccctt taccggcaat 240

atcaggcgcg gagccatgaa caggttcaaa cagatgaagg ccagagcttg ataggctggc 300

tgacgggagc attccgagcg agcctgtaag catggacgct tcatcgctta aaatatcacc 360

gaacatattt tcagtcacca cgacatcaaa ttgattcggt gcataaatta gctgcatggc 420

cgcgttatcc acaagcatgt gctcaagctt cacatcagga aattcttgtg caacgtcctc 480

agccacttca cgccacagcc ggcttgattc aagaacattc gctttatcta cagaggtcac 540

tttgcctttt ctggctgccg ccattttgaa gccctctctg atcactcgtt caatttccgt 600

tcgcttataa aacagtgtat ctactgcttc ctgctcacct tcagtgttta cataacgttt 660

gctcggctgg ccgaaataca agccgcctgt gagctcacga acgataacga aatcaacatt 720

atctatatat tctttttca aaggcgaagc gtcagaaagg ctttcaaata ccttcacagg 780

ccgcaaattc gcaaacaaat caagctgttt tctgatgctg agcagccctt tttccggtct 840

cagttccgaa ggattttgat cccatttcgg tccgccgaca gcaccaagca atatcgcgtc 900

tgcattttta caagcagcaa cggtttcctc cgggaggggg ttatgatgtt catcaatagc 960

cgcccctcca atcaggccat attcaaattc aaattcatgg ttaaagcgtt cggcaacact 1020

tttcagtacg tctgtcgctg attctaatac ttcagggccg atcccgtctc cgggcaatag 1080

agcaatacgt ttcttcaa 1098

<210> 176
<211> 364
<212> PRT
<213> Saccharomyces cerevisiae

<220>
<221> MISC_FEATURE
<223> 3-isopropylmalate dehydrogenase

<400> 176

Met Ser Ala Pro Lys Lys Ile Val Val Leu Pro Gly Asp His Val Gly
1               5                   10                  15

Gln Glu Ile Thr Ala Glu Ala Ile Lys Val Leu Lys Ala Ile Ser Asp
            20                  25                  30

Val Arg Ser Asn Val Lys Phe Asp Phe Glu Asn His Leu Ile Gly Gly
        35                  40                  45

Ala Ala Ile Asp Ala Thr Gly Val Pro Leu Pro Asp Glu Ala Leu Glu
    50                  55                  60

Ala Ser Lys Lys Ala Asp Ala Val Leu Leu Gly Ala Val Gly Gly Pro
65                  70                  75                  80

Lys Trp Gly Thr Gly Ser Val Arg Pro Glu Gln Gly Leu Leu Lys Ile
                85                  90                  95

Arg Lys Glu Leu Gln Leu Tyr Ala Asn Leu Arg Pro Cys Asn Phe Ala
            100                 105                 110

Ser Asp Ser Leu Leu Asp Leu Ser Pro Ile Lys Pro Gln Phe Ala Lys
        115                 120                 125

```
Gly Thr Asp Phe Val Val Val Arg Glu Leu Val Gly Gly Ile Tyr Phe
    130                 135                 140

Gly Lys Arg Lys Glu Asp Asp Gly Asp Gly Val Ala Trp Asp Ser Glu
145                 150                 155                 160

Gln Tyr Thr Val Pro Glu Val Gln Arg Ile Thr Arg Met Ala Ala Phe
                165                 170                 175

Met Ala Leu Gln His Glu Pro Pro Leu Pro Ile Trp Ser Leu Asp Lys
            180                 185                 190

Ala Asn Val Leu Ala Ser Ser Arg Leu Trp Arg Lys Thr Val Glu Glu
        195                 200                 205

Thr Ile Lys Asn Glu Phe Pro Thr Leu Lys Val Gln His Gln Leu Ile
    210                 215                 220

Asp Ser Ala Ala Met Ile Leu Val Lys Asn Pro Thr His Leu Asn Gly
225                 230                 235                 240

Ile Ile Ile Thr Ser Asn Met Phe Gly Asp Ile Ile Ser Asp Glu Ala
                245                 250                 255

Ser Val Ile Pro Gly Ser Leu Gly Leu Leu Pro Ser Ala Ser Leu Ala
            260                 265                 270

Ser Leu Pro Asp Lys Asn Thr Ala Phe Gly Leu Tyr Glu Pro Cys His
        275                 280                 285

Gly Ser Ala Pro Asp Leu Pro Lys Asn Lys Val Asn Pro Ile Ala Thr
    290                 295                 300

Ile Leu Ser Ala Ala Met Met Leu Lys Leu Ser Leu Asn Leu Pro Glu
305                 310                 315                 320

Glu Gly Lys Ala Ile Glu Asp Ala Val Lys Lys Val Leu Asp Ala Gly
                325                 330                 335

Ile Arg Thr Gly Asp Leu Gly Gly Ser Asn Ser Thr Thr Glu Val Gly
            340                 345                 350

Asp Ala Val Ala Glu Glu Val Lys Lys Ile Leu Ala
        355                 360
```

<210> 177
<211> 1095
<212> DNA

<213> Saccharomyces cerevisiae

<220>
<221> misc_feature
<223> Encodes 3-isopropylmalate dehydrogenase

<400> 177
atgtctgccc ctaagaagat cgtcgttttg ccaggtgacc acgttggtca agaaatcaca 60

gccgaagcca ttaaggttct taaagctatt tctgatgttc gttccaatgt caagttcgat 120

ttcgaaaatc atttaattgg tggtgctgct atcgatgcta caggtgttcc acttccagat 180

gaggcgctgg aagcctccaa gaaggctgat gccgttttgt taggtgctgt gggtggtcct 240

aaatggggta ccggtagtgt tagacctgaa caaggtttac taaaaatccg taaagaactt 300

caattgtacg ccaacttaag accatgtaac tttgcatccg actctctttt agacttatct 360

ccaatcaagc cacaatttgc taaaggtact gacttcgttg ttgtcagaga attagtggga 420

ggtatttact ttggtaagag aaaggaagac gatggtgatg gtgtcgcttg ggatagtgaa 480

caatacaccg ttccagaagt gcaaagaatc acaagaatgg ccgctttcat ggccctacaa 540

catgagccac cattgcctat ttggtccttg gataaagcta atgttttggc ctcttcaaga 600

ttatggagaa aaactgtgga ggaaaccatc aagaacgaat tccctacatt gaaggttcaa 660

catcaattga ttgattctgc cgccatgatc ctagttaaga acccaaccca cctaaatggt 720

attataatca ccagcaacat gtttggtgat atcatctccg atgaagcctc cgttatccca 780

ggttccttgg gtttgttgcc atctgcgtcc ttggcctctt tgccagacaa gaacaccgca 840

tttggtttgt acgaaccatg ccacggttct gctccagatt tgccaaagaa taaggtcaac 900

cctatcgcca ctatcttgtc tgctgcaatg atgttgaaat tgtcattgaa cttgcctgaa 960

gaaggtaagg ccattgaaga tgcagttaaa aaggttttgg atgcaggtat cagaactggt 1020

gatttaggtg gttccaacag taccaccgaa gtcggtgatg ctgtcgccga agaagttaag 1080

aaaatccttg cttaa 1095

<210> 178
<211> 562
<212> PRT
<213> Escherichia coli

<220>
<221> MISC_FEATURE
<223> acetohydroxyacid synthase I, large subunit

<400> 178

Met Ala Ser Ser Gly Thr Thr Ser Thr Arg Lys Arg Phe Thr Gly Ala
1 5 10 15

```
Glu Phe Ile Val His Phe Leu Glu Gln Gln Gly Ile Lys Ile Val Thr
            20                  25                  30

Gly Ile Pro Gly Gly Ser Ile Leu Pro Val Tyr Asp Ala Leu Ser Gln
        35                  40                  45

Ser Thr Gln Ile Arg His Ile Leu Ala Arg His Glu Gln Gly Ala Gly
    50                  55                  60

Phe Ile Ala Gln Gly Met Ala Arg Thr Asp Gly Lys Pro Ala Val Cys
65                  70                  75                  80

Met Ala Cys Ser Gly Pro Gly Ala Thr Asn Leu Val Thr Ala Ile Ala
                85                  90                  95

Asp Ala Arg Leu Asp Ser Ile Pro Leu Ile Cys Ile Thr Gly Gln Val
            100                 105                 110

Pro Ala Ser Met Ile Gly Thr Asp Ala Phe Gln Glu Val Asp Thr Tyr
        115                 120                 125

Gly Ile Ser Ile Pro Ile Thr Lys His Asn Tyr Leu Val Arg His Ile
    130                 135                 140

Glu Glu Leu Pro Gln Val Met Ser Asp Ala Phe Arg Ile Ala Gln Ser
145                 150                 155                 160

Gly Arg Pro Gly Pro Val Trp Ile Asp Ile Pro Lys Asp Val Gln Thr
                165                 170                 175

Ala Val Phe Glu Ile Glu Thr Gln Pro Ala Met Ala Glu Lys Ala Ala
            180                 185                 190

Ala Pro Ala Phe Ser Glu Glu Ser Ile Arg Asp Ala Ala Ala Met Ile
        195                 200                 205

Asn Ala Ala Lys Arg Pro Val Leu Tyr Leu Gly Gly Gly Val Ile Asn
    210                 215                 220

Ala Pro Ala Arg Val Arg Glu Leu Ala Glu Lys Ala Gln Leu Pro Thr
225                 230                 235                 240

Thr Met Thr Leu Met Ala Leu Gly Met Leu Pro Lys Ala His Pro Leu
                245                 250                 255

Ser Leu Gly Met Leu Gly Met His Gly Val Arg Ser Thr Asn Tyr Ile
            260                 265                 270
```

```
Leu Gln Glu Ala Asp Leu Leu Ile Val Leu Gly Ala Arg Phe Asp Asp
        275                 280                 285

Arg Ala Ile Gly Lys Thr Glu Gln Phe Cys Pro Asn Ala Lys Ile Ile
    290                 295                 300

His Val Asp Ile Asp Arg Ala Glu Leu Gly Lys Ile Lys Gln Pro His
305                 310                 315                 320

Val Ala Ile Gln Ala Asp Val Asp Asp Val Leu Ala Gln Leu Ile Pro
                325                 330                 335

Leu Val Glu Ala Gln Pro Arg Ala Glu Trp His Gln Leu Val Ala Asp
            340                 345                 350

Leu Gln Arg Glu Phe Pro Cys Pro Ile Pro Lys Ala Cys Asp Pro Leu
        355                 360                 365

Ser His Tyr Gly Leu Ile Asn Ala Val Ala Ala Cys Val Asp Asp Asn
    370                 375                 380

Ala Ile Ile Thr Thr Asp Val Gly Gln His Gln Met Trp Thr Ala Gln
385                 390                 395                 400

Ala Tyr Pro Leu Asn Arg Pro Arg Gln Trp Leu Thr Ser Gly Gly Leu
                405                 410                 415

Gly Thr Met Gly Phe Gly Leu Pro Ala Ala Ile Gly Ala Ala Leu Ala
            420                 425                 430

Asn Pro Asp Arg Lys Val Leu Cys Phe Ser Gly Asp Gly Ser Leu Met
        435                 440                 445

Met Asn Ile Gln Glu Met Ala Thr Ala Ser Glu Asn Gln Leu Asp Val
    450                 455                 460

Lys Ile Ile Leu Met Asn Asn Glu Ala Leu Gly Leu Val His Gln Gln
465                 470                 475                 480

Gln Ser Leu Phe Tyr Glu Gln Gly Val Phe Ala Ala Thr Tyr Pro Gly
                485                 490                 495

Lys Ile Asn Phe Met Gln Ile Ala Ala Gly Phe Gly Leu Glu Thr Cys
            500                 505                 510

Asp Leu Asn Asn Glu Ala Asp Pro Gln Ala Ser Leu Gln Glu Ile Ile
        515                 520                 525
```

Asn Arg Pro Gly Pro Ala Leu Ile His Val Arg Ile Asp Ala Glu Glu
    530                 535                 540

Lys Val Tyr Pro Met Val Pro Pro Gly Ala Ala Asn Thr Glu Met Val
545                 550                 555                 560

Gly Glu

<210>  179
<211>  1689
<212>  DNA
<213>  Escherichia coli

<220>
<221>  misc_feature
<223>  Encodes acetohydroxyacid synthase I, large subunit

<400>  179
ttattccccc accatttcag tattcgccgc acctggcggc accatcgggt aaactttttc        60

ttcggcatca atgcgcacat ggatcagcgc cgggccaggg cgattgatga tttcctgcaa       120

tgaagcctgc ggatcggctt cgttattcaa atcacaggtt tcgaggccga atccggcggc       180

aatctgcata aagttgattt tgcccggata ggtggcggca aaaacgcctt gctcgtagaa       240

cagactctgt tgctgatgca ccagccccag cgcttcgttg ttcatcagaa tgattttgac       300

atccagctga ttttcactgg cggtcgccat ctcctgaata ttcatcatca ggctgccgtc       360

gccggagaaa cacaacactt tgcgatccgg gttcgccagc gcagcgccaa tcgccgcagg       420

caggccaaaa cccatcgtgc ccagcccacc ggaggtcagc cactggcgtg ggcgattgag       480

cggataagct tgcgcggtcc acatctgatg ctgaccaacg tcggtggtga taattgcatt       540

gtcatcgaca caggcggcaa cggcgttgat caggccgtaa tggcttaacg gatcgcacgc       600

tttcgggatt ggacacggaa actcacgctg caaatccgct accaactggt gccactctgc       660

acgcggttgc gcttccacca gcgggatcaa ctgcgccagc acgtcatcaa catccgcctg       720

aatcgccacg tgcggctgct tgattttacc cagctctgca cggtcgatat cgacatgaat       780

gattttggca ttcggacaga actgctcggt tttgccaatc gcccggtcat caaaacgcgc       840

accgagcact atcaacaaat ccgcctcctg caaaatatag ttggtgctgc gcacgccgtg       900

catccccagc atacccagcg acaacggatg cgcttttggc aacatgccca gcgccattaa       960

agtcatggtg gtaggcagtt gcgctttctc cgccagttca cgcacccgtg cgggcgcatt      1020

gatcacaccg ccgcccagat aaagcaccgg gcgtttggca gcgttaatca tcgccgctgc      1080

gtcacgaatg ctttcttcgc taaaggcggg ggcggcggct ttttctgcca tagcgggctg      1140

tgtttcaatc tcaaaaactg ccgtttgcac atccttagga atgtctatcc acaccgggcc 1200

tgggcggcct gattgcgcaa tgcggaaggc atcgctcatg acctgcggga gttcttcgat 1260

atgtctgacc agatagttgt gtttggtgat ggggatagag atgccgtagg tgtccacttc 1320

ctggaaggcg tcggtgccga tcatcgaggc gggaacctga ccagtgatgc aaatcagcgg 1380

gatggagtcc agccgcgcat cggcaatggc ggtcaccagg ttagtcgcac ccggtccgct 1440

acaggccata cagaccgccg gtttaccgtc ggtgcgcgcc attccctgag cgataaagcc 1500

cgcgccctgt tcatgacggg ccagaatatg gcggatttgc gtgctttggc ttaaggcatc 1560

gtaaacaggc aggatagaac cgcccggaat gcctgtcaca atcttaatgc cctgctgttc 1620

caggaaatga acgataaatt ctgcgccggt aaagcgctta cgcgtcgatg ttgtgcccga 1680

acttgccat 1689

<210> 180
<211> 96
<212> PRT
<213> Escherichia coli

<220>
<221> MISC_FEATURE
<223> acetohydroxyacid synthase I, small subunit

<400> 180

Met Gln Asn Thr Thr His Asp Asn Val Ile Leu Glu Leu Thr Val Arg
1 5 10 15

Asn His Pro Gly Val Met Thr His Val Cys Gly Leu Phe Ala Arg Arg
20 25 30

Ala Phe Asn Val Glu Gly Ile Leu Cys Leu Pro Ile Gln Asp Ser Asp
35 40 45

Lys Ser His Ile Trp Leu Leu Val Asn Asp Asp Gln Arg Leu Glu Gln
50 55 60

Met Ile Ser Gln Ile Asp Lys Leu Glu Asp Val Val Lys Val Gln Arg
65 70 75 80

Asn Gln Ser Asp Pro Thr Met Phe Asn Lys Ile Ala Val Phe Phe Gln
85 90 95

<210> 181
<211> 291
<212> DNA
<213> Escherichia coli

<220>
<221> misc_feature
<223> Encodes acetohydroxyacid synthase I, small subunit

<400> 181
ttactgaaaa aacaccgcga tcttgttaaa catcgtcgga tcggactgat tacgctgcac 60

tttcacgaca tcttccagct tatcgatttg gcttatcatc tgctccagac gctggtcgtc 120

attgaccagt agccagatat ggcttttgtc gctgtcctga atcggcagac aaagaatgcc 180

ttcaacgtta aaagcgcggc gggcaaaaag gccacaaacg tgggtcatta cgcccggatg 240

gttgcgaacg gtgagctcca gaattacgtt gtcatgagtt gtgttttgca t 291

<210> 182
<211> 548
<212> PRT
<213> Escherichia coli

<220>
<221> MISC_FEATURE
<223> acetohydroxyacid synthase II, large subunit

<400> 182

Met Asn Gly Ala Gln Trp Val Val His Ala Leu Arg Ala Gln Gly Val
1 5 10 15

Asn Thr Val Phe Gly Tyr Pro Gly Gly Ala Ile Met Pro Val Tyr Asp
20 25 30

Ala Leu Tyr Asp Gly Gly Val Glu His Leu Leu Cys Arg His Glu Gln
35 40 45

Gly Ala Ala Met Ala Ala Ile Gly Tyr Ala Arg Ala Thr Gly Lys Thr
50 55 60

Gly Val Cys Ile Ala Thr Ser Gly Pro Gly Ala Thr Asn Leu Ile Thr
65 70 75 80

Gly Leu Ala Asp Ala Leu Leu Asp Ser Ile Pro Val Val Ala Ile Thr
85 90 95

Gly Gln Val Ser Ala Pro Phe Ile Gly Thr Asp Ala Phe Gln Glu Val
100 105 110

Asp Val Leu Gly Leu Ser Leu Ala Cys Thr Lys His Ser Phe Leu Val
115 120 125

Gln Ser Leu Glu Glu Leu Pro Arg Ile Met Ala Glu Ala Phe Asp Val
130 135 140

```
Ala Cys Ser Gly Arg Pro Gly Pro Val Leu Val Asp Ile Pro Lys Asp
145                 150                 155                 160

Ile Gln Leu Ala Ser Gly Asp Leu Glu Pro Trp Phe Thr Thr Val Glu
                165                 170                 175

Asn Glu Val Thr Phe Pro His Ala Glu Val Glu Gln Ala Arg Gln Met
            180                 185                 190

Leu Ala Lys Ala Gln Lys Pro Met Leu Tyr Val Gly Gly Gly Val Gly
        195                 200                 205

Met Ala Gln Ala Val Pro Ala Leu Arg Glu Phe Leu Ala Ala Thr Lys
    210                 215                 220

Met Pro Ala Thr Cys Thr Leu Lys Gly Leu Gly Ala Val Glu Ala Asp
225                 230                 235                 240

Tyr Pro Tyr Tyr Leu Gly Met Leu Gly Met His Gly Thr Lys Ala Ala
                245                 250                 255

Asn Phe Ala Val Gln Glu Cys Asp Leu Leu Ile Ala Val Gly Ala Arg
            260                 265                 270

Phe Asp Asp Arg Val Thr Gly Lys Leu Asn Thr Phe Ala Pro His Ala
        275                 280                 285

Ser Val Ile His Met Asp Ile Asp Pro Ala Glu Met Asn Lys Leu Arg
    290                 295                 300

Gln Ala His Val Ala Leu Gln Gly Asp Leu Asn Ala Leu Leu Pro Ala
305                 310                 315                 320

Leu Gln Gln Pro Leu Asn Ile Asn Asp Trp Gln Leu His Cys Ala Gln
                325                 330                 335

Leu Arg Asp Glu His Ala Trp Arg Tyr Asp His Pro Gly Asp Ala Ile
            340                 345                 350

Tyr Ala Pro Leu Leu Leu Lys Gln Leu Ser Asp Arg Lys Pro Ala Asp
        355                 360                 365

Cys Val Val Thr Thr Asp Val Gly Gln His Gln Met Trp Ala Ala Gln
    370                 375                 380

His Ile Ala His Thr Arg Pro Glu Asn Phe Ile Thr Ser Ser Gly Leu
385                 390                 395                 400
```

Gly Thr Met Gly Phe Gly Leu Pro Ala Ala Val Gly Ala Gln Val Ala
405 410 415

Arg Pro Asn Asp Thr Val Val Cys Ile Ser Gly Asp Gly Ser Phe Met
420 425 430

Met Asn Val Gln Glu Leu Gly Thr Val Lys Arg Lys Gln Leu Pro Leu
435 440 445

Lys Ile Val Leu Leu Asp Asn Gln Arg Leu Gly Met Val Arg Gln Trp
450 455 460

Gln Gln Leu Phe Phe Gln Glu Arg Tyr Ser Glu Thr Thr Leu Thr Asp
465 470 475 480

Asn Pro Asp Phe Leu Met Leu Ala Ser Ala Phe Gly Ile Pro Gly Gln
485 490 495

His Ile Thr Arg Lys Asp Gln Val Glu Ala Ala Leu Asp Thr Met Leu
500 505 510

Asn Ser Asp Gly Pro Tyr Leu Leu His Val Ser Ile Asp Glu Leu Glu
515 520 525

Asn Val Trp Pro Leu Val Pro Pro Gly Ala Ser Asn Ser Glu Met Leu
530 535 540

Glu Lys Leu Ser
545

<210> 183
<211> 1647
<212> DNA
<213> Escherichia coli

<220>
<221> misc_feature
<223> Encodes acetohydroxyacid synthase II, large subunit

<400> 183
atgaatggcg cacagtgggt ggtacatgcg ttgcgggcac agggtgtgaa caccgttttc 60

ggttatccgg gtggcgcaat tatgccggtt tacgatgcat tgtatgacgg cggcgtggag 120

cacttgctat gccgacatga gcagggtgcg gcaatggcgg ctatcggtta tgctcgtgct 180

accggcaaaa ctggcgtatg tatcgccacg tctggtccgg gcgcaaccaa cctgataacc 240

gggcttgcgg acgcactgtt agattccatc cctgttgttg ccatcaccgg tcaagtgtcc 300

gcaccgttta tcggcactga cgcatttcag gaagtggatg tcctgggatt gtcgttagcc 360

tgtaccaagc acagctttct ggtgcagtcg ctggaagagt tgccgcgcat catggctgaa 420

gcattcgacg ttgcctgctc aggtcgtcct ggtccggttc tggtcgatat cccaaaagat 480

atccagttag ccagcggtga cctggaaccg tggttcacca ccgttgaaaa cgaagtgact 540

ttcccacatg ccgaagttga gcaagcgcgc cagatgctgg caaaagcgca aaaaccgatg 600

ctgtacgttg gcggtggcgt gggtatggcg caggcagttc cggctttgcg tgaatttctc 660

gctgccacaa aaatgcctgc cacctgtacg ctgaaagggc tgggcgcagt agaagcagat 720

tatccgtact atctgggcat gctgggaatg catggcacca aagcggcgaa cttcgcggtg 780

caggagtgcg acttgctgat cgccgtgggt gcacgttttg atgaccgggt gaccggcaaa 840

ctgaacacct tcgcaccaca cgccagtgtt atccatatgg atatcgaccc ggcagaaatg 900

aacaagctgc gtcaggcaca tgtggcatta caaggtgatt taaatgctct gttaccagca 960

ttacagcagc cgttaaatat caatgactgg cagctacact gcgcgcagct gcgtgatgaa 1020

catgcctggc gttacgacca tcccggtgac gctatctacg cgccgttgtt gttaaaacaa 1080

ctgtcagatc gtaaacctgc ggattgcgtc gtgaccacag atgtggggca gcaccagatg 1140

tgggctgcgc agcacatcgc ccacactcgc ccggaaaatt tcatcacctc cagcggctta 1200

ggcaccatgg gttttggttt accggcggcg gttggcgcgc aagtcgcgcg accaaacgat 1260

accgtcgtct gtatctccgg tgacggctct ttcatgatga atgtgcaaga gctgggcacc 1320

gtaaaacgca agcagttacc gttgaaaatc gtcttactcg ataaccaacg gttagggatg 1380

gttcgacaat ggcagcaact gtttttccag gaacgatata gcgaaaccac ccttaccgat 1440

aaccccgatt tcctcatgtt agccagcgcc ttcggcatcc ctggccaaca catcacccgt 1500

aaagaccagg ttgaagcggc actcgacacc atgctgaaca gtgatgggcc atacctgctt 1560

catgtctcaa tcgacgaact tgagaacgtc tggccgctgg tgccgcctgg tgccagtaat 1620

tcagaaatgt tggagaaatt atcatga 1647

<210> 184
<211> 87
<212> PRT
<213> Escherichia coli

<220>
<221> MISC_FEATURE
<223> acetohydroxyacid synthase II, small subunit

<400> 184

Met Met Gln His Gln Val Asn Val Ser Ala Arg Phe Asn Pro Glu Thr
1 5 10 15

Leu Glu Arg Val Leu Arg Val Val Arg His Arg Gly Phe His Val Cys
20 25 30

Ser Met Asn Met Ala Ala Ala Ser Asp Ala Gln Asn Ile Asn Ile Glu
35 40 45

Leu Thr Val Ala Ser Pro Arg Ser Val Asp Leu Leu Phe Ser Gln Leu
50 55 60

Asn Lys Leu Val Asp Val Ala His Val Ala Ile Cys Gln Ser Thr Thr
65 70 75 80

Thr Ser Gln Gln Ile Arg Ala
85

<210> 185
<211> 264
<212> DNA
<213> Escherichia coli

<220>
<221> misc_feature
<223> Encodes acetohydroxyacid synthase II, small subunit

<400> 185
atgatgcaac atcaggtcaa tgtatcagct cgcttcaatc cggaaacctt agaacgtgtt 60

ttacgcgtgg tgcgtcatcg tggtttccac gtctgctcaa tgaatatggc cgccgccagc 120

gatgcacaaa atataaatat cgaattgacc gttgccagcc cacggtcggt cgacttactg 180

tttagtcagt taaataaact ggtggacgtc gcacacgttg ccatctgcca gagcacaacc 240

acatcacaac aaatccgcgc ctga 264

<210> 186
<211> 574
<212> PRT
<213> Escherichia coli

<220>
<221> MISC_FEATURE
<223> acetohydroxyacid synthase III, large subunit

<400> 186

Met Glu Met Leu Ser Gly Ala Glu Met Val Val Arg Ser Leu Ile Asp
1 5 10 15

Gln Gly Val Lys Gln Val Phe Gly Tyr Pro Gly Gly Ala Val Leu Asp
20 25 30

Ile Tyr Asp Ala Leu His Thr Val Gly Gly Ile Asp His Val Leu Val

35 40 45

Arg His Glu Gln Ala Ala Val His Met Ala Asp Gly Leu Ala Arg Ala
50 55 60

Thr Gly Glu Val Gly Val Val Leu Val Thr Ser Gly Pro Gly Ala Thr
65 70 75 80

Asn Ala Ile Thr Gly Ile Ala Thr Ala Tyr Met Asp Ser Ile Pro Leu
85 90 95

Val Val Leu Ser Gly Gln Val Ala Thr Ser Leu Ile Gly Tyr Asp Ala
100 105 110

Phe Gln Glu Cys Asp Met Val Gly Ile Ser Arg Pro Val Val Lys His
115 120 125

Ser Phe Leu Val Lys Gln Thr Glu Asp Ile Pro Gln Val Leu Lys Lys
130 135 140

Ala Phe Trp Leu Ala Ala Ser Gly Arg Pro Gly Pro Val Val Val Asp
145 150 155 160

Leu Pro Lys Asp Ile Leu Asn Pro Ala Asn Lys Leu Pro Tyr Val Trp
165 170 175

Pro Glu Ser Val Ser Met Arg Ser Tyr Asn Pro Thr Thr Thr Gly His
180 185 190

Lys Gly Gln Ile Lys Arg Ala Leu Gln Thr Leu Val Ala Ala Lys Lys
195 200 205

Pro Val Val Tyr Val Gly Gly Gly Ala Ile Thr Ala Gly Cys His Gln
210 215 220

Gln Leu Lys Glu Thr Val Glu Ala Leu Asn Leu Pro Val Val Cys Ser
225 230 235 240

Leu Met Gly Leu Gly Ala Phe Pro Ala Thr His Arg Gln Ala Leu Gly
245 250 255

Met Leu Gly Met His Gly Thr Tyr Glu Ala Asn Met Thr Met His Asn
260 265 270

Ala Asp Val Ile Phe Ala Val Gly Val Arg Phe Asp Asp Arg Thr Thr
275 280 285

```
Asn Asn Leu Ala Lys Tyr Cys Pro Asn Ala Thr Val Leu His Ile Asp
    290                 295                 300

Ile Asp Pro Thr Ser Ile Ser Lys Thr Val Thr Ala Asp Ile Pro Ile
305                 310                 315                 320

Val Gly Asp Ala Arg Gln Val Leu Glu Gln Met Leu Glu Leu Leu Ser
                325                 330                 335

Gln Glu Ser Ala His Gln Pro Leu Asp Glu Ile Arg Asp Trp Trp Gln
            340                 345                 350

Gln Ile Glu Gln Trp Arg Ala Arg Gln Cys Leu Lys Tyr Asp Thr His
        355                 360                 365

Ser Glu Lys Ile Lys Pro Gln Ala Val Ile Glu Thr Leu Trp Arg Leu
    370                 375                 380

Thr Lys Gly Asp Ala Tyr Val Thr Ser Asp Val Gly Gln His Gln Met
385                 390                 395                 400

Phe Ala Ala Leu Tyr Tyr Pro Phe Asp Lys Pro Arg Arg Trp Ile Asn
                405                 410                 415

Ser Gly Gly Leu Gly Thr Met Gly Phe Gly Leu Pro Ala Ala Leu Gly
            420                 425                 430

Val Lys Met Ala Leu Pro Glu Glu Thr Val Val Cys Val Thr Gly Asp
        435                 440                 445

Gly Ser Ile Gln Met Asn Ile Gln Glu Leu Ser Thr Ala Leu Gln Tyr
    450                 455                 460

Glu Leu Pro Val Leu Val Val Asn Leu Asn Asn Arg Tyr Leu Gly Met
465                 470                 475                 480

Val Lys Gln Trp Gln Asp Met Ile Tyr Ser Gly Arg His Ser Gln Ser
                485                 490                 495

Tyr Met Gln Ser Leu Pro Asp Phe Val Arg Leu Ala Glu Ala Tyr Gly
            500                 505                 510

His Val Gly Ile Gln Ile Ser His Pro His Glu Leu Glu Ser Lys Leu
        515                 520                 525

Ser Glu Ala Leu Glu Gln Val Arg Asn Asn Arg Leu Val Phe Val Asp
    530                 535                 540
```

Val Thr Val Asp Gly Ser Glu His Val Tyr Pro Met Gln Ile Arg Gly
545                 550                 555                 560

Gly Gly Met Asp Glu Met Trp Leu Ser Lys Thr Glu Arg Thr
                565                 570

<210> 187
<211> 1725
<212> DNA
<213> Escherichia coli

<220>
<221> misc_feature
<223> Encodes acetohydroxyacid synthase III, large subunit

<400> 187
atggagatgt tgtctggagc cgagatggtc gtccgatcgc ttatcgatca gggcgttaaa 60

caagtattcg gttatcccgg aggcgcagtc cttgatattt atgatgcatt gcataccgtg 120

ggtggtattg atcatgtatt agttcgtcat gagcaggcgg cggtgcatat ggccgatggc 180

ctggcgcgcg cgaccgggga agtcggcgtc gtgctggtaa cgtcgggtcc aggggcgacc 240

aatgcgatta ctggcatcgc caccgcttat atggattcca ttccattagt tgtcctttcc 300

gggcaggtag cgacctcgtt gataggttac gatgcctttc aggagtgcga catggtgggg 360

atttcgcgac cggtggttaa acacagtttt ctggttaagc aaacggaaga cattccgcag 420

gtgctgaaaa aggctttctg gctggcggca agtggtcgcc caggaccagt agtcgttgat 480

ttaccgaaag atattcttaa tccggcgaac aaattaccct atgtctggcc ggagtcggtc 540

agtatgcgtt cttacaatcc cactactacc ggacataaag ggcaaattaa gcgtgctctg 600

caaacgctgg tagcggcaaa aaaaccggtt gtctacgtag gcggtggggc aatcacggcg 660

ggctgccatc agcagttgaa agaaacggtg gaggcgttga atctgcccgt tgtttgctca 720

ttgatggggc tgggggcgtt tccggcaacg catcgtcagg cactgggcat gctgggaatg 780

cacggtacct acgaagccaa tatgacgatg cataacgcgg atgtgatttt cgccgtcggg 840

gtacgatttg atgaccgaac gacgaacaat ctggcaaagt actgcccaaa tgccactgtt 900

ctgcatatcg atattgatcc tacttccatt tctaaaaccg tgactgcgga tatcccgatt 960

gtgggggatg ctcgccaggt cctcgaacaa atgcttgaac tcttgtcgca agaatccgcc 1020

catcaaccac tggatgagat ccgcgactgg tggcagcaaa ttgaacagtg gcgcgctcgt 1080

cagtgcctga aatatgacac tcacagtgaa aagattaaac cgcaggcggt gatcgagact 1140

ctttggcggt tgacgaaggg agacgcttac gtgacgtccg atgtcgggca gcaccagatg 1200

tttgctgcac tttattatcc attcgacaaa ccgcgtcgct ggatcaattc cggtggcctc 1260

ggcacgatgg gttttggttt acctgcggca ctgggcgtca aaatggcgtt gccagaagaa 1320

accgtggttt gcgtcactgg cgacggcagt attcagatga acatccagga actgtctacc 1380

gcgttgcaat acgagttgcc cgtactggtg gtgaatctca ataaccgcta tctggggatg 1440

gtgaagcagt ggcaggacat gatctattcc ggccgtcatt cacaatctta tatgcaatcg 1500

ctacccgatt tcgtccgtct ggcggaagcc tatgggcatg tcgggatcca gatttctcat 1560

ccgcatgagc tggaaagcaa acttagcgag gcgctggaac aggtgcgcaa taatcgcctg 1620

gtgtttgttg atgttaccgt cgatggcagc gagcacgtct acccgatgca gattcgcggg 1680

ggcggaatgg atgaaatgtg gttaagcaaa acggagagaa cctga 1725

<210> 188
<211> 163
<212> PRT
<213> Escherichia coli

<220>
<221> MISC_FEATURE
<223> acetohydroxyacid synthase III, small subunit

<400> 188

Met Arg Arg Ile Leu Ser Val Leu Leu Glu Asn Glu Ser Gly Ala Leu
1 5 10 15

Ser Arg Val Ile Gly Leu Phe Ser Gln Arg Gly Tyr Asn Ile Glu Ser
20 25 30

Leu Thr Val Ala Pro Thr Asp Asp Pro Thr Leu Ser Arg Met Thr Ile
35 40 45

Gln Thr Val Gly Asp Glu Lys Val Leu Glu Gln Ile Glu Lys Gln Leu
50 55 60

His Lys Leu Val Asp Val Leu Arg Val Ser Glu Leu Gly Gln Gly Ala
65 70 75 80

His Val Glu Arg Glu Ile Met Leu Val Lys Ile Gln Ala Ser Gly Tyr
85 90 95

Gly Arg Asp Glu Val Lys Arg Asn Thr Glu Ile Phe Arg Gly Gln Ile
100 105 110

Ile Asp Val Thr Pro Ser Leu Tyr Thr Val Gln Leu Ala Gly Thr Ser
115 120 125

Gly Lys Leu Asp Ala Phe Leu Ala Ser Ile Arg Asp Val Ala Lys Ile
130 135 140

Val Glu Val Ala Arg Ser Gly Val Val Gly Leu Ser Arg Gly Asp Lys
145 150 155 160

Ile Met Arg

<210> 189
<211> 492
<212> DNA
<213> Escherichia coli

<220>
<221> misc_feature
<223> Encodes acetohydroxyacid synthase III, small subunit

<400> 189
atgcgccgga tattatcagt cttactcgaa aatgaatcag gcgcgttatc ccgcgtgatt 60

ggcctttttt cccagcgtgg ctacaacatt gaaagcctga ccgttgcgcc aaccgacgat 120

ccgacattat cgcgtatgac catccagacc gtgggcgatg aaaaagtact tgagcagatc 180

gaaaagcaat tacacaaact ggtcgatgtc ttgcgcgtga gtgagttggg gcagggcgcg 240

catgttgagc gggaaatcat gctggtgaaa attcaggcca gcggttacgg gcgtgacgaa 300

gtgaaacgta atacggaaat attccgtggg caaattatcg atgtcacacc ctcgctttat 360

accgttcaat tagcaggcac cagcggtaag cttgatgcat ttttagcatc gattcgcgat 420

gtggcgaaaa ttgtggaggt tgctcgctct ggtgtggtcg gactttcgcg cggcgataaa 480

ataatgcgtt ga 492

<210> 190
<211> 573
<212> PRT
<213> Listeria monocytogenes

<220>
<221> MISC_FEATURE
<223> acetohydroxyacid synthase, large subunit

<400> 190

Met Ile Asp Thr Thr Lys Thr Asn Glu Lys Ala Thr Lys Lys Gln Ser
1 5 10 15

Lys Ser Gly Ala Glu Leu Leu Ile Asp Ala Leu Lys Lys Gln Gln Val
20 25 30

Asp Met Ile Phe Gly Tyr Pro Gly Gly Ala Val Leu Pro Leu Tyr Asp
35 40 45

```
Ala Phe Tyr Asp Cys Asp Ile Pro His Ile Leu Thr Arg His Glu Gln
    50                  55                  60

Gly Ala Ile His Ala Ala Glu Gly Tyr Ala Arg Val Thr Gly Lys Pro
65                  70                  75                  80

Gly Val Val Val Val Thr Ser Gly Pro Gly Ala Thr Asn Val Leu Thr
                85                  90                  95

Gly Ile Ala Asp Ala Met Ser Asp Ser Ile Pro Leu Val Ile Phe Thr
            100                 105                 110

Gly Gln Val His Thr Pro Gly Ile Gly Lys Asp Ala Phe Gln Glu Ala
        115                 120                 125

Asp Met Ile Gly Leu Thr Ile Pro Ile Thr Lys Tyr Asn Tyr Gln Val
    130                 135                 140

Arg Asp Val Arg Asp Leu Pro Lys Ile Val Asn Glu Ala Phe His Ile
145                 150                 155                 160

Ala Ser Thr Gly Arg Lys Gly Pro Val Val Val Asp Ile Pro Lys Asp
                165                 170                 175

Met Gly Ile Ile Gln Thr Glu Ser Val Arg Pro Asp Thr Ile Asp Leu
            180                 185                 190

Pro Gly Tyr Gln Pro Thr Tyr Ser Pro Asn Pro Leu Gln Leu Glu Lys
        195                 200                 205

Leu Met Gln Ser Leu Ser Ala Ala Ser Lys Pro Leu Ile Leu Ala Gly
    210                 215                 220

Ala Gly Val Asn His Ala Arg Ala Thr Ala Glu Leu Leu Glu Phe Ala
225                 230                 235                 240

Glu Arg Tyr Gln Ile Pro Ile Val Asn Thr Leu Leu Gly Leu Gly Ser
                245                 250                 255

Phe Pro Gln Ser His Asp Leu Phe Leu Gly Met Gly Gly Met His Gly
            260                 265                 270

Ser Tyr Ala Ala Asn Met Ala Leu Thr Asp Cys Asp Leu Leu Ile Asn
        275                 280                 285

Phe Gly Ser Arg Phe Asp Asp Arg Leu Ala Ser Ala Pro Lys Glu Phe
    290                 295                 300
```

```
Ala Thr Lys Ala Thr Ile Ala His Ile Asp Ile Asp Pro Ala Glu Ile
305                 310                 315                 320

Gly Lys Ile Ile Glu Thr Gln Ile Pro Ile Val Ala Asp Ile Asn Glu
                325                 330                 335

Thr Leu Thr Gln Leu Leu Gln Met Glu Leu Pro Val His Pro Asp Thr
            340                 345                 350

Ser His Trp Tyr Lys Leu Asn Met Thr Arg Lys Asn Arg His Pro Phe
        355                 360                 365

Asn Phe Asp Arg Thr Lys Lys Ala Glu Ile Lys Pro Gln Arg Val Ile
    370                 375                 380

Glu Leu Ile Gly Glu Ile Thr Gln Gly Glu Ala Leu Val Ser Thr Asp
385                 390                 395                 400

Val Gly Gln His Gln Met Trp Ala Ala Gln Phe Tyr Pro Phe Gln Phe
                405                 410                 415

Asp His Gln Ile Ile Thr Ser Gly Gly Leu Gly Thr Met Gly Phe Gly
            420                 425                 430

Phe Pro Ala Ala Val Gly Ala Gln Leu Ala Phe Pro Asp Lys Thr Val
        435                 440                 445

Val Ala Ile Val Gly Asp Gly Gly Phe Gln Met Thr Asn Gln Glu Leu
    450                 455                 460

Ala Ile Leu Asn Asp Tyr Gln Ile Asn Val Lys Thr Val Ile Ile Asn
465                 470                 475                 480

Asn Gly Ser Leu Gly Met Val Arg Gln Trp Gln Glu Lys Phe His Gly
                485                 490                 495

Glu Arg Tyr Ser His Ser Ile Phe Thr Ser Gln Pro Asp Phe Val Lys
            500                 505                 510

Leu Ser Glu Ala Tyr Gly Val Lys Gly Val Arg Leu Thr Asn Pro Glu
        515                 520                 525

Thr Leu Glu Lys Asp Leu Arg Lys Ala Phe Ala His Pro Gly Pro Ile
    530                 535                 540

Val Ile Asp Val His Val Ala Lys Asp Glu Leu Val Leu Pro Met Val
545                 550                 555                 560
```

Pro Ser Gly Lys Pro Asn His Gln Met Glu Gly Val Glu
565 570

<210> 191
<211> 1722
<212> DNA
<213> Listeria monocytogenes

<220>
<221> misc_feature
<223> Encodes acetohydroxyacid synthase, large subunit

<400> 191
gtgattgata cgacgaagac aaatgaaaaa gcgacaaaaa agcaaagtaa aagtggagca 60

gaacttttaa ttgacgcctt aaaaaaacaa caagtagaca tgatttttgg ttatccgggt 120

ggcgcggttc ttcctctcta tgatgccttt tatgactgtg atattccgca tattctaaca 180

cgacatgagc aaggagcgat tcatgcggcg gaaggttatg ctcgtgttac cgggaaacct 240

ggtgtcgttg ttgtaactag tggacctggt gcaaccaacg tactaactgg tatcgcggat 300

gcaatgagtg actcgattcc attagtaatt tttaccggac aagttcatac gcctggtatc 360

ggaaaagatg ctttccaaga agccgatatg attgggctaa cgattccaat tactaaatac 420

aattaccaag tgcgtgatgt tcgtgactta ccaaaaatcg tcaatgaagc ttttcatatt 480

gcaagtactg gtcgtaaagg ccctgttgtc gtggatattc caaaagacat ggggattatc 540

caaacagagt ctgtccggcc agatacgatt gatctgccgg gttatcaacc gacttattca 600

ccgaatccac ttcaacttga aaaactaatg caatctcttt ccgccgcaag taagccacta 660

atacttgccg gagctggcgt caatcatgcg agagctacag ctgaattact agaattcgct 720

gaacgctatc aaattcctat cgtgaataca ctgcttggtc taggaagttt cccgcaaagc 780

catgatttat tccttggtat gggtggtatg cacggttctt atgcagcaaa tatggcgcta 840

acggattgcg acttgctgat taattttggc tctagattcg atgaccgtct tgcaagcgca 900

ccgaaagaat ttgccacgaa agcaaccatt gctcatatcg acattgatcc cgcggaaatc 960

ggcaaaatta tcgaaacgca aattccaatc gtggcggata tcaacgaaac gctcacgcaa 1020

ctactacaaa tggaacttcc ggttcatccg gatacatccc attggtacaa attaaatatg 1080

actcgtaaaa accgtcatcc ttttaatttt gatagaacga aaaaagcgga aattaaacca 1140

cagcgtgtca ttgagctaat tggtgaaatt acgcaaggtg aagcgcttgt ttctaccgat 1200

gttggacagc atcaaatgtg ggcggcgcag ttttatccat tccaattcga tcatcaaatt 1260

attacaagtg gtggtcttgg cacaatgggc tttggattcc ccgcagccgt tggggcacaa 1320

ctcgctttcc cagataaaac ggtagtcgca attgttggcg atggcggttt ccaaatgacc 1380

```
aaccaagaac tcgcgatttt aaacgattat caaatcaatg taaaaacggt cattatcaat     1440

aatggttcgc ttgggatggt tcgtcagtgg caagaaaaat tccacggtga aagatattcc     1500

cattcgattt tcacgagcca accagatttt gtcaaactat ctgaagctta cggtgtcaaa     1560

ggtgttcgcc tgacaaatcc ggaaacacta gaaaaagatt taagaaaagc gttcgctcac     1620

ccagggccaa tcgtaattga tgtccatgta gcaaaagacg aactagtact tccaatggtt     1680

ccaagcggca aaccaaatca ccaaatggaa ggggtggaat aa                        1722


<210>  192
<211>  163
<212>  PRT
<213>  Listeria monocytogenes


<220>
<221>  MISC_FEATURE
<223>  acetohydroxyacid synthase, small subunit

<400>  192

Met Arg Arg Ile Ile Thr Ala Thr Val Asn Asn Ser Ser Gly Val Leu
1               5                   10                  15


Asn Arg Ile Thr Gly Val Ile Ser Arg Arg Gln Tyr Asn Ile Asp Ser
            20                  25                  30


Ile Ser Val Gly Trp Thr Glu Ile Pro Asn Val Ser Arg Ile Thr Ile
        35                  40                  45


Val Val His Val Asp Ser Leu Tyr Glu Ile Glu Gln Val Thr Lys Gln
    50                  55                  60


Leu Asn Lys Gln Ile Asp Val Leu Lys Val Ser Asp Ile Thr Asp Asp
65                  70                  75                  80


Ala His Ile Glu Arg Glu Leu Ala Leu Leu Lys Ile Asn Ser Pro Ala
                85                  90                  95


Ala Leu Arg Ser Glu Leu Asn Ala Val Ile Glu Pro Phe Arg Ala Thr
            100                 105                 110


Val Ile Asp Val Gly Thr Lys Asn Val Val Ile Gln Val Thr Gly Thr
        115                 120                 125


Ser Glu Lys Ile Asp Ala Phe Val Asp Thr Val Arg Pro Tyr Gly Ile
    130                 135                 140


Lys Gln Met Ala Arg Thr Gly Val Thr Gly Phe Thr Arg Ser Pro Lys
145                 150                 155                 160
```

Lys Met Gly

<210> 193
<211> 492
<212> DNA
<213> Listeria monocytogenes

<220>
<221> misc_feature
<223> Encodes acetohydroxyacid synthase, small subunit

<400> 193
atgcgccgaa tcattaccgc tacagtaaac aactcttctg gcgtattaaa tcgtatcact 60

ggggttatct ccagacgcca atacaatatt gatagcatct ctgtcggctg gaccgaaatc 120

ccaaatgttt cccggattac catcgtcgtc catgtcgatt ccttatacga aatcgaacaa 180

gtaacaaaac agctcaacaa acaaattgac gtgctcaaag tgagcgatat tactgacgat 240

gcgcatatcg aacgcgaact agctttactt aaaatcaact cccccgccgc cttacgatct 300

gagttaaacg cagtcatcga accatttcgc gcaaccgtca ttgatgtcgg cacaaaaaat 360

gtcgtcattc aagtaactgg cacaagcgaa aaaattgatg ctttcgttga cacggttcgt 420

ccctacggca ttaaacaaat ggcaagaact ggcgtgactg gctttacaag aagccctaaa 480

aaaatgggtt ga 492

<210> 194
<211> 491
<212> PRT
<213> Escherichia coli

<220>
<221> MISC_FEATURE
<223> acetohydroxyacid isomeroreductase

<400> 194

Met Ala Asn Tyr Phe Asn Thr Leu Asn Leu Arg Gln Gln Leu Ala Gln
1 5 10 15

Leu Gly Lys Cys Arg Phe Met Gly Arg Asp Glu Phe Ala Asp Gly Ala
20 25 30

Ser Tyr Leu Gln Gly Lys Lys Val Val Ile Val Gly Cys Gly Ala Gln
35 40 45

Gly Leu Asn Gln Gly Leu Asn Met Arg Asp Ser Gly Leu Asp Ile Ser
50 55 60

```
Tyr Ala Leu Arg Lys Glu Ala Ile Ala Glu Lys Arg Ala Ser Trp Arg
65                  70                  75                  80

Lys Ala Thr Glu Asn Gly Phe Lys Val Gly Thr Tyr Glu Glu Leu Ile
                85                  90                  95

Pro Gln Ala Asp Leu Val Ile Asn Leu Thr Pro Asp Lys Gln His Ser
            100                 105                 110

Asp Val Val Arg Thr Val Gln Pro Leu Met Lys Asp Gly Ala Ala Leu
        115                 120                 125

Gly Tyr Ser His Gly Phe Asn Ile Val Glu Val Gly Glu Gln Ile Arg
    130                 135                 140

Lys Asp Ile Thr Val Val Met Val Ala Pro Lys Cys Pro Gly Thr Glu
145                 150                 155                 160

Val Arg Glu Glu Tyr Lys Arg Gly Phe Gly Val Pro Thr Leu Ile Ala
                165                 170                 175

Val His Pro Glu Asn Asp Pro Lys Gly Glu Gly Met Ala Ile Ala Lys
            180                 185                 190

Ala Trp Ala Ala Ala Thr Gly Gly His Arg Ala Gly Val Leu Glu Ser
        195                 200                 205

Ser Phe Val Ala Glu Val Lys Ser Asp Leu Met Gly Glu Gln Thr Ile
    210                 215                 220

Leu Cys Gly Met Leu Gln Ala Gly Ser Leu Leu Cys Phe Asp Lys Leu
225                 230                 235                 240

Val Glu Glu Gly Thr Asp Pro Ala Tyr Ala Glu Lys Leu Ile Gln Phe
                245                 250                 255

Gly Trp Glu Thr Ile Thr Glu Ala Leu Lys Gln Gly Gly Ile Thr Leu
            260                 265                 270

Met Met Asp Arg Leu Ser Asn Pro Ala Lys Leu Arg Ala Tyr Ala Leu
        275                 280                 285

Ser Glu Gln Leu Lys Glu Ile Met Ala Pro Leu Phe Gln Lys His Met
    290                 295                 300

Asp Asp Ile Ile Ser Gly Glu Phe Ser Ser Gly Met Met Ala Asp Trp
305                 310                 315                 320
```

Ala Asn Asp Asp Lys Lys Leu Leu Thr Trp Arg Glu Glu Thr Gly Lys
325 330 335

Thr Ala Phe Glu Thr Ala Pro Gln Tyr Glu Gly Lys Ile Gly Glu Gln
340 345 350

Glu Tyr Phe Asp Lys Gly Val Leu Met Ile Ala Met Val Lys Ala Gly
355 360 365

Val Glu Leu Ala Phe Glu Thr Met Val Asp Ser Gly Ile Ile Glu Glu
370 375 380

Ser Ala Tyr Tyr Glu Ser Leu His Glu Leu Pro Leu Ile Ala Asn Thr
385 390 395 400

Ile Ala Arg Lys Arg Leu Tyr Glu Met Asn Val Val Ile Ser Asp Thr
405 410 415

Ala Glu Tyr Gly Asn Tyr Leu Phe Ser Tyr Ala Cys Val Pro Leu Leu
420 425 430

Lys Pro Phe Met Ala Glu Leu Gln Pro Gly Asp Leu Gly Lys Ala Ile
435 440 445

Pro Glu Gly Ala Val Asp Asn Gly Gln Leu Arg Asp Val Asn Glu Ala
450 455 460

Ile Arg Ser His Ala Ile Glu Gln Val Gly Lys Lys Leu Arg Gly Tyr
465 470 475 480

Met Thr Asp Met Lys Arg Ile Ala Val Ala Gly
485 490

<210> 195
<211> 1476
<212> DNA
<213> Escherichia coli

<220>
<221> misc_feature
<223> Encodes acetohydroxyacid isomeroreductase

<400> 195
atggctaact acttcaatac actgaatctg cgccagcagc tggcacagct gggcaaatgt 60

cgctttatgg gccgcgatga attcgccgat ggcgcgagct accttcaggg taaaaaagta 120

gtcatcgtcg gctgtggcgc acagggtctg aaccagggcc tgaacatgcg tgattctggt 180

ctcgatatct cctacgctct gcgtaaagaa gcgattgccg agaagcgcgc gtcctggcgt 240

aaagcgaccg aaaatggttt taaagtgggt acttacgaag aactgatccc acaggcggat 300

ctggtgatta acctgacgcc ggacaagcag cactctgatg tagtgcgcac cgtacagcca 360

ctgatgaaag acggcgcggc gctgggctac tcgcacggtt tcaacatcgt cgaagtgggc 420

gagcagatcc gtaaagatat caccgtagtg atggttgcgc cgaaatgccc aggcaccgaa 480

gtgcgtgaag agtacaaacg tgggttcggc gtaccgacgc tgattgccgt tcacccggaa 540

aacgatccga aaggcgaagg catggcgatt gccaaagcct gggcggctgc aaccggtggt 600

caccgtgcgg gtgtgctgga atcgtccttc gttgcggaag tgaaatctga cctgatgggc 660

gagcaaacca tcctgtgcgg tatgttgcag gctggctctc tgctgtgctt cgacaagctg 720

gtggaagaag gtaccgatcc agcatacgca gaaaaactga ttcagttcgg ttgggaaacc 780

atcaccgaag cactgaaaca gggcggcatc accctgatga tggaccgtct ctctaacccg 840

gcgaaactgc gtgcttatgc gctttctgaa cagctgaaag agatcatggc acccctgttc 900

cagaaacata tggacgacat catctccggc gaattctctt ccggtatgat ggcggactgg 960

gccaacgatg ataagaaact gctgacctgg cgtgaagaga ccggcaaaac cgcgtttgaa 1020

accgcgccgc agtatgaagg caaaatcggc gagcaggagt acttcgataa aggcgtactg 1080

atgattgcga tggtgaaagc gggcgttgaa ctggcgttcg aaaccatggt cgattccggc 1140

atcattgaag agtctgcata ttatgaatca ctgcacgagc tgccgctgat tgccaacacc 1200

atcgcccgta agcgtctgta cgaaatgaac gtggttatct ctgataccgc tgagtacggt 1260

aactatctgt tctcttacgc ttgtgtgccg ttgctgaaac cgtttatggc agagctgcaa 1320

ccgggcgacc tgggtaaagc tattccggaa ggcgcggtag ataacgggca actgcgtgat 1380

gtgaacgaag cgattcgcag ccatgcgatt gagcaggtag gtaagaaact gcgcggctat 1440

atgacagata tgaaacgtat tgctgttgcg ggttaa 1476

<210> 196
<211> 342
<212> PRT
<213> Bacillus subtilis

<220>
<221> MISC_FEATURE
<223> acetohydroxyacid isomeroreductase

<400> 196

Met Val Lys Val Tyr Tyr Asn Gly Asp Ile Lys Glu Asn Val Leu Ala
1 5 10 15

Gly Lys Thr Val Ala Val Ile Gly Tyr Gly Ser Gln Gly His Ala His
20 25 30

Ala Leu Asn Leu Lys Glu Ser Gly Val Asp Val Ile Val Gly Val Arg
35 40 45

Gln Gly Lys Ser Phe Thr Gln Ala Gln Glu Asp Gly His Lys Val Phe
50 55 60

Ser Val Lys Glu Ala Ala Ala Gln Ala Glu Ile Ile Met Val Leu Leu
65 70 75 80

Pro Asp Glu Gln Gln Gln Lys Val Tyr Glu Ala Glu Ile Lys Asp Glu
85 90 95

Leu Thr Ala Gly Lys Ser Leu Val Phe Ala His Gly Phe Asn Val His
100 105 110

Phe His Gln Ile Val Pro Pro Ala Asp Val Asp Val Phe Leu Val Ala
115 120 125

Pro Lys Gly Pro Gly His Leu Val Arg Arg Thr Tyr Glu Gln Gly Ala
130 135 140

Gly Val Pro Ala Leu Phe Ala Ile Tyr Gln Asp Val Thr Gly Glu Ala
145 150 155 160

Arg Asp Lys Ala Leu Ala Tyr Ala Lys Gly Ile Gly Gly Ala Arg Ala
165 170 175

Gly Val Leu Glu Thr Thr Phe Lys Glu Glu Thr Glu Thr Asp Leu Phe
180 185 190

Gly Glu Gln Ala Val Leu Cys Gly Gly Leu Ser Ala Leu Val Lys Ala
195 200 205

Gly Phe Glu Thr Leu Thr Glu Ala Gly Tyr Gln Pro Glu Leu Ala Tyr
210 215 220

Phe Glu Cys Leu His Glu Leu Lys Leu Ile Val Asp Leu Met Tyr Glu
225 230 235 240

Glu Gly Leu Ala Gly Met Arg Tyr Ser Ile Ser Asp Thr Ala Gln Trp
245 250 255

Gly Asp Phe Val Ser Gly Pro Arg Val Val Asp Ala Lys Val Lys Glu
260 265 270

Ser Met Lys Glu Val Leu Lys Asp Ile Gln Asn Gly Thr Phe Ala Lys

275 280 285

Glu Trp Ile Val Glu Asn Gln Val Asn Arg Pro Arg Phe Asn Ala Ile
290 295 300

Asn Ala Ser Glu Asn Glu His Gln Ile Glu Val Val Gly Arg Lys Leu
305 310 315 320

Arg Glu Met Met Pro Phe Val Lys Gln Gly Lys Lys Lys Glu Ala Val
325 330 335

Val Ser Val Ala Gln Asn
340

<210> 197
<211> 1029
<212> DNA
<213> Bacillus subtilis

<220>
<221> misc_feature
<223> Encodes acetohydroxyacid isomeroreductase

<400> 197
ttaattttgc gcaacggaga ccaccgcttc cttcttcttg ccttgtttca caaacggcat 60

catttcacga agctttcttc ccactacttc gatttgatgt tcgttctcgc ttgcattgat 120

agcgttgaaa cgaggacggt ttacttggtt ttcgacgatc cactcttttg cgaatgtacc 180

gttttggata tcttttaata cttctttcat agattctttt actttggcgt ccacaacgcg 240

agggcctgat acgaaatctc cccactgtgc tgtgtcagag attgaatatc tcattcctgc 300

aagtccttct tcgtacataa ggtctacgat taatttcagc tcatgaagac actcgaagta 360

tgcaagttca ggctgataac ctgcttcagt taaggtttca aatccggctt tgacaagcgc 420

gcttaatccg ccgcaaagaa ctgcttgctc accgaacaaa tctgtttctg tttcttcttt 480

aaatgtcgtt tctaatacgc ccgctcttgc gccgccgatt cctttagcat aagcgagggc 540

tttgtctctt gcttctccag tcacatcttg atagattgcg aacaatgcag gtacgccagc 600

tccttgctca tatgttcttc ttaccaagtg tcccgggcct ttaggggcca ctaagaatac 660

atctacatcc gccggaggaa caatttgatg gaaatgcacg ttaaatccat gagcgaatac 720

taatgatttt cctgctgtca attcatcttt gatttcagct tcgtatactt tttgctgctg 780

ctcatccgga agcagaacca tgatgatttc ggcttgggct gccgcttctt ttactgaaaa 840

tactttatgt ccgtcttctt gggcttgagt gaaagatttt ccttgtctaa caccgacgat 900

cacgtctact ccgctttctt taaggttcag ggcatgtgcg tggccttgcg aaccgtaccc 960

gataaccgct actgtttttc cagccaatac gttctctttg atatcaccgt tataatatac 1020

ttttaccat 1029

<210> 198
<211> 616
<212> PRT
<213> Escherichia coli

<220>
<221> MISC_FEATURE
<223> dihydroxyacid dehydratase

<400> 198

Met Pro Lys Tyr Arg Ser Ala Thr Thr Thr His Gly Arg Asn Met Ala
1 5 10 15

Gly Ala Arg Ala Leu Trp Arg Ala Thr Gly Met Thr Asp Ala Asp Phe
20 25 30

Gly Lys Pro Ile Ile Ala Val Val Asn Ser Phe Thr Gln Phe Val Pro
35 40 45

Gly His Val His Leu Arg Asp Leu Gly Lys Leu Val Ala Glu Gln Ile
50 55 60

Glu Ala Ala Gly Gly Val Ala Lys Glu Phe Asn Thr Ile Ala Val Asp
65 70 75 80

Asp Gly Ile Ala Met Gly His Gly Gly Met Leu Tyr Ser Leu Pro Ser
85 90 95

Arg Glu Leu Ile Ala Asp Ser Val Glu Tyr Met Val Asn Ala His Cys
100 105 110

Ala Asp Ala Met Val Cys Ile Ser Asn Cys Asp Lys Ile Thr Pro Gly
115 120 125

Met Leu Met Ala Ser Leu Arg Leu Asn Ile Pro Val Ile Phe Val Ser
130 135 140

Gly Gly Pro Met Glu Ala Gly Lys Thr Lys Leu Ser Asp Gln Ile Ile
145 150 155 160

Lys Leu Asp Leu Val Asp Ala Met Ile Gln Gly Ala Asp Pro Lys Val
165 170 175

Ser Asp Ser Gln Ser Asp Gln Val Glu Arg Ser Ala Cys Pro Thr Cys
180 185 190

```
Gly Ser Cys Ser Gly Met Phe Thr Ala Asn Ser Met Asn Cys Leu Thr
        195                 200                 205

Glu Ala Leu Gly Leu Ser Gln Pro Gly Asn Gly Ser Leu Leu Ala Thr
    210                 215                 220

His Ala Asp Arg Lys Gln Leu Phe Leu Asn Ala Gly Lys Arg Ile Val
225                 230                 235                 240

Glu Leu Thr Lys Arg Tyr Tyr Glu Gln Asn Asp Glu Ser Ala Leu Pro
                245                 250                 255

Arg Asn Ile Ala Ser Lys Ala Ala Phe Glu Asn Ala Met Thr Leu Asp
            260                 265                 270

Ile Ala Met Gly Gly Ser Thr Asn Thr Val Leu His Leu Leu Ala Ala
        275                 280                 285

Ala Gln Glu Ala Glu Ile Asp Phe Thr Met Ser Asp Ile Asp Lys Leu
    290                 295                 300

Ser Arg Lys Val Pro Gln Leu Cys Lys Val Ala Pro Ser Thr Gln Lys
305                 310                 315                 320

Tyr His Met Glu Asp Val His Arg Ala Gly Gly Val Ile Gly Ile Leu
                325                 330                 335

Gly Glu Leu Asp Arg Ala Gly Leu Leu Asn Arg Asp Val Lys Asn Val
            340                 345                 350

Leu Gly Leu Thr Leu Pro Gln Thr Leu Glu Gln Tyr Asp Val Met Leu
        355                 360                 365

Thr Gln Asp Asp Ala Val Lys Asn Met Phe Arg Ala Gly Pro Ala Gly
    370                 375                 380

Ile Arg Thr Thr Gln Ala Phe Ser Gln Asp Cys Arg Trp Asp Thr Leu
385                 390                 395                 400

Asp Asp Asp Arg Ala Asn Gly Cys Ile Arg Ser Leu Glu His Ala Tyr
                405                 410                 415

Ser Lys Asp Gly Gly Leu Ala Val Leu Tyr Gly Asn Phe Ala Glu Asn
            420                 425                 430

Gly Cys Ile Val Lys Thr Ala Gly Val Asp Asp Ser Ile Leu Lys Phe
        435                 440                 445
```

Thr Gly Pro Ala Lys Val Tyr Glu Ser Gln Asp Asp Ala Val Glu Ala
450 455 460

Ile Leu Gly Gly Lys Val Val Ala Gly Asp Val Val Val Ile Arg Tyr
465 470 475 480

Glu Gly Pro Lys Gly Gly Pro Gly Met Gln Glu Met Leu Tyr Pro Thr
485 490 495

Ser Phe Leu Lys Ser Met Gly Leu Gly Lys Ala Cys Ala Leu Ile Thr
500 505 510

Asp Gly Arg Phe Ser Gly Gly Thr Ser Gly Leu Ser Ile Gly His Val
515 520 525

Ser Pro Glu Ala Ala Ser Gly Gly Ser Ile Gly Leu Ile Glu Asp Gly
530 535 540

Asp Leu Ile Ala Ile Asp Ile Pro Asn Arg Gly Ile Gln Leu Gln Val
545 550 555 560

Ser Asp Ala Glu Leu Ala Ala Arg Arg Glu Ala Gln Asp Ala Arg Gly
565 570 575

Asp Lys Ala Trp Thr Pro Lys Asn Arg Glu Arg Gln Val Ser Phe Ala
580 585 590

Leu Arg Ala Tyr Ala Ser Leu Ala Thr Ser Ala Asp Lys Gly Ala Val
595 600 605

Arg Asp Lys Ser Lys Leu Gly Gly
610 615

<210> 199
<211> 1851
<212> DNA
<213> Escherichia coli

<220>
<221> misc_feature
<223> Encodes dihydroxyacid dehydratase

<400> 199
atgcctaagt accgttccgc caccaccact catggtcgta atatggcggg tgctcgtgcg 60

ctgtggcgcg ccaccggaat gaccgacgcc gatttcggta agccgattat cgcggttgtg 120

aactcgttca cccaatttgt accgggtcac gtccatctgc gcgatctcgg taaactggtc 180

gccgaacaaa ttgaagcggc tggcggcgtt gccaaagagt tcaacaccat tgcggtggat 240
gatgggattg ccatgggcca cggggggatg ctttattcac tgccatctcg cgaactgatc 300
gctgattccg ttgagtatat ggtcaacgcc cactgcgccg acgccatggt ctgcatctct 360
aactgcgaca aaatcacccc ggggatgctg atggcttccc tgcgcctgaa tattccggtg 420
atctttgttt ccggcggccc gatggaggcc gggaaaacca aactttccga tcagatcatc 480
aagctcgatc tggttgatgc gatgatccag ggcgcagacc cgaaagtatc tgactcccag 540
agcgatcagg ttgaacgttc cgcgtgtccg acctgcggtt cctgctccgg gatgtttacc 600
gctaactcaa tgaactgcct gaccgaagcg ctgggcctgt cgcagccggg caacggctcg 660
ctgctggcaa cccacgccga ccgtaagcag ctgttcctta atgctggtaa acgcattgtt 720
gaattgacca aacgttatta cgagcaaaac gacgaaagtg cactgccgcg taatatcgcc 780
agtaaggcgg cgtttgaaaa cgccatgacg ctggatatcg cgatgggtgg atcgactaac 840
accgtacttc acctgctggc ggcggcgcag gaagcggaaa tcgacttcac catgagtgat 900
atcgataagc tttcccgcaa ggttccacag ctgtgtaaag ttgcgccgag cacccagaaa 960
taccatatgg aagatgttca ccgtgctggt ggtgttatcg gtattctcgg cgaactggat 1020
cgcgcggggt tactgaaccg tgatgtgaaa aacgtacttg gcctgacgtt gccgcaaacg 1080
ctggaacaat acgacgttat gctgacccag gatgacgcgg taaaaaatat gttccgcgca 1140
ggtcctgcag gcattcgtac cacacaggca ttctcgcaag attgccgttg ggatacgctg 1200
gacgacgatc gcgccaatgg ctgtatccgc tcgctggaac acgcctacag caaagacggc 1260
ggcctggcgg tgctctacgg taactttgcg gaaaacggct gcatcgtgaa aacggcaggc 1320
gtcgatgaca gcatcctcaa attcaccggc ccggcgaaag tgtacgaaag ccaggacgat 1380
gcggtagaag cgattctcgg cggtaaagtt gtcgccggag atgtggtagt aattcgctat 1440
gaaggcccga aaggcggtcc ggggatgcag gaaatgctct acccaaccag cttcctgaaa 1500
tcaatgggtc tcggcaaagc ctgtgcgctg atcaccgacg gtcgtttctc tggtggcacc 1560
tctggtcttt ccatcggcca cgtctcaccg gaagcggcaa gcggcggcag cattggcctg 1620
attgaagatg gtgacctgat cgctatcgac atcccgaacc gtggcattca gttacaggta 1680
agcgatgccg aactggcggc gcgtcgtgaa gcgcaggacg ctcgaggtga caaagcctgg 1740
acgccgaaaa atcgtgaacg tcaggtctcc tttgccctgc gtgcttatgc cagcctggca 1800
accagcgccg acaaaggcgc ggtgcgcgat aaatcgaaac tgggggtta a 1851

<210> 200
<211> 558
<212> PRT
<213> Bacillus subtilis

<220>
<221> MISC_FEATURE
<223> dihydroxyacid dehydratase

<400> 200

Met Ala Glu Leu Arg Ser Asn Met Ile Thr Gln Gly Ile Asp Arg Ala
1 5 10 15

Pro His Arg Ser Leu Leu Arg Ala Ala Gly Val Lys Glu Glu Asp Phe
20 25 30

Gly Lys Pro Phe Ile Ala Val Cys Asn Ser Tyr Ile Asp Ile Val Pro
35 40 45

Gly His Val His Leu Gln Glu Phe Gly Lys Ile Val Lys Glu Ala Ile
50 55 60

Arg Glu Ala Gly Gly Val Pro Phe Glu Phe Asn Thr Ile Gly Val Asp
65 70 75 80

Asp Gly Ile Ala Met Gly His Ile Gly Met Arg Tyr Ser Leu Pro Ser
85 90 95

Arg Glu Ile Ile Ala Asp Ser Val Glu Thr Val Val Ser Ala His Trp
100 105 110

Phe Asp Gly Met Val Cys Ile Pro Asn Cys Asp Lys Ile Thr Pro Gly
115 120 125

Met Leu Met Ala Ala Met Arg Ile Asn Ile Pro Thr Ile Phe Val Ser
130 135 140

Gly Gly Pro Met Ala Ala Gly Arg Thr Ser Asp Gly Arg Lys Ile Ser
145 150 155 160

Leu Ser Ser Val Phe Glu Gly Val Gly Ala Tyr Gln Ala Gly Lys Ile
165 170 175

Asn Glu Asn Glu Leu Gln Glu Leu Glu Gln Phe Gly Cys Pro Thr Cys
180 185 190

Gly Ser Cys Ser Gly Met Phe Thr Ala Asn Ser Met Asn Cys Leu Ser
195 200 205

Glu Ala Leu Gly Leu Ala Leu Pro Gly Asn Gly Thr Ile Leu Ala Thr
210 215 220

Ser Pro Glu Arg Lys Glu Phe Val Arg Lys Ser Ala Ala Gln Leu Met

225 230 235 240

Glu Thr Ile Arg Lys Asp Ile Lys Pro Arg Asp Ile Val Thr Val Lys
245 250 255

Ala Ile Asp Asn Ala Phe Ala Leu Asp Met Ala Leu Gly Gly Ser Thr
260 265 270

Asn Thr Val Leu His Thr Leu Ala Leu Ala Asn Glu Ala Gly Val Glu
275 280 285

Tyr Ser Leu Glu Arg Ile Asn Glu Val Ala Glu Arg Val Pro His Leu
290 295 300

Ala Lys Leu Ala Pro Ala Ser Asp Val Phe Ile Glu Asp Leu His Glu
305 310 315 320

Ala Gly Gly Val Ser Ala Ala Leu Asn Glu Leu Ser Lys Lys Glu Gly
325 330 335

Ala Leu His Leu Asp Ala Leu Thr Val Thr Gly Lys Thr Leu Gly Glu
340 345 350

Thr Ile Ala Gly His Glu Val Lys Asp Tyr Asp Val Ile His Pro Leu
355 360 365

Asp Gln Pro Phe Thr Glu Lys Gly Gly Leu Ala Val Leu Phe Gly Asn
370 375 380

Leu Ala Pro Asp Gly Ala Ile Ile Lys Thr Gly Gly Val Gln Asn Gly
385 390 395 400

Ile Thr Arg His Glu Gly Pro Ala Val Val Phe Asp Ser Gln Asp Glu
405 410 415

Ala Leu Asp Gly Ile Ile Asn Arg Lys Val Lys Glu Gly Asp Val Val
420 425 430

Ile Ile Arg Tyr Glu Gly Pro Lys Gly Gly Pro Gly Met Pro Glu Met
435 440 445

Leu Ala Pro Thr Ser Gln Ile Val Gly Met Gly Leu Gly Pro Lys Val
450 455 460

Ala Leu Ile Thr Asp Gly Arg Phe Ser Gly Ala Ser Arg Gly Leu Ser
465 470 475 480

Ile Gly His Val Ser Pro Glu Ala Ala Glu Gly Gly Pro Leu Ala Phe
485 490 495

Val Glu Asn Gly Asp His Ile Ile Val Asp Ile Glu Lys Arg Ile Leu
500 505 510

Asp Val Gln Val Pro Glu Glu Glu Trp Glu Lys Arg Lys Ala Asn Trp
515 520 525

Lys Gly Phe Glu Pro Lys Val Lys Thr Gly Tyr Leu Ala Arg Tyr Ser
530 535 540

Lys Leu Val Thr Ser Ala Asn Thr Gly Gly Ile Met Lys Ile
545 550 555

<210> 201
<211> 1677
<212> DNA
<213> Bacillus subtilis

<220>
<221> misc_feature
<223> Encodes dihydroxyacid dehydratase

<400> 201
ctagatttc ataataccgc cggtgttggc acttgtcaca agtttagaat aacgtgccag 60

gtagccggtt ttcactttcg gttcaaaacc tttccagttc gcttttcgtt tttcccactc 120

ttcttctggc acttgtacat ccaagatgcg tttttcaata tcaacgataa tatggtctcc 180

gttttcaaca aaggcaagcg gcccgccctc agcggcctca ggtgatacgt ggccgattga 240

gaggccacgg gaggctccgg aaaaacgtcc gtccgtaatc aatgccactt ttggcccgag 300

tcccattcca acgatttggg atgttggcgc cagcatttcc ggcatgccag gtccgccttt 360

tggcccttcg tatctgatga tgacaacgtc gccttctttt acttttcggt tgataatgcc 420

gtcaagcgcc tcgtcctgag aatcgaatac gacagccggc ccttcgtgtc ttgtaatccc 480

attctgtacg ccgcctgttt taatgatagc gccgtccgga gctagattac cgaataaaac 540

agcaaggcct cccttttcag tgaatggttg atccagcggg tgaatgacgt cataatcctt 600

tacttcatgt ccggcaatgg tttctccaag agtttttcct gtaacagtca gcgcatctaa 660

atgaagcgct ccttctttct tcgaaagctc attcagagcc gctgaaacgc cgcccgcttc 720

gtgaagatct tcaataaaca catccgatgc aggcgccagc ttagccaagt gcggcacgcg 780

ctcagcgact tcgttaatgc gttctaaaga gtattcaacg ccggcttcgt ttgcaagggc 840

aagggtatga agaacggtat ttgtagaacc tccgagcgcc atatcgagtg caaacgcgtt 900

atcaatcgct tttactgtaa caatatcacg cggtttgata tctttgcgaa tcgtttccat 960

taattgcgca gccgattttc tcacaaactc tttgcgttcc ggagatgttg ccagaatggt 1020

tccattaccc ggcaaagcaa gaccaagtgc ttctgacaga cagttcattg agttcgccgt 1080

aaacatgcct gagcaagacc cgcacgttgg gcatccgaac tgctctagtt cttgaagctc 1140

gttttcgttg attttccctg cttggtaggc gcctacccct tcgaatactg aggaaaggga 1200

gatttttcgc ccgtcacttg ttcttcctgc cgccatcggt ccgccgctga caaaaatcgt 1260

cggaatgttg atgcgcattg ccgccataag cattcccggt gtgattttgt cgcagttcgg 1320

aatacagacc attccgtcaa accagtgtgc ggatacaacc gtttccacag agtctgcgat 1380

aatttcacgg cttggcagcg aatatctcat accgatatgc cccattgcga tgccatcatc 1440

taccccaatg gtattaaatt caaacggaac gccccctgct tctctgattg cttcttttac 1500

gattttccca aactcctgca agtgaacatg accgggaacg atatcaatgt atgaattaca 1560

caccgcaata aacggcttgc cgaaatcctc ttcttttacc cctgctgcac gaagcaaact 1620

gcggtgcgga gctctatcga ttccttgtgt gatcatatta ctgcgtaatt ctgccat 1677

<210> 202
<211> 208
<212> PRT
<213> Escherichia coli

<220>
<221> MISC_FEATURE
<223> thioesterase (TesA)

<400> 202

Met Met Asn Phe Asn Asn Val Phe Arg Trp His Leu Pro Phe Leu Phe
1 5 10 15

Leu Val Leu Leu Thr Phe Arg Ala Ala Ala Ala Asp Thr Leu Leu Ile
20 25 30

Leu Gly Asp Ser Leu Ser Ala Gly Tyr Arg Met Ser Ala Ser Ala Ala
35 40 45

Trp Pro Ala Leu Leu Asn Asp Lys Trp Gln Ser Lys Thr Ser Val Val
50 55 60

Asn Ala Ser Ile Ser Gly Asp Thr Ser Gln Gln Gly Leu Ala Arg Leu
65 70 75 80

Pro Ala Leu Leu Lys Gln His Gln Pro Arg Trp Val Leu Val Glu Leu
85 90 95

Gly Gly Asn Asp Gly Leu Arg Gly Phe Gln Pro Gln Gln Thr Glu Gln
100 105 110

Thr Leu Arg Gln Ile Leu Gln Asp Val Lys Ala Ala Asn Ala Glu Pro
115 120 125

Leu Leu Met Gln Ile Arg Leu Pro Ala Asn Tyr Gly Arg Arg Tyr Asn
130 135 140

Glu Ala Phe Ser Ala Ile Tyr Pro Lys Leu Ala Lys Glu Phe Asp Val
145 150 155 160

Pro Leu Leu Pro Phe Phe Met Glu Glu Val Tyr Leu Lys Pro Gln Trp
165 170 175

Met Gln Asp Asp Gly Ile His Pro Asn Arg Asp Ala Gln Pro Phe Ile
180 185 190

Ala Asp Trp Met Ala Lys Gln Leu Gln Pro Leu Val Asn His Asp Ser
195 200 205

<210> 203
<211> 627
<212> DNA
<213> Escherichia coli

<220>
<221> misc_feature
<223> Encodes thioesterase (TesA)

<400> 203
ttatgagtca tgatttacta aaggctgcaa ctgcttcgcc atccagtcgg caataaacgg 60

ctgggcgtcg cggttgggat gaataccgtc atcctgcatc cattgtggct tgaggtagac 120

ctcttccata aaaaagggca gcagcggaac atcaaactct ttggcgagtt tggggtaaat 180

ggcgctaaag gcttcattat aacggcgacc atagtttgca ggcagacgta tttgcattaa 240

caatggttca gcgttggcgg ctttgacatc ctgcaaaatc tggcgcagcg tttgctcggt 300

ttgctgtggc tgaaaaccac gcaaaccgtc attgccgccc agttcaacca gcacccaacg 360

cggctgatgc tgtttcagca gagccggaag gcgcgccagt ccttgttgcg aggtgtcgcc 420

gctgatgctg gcattaacta ccgacgtttt actctgccac ttatcattca acaaggcagg 480

ccaggccgcg ctggcagaca ttcgataccc ggcgctcagg ctatcaccca gaatcaataa 540

cgtgtccgct gcggcggcac ggaaggttaa caggaccagg aacaggaagg gcaaatgcca 600

gcggaaaaca ttgttgaagt tcatcat 627

<210> 204
<211> 183
<212> PRT

<213>  Escherichia coli mutant

<220>
<221>  MISC_FEATURE
<223>  Escherichia coli TesA mutant lacking leader sequence

<400>  204

```
Met Ala Asp Thr Leu Leu Ile Leu Gly Asp Ser Leu Ser Ala Gly Tyr
1               5                   10                  15

Arg Met Ser Ala Ser Ala Ala Trp Pro Ala Leu Leu Asn Asp Lys Trp
            20                  25                  30

Gln Ser Lys Thr Ser Val Val Asn Ala Ser Ile Ser Gly Asp Thr Ser
        35                  40                  45

Gln Gln Gly Leu Ala Arg Leu Pro Ala Leu Leu Lys Gln His Gln Pro
    50                  55                  60

Arg Trp Val Leu Val Glu Leu Gly Gly Asn Asp Gly Leu Arg Gly Phe
65                  70                  75                  80

Gln Pro Gln Gln Thr Glu Gln Thr Leu Arg Gln Ile Leu Gln Asp Val
                85                  90                  95

Lys Ala Ala Asn Ala Glu Pro Leu Leu Met Gln Ile Arg Leu Pro Ala
            100                 105                 110

Asn Tyr Gly Arg Arg Tyr Asn Glu Ala Phe Ser Ala Ile Tyr Pro Lys
        115                 120                 125

Leu Ala Lys Glu Phe Asp Val Pro Leu Leu Pro Phe Phe Met Glu Glu
    130                 135                 140

Val Tyr Leu Lys Pro Gln Trp Met Gln Asp Asp Gly Ile His Pro Asn
145                 150                 155                 160

Arg Asp Ala Gln Pro Phe Ile Ala Asp Trp Met Ala Lys Gln Leu Gln
                165                 170                 175

Pro Leu Val Asn His Asp Ser
            180
```

<210>  205
<211>  552
<212>  DNA
<213>  Escherichia coli mutant

<220>
<221> misc_feature
<223> Encodes Escherichia coli TesA mutant lacking leader sequence

<400> 205
atggcggaca cgttattgat tctgggtgat agcctgagcg ccgggtatcg aatgtctgcc 60

agcgcggcct ggcctgcctt gttgaatgat aagtggcaga gtaaaacgtc ggtagttaat 120

gccagcatca gcggcgacac ctcgcaacaa ggactggcgc gccttccggc tctgctgaaa 180

cagcatcagc cgcgttgggt gctggttgaa ctgggcggca atgacggttt gcgtggtttt 240

cagccacagc aaaccgagca aacgctgcgc cagattttgc aggatgtcaa agccgccaac 300

gctgaaccat tgttaatgca aatacgtctg cctgcaaact atggtcgccg ttataatgaa 360

gcctttagcg ccatttaccc caaactcgcc aaagagtttg atgttccgct gctgcccttt 420

tttatggaag aggtctacct caagccacaa tggatgcagg atgacggtat tcatcccaac 480

cgcgacgccc agccgtttat tgccgactgg atggcgaagc agttgcagcc tttagtaaat 540

catgactcat aa 552

<210> 206
<211> 286
<212> PRT
<213> Escherichia coli

<220>
<221> MISC_FEATURE
<223> thioesterase (TesB)

<400> 206

Met Ser Gln Ala Leu Lys Asn Leu Leu Thr Leu Leu Asn Leu Glu Lys
1               5                   10                  15

Ile Glu Glu Gly Leu Phe Arg Gly Gln Ser Glu Asp Leu Gly Leu Arg
            20                  25                  30

Gln Val Phe Gly Gly Gln Val Val Gly Gln Ala Leu Tyr Ala Ala Lys
        35                  40                  45

Glu Thr Val Pro Glu Glu Arg Leu Val His Ser Phe His Ser Tyr Phe
    50                  55                  60

Leu Arg Pro Gly Asp Ser Lys Lys Pro Ile Ile Tyr Asp Val Glu Thr
65                  70                  75                  80

Leu Arg Asp Gly Asn Ser Phe Ser Ala Arg Arg Val Ala Ala Ile Gln
                85                  90                  95

Asn Gly Lys Pro Ile Phe Tyr Met Thr Ala Ser Phe Gln Ala Pro Glu

100 105 110

Ala Gly Phe Glu His Gln Lys Thr Met Pro Ser Ala Pro Ala Pro Asp
115 120 125

Gly Leu Pro Ser Glu Thr Gln Ile Ala Gln Ser Leu Ala His Leu Leu
130 135 140

Pro Pro Val Leu Lys Asp Lys Phe Ile Cys Asp Arg Pro Leu Glu Val
145 150 155 160

Arg Pro Val Glu Phe His Asn Pro Leu Lys Gly His Val Ala Glu Pro
165 170 175

His Arg Gln Val Trp Ile Arg Ala Asn Gly Ser Val Pro Asp Asp Leu
180 185 190

Arg Val His Gln Tyr Leu Leu Gly Tyr Ala Ser Asp Leu Asn Phe Leu
195 200 205

Pro Val Ala Leu Gln Pro His Gly Ile Gly Phe Leu Glu Pro Gly Ile
210 215 220

Gln Ile Ala Thr Ile Asp His Ser Met Trp Phe His Arg Pro Phe Asn
225 230 235 240

Leu Asn Glu Trp Leu Leu Tyr Ser Val Glu Ser Thr Ser Ala Ser Ser
245 250 255

Ala Arg Gly Phe Val Arg Gly Glu Phe Tyr Thr Gln Asp Gly Val Leu
260 265 270

Val Ala Ser Thr Val Gln Glu Gly Val Met Arg Asn His Asn
275 280 285

<210> 207
<211> 861
<212> DNA
<213> Escherichia coli

<220>
<221> misc_feature
<223> Encodes thioesterase (TesB)

<400> 207
ttaattgtga ttacgcatca ccccttcctg aacggtcgag gcaaccagta cgccgtcttg 60

ggtataaaac tcaccgcgca caaagccacg tgcgctggac gccgaggtgc tctccacgct 120

atacagcagc cattcattca aattaaacgg gcgatggaac cacatggaat ggtcaatggt 180

ggcaatctga atccccggtt cgagaaaacc gatgccgtgc ggctgtagag ctaccggcag 240

gaagttaaga tcagaagcgt aaccgagcag atactgatga acgcgcaggt catccggcac 300

gctaccattt gcgcggatcc acacctgacg atgtggttct gcgacgtgac ctttcagtgg 360

gttatgaaac tccaccggac ggacttccag cggacgatcg cagatgaatt tatctttcag 420

cactggcggc agcaggtgcg ccagcgattg ggcgatttgc gtttccgaag ggaggccatc 480

aggcgctggc gcggacggca ttgtttttg atgttcgaaa cccgcttctg gtgcctggaa 540

agaggcagtc atataaaaa tcggtttgcc gttttgaata gcagcaaccc ggcgggcgct 600

gaagctgtta ccgtcacgca gcgtttcgac atcataaata atcggcttct tactatcgcc 660

agggcgaaga aagtagctgt gaaacgaatg taccagccgc tcttcaggga cggtctcttt 720

tgcagcatac aaggcctgac ccacgacctg gccgccaaac acctggcgta aacctaaatc 780

ttcactctgg ccgcgaaaga gtccttcctc aatttttcc agatttaaca atgtcagtaa 840

attttttagc gcctgactca t 861

<210> 208
<211> 362
<212> PRT
<213> Arabidopsis thaliana

<220>
<221> MISC_FEATURE
<223> thioesterase (FatA)

<400> 208

Met Leu Lys Leu Ser Cys Asn Val Thr Asp Ser Lys Leu Gln Arg Ser
1 5 10 15

Leu Leu Phe Phe Ser His Ser Tyr Arg Ser Asp Pro Val Asn Phe Ile
20 25 30

Arg Arg Arg Ile Val Ser Cys Ser Gln Thr Lys Lys Thr Gly Leu Val
35 40 45

Pro Leu Arg Ala Val Val Ser Ala Asp Gln Gly Ser Val Val Gln Gly
50 55 60

Leu Ala Thr Leu Ala Asp Gln Leu Arg Leu Gly Ser Leu Thr Glu Asp
65 70 75 80

Gly Leu Ser Tyr Lys Glu Lys Phe Val Val Arg Ser Tyr Glu Val Gly
85 90 95

Ser Asn Lys Thr Ala Thr Val Glu Thr Ile Ala Asn Leu Leu Gln Glu

100 105 110

Val Gly Cys Asn His Ala Gln Ser Val Gly Phe Ser Thr Asp Gly Phe
115 120 125

Ala Thr Thr Thr Thr Met Arg Lys Leu His Leu Ile Trp Val Thr Ala
130 135 140

Arg Met His Ile Glu Ile Tyr Lys Tyr Pro Ala Trp Gly Asp Val Val
145 150 155 160

Glu Ile Glu Thr Trp Cys Gln Ser Glu Gly Arg Ile Gly Thr Arg Arg
165 170 175

Asp Trp Ile Leu Lys Asp Ser Val Thr Gly Glu Val Thr Gly Arg Ala
180 185 190

Thr Ser Lys Trp Val Met Met Asn Gln Asp Thr Arg Arg Leu Gln Lys
195 200 205

Val Ser Asp Asp Val Arg Asp Glu Tyr Leu Val Phe Cys Pro Gln Glu
210 215 220

Pro Arg Leu Ala Phe Pro Glu Glu Asn Asn Arg Ser Leu Lys Lys Ile
225 230 235 240

Pro Lys Leu Glu Asp Pro Ala Gln Tyr Ser Met Ile Gly Leu Lys Pro
245 250 255

Arg Arg Ala Asp Leu Asp Met Asn Gln His Val Asn Asn Val Thr Tyr
260 265 270

Ile Gly Trp Val Leu Glu Ser Ile Pro Gln Glu Ile Val Asp Thr His
275 280 285

Glu Leu Gln Val Ile Thr Leu Asp Tyr Arg Arg Glu Cys Gln Gln Asp
290 295 300

Asp Val Val Asp Ser Leu Thr Thr Thr Thr Ser Glu Ile Gly Gly Thr
305 310 315 320

Asn Gly Ser Ala Thr Ser Gly Thr Gln Gly His Asn Asp Ser Gln Phe
325 330 335

Leu His Leu Leu Arg Leu Ser Gly Asp Gly Gln Glu Ile Asn Arg Gly
340 345 350

```
Thr Thr Leu Trp Arg Lys Lys Pro Ser Ser
        355                 360


<210>  209
<211>  1089
<212>  DNA
<213>  Arabidopsis thaliana


<220>
<221>  misc_feature
<223>  Encodes thioesterase (FatA)

<400>  209
atgttgaagc tttcgtgtaa tgtgactgat tctaagttac agagaagctt actcttcttc        60

tcccattcat atcgatctga tccggtgaat ttcatccgtc ggagaattgt ctcttgttct       120

cagacgaaga agacaggttt ggttcctttg cgtgctgttg tatctgctga tcaaggaagt       180

gtggttcaag gtttggctac tctcgcggat cagctccgat taggtagttt gactgaagat       240

ggtttatctt ataaagagaa gtttgttgtt agatcttacg aagtgggtag taacaaaacc       300

gctactgttg aaaccattgc taatctttta caggaggtgg gatgtaatca tgcacaaagt       360

gttggttttt cgactgatgg gtttgcaaca acaactacta tgaggaagtt gcatctcatt       420

tgggttactg cgagaatgca tatcgagatc tataagtacc ctgcttgggg tgatgtggtt       480

gagatagaga cttggtgtca gagtgaagga aggattggga caaggcgtga ttggattctt       540

aaggattctg tcactggtga agtcactggc cgtgctacaa gcaagtgggt gatgatgaac       600

caagacacga gacggcttca gaaagtttct gatgatgttc gggacgagta cttggtcttc       660

tgtcctcaag aaccgaggtt agcatttccg gaagagaata acagaagctt gaagaaaatc       720

ccgaaactcg aagatccggc tcagtattca atgattgggc ttaagcctag acgagctgat       780

ctcgacatga accagcatgt caataatgtc acctatattg gatgggttct cgagagcata       840

ccacaagaaa ttgtagacac gcacgagctt caggtcataa ctctggatta tagaagagaa       900

tgtcaacaag acgatgtggt ggattcactc accaccacca cctctgaaat tggtggaacc       960

aatggctctg ccacgtctgg cacacagggc cacaacgata gccagttctt gcacctcctg      1020

aggttgtctg gagatggtca ggagatcaac cgcgggacaa ctctgtggag aaagaagcct      1080

tcaagttaa                                                              1089


<210>  210
<211>  412
<212>  PRT
<213>  Arabidopsis thaliana


<220>
<221>  MISC_FEATURE
<223>  thioesterase (FatB)
```

<400>  210

```
Met Val Ala Thr Ser Ala Thr Ser Ser Phe Phe Pro Val Pro Ser Ser
1               5                   10                  15

Ser Leu Asp Pro Asn Gly Lys Gly Asn Lys Ile Gly Ser Thr Asn Leu
            20                  25                  30

Ala Gly Leu Asn Ser Ala Pro Asn Ser Gly Arg Met Lys Val Lys Pro
        35                  40                  45

Asn Ala Gln Ala Pro Pro Lys Ile Asn Gly Lys Lys Val Gly Leu Pro
    50                  55                  60

Gly Ser Val Asp Ile Val Arg Thr Asp Thr Glu Thr Ser Ser His Pro
65                  70                  75                  80

Ala Pro Arg Thr Phe Ile Asn Gln Leu Pro Asp Trp Ser Met Leu Leu
                85                  90                  95

Ala Ala Ile Thr Thr Ile Phe Leu Ala Ala Glu Lys Gln Trp Met Met
            100                 105                 110

Leu Asp Trp Lys Pro Arg Arg Ser Asp Met Leu Val Asp Pro Phe Gly
        115                 120                 125

Ile Gly Arg Ile Val Gln Asp Gly Leu Val Phe Arg Gln Asn Phe Ser
    130                 135                 140

Ile Arg Ser Tyr Glu Ile Gly Ala Asp Arg Ser Ala Ser Ile Glu Thr
145                 150                 155                 160

Val Met Asn His Leu Gln Glu Thr Ala Leu Asn His Val Lys Thr Ala
                165                 170                 175

Gly Leu Leu Gly Asp Gly Phe Gly Ser Thr Pro Glu Met Phe Lys Lys
            180                 185                 190

Asn Leu Ile Trp Val Val Thr Arg Met Gln Val Val Val Asp Lys Tyr
        195                 200                 205

Pro Thr Trp Gly Asp Val Val Glu Val Asp Thr Trp Val Ser Gln Ser
    210                 215                 220

Gly Lys Asn Gly Met Arg Arg Asp Trp Leu Val Arg Asp Cys Asn Thr
225                 230                 235                 240
```

Gly Glu Thr Leu Thr Arg Ala Ser Ser Val Trp Val Met Met Asn Lys
                245                 250                 255

Leu Thr Arg Arg Leu Ser Lys Ile Pro Glu Glu Val Arg Gly Glu Ile
            260                 265                 270

Glu Pro Tyr Phe Val Asn Ser Asp Pro Val Leu Ala Glu Asp Ser Arg
        275                 280                 285

Lys Leu Thr Lys Ile Asp Asp Lys Thr Ala Asp Tyr Val Arg Ser Gly
    290                 295                 300

Leu Thr Pro Arg Trp Ser Asp Leu Asp Val Asn Gln His Val Asn Asn
305                 310                 315                 320

Val Lys Tyr Ile Gly Trp Ile Leu Glu Ser Ala Pro Val Gly Ile Met
                325                 330                 335

Glu Arg Gln Lys Leu Lys Ser Met Thr Leu Glu Tyr Arg Arg Glu Cys
            340                 345                 350

Gly Arg Asp Ser Val Leu Gln Ser Leu Thr Ala Val Thr Gly Cys Asp
        355                 360                 365

Ile Gly Asn Leu Ala Thr Ala Gly Asp Val Glu Cys Gln His Leu Leu
    370                 375                 380

Arg Leu Gln Asp Gly Ala Glu Val Val Arg Gly Arg Thr Glu Trp Ser
385                 390                 395                 400

Ser Lys Thr Pro Thr Thr Thr Trp Gly Thr Ala Pro
                405                 410

<210> 211
<211> 1239
<212> DNA
<213> Arabidopsis thaliana

<220>
<221> misc_feature
<223> Encodes thioesterase (FatB)

<400> 211
atggtggcca cctctgctac gtcgtcattc tttcctgtac catcttcttc acttgatcct 60

aatggaaaag gcaataagat tgggtctacg aatcttgctg gactcaattc tgcacctaac 120

tctggtagga tgaaggttaa accaaacgct caggctccac ctaagattaa tgggaaaaag 180

gttggtttgc ctggttctgt agatattgta aggactgata ccgagacctc atcacaccct 240

gcgccgagaa ctttcatcaa ccagttacct gactggagca tgcttcttgc tgctataact 300

acgattttct tagcggctga gaaacagtgg atgatgcttg attggaaacc taggcgttct 360

gacatgctgg tggatccttt tggtataggg agaattgttc aggatggcct tgtgttccgt 420

cagaattttt ctattaggtc atatgaaata ggtgctgatc gctctgcatc tatagaaacc 480

gtcatgaatc atctgcagga aacggcgctt aatcatgtta agactgctgg attgcttgga 540

gatgggtttg gctctacacc tgagatgttt aagaagaact tgatatgggt tgtcactcgt 600

atgcaggttg tggttgataa atatcctact tggggagatg ttgttgaagt agacacctgg 660

gtcagtcagt ctggaaagaa tggtatgcgt cgtgattggc tagttcggga ctgtaatact 720

ggagaaacct taacacgagc atcaagtgtg tgggtgatga tgaataaact gacaaggaga 780

ttgtcaaaga ttcctgaaga ggttcgaggg gaaatagagc cttattttgt gaattctgat 840

cctgtccttg ccgaggacag cagaaagtta acaaaaattg atgacaagac tgctgactat 900

gttcgatctg gtctcactcc tcgatggagt gacctagatg ttaaccagca tgtgaataat 960

gtaaagtaca ttgggtggat cctggagagt gctccagtgg gaataatgga gaggcagaag 1020

ctgaaaagca tgactctgga gtatcggagg gaatgcggga gagacagtgt gcttcagtcc 1080

ctcactgcag ttacgggttg cgatatcggt aacctggcaa cagcggggga tgtggaatgt 1140

cagcatttgc tccgactcca ggatggagcg gaagtggtga gaggaagaac agagtggagt 1200

agtaaaacac caacaacaac ttggggaact gcaccgtaa 1239

<210> 212
<211> 382
<212> PRT
<213> Umbellularia californica

<220>
<221> MISC_FEATURE
<223> thioesterase

<400> 212

Met Ala Thr Thr Ser Leu Ala Ser Ala Phe Cys Ser Met Lys Ala Val
1 5 10 15

Met Leu Ala Arg Asp Gly Arg Gly Met Lys Pro Arg Ser Ser Asp Leu
20 25 30

Gln Leu Arg Ala Gly Asn Ala Pro Thr Ser Leu Lys Met Ile Asn Gly
35 40 45

Thr Lys Phe Ser Tyr Thr Glu Ser Leu Lys Arg Leu Pro Asp Trp Ser
50 55 60

```
Met Leu Phe Ala Val Ile Thr Thr Ile Phe Ser Ala Ala Glu Lys Gln
65                  70                  75                  80

Trp Thr Asn Leu Glu Trp Lys Pro Lys Pro Lys Leu Pro Gln Leu Leu
                85                  90                  95

Asp Asp His Phe Gly Leu His Gly Leu Val Phe Arg Arg Thr Phe Ala
            100                 105                 110

Ile Arg Ser Tyr Glu Val Gly Pro Asp Arg Ser Thr Ser Ile Leu Ala
        115                 120                 125

Val Met Asn His Met Gln Glu Ala Thr Leu Asn His Ala Lys Ser Val
    130                 135                 140

Gly Ile Leu Gly Asp Gly Phe Gly Thr Thr Leu Glu Met Ser Lys Arg
145                 150                 155                 160

Asp Leu Met Trp Val Val Arg Arg Thr His Val Ala Val Glu Arg Tyr
                165                 170                 175

Pro Thr Trp Gly Asp Thr Val Glu Val Glu Cys Trp Ile Gly Ala Ser
            180                 185                 190

Gly Asn Asn Gly Met Arg Arg Asp Phe Leu Val Arg Asp Cys Lys Thr
        195                 200                 205

Gly Glu Ile Leu Thr Arg Cys Thr Ser Leu Ser Val Leu Met Asn Thr
    210                 215                 220

Arg Thr Arg Arg Leu Ser Thr Ile Pro Asp Glu Val Arg Gly Glu Ile
225                 230                 235                 240

Gly Pro Ala Phe Ile Asp Asn Val Ala Val Lys Asp Asp Glu Ile Lys
                245                 250                 255

Lys Leu Gln Lys Leu Asn Asp Ser Thr Ala Asp Tyr Ile Gln Gly Gly
            260                 265                 270

Leu Thr Pro Arg Trp Asn Asp Leu Asp Val Asn Gln His Val Asn Asn
        275                 280                 285

Leu Lys Tyr Val Ala Trp Val Phe Glu Thr Val Pro Asp Ser Ile Phe
    290                 295                 300

Glu Ser His His Ile Ser Ser Phe Thr Leu Glu Tyr Arg Arg Glu Cys
305                 310                 315                 320
```

Thr Arg Asp Ser Val Leu Arg Ser Leu Thr Thr Val Ser Gly Gly Ser
325 330 335

Ser Glu Ala Gly Leu Val Cys Asp His Leu Leu Gln Leu Glu Gly Gly
340 345 350

Ser Glu Val Leu Arg Ala Arg Thr Glu Trp Arg Pro Lys Leu Thr Asp
355 360 365

Ser Phe Arg Gly Ile Ser Val Ile Pro Ala Glu Pro Arg Val
370 375 380

<210> 213
<211> 1149
<212> DNA
<213> Umbellularia californica

<220>
<221> misc_feature
<223> Encodes thioesterase

<400> 213
atggccacca cctctttagc ttccgctttc tgctcgatga aagctgtaat gttggctcgt 60

gatggccggg gcatgaaacc caggagcagt gatttgcagc tgagggcggg aaatgcgcca 120

acctctttga agatgatcaa tgggaccaag ttcagttaca cggagagctt gaaaaggttg 180

cctgactgga gcatgctctt tgcagtgatc acaaccatct tttcggctgc tgagaagcag 240

tggaccaatc tagagtggaa gccgaagccg aagctacccc agttgcttga tgaccatttt 300

ggactgcatg ggttagtttt caggcgcacc tttgccatca gatcttatga ggtgggacct 360

gaccgctcca catctatact ggctgttatg aatcacatgc aggaggctac acttaatcat 420

gcgaagagtg tgggaattct aggagatgga ttcgggacga cgctagagat gagtaagaga 480

gatctgatgt gggttgtgag acgcacgcat gttgctgtgg aacggtaccc tacttggggt 540

gatactgtag aagtagagtg ctggattggt gcatctggaa ataatggcat gcgacgtgat 600

ttccttgtcc gggactgcaa aacaggcgaa attcttacaa gatgtaccag cctttcggtg 660

ctgatgaata caaggacaag gaggttgtcc acaatccctg acgaagttag aggggagata 720

gggcctgcat tcattgataa tgtggctgtc aaggacgatg aaattaagaa actacagaag 780

ctcaatgaca gcactgcaga ttacatccaa ggaggtttga ctcctcgatg gaatgatttg 840

gatgtcaatc agcatgtgaa caacctcaaa tacgttgcct gggtttttga gaccgtccca 900

gactccatct ttgagagtca tcatatttcc agcttcactc ttgaatacag gagagagtgc 960

acgagggata gcgtgctgcg gtccctgacc actgtctctg gtggctcgtc ggaggctggg 1020

ttagtgtgcg atcacttgct ccagcttgaa ggtgggtctg aggtattgag ggcaagaaca 1080

gagtggaggc ctaagcttac cgatagtttc agagggatta gtgtgatacc cgcagaaccg 1140

agggtgtaa 1149

<210> 214
<211> 376
<212> PRT
<213> Cuphea hookeriana

<220>
<221> MISC_FEATURE
<223> thioesterase (FatA1)

<400> 214

Met Leu Lys Leu Ser Cys Asn Ala Ala Thr Asp Gln Ile Leu Ser Ser
1 5 10 15

Ala Val Ala Gln Thr Ala Leu Trp Gly Gln Pro Arg Asn Arg Ser Phe
20 25 30

Ser Met Ser Ala Arg Arg Arg Gly Ala Val Cys Cys Ala Pro Pro Ala
35 40 45

Ala Gly Lys Pro Pro Ala Met Thr Ala Val Ile Pro Lys Asp Gly Val
50 55 60

Ala Ser Ser Gly Ser Gly Ser Leu Ala Asp Gln Leu Arg Leu Gly Ser
65 70 75 80

Arg Thr Gln Asn Gly Leu Ser Tyr Thr Glu Lys Phe Ile Val Arg Cys
85 90 95

Tyr Glu Val Gly Ile Asn Lys Thr Ala Thr Val Glu Thr Met Ala Asn
100 105 110

Leu Leu Gln Glu Val Gly Cys Asn His Ala Gln Ser Val Gly Phe Ser
115 120 125

Thr Asp Gly Phe Ala Thr Thr Pro Thr Met Arg Lys Leu Asn Leu Ile
130 135 140

Trp Val Thr Ala Arg Met His Ile Glu Ile Tyr Lys Tyr Pro Ala Trp
145 150 155 160

Ser Asp Val Val Glu Ile Glu Thr Trp Cys Gln Ser Glu Gly Arg Ile
165 170 175

Gly Thr Arg Arg Asp Trp Ile Leu Lys Asp Tyr Gly Asn Gly Glu Val

180 185 190

Ile Gly Arg Ala Thr Ser Lys Trp Val Met Met Asn Gln Asn Thr Arg
195 200 205

Arg Leu Gln Lys Val Asp Asp Ser Val Arg Glu Glu Tyr Met Val Phe
210 215 220

Cys Pro Arg Glu Pro Arg Leu Ser Phe Pro Glu Glu Asn Asn Arg Ser
225 230 235 240

Leu Arg Lys Ile Ser Lys Leu Glu Asp Pro Ala Glu Tyr Ser Arg Leu
245 250 255

Gly Leu Thr Pro Arg Arg Ala Asp Leu Asp Met Asn Gln His Val Asn
260 265 270

Asn Val Ala Tyr Ile Gly Trp Ala Leu Glu Ser Val Pro Gln Glu Ile
275 280 285

Ile Asp Ser Tyr Glu Leu Glu Thr Ile Thr Leu Asp Tyr Arg Arg Glu
290 295 300

Cys Gln Gln Asp Asp Val Val Asp Ser Leu Thr Ser Val Leu Ser Asp
305 310 315 320

Glu Glu Ser Gly Thr Leu Pro Glu Leu Lys Gly Thr Asn Gly Ser Ala
325 330 335

Ser Thr Pro Leu Lys Arg Asp His Asp Gly Ser Arg Gln Phe Leu His
340 345 350

Leu Leu Arg Leu Ser Pro Asp Gly Leu Glu Ile Asn Arg Gly Arg Thr
355 360 365

Glu Trp Arg Lys Lys Ser Thr Lys
370 375

<210> 215
<211> 1131
<212> DNA
<213> Cuphea hookeriana

<220>
<221> misc_feature
<223> Encodes thioesterase (FatA1)

<400> 215
atgttgaagc tttcttgcaa tgccgccacc gaccagattc tgtcgtcggc cgtggctcaa 60

accgcattat ggggtcaacc cagaaacaga tccttttcaa tgtccgcccg gagaagggga 120

gccgtttgct gcgcgcctcc agctgctgga aagccccctg ccatgaccgc tgttatccca 180

aaagacgggg tggcctcgtc cgggtccggc agcctggccg accagctgag gctcgggagc 240

cgtacgcaga atgggctgtc gtacacggag aagttcattg tcaggtgcta cgaggtcggt 300

attaacaaga cagccactgt cgaaaccatg gccaatctct tgcaggaagt aggttgtaac 360

catgctcaga gtgttggatt ctcaactgac gggtttgcga cgacgcctac catgaggaaa 420

ttgaatctga tatgggttac tgctcgaatg cacatagaaa tttataagta cccagcatgg 480

agtgatgtgg ttgaaatcga gacttggtgc caaagtgaag gaagaatcgg aacaagaagg 540

gattggattc tcaaggatta tggtaatggt gaagttattg gaagagccac aagcaagtgg 600

gtgatgatga accagaacac tagacgactc caaaaagttg atgattccgt tcgagaagag 660

tatatggttt tctgtccacg cgaaccaagg ttatcatttc ctgaagagaa caatcggagt 720

ttgagaaaaa tatctaaatt ggaagatcct gctgagtatt cgagacttgg tcttacgcct 780

agaagagctg atctggatat gaaccagcat gtcaacaacg ttgcttacat aggttgggct 840

ctggagagtg tacctcaaga aataatcgac tcttatgagc tggaaactat cactctggac 900

tacagaagag aatgccaaca ggatgacgta gtcgattcgc tcaccagtgt tctgtcagat 960

gaggaatcag gaacattacc agagctcaag ggaacaaatg gatctgcatc caccccactg 1020

aaacgtgacc atgatggctc tcgccagttc ttgcacttgc tgaggctctc cccgacggg 1080

ctagaaataa accgtggccg aactgaatgg agaaagaaat ccacgaaata g 1131

<210> 216
<211> 415
<212> PRT
<213> Cuphea hookeriana

<220>
<221> MISC_FEATURE
<223> thioesterase (FatB2)

<400> 216

Met Val Ala Ala Ala Ala Ser Ser Ala Phe Phe Pro Val Pro Ala Pro
1               5                   10                  15

Gly Ala Ser Pro Lys Pro Gly Lys Phe Gly Asn Trp Pro Ser Ser Leu
            20                  25                  30

Ser Pro Ser Phe Lys Pro Lys Ser Ile Pro Asn Gly Gly Phe Gln Val
        35                  40                  45

Lys Ala Asn Asp Ser Ala His Pro Lys Ala Asn Gly Ser Ala Val Ser

```
        50                  55                  60

Leu Lys Ser Gly Ser Leu Asn Thr Gln Glu Asp Thr Ser Ser Ser Pro
65                  70                  75                  80

Pro Pro Arg Thr Phe Leu His Gln Leu Pro Asp Trp Ser Arg Leu Leu
                85                  90                  95

Thr Ala Ile Thr Thr Val Phe Val Lys Ser Lys Arg Pro Asp Met His
            100                 105                 110

Asp Arg Lys Ser Lys Arg Pro Asp Met Leu Val Asp Ser Phe Gly Leu
        115                 120                 125

Glu Ser Thr Val Gln Asp Gly Leu Val Phe Arg Gln Ser Phe Ser Ile
    130                 135                 140

Arg Ser Tyr Glu Ile Gly Thr Asp Arg Thr Ala Ser Ile Glu Thr Leu
145                 150                 155                 160

Met Asn His Leu Gln Glu Thr Ser Leu Asn His Cys Lys Ser Thr Gly
                165                 170                 175

Ile Leu Leu Asp Gly Phe Gly Arg Thr Leu Glu Met Cys Lys Arg Asp
            180                 185                 190

Leu Ile Trp Val Val Ile Lys Met Gln Ile Lys Val Asn Arg Tyr Pro
        195                 200                 205

Ala Trp Gly Asp Thr Val Glu Ile Asn Thr Arg Phe Ser Arg Leu Gly
    210                 215                 220

Lys Ile Gly Met Gly Arg Asp Trp Leu Ile Ser Asp Cys Asn Thr Gly
225                 230                 235                 240

Glu Ile Leu Val Arg Ala Thr Ser Ala Tyr Ala Met Met Asn Gln Lys
                245                 250                 255

Thr Arg Arg Leu Ser Lys Leu Pro Tyr Glu Val His Gln Glu Ile Val
            260                 265                 270

Pro Leu Phe Val Asp Ser Pro Val Ile Glu Asp Ser Asp Leu Lys Val
        275                 280                 285

His Lys Phe Lys Val Lys Thr Gly Asp Ser Ile Gln Lys Gly Leu Thr
    290                 295                 300
```

Pro Gly Trp Asn Asp Leu Asp Val Asn Gln His Val Ser Asn Val Lys
305                 310                 315                 320

Tyr Ile Gly Trp Ile Leu Glu Ser Met Pro Thr Glu Val Leu Glu Thr
                325                 330                 335

Gln Glu Leu Cys Ser Leu Ala Leu Glu Tyr Arg Arg Glu Cys Gly Arg
            340                 345                 350

Asp Ser Val Leu Glu Ser Val Thr Ala Met Asp Pro Ser Lys Val Gly
        355                 360                 365

Val Arg Ser Gln Tyr Gln His Leu Leu Arg Leu Glu Asp Gly Thr Ala
    370                 375                 380

Ile Val Asn Gly Ala Thr Glu Trp Arg Pro Lys Asn Ala Gly Ala Asn
385                 390                 395                 400

Gly Ala Ile Ser Thr Gly Lys Thr Ser Asn Gly Asn Ser Val Ser
                405                 410                 415

<210> 217
<211> 1248
<212> DNA
<213> Cuphea hookeriana

<220>
<221> misc_feature
<223> Encodes thioesterase (FatB2)

<400> 217
atggtggctg ctgcagcaag ttccgcattc ttccctgttc cagccccggg agcctcccct 60

aaacccggga agttcggaaa ttggccctcg agcttgagcc cttccttcaa gcccaagtca 120

atccccaatg gcggatttca ggttaaggca aatgacagcg cccatccaaa ggctaacggt 180

tctgcagtta gtctaaagtc tggcagcctc aacactcagg aggacacttc gtcgtcccct 240

cctcctcgga ctttccttca ccagttgcct gattggagta ggcttctgac tgcaatcacg 300

accgtgttcg tgaaatctaa gaggcctgac atgcatgatc ggaaatccaa gaggcctgac 360

atgctggtgg actcgtttgg gttggagagt actgttcagg atgggctcgt gttccgacag 420

agtttttcga ttaggtctta tgaaataggc actgatcgaa cggcctctat agagacactt 480

atgaaccact tgcaggaaac atctctcaat cattgtaaga gtaccggtat tctccttgac 540

ggcttcggtc gtactcttga gatgtgtaaa agggacctca tttgggtggt aataaaaatg 600

cagatcaagg tgaatcgcta tccagcttgg ggcgatactg tcgagatcaa tacccggttc 660

tcccggttgg ggaaaatcgg tatgggtcgc gattggctaa taagtgattg caacacagga 720

gaaattcttg taagagctac gagcgcgtat gccatgatga atcaaaagac gagaagactc 780

tcaaaacttc catacgaggt tcaccaggag atagtgcctc tttttgtcga ctctcctgtc 840

attgaagaca gtgatctgaa agtgcataag tttaaagtga agactggtga ttccattcaa 900

aagggtctaa ctccggggtg gaatgacttg gatgtcaatc agcacgtaag caacgtgaag 960

tacattgggt ggattctcga gagtatgcca acagaagttt tggagaccca ggagctatgc 1020

tctctcgccc ttgaatatag gcgggaatgc ggaagggaca gtgtgctgga gtccgtgacc 1080

gctatggatc cctcaaaagt tggagtccgt tctcagtacc agcaccttct gcggcttgag 1140

gatgggactg ctatcgtgaa cggtgcaact gagtggcggc cgaagaatgc aggagctaac 1200

ggggcgatat caacgggaaa gacttcaaat ggaaactcgg tctcttag 1248

<210> 218
<211> 394
<212> PRT
<213> Cuphea hookeriana

<220>
<221> MISC_FEATURE
<223> thioesterase (FatB3)

<400> 218

Met Val Ala Ala Ala Ala Ser Ser Ala Phe Phe Ser Val Pro Thr Pro
1               5                   10                  15

Gly Ile Ser Pro Lys Pro Gly Lys Phe Gly Asn Gly Gly Phe Gln Val
            20                  25                  30

Lys Ala Asn Ala Asn Ala His Pro Ser Leu Lys Ser Gly Ser Leu Glu
        35                  40                  45

Thr Glu Asp Asp Thr Ser Ser Ser Ser Pro Pro Pro Arg Thr Phe Ile
    50                  55                  60

Asn Gln Leu Pro Asp Trp Ser Met Leu Leu Ser Ala Ile Thr Thr Ile
65                  70                  75                  80

Phe Gly Ala Ala Glu Lys Gln Trp Met Met Leu Asp Arg Lys Ser Lys
                85                  90                  95

Arg Pro Asp Met Leu Met Glu Pro Phe Gly Val Asp Ser Ile Val Gln
            100                 105                 110

Asp Gly Val Phe Phe Arg Gln Ser Phe Ser Ile Arg Ser Tyr Glu Ile
        115                 120                 125

```
Gly Ala Asp Arg Thr Thr Ser Ile Glu Thr Leu Met Asn Met Phe Gln
    130                 135                 140

Glu Thr Ser Leu Asn His Cys Lys Ser Asn Gly Leu Leu Asn Asp Gly
145                 150                 155                 160

Phe Gly Arg Thr Pro Glu Met Cys Lys Lys Gly Leu Ile Trp Val Val
                165                 170                 175

Thr Lys Met Gln Val Glu Val Asn Arg Tyr Pro Ile Trp Gly Asp Ser
            180                 185                 190

Ile Glu Val Asn Thr Trp Val Ser Glu Ser Gly Lys Asn Gly Met Gly
        195                 200                 205

Arg Asp Trp Leu Ile Ser Asp Cys Ser Thr Gly Glu Ile Leu Val Arg
    210                 215                 220

Ala Thr Ser Val Trp Ala Met Met Asn Gln Lys Thr Arg Arg Leu Ser
225                 230                 235                 240

Lys Phe Pro Phe Glu Val Arg Gln Glu Ile Ala Pro Asn Phe Val Asp
                245                 250                 255

Ser Val Pro Val Ile Glu Asp Asp Arg Lys Leu His Lys Leu Asp Val
            260                 265                 270

Lys Thr Gly Asp Ser Ile His Asn Gly Leu Thr Pro Arg Trp Asn Asp
        275                 280                 285

Leu Asp Val Asn Gln His Val Asn Asn Val Lys Tyr Ile Gly Trp Ile
    290                 295                 300

Leu Lys Ser Val Pro Thr Asp Val Phe Glu Ala Gln Glu Leu Cys Gly
305                 310                 315                 320

Val Thr Leu Glu Tyr Arg Arg Glu Cys Gly Arg Asp Ser Val Met Glu
                325                 330                 335

Ser Val Thr Ala Met Asp Pro Ser Lys Glu Gly Asp Arg Ser Val Tyr
            340                 345                 350

Gln His Leu Leu Arg Leu Glu Asp Gly Ala Asp Ile Ala Ile Gly Arg
        355                 360                 365

Thr Glu Trp Arg Pro Lys Asn Ala Gly Ala Asn Gly Ala Ile Ser Thr
    370                 375                 380
```

```
Gly Lys Thr Ser Asn Arg Asn Ser Val Ser
385                 390


<210>  219
<211>  1185
<212>  DNA
<213>  Cuphea hookeriana


<220>
<221>  misc_feature
<223>  Encodes thioesterase (FatB3)

<400>  219
atggtggctg ccgcagcaag ttctgcattc ttctccgttc caaccccggg aatctcccct      60

aaacccggga agttcggtaa tggtggcttt caggttaagg caaacgccaa tgcccatcct     120

agtctaaagt ctggcagcct cgagactgaa gatgacactt catcgtcgtc ccctcctcct     180

cggactttca ttaaccagtt gcccgactgg agtatgcttc tgtccgcaat cacgactatc     240

ttcggggcag ctgagaagca gtggatgatg cttgatagga aatctaagag acccgacatg     300

ctcatggaac cgtttggggt tgacagtatt gttcaggatg gggttttttt cagacagagt     360

ttttcgatta gatcttacga aataggcgct gatcgaacaa cctcaataga gacgctgatg     420

aacatgttcc aggaaacgtc tttgaatcat tgtaagagta acggtcttct caatgacggc     480

tttggtcgta ctcctgagat gtgtaagaag ggcctcattt gggtggttac gaaaatgcag     540

gtcgaggtga atcgctatcc tatttggggt gattctatcg aagtcaatac ttgggtctcc     600

gagtcgggga aaaacggtat gggtcgtgat tggctgataa gtgattgcag tacaggagaa     660

attcttgtaa gagcaacgag cgtgtgggct atgatgaatc aaaagacgag aagattgtca     720

aaatttccat ttgaggttcg acaagagata gcgcctaatt ttgtcgactc tgttcctgtc     780

attgaagacg atcgaaaatt acacaagctt gatgtgaaga cgggtgattc cattcacaat     840

ggtctaactc caaggtggaa tgacttggat gtcaatcagc acgttaacaa tgtgaaatac     900

attgggtgga ttctcaagag tgttccaaca gatgtttttg aggcccagga gctatgtgga     960

gtcacccttg agtataggcg ggaatgcgga agggacagtg tgatggagtc cgttacagct    1020

atggatccct caaaagaggg agaccgatca gtgtaccagc accttcttcg gcttgaggat    1080

ggggctgata tcgccatagg cagaaccgag tggcggccga agaatgcagg agccaatggg    1140

gcaatatcaa caggaaagac ttcaaataga aactctgtct cttag                   1185


<210>  220
<211>  382
<212>  PRT
<213>  Cinnamomum camphora
```

<220>
<221> MISC_FEATURE
<223> thioesterase (FatB)

<400> 220

```
Met Ala Thr Thr Ser Leu Ala Ser Ala Phe Cys Ser Met Lys Ala Val
1               5                   10                  15

Met Leu Ala Arg Asp Gly Arg Gly Met Lys Pro Arg Ser Ser Asp Leu
            20                  25                  30

Gln Leu Arg Ala Gly Asn Ala Gln Thr Ser Leu Lys Met Ile Asn Gly
        35                  40                  45

Thr Lys Phe Ser Tyr Thr Glu Ser Leu Lys Lys Leu Pro Asp Trp Ser
    50                  55                  60

Met Leu Phe Ala Val Ile Thr Thr Ile Phe Ser Ala Ala Glu Lys Gln
65                  70                  75                  80

Trp Thr Asn Leu Glu Trp Lys Pro Lys Pro Asn Pro Pro Gln Leu Leu
                85                  90                  95

Asp Asp His Phe Gly Pro His Gly Leu Val Phe Arg Arg Thr Phe Ala
            100                 105                 110

Ile Arg Ser Tyr Glu Val Gly Pro Asp Arg Ser Thr Ser Ile Val Ala
        115                 120                 125

Val Met Asn His Leu Gln Glu Ala Ala Leu Asn His Ala Lys Ser Val
    130                 135                 140

Gly Ile Leu Gly Asp Gly Phe Gly Thr Thr Leu Glu Met Ser Lys Arg
145                 150                 155                 160

Asp Leu Ile Trp Val Val Lys Arg Thr His Val Ala Val Glu Arg Tyr
                165                 170                 175

Pro Ala Trp Gly Asp Thr Val Glu Val Glu Cys Trp Val Gly Ala Ser
            180                 185                 190

Gly Asn Asn Gly Arg Arg His Asp Phe Leu Val Arg Asp Cys Lys Thr
        195                 200                 205

Gly Glu Ile Leu Thr Arg Cys Thr Ser Leu Ser Val Met Met Asn Thr
    210                 215                 220

Arg Thr Arg Arg Leu Ser Lys Ile Pro Glu Glu Val Arg Gly Glu Ile
```

225 230 235 240

Gly Pro Ala Phe Ile Asp Asn Val Ala Val Lys Asp Glu Glu Ile Lys
245 250 255

Lys Pro Gln Lys Leu Asn Asp Ser Thr Ala Asp Tyr Ile Gln Gly Gly
260 265 270

Leu Thr Pro Arg Trp Asn Asp Leu Asp Ile Asn Gln His Val Asn Asn
275 280 285

Ile Lys Tyr Val Asp Trp Ile Leu Glu Thr Val Pro Asp Ser Ile Phe
290 295 300

Glu Ser His His Ile Ser Ser Phe Thr Ile Glu Tyr Arg Arg Glu Cys
305 310 315 320

Thr Met Asp Ser Val Leu Gln Ser Leu Thr Thr Val Ser Gly Gly Ser
325 330 335

Ser Glu Ala Gly Leu Val Cys Glu His Leu Leu Gln Leu Glu Gly Gly
340 345 350

Ser Glu Val Leu Arg Ala Lys Thr Glu Trp Arg Pro Lys Leu Thr Asp
355 360 365

Ser Phe Arg Gly Ile Ser Val Ile Pro Ala Glu Ser Ser Val
370 375 380

<210> 221
<211> 1149
<212> DNA
<213> Cinnamomum camphora

<220>
<221> misc_feature
<223> Encodes thioesterase (FatB)

<400> 221
atggccacca cctctttagc ttctgctttc tgctcgatga aagctgtaat gttggctcgt 60

gatggcaggg gcatgaaacc caggagcagt gatttgcagc tgagggcggg aaatgcacaa 120

acctctttga agatgatcaa tgggaccaag ttcagttaca cagagagctt gaaaaagttg 180

cctgactgga gcatgctctt tgcagtgatc acgaccatct tttcggctgc tgagaagcag 240

tggaccaatc tagagtggaa gccgaagccg aatccacccc agttgcttga tgaccatttt 300

gggccgcatg ggttagtttt caggcgcacc tttgccatca gatcgtatga ggtgggacct 360

gaccgctcca catctatagt ggctgttatg aatcacttgc aggaggctgc acttaatcat 420

gcgaagagtg tgggaattct aggagatgga ttcggtacga cgctagagat gagtaagaga 480

gatctgatat gggttgtgaa acgcacgcat gttgctgtgg aacggtaccc tgcttggggt 540

gatactgttg aagtagagtg ctgggttggt gcatcgggaa ataatggcag gcgccatgat 600

ttccttgtcc gggactgcaa aacaggcgaa attcttacaa gatgtaccag tctttcggtg 660

atgatgaata caaggacaag gaggttgtcc aaaatccctg aagaagttag aggggagata 720

gggcctgcat tcattgataa tgtggctgtc aaggacgagg aaattaagaa accacagaag 780

ctcaatgaca gcactgcaga ttacatccaa ggaggattga ctcctcgatg gaatgatttg 840

gatatcaatc agcacgttaa caacatcaaa tacgttgact ggattcttga gactgtccca 900

gactcaatct ttgagagtca tcatatttcc agcttcacta ttgaatacag gagagagtgc 960

acgatggata gcgtgctgca gtccctgacc actgtctccg gtggctcgtc ggaagctggg 1020

ttagtgtgcg agcacttgct ccagcttgaa ggtgggtctg aggtattgag ggcaaaaaca 1080

gagtggaggc ctaagcttac cgatagtttc agagggatta gtgtgatacc cgcagaatcg 1140

agtgtctaa 1149

<210> 222
<211> 366
<212> PRT
<213> Brassica juncea

<220>
<221> MISC_FEATURE
<223> thioesterase (FatA)

<400> 222

Met Leu Lys Leu Ser Cys Asn Val Thr Asn His Leu His Thr Phe Phe
1 5 10 15

Phe Ser Ser Asp Ser Ser Leu Phe Ile Pro Gly Asn Arg Arg Thr Ile
20 25 30

Ala Val Ser Ser Ser Gln Pro Arg Lys Pro Ala Leu Asp Pro Leu Arg
35 40 45

Ala Val Ile Ser Ala Asp Gln Gly Ser Ile Ser Pro Val Asn Ser Tyr
50 55 60

Thr Pro Ala Asp Arg Phe Arg Ala Gly Arg Leu Met Glu Asp Gly Tyr
65 70 75 80

Ser Tyr Lys Glu Lys Phe Ile Val Arg Ser Tyr Glu Val Gly Ile Asn
85 90 95

```
Lys Thr Ala Thr Val Glu Thr Ile Ala Asn Leu Leu Gln Lys Val Ala
            100                 105                 110

Cys Thr His Val Gln Asn Val Gly Phe Ser Thr Asp Gly Phe Ala Thr
        115                 120                 125

Thr Leu Thr Met Arg Lys Leu His Leu Ile Trp Val Thr Ala Arg Met
    130                 135                 140

His Ile Glu Ile Tyr Arg Tyr Pro Ala Trp Ser Asp Val Val Glu Ile
145                 150                 155                 160

Glu Thr Trp Cys Gln Ser Glu Gly Arg Ile Gly Thr Arg Arg Asp Trp
                165                 170                 175

Ile Leu Arg Asp Ser Ala Thr Asn Glu Val Ile Gly Arg Ala Thr Ser
            180                 185                 190

Lys Trp Val Met Met Asn Gln Asp Thr Arg Arg Leu Gln Arg Val Thr
        195                 200                 205

Asp Glu Val Arg Asp Glu Tyr Leu Val Phe Cys Pro Arg Glu Pro Arg
    210                 215                 220

Leu Ala Phe Pro Glu Glu Asn Asn Ser Ser Leu Lys Lys Ile Pro Lys
225                 230                 235                 240

Leu Glu Asp Pro Ala Gln Tyr Ser Met Leu Glu Leu Lys Pro Arg Gln
                245                 250                 255

Ala Asp Leu Gly Met Asn Gln His Val Asn Asn Val Thr Tyr Ile Gly
            260                 265                 270

Trp Val Leu Glu Ser Ile Pro Gln Glu Ile Ile Asp Thr His Glu Leu
        275                 280                 285

Gln Val Ile Thr Leu Asp Tyr Arg Arg Glu Cys Gln Gln Asp Asp Ile
    290                 295                 300

Val Asp Ser Leu Thr Thr Ser Glu Ile Pro Asp Asp Pro Ile Ser Lys
305                 310                 315                 320

Leu Thr Gly Thr Asn Gly Ser Ala Thr Ser Arg Val Gln Gly His Asn
                325                 330                 335

Glu Ser Gln Phe Leu His Met Leu Arg Leu Ser Glu Asn Gly Gln Glu
            340                 345                 350
```

```
Ile Asn Arg Gly Arg Thr Gln Trp Arg Lys Lys Ser Pro Arg
        355                 360                 365


<210>  223
<211>  1101
<212>  DNA
<213>  Brassica juncea


<220>
<221>  misc_feature
<223>  Encodes thioesterase (FatA)

<400>  223
atgttgaagc tttcgtgcaa tgtgactaac cacttgcaca ccttcttctt ctcctccgat      60

tcctcccttt tcatcccggg taatcgccgt accatcgccg tctcctcttc tcagccaagg     120

aaaccggctt tagatcctct acgggcagtt atctccgccg atcagggaag catcagtcct     180

gttaattcgt atacgccggc ggatcggttc cgagctggtc gattgatgga agatggttat     240

tcatataaag agaagttcat tgttagaagc tatgaggttg gaatcaacaa aaccgccacc     300

gtcgagacaa ttgctaatct cttacagaag gtggcatgta cccatgttca gaacgttgga     360

ttctcgacgg atggatttgc cacaacactc accatgagga aactgcatct catatgggtc     420

actgcaagaa tgcacattga gatctacaga tatccagctt ggagtgatgt ggttgagata     480

gagacatggt gccagagtga aggaaggatt ggaacaagac gtgattggat tctaagggac     540

tctgctacaa atgaagttat tgggcgtgct acaagcaagt gggtgatgat gaatcaagac     600

acaaggcggc ttcaaagagt tacagatgaa gttcgggacg agtacttggt tttctgtcct     660

cgagaaccca gactagcgtt tccggaagag aacaatagca gcttaaagaa aatcccaaaa     720

ctagaagatc cagctcagta ttctatgctt gagcttaagc ctcggcaagc tgatctgggc     780

atgaaccagc acgtgaataa cgtcacctac atcggatggg tgcttgagag catacctcaa     840

gaaatcattg acacgcatga gcttcaagtt ataactctag attacagaag agaatgccag     900

caagatgaca ttgtcgattc actgaccacc tccgaaatcc ccgacgaccc gatctcaaag     960

cttactggga ccaacggatc tgccacgtca cgcgtacaag gacacaatga gagccaattc    1020

ttgcatatgc tgaggttgtc agaaaatggt caggagatca atcgtgggag aacacaatgg    1080

agaaagaaat ccccacgatg a                                              1101


<210>  224
<211>  365
<212>  PRT
<213>  Helianthus annuus


<220>
```

<221> MISC_FEATURE
<223> thioesterase (FatA1)

<400> 224

Met Leu Ser Arg Gly Val Pro Thr Ala Thr Ala Thr Ala Tyr Asn Asp
1               5                   10                  15

Val Ile Lys Glu Thr Ala Arg Ser Val Thr Asn Ser Arg Ser Ile Asp
            20                  25                  30

Ser Val Ser Ile Arg Arg Arg Asn Asn Gly Phe Val Ser Asn Ser Leu
        35                  40                  45

Cys Arg Arg Val Ala Pro Val Met Ala Val Lys Val Asp Glu Gln Arg
    50                  55                  60

Thr Gly Val Ala Val Asp Val Thr Glu Lys Arg Leu Ala Asp Arg Leu
65                  70                  75                  80

Arg Met Gly Ser Leu Thr Glu Asp Gly Leu Ser Tyr Lys Glu Arg Phe
                85                  90                  95

Ile Ile Arg Cys Tyr Glu Val Gly Ile Asn Lys Thr Ala Thr Val Glu
            100                 105                 110

Thr Ile Ala Asn Leu Leu Gln Glu Val Gly Gly Asn His Ala Gln Ser
        115                 120                 125

Val Gly Phe Ser Thr Asp Gly Phe Ala Thr Thr Thr Thr Met Arg Lys
    130                 135                 140

Leu Asn Leu Ile Trp Val Thr Ser Arg Met His Ile Glu Ile Tyr Arg
145                 150                 155                 160

Tyr Pro Ala Trp Ser Asp Val Val Glu Ile Glu Thr Trp Cys Gln Gly
                165                 170                 175

Glu Gly Arg Ile Gly Thr Arg Arg Asp Trp Ile Ile Lys Asp His Ala
            180                 185                 190

Asn Gly Glu Val Ile Gly Arg Ala Thr Ser Lys Trp Val Met Met Asn
        195                 200                 205

Ser Glu Thr Arg Arg Leu Gln Lys Val Asn Asp Asp Ile Arg Asp Glu
    210                 215                 220

Tyr Leu Ile Phe Cys Pro Lys Thr Leu Arg Leu Ala Phe Pro Glu Glu
225                 230                 235                 240

Asn Asn Asn Ser Leu Lys Lys Ile Ala Lys Leu Glu Asp Pro Ala Glu
245 250 255

Cys Ser Thr Leu Gly Leu Val Pro Arg Arg Ala Asp Leu Asp Met Asn
260 265 270

Lys His Val Asn Asn Val Thr Tyr Ile Gly Trp Val Leu Glu Ser Ile
275 280 285

Pro Gln Glu Val Ile Asp Thr His Glu Leu Gln Thr Ile Thr Leu Asp
290 295 300

Tyr Arg Arg Glu Cys Gln His Asp Asp Val Val Asp Ser Leu Thr Ser
305 310 315 320

Ser Glu Ser Pro Ala Val Asn Gly Cys Ala Ser Gly Glu Asn Leu Ser
325 330 335

Lys Phe Leu His Leu Leu Arg Ser Ser Gly Glu Gly Leu Glu Leu Asn
340 345 350

Arg Gly Arg Thr Glu Trp Arg Lys Lys Pro Ala Lys Lys
355 360 365

<210> 225
<211> 1095
<212> DNA
<213> Helianthus annuus

<220>
<221> misc_feature
<223> Encodes thioesterase (FatA1)

<400> 225
atgctctcca gaggtgttcc gaccgccacc gccaccgcct acaatgacgt aatcaaagaa 60

accgctcgtt ccgtcaccaa ttcccgatct atcgattccg tttcaattcg acgaagaaac 120

aacggttttg tgtccaattc gttgtgccgg agagttgcgc cggtaatggc ggtgaaggtt 180

gatgagcagc ggaccggagt cgcggtcgat gttacggaga aacggctcgc ggaccggttg 240

cggatgggga gcttgacgga agacggattg tcgtataagg agaggtttat tattaggtgt 300

tatgaggtcg ggattaataa gacggcgact gttgagacga ttgcgaatct gttgcaggag 360

gtaggaggaa atcatgctca gagtgtcgga ttttcgacgg atggatttgc aactacaacc 420

accatgagaa agttgaatct catatgggtc acttcacgaa tgcacattga aatttacaga 480

tatcctgctt ggagtgatgt ggttgaaatt gagacttggt gtcaaggtga agggagaatc 540

gggactagac gtgattggat tatcaaagat catgccaatg gtgaggtcat cggaagggct 600

acaagcaagt gggtgatgat gaactcagaa actagaagac tccagaaagt caatgacgat 660

ataagagatg aatatttaat attttgtccc aagacactga gattagcatt ccctgaagag 720

aacaacaata gcctgaaaaa aatagcaaaa ctggaagatc ctgctgagtg ttcgacactc 780

ggtcttgtgc ccagacgagc ggatcttgat atgaacaagc atgttaacaa tgttacctac 840

attggatggg ttctcgagag catcccacaa gaagttatcg acactcatga actacaaaca 900

atcaccttag attacaggcg tgaatgccag catgatgatg tagtcgattc cctcacgagt 960

tctgaatcac ccgctgttaa cggttgtgct agtggtgaaa atttaagcaa gtttttgcat 1020

ctactaagat catctggtga aggtcttgaa ctaaacaggg gtcgcactga atggaggaaa 1080

aaacccgcaa aaaaa 1095

<210> 226
<211> 355
<212> PRT
<213> Clostridium acetobutylicum

<220>
<221> MISC_FEATURE
<223> butyrate kinase

<400> 226

Met Ser Tyr Lys Leu Leu Ile Ile Asn Pro Gly Ser Thr Ser Thr Lys
1 5 10 15

Ile Gly Val Tyr Glu Gly Glu Lys Glu Leu Phe Glu Glu Thr Leu Arg
20 25 30

His Thr Asn Glu Glu Ile Lys Arg Tyr Asp Thr Ile Tyr Asp Gln Phe
35 40 45

Glu Phe Arg Lys Glu Val Ile Leu Asn Val Leu Lys Glu Lys Asn Phe
50 55 60

Asp Ile Lys Thr Leu Ser Ala Ile Val Gly Arg Gly Gly Met Leu Arg
65 70 75 80

Pro Val Glu Gly Gly Thr Tyr Ala Val Asn Asp Ala Met Val Glu Asp
85 90 95

Leu Lys Val Gly Val Gln Gly Pro His Ala Ser Asn Leu Gly Gly Ile
100 105 110

Ile Ala Lys Ser Ile Gly Asp Glu Leu Asn Ile Pro Ser Phe Ile Val
115 120 125

```
Asp Pro Val Val Thr Asp Glu Leu Ala Asp Val Ala Arg Leu Ser Gly
    130                 135                 140

Val Pro Glu Leu Pro Arg Lys Ser Lys Phe His Ala Leu Asn Gln Lys
145                 150                 155                 160

Ala Val Ala Lys Arg Tyr Gly Lys Glu Ser Gly Gln Gly Tyr Glu Asn
                165                 170                 175

Leu Asn Leu Val Val Val His Met Gly Gly Gly Val Ser Val Gly Ala
            180                 185                 190

His Asn His Gly Lys Val Val Asp Val Asn Asn Ala Leu Asp Gly Asp
        195                 200                 205

Gly Pro Phe Ser Pro Glu Arg Ala Gly Ser Val Pro Ile Gly Asp Leu
    210                 215                 220

Val Lys Met Cys Phe Ser Gly Lys Tyr Ser Glu Ala Glu Val Tyr Gly
225                 230                 235                 240

Lys Ala Val Gly Lys Gly Gly Phe Val Gly Tyr Leu Asn Thr Asn Asp
                245                 250                 255

Val Lys Gly Val Ile Asp Lys Met Glu Glu Gly Asp Lys Glu Cys Glu
            260                 265                 270

Ser Ile Tyr Lys Ala Phe Val Tyr Gln Ile Ser Lys Ala Ile Gly Glu
        275                 280                 285

Met Ser Val Val Leu Glu Gly Lys Val Asp Gln Ile Ile Phe Thr Gly
    290                 295                 300

Gly Ile Ala Tyr Ser Pro Thr Leu Val Pro Asp Leu Lys Ala Lys Val
305                 310                 315                 320

Glu Trp Ile Ala Pro Val Thr Val Tyr Pro Gly Glu Asp Glu Leu Leu
                325                 330                 335

Ala Leu Ala Gln Gly Ala Ile Arg Val Leu Asp Gly Glu Glu Gln Ala
            340                 345                 350

Lys Val Tyr
        355

<210>  227
```

<211> 296
<212> PRT
<213> Clostridium acetobutylicum

<220>
<221> MISC_FEATURE
<223> phosphotransbutyrylase

<400> 227

Met Ile Lys Ser Phe Asn Glu Ile Ile Met Lys Val Lys Ser Lys Glu
1               5                   10                  15

Met Lys Lys Val Ala Val Ala Val Ala Gln Asp Glu Pro Val Leu Glu
            20                  25                  30

Ala Val Arg Asp Ala Lys Lys Asn Gly Ile Ala Asp Ala Ile Leu Val
        35                  40                  45

Gly Asp His Asp Glu Ile Val Ser Ile Ala Leu Lys Ile Gly Met Asp
    50                  55                  60

Val Asn Asp Phe Glu Ile Val Asn Glu Pro Asn Val Lys Lys Ala Ala
65                  70                  75                  80

Leu Lys Ala Val Glu Leu Val Ser Thr Gly Lys Ala Asp Ile Leu Met
                85                  90                  95

Asn Gly Leu Val Asn Thr Ala Thr Phe Leu Lys Ile Cys Ile Leu Asn
            100                 105                 110

Lys Glu Val Gly Leu Arg Thr Gly Lys Thr Met Ser His Val Ala Val
        115                 120                 125

Phe Glu Thr Glu Thr Ser Asp Arg Leu Ser Phe Leu Thr Asp Val Ala
    130                 135                 140

Phe Asn Thr Tyr Pro Glu Leu Lys Glu Lys Ile Asp Ile Val Asn Asn
145                 150                 155                 160

Ser Val Lys Val Ala His Ala Ile Gly Ile Val Asn Pro Lys Val Ala
                165                 170                 175

Pro Ile Cys Ala Val Glu Val Ile Asn Pro Lys Met Pro Ser Thr Leu
            180                 185                 190

Asp Ala Ala Met Leu Ser Lys Met Ser Asp Arg Gly Gln Ile Lys Gly
        195                 200                 205

```
Cys Val Val Asp Gly Pro Leu Ala Leu Asp Ile Ala Leu Ser Glu Glu
    210                 215                 220

Ala Ala His His Lys Gly Val Thr Gly Glu Val Ala Gly Lys Ala Asp
225                 230                 235                 240

Ile Phe Leu Met Pro Asn Ile Glu Thr Gly Asn Val Met Tyr Lys Thr
                245                 250                 255

Leu Thr Tyr Thr Thr Asp Ser Lys Asn Gly Gly Ile Leu Val Gly Thr
            260                 265                 270

Ser Ala Pro Val Val Leu Thr Ser Arg Ala Asp Ser His Glu Thr Lys
        275                 280                 285

Met Asn Ser Ile Ala Leu Ala Ala
    290                 295


<210>  228
<211>  5544
<212>  DNA
<213>  Artificial sequence

<220>
<223>  pGL10.173B plasmid

<400>  228
tcgcgacgcg aggctggatg gccttcccca ttatgattct tctcgcttcc ggcggcatcg      60

ggatgcccgc gttgcaggcc atgctgtcca ggcaggtaga tgacgaccat cagggacagc     120

ttcaaggatc gctcgcggct cttaccagcc taacttcgat cactggaccg ctgatcgtca     180

cggcgattta tgccgcctcg gcgagcacat ggaacgggtt ggcatggatt gtaggcgccg     240

ccctatacct tgtctgcctc cccgcgttgc gtcgcggtgc atggagccgg gccacctcga     300

cctgaatgga agccggcggc acctcgctaa cggattcacc actccaagaa ttggagccaa     360

tcaattcttg cggagaactg tgaatgcgca aaccaaccct tggcagaaca tatccatcgc     420

gtccgccatc tccagcagcc gcacgcggcg catctcgggc agcgttgggt cctggccacg     480

ggtgcgcatg atcgtgctcc tgtcgttgag gacccggcta ggctggcggg gttgccttac     540

tggttagcag aatgaatcac cgatacgcga gcgaacgtga agcgactgct gctgcaaaac     600

gtctgcgacc tgagcaacaa catgaatggt cttcggtttc cgtgtttcgt aaagtctgga     660

aacgcggaag tcagcgccct gcaccattat gttccggatc tgcatcgcag gatgctgctg     720

gctaccctgt ggaacaccta catctgtatt aacgaagcgc tggcattgac cctgagtgat     780

ttttctctgg tcccgccgca tccataccgc cagttgttta ccctcacaac gttccagtaa     840

ccgggcatgt tcatcatcag taacccgtat cgtgagcatc ctctctcgtt tcatcggtat     900
```

cattaccccc atgaacagaa atccccctta cacggaggca tcagtgacca aacaggaaaa 960
aaccgccctt aacatggccc gctttatcag aagccagaca ttaacgcttc tggagaaact 1020
caacgagctg gacgcggatg aacaggcaga catctgtgaa tcgcttcacg accacgctga 1080
tgagctttac cgcagctgcc tcgcgcgttt cggtgatgac ggtgaaaacc tctgacacat 1140
gcagctcccg gagacggtca cagcttgtct gtaagcggat gccgggagca gacaagcccg 1200
tcagggcgcg tcagcgggtg ttggcgggtg tcggggcgca gccatgaccc agtcacgtag 1260
cgatagcgga gtgtatactg gcttaactat gcggcatcag agcagattgt actgagagtg 1320
caccatatgc ggtgtgaaat accgcacaga tgcgtaagga gaaaataccg catcaggcgc 1380
tcttccgctt cctcgctcac tgactcgctg cgctcggtcg ttcggctgcg gcgagcggta 1440
tcagctcact caaaggcggt aatacggtta tccacagaat caggggataa cgcaggaaag 1500
aacatgtgag caaaaggcca gcaaaaggcc aggaaccgta aaaaggccgc gttgctggcg 1560
tttttccata ggctccgccc ccctgacgag catcacaaaa atcgacgctc aagtcagagg 1620
tggcgaaacc cgacaggact ataaagatac caggcgtttc cccctggaag ctccctcgtg 1680
cgctctcctg ttccgaccct gccgcttacc ggatacctgt ccgcctttct cccttcggga 1740
agcgtggcgc tttctcatag ctcacgctgt aggtatctca gttcggtgta ggtcgttcgc 1800
tccaagctgg gctgtgtgca cgaacccccc gttcagcccg accgctgcgc cttatccggt 1860
aactatcgtc ttgagtccaa cccggtaaga cacgacttat cgccactggc agcagccact 1920
ggtaacagga ttagcagagc gaggtatgta ggcggtgcta cagagttctt gaagtggtgg 1980
cctaactacg gctacactag aaggacagta tttggtatct gcgctctgct gaagccagtt 2040
accttcggaa aaagagttgg tagctcttga tccggcaaac aaaccaccgc tggtagcggt 2100
ggtttttttg tttgcaagca gcagattacg cgcagaaaaa aaggatctca agaagatcct 2160
ttgatctttt ctacggggtc tgacgctcag tggaacgaaa actcacgtta agggattttg 2220
gtcatgagat tatcaaaaag gatcttcacc tagatccttt taaattaaaa atgaagtttt 2280
aaatcaatct aaagtatata tgagtaaact tggtctgaca gttaccaatg cttaatcagt 2340
gaggcaccta tctcagcgat ctgtctattt cgttcatcca tagttgcctg actccccgtc 2400
gtgtagataa ctacgatacg ggagggctta ccatctggcc ccagtgctgc aatgataccg 2460
cgagacccac gctcaccggc tccagattta tcagcaataa accagccagc cggaagggcc 2520
gagcgcagaa gtggtcctgc aactttatcc gcctccatcc agtctattaa ttgttgccgg 2580
gaagctagag taagtagttc gccagttaat agtttgcgca acgttgttgc cattgctgca 2640
ggcatcgtgg tgtcacgctc gtcgtttggt atggcttcat tcagctccgg ttcccaacga 2700
tcaaggcgag ttacatgatc ccccatgttg tgcaaaaaag cggttagctc cttcggtcct 2760
ccgatcgttg tcagaagtaa gttggccgca gtgttatcac tcatggttat ggcagcactg 2820

cataattctc ttactgtcat gccatccgta agatgctttt ctgtgactgg tgagtactca 2880
accaagtcat tctgagaata gtgtatgcgg cgaccgagtt gctcttgccc ggcgtcaaca 2940
cgggataata ccgcgccaca tagcagaact ttaaaagtgc tcatcattgg aaaacgttct 3000
tcggggcgaa aactctcaag gatcttaccg ctgttgagat ccagttcgat gtaacccact 3060
cgtgcaccca actgatcttc agcatctttt actttcacca gcgtttctgg gtgagcaaaa 3120
acaggaaggc aaaatgccgc aaaaaaggga ataagggcga cacggaaatg ttgaatactc 3180
atactcttcc tttttcaata ttattgaagc atttatcagg gttattgtct catgagcgga 3240
tacatatttg aatgtattta gaaaaataaa caaatagggg ttccgcgcac atttccccga 3300
aaagtgccac ctgacgtctt aattaatcag gagagcgttc accgacaaac aacagataaa 3360
acgaaaggcc cagtctttcg actgagcctt tcgttttatt tgatgcctgg cagttcccta 3420
ctctcgcatg gggagacccc acactaccat cggcgctacg gcgtttcact tctgagttcg 3480
gcatggggtc aggtgggacc accgcgctac tgccgccagg caaattctgt tttatcagac 3540
cgcttctgcg ttctgattta atctgtatca ggctgaaaat cttctctcat ccgccaaaac 3600
agccaagctg gagaccgttt aaactcaatg atgatgatga tgatggtcga cggcgctatt 3660
cagatcctct tctgagatga gtttttgttc gggcccaagc ttcgaattcc catatggtac 3720
cagctgcaga tctcgagctc ggatccatgg tttattcctc cttatttaat cgatacatta 3780
atatatacct ctttaatttt taataataaa gttaatcgat aattccggtc gagtgcccac 3840
acagattgtc tgataaattg ttaaagagca gtgccgcttc gctttttctc agcggcgctg 3900
tttcctgtgt gaaattgtta tccgctcaca attccacaca ttatacgagc cggatgatta 3960
attgtcaaca gctcatttca gaatatttgc cagaaccgtt atgatgtcgg cgcaaaaaac 4020
attatccaga acgggagtgc gccttgagcg acacgaatta tgcagtgatt tacgacctgc 4080
acagccatac cacagcttcc gatggctgcc tgacgccaga agcattggtg caccgtgcag 4140
tcgatgataa gctgtcaaac cagatcaatt cgcgctaact cacattaatt gcgttgcgct 4200
cactgcccgc tttccagtcg ggaaacctgt cgtgccagct gcattaatga atcggccaac 4260
gcgcggggag aggcggtttg cgtattgggc gccagggtgg tttttctttt caccagtgag 4320
acgggcaaca gctgattgcc cttcaccgcc tggccctgag agagttgcag caagcggtcc 4380
acgctggttt gccccagcag gcgaaaatcc tgtttgatgg tggttgacgg cgggatataa 4440
catgagctgt cttcggtatc gtcgtatccc actaccgaga tatccgcacc aacgcgcagc 4500
ccggactcgg taatggcgcg cattgcgccc agcgccatct gatcgttggc aaccagcatc 4560
gcagtgggaa cgatgccctc attcagcatt tgcatggttt gttgaaaacc ggacatggca 4620
ctccagtcgc cttcccgttc cgctatcggc tgaatttgat tgcgagtgag atatttatgc 4680

cagccagcca gacgcagacg cgccgagaca gaacttaatg ggcccgctaa cagcgcgatt 4740

tgctggtgac ccaatgcgac cagatgctcc acgcccagtc gcgtaccgtc ttcatgggag 4800

aaaataatac tgttgatggg tgtctggtca gagacatcaa gaaataacgc cggaacatta 4860

gtgcaggcag cttccacagc aatggcatcc tggtcatcca gcggatagtt aatgatcagc 4920

ccactgacgc gttgcgcgag aagattgtgc accgccgctt tacaggcttc gacgccgctt 4980

cgttctacca tcgacaccac cacgctggca cccagttgat cggcgcgaga tttaatcgcc 5040

gcgacaattt gcgacggcgc gtgcagggcc agactggagg tggcaacgcc aatcagcaac 5100

gactgtttgc ccgccagttg ttgtgccacg cggttgggaa tgtaattcag ctccgccatc 5160

gccgcttcca ctttttcccg cgttttcgca gaaacgtggc tggcctggtt caccacgcgg 5220

gaaacggtct gataagagac accggcatac tctgcgacat cgtataacgt tactggtttc 5280

acattcacca ccctgaattg actctcttcc gggcgctatc atgccatacc gcgaaaggtt 5340

ttgcaccatt cgatggtgtc aacgtaaatg catgccgctt cgccttcgcg cgcgaattga 5400

tctgctgcct cgcgcgtttc ggtgatgacg gtgaaaacct ctgacacatg cagctcccgg 5460

agacggtcac agcttgtctg taagcggatg ccgggagcag acaagcccgt cagggcgcgt 5520

cagcgggtgt tggcggggcc ggcc 5544

<210> 229
<211> 4559
<212> DNA
<213> Artificial sequence

<220>
<223> pDG2 plasmid

<400> 229
ggggaattgt gagcggataa caattcccct gtagaaataa ttttgtttaa ctttaataag 60

gagatatacc atggcgcaac tcactcttct tttagtcggc aattccgacg ccatcacgcc 120

attacttgct aaagctgact ttgaacaacg ttcgcgtctg cagattattc ctgcgcagtc 180

agttatcgcc agtgatgccc ggccttcgca agctatccgc gccagtcgtg ggagttcaat 240

gcgcgtggcc ctggagctgg tgaaagaagg tcgagcgcaa gcctgtgtca gtgccggtaa 300

taccggggcg ctgatggggc tggcaaaatt attactcaag cccctggagg ggattgagcg 360

tccggcgctg gtgacggtat taccacatca gcaaaagggc aaaacggtgg tccttgactt 420

aggggccaac gtcgattgtg acagcacaat gctggtgcaa tttgccatta tgggctcagt 480

tctggctgaa gaggtggtgg aaattcccaa tcctcgcgtg gcgttgctca atattggtga 540

agaagaagta aagggtctcg acagtattcg ggatgcctca gcggtgctta aaacaatccc 600

ttctatcaat tatatcggct atcttgaagc caatgagttg ttaactggca agacagatgt 660

gctggtttgt gacggcttta caggaaatgt cacattaaag acgatggaag gtgttgtcag 720

gatgttcctt tctctgctga aatctcaggg tgaagggaaa aaacggtcgt ggtggctact 780
gttattaaag cgttggctac aaaagagcct gacgaggcga ttcagtcacc tcaaccccga 840
ccagtataac ggcgcctgtc tgttaggatt gcgcggcacg gtgataaaaa gtcatggtgc 900
agccaatcag cgagcttttg cggtcgcgat tgaacaggca gtgcaggcgg tgcagcgaca 960
agttcctcag cgaattgccg ctcgcctgga atctgtatac ccagctggtt ttgagctgct 1020
ggacggtggc aaaagcggaa ctctgcggta gcaggacgct gccagcgaac tcgcagtttg 1080
caagtgacgg tatataaccg aaaagtgact gagcgcatat gtatacgaag actcgagtct 1140
ggtaaagaaa ccgctgctgc gaaatttgaa cgccagcaca tggactcgtc tactagcgca 1200
gcttaattaa cctaggctgc tgccaccgct gagcaataac tagcataacc ccttggggcc 1260
tctaaacggg tcttgagggg ttttttgctg aaacctcagg catttgagaa gcacacggtc 1320
acactgcttc cggtagtcaa taaaccggta aaccagcaat agacataagc ggctatttaa 1380
cgaccctgcc ctgaaccgac gaccgggtca tcgtggccgg atcttgcggc ccctcggctt 1440
gaacgaattg ttagacatta tttgccgact accttggtga tctcgccttt cacgtagtgg 1500
acaaattctt ccaactgatc tgcgcgcgag gccaagcgat cttcttcttg tccaagataa 1560
gcctgtctag cttcaagtat gacgggctga tactgggccg gcaggcgctc cattgcccag 1620
tcggcagcga catccttcgg cgcgattttg ccggttactg cgctgtacca aatgcgggac 1680
aacgtaagca ctacatttcg ctcatcgcca gcccagtcgg gcggcgagtt ccatagcgtt 1740
aaggtttcat ttagcgcctc aaatagatcc tgttcaggaa ccggatcaaa gagttcctcc 1800
gccgctggac ctaccaaggc aacgctatgt tctcttgctt ttgtcagcaa gatagccaga 1860
tcaatgtcga tcgtggctgg ctcgaagata cctgcaagaa tgtcattgcg ctgccattct 1920
ccaaattgca gttcgcgctt agctggataa cgccacggaa tgatgtcgtc gtgcacaaca 1980
atggtgactt ctacagcgcg gagaatctcg ctctctccag gggaagccga agtttccaaa 2040
aggtcgttga tcaaagctcg ccgcgttgtt tcatcaagcc ttacggtcac cgtaaccagc 2100
aaatcaatat cactgtgtgg cttcaggccg ccatccactg cggagccgta caaatgtacg 2160
gccagcaacg tcggttcgag atggcgctcg atgacgccaa ctacctctga tagttgagtc 2220
gatacttcgg cgatcaccgc ttccctcata ctcttccttt ttcaatatta ttgaagcatt 2280
tatcagggtt attgtctcat gagcggatac atatttgaat gtatttagaa aaataaacaa 2340
atagctagct cactcggtcg ctacgctccg ggcgtgagac tgcggcgggc gctgcggaca 2400
catacaaagt tacccacaga ttccgtggat aagcagggga ctaacatgtg aggcaaaaca 2460
gcagggccgc gccggtggcg tttttccata ggctccgccc tcctgccaga gttcacataa 2520
acagacgctt ttccggtgca tctgtgggag ccgtgaggct caaccatgaa tctgacagta 2580

cgggcgaaac ccgacaggac ttaaagatcc ccaccgtttc cggcgggtcg ctccctcttg 2640

cgctctcctg ttccgaccct gccgtttacc ggatacctgt tccgcctttc tcccttacgg 2700

gaagtgtggc gctttctcat agctcacaca ctggtatctc ggctcggtgt aggtcgttcg 2760

ctccaagctg ggctgtaagc aagaactccc cgttcagccc gactgctgcg ccttatccgg 2820

taactgttca cttgagtcca acccggaaaa gcacggtaaa acgccactgg cagcagccat 2880

tggtaactgg gagttcgcag aggatttgtt tagctaaaca cgcggttgct cttgaagtgt 2940

gcgccaaagt ccggctacac tggaaggaca gatttggttg ctgtgctctg cgaaagccag 3000

ttaccacggt taagcagttc cccaactgac ttaaccttcg atcaaaccac ctccccaggt 3060

ggtttttttcg tttacagggc aaaagattac gcgcagaaaa aaaggatctc aagaagatcc 3120

tttgatcttt tctactgaac cgctctagat ttcagtgcaa tttatctctt caaatgtagc 3180

acctgaagtc agccccatac gatataagtt gtaattctca tgttagtcat gccccgcgcc 3240

caccggaagg agctgactgg gttgaaggct ctcaagggca tcggtcgaga tcccggtgcc 3300

taatgagtga gctaacttac attaattgcg ttgcgctcac tgcccgcttt ccagtcggga 3360

aacctgtcgt gccagctgca ttaatgaatc ggccaacgcg cggggagagg cggtttgcgt 3420

attgggcgcc agggtggttt ttcttttcac cagtgagacg ggcaacagct gattgccctt 3480

caccgcctgg ccctgagaga gttgcagcaa gcggtccacg ctggtttgcc ccagcaggcg 3540

aaaatcctgt ttgatggtgg ttaacggcgg gatataacat gagctgtctt cggtatcgtc 3600

gtatcccact accgagatgt ccgcaccaac gcgcagcccg gactcggtaa tggcgcgcat 3660

tgcgcccagc gccatctgat cgttggcaac cagcatcgca gtgggaacga tgccctcatt 3720

cagcatttgc atggtttgtt gaaaaccgga catggcactc cagtcgcctt cccgttccgc 3780

tatcggctga atttgattgc gagtgagata tttatgccag ccagccagac gcagacgcgc 3840

cgagacagaa cttaatgggc ccgctaacag cgcgatttgc tggtgaccca atgcgaccag 3900

atgctccacg cccagtcgcg taccgtcttc atgggagaaa ataatactgt tgatgggtgt 3960

ctggtcagag acatcaagaa ataacgccgg aacattagtg caggcagctt ccacagcaat 4020

ggcatcctgg tcatccagcg gatagttaat gatcagccca ctgacgcgtt gcgcgagaag 4080

attgtgcacc gccgctttac aggcttcgac gccgcttcgt tctaccatcg acaccaccac 4140

gctggcaccc agttgatcgg cgcgagattt aatcgccgcg acaatttgcg acggcgcgtg 4200

cagggccaga ctggaggtgg caacgccaat cagcaacgac tgtttgcccg ccagttgttg 4260

tgccacgcgg ttgggaatgt aattcagctc cgccatcgcc gcttccactt tttcccgcgt 4320

tttcgcagaa acgtggctgg cctggttcac cacgcgggaa acggtctgat aagagacacc 4380

ggcatactct gcgacatcgt ataacgttac tggtttcaca ttcaccaccc tgaattgact 4440

ctcttccggg cgctatcatg ccataccgcg aaaggttttg cgccattcga tggtgtccgg 4500

gatctcgacg ctctccctta tgcgactcct gcattaggaa attaatacga ctcactata 4559

<210> 230
<211> 5502
<212> DNA
<213> Artificial sequence

<220>
<223> pDG5 plasmid

<400> 230
ggggaattgt gagcggataa caattcccct gtagaaataa ttttgtttaa ctttaataag 60

gagatatacc atggcgcaac tcactcttct tttagtcggc aattccgacg ccatcacgcc 120

attacttgct aaagctgact ttgaacaacg ttcgcgtctg cagattattc ctgcgcagtc 180

agttatcgcc agtgatgccc ggccttcgca agctatccgc gccagtcgtg ggagttcaat 240

gcgcgtggcc ctggagctgg tgaaagaagg tcgagcgcaa gcctgtgtca gtgccggtaa 300

taccggggcg ctgatggggc tggcaaaatt attactcaag cccctggagg ggattgagcg 360

tccggcgctg gtgacggtat taccacatca gcaaaagggc aaaacggtgg tccttgactt 420

aggggccaac gtcgattgtg acagcacaat gctggtgcaa tttgccatta tgggctcagt 480

tctggctgaa gaggtggtgg aaattcccaa tcctcgcgtg gcgttgctca atattggtga 540

agaagaagta aagggtctcg acagtattcg ggatgcctca gcggtgctta aaacaatccc 600

ttctatcaat tatatcggct atcttgaagc caatgagttg ttaactggca agacagatgt 660

gctggtttgt gacggcttta caggaaatgt cacattaaag acgatggaag gtgttgtcag 720

gatgttcctt tctctgctga aatctcaggg tgaagggaaa aaacggtcgt ggtggctact 780

gttattaaag cgttggctac aaaagagcct gacgaggcga ttcagtcacc tcaaccccga 840

ccagtataac ggcgcctgtc tgttaggatt gcgcggcacg gtgataaaaa gtcatggtgc 900

agccaatcag cgagcttttg cggtcgcgat tgaacaggca gtgcaggcgg tgcagcgaca 960

agttcctcag cgaattgccg ctcgcctgga atctgtatac ccagctggtt ttgagctgct 1020

ggacggtggc aaaagcggaa ctctgcggta gcaggacgct gccagcgaac tcgcagtttg 1080

caagtgacgg tatataaccg aaaagtgact gagcgcatat gaaagctggc attcttggtg 1140

ttggacgtta cattcctgag aaggttttaa caaatcatga tcttgaaaaa atggttgaaa 1200

cttctgacga gtggattcgt acaagaacag gaatagaaga aagaagaatc gcagcagatg 1260

atgtgttttc atcacacatg gctgttgcag cagcgaaaaa tgcgctggaa caagctgaag 1320

tggctgctga ggatctggat atgatcttgg ttgcaactgt tacacctgat cagtcattcc 1380

ctacggtgtc ttgtatgatt caagaacaac tcggcgcgaa gaaagcgtgt gctatggata 1440

tcagcgcggc ttgtgcgggc ttcatgtacg ggttgtaacc ggtaaacaa tttattgaat 1500

ccggaaccta caagcatgtt ctagttgttg gtgtagagaa gctctcaagc attaccgact 1560

gggaagaccg caatacagcc gttctgtttg gagacggagc aggcgctgcg gtagtcgggc 1620

cagtcagtga tgacagagga atcctttcat ttgaactagg agccgacggc acaggcggtc 1680

agcacttgta tctgaatgaa aaacgacata caatcatgaa tggacgagaa gttttcaaat 1740

ttgcagtccg ccaaatggga gaatcatgcg taaatgtcat tgaaaaagcc ggactttcaa 1800

aagaggatgt ggactttttg attccgcatc aggcgaacat ccgtatcatg gaagctgctc 1860

gcgagcgttt agagcttcct gtcgaaaaga tgtctaaaac tgttcataaa tatggaaata 1920

cttctgccgc atccattccg atctctcttg tagaagaatt ggaagccggt aaaatcaaag 1980

acggcgatgt ggtcgttatg gtagggttcg gcggaggact aacatggggc gccattgcaa 2040

tccgctgggg ccgataaaaa aaaggtgagg tgcactcgag tctggtaaag aaaccgctgc 2100

tgcgaaattt gaacgccagc acatggactc gtctactagc gcagcttaat taacctaggc 2160

tgctgccacc gctgagcaat aactagcata accccttggg gcctctaaac gggtcttgag 2220

gggttttttg ctgaaacctc aggcatttga gaagcacacg gtcacactgc ttccggtagt 2280

caataaaccg gtaaaccagc aatagacata agcggctatt taacgaccct gccctgaacc 2340

gacgaccggg tcatcgtggc cggatcttgc ggcccctcgg cttgaacgaa ttgttagaca 2400

ttatttgccg actaccttgg tgatctcgcc tttcacgtag tggacaaatt cttccaactg 2460

atctgcgcgc gaggccaagc gatcttcttc ttgtccaaga taagcctgtc tagcttcaag 2520

tatgacgggc tgatactggg ccggcaggcg ctccattgcc cagtcggcag cgacatcctt 2580

cggcgcgatt ttgccggtta ctgcgctgta ccaaatgcgg gacaacgtaa gcactacatt 2640

tcgctcatcg ccagcccagt cgggcggcga gttccatagc gttaaggttt catttagcgc 2700

ctcaaataga tcctgttcag gaaccggatc aaagagttcc tccgccgctg gacctaccaa 2760

ggcaacgcta tgttctcttg cttttgtcag caagatagcc agatcaatgt cgatcgtggc 2820

tggctcgaag atacctgcaa gaatgtcatt gcgctgccat tctccaaatt gcagttcgcg 2880

cttagctgga taacgccacg gaatgatgtc gtcgtgcaca acaatggtga cttctacagc 2940

gcggagaatc tcgctctctc caggggaagc cgaagtttcc aaaaggtcgt tgatcaaagc 3000

tcgccgcgtt gtttcatcaa gccttacggt caccgtaacc agcaaatcaa tatcactgtg 3060

tggcttcagg ccgccatcca ctgcggagcc gtacaaatgt acggccagca acgtcggttc 3120

gagatggcgc tcgatgacgc caactacctc tgatagttga gtcgatactt cggcgatcac 3180

cgcttccctc atactcttcc tttttcaata ttattgaagc atttatcagg gttattgtct 3240

catgagcgga tacatatttg aatgtattta gaaaaataaa caaatagcta gctcactcgg 3300

tcgctacgct ccgggcgtga gactgcggcg ggcgctgcgg acacatacaa agttacccac 3360

agattccgtg gataagcagg ggactaacat gtgaggcaaa acagcagggc cgcgccggtg 3420

gcgtttttcc ataggctccg ccctcctgcc agagttcaca taaacagacg cttttccggt 3480
gcatctgtgg gagccgtgag gctcaaccat gaatctgaca gtacgggcga aacccgacag 3540
gacttaaaga tccccaccgt ttccggcggg tcgctccctc ttgcgctctc ctgttccgac 3600
cctgccgttt accggatacc tgttccgcct ttctccctta cgggaagtgt ggcgctttct 3660
catagctcac acactggtat ctcggctcgg tgtaggtcgt tcgctccaag ctgggctgta 3720
agcaagaact ccccgttcag cccgactgct gcgccttatc cggtaactgt tcacttgagt 3780
ccaacccgga aagcacggt aaaacgccac tggcagcagc cattggtaac tgggagttcg 3840
cagaggattt gtttagctaa acacgcggtt gctcttgaag tgtgcgccaa agtccggcta 3900
cactggaagg acagatttgg ttgctgtgct ctgcgaaagc cagttaccac ggttaagcag 3960
ttccccaact gacttaacct tcgatcaaac cacctcccca ggtggttttt tcgtttacag 4020
ggcaaaagat tacgcgcaga aaaaaaggat ctcaagaaga tcctttgatc ttttctactg 4080
aaccgctcta gatttcagtg caatttatct cttcaaatgt agcacctgaa gtcagcccca 4140
tacgatataa gttgtaattc tcatgttagt catgccccgc gcccaccgga aggagctgac 4200
tgggttgaag gctctcaagg gcatcggtcg agatcccggt gcctaatgag tgagctaact 4260
tacattaatt gcgttgcgct cactgcccgc tttccagtcg ggaaacctgt cgtgccagct 4320
gcattaatga atcggccaac gcgcggggag aggcggtttg cgtattgggc gccagggtgg 4380
tttttctttt caccagtgag acgggcaaca gctgattgcc cttcaccgcc tggccctgag 4440
agagttgcag caagcggtcc acgctggttt gccccagcag gcgaaaatcc tgtttgatgg 4500
tggttaacgg cgggatataa catgagctgt cttcggtatc gtcgtatccc actaccgaga 4560
tgtccgcacc aacgcgcagc ccggactcgg taatggcgcg cattgcgccc agcgccatct 4620
gatcgttggc aaccagcatc gcagtgggaa cgatgccctc attcagcatt tgcatggttt 4680
gttgaaaacc ggacatggca ctccagtcgc cttcccgttc cgctatcggc tgaatttgat 4740
tgcgagtgag atatttatgc cagccagcca gacgcagacg cgccgagaca gaacttaatg 4800
ggcccgctaa cagcgcgatt tgctggtgac ccaatgcgac cagatgctcc acgcccagtc 4860
gcgtaccgtc ttcatgggag aaaataatac tgttgatggg tgtctggtca gagacatcaa 4920
gaaataacgc cggaacatta gtgcaggcag cttccacagc aatggcatcc tggtcatcca 4980
gcggatagtt aatgatcagc ccactgacgc gttgcgcgag aagattgtgc accgccgctt 5040
tacaggcttc gacgccgctt cgttctacca tcgacaccac cacgctggca cccagttgat 5100
cggcgcgaga tttaatcgcc gcgacaattt gcgacggcgc gtgcagggcc agactggagg 5160
tggcaacgcc aatcagcaac gactgtttgc ccgccagttg ttgtgccacg cggttgggaa 5220
tgtaattcag ctccgccatc gccgcttcca ctttttcccg cgttttcgca gaaacgtggc 5280

tggcctggtt caccacgcgg gaaacggtct gataagagac accggcatac tctgcgacat 5340

cgtataacgt tactggtttc acattcacca ccctgaattg actctcttcc gggcgctatc 5400

atgccatacc gcgaaaggtt ttgcgccatt cgatggtgtc cgggatctcg acgctctccc 5460

ttatgcgact cctgcattag gaaattaata cgactcacta ta 5502

<210> 231
<211> 10460
<212> DNA
<213> Artificial sequence

<220>
<223> pKZ4 plasmid

<400> 231
tcgcgacgcg aggctggatg gccttcccca ttatgattct tctcgcttcc ggcggcatcg 60

ggatgcccgc gttgcaggcc atgctgtcca ggcaggtaga tgacgaccat cagggacagc 120

ttcaaggatc gctcgcggct cttaccagcc taacttcgat cactggaccg ctgatcgtca 180

cggcgattta tgccgcctcg gcgagcacat ggaacgggtt ggcatggatt gtaggcgccg 240

ccctatacct tgtctgcctc cccgcgttgc gtcgcggtgc atggagccgg gccacctcga 300

cctgaatgga agccggcggc acctcgctaa cggattcacc actccaagaa ttggagccaa 360

tcaattcttg cggagaactg tgaatgcgca aaccaaccct tggcagaaca tatccatcgc 420

gtccgccatc tccagcagcc gcacgcggcg catctcgggc agcgttgggt cctggccacg 480

ggtgcgcatg atcgtgctcc tgtcgttgag gacccggcta ggctggcggg gttgccttac 540

tggttagcag aatgaatcac cgatacgcga gcgaacgtga agcgactgct gctgcaaaac 600

gtctgcgacc tgagcaacaa catgaatggt cttcggtttc cgtgtttcgt aaagtctgga 660

aacgcggaag tcagcgccct gcaccattat gttccggatc tgcatcgcag gatgctgctg 720

gctaccctgt ggaacaccta catctgtatt aacgaagcgc tggcattgac cctgagtgat 780

ttttctctgg tcccgccgca tccataccgc cagttgttta ccctcacaac gttccagtaa 840

ccgggcatgt tcatcatcag taacccgtat cgtgagcatc ctctctcgtt tcatcggtat 900

cattaccccc atgaacagaa atcccccctta cacggaggca tcagtgacca aacaggaaaa 960

aaccgccctt aacatggccc gctttatcag aagccagaca ttaacgcttc tggagaaact 1020

caacgagctg gacgcggatg aacaggcaga catctgtgaa tcgcttcacg accacgctga 1080

tgagctttac cgcagctgcc tcgcgcgttt cggtgatgac ggtgaaaacc tctgacacat 1140

gcagctcccg gagacggtca cagcttgtct gtaagcggat gccgggagca gacaagcccg 1200

tcagggcgcg tcagcgggtg ttggcgggtg tcggggcgca gccatgaccc agtcacgtag 1260

cgatagcgga gtgtatactg gcttaactat gcggcatcag agcagattgt actgagagtg 1320

caccatatgc ggtgtgaaat accgcacaga tgcgtaagga gaaaataccg catcaggcgc 1380

tcttccgctt cctcgctcac tgactcgctg cgctcggtcg ttcggctgcg gcgagcggta 1440
tcagctcact caaaggcggt aatacggtta tccacagaat caggggataa cgcaggaaag 1500
aacatgtgag caaaaggcca gcaaaaggcc aggaaccgta aaaaggccgc gttgctggcg 1560
tttttccata ggctccgccc ccctgacgag catcacaaaa atcgacgctc aagtcagagg 1620
tggcgaaacc cgacaggact ataaagatac caggcgtttc cccctggaag ctccctcgtg 1680
cgctctcctg ttccgaccct gccgcttacc ggatacctgt ccgcctttct cccttcggga 1740
agcgtggcgc tttctcatag ctcacgctgt aggtatctca gttcggtgta ggtcgttcgc 1800
tccaagctgg gctgtgtgca cgaacccccc gttcagcccg accgctgcgc cttatccggt 1860
aactatcgtc ttgagtccaa cccggtaaga cacgacttat cgccactggc agcagccact 1920
ggtaacagga ttagcagagc gaggtatgta ggcggtgcta cagagttctt gaagtggtgg 1980
cctaactacg gctacactag aaggacagta tttggtatct gcgctctgct gaagccagtt 2040
accttcggaa aaagagttgg tagctcttga tccggcaaac aaaccaccgc tggtagcggt 2100
ggtttttttg tttgcaagca gcagattacg cgcagaaaaa aaggatctca agaagatcct 2160
ttgatctttt ctacggggtc tgacgctcag tggaacgaaa actcacgtta agggattttg 2220
gtcatgagat tatcaaaaag gatcttcacc tagatccttt taaattaaaa atgaagtttt 2280
aaatcaatct aaagtatata tgagtaaact tggtctgaca gttaccaatg cttaatcagt 2340
gaggcaccta tctcagcgat ctgtctattt cgttcatcca tagttgcctg actccccgtc 2400
gtgtagataa ctacgatacg ggagggctta ccatctggcc ccagtgctgc aatgataccg 2460
cgagacccac gctcaccggc tccagattta tcagcaataa accagccagc cggaagggcc 2520
gagcgcagaa gtggtcctgc aactttatcc gcctccatcc agtctattaa ttgttgccgg 2580
gaagctagag taagtagttc gccagttaat agtttgcgca acgttgttgc cattgctgca 2640
ggcatcgtgg tgtcacgctc gtcgtttggt atggcttcat tcagctccgg ttcccaacga 2700
tcaaggcgag ttacatgatc ccccatgttg tgcaaaaaag cggttagctc cttcggtcct 2760
ccgatcgttg tcagaagtaa gttggccgca gtgttatcac tcatggttat ggcagcactg 2820
cataattctc ttactgtcat gccatccgta agatgctttt ctgtgactgg tgagtactca 2880
accaagtcat tctgagaata gtgtatgcgg cgaccgagtt gctcttgccc ggcgtcaaca 2940
cgggataata ccgcgccaca tagcagaact ttaaaagtgc tcatcattgg aaaacgttct 3000
tcggggcgaa aactctcaag gatcttaccg ctgttgagat ccagttcgat gtaacccact 3060
cgtgcaccca actgatcttc agcatctttt actttcacca gcgtttctgg gtgagcaaaa 3120
acaggaaggc aaaatgccgc aaaaaaggga ataagggcga cacggaaatg ttgaatactc 3180
atactcttcc tttttcaata ttattgaagc atttatcagg gttattgtct catgagcgga 3240

tacatatttg aatgtattta gaaaaataaa caaatagggg ttccgcgcac atttccccga 3300

aaagtgccac ctgacgtctt aattaatcag gagagcgttc accgacaaac aacagataaa 3360

acgaaaggcc cagtctttcg actgagcctt tcgttttatt tgatgcctgg cagttcccta 3420

ctctcgcatg gggagacccc acactaccat cggcgctacg gcgtttcact tctgagttcg 3480

gcatggggtc aggtgggacc accgcgctac tgccgccagg caaattctgt tttatcagac 3540

cgcttctgcg ttctgattta atctgtatca ggctgaaaat cttctctcat ccgccaaaac 3600

agccaagctg gagaccgttt aaactcaatg atgatgatga tgatggtcga cggcgctatt 3660

cagatcctct tctgagatga gtttttgttc gggcccaagc ttcgaattct cagatatgca 3720

aggcgtggcc caacgcgcgt agggcggctt cttgcaccgc ttcacccaac gtggggtggg 3780

catgaatggt gccggccaca tcttccaggc acgcgcccat ctccagcgat tgggcaaacg 3840

cggtggacag ctcggagacc gccacgccaa ccgcctgcca acccacgatc aggtggttgt 3900

cacggcgcgc caccacccgc acgaaaccgc ttttcgactc caggctcatg gcccggccat 3960

tggcggcaaa cgggaactgc gcgacgatgc agtccagggc ctgctggctg gcttgttccg 4020

gggtcttgcc gaccaccacc acttccgggt cggtaaagca cacggcggca atcgctgccg 4080

gctcgaagcg tcgggccttg ccggcgatga tttcggcgac catctcgcct tgggccatgg 4140

cccggtgcgc cagcatcggt tcgccagcga cgtcgccaat ggcccagacg ttgtgcatgc 4200

tggtatgaca gcgctcgtcg atggcaatgg cggtgccgtt catcttcagg tccaggcatt 4260

ccaggttgaa gcccttggtg cgtggccggc ggcccacggc caccagtacc tgatcggctt 4320

caagacgcag ttgcccaccc ttgccgtcgc tggccagcag gcagccattt tcgtagccct 4380

cgacgctgtg gcccaggtgc aacgcgatgc ccagtttctt cagcgactcg gccaccgggg 4440

cggtcaattc gctgtcgtag gtcggcagga tgcgttcgcg cgcttccacc acactcacct 4500

gtgcacccag cttgcgatag gcaatgccca gctccaggcc gatatagcca ccgccgacca 4560

ccaccaggtg ttgcggcagg gctttcggcg ccagggcttc ggtcgaggaa atcaccgggc 4620

cacccagcgg cagcatcggc agttcgacac tggtggaacc ggtcgccagc aacagatgct 4680

cgcactggat acgctggcca tcgacctcga cctgcttgcc gtccagtacc ttggcccagc 4740

catgcaccac tttcaccccg tgctttttca gcaaggcggc aacaccggtg gtcagacggt 4800

cgacaatgcc gtccttccag gtgacgctct ggccgatgtc caggcgcggc gaagccacgc 4860

tgatgcccag cggcgagggt tcggtaaagc gcgaggcttg gtgaaactgc tcggccacgt 4920

ggatcagcgc cttggacggg atgcagccga tgttcaggca ggtgccgccc agtgcctggc 4980

cttccaccag tacggtagga atgcccagtt gcccggcgcg gatggctgct acatagccgc 5040

cagggccgcc gccgatgatc aacagggtag tctggataat ctgttgcatg ctcactccac 5100

gaacaggcag gcgggttgtt cgagcaggcc acgcacggcc tggatgaaca gggcggcgtc 5160

catgccatcg accacgcggt ggtcgaacga gctggacagg ttcatcatct tgcgcacgac 5220

gatctggcca tcaatcacca ccggtcgttc gaccatgcgg ttgaccccga cgattgccac 5280

ttccggggtg ttgaccaccg gcgtgctgac aatgccaccc aaggcgccga ggctggtcag 5340

ggtgatggtc gagccggaca gctcctcgcg gctggccttg ttgttacgtg cagcgttggc 5400

caggcgcgaa atctcgccgg cattggccca caggctgccc gcttcggcgt ggcgcagcac 5460

gggtaccatc aggccgttgt caccctgggt ggcaatgccc acatgcaccg cgccatggcg 5520

ggtgatgatc tgcgcttcgt cgtcgtaggt cgcgttgatc tgcgggaagt cacgcagcgc 5580

cacgacgagg gcgcgcacca ggaatggcag caaggtcagt ttgccgcggc tgtcgccgtg 5640

cttgctgttg agttgctggc gcagggcttc cagggcggtg acgtcgattt cctcgacata 5700

actgaagtgc gcgacccggc gtttggcgtc ctgcatgcgc tgggcgatct tgcggcgcag 5760

gccgatcacc ggcacctgct cgctgtcggt gcgcttggca taaccatcag gtgcttgccc 5820

ggcattgctt tgcggcttgc tcatgaaggc gtcgaggtct tcgtgcagaa tgcgcccggc 5880

cgggccgcta ccatgcacat aacgcagttc gataccggcg tccagggcgc gtttgcgcac 5940

ggccggcgag gccagcggct tgtcgcccgg ctggcgcggc acgatgggcg cagcttcgtg 6000

gttggcgggc gcctggtaca cggcgggttt tacgtctttc tgcggttccg gcttggctgc 6060

aatcggggcg gccggggcct ctaccggttt tggctgaggc acgtccacat ggttgccgct 6120

gccttccact tcgatgcgga tcagttcgct accgaccgcc atcacttccc cgggctggcc 6180

acccagggcc aacaccttgc cgctgaccgg cgaggggatt tccacggtgg ccttgtcggt 6240

catgacgtcg gccaccacct ggtcctcggc gatgatgtcg ccgaccttga cgaaccattc 6300

caccaactcg acctgcgcga tgccttcgcc aatgtccggc atcttgatga cgtgcgtgcc 6360

cattcagacc tccatgacct tttcagtgc cgcacctacc cgcgaagggc cggggaagta 6420

agcccattcc tgtgcgtgag ggtagggggt gtcccagccg gtgacgcgct cgatcggcgc 6480

ctccaggtgg tggaagcagt gctcctgcac cagcgacacc agctcggcac cgaagccgca 6540

ggtgcgggtg gcctcgtgca ccaccacgca acggccagtc tttttcaccg actcgacgat 6600

agtgtccagg tccagcggcc acaggctgcg caggtcgatc acttcggcat cgacgccgct 6660

ttcttcggcg gccacctggg ccacgtacac cgtggtgccg taagtcagta cggtcacgtc 6720

attgccaggg cgggtaatgg cggccttgtc cagcggtacg gtgtaatagc cgtcgggcac 6780

ggcgctgtgc gggtgcttcg accatggggt tacagggcgg tcgtggtggc catcgaacgg 6840

gccgttgtac agacgtttgg gctccaggaa gattaccggg tcgtcgcatt cgatcgaggc 6900

aatcagcagg cctttggcgt cataagggtt ggacggcatc acggtgcgca ggccgcagac 6960

ctgggtgaac atcgcttccg ggctctggct gtgagtctgg ccgccataga tgccgccgcc 7020

gcaaggcatg cgcagggtca gcggggcaat gaactcgccg gccgaccggt aacgcaggcg 7080

ggccagctcg gagacgatct ggtcggaggc cgggtagaag tagtcggcga actggatctc 7140

caccaccggg cgcaggccat aggcgcccat gcctacggcg gtaccgacga tgccgctctc 7200

ggagatgggc gcgtcgaaca cgcgcgattt gccgtacttg ttctgcaggc cttcggtgca 7260

gcggaacacg ccgccgaagt aaccgacgtc ctggccgtac accaccacat tgtcgtcgcg 7320

ctcaagcatg acatccatgg ccgagcgcag ggcctggatc atggtcatgg tagtggtggc 7380

catggcggtt tccgggttga tgctgttgtt gtggtcgttc atctcaaacc cccagttcct 7440

ggcgttgacg gcgcaggtgt tcgggcatct ccttgtacac atcctcgaac atcgaggcgg 7500

cgctcgggat gtgcccgtta gccagggtgc cgtactgctc ggcttctttc tgtgcggcaa 7560

tcaccgcagc ttcgagctcg gccgtgacgg cttggtgttc ttcttcggac cagtggccga 7620

tcttgatcag gtgctgcttc aggcgggcga tcgggtcacc cagcgggaag tggctccagt 7680

catcggcagg gcggtacttg gaggggtcgt ccgacgtcga gtgcgggccg gcacggtagg 7740

tgacccactc gatcaggctt gggcccaggc cgcggcgggc gcgctcggca gcccagcgcg 7800

aggcggcgta cacggcgacg aagtcgttgc cgtcaacccg cagcgaggca atgccgcagc 7860

ccacgccacg gccggcgaag gtggtcgact cgccaccggc gatggcctgg aaggtagaaa 7920

tcgcccactg gttgttgacc acattgagga tcaccggggc gcggtaaacg tgggcaaagg 7980

tgagggcggt gtggaagtcc gactcggcgg tggctccgtc accgatccac gccgaagcaa 8040

tcttggtatc gcccttgatc gccgaggcca tggcccagcc gactgcctgc acgaactggg 8100

tcgccaggtt gccgctgatg gtgaagaagc cggcttcgcg caccgagtac atgatcggca 8160

actggcggcc cttgaggggg tcgcgctcgt tggacagcag ttggcagatc atctcgacca 8220

gcgatacgtc gcgggccatc aggatgcttt gctggcggta ggtcgggaag cacatgtcgg 8280

tgcggttcag cgccagcgcc tggccactgc cgatggcttc ttcgcccagg ctttgcatgt 8340

agaaggacat cttcttctgg cgctgggcaa ccaccatgcg gctgtcgaag atccgcgtct 8400

tgagcatggc gcgcatgcct tgacgaagga tctgtgggtc gatgtcttcg gcccaggggc 8460

cttgcgcatc accttgctcg tcgagcacgc ggaccaggct gtaggacagg tcggcagtgt 8520

cggcagcatc gacatcgatc gcgggtttac gggcttgacc tgcatcgttg aggcgcaggt 8580

aggaaaaatc ggtctggcag cctggccggc cggtgggctc gggcacatgc aaacgcaggg 8640

gggcgtactc gttcatggat ccatggttta ttcctcctta tttaatcgat acattaatat 8700

atacctcttt aatttttaat aataaagtta atcgataatt ccggtcgagt gcccacacag 8760

attgtctgat aaattgttaa agagcagtgc cgcttcgctt tttctcagcg gcgctgtttc 8820

ctgtgtgaaa ttgttatccg ctcacaattc cacacattat acgagccgga tgattaattg 8880

tcaacagctc atttcagaat atttgccaga accgttatga tgtcggcgca aaaaacatta 8940

tccagaacgg gagtgcgcct tgagcgacac gaattatgca gtgatttacg acctgcacag 9000
ccataccaca gcttccgatg gctgcctgac gccagaagca ttggtgcacc gtgcagtcga 9060
tgataagctg tcaaaccaga tcaattcgcg ctaactcaca ttaattgcgt tgcgctcact 9120
gcccgctttc cagtcgggaa acctgtcgtg ccagctgcat taatgaatcg gccaacgcgc 9180
ggggagaggc ggtttgcgta ttgggcgcca gggtggtttt tcttttcacc agtgagacgg 9240
gcaacagctg attgcccttc accgcctggc cctgagagag ttgcagcaag cggtccacgc 9300
tggtttgccc cagcaggcga aaatcctgtt tgatggtggt tgacggcggg atataacatg 9360
agctgtcttc ggtatcgtcg tatcccacta ccgagatatc cgcaccaacg cgcagcccgg 9420
actcggtaat ggcgcgcatt gcgcccagcg ccatctgatc gttggcaacc agcatcgcag 9480
tgggaacgat gccctcattc agcatttgca tggtttgttg aaaaccggac atggcactcc 9540
agtcgccttc ccgttccgct atcggctgaa tttgattgcg agtgagatat ttatgccagc 9600
cagccagacg cagacgcgcc gagacagaac ttaatgggcc cgctaacagc gcgatttgct 9660
ggtgacccaa tgcgaccaga tgctccacgc ccagtcgcgt accgtcttca tgggagaaaa 9720
taatactgtt gatgggtgtc tggtcagaga catcaagaaa taacgccgga acattagtgc 9780
aggcagcttc cacagcaatg gcatcctggt catccagcgg atagttaatg atcagcccac 9840
tgacgcgttg cgcgagaaga ttgtgcaccg ccgctttaca ggcttcgacg ccgcttcgtt 9900
ctaccatcga caccaccacg ctggcaccca gttgatcggc gcgagattta atcgccgcga 9960
caatttgcga cggcgcgtgc agggccagac tggaggtggc aacgccaatc agcaacgact 10020
gtttgcccgc cagttgttgt gccacgcggt tgggaatgta attcagctcc gccatcgccg 10080
cttccacttt ttcccgcgtt ttcgcagaaa cgtggctggc ctggttcacc acgcgggaaa 10140
cggtctgata agagacaccg gcatactctg cgacatcgta taacgttact ggtttcacat 10200
tcaccaccct gaattgactc tcttccgggc gctatcatgc cataccgcga aaggttttgc 10260
accattcgat ggtgtcaacg taaatgcatg ccgcttcgcc ttcgcgcgcg aattgatctg 10320
ctgcctcgcg cgtttcggtg atgacggtga aaacctctga cacatgcagc tcccggagac 10380
ggtcacagct tgtctgtaag cggatgccgg gagcagacaa gcccgtcagg gcgcgtcagc 10440
gggtgttggc ggggccggcc 10460

<210> 232
<211> 4732
<212> DNA
<213> Listeria monocytogenes

<220>
<221> misc_feature
<223> BKD operon

<400> 232
gtggcaacag aatatgatgt cgttattctt ggtggaggaa ctggcggtta cgtcgcagcc 60

attcaagcag ctaagaatgg ccaaaaagta gccgtcgttg aaaaagggaa agttggtgga 120

acgtgtcttc accgtggatg tattccaacg aaagcgttat tacgttcagc agaagttctg 180

caaacagtaa aaaaagcaag tgaatttggt atttctgtag aaggaactgc tggaatcaat 240

tttttacaag cacaagaaag aaaacaacaa atagtcgatc aattagaaaa aggaatccat 300

caattattta aacaaggtaa aattgacttg tttgtaggga cgggaaccat tttaggacca 360

tctatttttt caccaacagc tggaacggtt tcagtcgaat ttgaagatgg ctctgaaaat 420

gaaatgctca ttcctaaaaa cttaattatc gcgacaggct ccaaaccgcg tacattaaat 480

ggtttaagca ttgatgaaga aaatgtttta tcatctgacg gcgcgctaaa cctagaaact 540

ttaccaaaat caattattat cgttggcggt ggagttatcg ggatggaatg ggcttcaatg 600

atgcatgatt ttggcgtaga agttaccgtg ctagaatatg cagatcgaat tttaccaaca 660

gaagataaag aagtagccaa agaattagca agactttata aaaagaaaaa attaaacatg 720

catacatctg ctgaagttca agcagctagt tataaaaaaa cggatactgg tgtggaaatt 780

aaagcaatta ttaaaggtga agagcaaact ttcacagcag acaaaattct cgtttctgtc 840

ggtcgttcag ctaacacaga aaatatcggt ttacaaaaca cggatatcgc gaccgaaaac 900

ggctttatcc aagtaaatga tttttatcaa acaaaagaaa gccacatcta cgcgattggt 960

gattgcattc caacaattca gcttgcgcac gttgcgatgg aagaaggaac gattgcagcc 1020

aaccatattg ccggaaaagt ggctgaaaaa cttgactacg acttagttcc ccgctgtatc 1080

tatacttcta cagaaatcgc aagtgtcggt atcacagaag aacaagcaaa agaacgtggt 1140

tatgaagtga aaaaaggcaa atttttcttc cgtggcatcg ggaaagcgct cgtttacgga 1200

gagtcagatg gcttcattaa acttattgca gataaaaaaa cagacgatat cctaggcgtt 1260

agcatgattg gaccgcacgt gacggacatg attagcgaag ccgctttagc ccaagtttta 1320

aatgcaacgc cgtgggaagt gggtaacacc attcacccgc acccaacttt atcagaaagt 1380

tttagagaag ctgcccttgc tgtggatggc aatgcaattc acggttaata cagctttaaa 1440

ggaggaacaa aaatgacttt aaaagaagca ggtttaacag aagataaatt aattaaaatg 1500

tatgaaacga tgctaatggc aagaagactg gatgaacgta tgtggttgct gaaccgttct 1560

gggaaaattc cttttactat ttctggacaa ggacaagaaa cggcacaaat tggcgctgcg 1620

tttgcctttg atttagataa agattatgca ttaccatatt accgtgattt agcagtggtt 1680

ctagcattcg ggatgacagc gaaagatatt atgttatccg cgttcgctaa agcggaagat 1740

ccaaactctg gcggacgaca aatgccagcc cactttggtc aaaaagaaaa ccgcattgtc 1800

actcaaagtt cgcctgtaac tacccagttc ccgcatgcaa cagggattgg acttgcagcg 1860

aaaatggctg gtgatgagat tgcgatatat gcttcaaccg gtgaaggatc ttctaaccaa 1920
ggcgatttcc acgaaggaat caactttgca tctgtacata agttgccagt tgttttcgtg 1980
attcacaata accaatatgc tatttccgtt ccagcatcga aacaatatgc cgctgaaaaa 2040
ctatccgacc gagcaattgg ttacggcatc cctggtgaac gtgtagatgg aacgaatatg 2100
ggagaagtat acgcggcatt taaacgtgca gcagatcgtg caagaaacgg cgagggacct 2160
actttaattg aaacagtttc ttaccgcttc acaccacact cctccgatga tgacgatagt 2220
agttatcgtt ccagagaaga agttgacgaa gcaaaaggaa aagatccact gaaaattttc 2280
caagcagaat tactggaaga aggttactta acagaagaaa aaatcgctga aatcgaaaaa 2340
aacattgcga aagaagttaa cgaagcaacc gattatgcgg aaagtgcagc atacgctgaa 2400
ccagaatcat ctttacttta tgtatatgat gaagaagcga atagctgatt aggagggaat 2460
ttgaatgcca gtcatttcat atattgatgc aataaccatg gcgcttaaag aagaaatgga 2520
gcgcgacgat aaagtattta ttttaggaga agatgtaggt aaaaaaggtg gcgtttttaa 2580
agcgactgct ggtttatatg atgagtttgg tgaagaccga gttcttgata caccacttgc 2640
tgaatcggct attgccggag taggaattgg agcggcgatg tatggctatc gtccagttgc 2700
agaaatgcag tttgcggact ttattatgcc tgctgtcaac caaattattt cagaagcttc 2760
cagaattcgc taccgttcta ataacgattg gtcttgccca atggttattc gcgcaccttt 2820
tggcggtggg gtacacggag cactttacca ttcacaatct gttgaaaaag tattctttgg 2880
acaaccaggt ttgaaaattg ttgttccatc ctcaccatat gatgcgaagg gacttttaaa 2940
agcagcgatt cgtgacaacg atccagtact tttctttgag cataaacgcg cctatcgctt 3000
gctgaaaggc gaagtaccag aaacagatta tatcgtacca atcggtgaag caaatgtcgt 3060
tcgtgaaggc gatgatatta cagttattac ttatggactt gctgttcaat ttgctcaaca 3120
agctgcggaa cgtttagccg ctgaaggtgt cgaagcgcat attctggatt tacgtacgat 3180
ttatccatta gaccaagaag cgattatcga agcaacgaaa aaaactggta aagtacttct 3240
tgttacggaa gataataaac aaggaagtat tattagtgaa gtggcggcaa tcatttcgga 3300
acattgtcta tttgatttag atgcaccgat tgctagactc gcaggaccag ataccccagc 3360
gatgcctttt gctccaacga tggaaaaaca ctttatgatt aatccagata aagtggcgga 3420
tgcaatgaaa gaattagcgg aattttagac ccgttttaaa ttaaaggagt gaagacccgt 3480
ggcagttgaa aaaatcacca tgcccaaatt aggggaaagt gtaacagaag gaacgattag 3540
ttcatggtta gttaaaccag gcgatacagt tgaaaagtat gatgctatcg cagaagtact 3600
tactgacaaa gtaacagcag aaatcccatc ttcttttagc ggaacaatta aagaaatttt 3660
agccgaagaa gatgaaacac tagaagtagg cgaagttatt tgtacaattg aaacagcaga 3720

cgcaggaagt tcggaacctg tagctgaagt agaacaaaca gaaacaaaag cgccagaaaa 3780

acaagaaaca aaacaagtga aactagcaga tgcaccagct agtggaagat tttctccagc 3840

ggtactacga attgctggtg aaaacaatat cgatttatcc actgtagaag gcacaggtaa 3900

aggtggccga attacaagaa aagacttact tcaagtcatt gaaaacggac cagtagctac 3960

taaagcagaa gtgcaaagtc aaagcgcacc gcaagaaaaa actgctacac cagctcctgt 4020

acgttcagca gcaggtgaca gagaaatccc aatcaatggc gtaagaaaag ccattgctaa 4080

acatatgagc gtgagcaaac aagaaattcc gcatgcttgg atgatggtgg aagtggatgc 4140

aacaggtctt gttcgctatc gtaatgcagt taaagacagc tttaaaaaag aagaaggtta 4200

ttcattaact tatttcgcct ttttcatcaa agccgttgca caagcattga aagaattccc 4260

gcaacttaac agcacatggg caggcgataa aattattgag cacgcgaata tcaatatttc 4320

gattgcgatt gcagctggtg atttattgta cgttccagtt attaaaaatg cggacgaaaa 4380

atccattaaa ggcattgctc gcgaaattag cgaacttgct ggaaaagcgc gtaatggtaa 4440

actaagccaa gccgatatgg aaggtggcac tttcacagtg aatagtactg gttcatttgg 4500

ctctgttcaa tcaatgggga ttattaatca cccacaagca gcaattcttc aagtggaatc 4560

cattgttaaa cgtcctgtca ttattgatga tatgattgct gtacgagata tggtgaacct 4620

atgtctttcc atcgatcacc gtattttaga tggcttacta gcaggtaaat tcttacaagc 4680

aattaaagcc aatgtcgaaa agatttccaa agaaaataca gcattgtatt aa 4732

<210> 233
<211> 5733
<212> DNA
<213> Artificial sequence

<220>
<223> pACYC-PTrc vector

<400> 233
actcaccagt cacagaaaag catcttacgg atggcatgac agtaagagaa ttatgcagtg 60

ctgccataac catgagtgat aacactgcgg ccaacttact tctgacaacg atcggaggac 120

cgaaggagct aaccgctttt ttgcacaaca tgggggatca tgtaactcgc cttgatcgtt 180

gggaaccgga gctgaatgaa gccataccaa acgacgagcg tgacaccacg atgcctgcag 240

caatggcaac aacgttgcgc aaactattaa ctggcgaact acttactcta gcttcccggc 300

aacaattaat agactggatg gaggcggata aagttgcagg accacttctg cgctcggccc 360

ttccggctgg ctggtttatt gctgataaat ctggagccgg tgagcgtggg tctcgcggta 420

tcattgcagc actggggcca gatggtaagc cctcccgtat cgtagttatc tacacgacgg 480

ggagtcaggc aactatggat gaacgaaata gacagatcgc tgagataggt gcctcactga 540

ttaagcattg gtaactgtca gaccaagttt actcatatat actttagatt gatttaaaac 600

ttcattttta atttaaaagg atctaggtga agatcctttt tgataatctc atgaccaaaa 660

tcccttaacg tgagttttcg ttccactgag cgtcagaccc cttaataaga tgatcttctt 720

gagatcgttt tggtctgcgc gtaatctctt gctctgaaaa cgaaaaaacc gccttgcagg 780

gcggtttttc gaaggttctc tgagctacca actctttgaa ccgaggtaac tggcttggag 840

gagcgcagtc accaaaactt gtcctttcag tttagcctta accggcgcat gacttcaaga 900

ctaactcctc taaatcaatt accagtggct gctgccagtg gtgcttttgc atgtctttcc 960

gggttggact caagacgata gttaccggat aaggcgcagc ggtcggactg aacggggggt 1020

tcgtgcatac agtccagctt ggagcgaact gcctacccgg aactgagtgt caggcgtgga 1080

atgagacaaa cgcggccata acagcggaat gacaccggta aaccgaaagg caggaacagg 1140

agagcgcacg agggagccgc caggggggaaa cgcctggtat ctttatagtc ctgtcgggtt 1200

tcgccaccac tgatttgagc gtcagatttc gtgatgcttg tcaggggggc ggagcctatg 1260

gaaaaacggc tttgccgcgg ccctctcact tccctgttaa gtatcttcct ggcatcttcc 1320

aggaaatctc cgccccgttc gtaagccatt tccgctcgcc gcagtcgaac gaccgagcgt 1380

agcgagtcag tgagcgagga agcggaatat atcctgtatc acatattctg ctgacgcacc 1440

ggtgcagcct tttttctcct gccacatgaa gcacttcact gacaccctca tcagtgccaa 1500

catagtaagc cagtatacac tccgctagcg ctgaggtctg cctcgtgaag aaggtgttgc 1560

tgactcatac caggcctgaa tcgccccatc atccagccag aaagtgaggg agccacggtt 1620

gatgagagct ttgttgtagg tggaccagtt ggtgattttg aacttttgct ttgccacgga 1680

acggtctgcg ttgtcgggaa gatgcgtgat ctgatccttc aactcagcaa aagttcgatt 1740

tattcaacaa agccacgttg tgtctcaaaa tctctgatgt tacattgcac aagataaaaa 1800

tatatcatca tgaacaataa aactgtctgc ttacataaac agtaatacaa ggggtgttat 1860

gagccatatt caacgggaaa cgtcttgctc gaggccgcga ttaaattcca acatggatgc 1920

tgatttatat gggtataaat gggctcgcga taatgtcggg caatcaggtg cgacaatcta 1980

tcgattgtat gggaagcccg atgcgccaga gttgtttctg aaacatggca aaggtagcgt 2040

tgccaatgat gttacagatg agatggtcag actaaactgg ctgacggaat ttatgcctct 2100

tccgaccatc aagcatttta tccgtactcc tgatgatgca tggttactca ccactgcgat 2160

ccccgggaaa acagcattcc aggtattaga agaatatcct gattcaggtg aaaatattgt 2220

tgatgcgctg gcagtgttcc tgcgccggtt gcattcgatt cctgtttgta attgtccttt 2280

taacagcgat cgcgtatttc gtctcgctca ggcgcaatca cgaatgaata acggtttggt 2340

tgatgcgagt gattttgatg acgagcgtaa tggctggcct gttgaacaag tctggaaaga 2400

aatgcataag cttttgccat tctcaccgga ttcagtcgtc actcatggtg atttctcact 2460

tgataacctt atttttgacg aggggaaatt aataggttgt attgatgttg gacgagtcgg 2520
aatcgcagac cgataccagg atcttgccat cctatggaac tgcctcggtg agttttctcc 2580
ttcattacag aaacggcttt ttcaaaaata tggtattgat aatcctgata tgaataaatt 2640
gcagtttcat ttgatgctcg atgagttttt ctaatcagaa ttggttaatt ggttgtaaca 2700
ctggcagagc attacgctga cttgacggga cggcggcttt gttgaataaa tcgaactttt 2760
gctgagttga aggatcagat cacgcatctt cccgacaacg cagaccgttc cgtggcaaag 2820
caaaagttca aaatcaccaa ctggtccacc tacaacaaag ctctcatcaa ccgtggctcc 2880
ctcactttct ggctggatga tggggcgatt caggcctggt atgagtcagc aacaccttct 2940
tcacgaggca gacctcagcg ctcaaagatg caggggtaaa agctaaccgc atctttaccg 3000
acaaggcatc cggcagttca acagatcggg aagggctgga tttgctgagg atgaaggtgg 3060
aggaaggtga tgtcattctg gtgaagaagc tcgaccgtct tggccgcgac accgccgaca 3120
tgatccaact gataaaagag tttgatgctc agggtgtagc ggttcggttt attgacgacg 3180
ggatcagtac cgacggtgat atggggcaaa tggtggtcac catcctgtcg gctgtggcac 3240
aggctgaacg ccggaggatc ctagagcgca cgaatgaggg ccgacaggaa gcaaagctga 3300
aaggaatcaa atttggccgc aggcgtaccg tggacaggaa cgtcgtgctg acgcttcatc 3360
agaagggcac tggtgcaacg gaaattgctc atcagctcag tattgcccgc tccacggttt 3420
ataaaattct tgaagacgaa agggcctcgt gatacgccta tttttatagg ttaatgtcat 3480
gataataatg gtttcttaga cgtcttaatt aatcaggaga gcgttcaccg acaaacaaca 3540
gataaaacga aaggcccagt ctttcgactg agcctttcgt tttatttgat gcctggcagt 3600
tccctactct cgcatgggga gaccccacac taccatcggc gctacggcgt ttcacttctg 3660
agttcggcat ggggtcaggt gggaccaccg cgctactgcc gccaggcaaa ttctgtttta 3720
tcagaccgct tctgcgttct gatttaatct gtatcaggct gaaaatcttc tctcatccgc 3780
caaaacagcc aagctggaga ccgtttaaac tcaatgatga tgatgatgat ggtcgacggc 3840
gctattcaga tcctcttctg agatgagttt ttgttcgggc ccaagcttcg aattcccata 3900
tggtaccagc tgcagatctc gagctcggat ccatggttta ttcctcctta tttaatcgat 3960
acattaatat atacctcttt aatttttaat aataaagtta atcgataatt ccggtcgagt 4020
gcccacacag attgtctgat aaattgttaa agagcagtgc cgcttcgctt tttctcagcg 4080
gcgctgtttc ctgtgtgaaa ttgttatccg ctcacaattc cacacattat acgagccgga 4140
tgattaattg tcaacagctc atttcagaat atttgccaga accgttatga tgtcggcgca 4200
aaaaacatta tccagaacgg gagtgcgcct tgagcgacac gaattatgca gtgatttacg 4260
acctgcacag ccataccaca gcttccgatg gctgcctgac gccagaagca ttggtgcacc 4320
gtgcagtcga tgataagctg tcaaaccaga tcaattcgcg ctaactcaca ttaattgcgt 4380

tgcgctcact gcccgctttc cagtcgggaa acctgtcgtg ccagctgcat taatgaatcg 4440

gccaacgcgc ggggagaggc ggtttgcgta ttgggcgcca gggtggtttt tcttttcacc 4500

agtgagacgg gcaacagctg attgcccttc accgcctggc cctgagagag ttgcagcaag 4560

cggtccacgc tggtttgccc cagcaggcga aaatcctgtt tgatggtggt tgacggcggg 4620

atataacatg agctgtcttc ggtatcgtcg tatcccacta ccgagatatc cgcaccaacg 4680

cgcagcccgg actcggtaat ggcgcgcatt gcgcccagcg ccatctgatc gttggcaacc 4740

agcatcgcag tgggaacgat gccctcattc agcatttgca tggtttgttg aaaaccggac 4800

atggcactcc agtcgccttc ccgttccgct atcggctgaa tttgattgcg agtgagatat 4860

ttatgccagc cagccagacg cagacgcgcc gagacagaac ttaatgggcc cgctaacagc 4920

gcgatttgct ggtgacccaa tgcgaccaga tgctccacgc ccagtcgcgt accgtcttca 4980

tgggagaaaa taatactgtt gatgggtgtc tggtcagaga catcaagaaa taacgccgga 5040

acattagtgc aggcagcttc cacagcaatg gcatcctggt catccagcgg atagttaatg 5100

atcagcccac tgacgcgttg cgcgagaaga ttgtgcaccg ccgctttaca ggcttcgacg 5160

ccgcttcgtt ctaccatcga caccaccacg ctggcaccca gttgatcggc gcgagattta 5220

atcgccgcga caatttgcga cggcgcgtgc agggccagac tggaggtggc aacgccaatc 5280

agcaacgact gtttgcccgc cagttgttgt gccacgcggt tgggaatgta attcagctcc 5340

gccatcgccg cttccacttt ttcccgcgtt ttcgcagaaa cgtggctggc ctggttcacc 5400

acgcgggaaa cggtctgata agagacaccg gcatactctg cgacatcgta taacgttact 5460

ggtttcacat tcaccaccct gaattgactc tcttccgggc gctatcatgc cataccgcga 5520

aaggttttgc accattcgat ggtgtcaacg taaatgcatg ccgcttcgcc ttcgcgcgcg 5580

aattgatctg ctgcctcgcg cgtttcggtg atgacggtga aaacctctga cacatgcagc 5640

tcccggagac ggtcacagct tgtctgtaag cggatgccgg gagcagacaa gcccgtcagg 5700

gcgcgtcagc gggtgttggc ggggccggcc tcg 5733

<210> 234
<211> 193
<212> DNA
<213> Artificial sequence

<220>
<223> PTrc promoter

<400> 234
ctgttgacaa ttaatcatcc ggctcgtata atgtgtggaa ttgtgagcgg ataacaattt 60

cacacaggaa acagcgccgc tgagaaaaag cgaagcggca ctgctcttta acaatttatc 120

agacaatctg tgtgggcact cgaccggaat tatcgattaa ctttattatt aaaaattaaa 180

gaggtatata tta 193

<210> 235
<211> 193
<212> DNA
<213> Artificial sequence

<220>
<223> PTrc2 promoter

<400> 235
ctgttgacaa ttaatcatcc ggctcgtgta atgtgtggaa ttgtgagcgg ataacaattt 60

cacacaggaa acagcgccgc tgagaaaaag cgaagcggca ctgctcttta acaatttatc 120

agacaatctg tgtgggcact cgaccggaat tatcgattaa ctttattatt aaaaattaaa 180

gaggtatata tta 193

<210> 236
<211> 5978
<212> DNA
<213> Artificial sequence

<220>
<223> pDS80 plasmid

<400> 236
cactatacca attgagatgg gctagtcaat gataattact agtccttttc ctttgagttg 60

tgggtatctg taaattctgc tagacctttg ctggaaaact tgtaaattct gctagaccct 120

ctgtaaattc cgctagacct ttgtgtgttt tttttgttta tattcaagtg gttataattt 180

atagaataaa gaaagaataa aaaaagataa aaagaataga tcccagccct gtgtataact 240

cactacttta gtcagttccg cagtattaca aaaggatgtc gcaaacgctg tttgctcctc 300

tacaaaacag accttaaaac cctaaaggcg tcggcatccg cttacagaca agctgtgacc 360

gtctccggga gctgcatgtg tcagaggttt tcaccgtcat caccgaaacg cgcgaggcag 420

cagatcaatt cgcgcgcgaa ggcgaagcgg catgcattta cgttgacacc atcgaatggt 480

gcaaaacctt tcgcggtatg gcatgatagc gcccggaaga gagtcaattc agggtggtga 540

atgtgaaacc agtaacgtta tacgatgtcg cagagtatgc cggtgtctct tatcagaccg 600

tttcccgcgt ggtgaaccag gccagccacg tttctgcgaa aacgcgggaa aaagtggaag 660

cggcgatggc ggagctgaat tacattccca accgcgtggc acaacaactg gcgggcaaac 720

agtcgttgct gattggcgtt gccacctcca gtctggccct gcacgcgccg tcgcaaattg 780

tcgcggcgat taaatctcgc gccgatcaac tgggtgccag cgtggtggtg tcgatggtag 840

aacgaagcgg cgtcgaagcc tgtaaagcgg cggtgcacaa tcttctcgcg caacgcgtca 900

gtgggctgat cattaactat ccgctggatg accaggatgc cattgctgtg gaagctgcct 960

gcactaatgt tccggcgtta tttcttgatg tctctgacca gacacccatc aacagtatta 1020

ttttctccca tgaagacggt acgcgactgg gcgtggagca tctggtcgca ttgggtcacc 1080

agcaaatcgc gctgttagcg ggcccattaa gttctgtctc ggcgcgtctg cgtctggctg 1140

gctggcataa atatctcact cgcaatcaaa ttcagccgat agcggaacgg gaaggcgact 1200

ggagtgccat gtccggtttt caacaaacca tgcaaatgct gaatgagggc atcgttccca 1260

ctgcgatgct ggttgccaac gatcagatgg cgctgggcgc aatgcgcgcc attaccgagt 1320

ccgggctgcg cgttggtgcg gatatctcgg tagtgggata cgacgatacc gaagacagct 1380

catgttatat cccgccgtta accaccatca aacaggattt tcgcctgctg gggcaaacca 1440

gcgtggaccg cttgctgcaa ctctctcagg gccaggcggt gaagggcaat cagctgttgc 1500

ccgtctcact ggtgaaaaga aaaaccaccc tggcgcccaa tacgcaaacc gcctctcccc 1560

gcgcgttggc cgattcatta atgcagctgg cacgacaggt ttcccgactg gaaagcgggc 1620

agtgagcgca acgcaattaa tgtaagttag cgcgaattga tctggtttga cagcttatca 1680

tcgactgcac ggtgcaccaa tgcttctggc gtcaggcagc catcggaagc tgtggtatgg 1740

ctgtgcaggt cgtaaatcac tgcataattc gtgtcgctca aggcgcactc ccgttctgga 1800

taatgttttt tgcgccgaca tcataacggt tctggcaaat attttcagat ctctcaccta 1860

ccaaacaatg ccccctgca aaaaataaat tcatataaaa aacatacaga taaccatctg 1920

cggtgataaa ttatctctgg cggtgttgac ataaatacca ctggcggtga tactgagcac 1980

agaatattca cacaggaaac agcgccgctg agaaaaagcg aagcggcact gctctttaac 2040

aatttatcag acaatctgtg tgggcactcg accggaatta tcgattaact ttattattaa 2100

aaattaaaga ggtatatatt aatgtatcga ttaaataagg aggaataaac catggatccg 2160

agctcgagat ctgcagctgg taccatatgg gaattcgaag cttgggcccg aacaaaaact 2220

catctcagaa gaggatctga atagcgccgt cgaccatcat catcatcatc attgagttta 2280

aacggtctcc agcttggctg ttttggcgga tgagagaaga ttttcagcct gatacagatt 2340

aaatcagaac gcagaagcgg tctgataaaa cagaatttgc ctggcggcag tagcgcggtg 2400

gtcccacctg accccatgcc gaactcagaa gtgaaacgcc gtagcgccga tggtagtgtg 2460

gggtctcccc atgcgagagt agggaactgc caggcatcaa ataaaacgaa aggctcagtc 2520

gaaagactgg gcctttcgtt ttatctgttg tttgtcggtg aacgctctcc tgacgcctga 2580

tgcggtattt tctccttacg catctgtgcg gtatttcaca ccgcatatgg tgcactctca 2640

gtacaatctg ctctgatgcc gcatagttaa gccagccccg acacccgcca acacccgctg 2700

acgagcttag taaagccctc gctagatttt aatgcggatg ttgcgattac ttcgccaact 2760

attgcgataa caagaaaaag ccagcctttc atgatatatc tcccaatttg tgtagggctt 2820

attatgcacg cttaaaaata ataaaagcag acttgacctg atagtttggc tgtgagcaat 2880

tatgtgctta gtgcatctaa cgcttgagtt aagccgcgcc gcgaagcggc gtcggcttga 2940
acgaattgtt agacattatt tgccgactac cttggtgatc tcgcctttca cgtagtggac 3000
aaattcttcc aactgatctg cgcgcgaggc caagcgatct tcttcttgtc caagataagc 3060
ctgtctagct tcaagtatga cgggctgata ctgggccggc aggcgctcca ttgcccagtc 3120
ggcagcgaca tccttcggcg cgattttgcc ggttactgcg ctgtaccaaa tgcgggacaa 3180
cgtaagcact acatttcgct catcgccagc ccagtcgggc ggcgagttcc atagcgttaa 3240
ggtttcattt agcgcctcaa atagatcctg ttcaggaacc ggatcaaaga gttcctccgc 3300
cgctggacct accaaggcaa cgctatgttc tcttgctttt gtcagcaaga tagccagatc 3360
aatgtcgatc gtggctggct cgaagatacc tgcaagaatg tcattgcgct gccattctcc 3420
aaattgcagt tcgcgcttag ctggataacg ccacggaatg atgtcgtcgt gcacaacaat 3480
ggtgacttct acagcgcgga gaatctcgct ctctccaggg gaagccgaag tttccaaaag 3540
gtcgttgatc aaagctcgcc gcgttgtttc atcaagcctt acggtcaccg taaccagcaa 3600
atcaatatca ctgtgtggct tcaggccgcc atccactgcg gagccgtaca aatgtacggc 3660
cagcaacgtc ggttcgagat ggcgctcgat gacgccaact acctctgata gttgagtcga 3720
tacttcggcg atcaccgctt ccctcatgat gtttaacttt gttttagggc gactgccctg 3780
ctgcgtaaca tcgttgctgc tccataacat caaacatcga cccacggcgt aacgcgcttg 3840
ctgcttggat gcccgaggca tagactgtac cccaaaaaaa cagtcataac aagccatgaa 3900
aaccgccact gcgccgttac caccgctgcg ttcggtcaag gttctggacc agttgcgtga 3960
gcgcatacgc tacttgcatt acagcttacg aaccgaacag gcttatgtcc actgggttcg 4020
tgccttcatc cgtttccacg gtgtgcgtca cccggcaacc ttgggcagca gcgaagtcga 4080
ggcatttctg tcctggctgg cgaacgagcg caaggtttcg gtctccacgc atcgtcaggc 4140
attggcggcc ttgctgttct tctacggcaa ggtgctgtgc acggatctgc cctggcttca 4200
ggagatcgga agacctcggc cgtcgcggcg cttgccggtg gtgctgaccc cggatgaagt 4260
ggttcgcatc ctcggttttc tggaaggcga gcatcgtttg ttcgcccagc ttctgtatgg 4320
aacgggcatg cggatcagtg agggtttgca actgcgggtc aaggatctgg atttcgatca 4380
cggcacgatc atcgtgcggg agggcaaggg ctccaaggat cgggccttga tgttacccga 4440
gagcttggca cccagcctgc gcgagcaggg gaattaattc ccacgggttt tgctgcccgc 4500
aaacgggctg ttctggtgtt gctagtttgt tatcagaatc gcagatccgg cttcagccgg 4560
tttgccggct gaaagcgcta tttcttccag aattgccatg atttttcccc cacgggaggc 4620
gtcactggct cccgtgttgt cggcagcttt gattcgataa gcagcatcgc ctgtttcagg 4680
ctgtctatgt gtgactgttg agctgtaaca agttgtctca ggtgttcaat ttcatgttct 4740
agttgctttg ttttactggt ttcacctgtt ctattaggtg ttacatgctg ttcatctgtt 4800

acattgtcga tctgttcatg gtgaacagct ttgaatgcac caaaaactcg taaaagctct 4860

gatgtatcta tcttttttac accgttttca tctgtgcata tggacagttt tcccttttgat 4920

atgtaacggt gaacagttgt tctacttttg tttgttagtc ttgatgcttc actgatagat 4980

acaagagcca taagaacctc agatccttcc gtatttagcc agtatgttct ctagtgtggt 5040

tcgttgtttt tgcgtgagcc atgagaacga accattgaga tcatacttac tttgcatgtc 5100

actcaaaaat tttgcctcaa aactggtgag ctgaattttt gcagttaaag catcgtgtag 5160

tgtttttctt agtccgttat gtaggtagga atctgatgta atggttgttg gtattttgtc 5220

accattcatt tttatctggt tgttctcaag ttcggttacg agatccattt gtctatctag 5280

ttcaacttgg aaaatcaacg tatcagtcgg gcggcctcgc ttatcaacca ccaatttcat 5340

attgctgtaa gtgtttaaat ctttacttat tggtttcaaa acccattggt taagcctttt 5400

aaactcatgg tagttatttt caagcattaa catgaactta aattcatcaa ggctaatctc 5460

tatatttgcc ttgtgagttt tcttttgtgt tagttctttt aataaccact cataaatcct 5520

catagagtat ttgttttcaa aagacttaac atgttccaga ttatatttta tgaatttttt 5580

taactggaaa agataaggca atatctcttc actaaaaact aattctaatt tttcgcttga 5640

gaacttggca tagtttgtcc actggaaaat ctcaaagcct ttaaccaaag gattcctgat 5700

ttccacagtt ctcgtcatca gctctctggt tgctttagct aatacaccat aagcattttc 5760

cctactgatg ttcatcatct gagcgtattg gttataagtg aacgataccg tccgttcttt 5820

ccttgtaggg ttttcaatcg tggggttgag tagtgccaca cagcataaaa ttagcttggt 5880

ttcatgctcc gttaagtcat agcgactaat cgctagttca tttgctttga aaacaactaa 5940

ttcagacata catctcaatt ggtctaggtg attttaat 5978

<210> 237
<211> 3227
<212> DNA
<213> Artificial sequence

<220>
<223> p100.38 plasmid

<400> 237
gacgaaaggg cctcgtgata cgcctatttt tataggttaa tgtcatgata ataatggttt 60

cttagacgtc aggtggcact tttcggggaa atgtgcgcgg aacccctatt tgtttatttt 120

tctaaataca ttcaaatatg tatccgctca tgagacaata accctgataa atgcttcaat 180

aatattgaaa aaggaagagt atgagtattc aacatttccg tgtcgccctt attccctttt 240

ttgcggcatt ttgccttcct gtttttgctc acccagaaac gctggtgaaa gtaaaagatg 300

ctgaagatca gttgggtgca cgagtgggtt acatcgaact ggatctcaac agcggtaaga 360

tccttgagag ttttcgcccc gaagaacgtt ttccaatgat gagcactttt aaagttctgc 420
tatgtggcgc ggtattatcc cgtattgacg ccgggcaaga gcaactcggt cgccgcatac 480
actattctca gaatgacttg gttgagtact caccagtcac agaaaagcat cttacggatg 540
gcatgacagt aagagaatta tgcagtgctg ccataaccat gagtgataac actgcggcca 600
acttacttct gacaacgatc ggaggaccga aggagctaac cgcttttttg cacaacatgg 660
gggatcatgt aactcgcctt gatcgttggg aaccggagct gaatgaagcc ataccaaacg 720
acgagcgtga caccacgatg cctgtagcaa tggcaacaac gttgcgcaaa ctattaactg 780
gcgaactact tactctagct tcccggcaac aattaataga ctggatggag gcggataaag 840
ttgcaggacc acttctgcgc tcggcccttc cggctggctg gtttattgct gataaatctg 900
gagccggtga gcgtgggtct cgcggtatca ttgcagcact ggggccagat ggtaagccct 960
cccgtatcgt agttatctac acgacgggga gtcaggcaac tatggatgaa cgaaatagac 1020
agatcgctga gataggtgcc tcactgatta agcattggta actgtcagac caagtttact 1080
catatatact ttagattgat ttaaaacttc atttttaatt tgtgcatccg aagatcagca 1140
gttcaacctg ttgatagtac gtactaagct ctcatgtttc acgtactaag ctctcatgtt 1200
taacgtacta agctctcatg tttaacgaac taaaccctca tggctaacgt actaagctct 1260
catggctaac gtactaagct ctcatgtttg aacaataaaa ttaatataaa tcagcaactt 1320
aaatagcctc taaggtttta agttttataa gaaaaaaaag aatatataag gcttttaaag 1380
ctagctttta aggtttcacc atgttctttc ctgcgttatc ccctgattct gtggataacc 1440
gtattaccgc ctttgagtga gctgataccg ctcgccgcag ccgaacgacc gagcgcagcg 1500
agtcagtgag cgaggaagcg gaagagcgcc caatacgcaa accgcctctc cccgcgcgtt 1560
ggccgattca ttaagacagc tgtctcttat acacatctca accctgaagc tcttgttggc 1620
tagtgcgtag tcgttggcaa gctttccgct gtttctgcat tcttacgttt taggatgcat 1680
atggcggccg cataacttcg tatagcatac attatacgaa gttatctaga gttgcatgcc 1740
tgcaggtccg cttattatca cttattcagg cgtagcaacc aggcgtttaa gggcaccaat 1800
aactgcctta aaaaaattac gccccgccct gccactcatc gcagtactgt tgtaattcat 1860
taagcattct gccgacatgg aagccatcac aaacggcatg atgaacctga atcgccagcg 1920
gcatcagcac cttgtcgcct tgcgtataat atttgcccat ggtgaaaacg ggggcgaaga 1980
agttgtccat attggccacg tttaaatcaa aactggtgaa actcacccag ggattggctg 2040
agacgaaaaa catattctca ataaaccctt tagggaaata ggccaggttt tcaccgtaac 2100
acgccacatc ttgcgaatat atgtgtagaa actgccggaa atcgtcgtgg tattcactcc 2160
agagcgatga aaacgtttca gtttgctcat ggaaaacggt gtaacaaggg tgaacactat 2220
cccatatcac cagctcaccg tctttcattg ccatacggaa ttccggatga gcattcatca 2280

ggcgggcaag aatgtgaata aaggccggat aaaacttgtg cttatttttc tttacggtct 2340

ttaaaaaggc cgtaatatcc agctgaacgg tctggttata ggtacattga gcaactgact 2400

gaaatgcctc aaaatgttct ttacgatgcc attgggatat atcaacggtg gtatatccag 2460

tgattttttt ctccatttta gcttccttag ctcctgaaaa tctcgataac tcaaaaaata 2520

cgcccggtag tgatcttatt tcattatggt gaaagttgga acctcttacg tgccgatcaa 2580

cgtctcattt tcgccaaaag ttggcccagg gcttcccggt atcaacaggg acaccaggat 2640

ttatttattc tgcgaagtga tcttccgtca caggtattta ttcgactcta gataacttcg 2700

tatagcatac attatacgaa gttatggatc cagcttatcg ataccgtcaa acaaatcata 2760

aaaaatttat ttgctttcag gaaaattttt ctgtataata gattcaattg cgatgacgac 2820

gaacacgcat taaggaggtg aagagctcga attcgagcca atatgcgaga acacccgaga 2880

aaattcatcg atgatggttg agatgtgtat aagagacagc tgtcgtaata gcgaagaggc 2940

ccgcaccgat cgcccttccc aacagttgcg cagcctgaat ggcgaatggc gcctgatgcg 3000

gtattttctc cttacgcatc tgtgcggtat ttcacaccgc atatggtgca ctctcagtac 3060

aatctgctct gatgccgcat agttaagcca gccccgacac ccgccaacac ccgctgacgc 3120

gccctgacgg gcttgtctgc tcccggcatc cgcttacaga caagctgtga ccgtctccgg 3180

gagctgcatg tgtcagaggt tttcaccgtc atcaccgaaa cgcgcga 3227

<210> 238
<211> 70
<212> DNA
<213> Artificial sequence

<220>
<223> Synthetic primer

<400> 238
aaaaacagca acaatgtgag ctttgttgta attatattgt aaacatattg attccgggga 60

tccgtcgacc 70

<210> 239
<211> 68
<212> DNA
<213> Artificial sequence

<220>
<223> Synthetic primer

<400> 239
aaacggagcc tttcggctcc gttattcatt tacgcggctt caactttcct gtaggctgga 60

gctgcttc 68

<210> 240

<211> 23
<212> DNA
<213> Artificial sequence

<220>
<223> Synthetic primer

<400> 240
cgggcaggtg ctatgaccag gac 23

<210> 241
<211> 23
<212> DNA
<213> Artificial sequence

<220>
<223> Synthetic primer

<400> 241
cgcggcgttg accggcagcc tgg 23

<210> 242
<211> 70
<212> DNA
<213> Artificial sequence

<220>
<223> Synthetic primer

<400> 242
atcattctcg tttacgttat cattcacttt acatcagaga tataccaatg attccgggga 60

tccgtcgacc 70

<210> 243
<211> 69
<212> DNA
<213> Artificial sequence

<220>
<223> Synthetic primer

<400> 243
gcacggaaat ccgtgcccca aaagagaaat tagaaacgga aggttgcggt tgtaggctgg 60

agctgcttc 69

<210> 244
<211> 21
<212> DNA
<213> Artificial sequence

<220>
<223> Synthetic primer

<400> 244
caacagcaac ctgctcagca a 21

<210> 245
<211> 21
<212> DNA
<213> Artificial sequence

<220>
<223> Synthetic primer

<400> 245
aagctggagc agcaaagcgt t 21

<210> 246
<211> 27
<212> DNA
<213> Artificial sequence

<220>
<223> Synthetic primer

<400> 246
gctctcgagt tagtaacaga tgtcttc 27

<210> 247
<211> 27
<212> DNA
<213> Artificial sequence

<220>
<223> Synthetic primer

<400> 247
gcggatccat ggcaactgag tatgacg 27

<210> 248
<211> 32
<212> DNA
<213> Artificial sequence

<220>
<223> Synthetic primer

<400> 248
ataaaccatg gatccatgaa cgagtacgcc cc 32

<210> 249
<211> 33
<212> DNA
<213> Artificial sequence

<220>
<223> Synthetic primer

<400> 249
ccaagcttcg aattctcaga tatgcaaggc gtg 33

<210> 250
<211> 41
<212> DNA

<213> Artificial sequence

<220>
<223> Synthetic primer

<400> 250
gaggaataaa ccgtggcaac agaatatgat gtcgttattc t 41

<210> 251
<211> 45
<212> DNA
<213> Artificial sequence

<220>
<223> Synthetic primer

<400> 251
cccaagcttc gaattttaat acaatgctgt attttctttg gaaat 45

<210> 252
<211> 36
<212> DNA
<213> Artificial sequence

<220>
<223> Synthetic primer

<400> 252
tgaattccat ggcgcaactc actcttcttt tagtcg 36

<210> 253
<211> 39
<212> DNA
<213> Artificial sequence

<220>
<223> Synthetic primer

<400> 253
cagtacctcg agtcttcgta tacatatgcg ctcagtcac 39

<210> 254
<211> 21
<212> DNA
<213> Artificial sequence

<220>
<223> Synthetic primer

<400> 254
ccttggggca tatgaaagct g 21

<210> 255
<211> 29
<212> DNA
<213> Artificial sequence

<220>

<223> Synthetic primer

<400> 255
tttagtcatc tcgagtgcac ctcaccttt 29

<210> 256
<211> 37
<212> DNA
<213> Artificial sequence

<220>
<223> Synthetic primer

<400> 256
gaggaataaa ccatgaacgc aggaatttta ggagtag 37

<210> 257
<211> 41
<212> DNA
<213> Artificial sequence

<220>
<223> Synthetic primer

<400> 257
cccaagcttc gaattcttac ttaccccaac gaatgattag g 41

<210> 258
<211> 35
<212> DNA
<213> Artificial sequence

<220>
<223> Synthetic primer

<400> 258
tttcgcgagg ccggccccgc caacacccgc tgacg 35

<210> 259
<211> 39
<212> DNA
<213> Artificial sequence

<220>
<223> Synthetic primer

<400> 259
aaggacgtct taattaatca ggagagcgtt caccgacaa 39

<210> 260
<211> 24
<212> DNA
<213> Artificial sequence

<220>
<223> Synthetic primer

<400> 260

cttaacttca tgtgaaaagt ttgt 24

<210> 261
<211> 24
<212> DNA
<213> Artificial sequence

<220>
<223> Synthetic primer

<400> 261
acaataccca tgtttatagg gcaa 24

<210> 262
<211> 71
<212> DNA
<213> Artificial sequence

<220>
<223> Synthetic primer

<400> 262
cctgacagtg cgggcttttt ttttcgacca aaggtaacga ggtaacaacc gtgtaggctg 60

gagctgcttc g 71

<210> 263
<211> 62
<212> DNA
<213> Artificial sequence

<220>
<223> Synthetic primer

<400> 263
gtatatatta atgtatcgat taaataagga ggaataaacc atgcgagtgt tgaagttcgg 60

cg 62

<210> 264
<211> 59
<212> DNA
<213> Artificial sequence

<220>
<223> Synthetic primer

<400> 264
ctgatgtacc gccgaacttc aacactcgca tggtttattc ctccttattt aatcgatac 59

<210> 265
<211> 28
<212> DNA
<213> Artificial sequence

<220>
<223> Synthetic primer

<400> 265
gcgcccgtat tttcgtggtg ctgattac 28

<210> 266
<211> 28
<212> DNA
<213> Artificial sequence

<220>
<223> Synthetic primer

<400> 266
gtaatcagca ccacgtaaat acgggcgc 28

<210> 267
<211> 25
<212> DNA
<213> Artificial sequence

<220>
<223> Synthetic primer

<400> 267
tcagactcct aacttccatg agagg 25

<210> 268
<211> 50
<212> DNA
<213> Artificial sequence

<220>
<223> Synthetic primer

<400> 268
aatatttgcc agaaccgtta tgatgtcggc attccgggga tccgtcgacc 50

<210> 269
<211> 55
<212> DNA
<213> Artificial sequence

<220>
<223> Synthetic primer

<400> 269
cttcgaactg caggtcgacg gatccccgga atgccgacat cataacggtt ctggc 55

<210> 270
<211> 29
<212> DNA
<213> Artificial sequence

<220>
<223> Synthetic primer

<400> 270
gctgatcatt aactatccgc tggatgacc 29

<210> 271
<211> 40
<212> DNA
<213> Artificial sequence

<220>
<223> Synthetic primer

<400> 271
actggaaagc gggcagtgag cgcaacgcaa ttaatgtaag 40

<210> 272
<211> 16
<212> DNA
<213> Artificial sequence

<220>
<223> Synthetic primer

<400> 272
tcactgcccg ctttcc 16

<210> 273
<211> 55
<212> DNA
<213> Artificial sequence

<220>
<223> Synthetic primer

<400> 273
accggcagat cgtatgtaat atgcatggtt tattcctcct tatttaatcg ataca 55

<210> 274
<211> 23
<212> DNA
<213> Artificial sequence

<220>
<223> Synthetic primer

<400> 274
atgcatatta catacgatct gcc 23

<210> 275
<211> 38
<212> DNA
<213> Artificial sequence

<220>
<223> Synthetic primer

<400> 275
ggtcgacgga tccccggaat taagcgtcaa cgaaaccg 38

<210> 276
<211> 38

<212> DNA
<213> Artificial sequence

<220>
<223> Synthetic primer

<400> 276
gaagcagctc cagcctacac cagacgatgg tgcaggat 38

<210> 277
<211> 21
<212> DNA
<213> Artificial sequence

<220>
<223> Synthetic primer

<400> 277
gcaaagacca gaccgttcat a 21

<210> 278
<211> 20
<212> DNA
<213> Artificial sequence

<220>
<223> Synthetic primer

<400> 278
attccgggga tccgtcgacc 20

<210> 279
<211> 20
<212> DNA
<213> Artificial sequence

<220>
<223> Synthetic primer

<400> 279
tgtaggctgg agctgcttcg 20

<210> 280
<211> 71
<212> DNA
<213> Artificial sequence

<220>
<223> Synthetic primer

<400> 280
cctgacagtg cgggcttttt ttttcgacca aaggtaacga ggtaacaacc gtgtaggctg 60

gagctgcttc g 71

<210> 281
<211> 62
<212> DNA

<213> Artificial sequence

<220>
<223> Synthetic primer

<400> 281
gtatatatta atgtatcgat taaataagga ggaataaacc atgcgagtgt tgaagttcgg 60

cg 62

<210> 282
<211> 74
<212> DNA
<213> Artificial sequence

<220>
<223> Synthetic primer

<400> 282
aaaaacagca acaatgtgag ctttgttgta attatattgt aaacatattg tccgctgttt 60

ctgcattctt acgt 74

<210> 283
<211> 72
<212> DNA
<213> Artificial sequence

<220>
<223> Synthetic primer

<400> 283
gatgacgacg aacacgcatt aaggaggtga ataaggagga ataacatatg aaagctggca 60

ttcttggtgt tg 72

<210> 284
<211> 72
<212> DNA
<213> Artificial sequence

<220>
<223> Synthetic primer

<400> 284
gtaacgtcca acaccaagaa tgccagcttt catatgttat tcctccttat tcacctcctt 60

aatgcgtgtt cg 72

<210> 285
<211> 70
<212> DNA
<213> Artificial sequence

<220>
<223> Synthetic primer

<400> 285
aaacggagcc tttcggctcc gttattcatt tacgcggctt caactttccg ttatcggccc 60

cagcggattg 70

<210> 286
<211> 70
<212> DNA
<213> Artificial sequence

<220>
<223> Synthetic primer

<400> 286
cgcagtttgc aagtgacggt atataaccga aaagtgactg agcgtacatg attccgggga 60

tccgtcgacc 70

<210> 287
<211> 70
<212> DNA
<213> Artificial sequence

<220>
<223> Synthetic primer

<400> 287
gcaaattgcg tcatgtttta atccttatcc tagaaacgaa ccagcgcgga tgtaggctgg 60

agctgcttcg 70

<210> 288
<211> 20
<212> DNA
<213> Artificial sequence

<220>
<223> Synthetic primer

<400> 288
gcagcgacaa gttcctcagc 20

<210> 289
<211> 21
<212> DNA
<213> Artificial sequence

<220>
<223> Synthetic primer

<400> 289
ccgcagaagc ttcagcaaac g 21

<210> 290
<211> 23
<212> DNA
<213> Artificial sequence

<220>
<223> Synthetic primer

<400> 290
cgggcaggtg ctatgaccag gac 23

<210> 291
<211> 21
<212> DNA
<213> Artificial sequence

<220>
<223> Synthetic primer

<400> 291
gggcaggata agctcgggag g 21

<210> 292
<211> 28
<212> DNA
<213> Artificial sequence

<220>
<223> Synthetic primer

<400> 292
atatgacgtc ggcatccgct tacagaca 28

<210> 293
<211> 32
<212> DNA
<213> Artificial sequence

<220>
<223> Synthetic primer

<400> 293
aattcttaag tcaggagagc gttcaccgac aa 32

<210> 294
<211> 41
<212> DNA
<213> Artificial sequence

<220>
<223> Synthetic primer

<400> 294
taaggaggaa taaaccatga gtgaaacata cgtgtctgag a 41

<210> 295
<211> 47
<212> DNA
<213> Artificial sequence

<220>
<223> Synthetic primer

<400> 295
cgggcccaag cttcgaattt tattaggtca agtattcgta ctcaggg 47

<210> 296
<211> 55
<212> DNA
<213> Artificial sequence

<220>
<223> Synthetic primer

<400> 296
cttcgaactg caggtcgacg gatccccgga atgccgacat cataacggtt ctggc 55

<210> 297
<211> 50
<212> DNA
<213> Artificial sequence

<220>
<223> Synthetic primer

<400> 297
aatatttgcc agaaccgtta tgatgtcggc attccgggga tccgtcgacc 50

<210> 298
<211> 68
<212> DNA
<213> Artificial sequence

<220>
<223> Synthetic primer

<400> 298
gtatatatta atgtatcgat taaataagga ggaataaacc atgatggtaa ggatatttga 60

tacaacac 68

<210> 299
<211> 60
<212> DNA
<213> Artificial sequence

<220>
<223> Synthetic primer

<400> 299
ctaagtgttg tatcaaatat ccttaccatc atggtttatt cctccttatt taatcgatac 60

<210> 300
<211> 66
<212> DNA
<213> Artificial sequence

<220>
<223> Synthetic primer

<400> 300
gatttgttgg ctatagttag agaagttact ggaaaattgt aacaaggaaa ccgtgtgatg 60

tcgaag 66

<210> 301
<211> 62
<212> DNA
<213> Artificial sequence

<220>
<223> Synthetic primer

<400> 301
gtaattcttc gacatcacac ggtttccttg ttacaatttt ccagtaactt ctctaactat 60

ag 62

<210> 302
<211> 22
<212> DNA
<213> Artificial sequence

<220>
<223> Synthetic primer

<400> 302
ggtagcgaag gttttgcccg gc 22

<210> 303
<211> 22
<212> DNA
<213> Artificial sequence

<220>
<223> Synthetic primer

<400> 303
gattggtgcc ccaggtgacc tg 22

<210> 304
<211> 72
<212> DNA
<213> Artificial sequence

<220>
<223> Synthetic primer

<400> 304
gagttgcaac gcaaagctca acacaacgaa aacaacaagg aaaccgtgtg agtgtaggct 60

ggagctgctt cg 72

<210> 305
<211> 19
<212> DNA
<213> Artificial sequence

<220>
<223> Synthetic primer

<400> 305
cttccacggc gtcggcctg 19

<210> 306
<211> 41
<212> DNA
<213> Artificial sequence

<220>
<223> Synthetic primer

<400> 306
gaggaataaa ccgtggcaac agaatatgat gtcgttattc t 41

<210> 307
<211> 45
<212> DNA
<213> Artificial sequence

<220>
<223> Synthetic primer

<400> 307
cccaagcttc gaattttaat acaatgctgt attttctttg gaaat 45

<210> 308
<211> 473
<212> PRT
<213> Marinobacter hydrocarbonoclasticus

<220>
<221> MISC_FEATURE
<223> ester synthase

<400> 308

Met Lys Arg Leu Gly Thr Leu Asp Ala Ser Trp Leu Ala Val Glu Ser
1 5 10 15

Glu Asp Thr Pro Met His Val Gly Thr Leu Gln Ile Phe Ser Leu Pro
20 25 30

Glu Gly Ala Pro Glu Thr Phe Leu Arg Asp Met Val Thr Arg Met Lys
35 40 45

Glu Ala Gly Asp Val Ala Pro Pro Trp Gly Tyr Lys Leu Ala Trp Ser
50 55 60

Gly Phe Leu Gly Arg Val Ile Ala Pro Ala Trp Lys Val Asp Lys Asp
65 70 75 80

Ile Asp Leu Asp Tyr His Val Arg His Ser Ala Leu Pro Arg Pro Gly
85 90 95

```
Gly Glu Arg Glu Leu Gly Ile Leu Val Ser Arg Leu His Ser Asn Pro
            100                 105                 110

Leu Asp Phe Ser Arg Pro Leu Trp Glu Cys His Val Ile Glu Gly Leu
        115                 120                 125

Glu Asn Asn Arg Phe Ala Leu Tyr Thr Lys Met His His Ser Met Ile
    130                 135                 140

Asp Gly Ile Ser Gly Val Arg Leu Met Gln Arg Val Leu Thr Thr Asp
145                 150                 155                 160

Pro Glu Arg Cys Asn Met Pro Pro Pro Trp Thr Val Arg Pro His Gln
                165                 170                 175

Arg Arg Gly Ala Lys Thr Asp Lys Glu Ala Ser Val Pro Ala Ala Val
            180                 185                 190

Ser Gln Ala Met Asp Ala Leu Lys Leu Gln Ala Asp Met Ala Pro Arg
        195                 200                 205

Leu Trp Gln Ala Gly Asn Arg Leu Val His Ser Val Arg His Pro Glu
    210                 215                 220

Asp Gly Leu Thr Ala Pro Phe Thr Gly Pro Val Ser Val Leu Asn His
225                 230                 235                 240

Arg Val Thr Ala Gln Arg Arg Phe Ala Thr Gln His Tyr Gln Leu Asp
                245                 250                 255

Arg Leu Lys Asn Leu Ala His Ala Ser Gly Gly Ser Leu Asn Asp Ile
            260                 265                 270

Val Leu Tyr Leu Cys Gly Thr Ala Leu Arg Arg Phe Leu Ala Glu Gln
        275                 280                 285

Asn Asn Leu Pro Asp Thr Pro Leu Thr Ala Gly Ile Pro Val Asn Ile
    290                 295                 300

Arg Pro Ala Asp Asp Glu Gly Thr Gly Thr Gln Ile Ser Phe Met Ile
305                 310                 315                 320

Ala Ser Leu Ala Thr Asp Glu Ala Asp Pro Leu Asn Arg Leu Gln Gln
                325                 330                 335

Ile Lys Thr Ser Thr Arg Arg Ala Lys Glu His Leu Gln Lys Leu Pro
            340                 345                 350
```

Lys Ser Ala Leu Thr Gln Tyr Thr Met Leu Leu Met Ser Pro Tyr Ile
355 360 365

Leu Gln Leu Met Ser Gly Leu Gly Gly Arg Met Arg Pro Val Phe Asn
370 375 380

Val Thr Ile Ser Asn Val Pro Gly Pro Glu Gly Thr Leu Tyr Tyr Glu
385 390 395 400

Gly Ala Arg Leu Glu Ala Met Tyr Pro Val Ser Leu Ile Ala His Gly
405 410 415

Gly Ala Leu Asn Ile Thr Cys Leu Ser Tyr Ala Gly Ser Leu Asn Phe
420 425 430

Gly Phe Thr Gly Cys Arg Asp Thr Leu Pro Ser Met Gln Lys Leu Ala
435 440 445

Val Tyr Thr Gly Glu Ala Leu Asp Glu Leu Glu Ser Leu Ile Leu Pro
450 455 460

Pro Lys Lys Arg Ala Arg Thr Arg Lys
465 470

<210> 309
<211> 341
<212> PRT
<213> Synechococcus elongatus

<220>
<221> MISC_FEATURE
<223> acyl-ACP reductase

<400> 309

Met Phe Gly Leu Ile Gly His Leu Thr Ser Leu Glu Gln Ala Arg Asp
1 5 10 15

Val Ser Arg Arg Met Gly Tyr Asp Glu Tyr Ala Asp Gln Gly Leu Glu
20 25 30

Phe Trp Ser Ser Ala Pro Pro Gln Ile Val Asp Glu Ile Thr Val Thr
35 40 45

Ser Ala Thr Gly Lys Val Ile His Gly Arg Tyr Ile Glu Ser Cys Phe
50 55 60

Leu Pro Glu Met Leu Ala Ala Arg Arg Phe Lys Thr Ala Thr Arg Lys

65 70 75 80

Val Leu Asn Ala Met Ser His Ala Gln Lys His Gly Ile Asp Ile Ser
85 90 95

Ala Leu Gly Gly Phe Thr Ser Ile Ile Phe Glu Asn Phe Asp Leu Ala
100 105 110

Ser Leu Arg Gln Val Arg Asp Thr Thr Leu Glu Phe Glu Arg Phe Thr
115 120 125

Thr Gly Asn Thr His Thr Ala Tyr Val Ile Cys Arg Gln Val Glu Ala
130 135 140

Ala Ala Lys Thr Leu Gly Ile Asp Ile Thr Gln Ala Thr Val Ala Val
145 150 155 160

Val Gly Ala Thr Gly Asp Ile Gly Ser Ala Val Cys Arg Trp Leu Asp
165 170 175

Leu Lys Leu Gly Val Gly Asp Leu Ile Leu Thr Ala Arg Asn Gln Glu
180 185 190

Arg Leu Asp Asn Leu Gln Ala Glu Leu Gly Arg Gly Lys Ile Leu Pro
195 200 205

Leu Glu Ala Ala Leu Pro Glu Ala Asp Phe Ile Val Trp Val Ala Ser
210 215 220

Met Pro Gln Gly Val Val Ile Asp Pro Ala Thr Leu Lys Gln Pro Cys
225 230 235 240

Val Leu Ile Asp Gly Gly Tyr Pro Lys Asn Leu Gly Ser Lys Val Gln
245 250 255

Gly Glu Gly Ile Tyr Val Leu Asn Gly Gly Val Val Glu His Cys Phe
260 265 270

Asp Ile Asp Trp Gln Ile Met Ser Ala Ala Glu Met Ala Arg Pro Glu
275 280 285

Arg Gln Met Phe Ala Cys Phe Ala Glu Ala Met Leu Leu Glu Phe Glu
290 295 300

Gly Trp His Thr Asn Phe Ser Trp Gly Arg Asn Gln Ile Thr Ile Glu
305 310 315 320

Lys Met Glu Ala Ile Gly Glu Ala Ser Val Arg His Gly Phe Gln Pro
325 330 335

Leu Ala Leu Ala Ile
340

<210> 310
<211> 22
<212> DNA
<213> Artificial sequence

<220>
<223> Synthetic primer

<400> 310
ggctggctgg cataaatatc tc 22

<210> 311
<211> 69
<212> DNA
<213> Artificial sequence

<220>
<223> Synthetic primer

<400> 311
gttatgatat gttggtcgga taagcgtcgc gccgcatccg acattgattg cgagagcgtt 60

caccgacaa 69

<210> 312
<211> 232
<212> PRT
<213> Nostoc punctiforme

<220>
<221> MISC_FEATURE
<223> aldehyde decarbonylase (ADC)

<400> 312

Met Gln Gln Leu Thr Asp Gln Ser Lys Glu Leu Asp Phe Lys Ser Glu
1 5 10 15

Thr Tyr Lys Asp Ala Tyr Ser Arg Ile Asn Ala Ile Val Ile Glu Gly
20 25 30

Glu Gln Glu Ala His Glu Asn Tyr Ile Thr Leu Ala Gln Leu Leu Pro
35 40 45

Glu Ser His Asp Glu Leu Ile Arg Leu Ser Lys Met Glu Ser Arg His
50 55 60

Lys Lys Gly Phe Glu Ala Cys Gly Arg Asn Leu Ala Val Thr Pro Asp

```
65                  70                  75                  80

Leu Gln Phe Ala Lys Glu Phe Phe Ser Gly Leu His Gln Asn Phe Gln
                85                  90                  95

Thr Ala Ala Ala Glu Gly Lys Val Val Thr Cys Leu Leu Ile Gln Ser
            100                 105                 110

Leu Ile Ile Glu Cys Phe Ala Ile Ala Ala Tyr Asn Ile Tyr Ile Pro
        115                 120                 125

Val Ala Asp Asp Phe Ala Arg Lys Ile Thr Glu Gly Val Val Lys Glu
    130                 135                 140

Glu Tyr Ser His Leu Asn Phe Gly Glu Val Trp Leu Lys Glu His Phe
145                 150                 155                 160

Ala Glu Ser Lys Ala Glu Leu Glu Leu Ala Asn Arg Gln Asn Leu Pro
                165                 170                 175

Ile Val Trp Lys Met Leu Asn Gln Val Glu Gly Asp Ala His Thr Met
            180                 185                 190

Ala Met Glu Lys Asp Ala Leu Val Glu Asp Phe Met Ile Gln Tyr Gly
        195                 200                 205

Glu Ala Leu Ser Asn Ile Gly Phe Ser Thr Arg Asp Ile Met Arg Leu
    210                 215                 220

Ser Ala Tyr Gly Leu Ile Gly Ala
225                 230


<210>  313
<211>  22
<212>  DNA
<213>  Artificial sequence

<220>
<223>  Synthetic primer

<400>  313
ggctggctgg cataaatatc tc                                              22


<210>  314
<211>  79
<212>  DNA
<213>  Artificial sequence

<220>
<223>  Synthetic primer
```

<400> 314
gcgttaaagt tgttctgctt catcagcagg atatcctgca ccatcgtctg gattttgaac 60

ttttgctttg ccacggaac 79

<210> 315
<211> 8203
<212> DNA
<213> Artificial sequence

<220>
<223> pSL67-87A plasmid

<400> 315
cactatacca attgagatgg gctagtcaat gataattact agtccttttc ctttgagttg 60

tgggtatctg taaattctgc tagacctttg ctggaaaact tgtaaattct gctagaccct 120

ctgtaaattc cgctagacct ttgtgtgttt tttttgttta tattcaagtg gttataattt 180

atagaataaa gaaagaataa aaaaagataa aaagaataga tcccagccct gtgtataact 240

cactacttta gtcagttccg cagtattaca aaaggatgtc gcaaacgctg tttgctcctc 300

tacaaaacag accttaaaac cctaaaggcg tcggcatccg cttacagaca agctgtgacc 360

gtctccggga gctgcatgtg tcagaggttt tcaccgtcat caccgaaacg cgcgaggcag 420

cagatcaatt cgcgcgcgaa ggcgaagcgg catgcattta cgttgacacc atcgaatggt 480

gcaaaacctt tcgcggtatg gcatgatagc gcccggaaga gagtcaattc agggtggtga 540

atgtgaaacc agtaacgtta tacgatgtcg cagagtatgc cggtgtctct tatcagaccg 600

tttcccgcgt ggtgaaccag gccagccacg tttctgcgaa aacgcgggaa aaagtggaag 660

cggcgatggc ggagctgaat tacattccca accgcgtggc acaacaactg gcgggcaaac 720

agtcgttgct gattggcgtt gccacctcca gtctggccct gcacgcgccg tcgcaaattg 780

tcgcggcgat taaatctcgc gccgatcaac tgggtgccag cgtggtggtg tcgatggtag 840

aacgaagcgg cgtcgaagcc tgtaaagcgg cggtgcacaa tcttctcgcg caacgcgtca 900

gtgggctgat cattaactat ccgctggatg accaggatgc cattgctgtg gaagctgcct 960

gcactaatgt tccggcgtta tttcttgatg tctctgacca gacacccatc aacagtatta 1020

ttttctccca tgaagacggt acgcgactgg gcgtggagca tctggtcgca ttgggtcacc 1080

agcaaatcgc gctgttagcg ggcccattaa gttctgtctc ggcgcgtctg cgtctggctg 1140

gctggcataa atatctcact cgcaatcaaa ttcagccgat agcggaacgg gaaggcgact 1200

ggagtgccat gtccggtttt caacaaacca tgcaaatgct gaatgagggc atcgttccca 1260

ctgcgatgct ggttgccaac gatcagatgg cgctgggcgc aatgcgcgcc attaccgagt 1320

ccgggctgcg cgttggtgcg gatatctcgg tagtgggata cgacgatacc gaagacagct 1380

catgttatat cccgccgtta accaccatca aacaggattt tcgcctgctg gggcaaacca 1440

gcgtggaccg cttgctgcaa ctctctcagg gccaggcggt gaagggcaat cagctgttgc 1500
ccgtctcact ggtgaaaaga aaaaccaccc tggcgcccaa tacgcaaacc gcctctcccc 1560
gcgcgttggc cgattcatta atgcagctgg cacgacaggt ttcccgactg gaaagcgggc 1620
agtgagcgca acgcaattaa tgtaagttag cgcgaattga tctggtttga cagcttatca 1680
tcgactgcac ggtgcaccaa tgcttctggc gtcaggcagc catcggaagc tgtggtatgg 1740
ctgtgcaggt cgtaaatcac tgcataattc gtgtcgctca aggcgcactc ccgttctgga 1800
taatgttttt tgcgccgaca tcataacggt tctggcaaat attctgaaat gagctgttga 1860
caattaatca tccggctcgt ataatgtgtg gaattgtgag cggataacaa tttcacacag 1920
gaaacagcgc cgctgagaaa aagcgaagcg gcactgctct ttaacaattt atcagacaat 1980
ctgtgtgggc actcgaccgg aattatcgat taactttatt attaaaaatt aaagaggtat 2040
atattaatgt atcgattaaa taaggaggaa taaaccatgg cattcggtct tatcggtcat 2100
ctcaccagtt tggagcaggc ccgcgacgtt tctcgcagga tgggctacga cgaatacgcc 2160
gatcaaggat tggagttttg gagtagcgct cctcctcaaa tcgttgatga aatcacagtc 2220
accagtgcca caggcaaggt gattcacggt cgctacatcg aatcgtgttt cttgccggaa 2280
atgctggcgg cgcgccgctt caaaacagcc acgcgcaaag ttctcaatgc catgtcccat 2340
gcccaaaaac acggcatcga catctcggcc ttgggggggct ttacctcgat tattttcgag 2400
aatttcgatt tggccagttt gcggcaagtg cgcgacacta ccttggagtt tgaacggttc 2460
accaccggca atactcacac ggcctacgta atctgtagac aggtggaagc cgctgctaaa 2520
acgctgggca tcgacattac ccaagcgaca gtagcggttg tcggcgcgac tggcgatatc 2580
ggtagcgctg tctgccgctg gctcgacctc aaactgggtg tcggtgattt gatcctgacg 2640
gcgcgcaatc aggagcgttt ggataacctg caggctgaac tcggccgggg caagattctg 2700
cccttggaag ccgctctgcc ggaagctgac tttatcgtgt gggtcgccag tatgcctcag 2760
ggcgtagtga tcgacccagc aaccctgaag caaccctgcg tcctaatcga cggggggctac 2820
cccaaaaact tgggcagcaa agtccaaggt gagggcatct atgtcctcaa tggcggggta 2880
gttgaacatt gcttcgacat cgactggcag atcatgtccg ctgcagagat ggcgcggccc 2940
gagcgccaga tgtttgcctg ctttgccgag gcgatgctct tggaatttga aggctggcat 3000
actaacttct cctggggccg caaccaaatc acgatcgaga agatggaagc gatcggtgag 3060
gcatcggtgc gccacggctt ccaacccttg gcattggcaa tttgagaatt cattccgggg 3120
atccgtcgac ctgcagttcg aagttcctat tctctagaaa gtataggaac ttcagagcgc 3180
ttttgaagct cacgctgccg caagcactca gggcgcaagg gctgctaaag gaagcggaac 3240
acgtagaaag ccagtccgca gaaacggtgc tgaccccgga tgaatgtcag ctactgggct 3300
atctggacaa gggaaaacgc aagcgcaaag agaaagcagg tagcttgcag tgggcttaca 3360

tggcgatagc tagactgggc ggttttatgg acagcaagcg aaccggaatt gccagctggg 3420
gcgccctctg gtaaggttgg gaagccctgc aaagtaaact ggatggcttt cttgccgcca 3480
aggatctgat ggcgcagggg atcaagatct gatcaagaga caggatgagg atcgtttcgc 3540
atgattgaac aagatggatt gcacgcaggt tctccggccg cttgggtgga gaggctattc 3600
ggctatgact gggcacaaca gacaatcggc tgctctgatg ccgccgtgtt ccggctgtca 3660
gcgcaggggc gcccggttct ttttgtcaag accgacctgt ccggtgccct gaatgaactg 3720
caggacgagg cagcgcggct atcgtggctg gccacgacgg gcgttccttg cgcagctgtg 3780
ctcgacgttg tcactgaagc gggaagggac tggctgctat tgggcgaagt gccggggcag 3840
gatctcctgt catctcacct tgctcctgcc gagaaagtat ccatcatggc tgatgcaatg 3900
cggcggctgc atacgcttga tccggctacc tgcccattcg accaccaagc gaaacatcgc 3960
atcgagcgag cacgtactcg gatggaagcc ggtcttgtcg atcaggatga tctggacgaa 4020
gagcatcagg ggctcgcgcc agccgaactg ttcgccaggc tcaaggcgcg catgcccgac 4080
ggcgaggatc tcgtcgtgac ccatggcgat gcctgcttgc cgaatatcat ggtggaaaat 4140
ggccgctttt ctggattcat cgactgtggc cggctgggtg tggcggaccg ctatcaggac 4200
atagcgttgg ctacccgtga tattgctgaa gagcttggcg gcgaatgggc tgaccgcttc 4260
ctcgtgcttt acggtatcgc cgctcccgat tcgcagcgca tcgccttcta tcgccttctt 4320
gacgagttct tctaataagg ggatcttgaa gttcctattc cgaagttcct attctctaga 4380
aagtatagga acttcgaagc agctccagcc tacacgaatt cgaagcttgg gcccgaacaa 4440
aaactcatct cagaagagga tctgaatagc gccgtcgacc atcatcatca tcatcattga 4500
gtttaaacgg tctccagctt ggctgttttg gcggatgaga gaagattttc agcctgatac 4560
agattaaatc agaacgcaga agcggtctga taaaacagaa tttgcctggc ggcagtagcg 4620
cggtggtccc acctgacccc atgccgaact cagaagtgaa acgccgtagc gccgatggta 4680
gtgtggggtc tccccatgcg agagtaggga actgccaggc atcaaataaa acgaaaggct 4740
cagtcgaaag actgggcctt tcgttttatc tgttgtttgt cggtgaacgc tctcctgacg 4800
cctgatgcgg tattttctcc ttacgcatct gtgcggtatt tcacaccgca tatggtgcac 4860
tctcagtaca atctgctctg atgccgcata gttaagccag ccccgacacc cgccaacacc 4920
cgctgacgag cttagtaaag ccctcgctag attttaatgc ggatgttgcg attacttcgc 4980
caactattgc gataacaaga aaaagccagc ctttcatgat atatctccca atttgtgtag 5040
ggcttattat gcacgcttaa aaataataaa agcagacttg acctgatagt ttggctgtga 5100
gcaattatgt gcttagtgca tctaacgctt gagttaagcc gcgccgcgaa gcggcgtcgg 5160
cttgaacgaa ttgttagaca ttatttgccg actaccttgg tgatctcgcc tttcacgtag 5220

tggacaaatt cttccaactg atctgcgcgc gaggccaagc gatcttcttc ttgtccaaga 5280
taagcctgtc tagcttcaag tatgacgggc tgatactggg ccggcaggcg ctccattgcc 5340
cagtcggcag cgacatcctt cggcgcgatt ttgccggtta ctgcgctgta ccaaatgcgg 5400
gacaacgtaa gcactacatt tcgctcatcg ccagcccagt cgggcggcga gttccatagc 5460
gttaaggttt catttagcgc ctcaaataga tcctgttcag gaaccggatc aaagagttcc 5520
tccgccgctg gacctaccaa ggcaacgcta tgttctcttg cttttgtcag caagatagcc 5580
agatcaatgt cgatcgtggc tggctcgaag atacctgcaa gaatgtcatt gcgctgccat 5640
tctccaaatt gcagttcgcg cttagctgga taacgccacg gaatgatgtc gtcgtgcaca 5700
acaatggtga cttctacagc gcggagaatc tcgctctctc caggggaagc cgaagtttcc 5760
aaaaggtcgt tgatcaaagc tcgccgcgtt gtttcatcaa gccttacggt caccgtaacc 5820
agcaaatcaa tatcactgtg tggcttcagg ccgccatcca ctgcggagcc gtacaaatgt 5880
acggccagca acgtcggttc gagatggcgc tcgatgacgc caactacctc tgatagttga 5940
gtcgatactt cggcgatcac cgcttccctc atgatgttta actttgtttt agggcgactg 6000
ccctgctgcg taacatcgtt gctgctccat aacatcaaac atcgacccac ggcgtaacgc 6060
gcttgctgct tggatgcccg aggcatagac tgtaccccaa aaaaacagtc ataacaagcc 6120
atgaaaaccg ccactgcgcc gttaccaccg ctgcgttcgg tcaaggttct ggaccagttg 6180
cgtgagcgca tacgctactt gcattacagc ttacgaaccg aacaggctta tgtccactgg 6240
gttcgtgcct tcatccgttt ccacggtgtg cgtcacccgg caaccttggg cagcagcgaa 6300
gtcgaggcat ttctgtcctg gctggcgaac gagcgcaagg tttcggtctc cacgcatcgt 6360
caggcattgg cggccttgct gttcttctac ggcaaggtgc tgtgcacgga tctgccctgg 6420
cttcaggaga tcggaagacc tcggccgtcg cggcgcttgc cggtggtgct gaccccggat 6480
gaagtggttc gcatcctcgg ttttctggaa ggcgagcatc gtttgttcgc ccagcttctg 6540
tatggaacgg gcatgcggat cagtgagggt ttgcaactgc gggtcaagga tctggatttc 6600
gatcacggca cgatcatcgt gcgggagggc aagggctcca aggatcgggc cttgatgtta 6660
cccgagagct tggcacccag cctgcgcgag caggggaatt aattcccacg ggttttgctg 6720
cccgcaaacg ggctgttctg gtgttgctag tttgttatca gaatcgcaga tccggcttca 6780
gccggtttgc cggctgaaag cgctatttct tccagaattg ccatgatttt ttccccacgg 6840
gaggcgtcac tggctcccgt gttgtcggca gctttgattc gataagcagc atcgcctgtt 6900
tcaggctgtc tatgtgtgac tgttgagctg taacaagttg tctcaggtgt tcaatttcat 6960
gttctagttg ctttgtttta ctggtttcac ctgttctatt aggtgttaca tgctgttcat 7020
ctgttacatt gtcgatctgt tcatggtgaa cagctttgaa tgcaccaaaa actcgtaaaa 7080
gctctgatgt atctatcttt tttacaccgt tttcatctgt gcatatggac agttttccct 7140

```
ttgatatgta acggtgaaca gttgttctac ttttgtttgt tagtcttgat gcttcactga     7200
tagatacaag agccataaga acctcagatc cttccgtatt tagccagtat gttctctagt     7260
gtggttcgtt gtttttgcgt gagccatgag aacgaaccat tgagatcata cttactttgc     7320
atgtcactca aaaattttgc ctcaaaactg gtgagctgaa tttttgcagt taaagcatcg     7380
tgtagtgttt ttcttagtcc gttatgtagg taggaatctg atgtaatggt tgttggtatt     7440
ttgtcaccat tcatttttat ctggttgttc tcaagttcgg ttacgagatc catttgtcta     7500
tctagttcaa cttggaaaat caacgtatca gtcgggcggc ctcgcttatc aaccaccaat     7560
ttcatattgc tgtaagtgtt taaatcttta cttattggtt tcaaaaccca ttggttaagc     7620
cttttaaact catggtagtt attttcaagc attaacatga acttaaattc atcaaggcta     7680
atctctatat ttgccttgtg agttttcttt tgtgttagtt cttttaataa ccactcataa     7740
atcctcatag agtatttgtt ttcaaaagac ttaacatgtt ccagattata ttttatgaat     7800
tttttaact ggaaaagata aggcaatatc tcttcactaa aaactaattc taatttttcg     7860
cttgagaact tggcatagtt tgtccactgg aaaatctcaa agcctttaac caaaggattc     7920
ctgatttcca cagttctcgt catcagctct ctggttgctt tagctaatac accataagca     7980
ttttccctac tgatgttcat catctgagcg tattggttat aagtgaacga taccgtccgt     8040
tctttccttg tagggttttc aatcgtgggg ttgagtagtg ccacacagca taaaattagc     8100
ttggtttcat gctccgttaa gtcatagcga ctaatcgcta gttcatttgc tttgaaaaca     8160
actaattcag acatacatct caattggtct aggtgatttt aat                       8203
```